(19)

Europäisches Patentamt
European Patent Office
Office européen des brevets

(11)    **EP 2 484 769 A2**

(12)    **EUROPEAN PATENT APPLICATION**

(43)   Date of publication:
      08.08.2012 Bulletin 2012/32

(51)   Int Cl.:
      **C12N 15/82** (2006.01)     **C07K 14/415** (2006.01)
      **A01H 5/00** (2006.01)

(21)   Application number: 12160506.7

(22)   Date of filing: 21.12.2005

(84)   Designated Contracting States:
      **AT BE BG CH CY CZ DE DK EE ES FI FR GB GR HU IE IS IT LI LT LU LV MC NL PL PT RO SE SI SK TR**

(62)   Document number(s) of the earlier application(s) in accordance with Art. 76 EPC:
      **05857224.9 / 1 962 577**

(71)   Applicant: **Monsanto Technology LLC**
      **St. Louis, MO 63167 (US)**

(72)   Inventor: **Abad, Mark**
      **St. Louis, MO 63167 (US)**

(74)   Representative: **von Kreisler Selting Werner**
      **Deichmannhaus am Dom**
      **Bahnhofsvorplatz 1**
      **50667 Köln (DE)**

Remarks:
      •This application was filed on 21-03-2012 as a divisional application to the application mentioned under INID code 62.
      •Claims filed after the date of filing of the application/ after the date of receipt of the divisional application (Rule 68(4) EPC).

(54)    **Transgenic plants with enhanced agronomic traits**

(57)    This invention provides transgenic plant cells with recombinant DNA for expression of proteins that are useful for imparting enhanced agronomic trait(s) to transgenic crop plants. This invention also provides transgenic plants and progeny seed comprising the transgenic plant cells where the plants are selected for having an enhanced trait selected from the group of traits consisting of enhanced water use efficiency, enhanced cold tolerance, increased yield, enhanced nitrogen use efficiency, enhanced seed protein and enhanced seed oil. Also disclosed are methods for manufacturing transgenic seed and plants with enhanced traits.

EP 2 484 769 A2

**Description**

**Cross Reference To Related Applications**

This application claims benefit under 35USC § 119(e) of United States provisional application Serial No. 60/638,099, filed 12/21/2004, herein incorporated by reference.

**Incorporation Of Sequence Listing**

Two copies of the sequence listing (Copy I and Copy 2) and a computer readable form (CRF) of the sequence listing, all on CD-Rs, each containing the text file named 38-21(53720)D_seqListing.txt, which is 192,434,176 bytes (measured in MS-WINDOWS) and was created on December 21, 2005, are herein incorporated by reference.

**Incorporation Of Computer Program Listing**

Computer Program Listing folders hmmer-2.3.2 and 347pfamDir are contained on a compact disc and is hereby incorporated herein by reference in their entirety. Folder hmmer-2.3.2 contains the source code and other associated file for implementing the HMMer software for Pfam analysis. Folder 347pfamDir contains 347 Pfam Hidden Markov Models. Both folders were created on the disk on December 21, 2005, having a total size of 53,825,536 bytes (measured in MS-WINDOWS).

**Incorporation Of Table**

Two copies of Tables 2 on CD-Rs, each containing the file named 38-21(53720)D_table2.txt, which is 24,334,336 bytes (measured in MS-WINDOWS), was created on December 21, 2005, and comprises 132 pages when viewed in MS Word, are herein incorporated by reference.

**Field Of The Invention**

Disclosed herein are inventions in the field of plant genetics and developmental biology. More specifically, the present inventions provide plant cells with recombinant DNA for providing an enhanced trait in a transgenic plant, plants comprising such cells, seed and pollen derived from such plants, methods of making and using such cells, plants, seeds and pollen.

**Background Of The Invention**

Transgenic plants with improved agronomic traits such as yield, environmental stress tolerance, pest resistance, herbicide tolerance, improved seed compositions, and the like are desired by both farmers and consumers. Although considerable efforts in plant breeding have provided significant gains in desired traits, the ability to introduce specific DNA into plant genomes provides further opportunities for generation of plants with improved and/or unique traits. Merely introducing recombinant DNA into a plant genome doesn't always produce a transgenic plant with an enhanced agronomic trait. Methods to select individual transgenic events from a population are required to identify those transgenic events that are characterized by the enhanced agronomic trait.

Summary Of The Invention

This invention employs recombinant DNA for expression of proteins that are useful for imparting enhanced agronomic traits to the transgenic plants. Recombinant DNA in this invention is provided in a construct comprising a promoter that is functional in plant cells and that is operably linked to DNA that encodes a protein having at least one amino acid domain in a sequence that exceeds the Pfam gathering cutoff for amino acid sequence alignment with a protein domain family identified by a Pfam name in the group of Pfam domain names as identified in Table 12. In more specific embodiments of the invention the protein expressed in plant cells has an amino acid sequence with at least 90% identity to a consensus amino acid sequence in the group of consensus amino acid sequences consisting of the consensus amino acid sequence constructed for SEQ ID NO: 742 and homologs thereof listed in Table 2 through the consensus amino acid sequence constructed for SEQ ID NO: 1482 and homologs thereof listed in Table 2. In even more specific embodiments of the invention the protein expressed in plant cells is a protein selected from the group of proteins identified in Table 1.

Other aspects of the invention are specifically directed to transgenic plant cells comprising the recombinant DNA of the invention, transgenic plants comprising a plurality of such plant cells, progeny transgenic seed, embryo and transgenic pollen from such plants. Such plant cells are selected from a population of transgenic plants regenerated from plant cells transformed with recombinant DNA and that express the protein by screening transgenic plants in the population for an enhanced trait as compared to control plants that do not have said recombinant DNA, where the enhanced trait is selected from group of enhanced traits consisting of enhanced water use efficiency, enhanced cold tolerance, increased yield, enhanced nitrogen use efficiency, enhanced seed protein and enhanced seed oil.

In yet another aspect of the invention the plant cells, plants, seeds, embryo and pollen further comprise DNA expressing a protein that provides tolerance from exposure to an herbicide applied at levels that are lethal to a wild type of said plant cell. Such tolerance is especially useful not only as a advantageous trait in such plants but is also useful in a selection step in the methods of the invention. In aspects of the invention the agent of such herbicide is a glyphosate, dicamba, or glufosinate compound.

Yet other aspects of the invention provide transgenic plants which are homozygous for the recombinant DNA and transgenic seed of the invention from corn, soybean, cotton, canola, alfalfa, wheat or rice plants. In other important embodiments for practice of various aspects of the invention in Argentina the recombinant DNA is provided in plant cells derived from corn lines that that are and maintain resistance to the Mal de Rio Cuarto virus or the *Puccina sorghi* fungus or both.

This invention also provides methods for manufacturing non-natural, transgenic seed that can be used to produce a crop of transgenic plants with an enhanced trait resulting from expression of stably-integrated, recombinant DNA for expressing a protein having at least one domain of amino acids in a sequence that exceeds the Pfam gathering cutoff for amino acid sequence alignment with a protein domain family identified by a Pfam name in the group of Pfam names identified in Table 12. More specifically the method comprises (a) screening a population of plants for an enhanced trait and a recombinant DNA, where individual plants in the population can exhibit the trait at a level less than, essentially the same as or greater than the level that the trait is exhibited in control plants which do not express the recombinant DNA, (b) selecting from the population one or more plants that exhibit the trait at a level greater than the level that said trait is exhibited in control plants, (c) verifying that the recombinant DNA is stably integrated in said selected plants, (d) analyzing tissue of a selected plant to determine the production of a protein having the function of a protein encoded by nucleotides in a sequence of one of SEQ ID NO:1-741; and (e) collecting seed from a selected plant. In one aspect of the invention the plants in the population further comprise DNA expressing a protein that provides tolerance to exposure to an herbicide applied at levels that are lethal to wild type plant cells and the selecting is effected by treating the population with the herbicide, e.g. a glyphosate, dicamba, or glufosinate compound. In another aspect of the invention the plants are selected by identifying plants with the enhanced trait. The methods are especially useful for manufacturing corn, soybean, cotton, alfalfa, wheat or rice seed.

Another aspect of the invention provides a method of producing hybrid corn seed comprising acquiring hybrid corn seed from a herbicide tolerant corn plant which also has stably-integrated, recombinant DNA comprising a promoter that is (a) functional in plant cells and (b) is operably linked to DNA that encodes a protein having at least one domain of amino acids in a sequence that exceeds the Pfam gathering cutoff for amino acid sequence alignment with a protein domain family identified by a Pfam name in the group of Pfam names identified in Table 12. The methods further comprise producing corn plants from said hybrid corn seed, wherein a fraction of the plants produced from said hybrid corn seed is homozygous for said recombinant DNA, a fraction of the plants produced from said hybrid corn seed is hemizygous for said recombinant DNA, and a fraction of the plants produced from said hybrid corn seed has none of said recombinant DNA; selecting corn plants which are homozygous and hemizygous for said recombinant DNA by treating with an herbicide; collecting seed from herbicide-treated-surviving corn plants and planting said seed to produce further progeny corn plants; repeating the selecting and collecting steps at least once to produce an inbred corn line; and crossing the inbred corn line with a second corn line to produce hybrid seed.

Another aspect of the invention provides a method of selecting a plant comprising plant cells of the invention by using an immunoreactive antibody to detect the presence of protein expressed by recombinant DNA in seed or plant tissue. Yet another aspect of the invention provides anti-counterfeit milled seed having, as an indication of origin, a plant cells of this invention.

Still other aspects of this invention relate to transgenic plants with enhanced water use efficiency or enhanced nitrogen use efficiency. For instance, this invention provides methods of growing a corn, cotton or soybean crop without irrigation water comprising planting seed having plant cells of the invention which are selected for enhanced water use efficiency. Alternatively methods comprise applying reduced irrigation water, e.g. providing up to 300 millimeters of ground water during the production of a corn crop. This invention also provides methods of growing a corn, cotton or soybean crop without added nitrogen fertilizer comprising planting seed having plant cells of the invention which are selected for enhanced nitrogen use efficiency.

**Detailed Description Of The Invention**

As used herein a "plant cell" means a plant cell that is transformed with stably-integrated, non-natural, recombinant DNA, e.g. by *Agrobacterium*-mediated transformation or by baombardment using microparticles coated with recombinant DNA or other means. A plant cell of this invention can be an originally-transformed plant cell that exists as a microorganism or as a progeny plant cell that is regenerated into differentiated tissue, e.g. into a transgenic plant with stably-integrated, non-natural recombinant DNA, or seed or pollen derived from a progeny transgenic plant.

As used herein a "transgenic plant" means a plant whose genome has been altered by the stable integration of recombinant DNA. A transgenic plant includes a plant regenerated from an originally-transformed plant cell and progeny transgenic plants from later generations or crosses of a transformed plant.

As used herein "recombinant DNA" means DNA which has been a genetically engineered and constructed outside of a cell including DNA containing naturally occurring DNA or cDNA or synthetic DNA.

As used herein "consensus sequence" means an artificial sequence of amino acids in a conserved region of an alignment of amino acid sequences of homologous proteins, e.g. as determined by a CLUSTALW alignment of amino acid sequence of homolog proteins.

As used herein "homolog" means a protein in a group of proteins that perform the same biological function, e.g. proteins that belong to the same Pfam protein family and that provide a common enhanced trait in transgenic plants of this invention. Homologs are expressed by homologous genes. Homologous genes include naturally occurring alleles and artificially-created variants. Degeneracy of the genetic code provides the possibility to substitute at least one base of the protein encoding sequence of a gene with a different base without causing the amino acid sequence of the polypeptide produced from the gene to be changed. Hence, a polynucleotide useful in the present invention may have any base sequence that has been changed from SEQ ID NO: I through SEQ ID NO: 741 substitution in accordance with degeneracy of the genetic code. Homologs are proteins that, when optimally aligned, have at least 60% identity, more preferably about 70% or higher, more preferably at least 80% and even more preferably at least 90% identity over the full length of a protein identified as being associated with imparting an enhanced trait when expressed in plant cells. Homologs include proteins with an amino acid sequence that has at least 90% identity to a consensus amino acid sequence of proteins and homologs disclosed herein.

Homologs are be identified by comparison of amino acid sequence, e.g. manually or by use of a computer-based tool using known homology-based search algorithms such as those commonly known and referred to as BLAST, FASTA, and Smith-Waterman. A local sequence alignment program, e.g. BLAST, can be used to search a database of sequences to find similar sequences, and the summary Expectation value (E-value) used to measure the sequence base similarity. As a protein hit with the best E-value for a particular organism may not necessarily be an ortholog or the only ortholog, a reciprocal query is used in the present invention to filter hit sequences with significant E-values for ortholog identification. The reciprocal query entails search of the significant hits against a database of amino acid sequences from the base organism that are similar to the sequence of the query protein. A hit is a likely ortholog, when the reciprocal query's best hit is the query protein itself or a protein encoded by a duplicated gene after speciation. A further aspect of the invention comprises functional homolog proteins that differ in one or more amino acids from those of disclosed protein as the result of conservative amino acid substitutions, for example substitutions are among: acidic (negatively charged) amino acids such as aspartic acid and glutamic acid; basic (positively charged) amino acids such as arginine, histidine, and lysine; neutral polar amino acids such as glycine, serine, threonine, cysteine, tyrosine, asparagine, and glutamine; neutral nonpolar (hydrophobic) amino acids such as alanine, leucine, isoleucine, valine, proline, phenylalanine, tryptophan, and methionine; amino acids having aliphatic side chains such as glycine, alanine, valine, leucine, and isoleucine; amino acids having aliphatic-hydroxyl side chains such as serine and threonine; amino acids having amide-containing side chains such as asparagine and glutamine; amino acids having aromatic side chains such as phenylalanine, tyrosine, and tryptophan; amino acids having basic side chains such as lysine, arginine, and histidine; amino acids having sulfur-containing side chains such as cysteine and methionine; naturally conservative amino acids such as valine-leucine, valine-isoleucine, phenylalanine-tyrosine, lysine-arginine, alanine-valine, aspartic acid-glutamic acid, and asparagine-glutamine. A further aspect of the homologs encoded by DNA useful in the transgenic plants of the invention are those proteins that differ from a disclosed protein as the result of deletion or insertion of one or more amino acids in a native sequence.

As used herein, "percent identity" means the extent to which two optimally aligned DNA or protein segments are invariant throughout a window of alignment of components, for example nucleotide sequence or amino acid sequence. An "identity fraction" for aligned segments of a test sequence and a reference sequence is the number of identical components that are shared by sequences of the two aligned segments divided by the total number of sequence components in the reference segment over a window of alignment which is the smaller of the full test sequence or the full reference sequence. "Percent identity" ("% identity") is the identity fraction times 100.

As used herein "Pfam" refers to a large collection of multiple sequence alignments and hidden Markov models covering many common protein families, e.g. Pfam version 18.0 (August 2005) contains alignments and models for 7973 protein families and is based on the Swissprot 47.0 and SP-TrEMBL 30.0 protein sequence databases. See S.R. Eddy, "Profile Hidden Markov Models", Bioinformatics 14:755-763, 1998. Pfam is currently maintained and updated by a Pfam Consortium. The alignments represent some evolutionary conserved structure that has implications for the protein's function. Profile hidden Markov models (profile HMMs) built from the Pfam alignments are useful for automatically recognizing that a new protein belongs to an existing protein family even if the homology by alignment appears to be low. Once one DNA is identified as encoding a protein which imparts an enhanced trait when expressed in transgenic plants, other DNA encoding proteins in the same protein family are identified by querying the amino acid sequence of protein encoded by candidate DNA against the Hidden Markov Model which characterizes the Pfam domain using HMMER software, a current version of which is provided in the appended computer listing. Candidate proteins meeting the gathering cutoff for the alignment of a particular Pfam are in the protein family and have cognate DNA that is useful in constructing recombinant DNA for the use in the plant cells of this invention. Hidden Markov Model databases for use with HMMER software in identifying DNA expressing protein in a common Pfam for recombinant DNA in the plant cells of this invention are also included in the appended computer listing. The HMMER software and Pfam databases are version 18.0 and were used to identify known domains in the proteins corresponding to amino acid sequence of SEQ ID NO: 742 through SEQ ID NO:1482. All DNA encoding proteins that have scores higher than the gathering cutoff disclosed in Table 12 by Pfam analysis disclosed herein can be used in recombinant DNA of the plant cells of this invention, e.g. for selecting transgenic plants having enhanced agronomic traits. The relevant Pfams for use in this invention, as more specifically disclosed below, are bZIP_1, bZIP_2, Meth_synt_1, Homeobox, Succ_DH_flav_C, RWP-RK, Meth_synt_2, CTP_synth_N, WD40, Sigma70_r2, Sigma70_r3, Fer4, Sigma70_r4, Sigma70_r1_2, CMAS, Sugar_tr, Rubrerythrin, Pro_dh, Ldh_1_C, START, HATPase_c, Cpn10, Glycos_transf_1, Glycos_transf_2, Pkinase, KH_1, cobW, Ldh_1_N, DUF393, SecY, PCI, SRF-TF, IF4E, Lectin_legA, MatE, Dehydrin, Lectin_legB, Ank, 2-Hacid_dh_C, Tic22, Chal_sti_synt_C, AA_kinase, ELFV_dehydrog_N, HLH, Ribonuctease_T2, HEM4, AT_hook, Peptidase_A22B, tRNA-synt_2b, Suc_Fer-like, Glyco_transf_20, MFS_1, HMA, Ketoacyl-synt_C, Steroid_dh, Hydrolase, Peptidase_C1, Ion_trans, Aa_trans, peroxidase, GAF, Cu-oxidase, ABC1, PMSR, B12D, Chromo, Lipase_GDSL, Ran_BP1, DUF125, Lig_chan, GAT, Tub, NPH3, BAH, GFO_IDH_MocA, DUF6, Orn_DAP_Arg_deC, F-box, 3_5_exonuc, NUDIX, Cyclin_C, Trehalase_Ca-bi, Acyltransferase, MtN3_slv, zf-B_box, PUA, AMPKBI, Peptidase_M20, Transaldolase, ketoacyl-synt, Cyclin_N, HisKA, Ribosoinal_L7Ae, Methyltransf_11, Methyltransf_12, Hexapep, Ribosomal_S2, Jacalin, ERp29, MFMR, Usp, DUF641, Pyr_redox_dim, Auxin_resp, Inhibitor_129, Transferase, cNMP_binding, BURP, Epimerase, Ribosomal_L39, Metallothio_2, Pyr_redox_2, WRKY, GSHPx, Kelch_1, Kelch_2, Aminotran_1_2, ABC_tran, UDPGT, Cystatin, YL1, AMP-binding, NTP_transferase, HALZ, Kunitz_legume, HSP20, DUF581, FGGY_N, Aminotran_3, PHD, B56, Aminotran_5, PSI_PsaF, malic, zf-C2H2, HEAT, UPF0057, Asn_synthase, K-box, HAMP, PTR2, SapB_1, Ammonium_transp, SapB_2, GATase, Pyr_redox, Cu-oxidase_2, Cu-oxidase_3, Cyclotide, Asp, M20_dimer, PA, Thiolase_C, FHA, Yjef_N, Citrate_synt, GTP_EFTU_D2, GTP_EFTU_D3, PK, GATA, Thiolase_N, Glycogen_syn, WHEP-TRS, B3, EF1_GNE, FAD_binding_3, ComA, Remorin_C, FAD_binding_7, RmID_sub_bind, CBS, ELFV_dehydrog, YL1,C, zf-Dof, Ribosomal_S11, ArfGap, GRAS, Metallophos, Annexin, Ras, NAC, Acetyltransf_1, Ribosomal_S17, NAF, DUF246, GST_C, CN_hydrolase, Na_Ca_ex, DUF1423, Ubie_methyltran, p450, PP2C, NAM, Histone, GST_N, Tubulin, 2-Hacid_dh, Ribosomal_L19e, CCT, Malic_M, PK_C, VHS, IPK, HSF_DNA-bind, Tubulin_C, Sina, JmjC, CH, Catalase, DUF250, HMG_box, PfkB, Yippee, DSPc, Pkinase_C, UbiA, Ribosomal_S27, ADH_zinc_N, Zip, Globin, JmjN, Cys_Met_Meta_PP, H10933_like, GH3, Bromodomain, ERO1, DAO, DUF760, Methyltransf_2, Gp_dh_C, HGTP_anticodon, Methyltransf_3, Aldo_ket_red, Thioredoxin, NmrA, SelR, LEA_5, Orn_Arg_deC_N, Polysacc_synt_2, Gp_dh_N, NifU_N, GFO_IDH_MocA_C, Gamma-thionin, FBA-1, H_PPase, ADH_N, Heme_oxygenase, AUX_IAA, NAD_binding_4, Auxin_inducible, LIM, Response_reg, Dirigent, E2F_TDP, Di19, Alpha_adaptinC2, efhand, ICL, Rieske, GTP_EFTU, ARID, adh_short, Transket_pyr, AA_permease, TPP_enzyme_C, NDK, RRM_1, Trypsin, Pro_CA, Hexokinase_1, CBFD_NFYB_HMF, Glyco__hydro_38C, TPP_enzyme_M, TPP_enzyme_N, Hexokinase_2, 3Beta_HSD, DUF788, Wzy_C, El_dh, Glycolytic, RuBisCO-small, ZF-HD_dimer, DUF1530, PARP, Pyridoxal_deC, IlvC, Ribosomal_L1, Alpha-amylase, EB I, CorA, Sucrose_synth, PGAM, IlvN, MAP1_LC3, DNA_photolyase, PAD_porph, Abhydrolase 1, Glyco_hydro_16, NTF2, CobW_C, GATase_2, Cation_efflux, Gln-synt_C, VQ, DUF296, W2, SAM_1, SAM_2, Gln-synt_N, Transketolase_C, PEPcase, GRIM-19, Pkinase_Tyr, DnaJ, MIP, PRA1, Trehalose_PPase, Transketolase_N, LRR_2, KA1, Mpv17_PMP22, Reticulon, Trp_syntA, YTH, Aldedh, zf-C3HC4, GIDA, Trp_Tyr_perm, UBA, PB1, PAS, Carb_kinase, zf-LSD1, CAF1, Xan_ur_permease, Hist-deacetyl, Cpn60_TCP1, XET_C, Ribosomal_L10e, Trehalase, ubiquitin, Glyco_hydro_38, AP2, Myb_DNA-binding, APS_kinase, PBD, FAE_3-kCoA_syn1, the databases for which are included in the appended computer listing.

As used herein "promoter" means regulatory DNA for initializing transcription. A "plant promoter" is a promoter capable of initiating transcription in plant cells whether or not its origin is a plant cell, e.g. is it well known that *Agrobacterium* promoters are functional in plant cells. Thus, plant promoters include promoter DNA obtained from plants, plant viruses and bacteria such as *Agrobacterium* and *Bradyrhizobium* bacteria. Examples of promoters under developmental control include promoters that preferentially initiate transcription in certain tissues, such as leaves, roots, or seeds. Such promoters are referred to as "tissue preferred". Promoters that initiate transcription only in certain tissues are referred to as "tissue specific". A "cell type" specific promoter primarily drives expression in certain cell types in one or more organs, for example, vascular cells in roots or leaves. An "inducible" or "repressible" promoter is a promoter which is under environmental control. Examples of environmental conditions that may effect transcription by inducible promoters include anaerobic conditions, or certain chemicals, or the presence of light. Tissue specific, tissue preferred, cell type specific, and inducible promoters constitute the class of "non-constitutive" promoters. A "constitutive" promoter is a promoter which is active under most conditions.

As used herein "operably linked" means the association of two or more DNA fragments in a DNA construct so that the function of one, e.g. protein-encoding DNA, is controlled by the other, e.g. a promoter.

As used herein "expressed" means produced, e.g. a protein is expressed in a plant cell when its cognate DNA is transcribed to mRNA that is translated to the protein.

As used herein a "control plant" means a plant that does not contain the recombinant DNA that expressed a protein that impart an enhanced trait. A control plant is to identify and select a transgenic plant that has an enhance trait. A suitable control plant can be a nontransgenic plant of the parental line used to generate a transgenic plant, i.e. devoid of recombinant DNA. A suitable control plant may in some cases be a progeny of a hemizygous transgenic plant line that is does not contain the recombinant DNA, known as a negative segregant.

As used herein an "enhanced trait" means a characteristic of a transgenic plant that includes, but is not limited to, an enhance agronomic trait characterized by enhanced plant morphology, physiology, growth and development, yield, nutritional enhancement, disease or pest resistance, or environmental or chemical tolerance. In more specific aspects of this invention enhanced trait is selected from group of enhanced traits consisting of enhanced water use efficiency, enhanced cold tolerance, increased yield, enhanced nitrogen use efficiency, enhanced seed protein and enhanced seed oil. In an important aspect of the invention the enhanced trait is enhanced yield including increased yield under non-stress conditions and increased yield under environmental stress conditions. Stress conditions may include, for example, drought, shade, fungal disease, viral disease, bacterial disease, insect infestation, nematode infestation, cold temperature exposure, heat exposure, osmotic stress, reduced nitrogen nutrient availability, reduced phosphorus nutrient availability and high plant density. "Yield" can be affected by many properties including without limitation, plant height, pod number, pod position on the plant, number of internodes, incidence of pod shatter, grain size, efficiency of nodulation and nitrogen fixation, efficiency of nutrient assimilation, resistance to biotic and abiotic stress, carbon assimilation, plant architecture, resistance to lodging, percent seed germination, seedling vigor, and juvenile traits. Yield can also affected by efficiency of germination (including germination in stressed conditions), growth rate (including growth rate in stressed conditions), ear number, seed number per ear, seed size, composition of seed (starch, oil, protein) and characteristics of seed fill.

Increased yield of a transgenic plant of the present invention can be measured in a number of ways, including test weight, seed number per plant, seed weight, seed number per unit area (i.e. seeds, or weight of seeds, per acre), bushels per acre, tonnes per acre, tons per acre, kilo per hectare. For example, maize yield may be measured as production of shelled corn kernels per unit of production area, for example in bushels per acre or metric tons per hectare, often reported on a moisture adjusted basis, for example at 15.5 percent moisture. Increased yield may result from improved utilization of key biochemical compounds, such as nitrogen, phosphorous and carbohydrate, or from improved responses to environmental stresses, such as cold, heat, drought, salt, and attack by pests or pathogens. Recombinant DNA used in this invention can also be used to provide plants having improved growth and development, and ultimately increased yield, as the result of modified expression of plant growth regulators or modification of cell cycle or photosynthesis pathways. Also of interest is the generation of transgenic plants that demonstrate enhanced yield with respect to a seed component that may or may not correspond to an increase in overall plant yield. Such properties include enhancements in seed oil, seed molecules such as tocopherol, protein and starch, or oil particular oil components as may be manifest by an alterations in the ratios of seed components.

A subset of the nucleic molecules of this invention includes fragments of the disclosed recombinant DNA consisting of oligonucleotides of at least 15, preferably at least 16 or 17, more preferably at least 18 or 19, and even more preferably at least 20 or more, consecutive nucleotides. Such oligonucleotides are fragments of the larger molecules having a sequence selected from the group consisting of SEQ ID NO:1 through SEQ ID NO: 741, and find use, for example as probes and primers for detection of the polynucleotides of the present invention.

In some embodiments of the present invention, a dominant negative mutant of a native gene is generated to achieve the desired effect. As used herein, "dominant negative mutant" means a mutant gene whose gene product adversely affects the normal, wild-type gene product within the same cell, usually by dimerizing (combining) with it. In cases of polymeric molecules, such as collagen, dominant negative mutations are often more deleterious than mutations causing the production of no gene product (null mutations or null alleles). For example, SEQ ID NO: 839-842 are constructed to encode agl 11 protein with K-box deleted or C-terminal domain deleted. AGL11 or MADS box protein can be considered as having three functional domains. There is an N-terminal DNA-binding domain (the MADS box), a more distal dimerization domain (the K-box) and a C-terminal domain that is usually involved in interactions with other proteins. In plants the region between the MADS box and the K-box has been shown to be important for DNA binding in some proteins and is often referred to as the I-box (Fan et al., 1997).Several different classes of dominant negative constructs are considered. Deletion or inactivation of the DNA-binding domain can create proteins that are able to dimerize with their native full length counterparts as well as other natural dimerization partners. Likewise, removal of the C-terminal domain can allows dimerization with both the native protein and it's natural dimerization partners. In both cases these types of constructs disable both the target protein and any other protein capable of interacting with the K-box.

In other embodiments of the invention a constitutively active mutant is constructed to achieve the desired effect. For example, SEQ ID NO: 831 and SEQ ID NO: 832 encode only the kinase domain from a calcium-dependent protein kinase (CDPK). CDPK1 has a domain structure similar to other calcium-dependant protein kinases in which the protein kinase domain is separated from four efhand domains by 42 amino acid "spacer" region. Calcium-dependant protein kinases are thought to be activated by a calcium-induced conformational change that results in movement of an autoinhibitory domain away from the protein kinase active site (Yokokura et al., 1995). Thus, constitutively active proteins can be made by over expressing the protein kinase domain alone.

DNA constructs are assembled using methods well known to persons of ordinary skill in the art and typically comprise a promoter operably linked to DNA, the expression of which provides the enhanced agronomic trait. Other construct components may include additional regulatory elements, such as 5' leaders and introns for enhancing transcription, 3' untranslated regions (such as polyadenylation signals and sites), DNA for transit or signal peptides.

Numerous promoters that are active in plant cells have been described in the literature. These include promoters present in plant genomes as well as promoters from other sources, including nopaline synthase (NOS) promoter and octopine synthase (OCS) promoters carried on tumor-inducing plasmids of *Agrobacterium tumefaciens,* caulimovirus promoters such as the cauliflower mosaic virus. For instance, see U.S. Patents No. 5,858,742 and 5,322,938, which disclose versions of the constitutive promoter derived from cauliflower mosaic virus (CaMV35S), U.S. Patent 5,641,876, which discloses a rice actin promoter, U.S. Patent Application Publication 2002/0192813A1, which discloses 5', 3' and intron elements useful in the design of effective plant expression vectors, U.S. patent application Serial No. 09/757,089, which discloses a maize chloroplast aldolase promoter, U.S. patent application Serial No. 08/706,946, which discloses a rice glutelin promoter, U.S. patent application Serial No.09/757,089, which discloses a maize aldolase (FDA) promoter, and U.S. patent application Serial No.60/310, 370, which discloses a maize nicotianamine synthase promoter, all of which are incorporated herein by reference. These and numerous other promoters that function in plant cells are known to those skilled in the art and available for use in recombinant polynucleotides of the present invention to provide for expression of desired genes in transgenic plant cells.

In other aspects of the invention, preferential expression in plant green tissues is desired. Promoters of interest for such uses include those from genes such as Arabidopsis thaliana ribulose-1,5-bisphosphate carboxylase (Rubisco) small subunit (Fischhoff et al. (1992) Plant Mol Biol. 20;81-93), aldolase and pyruvate orthophosphate dikinase (PPDK) (Taniguchi et al. (2000) Plant Cell Physiol. 41(1):42-48).

Furthermore, the promoters may be altered to contain multiple "enhancer sequences" to assist in elevating gene expression. Such enhancers are known in the art. By including an enhancer sequence with such constructs, the expression of the selected protein may be enhanced. These enhancers often are found 5' to the start of transcription in a promoter that functions in eukaryotic cells, but can often be inserted upstream (5') or downstream (3') to the coding sequence. In some instances, these 5' enhancing elements are introns. Particularly useful as enhancers are the 5' introns of the rice actin I (see US Patent 5,641,876)and rice actin 2 genes, the maize alcohol dehydrogenase gene intron, the maize heat shock protein 70 gene intron (U.S. Patent 5,593,874) and the maize shrunken 1 gene.

In other aspects of the invention, sufficient expression in plant seed tissues is desired to effect improvements in seed composition. Exemplary promoters for use for seed composition modification include promoters from seed genes such as napin (U.S. 5,420,034), maize L3 oleosin (U.S. 6,433,252), zein Z27 (Russell et al. (1997) Transgenic Res. 6 (2):157-166), globulin I (Belanger et al (1991) Genetics 129:863-872), glutelin I (Russell (1997) *supra*), and peroxiredoxin antioxidant (Per 1) (Stacy et al. (1996) Plant Mol Biol. 31(6):1205-1216).

Recombinant DNA constructs prepared in accordance with the invention will also generally include a 3' element that typically contains a polyadenylation signal and site. Well-known 3' elements include those from *Agrobacterium tumefaciens* genes such as *nos 3', tml 3', tmr 3', tms 3', ocs 3', tr7 3',* for example disclosed in U.S. 6,090,627, incorporated herein by reference; 3' elements from plant genes such as wheat (*Triticum aesevitum*) heat shock protein 17 (*Hsp17 3'*), a wheat ubiquitin gene, a wheat fructose-1,6-biphosphatase gene, a rice glutelin gene a rice lactate dehydrogenase gene and a rice beta-tubulin gene, all of which are disclosed in U.S. published patent application 2002/0192813 A1, incorporated herein by reference; and the pea (*Pisum sativum*) ribulose biphosphate carboxylase gene (*rbs 3'*), and 3' elements from the genes within the host plant.

Constructs and vectors may also include a transit peptide for targeting of a gene target to a plant organelle, particularly to a chloroplast, leucoplast or other plastid organelle. For descriptions of the use of chloroplast transit peptides see U.S. Patent 5, 188,642 and U.S. Patent No. 5,728,925, incorporated herein by reference. For description of the transit peptide region of an Arabidopsis EPSPS gene useful in the present invention, see Klee, H.J. et al (MGG (1987) 210:437-442).

Transgenic plants comprising or derived from plant cells of this invention transformed with recombinant DNA can be further enhanced with stacked traits, e.g. a crop plant having an enhanced trait resulting from expression of DNA disclosed herein in combination with herbicide and/or pest resistance traits. For example, genes of the current invention can be stacked with other traits of agronomic interest, such as a trait providing herbicide resistance, or insect resistance, such as using a gene from *Bacillus thuringensis* to provide resistance against lepidopteran, coliopteran, homopteran, hemiopteran, and other insects. Herbicides for which transgenic plant tolerance has been demonstrated and the method of the present invention can be applied include, but are not limited to, glyphosate, dicamba, glufosinate, sulfonylurea, bromoxynil and norflurazon herbicides. Polynucleotide molecules encoding proteins involved in herbicide tolerance are well-known in the art and include, but are not limited to, a polynucleotide molecule encoding 5-enolpyruvylshikimate-3-phosphate synthase (EPSPS) disclosed in U.S. Patent 5,094,945; 5,627,061; 5,633,435 and 6,040,497 for imparting glyphosate tolerance; polynucleotide molecules encoding a glyphosate oxidoreductase (GOX) disclosed in U.S. Patent 5,463,175 and a glyphosate-N-acetyl transferase (GAT) disclosed in U.S. Patent Application publication 2003/0083480 A1 also for imparting glyphosate tolerance; dicamba monooxygenase disclosed in U.S. Patent Application publication 2003/0135879 A1 for imparting dicamba tolerance; a polynucleotide molecule encoding bromoxynil nitrilase (*Bxn*) disclosed in U.S. Patent 4,810,648 for imparting bromoxynil tolerance; a polynucleotide molecule encoding phytoene desaturase (*crtl*) described in Misawa et al, (1993) Plant J. 4:833-840 and Misawa et al, (1994) Plant J. 6:481-489 for norflurazon tolerance; a polynucleotide molecule encoding acetohydroxyacid synthase (AHAS, *aka* ALS) described in Sathasiivan et al. (1990) Nucl. Acids Res. 18:2188-2193 for imparting tolerance to sulfonylurea herbicides; polynucleotide molecules known as *bar* genes disclosed in DeBlock, et al. (1987) EMBO J. 6:2513-2519 for imparting glufosinate and bialaphos tolerance; polynucleotide molecules disclosed in U.S. Patent Application Publication 2003/010609 A1 for imparting N-amino methyl phosphonic acid tolerance; polynucleotide molecules disclosed in U.S. Patent 6,107,549 for imparting pyridine herbicide resistance; molecules and methods for imparting tolerance to multiple herbicides such as glyphosate, atrazine, ALS inhibitors, isoxoflutole and glufosinate herbicides are disclosed in U.S. Patent 6,376,754 and U.S. Patent Application Publication 2002/0112260, all of said U.S. Patents and Patent Application Publications are incorporated herein by reference. Molecules and methods for imparting insect/nematode/virus resistance is disclosed in U.S. Patents 5,250,515; 5,880,275; 6,506,599; 5,986,175 and U.S. Patent Application Publication 2003/0150017 A1, all of which are incorporated herein by reference.

In particular embodiments, the inventors contemplate the use of antibodies, either monoclonal or polyclonal which bind to the proteins disclosed herein. Means for preparing and characterizing antibodies are well known in the art (See, e.g., Antibodies: A Laboratory Manual, Cold Spring Harbor Laboratory, 1988; incorporated herein by reference). The methods for generating monoclonal antibodies (mAbs) generally begin along the same lines as those for preparing polyclonal antibodies. Briefly, a polyclonal antibody is prepared by immunizing an animal with an immunogenic composition in accordance with the present invention and collecting antisera from that immunized animal. A wide range of animal species can be used for the production of antisera. Typically the animal used for production of anti-antisera is a rabbit, a mouse, a rat, a hamster, a guinea pig or a *goat.* Because of the relatively large blood volume of rabbits, a rabbit is a preferred choice for production of polyclonal antibodies.

As is well known in the art, a given composition may vary in its immunogenicity. It is often necessary therefore to boost the host immune system, as may be achieved by coupling a peptide or polypeptide immunogen to a carrier. Exemplary and preferred carriers are keyhole limpet hemocyanin (KLH) and bovine serum albumin (BSA). Other albumins such as ovalbumin, mouse serum albumin or rabbit serum albumin can also be used as carriers. Means for conjugating a polypeptide to a carrier protein are well known in the art and include using glutaraldehyde, m-maleimidobencoyl-N-hydroxysuccinimide ester, carbodiimide and bis-biazotized benzidine.

As is also well known in the art, the immunogenicity of a particular immunogen composition can be enhanced by the use of non-specific stimulators of the immune response, known as adjuvants. Exemplary and preferred adjuvants include complete Freund's adjuvant (a non-specific stimulator of the immune response containing killed *Mycobacterium tuberculosis*), incomplete Freund's adjuvants and aluminum hydroxide adjuvant.

The amount of immunogen composition used in the production of polyclonal antibodies varies upon the nature of the immunogen as well as the animal used for immunization. A variety of routes can be used to administer the immunogen (subcutaneous, intramuscular, intradermal, intravenous and intraperitoneal). The production of polyclonal antibodies may be monitored by sampling blood of the immunized animal at various points following immunization. A second, booster, injection may also be given. The process of boosting and titering is repeated until a suitable titer is achieved. When a desired level of immunogenicity is obtained, the immunized animal can be bled and the serum isolated and stored, and/or the animal can be used to generate mAbs.

mAbs may be readily prepared through use of well-known techniques, such as those exemplified in U.S. Pat. No. 4,196,265, incorporated herein by reference. Typically, this technique involves immunizing a suitable animal with a selected immunogen composition, e.g., a purified or partially purified antifungal protein, polypeptide or peptide. The immunizing composition is administered in a manner effective to stimulate antibody producing cells. Rodents such as mice and rats are preferred animals, however, the use of rabbit, sheep, or frog cells is also possible. The use of rats may provide certain advantages (Goding, 1986, pp. 60-61), but mice are preferred, with the BALB/c mouse being most preferred as this is most routinely used and generally gives a higher percentage of stable fusions.

Following immunization, somatic cells with the potential for producing antibodies, specifically B lymphocytes (B cells), are selected for use in the mAb generating protocol. These cells may be obtained from biopsied spleens, tonsils or lymph nodes, or from a peripheral blood sample. Spleen cells and peripheral blood cells are preferred, the former because they are a rich source of antibody-producing cells that are in the dividing plasmablast stage, and the latter because peripheral blood is easily accessible. Often, a panel of animals will have been immunized and the spleen of animal with the highest antibody titer will be removed and the spleen lymphocytes obtained by homogenizing the spleen with a syringe. Typically, a spleen from an immunized mouse contains approximately $5\times 10^7$ to $2\times 10^8$ lymphocytes.

The antibody-producing B lymphocytes from the immunized animal are then fused with cells of an immortal myeloma cell, generally one of the same species as the animal that was immunized. Myeloma cell lines suited for use in hybridoma-producing fusion procedures preferably are non-antibody-producing, have high fusion efficiency, and enzyme deficiencies that render them incapable of growing in certain selective media which support the growth of only the desired fused cells (hybridomas).

Any one of a number of myeloma cells may be used, as are known to those of skill in the art (Goding, 1986, pp. 65-66; Campbell, 1984, pp. 75-83). For example, where the immunized animal is a mouse, one may use P3-X63/Ag8, X63-Ag8.653, NS1/1.Ag 4 1, Sp210-Ag14, FO, NSO/U, MPC-11, MPC11-X45-GTG 1.7 and S19415XX0 Bul; for rats, one may use R210.RCY3, Y3-Ag 1.2.3, IR983F and 4B210; and U-266, GM1500-GRG2, LICR-LON-HMy2 and UC729-6 are all useful in connection with human cell fusions.

One preferred murine myeloma cell is the NS-1 myeloma cell line (also termed P3-NS-1-Ag4-1), which is readily available from the NIGMS Human Genetic Mutant Cell Repository by requesting cell line repository number GM3573. Another mouse myeloma cell line that may be used is the 8-azaguanine-resistant mouse murine myeloma SP2/0 non-producer cell line.

Methods for generating hybrids of antibody-producing spleen or lymph node cells and myeloma cells usually comprise mixing somatic cells with myeloma cells in a 2:1 ratio, though the ratio may vary from about 20:1 to about 1:1, respectively, in the presence of an agent or agents (chemical or electrical) that promote the fusion of cell membranes. Fusion methods using Spend virus have been described (Kohler and Milstein, 1975; 1976), and those using polyethylene glycol (PEG), such as 37% (v/v) PEG, (Gefter et al., 1977). The use of electrically induced fusion methods is also appropriate (Goding, 1986, pp. 71-74).

Fusion procedures usually produce viable hybrids at low frequencies, about $1\times 10^{-6}$ to $1\times 10^{-8}$. However, this does not pose a problem, as the viable, fused hybrids are differentiated from the parental, unfused cells (particularly the unfused myeloma cells that would normally continue to divide indefinitely) by culturing in a selective medium. The selective medium is generally one that contains an agent that blocks the de novo synthesis of nucleotides in the tissue culture media. Exemplary and preferred agents are aminopterin, methotrexate, and azaserine. Aminopterin and methotrexate block de novo synthesis of both purines and pyrimidines, whereas azasenne blocks only purine synthesis. Where aminopterin or methotrexate is used, the media is supplemented with hypoxanthine and thymidine as a source of nucleotides (HAT medium). Where azaserine is used, the media is supplemented with hypoxanthine.

The preferred selection medium is HAT. Only cells capable of operating nucleotide salvage pathways are able to survive in HAT medium. The myeloma cells are defective in key enzymes of the salvage pathway, e.g., hypoxanthine phosphoribosyl transferase (HPRT), and they cannot survive. The B-cells can operate this pathway, but they have a limited life span in culture and generally die within about two weeks. Therefore, the only cells that can survive in the selective media are those hybrids formed from myeloma and B-cells.

This culturing provides a population of hybridomas from which specific hybridomas are selected. Typically, selection of hybridomas is performed by culturing the cells by single-clone dilution in microtiter plates, followed by testing the individual clonal supernatants (after about two to three weeks) for the desired reactivity. The assay should be sensitive, simple and rapid, such as radioimmunoassays, enzyme immunoassays, cytotoxicity assays, plaque assays, dot immunobinding assays, and the like.

The selected hybridomas would then be serially diluted and cloned into individual antibody-producing cell lines, which clones can then be propagated indefinitely to provide mAbs. The cell lines may be exploited for mAb production in two basic ways. A sample of the hybridoma can be injected (often into the peritoneal cavity) into a histocompatible animal of the type that was used to provide the somatic and myeloma cells for the original fusion. The injected animal develops tumors secreting the specific monoclonal antibody produced by the fused cell hybrid. The body fluids of the animal, such as serum or ascites fluid, can then be tapped to provide mAbs in high concentration. The individual cell lines could also be cultured in vitro, where the mAbs are naturally secreted into the culture medium from which they can be readily obtained in high concentrations. mAbs produced by either means may be further purified, if desired, using filtration, centrifugation and various chromatographic methods such as HPLC or affinity chromatography.

**Plant Cell Transformation Methods**

Numerous methods for transforming plant cells with recombinant DNA are known in the art and may be used in the present invention. Two commonly used methods for plant transformation are *Agrobacterium*-mediated transformation and microprojectile bombardment. Microprojectile bombardment methods are illustrated in U.S. Patents 5,015,580 (soybean); 5,550,318 (corn); 5,538,880 (corn); 5,914,451 (soybean); 6,160,208 (corn); 6,399,861 (corn) and 6,153,812 (wheat) and *Agrobacterium*-mediated transformation is described in U.S. Patents 5,159,135 (cotton); 5,824,877 (soybean); 5,591,616 (corn); and 6,384,301 (soybean), all of which are incorporated herein by reference. For *Agrobacterium tumefaciens* based plant transformation system, additional elements present on transformation constructs will include T-DNA left and right border sequences to facilitate incorporation of the recombinant polynucleotide into the plant genome.

In general it is useful to introduce recombinant DNA randomly, i.e. at a non-specific location, in the genome of a target plant line. In special cases it may be useful to target recombinant DNA insertion in order to achieve site-specific integration, for example to replace an existing gene in the genome, to use an existing promoter in the plant genome, or to insert a recombinant polynucleotide at a predetermined site known to be active for gene expression. Several site specific recombination systems exist which are known to function implants include cre-lox as disclosed in U.S. Patent 4,959,317 and FLP-FRT as disclosed in U.S. Patent 5,527,695, both incorporated herein by reference.

Transformation methods of this invention are preferably practiced in tissue culture on media and in a controlled environment. "Media" refers to the numerous nutrient mixtures that are used to grow cells *in vitro,* that is, outside of the intact living organism. Recipient cell targets include, but are not limited to, meristem cells, callus, immature embryos and gametic cells such as microspores, pollen, sperm and egg cells. It is contemplated that any cell from which a fertile plant may be regenerated is useful as a recipient cell. Callus may be initiated from tissue sources including, but not limited to, immature embryos, seedling apical meristems, microspores and the like. Cells capable of proliferating as callus are also recipient cells for genetic transformation. Practical transformation methods and materials for making transgenic plants of this invention, for example various media and recipient target cells, transformation of immature embryo cells and subsequent regeneration of fertile transgenic plants are disclosed in U.S. Patents 6,194,636 and 6,232,526, which are incorporated herein by reference.

The seeds of transgenic plants can be harvested from fertile transgenic plants and be used to grow progeny generations of transformed plants of this invention including hybrid plants line for selection of plants having an enhanced trait. In addition to direct transformation of a plant with a recombinant DNA, transgenic plants can be prepared by crossing a first plant having a recombinant DNA with a second plant lacking the DNA. For example, recombinant DNA can be introduced into first plant line that is amenable to transformation to produce a transgenic plant which can be crossed with a second plant line to introgress the recombinant DNA into the second plant line. A transgenic plant with recombinant DNA providing an enhanced trait, e.g. enhanced yield, can be crossed with transgenic plant line having other recombinant DNA that confers another trait, for example herbicide resistance or pest resistance, to produce progeny plants having recombinant DNA that confers both traits. Typically, in such breeding for combining traits the transgenic plant donating the additional trait is a male line and the transgenic plant carrying the base traits is the female line. The progeny of this cross will segregate such that some of the plants will carry the DNA for both parental traits and some will carry DNA for one parental trait; such plants can be identified by markers associated with parental recombinant DNA, e.g. marker identification by analysis for recombinant DNA or, in the case where a selectable marker is linked to the recombinant, by application of the selecting agent such as a herbicide for use with a herbicide tolerance marker, or by selection for the enhanced trait. Progeny plants carrying DNA for both parental traits can be crossed back into the female parent line multiple times, for example usually 6 to 8 generations, to produce a progeny plant with substantially the same genotype as one original transgenic parental line but for the recombinant DNA of the other transgenic parental line

In the practice of transformation DNA is typically introduced into only a small percentage of target plant cells in any one transformation experiment. Marker genes are used to provide an efficient system for identification of those cells that are stably transformed by receiving and integrating a transgenic DNA construct into their genomes. Preferred marker genes provide selective markers which confer resistance to a selective agent, such as an antibiotic or herbicide. Any of the herbicides to which plants of this invention may be resistant are useful agents for selective markers. Potentially transformed cells are exposed to the selective agent. In the population of surviving cells will be those cells where, generally, the resistance-conferring gene is integrated and expressed at sufficient levels to permit cell survival. Cells may be tested further to confirm stable integration of the exogenous DNA. Commonly used selective marker genes include those conferring resistance to antibiotics such as kanamycin and paromomycin (*nptII*), hygromycin B (*aph IV*) and gentamycin (*aac3* and *aac*C4) or resistance to herbicides such as glufosinate (*bar* or *pat*) and glyphosate (*aroA* or EPSPS). Examples of such selectable are illustrated in U.S. Patents 5,550,318; 5,633,435; 5,780,708 and 6,118,047, all of which are incorporated herein by reference. Selectable markers which provide an ability to visually identify transformants can also be employed, for example, a gene expressing a colored or fluorescent protein such as a luciferase or green fluorescent protein (GFP) or a gene expressing a beta-glucuronidase or *uid*A gene (GUS) for which various chromogenic substrates are known.

Plant cells that survive exposure to the selective agent, or plant cells that have been scored positive in a screening assay, may be cultured in regeneration media and allowed to mature into plants. Developing plantlets regenerated from transformed plant cells can be transferred to plant growth mix, and hardened off, for example, in an environmentally controlled chamber at about 85% relative humidity, 600 ppm $CO_2$, and 25-250 microeinsteins m$^2$ s$^{-1}$ of light, prior to transfer to a greenhouse or growth chamber for maturation. Plants are regenerated from about 6 weeks to 10 months after a transformant is identified, depending on the initial tissue. Plants may be pollinated using conventional plant breeding methods known to those of skill in the art and seed produced, for example self-pollination is commonly used with transgenic corn. The regenerated transformed plant or its progeny seed or plants can be tested for expression of the recombinant DNA and selected for the presence of enhanced agronomic trait.

**Transgenic Plants and Seeds**

Transgenic plants derived from the plant cells of this invention are grown to generate transgenic plants having an enhanced trait as compared to a control plant and produce transgenic seed and haploid pollen of this invention. Such plants with enhanced traits are identified by selection of transformed plants or progeny seed for the enhanced trait. For efficiency a selection method is designed to evaluate multiple transgenic plants (events) comprising the recombinant DNA , for example multiple plants from 2 to 20 or more transgenic events. Transgenic plants grown from transgenic seed provided herein demonstrate improved agronomic traits that contribute to increased yield or other trait that provides increased plant value, including, for example, improved seed quality. Of particular interest are plants having enhanced water use efficiency, enhanced cold tolerance, increased yield, enhanced nitrogen use efficiency, enhanced seed protein and enhanced seed oil.

Table 1 provides a list of protein encoding DNA ("genes") that are useful as recombinant DNA for production of transgenic plants with enhanced agronomic trait, the elements of Table 1 are described by reference to: "PEP SEQ" which identifies an amino acid sequence from SEQ ID NO: 742 to 1482. "NUC SEQ" which identifies a DNA sequence from SEQ ID NO: I to 741. "Base Vector" is a reference to the identifying number in Table 4 of base vectors used for construction of the transformation vectors of the recombinant DNA. Construction of plant transformation constructs is illustrated in Example 1. "PROTEIN NAME" which is a common name for protein encoded by the recombinant DNA.

Table 1.

| PEP SEQ ID NO | NUC SEQ ID NO | GENE ID | Base Vector | PPROTEIN NAME |
|---|---|---|---|---|
| 742 | 1 | PHE0001089_1179 | 1 | Arabidopsis NAC domain transcription factor |
| 743 | 2 | PHE0001133_1223 | 1 | yeast aspartate aminotransferase (AAT2) |
| 744 | 3 | PHE0001134_1224 | 1 | yeast aspartate aminotransferase (AAT1)-CAA97550 |
| 745 | 4 | PHE0001135_1225 | 1 | E. coli aspC - 1651442 |
| 746 | 5 | PHE0001181_1271 | 1 | rice IAA1-like 1 - AJ251791 |
| 747 | 6 | PHE0001227_1317 | 1 | yeas CTT1 - NP_011602 |
| 748 | 7 | PHE0001228_1318 | 1 | yeast STE20 - AAB69747 |
| 749 | 8 | PHE0001247_1338 | 1 | Arabidopsis cyclin D3-like - CGPG 710 |
| 750 | 9 | PHE0001252_1343 | 1 | Arabidopsis arabinogalactan-protein 1 |
| 751 | 10 | PHE0001267_1358 | 1 | Arabidopsis hypothetical protein |
| 752 | 11 | PHE0001275_1336 | 1 | Arabidopsis CIP8 |
| 753 | 12 | PHE0001287_1377 | 1 | Arabidopsis hypothetical protein |
| 754 | 13 | PHE0001382_1474 | 1 | rice Short-Root SHR-like transcriptional factor 1 sequence- |
| 755 | 14 | PHE0001531_1622 | 1 | Bacillus subtilis GDH |

(continued)

| PEP SEQ ID NO | NUC SEQ ID NO | GENE ID | Base Vector | PPROTEIN NAME |
|---|---|---|---|---|
| 756 | 15 | PHE0001532_1623 | 1 | Saccharomyces cerevisiae GDH - 1431821 |
| 757 | 16 | PHE0001535_1626 | 1 | Pseudomonas fluorescens GDH |
| 758 | 17 | PHE0001536_1627 | 1 | Bacillus halodurans GDH |
| 759 | 18 | PHE0001538_1629 | 1 | Synechocystis sp. 6803 GDH |
| 760 | 19 | PHE0001593_1699 | 5 | yeast transketolase |
| 761 | 20 | PHE0001593_1700 | 1 | yeast transketolase |
| 762 | 21 | PHE0002070_2180 | 1 | rice G664-like 2 |
| 763 | 22 | PHE0002128_2236 | 1 | E. coli phosphoenolpyruvate carboxylase |
| 764 | 23 | PHE0002258_69 | 2 | corn nphI-S851D |
| 765 | 24 | PHE0002259_68 | 2 | corn nphI-S849D |
| 766 | 25 | PHE0002260_70 | 2 | corn nphI-S849D-S851D |
| 767 | 26 | PHB0002645_2764 | 1 | Arabidopsis GH3 protein 3 |
| 768 | 27 | PHE0002976_3126 | 1 | Yeast Serine/Threonine Protein Kinase |
| 769 | 28 | PHE0002999_3149 | 1 | Yeast Stereospecific (2R, 3R)-2,3-butanediol dehydrogenase with similarity to alcohol/sorbitol dehydrogenases |
| 770 | 29 | PHE0003000_3150 | 1 | Yeast Car2p: Ornithine aminotransferase |
| 771 | 30 | PHE000300_3151 | 1 | Yeast alpha-Mannosidase |
| 772 | 31 | PHE0003002_3152 | 1 | Yeast Putative indole-3-pyruvate decarboxylase |
| 773 | 32 | PHE0003004_3154 | 1 | Yeast Citrate synthase |
| 774 | 33 | PHE0003006_3156 | 1 | Yeast Galactose-induced protein with strong similarity to crystallin protein of vertebrate eye lens |
| 775 | 34 | PHE0003007_3157 | 1 | Yeast Phosphoglycerate mutase |
| 776 | 35 | PHE0003008_3158 | 1 | Yeast Heat shock protein of 26 kDa, expressed during entry to stationary phase and induced by osmostress |
| 777 | 36 | PHE0003012_3162 | 1 | Yeast Neutral trehalase (alpha, alpha-trehalase), catalyzes the conversion of intracellular trehalose to glucose |
| 778 | 37 | PHE0003014_3164 | 1 | YEAST P16547 MITOCHONDRIAL OUTER MEMBRANE 45 KD PROTEIN |
| 779 | 38 | PHE0003022_3172 | 1 | Yeast Protein of unknown function, has strong similarity to Tallp (Transaldolase PFAM domain) |

(continued)

| PEP SEQ ID NO | NUC SEQ ID NO | GENE ID | Base Vector | PPROTEIN NAME |
|---|---|---|---|---|
| 780 | 39 | PHE0003031_3181 | 1 | Yeast Long-chain fatty acid CoA ligase (fatty acid activator 1) |
| 781 | 40 | PHE0003035_3185 | 1 | Yeast Glucokinase, specific for aldohexoses |
| 782 | 41 | PHE0003037_3187 | 1 | Yeast Aldose reductase with NADPH specificity; induced by osmotic stress |
| 783 | 42 | PHE0003044_3194 | 1 | Yeast Calcium/calmodulin-dependent serine/threonine protein kinase (CaM kinase) type II |
| 784 | 43 | PHE0003056_3206 | 1 | yeast CBK1 |
| 785 | 44 | PHE0003166_3368 | 6 | Agrobacterium tumefaciens str. C58 sucrose phosphorylase |
| 786 | 45 | PHE0003191_3390 | 1 | rice G1225-like 1 |
| 787 | 46 | PHE0003266_3485 | 1 | Arabidopsis unknown protein |
| 788 | 47 | PHE0003295_3515 | 6 | rice unknown protein |
| 789 | 48 | PHE0003358_3581 | 16 | rise dwarf4-like |
| 790 | 49 | PHE0003416_3656 | 16 | Arabidopsis homogentisate phytylprenyltransferase |
| 791 | 50 | PHE0003457_3688 | 1 | rice G1660 like 1 |
| 792 | 51 | PHE0003502_3748 | 4 | rice G2239 like 1 |
| 793 | 52 | PHE0003506_3752 | 1 | rice G2317 like2 |
| 794 | 53 | PHE0003522_3768 | 1 | rice G2536 like1 |
| 795 | 54 | PHE0003592_3838 | 1 | rice G864 like1 |
| 796 | 55 | PHE0003595_3841 | 1 | rice G46 like 1 |
| 797 | 56 | PHE0003633_3891 | 1 | rice G2930 like 1 |
| 798 | 57 | PHE0003635_3893 | 1 | rice G2969 like2 |
| 799 | 58 | PHE0003663_3921 | 1 | rice G1108 like2 |
| 800 | 59 | PHE0003670_3928 | 1 | rice G1792 like3 |
| 801 | 60 | PHE0003672_3930 | 4 | rice G1013 like2 |
| 802 | 61 | PHE0003675_3933 | 1 | rice G1493 like3 |
| 803 | 62 | PHE0003945_4522 | 4 | Arabidopsis CGPG2571 |
| 804 | 63 | PHE0001066_1156 | 1 | yeast SIP1-AAB64887 |
| 805 | 64 | PHE0001471_1563 | 1 | rice NH4-uniport AMT1-like sequence- |
| 806 | 65 | PHE0001530_1621 | 1 | Emericella nidulans GDH |
| 807 | 66 | PHE0002940_3090 | 1 | Yeast Proline oxidase (proline dehydrogenase) |
| 808 | 67 | PHE0002957_3107 | 1 | Yeast MIP (member of major intrinsic protein family of transmembrane channels) |

(continued)

| PEP SEQ ID NO | NUC SEQ ID NO | GENE ID | Base Vector | PPROTEIN NAME |
|---|---|---|---|---|
| 809 | 68 | PHE0002961_3111 | 1 | Yeast protein with 3 RRM domains (RNA recongnition motifs) |
| 810 | 69 | PHE0002962_3112 | 1 | Yeast Putative Dual Specificity Phosphatase |
| 811 | 70 | PHE0003003_3153 | 1 | Yeast Aromatic amino acid aminotransferase II |
| 812 | 71 | PHE0003024_3174 | 1 | Yeast protein of unknown function |
| 813 | 72 | PHE0003042_3192 | 1 | Yeast Possible 6-phosphogluconolactonase |
| 814 | 73 | PHE0003236_3451 | 1 | Arabidopsis unknown protein |
| 815 | 74 | PHE0003280_3499 | 1 | Arabidopsis DUF6 |
| 816 | 75 | PHE0000035_61 | 1 | corn lip19 |
| 817 | 76 | PHE0000036_62 | 1 | corn EFl-alpha |
| 818 | 77 | PHE0000130_220 | 2 | b-glucosidase-aggregating factor |
| 819 | 78 | PHE0000134_224 | 2 | LEA protein EMB5 |
| 820 | 79 | PHE0000135_225 | 2 | PP2C |
| 821 | 80 | PHE0000136_226 | 2 | histone deacetylase |
| 822 | 81 | PHE0000139_229 | 2 | ascorbate peroxidase |
| 823 | 82 | PHE0000141_231 | 1 | cp drought-induced stress protein |
| 824 | 83 | PHE0000142_232 | 2 | rab7a |
| 825 | 84 | PHE0000146_236 | 1 | rab11d |
| 826 | 85 | PHE0000148_238 | 2 | protein kinase |
| 827 | 86 | PHE0000149_239 | 2 | EREbp-like AP2 domain TF |
| 828 | 87 | PHE0000150_240 | 2 | protein kinase |
| 829 | 88 | PHE0000189_282 | 1 | soy HSP20 (HS11) |
| 830 | 89 | PHE0000200_293 | 1 | protein phosphatase 2C-like |
| 831 | 90 | PHE0000211_304 | 2 | CDPK kinase domain |
| 832 | 91 | PHE0000213_306 | 2 | CDPK kinase domain |
| 833 | 92 | PHE0000368_459 | 1 | rice mlip19 |
| 834 | 93 | PHE0000369_460 | 1 | OSK5 |
| 835 | 94 | PHE0000370_461 | 1 | abscisic acid-inducible protein kinase |
| 836 | 95 | PHE0000780_853 | 1 | Arabidopsis agl11 |
| 837 | 96 | PHE0000782_855 | 4 | corn agl11-like 1 |
| 838 | 97 | PHE0000783_856 | 1 | rice MADS3 - L37528 |
| 839 | 98 | PHE0000785_858 | 1 | Arabidopsis agl11 delta C-terminus |
| 840 | 99 | PHE0000791_864 | 1 | soy agl11-like 1 delta C-terminus |
| 841 | 100 | PHE0000792_865 | 1 | soy agl11-like 1 delta K-box |
| 842 | 101 | PHE0000794_867 | 1 | rice MADS3 delta C-terminus - L37528 |

(continued)

| PEP SEQ ID NO | NUC SEQ ID NO | GENE ID | Base Vector | PPROTEIN NAME |
|---|---|---|---|---|
| 843 | 102 | PHE0000821_896 | 1 | corn duf6 10 |
| 844 | 103 | PHE0001051_1141 | 1 | corn zinc finger protein |
| 845 | 104 | PHE0001053_4292 | 11 | corn NAM-like protein |
| 846 | 105 | PHE0001054_1144 | 1 | corn QKI-like RNA-binding protein |
| 847 | 106 | PHE0001055_1145 | 1 | corn putative kinase regulatory subunit |
| 848 | 107 | PHE0001056_1146 | 1 | corn unknown protein |
| 849 | 108 | PHE0001057_1147 | 1 | corn thionin-like protease inhibitor |
| 850 | 109 | PHE0001064_1154 | 1 | yeast GAL83-Q04739 |
| 851 | 110 | PHE0001071_1161 | 1 | Arabidopsis AP2 domain transcription factor |
| 852 | 111 | PHE000 1073_1163 | 1 | Arabidopsis myb domain transcription factor |
| 853 | 112 | PHE0001075_1165 | 1 | Arabidopsis myb domain transcription factor |
| 854 | 113 | PHE0001077_1167 | 1 | Arabidopsis GARP domain transcription factor |
| 855 | 114 | PHE0001083_1173 | 1 | Arabidopsis AP2 domain transcription factor |
| 856 | 115 | PHE0001095_1185 | 1 | Arabidopsis bZIP domain transcription factor |
| 857 | 116 | PHE0001096_1186 | 1 | Arabidopsis bZIP domain transcription factor |
| 858 | 117 | PHE0001097_1187 | 1 | Arabidopsis bZIP/TGA domain transcription factor- |
| 859 | 118 | PHE0001098_1188 | 1 | Arabidopsis GATA domain transcription factor |
| 860 | 119 | PHE0001099_1189 | 1 | Arabidopsis bZIP domain transcription factor |
| 861 | 120 | PHE0001101_1191 | 1 | Arabidopsis GATA domain transcription factor |
| 862 | 121 | PHE0001107_1197 | 1 | rice CCA1-like 1 |
| 863 | 122 | PHE0001108_1198 | 1 | rice alanine aminotransferase 2 - AAK52114 |
| 864 | 123 | PHE0001109_1199 | 1 | corn alanine aminotransferase 1 |
| 865 | 124 | PHE0001110_1200 | 1 | corn alanine aminotransferase 4 |
| 866 | 125 | PHE0001115_1205 | 5 | yeast 5-aminolevulinic acid synthase mature form - P09950 |
| 867 | 126 | PHE0001120_1210 | 4 | corn G1820 like 1 |
| 868 | 127 | PHE0001123_1213 | 1 | corn G1820-like 2 |
| 869 | 128 | PHE0001125_1215 | 1 | soy G1820 like 2 |

(continued)

| PEP SEQ ID NO | NUC SEQ ID NO | GENE ID | Base Vector | PPROTEIN NAME |
|---|---|---|---|---|
| 870 | 129 | PHE0001126_1216 | 1 | corn putative myb-related transcription factor |
| 871 | 130 | PHE0001127_1217 | 1 | corn putative dehydration-responsive protein RD22 precursor- |
| 872 | 131 | PHE0001128_1218 | 1 | corn homeodomain leucine zipper protein |
| 873 | 132 | PHE0001130_1220 | 5 | corn acetohydroxyacid reductoisomerase, chloroplast precursor- |
| 874 | 133 | PHE0001132_1222 | 1 | corn cytosolic aspartate transaminase |
| 875 | 134 | PHE0001138_1228 | 1 | corn hypothetical protein |
| 876 | 135 | PHE0001145_1235 | 1 | corn unknown protein |
| 877 | 136 | PHE0001146_1236 | 1 | corn MAP kinase kinase |
| 878 | 137 | PHE0001147_1237 | 1 | corn hypothetical protein |
| 879 | 138 | PHE0001148_1238 | 1 | corn transfactor-like protein |
| 880 | 139 | PHE0001151_1241 | 1 | corn dehydration-responsive protein RD22 precursor- |
| 881 | 140 | PHE0001152_1242 | 1 | corn histone H3 |
| 882 | 141 | PHE0001153_1243 | 1 | corn calcium-dependent protein kinase |
| 883 | 142 | PHB0001154_1244 | 1 | corn histone H2B |
| 884 | 143 | PHE0001156_1246 | 1 | corn ATFP4-like |
| 885 | 144 | PHE0001157_1247 | 1 | corn unknown protein |
| 886 | 145 | PHE0001 158_1248 | 1 | corn glucose-6-phosphate/ phosphate-translocator precursor- |
| 887 | 146 | PHE0001159_1249 | 1 | corn DnaJ-like protein |
| 888 | 147 | PHE0001167_257 | 1 | rem1-Mu |
| 889 | 148 | PHE0001171_1261 | 1 | soy TU8-like 1 |
| 890 | 149 | PHE0001172_1262 | 1 | soy TU8-like protein 2 |
| 891 | 150 | PHE0001179_1269 | 1 | corn AXR2-like 2 |
| 892 | 151 | PHE0001198_1288 | 1 | soybean G 175-like 2 |
| 893 | 152 | PHE0001199_1289 | 1 | soybean G175-like 1 |
| 894 | 153 | PHE0001208_1298 | 1 | soybean G22-like 1 |
| 895 | 154 | PHE0001211_1301 | 1 | soybean G867-like 1 |
| 896 | 155 | PHE0001212_1302 | 1 | STE11-NP 013466 |
| 897 | 156 | PHE0001214_1304 | 1 | soybean STE11-like 1 |
| 898 | 157 | PHE0001215_1305 | 1 | soybean G1836-like 1 |
| 899 | 158 | PHE0001220_1310 | 1 | soybean AtGSK3-like 2 |
| 900 | 159 | PHE0001221_1311 | 1 | maize AtGSK3-like 1 |
| 901 | 160 | PHE0001225_1315 | 1 | maize catalase-like 1 |

(continued)

| PEP SEQ ID NO | NUC SEQ ID NO | GENE ID | Base Vector | PPROTEIN NAME |
|---|---|---|---|---|
| 902 | 161 | PHE0001238_1328 | 1 | maize cup 1a-likel 1 |
| 903 | 162 | PHE0001239_1329 | 1 | maizeAtGSK3-like 2 |
| 904 | 163 | PHE0001241_1331 | 1 | Putative RNA Binding protein- |
| 905 | 164 | PHE0001242_1332 | 1 | Protein Disulfide Isomerase- |
| 906 | 165 | PHE0001244_1334 | 1 | LIM DOmain Transcription Factor SF3- |
| 907 | 166 | PHE0001245_1335 | 1 | Unkown Protein |
| 908 | 167 | PHE0001246_1337 | 1 | soy CIP8-like 1 |
| 909 | 168 | The0001254_1345 | 1 | Arabidopsis hypothetical protein [NM_111482] |
| 910 | 169 | The0001258_1349 | 1 | corn NADH:ubiquinone oxidoreductase |
| 911 | 170 | PHE0001259_1350 | 1 | soy NADH:ubiquinone oxidoreductase |
| 912 | 171 | PHE0001279_1369 | 1 | corn cysteine proteinase inhibitor |
| 913 | 172 | PHE0001280_1370 | 1 | soy cysteine proteinase inhibitor |
| 914 | 173 | PHE0001282_1372 | 1 | corn fatty acid elongase 1 |
| 915 | 174 | PHE0001284_1374 | 1 | soy ADP-ribosylation factor 1 GTPase activating protein- |
| 916 | 175 | PHE0001285_1375 | 1 | corn ADP-ribosylation factor 1 GTPase activating protein- |
| 917 | 176 | PHE0001291_1381 | 1 | soy hypothetical protein |
| 918 | 177 | PHE0001292_1382 | 1 | corn hypothetical protein |
| 919 | 178 | PHE0001297_387 | 1 | corn cryptochrome like protein 1 |
| 920 | 179 | PHE0001299_1389 | 1 | corn cryptochrome like protein 5 |
| 921 | 180 | PHE0001303_1393 | 1 | rice Pra2-like protein 2 |
| 922 | 181 | PHE0001306_1396 | 1 | soy Pra2-like protein 1 |
| 923 | 182 | PHE0001312_1402 | 1 | corn CGPG 1145-like protein 1 |
| 924 | 183 | PHE0001313_1403 | 1 | corn CGPG 1145-like protein 2 |
| 925 | 184 | PHE0001314_1404 | 1 | corn CGPG 1145-like protein 3 |
| 926 | 185 | PHE0001332_1423 | 1 | rice SNF1-like protein 4-BAB61199 |
| 927. | 186 | PHE0001333_1424 | 1 | rice SNFI - like protein 5-BAA96628 |
| 928 | 187 | PHE0001334_1425 | 1 | rice SNFI-like protein 6 [OSKI]-D82039 |
| 929 | 188 | PHE0001337_1428 | 1 | rice AKIN-like protein 1 |
| 930 | 189 | PHE0001339_1430 | 1 | soy AKIN-like protein 1 |
| 931 | 190 | PHE0001343_1434 | 1 | soy SNP1-like protein 3 |
| 932 | 191 | PHE0001346_1437 | 1 | corn SNF1-like protein 1 |
| 933 | 192 | PHE0001347_1438 | 1 | corn SNF1-like protein 2 |
| 934 | 193 | PHE0001349_1440 | 1 | corn SNF1-like protein 4 |

(continued)

| PEP SEQ ID NO | NUC SEQ ID NO | GENE ID | Base Vector | PPROTEIN NAME |
|---|---|---|---|---|
| 935 | 194 | PHE0001350_1441 | 1 | corn SNF1-like protein 5 |
| 936 | 195 | PHE0001354_1445 | 1 | corn SNF1-like protein 9 |
| 937 | 196 | PHE001355_1446 | 1 | corn SNF1-like protein 10 |
| 938 | 197 | PHE0001356_1447 | 1 | corn SNF1-like protein 11 |
| 939 | 198 | PHE0001357_1448 | 1 | corn SNF1-like protein 13 |
| 940 | 199 | PHE0001358_1449 | 1 | corn SNF1-like protein 14 |
| 941 | 200 | PHE0001359_1450 | 1 | corn SNF1-like protein 15 |
| 942 | 201 | PHE0001360_1451 | 1 | Corn Putative CCR4 Associated Factor |
| 943 | 202 | PHE0001361_1452 | 1 | Soy Putative AP2 Domain Transcription Factor |
| 944 | 203 | PHE0001362_1453 | 1 | Soy GATA Binding Transcription Factor |
| 945 | 204 | PHE0001363_1455 | 1 | NAC 1 type transcriptional activator- |
| 946 | 205 | PHE0001364_1456 | 1 | Corn Putative Transcriptional Regulator X2 |
| 947 | 206 | PHE0001375_1467 | 1 | Corn PRL 1 |
| 948 | 207 | PHE0001377_1469 | 1 | rice PINOID-like protein kinase 1 sequence- |
| 949 | 208 | PHE0001380_1472 | 1 | rice PP2A regulatory subunit A RCN 1-like 1 sequence- |
| 950 | 209 | PHE0001385_1477 | 1 | maize IAA-alanine resistance protein IAR1-like sequence- |
| 951 | 210 | PHE0001386_1478 | 1 | maize IAA-Ala hydrolase like 1 sequence- |
| 952 | 211 | PHE0001389_1481 | 1 | maize nitrilase 1 like 1 sequence- |
| 953 | 212 | PHE0001392_1484 | 1 | soybean root-specific kinase ARSK1 like sequence- |
| 954 | 213 | PHE0001394_1486 | 1 | soybean MADS-box protein AGL14-like sequence- |
| 955 | 214 | PHE0001397_1489 | 1 | soybean Ran binding protein RanBP1-tike 1 sequence- |
| 956 | 215 | PHE0001398_1490 | 1 | maize Ran binding protein RanBP1-Like 1 sequence - |
| 957 | 216 | PHE0001405_1497 | 1 | nifU like protein Homolog- |
| 958 | 217 | PHE0001411_1503 | 1 | photosystem-I PSI-F subunit precursor- |
| 959 | 218 | PHE0001416_1508 | 1 | growth-on protein GRO 10- |
| 960 | 219 | PHE0001417_1509 | 1 | Transcription Factor Homolog BTF3 - |
| 961 | 220 | PHE0001418_1510 | 1 | Putative CGI- 19 Protein Homolog- |
| 962 | 221 | PH E0001421_1513 | 1 | glutamate dehydrogenase- |

(continued)

| PEP SEQ ID NO | NUC SEQ ID NO | GENE ID | Base Vector | PPROTEIN NAME |
|---|---|---|---|---|
| 963 | 222 | PHE0001426_1518 | 1 | Rice GATA Factor Homologs- |
| 964 | 223 | PHE0001436_1528 | 1 | Methionine Synthase- |
| 965 | 224 | PHE0001437_1529 | 1 | Putative ABC Transporter- |
| 966 | 225 | PHE0001442_1534 | 1 | corn C-24 sterol methyltransferase |
| 967 | 226 | PHE0001444_1536 | 1 | soy hypothetical protein |
| 968 | 227 | PHE0001452_1544 | 1 | maize root hair Ted2-like sequence- |
| 969 | 228 | PHE00014G3_1555 | 1 | soybean homeodomain protein (GLABRA2) like sequence- |
| 970 | 229 | PHE0001477_1569 | 1 | soybean werewolf (WER) like sequence- |
| 971 | 230 | PHE0001481_1573 | 1 | rice ethylene-response ETR1 like sequence- |
| 972 | 231 | PHE0001483_1575 | 1 | soybean xyloglucan endotransglycosylase (XET) like 1 sequence- |
| 973 | 232 | PHE0001516_1607 | 1 | Zea Mays SelR Domain protein |
| 974 | 233 | PHE0001522_1613 | 1 | corn LPE1-like permease 6 |
| 975 | 234 | PHE0001539_1630 | 1 | soy glutamate dehydrogenase |
| 976 | 235 | PHE0001577_1676 | 1 | Rice Homolog Putative Transcription Factor X2- |
| 977 | 236 | PHE0001602_1713 | 1 | rice rac-like GTP binding protein like 1 sequence |
| 978 | 237 | PHE0001604_1715 | 1 | rice rac-like GTP binding protein like 3 sequence |
| 979 | 238 | PHE0001605_1716 | 1 | rice rac-like GTP binding protein like 4 sequence |
| 980 | 239 | PHE0001606_1717 | 1 | rice rac-like GTP binding protein like 5 sequence |
| 981 | 240 | PHE0001610_1721 | 1 | maize translation initiation factor 3 delta subunit like sequence |
| 982 | 241 | PHE0001617_1728 | 1 | maize metal transporter ZIP8 like 1sequence |
| 983 | 242 | PHE0001624_1735 | 1 | maize magnesium transporter, mrs2-2-like 1 sequence |
| 984 | 243 | PHE0001629_1740 | 1 | maize low temperature and salt responsive protein LTI6A-like 1 sequence |
| 985 | 244 | PHE0001642 1753 | 1 | rice salt-induced-zinc-finger-protein 1sequence |
| 986 | 245 | PHE0001649_1760 | 1 | soybean salt-tolerance protein 1 seqeunce |
| 987 | 246 | PHE0002017_ 2128 | 1 | Corn RING Finger Transcription Factor |

(continued)

| PEP SEQ ID NO | NUC SEQ ID NO | GENE ID | Base Vector | PPROTEIN NAME |
|---|---|---|---|---|
| 988 | 247 | PHE0002023_2134 | 1 | Corn RING Finger Transcription Factor II |
| 989 | 248 | PHE0002024_2135 | 1 | Corn RING Finger Transcription Factor III |
| 990 | 249 | PHE0002027_2138 | 1 | Glutamine Synthetase |
| 991 | 250 | PHE0002041_2151 | 1 | Corn ubiquitin fusion protein/ ribosomal protein S27a |
| 992 | 251 | PHE0002042_2152 | 1 | soybean AtHSP17.6A like 1 sequence |
| 993 | 252 | PHE0002049_2159 | 1 | Ribulose bisphosphate carboxylase small chain chloroplast precursor |
| 994 | 253 | PHE0002052_2162 | 1 | Corn ligand-gated channel-like protein precursor |
| 995 | 254 | PHE0002054_2164 | 1 | Corn Nitrilase 1 |
| 996 | 255 | PHE0002055_2163 | 1 | Corn Tic 22 Like protein |
| 997 | 256 | PHE0002067_2177 | 1 | corn G664-like 5 |
| 998 | 257 | PHE0002068_2178 | 1 | corn G664-like 6 |
| 999 | 258 | PHE0002071_2181 | 1 | soy G664-like 2 |
| 1000 | 259 | PHE0002080_4290 | 11 | corn sec61 |
| 1001 | 260 | PHE0002081_2191 | 1 | soy sec61 |
| 1002 | 261 | PHE0002088_2198 | 1 | rice osr8 |
| 1003 | 262 | PHE0002095_2205 | 1 | Corn Calmodulin EF hand |
| 1004 | 263 | PHE0002096_2206 | 1 | Rice Calmodulin EF Hand |
| 1005 | 264 | The0002099_2209 | 1 | Rice Putative Calmodulin EF Hand Protein |
| 1006 | 265 | PHE0002103_2213 | 1 | rice Bobs kinase 2 |
| 1007 | 266 | PHE0002123_2231 | 4 | rice phosphoenolpyruvate carboxylase 2 |
| 1008 | 267 | PHE0002125_2233 | 1 | corn phosphoenolpyruvate carboxylase 2 |
| 1009 | 268 | PHE0002127_2235 | 1 | soy phosphoenolpyruvate carboxylase 2 |
| 1010 | 269 | PHE0002145_2253 | 1 | Corn Unkown Protein |
| 1011 | 270 | PHE0002148_2256 | 1 | Corn Cytochrome P450 |
| 1012 | 271 | PHE0002163_2270 | 1 | Corn Protein (Similar to peptidyl prolyl isomerase) |
| 1013 | 272 | PHE0002164_2271 | 1 | Corn Protein (similar to yippie-1 & a peptidyl prolyl isomerase) |
| 1014 | 273 | PHE0002183_2290 | 1 | soybean Hsp17.7 like 1 sequence |
| 1015 | 274 | PHE0002189_2296 | 1 | maize pyrroline-5-carboxylate synthetase like 2 sequence |
| 1016 | 275 | PHE0002202_2309 | 1 | soy annexin 1 |

(continued)

| PEP SEQ ID NO | NUC SEQ ID NO | GENE ID | Base Vector | PPROTEIN NAME |
|---|---|---|---|---|
| 1017 | 276 | PHE0002203_2310 | 1 | soy annexin 3 |
| 1018 | 277 | PHE0002204_2311 | 1 | soy annexin 4 |
| 1019 | 278 | PHE0002210_2317 | 1 | soybean drought-induced protein BnD22 like 1 sequence |
| 1020 | 279 | PHE0002224_2331 | 1 | maize drought-induced protein Di19-like sequence |
| 1021 | 280 | PHE0002227_2334 | 1 | maize protease inhibitor like 2 sequence |
| 1022 | 281 | PHE0002232_2339 | 1 | soybean ABA-responsive element binding protein 2 (AREB2) like sequence |
| 1023 | 282 | PHE0002233_2340 | 1 | Arabidopsis ABA-responsive element binding protein 3 (AREB3) like sequence |
| 1024 | 283 | PHE0002238_2345 | 1 | soybean CPRD14 like 1 sequence |
| 1025 | 284 | PHE0002239_2346 | 1 | Brassica napus CPRD14 like I sequence |
| 1026 | 285 | PHE0002240_2347 | 1 | soybean CPRD14 like 2 sequence |
| 1027 | 286 | PHE0002242_2349 | 1 | maize CPRD14 like 1 sequence |
| 1028 | 287 | PHE0002244_2351 | 1 | Arabidopsis drought inducible heat shock transcription factor like sequence |
| 1029 | 288 | PHE0002246_2353 | 1 | soybean protein phosphatase 2C like sequence |
| 1030 | 289 | PHE0002254_2361 | 1 | soybean CPRD12 like 3 sequence |
| 1031 | 290 | PHE0002268_2371 | 1 | soybean calcium-dependent protein kinase like 1 sequence |
| 1032 | 291 | PHE0002269_2372 | 1 | rice calcium-dependent protein kinase like 1 sequence |
| 1033 | 292 | PHE0002272_2375 | 1 | rice calcium-dependent protein kinase like 4 sequence |
| 1034 | 293 | PHE0002298_2400 | 4 | rice Dehydrin ERD10 like 1 sequence |
| 1035 | 294 | PHE0002299_2401 | 1 | rice drought-induced S-like ribonuclease like 1 sequence |
| 1036 | 295 | PHE0002321_2422 | 1 | maize Di19 like sequence |
| 1037 | 296 | PHE0002331_2432 | 1 | soybean PIP like 1sequence |
| 1038 | 297 | PHE0002340_2441 | 1 | soybean MIP4 like 1sequence |
| 1039 | 298 | PHE0002354_2455 | 1 | maize glutathione transferase like 2 sequence |
| 1040 | 299 | PHE0002357_2458 | 1 | maize 8-oxoguanine-DNA glycosylase like sequence |
| 1041 | 300 | PHE0002361_2462 | 1 | rice CEO1 like sequence |

(continued)

| PEP SEQ ID NO | NUC SEQ ID NO | GENE ID | Base Vector | PPROTEIN NAME |
|---|---|---|---|---|
| 1042 | 301 | PHE0002362_2463 | 1 | maize dolichyl-phosphate beta-glucosyltransferase like sequence |
| 1043 | 302 | PHE0002364_2465 | 1 | maize glutathione reductase (GR2) like 1 sequence |
| 1044 | 303 | PHE0002365_2466 | 1 | soybean glutathione reductase (GR2) like 1sequence |
| 1045 | 304 | PHE0002367_2468 | 1 | rice glutathione reductase (GR2) like 1 sequence |
| 1046 | 305 | PHE0002370_2471 | 1 | soybean glutathione-peroxidase like 1sequence |
| 1047 | 306 | PHE0002372_2473 | 1 | maize glutathione-peroxidase like 2 sequence |
| 1048 | 307 | PHE0002373_2474 | 1 | maize glutathione-peroxidase like 3 sequence |
| 1049 | 308 | PHE0002383_2484 | 1 | maize T-complex polypeptide 1alpha subunit like sequence |
| 1050 | 309 | PHE0002390_2491 | 1 | soybean methionine sulfoxide reductase (msr) like sequence |
| 1051 | 310 | PHE0002414_2514 | 1 | Arabidopsis GAD2 |
| 1052 | 311 | PHE0002431_2531 | 1 | Rice Leucine Zipper Protein similar to At103 and PNIL34 |
| 1053 | 312 | PHE0002447_2547 | 1 | soybean arabidopsis-heat-shock-TF like 1 sequence |
| 1054 | 313 | PHE0002449_2549 | 1 | soybean heat shock factor 6 like 1sequence |
| 1055 | 314 | PHE0002459_2559 | 1 | rice heat shock TF like sequence |
| 1056 | 315 | PHE0002461_2561 | 1 | soybean HSF1 like 1 sequence |
| 1057 | 316 | PHE0002462_2562 | 1 | soybean HSF4 like 1 sequence |
| 1058 | 317 | PHE0002464_2564 | 1 | soybean HSF4 like 3 sequence |
| 1059 | 318 | PHE0002470_2570 | 1 | soybean hsp17.4 like 2 sequence |
| 1060 | 319 | PHE0002471_2571 | 1 | soybean hsp17.4 like 3 sequence |
| 1061 | 320 | PHE0002484_2584 | 1 | Corn Sucrose Synthase |
| 1062 | 321 | PHE0002486_2586 | 1 | Corn Putative protein phosphatase 2A regulatory subunit B |
| 1063 | 322 | PHE0002489_2589 | 1 | Arabidopsis CycD2 |
| 1064 | 323 | PHE0002490_2590 | 1 | Arabidopsis CycD3 |
| 1065 | 324 | PHE0002490_3963 | 16 | Arabidopsis CycD3 |
| 1066 | 325 | PHE0002491_2591 | 1 | Arabidopsis CycB1 |
| 1067 | 326 | PHE0002498_2598 | 1 | Corn Protein Kinase like protein |
| 1068 | 327 | PHE0002502_2602 | 1 | Corn 20 KDA CHAPERONIN, CHLOROPLAST PRECURSOR (PROTEIN CPN21) |

(continued)

| PEP SEQ ID NO | NUC SEQ ID NO | GENE ID | Base Vector | PPROTEIN NAME |
|---|---|---|---|---|
| 1069 | 328 | PHE0002506_2606 | 1 | Corn protein of unknown function wi PFAM domain similar to human Reticulon and may associate with ER |
| 1070 | 329 | PHE0002507_2607 | 1 | Corn Transfactor like protein |
| 1071 | 330 | PHE0002514_264 | 1 | Corn branched-chain alpha keto-acid dehydrogenase E1 alpha subunit |
| 1072 | 331 | PHE0002515_2615 | 1 | Corn haloacid dehalogenase-like hydrolase Putative ripening related protein |
| 1073 | 332 | PHE0002530_2630 | 1 | Rice Dof zinc finger protein |
| 1074 | 333 | PHE0002532_2632 | 1 | Corn ribosomal protein L1p/L10e family |
| 1075 | 334 | PHE0002536_2636 | 1 | Rice auxin response transcription factor 3-like protein |
| 1076 | 335 | PHE0002540_2640 | 1 | Oryza Sativa auxin response factor 2 |
| 1077 | 336 | PHE0002547_2647 | 1 | Corn homolog to Arabidopsis protein wi hydrolase fold |
| 1078 | 337 | PHE0002551_2651 | 1 | Corn TF with 3 leucine zippers & PFAM mTERF domain |
| 1079 | 338 | PHE0002552_2632 | 1 | Corn protein with Duf2 10 PFAM domain |
| 1080 | 339 | PHE0002565_2664 | 1 | Corn metallothionein-like protein |
| 1081 | 340 | PHE0002581_2680 | 1 | maize hsp60 like 1 sequence |
| 1082 | 341 | PHE0002582_2681 | 1 | maize hsp60 like 2 sequence |
| 1083 | 342 | PHE0002583_2682 | 1 | maize hsp60 like 3 sequence |
| 1084 | 343 | PHE0002586_2685 | 1 | rice hsp60 like 1 sequence |
| 1085 | 344 | PHE0002588_2687 | 1 | Corn protein with "Universal Stress Protein Family" PFAM domain |
| 1086 | 345 | PHE0002591_2690 | 1 | Corn protein similar to Yersinia pestis membrane protein and Bacillus Subtilis yuxK |
| 1087 | 346 | PHE0002596_2695 | 1 | Corn Myb related Transcription Factor |
| 1088 | 347 | PHE0002604_2703 | 1 | Rice putative putative photoreceptor-interacting protein-like protein |
| 1089 | 348 | PHE0002608_2707 | 1 | Arabidopsis sigma factor 2 |
| 1090 | 349 | PHE0002615_2714 | 1 | corn sigA binding protein 2 |
| 1091 | 350 | PHE0002622_2739 | 1 | Arabidopsis GLK1 |
| 1092 | 351 | PHE0002629_2748 | 1 | rice trehalose-6-P synthase like 1 sequence |

(continued)

| PEP SEQ ID NO | NUC SEQ ID NO | GENE ID | Base Vector | PPROTEIN NAME |
|---|---|---|---|---|
| 1093 | 352 | PHE0002634_2753 | 1 | rice DL-glycerol-3-phosphatase like 1 sequence |
| 1094 | 353 | PHE0002639_2758 | 1 | rice GH3 protein 8 |
| 1095 | 354 | PHE0002640_ 2759 | 1 | rice GH3 protein 3 |
| 1096 | 355 | PHE0002643_2762 | 1 | rice GH3 protein 2 |
| 1097 | 356 | PHE0002644_2763 | 1 | rice GH3 protein 9 |
| 1098 | 357 | PHE0002649_2768 | 1 | wheat AGL21-like 1 |
| 1099 | 358 | PHE0002661_2781 | 5 | Synechocystis sp. PCC 6803 ADP-glucose pyrophosphorylase |
| 1100 | 359 | PHE0002664_2787 | 6 | rice ADP-glucose pyrophosphorylase 5 |
| 1101 | 360 | PHE0002688_2821 | 1 | rice beta-3 tubulin like 1 sequence |
| 1102 | 361 | PHE0002689_2822 | 1 | rice beta-3 tubulin like 2 sequence |
| 1103 | 362 | PHE0002690_2823 | 1 | Corn protein similar to cell division related protein kinase |
| 1104 | 363 | PHE0002703_2836 | 1 | rice VTC2 like 1 sequence |
| 1105 | 364 | PHE0002710_2843 | 1 | Zea Mays cytoplasmic malate dehydrogenase |
| 1106 | 365 | PHE0002715_2848 | 1 | Oryza sativa putative thiolase |
| 1107 | 366 | PHE0002717_2850 | 1 | Corn Translation Elongation factor EF1-beta |
| 1108 | 367 | PHE0002721_2854 | 4 | Maize fructose-bisphosphate aldolase |
| 1109 | 368 | PHE0002724_2859 | 1 | Corn L19 Like ribosomal protein |
| 1110 | 369 | PHE0002728_2861 | 1 | maize sucrose transport protein SUC2 like 1 sequence |
| 1111 | 370 | PHE0002729_2863 | 1 | Corn 60S ribosomal protein L10 (probable transcription factor) |
| 1112 | 371 | PHE0002733_2866 | 1 | Corn Ribosomal protein S11 |
| 1113 | 372 | PHE0002734_2867 | 1 | Corn Ribosomal protein S12 |
| 1114 | 373 | PHE0002749_2882 | 1 | rice sucrase-like 1 sequence |
| 1115 | 374 | PHE0002751_2884 | 1 | Corn Ribosomal protein S14 |
| 1116 | 375 | PHE0002752_2885 | 1 | Corn Homolog to putative 40S Ribosomal protein |
| 1117 | 376 | PHE0002771_2904 | 1 | [Zea mays] beta-glucosidase aggregating factor precursor |
| 1118 | 377 | PHE0002790_2925 | 1 | rice CYP72A5 like 1 sequence |
| 1119 | 378 | PHE0002846_2981 | 1 | Zea Mays trehalose-6-phosphate phosphatase |
| 1120 | 379 | PHE0002864_2999 | 1 | soy CDKA 8 |
| 1121 | 380 | PHE0002869_3004 | 1 | corn CDKD 12 |

(continued)

| PEP SEQ ID NO | NUC SEQ ID NO | GENE ID | Base Vector | PPROTEIN NAME |
|---|---|---|---|---|
| 1122 | 381 | PHE0002875_3010 | 1 | Corn homolog to Arabidopsis unknown expressed protein |
| 1123 | 382 | PHE0002889_3024 | 1 | soy dsPTP 3 |
| 1124 | 383 | PHE0002896_3031 | 1 | rice dsPTP 1 |
| 1125 | 384 | PHE0002918_3053 | 1 | Oryza Sativa putative Hexose Transporter IV (distant homology to Yeast Maltose permease) |
| 1126 | 385 | PHE0002946_3096 | 1 | Zea mays Putative Polyamine Transporter |
| 1127 | 386 | PHE0002963_3113 | 1 | Zea Mays Dual Specificity Phosphatase I (similar to human YVH1) |
| 1128 | 387 | PHE0002966_3116 | 1 | Oryza Sativa Dual Specificity Phosphatase II |
| 1129 | 388 | PHE0002984_3134 | 1 | Zea mays 3-phosphoinositide-dependent protein kinase |
| 1130 | 389 | PHE0003061_3211 | 1 | [Oryza sativa] Putative integral membrane protein |
| 1131 | 390 | PHE0003074_3224 | 1 | ZeaMays RNA Binding Protein |
| 1132 | 391 | PHE0003101_3969 | 16 | soy nonsymbiotic hemoglobin |
| 1133 | 392 | PHE0003101_76 | 3 | soy nonsymbiotic hemoglobin |
| 1134 | 393 | PHE0003124_3270 | 1 | soy adenylylsulfate kinase like |
| 1135 | 394 | PHE0003138_3292 | 6 | rice fructokinase 1 |
| 1136 | 395 | PHE0003139_3295 | 1 | corn fructokinase 1 |
| 1137 | 396 | PHE0003190_3389 | 1 | soy G1225-like 2 |
| 1138 | 397 | The0003196_3395 | 1 | Arabidopsis seven-in-absentia 1 |
| 1139 | 398 | PHE0003198_3397 | 1 | corn seven-in-absentia 5 |
| 1140 | 399 | PHE0003211H_3417 | 4 | Arabidopsis AVP1 |
| 1141 | 400 | PHE0003211_3967 | 16 | Arabidopsis AVP1 |
| 1142 | 401 | PHE0003217_3423 | 1 | soy G200-like 13 |
| 1143 | 402 | PHE0003224_3432 | 1 | corn-CGPG1897-like5 |
| 1144 | 403 | PHE0003228_3444 | 16 | soy SAG13 full length |
| 1145 | 404 | PHE0003229_3445 | 16 | soy SAG13 truncated |
| 1146 | 405 | PHE0003237_3453 | 1 | rice-CGPG1264-like1 |
| 1147 | 406 | PHE0003240_3456 | 1 | soy-CGPG1857-like1 |
| 1148 | 407 | PHE0003243_3459 | 1 | corn-CGPG867-like4 |
| 1149 | 408 | PHE0003253_3470 | 1 | soy-CGPG1276-like1 |
| 1150 | 409 | PHE0003257_3474 | 1 | soy-CGPG1294-like2 |
| 1151 | 410 | PHE0003273 3492 | 1 | soy-CGPG1287-like1 |
| 1152 | 411 | PHE0003276_3493 | 1 | Arabidopsis dehydrogenase |
| 1153 | 412 | PHE0003282_3501 | 1 | corn-CGPG241-like3 [DUF6] |

(continued)

| PEP SEQ ID NO | NUC SEQ ID NO | GENE ID | Base Vector | PPROTEIN NAME |
|---|---|---|---|---|
| 1154 | 413 | PHE0003286_3505 | 1 | rice CTP synthase |
| 1155 | 414 | PHE0003287_3506 | 1 | corn CTP synthase |
| 1156 | 415 | PHE0003304_3524 | 6 | corn unknown protein |
| 1157 | 416 | PHE0003309_3528 | 1 | soy-CGPG1307-like1 |
| 1158 | 417 | PHE0003312_3531 | 1 | Arabidopsis nitrate transporter |
| 1159 | 418 | PHE0003321_3540 | 1 | Arabidopsis PDK1 like 2 |
| 1160 | 419 | PHE0003327_3546 | 1 | corn NEK like - 1 |
| 1161 | 420 | PHE0003328_3547 | 1 | corn NEK like -2 |
| 1162 | 421 | PHE0003330_3549 | 1 | arabidopsis elFiso4E like p28 subunit |
| 1163 | 422 | PHE0003333_3552 | 1 | soy eIF4E like 1 |
| 1164 | 423 | PHE0003333_4250 | 17 | soy eIF4E like 1 |
| 1165 | 424 | PHE0003336_3555 | 1 | Arabidopsis elF4E |
| 1166 | 425 | PHE0003353_3572 |  | corn seven-in-absentia 5 C57S |
| 1167 | 426 | PHE0003361_3584 | 1 | malate dehydrogenase |
| 1168 | 427 | PHE0003362_3585 | 1 | hexokinase |
| 1169 | 428 | PHE0003364_3587 | 1 | aminotransferase-like protein |
| 1170 | 429 | PHE0003369_3592 | 4 | glyceraldehyde 3-phosphate dehydrogenase |
| 1171 | 430 | PHE0003373_3596 | 1 | putative purple acid phosphatase |
| 1172 | 431 | PHE0003381_3604 | 1 | hypothetical protein2 |
| 1173 | 432 | PHE0003385_3608 | 1 | expressed protein2 |
| 1174 | 433 | PHE0003391_3614 | 1 | serine/threonine protein kinase like protein |
| 1175 | 434 | PHE0003392_3615 | 1 | lipase-like protein |
| 1176 | 435 | PHE0003393_3616 | 1 | Putative Squalene monooxygenase |
| 1177 | 436 | PHE0000132_222 | 2 | FUSS |
| 1178 | 437 | PHE0000285_375 | 1 | sorghum mLIP15 |
| 1179 | 438 | PHE0000668_771 | 1 | Arabidopsis glycerol-3-phosphate acyltransferase-D00673 |
| 1180 | 439 | PHE0000790_863 | 1 | soy agl11-like 1 delta MADS-box |
| 1181 | 440 | PHE0001074_1164 | 1 | Arabidopsis NAC domain transcription factor |
| 1182 | 441 | P14E0001078_1168 | 1 | Arabidopsis NAC domain transcription factor |
| 1183 | 442 | PHE0001079_1169 | 1 | Arabidopsis NAC domain transcription factor |
| 1184 | 443 | PHE0001082_1172 | 1 | Arabidopsis NAC domain transcription factor |
| 1185 | 444 | PHE0001085_1175 | 1 | Arabidopsis myc domain transcription factor |

(continued)

| PEP SEQ ID NO | NUC SEQ ID NO | GENE ID | Base Vector | PPROTEIN NAME |
|---|---|---|---|---|
| 1186 | 445 | PHE0001H3_1203 | 1 | Arabidopsis serine glyoxylate aminotransferase - AB048945 |
| 1187 | 446 | PHE0001136_1226 | 1 | rice aspartate aminotransferase - D14673 |
| 1188 | 447 | PHE0001142_1232 | 1 | corn caffeoyl-CoA 3-O-methyltransferase |
| 1189 | 448 | PHE0001216 1306 | 1 | maize G1836-like 1 |
| 1190 | 449 | PHE0001240_1330 | 1 | Inorganic pyrophosphatase- |
| 1191 | 450 | PHE0001243_1333 | 1 | Tubby Protein- |
| 1192 | 451 | PHE0001248_1339 | 1 | Arabidopsis hypothetical protein |
| 1193 | 452 | PHE0001257_1348 | 1 | Arabidopsis NADH:ubiquinone oxidoreductase |
| 1194 | 453 | PHE0001263_1354 | 1 | Arabidopsis hypothetical protein [NM_114619] |
| 1195 | 454 | PHE0001266_1357 | 1 | corn hypothetical protein |
| 1196 | 455 | PHE0001283_1373 | 1 | Arabidopsis Asp1 |
| 1197 | 456 | PHE0001286_1376 | 1 | Arabidopsis putative inositol hexaphosphate 1 kinase- |
| 1198 | 457 | PHE0001301_1391 | 1 | Arabidopsis Pra2-like protein - AAF97325 |
| 1199 | 458 | PHE0001304_1394 | 1 | corn Pra2-like protein 1 |
| 1200 | 459 | PHE0001316_1406 | 1 | rice CGPG 1145-like protein 1 |
| 1201 | 460 | PHE0001317_1407 | 1 | rice CGPG 1145-like protein 2 |
| 1202 | 461 | PHE0001319_1409 | 1 | Arabidopsis hypothetical protein |
| 1203 | 462 | PHE0001323_1413 | 1 | Arabidopsis hypothetical protein [NM_111447] |
| 1204 | 463 | PHE0001324_1414 | 1 | Arabidopsis expressed protein |
| 1205 | 464 | PHE0001466_1558 | 1 | Arabidopsis thaliana ROOT HAIRLESS 1 (RHL1)-AAC23500.1 |
| 1206 | 465 | PHE0001564_1663 | 4 | Xanthomonas campestris asparagine synthase |
| 1207 | 466 | PHE0002094_2204 | 1 | Corn serine /threonine kinase |
| 1208 | 467 | PHE0002121_2229 | 1 | corn G664-like 4 |
| 1209 | 468 | PHE0002225_2332 | 1 | rice drought-induced protein Di 19-like sequence |
| 1210 | 469 | PHE0002226_2333 | 1 | maize protease inhibitor like 1 sequence |
| 1211 | 470 | PHE0002293_2395 | 1 | soybean drought-inducible cysteine proteinase like 1 sequence |
| 1212 | 471 | PHE0002338_2439 | 1 | maize MIP3 like 3 sequence |
| 1213 | 472 | PHE0002524_2624 | 1 | Rice putative thioredoxin like protein |
| 1214 | 473 | PHE0002567_2666 | 1 | Arabidopsis VPE1 |

(continued)

| PEP SEQ ID NO | NUC SEQ ID NO | GENE ID | Base Vector | PPROTEIN NAME |
|---|---|---|---|---|
| 1215 | 474 | PHE0002607_2706 | 1 | Arabidopsis Sigma factor 1 |
| 1216 | 475 | PHE0002609_2708 | 1 | Arabidopsis sigma factor 3 |
| 1217 | 476 | PHE0002625_2744 | 1 | rice waxy like 1 sequence |
| 1218 | 477 | PHE0002939_3089 | 1 | Oryza Sativa Proline oxidase (proline dehydrogenase) |
| 1219 | 478 | PHE0003057_3207 | 1 | Schizosaccharomyces pombe orb6 |
| 1220 | 479 | PHE0003182_3341 | 1 | Neurospora_crassa Glycogen synthase like sequence |
| 1221 | 480 | PHE0003232_3448 | 1 | soy-CGPG 1264-like3 |
| 1222 | 481 | PHE0003234_3450 | 1 | rice-CGPG 1464-like3 |
| 1223 | 482 | PHE0003239_3455 | 1 | com-CGPG1857-like2 |
| 1224 | 483 | PHE0003246_3462 | 1 | Arabidopsis hypothetical protein |
| 1225 | 484 | PHE0003259_33476 | 1 | Arabidopsis Microtubule associated protein |
| 1226 | 485 | PHE0003267_3486 | 1 | Arabidopsis Glycine-tRNA |
| 1227 | 486 | PHE0003269_3488 | 1 | Arabidopsis unknown protein |
| 1228 | 487 | PHE0003274_3493 | 1 | Arabidopsis Unknown protein |
| 1229 | 488 | PHE0003285_3504 | 1 | rice-CGPG566-like1 |
| 1230 | 489 | PHE0003289_3508 | 8 | corn aluminium-induced protein |
| 1231 | 490 | PHE0003311_3530 | 1 | Arabidopsis hypothetical protein |
| 1232 | 491 | PHE0003360_3583 | 1 | 3-ketoacyl-ACP synthase |
| 1233 | 492 | PHE0003365_3588 | 1 | putative chlorophyll synthase |
| 1234 | 493 | PHE0003374_3597 | 1 | putative xyloglucan endotransglycosylase |
| 1235 | 494 | PHE0003375 3598 | 1 | Tryptophan synthase alpha chain |
| 1236 | 495 | PHE0003378_3601 | 1 | MtN3 |
| 1237 | 496 | PHE0003379_3602 | 1 | hypothetical protein 1 |
| 1238 | 497 | PHE0003383_3606 | 1 | expressed protein 1 |
| 1239 | 498 | PHE0003387_3610 | 4 | nodulin-like protein |
| 1240 | 499 | PHE0003401_3624 | 1 | corn sterol 5 alpha desaturase |
| 1241 | 500 | PHE0003417_3657 | 19 | soy G1634-like 1 |
| 1242 | 501 | PHE0003418_3658 | 19 | Arabidopsis isocitrate lyase |
| 1243 | 502 | PHE0003433_3642 | 1 | corn dehalogenase-phosphatase 1 |
| 1244 | 503 | PHE0003450_3681 | 1 | soy G1274 like 1 |
| 1245 | 504 | PHE0003458_3689 | 1 | corn G1660 like 1 |
| 1246 | 505 | PHE0003459_3690 | 1 | corn G1730 like 1 |
| 1247 | 506 | PHE0003476_3707 | 1 | soy G1988 like 1 |
| 1248 | 507 | PHE0003484_3730 | 1 | corn G2035 like 1 |
| 1249 | 508 | PHE0003491_3737 | 1 | soy G2063 like2 |

(continued)

| PEP SEQ ID NO | NUC SEQ ID NO | GENE ID | Base Vector | PPROTEIN NAME |
|---|---|---|---|---|
| 1250 | 509 | PHE0003499_3745 | 1 | rice G2207 like2 |
| 1251 | 510 | PHE0003518_3764 | 1 | soy G2505 like1 |
| 1252 | 511 | PHE0003524_3770 | 1 | soy G2536 like1 |
| 1253 | 512 | PHE0003570_3816 | 1 | soy G922 like1 |
| 1254 | 513 | PHE0003576_3822 | 1 | soy G975 like1 |
| 1255 | 514 | PHE0003583_3829 | 1 | corn G728 like1 |
| 1256 | 515 | PHE0003587_3833 | 1 | soy G3083 like1 |
| 1257 | 516 | PHE0003599_3845 | 1 | corn G2981 like1 |
| 1258 | 517 | PHE0003600 3846 | 4 | corn R-S |
| 1259 | 518 | PHE0003630_3888 | 1 | corn G1206 like 2 |
| 1260 | 519 | PHE0003641_3899 | 4 | corn G2998 like2 |
| 1261 | 520 | PHE0003644 3902 | 1 | corn G303 like2 |
| 1262 | 521 | PHE0003650_3908 | 1 | soy G355 like2 |
| 1263 | 522 | PHE0003678_3936 | 1 | com G1052 like2 |
| 1264 | 523 | PHE0003686_3961 | 16 | soy NDPK2 |
| 1265 | 524 | PHE0003740_4042 | 16 | soy G481-like 3 |
| 1266 | 525 | PHE0003742_4044 | 4 | corn UDP-glucose 4-epimerase CGPG1003 like2 |
| 1267 | 526 | PHE0003743_4046 | 4 | rice UDP-glucose 4-epimerase CGPG 1003 like1 |
| 1268 | 527 | PHE0003825_4192 | 4 | soy G1412 like1 |
| 1269 | 528 | PHE0003839 4208 | 4 | rice G2604 like1 |
| 1270 | 529 | PHE0003885_4266 | 4 | wheat hemoglobin |
| 1271 | 530 | PHE0003886_4267 | 4 | Arabidopsis hemoglobin-like 1 |
| 1272 | 531 | PHE0003927_4321 | 4 | Zea mays AIH-like |
| 1273 | 532 | PHE0003959_4544 | 4 | Glycine max CGPG7857 pseudo-response regulator |
| 1274 | 533 | PHE0003961_4662 | 14 | maize Kas I |
| 1275 | 534 | PHE0003965_4553 | 4 | corn ABI5 like |
| 1276 | 535 | PHE0003966_4534 | 4 | Corn AtUPS1-like |
| 1277 | 536 | PHE0003977_4565 | 4 | corn AfMONFEED001213 uroporphyrinogen III synthase |
| 1278 | 537 | PHE0003978_4566 | 4 | corn AfMONFEED000902 proteophosphoglycan |
| 1279 | 538 | PHE0003982_4569 | 4 | corn AfMONFEED000317 serine carboxypeptidase II-1 |
| 1280 | 539 | PHE0003993_4579 | 4 | corn AfMONFEED001219 putative Ca2+/H+-exchanging protein |
| 1281 | 540 | PHE0003994_4580 | 4 | corn AfMONFEED001169 putative pathogenesis related protein |

(continued)

| PEP SEQ ID NO | NUC SEQ ID NO | GENE ID | Base Vector | PPROTEIN NAME |
|---|---|---|---|---|
| 1282 | 541 | PHE0003996_4582 | 4 | corn AfMONFEED000906 auxin-induced protein-related |
| 1283 | 542 | PHE0003997_4583 | 4 | corn AfMONFEED001366 isoflavone reductase homolog |
| 1284 | 543 | PHE0003999_4585 | 4 | corn AfMONFEED000689 proline-rich protein |
| 1285 | 544 | PHE0004000_4586 | 4 | corn AfMONFEED000920 nodulin-related protein |
| 1286 | 545 | PHE0004002_4588 | 4 | corn AfMONFEED001475 hypothetical protein |
| 1287 | 546 | PHE0004003_4589 | 4 | corn AfMONFEED000648 acyltransferase |
| 1288 | 547 | PHE0004004_4590 | 4 | corn AtMONFEED000503 pathogenesis-related protein 4 |
| 1289 | 548 | PHE0004005_4591 | 4 | corn AfMONFEED001364 unknown protein |
| 1290 | 549 | PHE0004006_4592 | 4 | corn AfMONFEED000623 hydrolase |
| 1291 | 550 | PHE0004009_4595 | 4 | corn AfMONFEED000074 unknown protein |
| 1292 | 551 | PHE0004010_4596 | 4 | corn AfMONFEED000152 Myb-like DNA-binding domain |
| 1293 | 552 | PHE0004011_4597 | 4 | corn AfMONFEED001001 ACT domain-containing protein |
| 1294 | 553 | PHE0000788_861 | 4 | corn agl11-like 1 delta C-terminus |
| 1295 | 554 | PHE0000789_862 | 4 | corn agl11-like 1 delta K-box |
| 1296 | 555 | PHE0000795_868 | 1 | rice MADS3 delta K-box-L37528 |
| 1297 | 556 | PHE0000881_964 | 1 | Synechocystis sp. PCC 6803 Hik33 |
| 1298 | 557 | PHE0001226_1316 | 1 | wheat catalase-like 1 |
| 1299 | 558 | PHE0001253_1344 | 1 | soy arabinoglactan-protein 1 |
| 1300 | 559 | PHE0001335_1426 | 1 | rice SNF1-like protein 8 [OsPK7]-BAA83689 |
| 1301 | 560 | PHE0001381_1473 | 1 | rice ANR1-like |
| 1302 | 561 | PHE0001521_1612 | 1 | soy LPEI-like permease 2 |
| 1303 | 562 | PHE0001630_1741 | 1 | maize low temperature and salt responsive protein LTI6A-like 2 sequence |
| 1304 | 563 | PHE0001659_1770 | 1 | corn SDD1-like 1 pre-pro domain |
| 1305 | 564 | PHE0002079_2189 | 1 | rice sec61 |
| 1306 | 565 | PHE0002185_2292 | 1 | rice Hsp17.7 like 1 sequence |
| 1307 | 566 | PHE0002229_2336 | 1 | rice protease inhibitor like 1 sequence |

(continued)

| PEP SEQ ID NO | NUC SEQ ID NO | GENE ID | Base Vector | PPROTEIN NAME |
|---|---|---|---|---|
| 1308 | 567 | PHE0002392_2493 | 1 | rice copper chaperone like 1 sequence |
| 1309 | 568 | PHE0002410_2510 | 1 | E. coli gadA |
| 1310 | 569 | PHE0002411_2511 | 1 | Synechocystis sp. PCC 6803 gad |
| 1311 | 570 | PHE0002485_2585 | 4 | Corn Amino Acid Transport Protein |
| 1312 | 571 | PHE0002496_2596 | 1 | Corn Glutamate-1-semialdehyde aminotransferase |
| 1313 | 572 | PHE0002611_2710 | 1 | soy sigma factor 1 |
| 1314 | 573 | PHE0002616_2723 | 1 | Arabidopsis proline/glycine betaine transporter 1 |
| 1315 | 574 | PHE0002670_2793 | 5 | Streptomyces coelicolor glucose-1-phosphate adenylyltransferase |
| 1316 | 575 | The0002727_2860 | 1 | rice sucrose synthase-1 like 2 sequence |
| 1317 | 576 | PHE0002950_3100 | 1 | Yeast protein involved in resistance to H2O2 |
| 1318 | 577 | PHE0002997_3147 | 1 | Yeast Protein of unknown function, has WD . (WD-40) repeats |
| 1319 | 578 | PHE0003011_3161 | 1 | Yeast Protein (wi Carbonic anhydrase PFAM domain) involved in protection against oxidative damage |
| 1320 | 579 | PHE0003021_3171 | 1 | Yeast protein of unknown function (may be involved in cell damage) |
| 1321 | 580 | PHE0003200_3399 | 1 | Arabidopsis seven-in-absentia 2 |
| 1322 | 581 | PHE0003227_3443 | 16 | soy G1820 like |
| 1323 | 582 | PHE0003261_3478 | 1 | rice-CGPG 1517-like2 |
| 1324 | 583 | PHE0003270_3489 | 1 | soy-CGPG 1191-like1 |
| 1325 | 584 | PHE0003277_3496 | 1 | soy-CGPG1430-like1 |
| 1326 | 585 | PHE0003352_3571 | 1 | Corn ethylene receptor 1 |
| 1327 | 586 | PHE0003366_3589 | 1 | transaldolase |
| 1328 | 587 | PHE0003370_3593 | 4 | Circulin B |
| 1329 | 588 | PHE0003526_3772 | 1 | soy G2567 like2 |
| 1330 | 589 | PHE0003581_3827 | 1 | rice G2603 like2 |
| 1331 | 590 | PHE0003681_3942 | 4 | rice G1792-like 3 |
| 1332 | 591 | PHE0003683_3944 | 16 | soy SUC1-like 1 |
| 1333 | 592 | PHE0003683_3945 | 18 | soy SUC1-like 1 |
| 1334 | 593 | PHE0003815_4288 | 4 | Synechocystis NblS-like |
| 1335 | 594 | PHE0003986_4573 | 4 | corn AfMONFEED001223 putative stearoyl-acyl-carrier protein desaturase |

(continued)

| PEP SEQ ID NO | NUC SEQ ID NO | GENE ID | Base Vector | PPROTEIN NAME |
|---|---|---|---|---|
| 1336 | 595 | PHE0003989_4575 | 4 | corn AfMONPEED000878 remorin-like protein |
| 1337 | 596 | PHE0003990_4576 | 4 | corn AfMONFEED001231 unknown protein |
| 1338 | 597 | PHE0003995_4581 | 4 | corn AfMONFEED001033 putative succinate dehydrogenase, |
| 1339 | 598 | PHE0004022_4657 | 4 | Arabidopsis Alfin-like |
| 1340 | 599 | PHE0000667_770 | 1 | chimeric glycerol-3-phosphate acyltransferase |
| 1341 | 600 | PHE0000784_857 | 1 | Arabidopsis agl11 delta MADS-box |
| 1342 | 601 | PHE0000880_963 | 1 | Nostoc sp. PCC7120 Hik33 |
| 1343 | 602 | PHE0000883_966 | 1 | Synechocystis sp. 6803 Rer1 |
| 1344 | 603 | PHE0001063_1153 | 1 | yeast SNF4-Z72637 |
| 1345 | 604 | PHE0001065_1155 | 1 | yeast SIP2-Z72730 |
| 1346 | 605 | PHE0001069_1159 | 1 | Arabidopsis bZIP domain transcription factor |
| 1347 | 606 | PHE0001076_1166 | 1 | Arabidopsis NAC domain transcription factor |
| 1348 | 607 | PHE0001080_1170 | 1 | Arabidopsis AP2 domain transcription factor |
| 1349 | 608 | PHE0001087_1177 | 1 | Arabidopsis homeodomain transcription factor |
| 1350 | 609 | PHE0001094_1184 | 1 | Arabidopsis zinc finger protein |
| 1351 | 610 | PHE0001100_1190 | 1 | Arabidopsis BZIP domain transcription factor |
| 1352 | 611 | PHE0001112_1202 | 1 | yeast alanine aminotransferase 2 - CAA88665 |
| 1353 | 612 | PHE0001131_1221 | 5 | yeast acetohydroxyacid reductoisomerase-AAB67753 |
| 1354 | 613 | PHE0001143_1233 | 1 | corn putative isoprenylated protein |
| 1355 | 614 | PHE0001149_1239 | 1 | corn HvB 12D homolog |
| 1356 | 615 | PHE0001169_1259 | 1 | Arabidopsis TFL2 - AF387639 |
| 1357 | 616 | PHE0001176_1266 | 5 | Synechocystis sp. PCC 6803 heme oxygenase-1651897 |
| 1358 | 617 | PHE0001177_1267 | 5 | Nostoc sp. PCC 7120 heme oxygenase-17132210 |
| 1359 | 618 | PHE0001184_1274 | 1 | rice SHY2-like 1 - BAA92982 |
| 1360 | 619 | PHE0001256_1347 | 1 | Arabidopsis protease HhoA precursor |
| 1361 | 620 | PHE0001264_1355 | 1 | Arabidopsis hypothetical protein |
| 1362 | 621 | PHE0001265_1356 | 1 | Arabidopsis hypothetical protein [NM_105159] |

(continued)

| PEP SEQ ID NO | NUC SEQ ID NO | GENE ID | Base Vector | PPROTEIN NAME |
|---|---|---|---|---|
| 1363 | 622 | PHE0001268_1359 | 1 | Arabidopsis hypothetical protein |
| 1364 | 623 | PHE0001270_1361 | 1 | yeast ero1 - CAA90553 |
| 1365 | 624 | PHE0001311_1401 | 1 | Arabidopsis expressed protein [NM_126228] |
| 1366 | 625 | PHE0001322_1412 | 1 | Arabidopsis expressed protein |
| 1367 | 626 | PHE0001519_1610 | 1 | rice WD domain protein |
| 1368 | 627 | PHE0001523_1614 | 1 | rice LPE1-like permase 1 - BAB61205 |
| 1369 | 628 | PHE0001527_1618 | 1 | Nostoc sp. PCC 7120 GDH |
| 1370 | 629 | PHE0001528_1619 | 1 | Nostoc punctiforme GDH |
| 1371 | 630 | PHE0001533_1624 | 1 | Streptomyces coelicolor GDH |
| 1372 | 631 | PHE0001541_1632 | 1 | rice glutamate dehydrogenase - 15787849 |
| 1373 | 632 | PHE0001567_1666 | 1 | Bacillus halodurans asparagine synthase -10174030 |
| 1374 | 633 | PHE0001568_1667 | 4 | Corynebacterium glutamicum Asparagine synthase-19551250 |
| 1375 | 634 | PHE0001611_1722 | 1 | rice translation initiation factor 3 delta subunit like sequence |
| 1376 | 635 | PHE0001632_1743 | 1 | arabidopsis magnesium/proton exchanger AtMHX |
| 1377 | 636 | PHE0002029_2140 | 1 | Corn Axi 1 |
| 1378 | 637 | PHE0002078_2188 | 1 | yeast sec61 |
| 1379 | 638 | PHE0002084_2194 | 1 | Corn cyclic nucleotide and calmodulin-regulated ion channel |
| 1380 | 639 | PHE0002089_2199 | 1 | Agrobacterium tumefaciens hypothetical protein |
| 1381 | 640 | PHE0002090_2200 | 1 | Nostoc sp. PCC 7120 stress induced hydrophobic peptide |
| 1382 | 641 | PHE0002151_2239 | 1 | Corn Kaurene Synthase |
| 1383 | 642 | PHE0002194_2301 | 1 | maize Betaine-aldehyde dehydrogenase like 1 sequence |
| 1384 | 643 | PHE0002483_2583 | 1 | maize heat shock TF like sequence |
| 1385 | 644 | PHE0002494_2594 | 1 | Arabidopsis ascorbate oxidase |
| 1386 | 645 | PHE0002509_2609 | 1 | cORN Hsp20/alpha crystallin family" |
| 1387 | 646 | PHE0002523_2623 | 1 | Corn putative thioredoxin protein |
| 1388 | 647 | PHE0002527_2627 | 1 | Hypothetical Nostoc protein similar to cobW |
| 1389 | 648 | PHE0002546_2646 | 1 | Corn dual-specificity protein phosphatase-like protein |
| 1390 | 649 | PHE0002557_2657 | 1 | soy G1792-like 10 |
| 1391 | 650 | PHE0002566_2665 | 1 | Ribosomal protein L39 |

(continued)

| PEP SEQ ID NO | NUC SEQ ID NO | GENE ID | Base Vector | PPROTEIN NAME |
|---|---|---|---|---|
| 1392 | 651 | PHE0002614_2713 | 1 | Brassica napus sigA binding protein 2 |
| 1393 | 652 | PHE0002648_2767 | 1 | soy AGL21-like 2 |
| 1394 | 653 | PHE0002652_2771 | 1 | Corn GDP-mannose pyrophosphorylase A |
| 1395 | 654 | PHE0002791_2926 | 1 | rice CYP72A5 like 2 sequence |
| 1396 | 655 | PHE0002849_2984 | 1 | Oryza Sativa Putative Glucosyl Transferase |
| 1397 | 656 | PHE0002855_2990 | 1 | sp unknown protein wi/ ABC1 PFAM domain (putative novel chaperonin) |
| 1398 | 657 | PHE0002856_2991 | 1 | Nostoc sp unknown protein wi/ ABC1 PRAM domain(putative novel chaperonin) |
| 1399 | 658 | PHE0002886_3021 | 1 | Brassica dsPTP 1 |
| 1400 | 659 | PHE0002888_3023 | 1 | soy dsPTP 2 |
| 1401 | 660 | PHE0002893_3028 | 1 | Corn dsPTP 3 |
| 1402 | 661 | PHE0002901_3036 | 1 | Yeast GAT2 GATA Zinc Finger TF |
| 1403 | 662 | PHE0002906 3041 | 1 | Zea Mays Zinc Finger Transcription Factor IV |
| 1404 | 663 | PHE0002910_3045 | 1 | Yeast IKS1 |
| 1405 | 664 | PHE0002923_3058 | 1 | Yeast GGA1 protein (mediator of protein traficking between Trans golgi network and vacoule) |
| 1406 | 665 | PHE0002928_3065 | 1 | Yeast Zinc Finger protein (DNA damage responsive repressor of PHR1) |
| 1407 | 666 | PHE0002941_3091 | 1 | Yeast SET6p putative Transcription Factor |
| 1408 | 667 | PHE0002947_3097 | 1 | Zea Mays Sugar & other (polyamine) transporter like protein |
| 1409 | 668 | PHE0002948_3098 | 1 | Yeast Xylulokinase |
| 1410 | 669 | PHE0002987_3137 | 1 | Yeast oxidoreductase of unknown function (PFAM NAD-binding Rossmann fold & C-terminal alpha/beta domain) |
| 1411 | 670 | PHE00030173167 | 1 | Yeast Transketolase 2 |
| 1412 | 671 | PHE0003018_3168 | 1 | Yeast Ubiquitin polyprotein |
| 1413 | 672 | PHE0003023_3173 | 1 | Yeast unknown protein (uncharacterized protein family) |
| 1414 | 673 | PHE0003025_3175 | 1 | Yeast Protein of unknown function, (similar to mouse MPV 17 a putative integral membrane peroxisomal protein) |

(continued)

| PEP SEQ ID NO | NUC SEQ ID NO | GENE ID | Base Vector | PPROTEIN NAME |
|---|---|---|---|---|
| 1415 | 674 | PHE0003026_3176 | 1 | Yeast Protein of unknown function, putative paralog of Ecm4p, a cell wall biogenesis protein |
| 1416 | 675 | PHE0003027_3177 | 1 | Yeast Protein of unknown function (Pfam Domain YjeF-related protein N-terminus) |
| 1417 | 676 | PHE0003028_3178 | 1 | Yeast Potential alpha-ketoisocaproate reductase / |
| 1418 | 677 | PHE0003030_3180 | 1 | Yeast Yapsin 6, GPI-anchored aspartyl protease |
| 1419 | 678 | PHE0003062_3212 | 1 | soy G571 |
| 1420 | 679 | PHE0003088_3240 | 1 | Arabidopsis AtPK1 |
| 1421 | 680 | PHE0003089_3237 | 1 | Arabidopsis AtPK19b |
| 1422 | 681 | PHE0003090_3238 | 1 | soy S6K1 |
| 1423 | 682 | PHE0003091_3239 | 1 | corn S6K1 |
| 1424 | 683 | PHE0003102_3244 | 6 | Clostridium acetobutylicum fructokinase |
| 1425 | 684 | PHE0003102_3579 | 16 | Clostridium acetobutylicum fructokinase |
| 1426 | 685 | PHE0003121_3267 | 1 | Arabidopsis cyt P450 like |
| 1427 | 686 | PHE0003130_3276 | 1 | Arabidopsis glucosyltransferase-like |
| 1428 | 687 | PHE0003134_3280 | 1 | Arabidopsis microtubule-associated protein EB1-like protein like |
| 1429 | 688 | PHE0003194_3393 | 1 | corn seven-in-absentia 2 |
| 1430 | 689 | PHE0003195_3394 | 1 | soy seven-in-absentia 1 |
| 1431 | 690 | PHE0003199_3398 | 1 | rice seven-in-absentia 2 |
| 1432 | 691 | PHE0003201_3400 | 1 | soy seven-in-absentia 4 |
| 1433 | 692 | PHE0003208_3410 | 7 | soy triose phosphate translocator |
| 1434 | 693 | PHE0003213_3419 | 1 | yeast TAT2 |
| 1435 | 694 | PHE0003214_3420 | 1 | yeast CUP1a |
| 1436 | 695 | PHE0003222_3428 | 1 | Corynebacterium glutamicum glutamate 5-kinase |
| 1437 | 696 | PHE0003238_3454 | 1 | Arabidopsis fructose-bisphosphate aldolase |
| 1438 | 697 | PHE0003242_3458 | 1 | Arabidopsis ABC transporter |
| 1439 | 698 | PHE0003251_3468 | I | Arabidopsis unknown protein |
| 1440 | 699 | PHE0003260_3477 | 1 | soy-CGPG1517-like1 |
| 1441 | 700 | PHE0003272_3491 | 1 | Arabidopsis hypothetical protein |
| 1442 | 701 | PHE0003275_3494 | 1 | Brassica-CGPG1391-like1 |
| 1443 | 702 | PHE0003302_3523 | 1 | Arabidopsis hypothetical protein |

(continued)

| PEP SEQ ID NO | NUC SEQ ID NO | GENE ID | Base Vector | PPROTEIN NAME |
|---|---|---|---|---|
| 1444 | 703 | PHE0003308_3527 | 1 | Arabidopsis Yippee putative zinc-binding protein |
| 1445 | 704 | PHE0003315_3534 | 1 | Arabidopsis unknown protein |
| 1446 | 705 | PHE0003320_3539 | 1 | Arabidopsis PDK1 like 1 |
| 1447 | 706 | PHE0003344_3562 | 1 | corn ADC like -1 |
| 1448 | 707 | PHE0003347_3565 | 1 | Arabidopsis GCN5 |
| 1449 | 708 | PHE0003354_3573 | 1 | corn G571 long splice form A196P, A197P |
| 1450 | 709 | PHE0003380_3603 | 1 | putative laccase |
| 1451 | 710 | PHE0003388_3611 | 4 | Caffeic acid 3-0-methyltransferase |
| 1452 | 711 | PHE0003395_3618 | 4 | hypothetical protein4 |
| 1453 | 712 | PHE0003397_3620 | 1 | hypothetical protein5 |
| 1454 | 713 | PHE0003403_3626 | 16 | potato twin LOV protein |
| 1455 | 714 | PHE0003411_3634 | 1 | Putative NAM protein |
| 1456 | 715 | PHE0003414_3654 | 19 | Streptococcus mutans gtfA (dicot codon modified) |
| 1457 | 716 | PHE0003415_3655 | 18 | Synechococcus sp. PCC 7942 IctB with / RuBisCO small subunit Ib CTP |
| 1458 | 717 | PHE0003431_3639 | 4 | Streptomyces coelicolor trehalose synthase 1 |
| 1459 | 718 | PHE0003434_3643 | 1 | corn dehalogenase-phosphatase 2 |
| 1460 | 719 | PHE0003436_3645 | 1 | Nostoc sp. PCC 7120 dehalogenase-phosphatase |
| 1461 | 720 | PHE0003437_3646 | 1 | yeast dehalogenase-phosphatase |
| 1462 | 721 | PHE0003447_3678 | 1 | soy DUF296 |
| 1463 | 722 | PHE0003483_3729 | 1 | soy G2035 like 1 |
| 1464 | 723 | PHE0003519_3765 | 1 | corn G2505 like2 |
| 1465 | 724 | PHE0003582_3828 | 1 | soy G2999 like 1 |
| 1466 | 725 | PHE0003586_3832 | 1 | Brassica G2763 like1 |
| 1467 | 726 | PHE0003588_3834 | 1 | soy G2776 like1 |
| 1468 | 727 | PHE0003598_3844 | 1 | soy G2981 like1 |
| 1469 | 728 | PHE0003606_3852 | 1 | soy G2998 like1 |
| 1470 | 729 | PHE0003613_3860 | 1 | corn GAD1-1 |
| 1471 | 730 | PHE0003617_3866 | 1 | Brassica G2839 like1 |
| 1472 | 731 | PHE0003632_3890 | 1 | rice G2982 like 1 |
| 1473 | 732 | PHE0003636_3894 | 1 | soy G2992 like1 |
| 1474 | 733 | PHE0003676_3934 | 1 | soy G1052 like2 |
| 1475 | 734 | PHE0003682_3943 | 4 | wheat wpk4 |
| 1476 | 735 | PHE0003788_4122 | 4 | corn NACI-like 1 |

(continued)

| PEP SEQ ID NO | NUC SEQ ID NO | GENE ID | Base Vector | PPROTEIN NAME |
|---|---|---|---|---|
| 1477 | 736 | PHE0003899_4284 | 17 | CGPG2101 Arabidopsis PHD-finger protein |
| 1478 | 737 | PHE0003948_4528 | 17 | Arabidopsis CGPG3676 |
| 1479 | 738 | PHE0003981_4568 | 4 | corn AfMONFEED000388 pyruvate kinase |
| 1480 | 739 | PHE0003984_4571 | 4 | corn AFMONFEED000668 unknown protein |
| 1481 | 740 | PHE0003998_4584 | 4 | corn AfMONEEED000499 putative indole-3-acetic acid-regulated protein |
| 1482 | 741 | PHE0004008_4594 | 4 | corn AfMONFEED000474 serine protease-like protein |

**Selection methods for transgenic plants with enhanced agronomic trait**

Within a population of transgenic plants regenerated from plant cells transformed with the recombinant DNA many plants that survive to fertile transgenic plants that produce seeds and progeny plants will not exhibit an enhanced agronomic trait. Selection from the population is necessary to identify one or more transgenic plant cells that can provide plants with the enhanced trait. Transgenic plants having enhanced traits are selected from populations of plants regenerated or derived from plant cells transformed as described herein by evaluating the plants in a variety of assays to detect an enhanced trait, e.g. enhanced water use efficiency, enhanced cold tolerance, increased yield, enhanced nitrogen use efficiency, enhanced seed protein and enhanced seed oil. These assays also may take many forms including, but not limited to, direct screening for the trait in a greenhouse or field trial or by screening for a surrogate trait. Such analyses can be directed to detecting changes in the chemical composition, biomass, physiological properties, morphology of the plant. Changes in chemical compositions such as nutritional composition of grain can be detected by analysis of the seed composition and content of protein, free amino acids, oil, free fatty acids, starch or tocopherols. Changes in biomass characteristics can be made on greenhouse or field grown plants and can include plant height, stem diameter, root and shoot dry weights; and, for corn plants, ear length and diameter. Changes in physiological properties can be identified by evaluating responses to stress conditions, for example assays using imposed stress conditions such as water deficit, nitrogen deficiency, cold growing conditions, pathogen or insect attack or light deficiency, or increased plant density. Changes in morphology can be measured by visual observation of tendency of a transformed plant with an enhanced agronomic trait to also appear to be a normal plant as compared to changes toward bushy, taller, thicker, narrower leaves, striped leaves, knotted trait, chlorosis, albino, anthocyanin production, or altered tassels, ears or roots. Other selection properties include days to pollen shed, days to silking, leaf extension rate, chlorophyll content, leaf temperature, stand, seedling vigor, internode length, plant height, leaf number, leaf area, tillering, brace roots, stay green, stalk lodging, root lodging, plant health, barreness/prolificacy, green snap, and pest resistance. In addition, phenotypic characteristics of harvested grain may be evaluated, including number of kernels per row on the ear, number of rows of kernels on the ear, kernel abortion, kernel weight, kernel size, kernel density and physical grain quality. Although the plant cells and methods of this invention can be applied to any plant cell, plant, seed or pollen, e.g. any fruit, vegetable, grass, tree or ornamental plant, the various aspects of the invention are preferably applied to corn, soybean, cotton, canola, alfalfa, wheat and rice plants. In many cases the invention is applied to corn plants that are inherently resistant to disease from the Mal de Rio Cuarto virus or the *Puccina sorghi,* fungus or both.

The following examples are included to demonstrate aspects of the invention, those of skill in the art should, in light of the present disclosure, appreciate that many changes can be made in the specific aspects which are disclosed and still obtain a like or similar results without departing from the spirit and scope of the invention.

**Example 1. Plant Expression Constructs**

A. Plant expression constructs for corn transformation

This example illustrates the construction of plasmids for transferring recombinant DNA into plant cells which can be regenerated into transgenic plants of this invention.

A base plant transformation vector pMON65154, as set forth in SEQ ID NO: 52768 was fabricated for use in preparing recombinant DNA for transformation into corn tissue using GATEWAY™ Destination plant expression vector systems (available from Invitrogen Life Technologies, Carlsbad, CA). With reference to the elements described in Table 3 below, pMON65154 comprises a selectable marker expression cassette and a template recombinant DNA expression cassette. The marker expression cassette comprises a CaMV 35S promoter operably linked to a gene encoding neomycin phosphotransferase II (*npt*II) followed by a 3' region of an *Agrobacterium tumefaciens* nopaline synthase gene (*nos*). The template recombinant DNA expression cassette is positioned tail to tail with the marker expression cassette. The template recombinant DNA expression cassette comprises 5' regulatory DNA including a rice actin 1 promoter, exon and intron, followed by a GATEWAY™ insertion site for recombinant DNA, followed by a 3' region of a potato proteinase inhibitor If (*pin*II) gene. Once recombinant DNA has been inserted into the insertion site, the plasmid is useful for plant transformation, e.g. by microprojectile bombardment.

Table 3

| FUNCTION | ELEMENT | REFERENCE |
|---|---|---|
| Plant gene of interest expression cassette | Rice actin 1 promoter | U.S. Patent 5,641,876 |
| | Rice actin 1 exon 1, intron 1 enhancer | U.S. Patent 5,641,876 |
| Gene of interest insertion site | *Att*R1 | GATEWAY™ Cloning Technology Instruction Manual |
| | CmR gene | GATEWAY™ Cloning Technology Instruction Manual |
| | *ccd*A, *ccd*B genes | GATEWAY™ Cloning Technology Instruction Manual |
| | *att*R2 | GATEWAY™ Cloning Technology Instruction Manual |
| Plant gene of interest expression cassette | Potato pinII 3' region | An et al. (1989) Plant Cell 1: 115-122 |
| Plant selectable marker expression cassette | CaMV 35S promoter | U.S. Patent 5,858,742 |
| | nptII selectable marker | U.S. Patent 5,858,742 |
| | nos 3' region | U.S. Patent 5,858,742 |
| Maintenance in *E. coli* | *ColE1* origin of replication | |
| | F1 origin of replication | |
| | *Bla* ampicillin resistance | |

A similar base vector plasmid pMON72472 (SEQ ID NO: 52769) was constructed for use in *Agrobacterium*-mediated methods of plant transformation similar to pMON65154 except (a) the 5' regulatory DNA in the template recombinant DNA expression cassette was a rice actin promoter and a rice actin intron, (b) left and right T-DNA border sequences from *Agrobacterium* are added with the right border sequence is located 5' to the rice actin 1 promoter and the left border sequence is located 3' to the 35S promoter and (c) DNA is added to facilitate replication of the plasmid in both *E. coli* and *Agrobacterium tumefaciens*. The DNA added to the plasmid outside of the T-DNA border sequences includes an *orV* wide host range origin of DNA replication functional in *Agrobacterium,* a pBR322 origin of replication functional in *E.coli,* and a spectinomycin/streptomycin resistance gene for selection in both *E. coli* and *Agrobacterium.*
Other base vectors similar to those described above were also constructed as listed in Table 4. See Table 4 for a summary of base vector plasmids and base vector ID's which are referenced in Table 1. Also see Table 5 for a summary of regulatory elements used in the gene expression cassette for these base vectors and SEQ ID NOs for elements.

Table 4

| Base Vector ID | Base Vector for Corn |
|---|---|
| 1 | pMON72472 |

(continued)

| Base Vector ID | Base Vector for Corn |
|---|---|
| 2 | pMON65154 |
| 3 | pMON84109 |
| 4 | pMON82060 |
| 5 | pMON74430 |
| 6 | pMON84107 |
| 7 | pMON81244 |
| 8 | pMON76274 |
| 9 | pMON74575 |
| 10 | pMON92667 |
| 11 | pMON84108 |
| 12 | pMON74582 |
| 13 | pMON74579 |
| 14 | pMON74577 |
| Base Vector ID | Base Vector for Soybean |
| 15 | pMON74552 |
| 16 | pMON74532 |
| 17 | pMON 82053 |
| 18 | pMON74537 |
| 19 | pMON74536 |
| 20 | pMON74548 |

Table 5

| Vector | promoter | SEQ ID NO | leader | SEQ ID NO | intron | SEQ ID NO | transit peptide | SEQ ID NO | terminator | SEQ ID NO |
|---|---|---|---|---|---|---|---|---|---|---|
| pMON65154 | P-Os.Act1-1:4:27 | 52789 | L-Os.Act1-1:1:3 | 52777 | I-Os.Act1-1:1:40 | 52772 | NONE | / | T-St.Pis4-1:4:3 | 5280 |
| pMON72472 | P-Os.Act1-1:1:8 | 52788 | L-Os.Act1-1:1:5 | 52778 | I-Os.Act1-1:1:3 | 52771 | NONE | / | T-St.Pis4-1:4:1 | 5280 |
| pMON74577 | P-Hv.Per1-1:1:7 | 52787 | L-Hv.Per1-1:1:1 | 52776 | I-Zm.DnaK-1:1:1 | 52773 | NONE | / | T-St.Pis4-1:4:1 | 5280 |
| pMON74579 | P-Zm.NAS2-1:1:1 | 52793 | L-Zm.NAS2-1:1:1 | 52781 | I-Zm.DnaK-1:1:1 | 52773 | NONE | / | T-St.Pis4-1:4:1 | 5280 |
| pMON74582 | P-Zm.PPDK-1:1:12 | 52795 | L-Zm.PPDK-1:1:2 | 52782 | I-Zm.DnaK-1:1:1 | 52773 | NONE | / | T-St.Pis4-1:4:1 | 5280 |
| pMON76274 | P-Os.GT1-1:1:10 | 52790 | L-Os.GT1-1:1:3 | 52779 | I-Zm.DnaK-1:1:1 | 52773 | NONE | / | T-St.Pis4-1:4:1 | 5280 |
| pMON81244 | P-Zm.PPDK-1:1:10 | 52794 | L-Zm.PPDK-1:1:2 | 52782 | I-Zm.DnaK-1:1:1 | 52773 | NONE | / | T-St.Pis4-1:4:3 | 5280 |
| pMON82060 | P-Os.Act1-1:1:8 | 52788 | L-Os.Act1-1:1:5 | 52778 | I-Os.Act1-1:1:3 | 52771 | NONE | / | T-St.Pis4-1:4:1 | 5280 |
| pMON84109 | P-Os.Act1-1:1:8 | 52788 | L-Os.Act1-1:1:5 | 52778 | I-Os.Act1-1:1:3 | 52771 | TS-At.ShkG-CTP2-1:1:1 | 52799 | T-St.Pis4-1:4:1 | 5280 |
| pMON74430 | P-Os.Act1-1:1:8 | 52788 | L-Os.Act1-1:1:5 | 52778 | I-Os.Act1-1:1:3 | 52771 | TS-At.ShkG-CTP2-1:1:1 | 52799 | T-St.Pis4-1:4:1 | 5280 |
| pMON84107 | P-Os.GT1-1:1:18 | 52791 | NONE | | I-Zm.DnaK-1:1:1 | 52773 | NONE | / | T-St.Pis4-1:4:1 | 5280 |
| pMON74575 | P-Zm.FDA-1:1:5 | 52792 | L-Zm.FDA-1:1:1 | 52780 | I-Zm.DnaK-1:1:1 | 52773 | NONE | / | T-St.Pis4-1:4:1 | 5280 |
| pMON92667 | P-Zm.SzeinC1-1:1:1 | 52797 | L-Zm.SceinC1-1:1:1 | 52783 | I-Zm.DnaK-1:1:1 | 52773 | NONE | / | T-St.Pis4-1:4:1 | 5280 |
| pMON74532 | P-CaMV.35S-enh-1:1:11 | 52786 | NONE | / | NONE | / | NONE | | T-Gb.E6-3b:1:1 | 5279 |
| pMON74552 | P-CaMV.35S-enh-1:1:11 | 52786 | NONE | / | NONE | / | TS-At.ShkG-CTP2-1:1:1 | 52799 | T-Gb.E6-3b:1:1 | 5279 |
| pMON82053 | P-CaMV.35S-enh-1:1:11 | 52786 | NONE | / | NONE | / | NONE | / | T-Gb.E6-3b:1:1 | 5279 |
| pMON74537 | P-At.RbcS4-1:1:2 | 52784 | L-At.RbcS4-1:1:3 | 52774 | NONE | / | NONE | / | T-Gb.E6-3b:1:1 | 5279 |
| pMON74536 | P-Br.Snap2-1:1:1 | 52785 | L-Br.Snap2-1:1:1 | 52775 | NONE | / | NONE | / | T-Gb.E6-3b:1:1 | 5279 |
| pMON74548 | P-Gm.Sphas1-1:1:1 | 52796 | L-Gm.Sphas1-1:1:1 | 52802 | NONE | / | NONE | / | T-Gb.E6-3b:1:1 | 5279 |
| pMON74539 | P-At.RbcS4-1:1:2 | 52784 | L-At.RbcS4-1:1:3 | 52774 | NONE | / | TS-At.ShkG-CTP2-1:1:1 | 52799 | T-Gb.E6-3b:1:1 | 5279 |

Plasmids for use in transformation of soybean were also prepared. Elements of an exemplary common expression vector plasmid pMON74532 are shown in Table 6 below.

Table 6

| Function | Element | Reference |
|---|---|---|
| Agro transformation | B-ARGtu.right border | Depicker, A. et al (1982) Mol Appl Genet 1:561-573 |
| Antibiotic resistance | CR-Ec.aadA-SPC/STR | |
| Repressor of primers from the ColE 1 plasmid | CR-Ec.rop | |
| Origin of replication | OR-Ec.oriV-RK2 | |
| Agro transformation | B-ARGtu.left border | Barker, R.F. et al (1983) Plant Mol Biol 2:335-350 |
| Plant selectable marker expression cassette | Promoter with intron and 5'UTR of Arabidopsis act 7 gene (AtAct7) | McDowell et al. (1996) Plant Physiol. 111:699-711. |
| | 5' UTR of Arabidopsis act 7 gene | |
| | Intron in 5'UTR of AtAct7 | |
| | Transit peptide region of Arabidopsis EPSPS | Klee, H.J. et al (1987) MGG 210: 437-442 |
| | Synthetic CP4 coding region with dicot preferred codon usage | |
| | A 3' UTR of the nopaline synthase gene of Agrobacterium tumefaciens Ti plasmid | U.S. Patent 5,858,742 |

(continued)

| Function | Element | Reference |
|---|---|---|
| Plant gene of interest expression cassette | Promoter for 35S RNA from CaMV containing a duplication of the -90 to -350 region | U.S. Patent 5,322,938 |
| | Gene of interest insertion site | |
| | Cotton E6 3' end | GenBank accession U30508 |

Primers for PCR amplification of protein coding nucleotides of recombinant DNA were designed at or near the start and stop codons of the coding sequence, in order to eliminate most of the 5' and 3' untranslated regions. Each recombinant DNA coding for a protein identified in Table 1 was amplified by PCR prior to insertion into the insertion site of one of the base vectors as referenced in Table 1.

**Example 2. Corn Transformation**

This example illustrates plant cell transformation methods useful in producing transgenic corn plant cells, plants, seeds and pollen of this invention and the production and identification of transgenic corn plants and seed with an enhanced trait, i.e. enhanced water use efficiency, enhanced cold tolerance, increased yield, enhanced nitrogen use efficiency, enhanced seed protein and enhanced seed oil. Plasmid vectors were prepared by cloning DNA identified in Table I in the identified base vectors for use in corn transformation of corn plant cells to produce transgenic corn plants and progeny plants, seed and pollen.

For Agrobacterium-mediated transformation of corn embryo cells corn plants of a readily transformable line (designated LH59) is grown in the greenhouse and ears harvested when the embryos are 1.5 to 2.0 mm in length. Ears are surface sterilized by spraying or soaking the ears in 80% ethanol, followed by air drying. Immature embryos are isolated from individual kernels on surface sterilized ears. Prior to inoculation of maize cells, *Agrobacterium* cells are grown overnight at room temperature. Immature maize embryo cells are inoculated with *Agrobacterium* shortly after excision, and incubated at room temperature with *Agrobacterium* for 5-20 minutes. Immature embryo plant cells are then co-cultured with *Agrobacterium* for 1 to 3 days at 23°C in the dark. Co-cultured embryos are transferred to selection media and cultured for approximately two weeks to allow embryogenic callus to develop. Embryogenic callus is transferred to culture medium containing 100 mg/L paromomycin and subcultured at about two week intervals. Transformed plant cells are recovered 6 to 8 weeks after initiation of selection.

For *Agrobacterium*-mediated transformation of maize callus immature embryos are cultured for approximately 8-21 days after excision to allow callus to develop. Callus is then incubated for about 30 minutes at room temperature with the *Agrobacterium* suspension, followed by removal of the liquid by aspiration. The callus and *Agrobacterium* are co-cultured without selection for 3-6 days followed by selection on paromomycin for approximately 6 weeks, with biweekly transfers to fresh media, and paromomycin resistant callus identified as containing the recombinant DNA in an expression cassette.

For transformation by microprojectile bombardment immature maize embryos are isolated and cultured 3-4 days prior to bombardment. Prior to microprojectile bombardment, a suspension of gold particles is prepared onto which the desired recombinant DNA expression cassettes are precipitated. DNA is introduced into maize cells as described in U.S. Patents 5,550,318 and 6,399,861 using the electric discharge particle acceleration gene delivery device. Following microprojectile bombardment, tissue is cultured in the dark at 27 degrees C. Additional transformation methods and materials for making transgenic plants of this invention, for example, various media and recipient target cells, transformation of immature embryos and subsequence regeneration of fertile transgenic plants are disclosed in U.S. Patents 6,194,636 and 6,232,526 and U.S. Patent application Serial No. 09/757,089, which are incorporated herein by reference.

To regenerate transgenic corn plants a callus of transgenic plant cells resulting from transformation is placed on media to initiate shoot development in plantlets which are transferred to potting soil for initial growth in a growth chamber at 26 degrees C followed by a mist bench before transplanting to 5 inch pots where plants are grown to maturity. The regenerated plants are self fertilized and seed is harvested for use in one or more methods to select seed, seedlings or progeny second generation transgenic plants (R2 plants) or hybrids, e.g. by selecting transgenic plants exhibiting an enhanced trait as compared to a control plant.

Transgenic corn plant cells are transformed with recombinant DNA from each of the genes identified in Table 1. Progeny transgenic plants and seed of the transformed plant cells are screened for enhanced water use efficiency, enhanced cold tolerance, increased yield, enhanced nitrogen use efficiency, enhanced seed protein and enhanced seed oil as reported in Example 5.

**Example 3. Soybean transformation**

This example illustrates plant transformation useful in producing the transgenic soybean plants of this invention and the production and identification of transgenic seed for transgenic soybean having enhanced water use efficiency, enhanced cold tolerance, increased yield, enhanced nitrogen use efficiency, enhanced seed protein and enhanced seed oil.

For *Agrobacterium* mediated transformation, soybean seeds are germinated overnight and the meristem explants excised. The meristems and the explants are placed in a wounding vessel. Soybean explants and induced *Agrobacterium* cells from a strain containing plasmid DNA with the gene of interest cassette and a plant selectable marker cassette are mixed no later than 14 hours from the time of initiation of seed germination and wounded using sonication. Following wounding, explants are placed in co-culture for 2-5 days at which point they are transferred to selection media for 6-8 weeks to allow selection and growth of transgenic shoots. Trait positive shoots are harvested approximately 6-8 weeks and placed into selective rooting media for 2-3 weeks. Shoots producing roots are transferred to the greenhouse and potted in soil. Shoots that remain healthy on selection, but do not produce roots are transferred to non-selective rooting media for an additional two weeks. Roots from any shoots that produce roots off selection are tested for expression of the plant selectable marker before they are transferred to the greenhouse and potted in soil. Additionally, a DNA construct can be transferred into the genome of a soybean cell by particle bombardment and the cell regenerated into a fertile soybean plant as described in U.S. Patent 5,015,580, herein incorporated by reference.

Transgenic soybean plant cells are transformed with recombinant DNA from each of the genes identified in Table 1. Progeny transgenic plants and seed of the transformed plant cells are screened for enhanced water use efficiency, enhanced cold tolerance, increased yield, enhanced nitrogen use efficiency, enhanced seed protein and enhanced seed oil as reported in Example 5.

**Example 4. Homolog Identification**

This example illustrates the identification of homologs of proteins encoded by the DNA identified in Table 1 which is used to provide transgenic seed and plants having enhanced agronomic traits. From the sequence of the homologs, homologous DNA sequence can be identified for preparing additional transgenic seeds and plants of this invention with enhanced agronomic traits.

An "All Protein Database" was constructed of known protein sequences using a proprietary sequence database and the National Center for Biotechnology Information (NCBI) non-redundant amino acid database (nr.aa). For each organism from which a polynucleotide sequence provided herein was obtained, an "Organism Protein Database" was constructed of known protein sequences of the organism; it is a subset of the All Protein Database based on the NCBI taxonomy ID for the organism.

The All Protein Database was queried using amino acid sequences provided herein as SEQ ID NO: 142 through SEQ ID NO:1482 using NCBI "blastp" program with E-value cutoff of 1e-8. Up to 1000 top hits were kept, and separated by organism names. For each organism other than that of the query sequence, a list was kept for hits from the query organism itself with a more significant E-value than the best hit of the organism. The list contains likely duplicated genes of the polynucleotides provided herein, and is referred to as the Core List. Another list was kept for all the hits from each organism, sorted by E-value, and referred to as the Hit List.

The Organism Protein Database was queried using polypeptide sequences provided herein as SEQ ID NO: 142 through SEQ ID NO: 1482 using NCBI "blastp" program with E-value cutoff of 1e-4. Up to 1000 top hits were kept. A B LAST searchable database was constructed based on these hits, and is referred to as "SubDB". SubDB was queried with each sequence in the Hit List using NCBI "blastp" program with E-value cutoff of 1e-8. The hit with the best E-value was compared with the Core List from the corresponding organism. The hit is deemed a likely ortholog if it belongs to the Core List, otherwise it is deemed not a likely ortholog and there is no further search of sequences in the Hit List for the same organism. Homologs from a large number of distinct organisms were identified and are reported by amino acid sequences of SEQ ID NO: 1483 through SEQ ID NO: 52767. These relationship of proteins of SEQ ID NO: 742 through 166 and homologs of SEQ ID NO:1482 through 52767 is identified in Table 2. The source organism for each homolog is found in the Sequence Listing.

**Example 5. Selection of transgenic plants with enhanced agronomic trait(s)**

This example illustrates identification of plant cells of the invention by screening derived plants and seeds for enhanced trait. Transgenic corn seed and plants with recombinant DNA identified in Table 1 were prepared by plant cells transformed with DNA that was stably integrated into the genome of the corn cell. The transgenic seed, plantlets and progeny plants were selected using the methods that measure Transgenic corn plant cells were transformed with recombinant DNA from each of the genes identified in Table 1. Progeny transgenic plants and seed of the transformed plant cells were screened for enhanced water use efficiency, enhanced cold tolerance, increased yield, enhanced nitrogen use efficiency, enhanced seed protein and enhanced seed oil as compared to control plants.

## A. Selection for enhanced Nitrogen Use Efficiency

The physiological efficacy of transgenic corn plants (tested as hybrids) can be tested for nitrogen use efficiency (NUE) traits in a high-throughput nitrogen (N) selection method. The collected data are compared to the measurements from wildtype controls using a statistical model to determine if the changes are due to the transgene. Raw data were analyzed by SAS software. Results shown herein are the comparison of transgenic plants relative to the wildtype controls.

(1) Media Preparation for Planting a NUE Protocol

Planting materials used: Metro Mix 200 (vendor: Hummert) Cat. # 10-0325, Scotts Micro Max Nutrients (vendor: Hummert) Cat. # 07-6330, OS 4 1/3" x 3 7/8" pots (vendor: Hummert) Cat. # 16-1415, OS trays (vendor: Hummert) Cat. # 16-1515, Hoagland's macronutrients solution, Plastic 5" stakes (vendor: Hummert) yellow Cat. # 49-1569, white Cat. # 49-1505, Labels with numbers indicating material contained in pots. Fill 500 pots to rim with Metro Mix 200 to a weight of ~140g/pot. Pots are filled uniformly by using a balancer. Add 0.4g of Micro Max nutrients to each pot. Stir ingredients with spatula to a depth of 3 inches while preventing material loss.

(2) Planting a NUE selection in the Greenhouse

(a) Seed Germination - Each pot is lightly atered twice using reverse osmosis purified water. The first watering is scheduled to occur just before planting; and the second watering, after the seed has been planted in the pot. Ten Seeds of each entry (1 seed per pot) are planted to select eight healthy uniform seedlings. Additional wild type controls are planted for use as border rows. Alternatively, 15 seeds of each entry (1 seed per pot) are planted to select 12 healthy uniform seedlings (this larger number of plantings is used for the second, or confirmation, planting). Place pots on each of the 12 shelves in the Conviron growth chamber for seven days. This is done to allow more uniform germination and early seedling growth. The following growth chamber settings are 25° C/day and 22° C/night, 14 hours light and ten hours dark, humidity ~ 80%, and light intensity -350 $\mu$mol/m$^2$/s (at pot level). Watering is done via capillary matting similar to greenhouse benches with duration of ten minutes three times a day.
(b) Seedling transfer - After seven days, the best eight or 12 seedlings for the first or confirmation pass runs, respectively, are chosen and transferred to greenhouse benches. The pots are spaced eight inches apart (center to center) and are positioned on the benches using the spacing patterns printed on the capillary matting. The Vattex matting creates a 384-position grid, randomizing all range, row combinations. Additional pots of controls are placed along the outside of the experimental block to reduce border effects.
Plants are allowed to grow for 28 days under the low N run or for 23 days under the high N run. The macronutrients are dispensed in the form of a macronutrient solution (see composition below) containing precise amounts of N added (2mM $NH_4NO_3$ for limiting N selection and 20mM $NH_4NO_3$ for high N selection runs). Each pot is manually dispensed 100ml of nutrient solution three times a week on alternate days starting at eight and ten days after planting for high N and low N runs, respectively. On the day of nutrient application, two 20 min waterings at 05:00 and 13:00 are skipped. The vattex matting should be changed every third run to avoid N accumulation and buildup of root matter. Table 7 shows the amount of nutrients in the nutrient solution for either the low or high nitrogen selection.

Table 7

| Nutrient Stock | 2mM $NH_4NO_3$ (Low Nitrogen Growth Condition, Low N) | 20mM $NH_4NO_3$ (high Nitrogen Growth Condition, High N) |
|---|---|---|
| | mL/L | mL/L |
| 1 M $NH_4NO_3$ | 2 | 20 |
| 1 M $KH_2PO_4$ | 0.5 | 0.5 |
| 1 M $MgSO_4.7H_2O$ | 2 | 2 |
| 1 M $CaCl_2$ | 2.5 | 2.5 |
| 1 M $K_2SO_4$ | 1 | 1 |
| Note: Adjust pH to 5.6 with HCl or KOH | | |

(c) Harvest Measurements and Data Collection - After 28 days of plant growth for low N runs and 23 days of plant growth for high N runs, the following measurements are taken (phenocodes in parentheses): total shoot fresh mass (g) (SFM) measured by Sartorius electronic balance, V6 leaf chlorophyll measured by Minolta SPAD meter (relative units) (LC), V6 leaf area ($cm^2$) (LA) measured by a Li-Cor leaf area meter, V6 leaf fresh mass (g) (LFM) measured by Sartorius electronic balance, and V6 leaf dry mass (g) (LDM) measured by Sartorius electronic balance. Raw data were analyzed by SAS software. Results shown are the comparison of transgenic plants relative to the wildtype controls.

To take a leaf reading, samples were excised from the V6 leaf. Since chlorophyll meter readings of corn leaves are affected by the part of the leaf and the position of the leaf on the plant that is sampled, SPAD meter readings were done on leaf six of the plants. Three measurements per leaf were taken, of which the first reading was taken from a point one-half the distance between the leaf tip and the collar and halfway from the leaf margin to the midrib while two were taken toward the leaf tip. The measurements were restricted in the area from 1/2 to 3/4 of the total length of the leaf (from the base) with approximately equal spacing between them. The average of the three measurements was taken from the SPAD machine.

Leaf fresh mass is recorded for an excised V6 leaf, the leaf is placed into a paper bag. The paper bags containing the leaves are then placed into a forced air oven at 80° C for 3 days. After 3 days, the paper bags are removed from the oven and the leaf dry mass measurements are taken.

From the collected data, two derived measurements are made: (1)Leaf chlorophyll area (LCA), which is a product of V6 relative chlorophyll content and its leaf area (relative units). Leaf chlorophyll area= leaf chlorophyll X leaf area. This parameter gives an indication of the spread of chlorophyll over the entire leaf area; (2)specific leaf area (LSA) is calculated as the ratio of V6 leaf area to its dry mass ($cm^2$/g dry mass), a parameter also recognized as a measure of NUE. The data are shown in Table 8.

Nitrogen use field efficacy assay

Level I. Transgenic plants provided by the present invention are planted in field without any nitrogen source being applied. Transgenic plants and control plants are grouped by genotype and construct with controls arranged randomly within genotype blocks. Each type of transgenic plants are tested by 3 replications and across 5 locations. Nitrogen levels in the fields are analyzed in early April pre-planting by collecting 30 sample soil cores from 0-24" and 24 to 48" soil layer. Soil samples are analyzed for nitrate-nitrogen, phosphorus(P), Potassium(K), organic matter and pH to provide baseline values. P, K and micronutrients are applied based upon soil test recommendations.

Level II. Transgenic plants provided by the present invention are planted in field with three levels of nitrogen (N) fertilizer being applied, i.e. low level (0 N), medium level (80 lb/ac) and high level (180 lb/ac). Liquid 28% or 32% UAN (Urea, Ammonium Nitrogen) are used as the N source and apply by broadcast boom and incorporate with a field cultivator with rear rolling basket in the same direction as intended crop rows. Although there is no N applied to the 0 N treatment the soil should still be disturbed in the same fashion as the treated area. Transgenic plants and control plants are grouped by genotype and construct with controls arranged randomly within genotype blocks. Each type of transgenic plants is tested by 3 replications and across 4 locations. Nitrogen levels in the fields are analyzed in early April pre-planting by collecting 30 sample soil cores from 0-24" and 24 to 48" soil layer. Soil samples are analyzed for nitrate-nitrogen, phosphorus(P), Potassium(K), organic matter and pH to provide baseline values. P, K and micronutrients are applied based upon soil test recommendations.

## B. Selection for increased yield

Many transgenic plants of this invention exhibit improved yield as compared to a control plant. Improved yield can result from enhanced seed sink potential, i.e. the number and size of endosperm cells or kernels and/or enhanced sink strength, i.e. the rate of starch biosynthesis. Sink potential can be established very early during kernel development, as endosperm cell number and size are determined within the first few days after pollination.

Much of the increase in corn yield of the past several decades has resulted from an increase in planting density. During that period, corn yield has been increasing at a rate of 2.1 bushels/acre/year, but the planting density has increased at a rate of 250 plants/acre/year. A characteristic of modern hybrid corn is the ability of these varieties to be planted at high density. Many studies have shown that a higher than current planting density should result in more biomass production, but current germplasm does not perform. well at these higher densities. One approach to increasing yield is to increase harvest index (HI), the proportion of biomass that is allocated to the kernel compared to total biomass, in high density plantings.

Effective yield selection of enhanced yielding transgenic corn events uses hybrid progeny of the transgenic event over multiple locations with plants grown under optimal production management practices, and maximum pest control. A useful target for improved yield is a 5% to 10% increase in yield as compared to yield produced by plants grown from seed for a control plant. Selection methods may be applied in multiple and diverse geographic locations, for example up to 16 or more locations, over one or more plating seasons, for example at least two planting seasons to statistically distinguish yield improvement from natural environmental effects. It is to plant multiple transgenic plants, positive and negative control plants, and pollinator plants in standard plots, for example 2 row plots, 20 feet long by 5 feet wide with 30 inches distance between rows and a 3 foot alley between ranges. Transgenic events can be grouped by recombinant DNA constructs with groups randomly placed in the field. A pollinator plot of a high quality corn line is planted for every two plots to allow open pollination when using male sterile transgenic events. A useful planting density is about 30,000 plants/acre. High planting density is greater than 30,000 plants/acre, preferably about 40,000 plants/acre, more preferably about 42,000 plants/acre, most preferably about 45,000 plants/acre. Surrogate indicators for yield improvement include source capacity (biomass), source output (sucrose and photosynthesis), sink components (kernel size, ear size, starch in the seed), development (light response, height, density tolerance), maturity, early flowering trait and physiological responses to high density planting, for example at 45,000 plants per acre, for example as illustrated in Table 10 and 11.

Table 8

| Timing | Evaluation | Description | comments |
|---|---|---|---|
| V2-3 | Early stand | Can be taken any time after germination and prior to removal of any plants. | |
| Pollen shed | GDU to 50% shed | GDU to 50% plants shedding 50% tassel. | |
| Silking | GDU to 50% silk | GDU to 50% plants showing silks. | |
| Maturity | Plant height | Height from soil surface to flag leaf attachment (inches). | 10 plants per plot - Yield team assistance |
| Maturity | Ear height | Height from soil surface to primary ear attachment node. | 10 plants per plot - Yield team assistance |
| Maturity | Leaves above ear | visual scores: erect, size, rolling | |
| Maturity | Tassel size | Visual scores +/- vs. WT | |
| Pre-Harvest | Final Stand | Final stand count prior to harvest, exclude tillers | |
| Pre-Harvest | Stalk lodging | No. of stalks broken below the primary ear attachment. Exclude leaning tillers | |
| Pre-Harvest | Root lodging | No. of stalks leaning >45° angle from perpendicular. | |
| Pre-Harvest | Stay green | After physiological maturity and when differences among genotypes are evident: Scale 1 (90-100% tissue green) - 9 (0-19% tissue green). | |
| Harvest | Grain Yield | Grain yield/plot (Shell weight) | |

Table 9

| Timing | Evaluation | Description |
|---|---|---|
| V8 - V12 | Chlorophyll | |
| V12-VT | Ear leaf area | |
| V15 - 15DAP | Chl fluorescence | |
| V15 - 15DAP | CER | |
| 15 - 25 DAP | Carbohydrates | sucrose, starch |

(continued)

| Timing | Evaluation | Description |
|---|---|---|
| Pre-Harvest | 1st internode diameter | |
| Pre-Harvest | Base 3 internode diameter | |
| Pre-Harvest | Ear internode diameter | |
| Maturity | Ear traits | diameter, length, kernel number, kernel weight |

Electron transport rates (ETR) and CO2 exchange rates (CER): ETR and CER are measured with Li6400LCF (Licor, Lincoln, NE) around V9-R1 stages. Leaf chlorophyll fluorescence is a quick way to monitor the source activity and is reported to be highly correlated with $CO_2$ assimilation under varies conditions (Photosyn Research, 37: 89-102). The youngest fully expanded leaf or 2 leaves above the ear leaf is measured with actinic light 1500 (with 10% blue light) micromol $m^{-2} s^{-1}$, 28oC, CO2 levels 450ppm. Ten plants are measured in each event. There were 2 readings for each plant.

A hand-held chlorophyll meter SPAD-502 (Minolta - Japan) is used to measure the total chlorophyll level on live transgenic plants and the wild type counterparts a. Three trifoliates from each plant are analyzed, and each trifoliate were analyzed three times. Then 9 data points are averaged to obtain the chlorophyll level. The number of analyzed plants of each genotype ranges from 5 to 8.

When selecting for yield improvement a useful statistical measurement approach comprises three components, i.e. modeling spatial autocorrelation of the test field separately for each location, adjusting traits of recombinant DNA events for spatial dependence for each location, and conducting an across location analysis. The first step in modeling spatial autocorrelation is estimating the covariance parameters of the semivariogram. A spherical covariance model is assumed to model the spatial autocorrelation. Because of the size and nature of the trial, it is likely that the spatial autocorrelation may change. Therefore, anisotropy is also assumed along with spherical covariance structure. The following set of equations describes the statistical form of the anisotropic spherical covariance model.

$$C(h;\theta) \doteq vI(h=0) + \sigma^2\left(1 - \frac{3}{2}h + \frac{1}{2}h^3\right)I(h<1),$$

where $I(\bullet)$ is the indicator function, $h = \sqrt{\dot{x}^2 + \dot{y}^2}$, and

$$\dot{x} = [\cos(\rho\pi/180)(x_1 - x_2) - \sin(\rho\pi/180)(y_1 - y_2)]/\omega_x$$

$$\dot{y} = [\sin(\rho\pi/180)(x_1 - x_2) + \cos(\rho\pi/180)(y_1 - y_2)]/\omega_y$$

where $s_1 = (x_1, y_1)$ are the spatial coordinates of one location and $s_2 = (x_2, y_2)$ are the spatial coordinates of the second location. There are 5 covariance parameters, $\theta = (v,\sigma^2,\rho,\omega_n,\omega_j)$, where $v$ is the nugget effect, $\sigma^2$ is the partial sill, $\rho$ is a rotation in degrees clockwise from north, $\omega_n$ is a scaling parameter for the minor axis and $\omega_j$ is a scaling parameter for the major axis of an anisotropical ellipse of equal covariance. The five covariance parameters that defines the spatial trend will then be estimated by using data from heavily replicated pollinator plots via restricted maximum likelihood approach. In a multi-location field trial, spatial trend are modeled separately for each location.

After obtaining the variance parameters of the model, a variance-covariance structure is generated for the data set to be analyzed. This variance-covariance structure contains spatial information required to adjust yield data for spatial dependence. In this case, a nested model that best represents the treatment and experimental design of the study is used along with the variance-covariance structure to adjust the yield data. During this process the nursery or the seed batch effects can also be modeled and estimated to adjust the yields for any yield parity caused by seed batch differences. After spatially adjusted data from different locations are generated, all adjusted data is combined and analyzed assuming locations as replications. In this analysis, intra and inter-location variances are combined to estimate the standard error of yield from transgenic plants and control plants. Relative mean comparisons are used to indicate statistically significant yield improvements.

**C. Selection for enhanced water use efficiency (WUE)**

Described in this example is a high-throughput method for greenhouse selection of transgenic corn plants to wild type corn plants (tested as inbreds or hybrids) for water use efficiency. This selection process imposes 3 drought/re-water cycles on plants over a total period of 15 days after an initial stress free growth period of I days. Each cycle consists of 5 days, with no water being applied for the first four days and a water quenching on the 5th day of the cycle. The primary phenotypes analyzed by the selection method are the changes in plant growth rate as determined by height and biomass during a vegetative drought treatment. The hydration status of the shoot tissues following the drought is also measured. The plant height are measured at three time points. The first is taken just prior to the onset drought when the plant is 11 days old, which is the shoot initial height (SIH). The plant height is also measured halfway throughout the drought/re-water regimen, on day 18 after planting, to give rise to the shoot mid-drought height (SMH). Upon the completion of the final drought cycle on day 26 after planting, the shoot portion of the plant is harvested and measured for a final height, which is the shoot wilt height (SWH) and also measured for shoot wilted biomass (SWM). The shoot is placed in water at 40 degree Celsius in the dark. Three days later, the shoot is weighted to give rise to the shoot turgid weight (STM). After drying in an oven for four days, the shoots are weighted for shoot dry biomass (SDM). The shoot average height (SAH) is the mean plant height across the 3 height measurements. The procedure described above may be adjusted for +/- ~ one day for each step given the situation.

To correct for slight differences between plants, a size corrected growth value is derived from SIH and SWH. This is the Relative Growth Rate (RGR). Relative Growth Rate (RGR) is calculated for each shoot using the formula [RGR% = (SWH-SIH)/((SWH+SIH)/2)* 100]. Relative water content (RWC) is a measurement of how much (%) of the plant was water at harvest. Water Content (RWC) is calculated for each shoot using the formula [RWC% = (SWM-SDM)/(STM-SDM)* 100]. Fully watered corn plants of this age run around 98% RWC.

**D. Selection for Growth Under Cold Stress**

(1) Cold germination assay - Three sets of seeds are used for the assay. The first set consists of positive transgenic events (F hybrid) where the genes of the present invention are expressed in the seed. The second seed set is nontransgenic, wild-type negative control made from the same genotype as the transgenic events. The third set consisted of two cold tolerant and one cold sensitive commercial check lines of corn. All seeds are treated with a fungicide " Captan" (MAESTRO® 80DF Fungicide, Arvesta Corporation, San Francisco, CA, USA). 0.43 mL Captan is applied per 45 g of corn seeds by mixing it well and drying the fungicide prior to the experiment.

Corn kernels are placed embryo side down on blotter paper within an individual cell (8.9 x 8.9 cm) of a germination tray (54 x 36 cm). Ten seeds from an event are placed into one cell of the germination tray. Each tray can hold 21 transgenic events and 3 replicates of wildtype (LH244SDms+LH59), which is randomized in a complete block design. For every event there are five replications (five trays). The trays are placed at 9.7C for 24 days (no light) in a Convrion growth chamber *(Conviron Model PGV36, Controlled Environments, Winnipeg, Canada).* Two hundred and fifty millilters of deionized water are added to each germination tray. Germination counts are taken 10th, 11th, 12th, 13th, 14th, 17th, 19th, 21st, and 24th day after start date of the experiment. Seeds are considered germinated if the emerged radicle size is1 cm. From the germination counts germination index is calculated.

*The germination index is calculated as per:*

$$Germination\ index = (\Sigma\ ([T+1-n_i]*[P_i-P_{i-1}]))/T$$

Where T is the total number of days for which the germination assay is performed. The number of days after planting is defined by n. "i" indicated the number oftimes the germination had been counted, including the current day. P is the percentage of seeds germinated during any given rating. Statistical differences are calculated between transgenic events and wild type control. After statistical analysis, the events that show a statistical significance at the p level of less than 0.1 relative to wild-type controls will advance to a secondary cold selection. The secondary cold screen is conducted in the same manner of the primary selection only increasing the number of repetitions to ten. Statistical analysis of the data from the secondary selection is conducted to identify the events that show a statistical significance at the p level of less than 0.05 relative to wild-type controls.

(2) Cold Shock assay - The experimental set-up for the cold shock assay is the same as described in the above cold germination assay except seeds were grown in potted media for the cold shock assay.

The desired numbers of 2.5" square plastic pots are placed on flats (n=32, 4x8). Pots were filled with Metro Mix 200 soil-less media containing 19:6:12 fertilizer (6 lbs/cubic yard) (Metro Mix, Pots and Flat are obtained from Hummert International, Earth City, MO). After planting seeds, pots are placed in a growth chamber set at 23° C, relative humidity of 65% with 12 hour day and night photoperiod (300 uE/m2-min). Planted seeds are watered for 20 minute every other day by sub-irrigation and flats were rotated every third day in a growth chamber for growing corn seedlings. On the 10th day after planting the transgenic positive and wild-type negative (WT) plants are positioned in flats in an alternating pattern. Chlorophyll fluorescence of plants is measured on the 10th day during the dark period of growth by using a PAM-2000 portable fluorometer as per the manufacturer's instructions (Walz, Germany). After chlorophyll measurements, leaf samples from each event are collected for confirming the expression of genes of the present invention. For expression analysis six V1 leaf tips from each selection are randomly harvested. The flats are moved to a growth chamber set at 5° C. All other conditions such as humidity, day/night cycle and light intensity are held constant in the growth chamber. The flats are sub-irrigated every day after transfer to the cold temperature. On the 4th day chlorophyll fluorescence is measured. Plants are transferred to normal growth conditions after six days of cold shock treatment and allowed to recover for the next three days. During this recovery period the length of the V3 leaf is measured on the 1st and 3rd days. After two days of recovery V2 leaf damage is determined visually by estimating percent of green V2 leaf.

Statistical differences in V3 leaf growth, V2 leaf necrosis and fluorescence during pre- shock and cold shock can be used for estimation of cold shock damage on corn plants.

(3) Early seedling growth assay - Three sets of seeds are used for the experiment. The first set consists of positive transgenic events (F1 hybrid) where the genes of the present invention are expressed in the seed. The second seed set is nontransgenic, wild-type negative control made from the same genotype as the transgenic events. The third seed set consists of two cold tolerant and two cold sensitive commercial check lines of corn. All seeds are treated with a fungicide " Captan", (3a,4,7,a-tetrahydro-2-[(trichloromethly)thio]-1H-isoindole-1,3(2H)-dione, Drex Chemical Co. Memphis, TN). Captan (0.43 mL) was applied per 45 g of corn seeds by mixing it well and drying the fungicide prior to the experiment.

Seeds are grown in germination paper for the early seedling growth assay. Three 12"x18" pieces of germination paper (Anchor Paper #SD7606) are used for each entry in the test (three repetitions per transgenic event). The papers are wetted in a solution of 0.5% $KNO_3$ and 0.1% Thyram.

For each paper fifteen seeds are placed on the line evenly spaced down the length of the paper. The fifteen seeds are positioned on the paper such that the radical would grow downward, for example longer distance to the paper's edge. The wet paper is rolled up starting from one of the short ends. The paper is rolled evenly and tight enough to hold the seeds in place. The roll is secured into place with two large paper clips, one at the top and one at the bottom. The rolls are incubated in a growth chamber at 23° C for three days in a randomized complete block design within an appropriate container. The chamber is set for 65% humidity with no light cycle. For the cold stress treatment the rolls are then incubated in a growth chamber at 12° C for twelve days. The chamber is set for 65% humidity with no light cycle.

After the cold treatment the germination papers are unrolled and the seeds that did not germinate are discarded. The lengths of the radicle and coleoptile for each seed are measured through an automated imaging program that automatically collects and processes the images. The imaging program automatically measures the shoot length, root length, and whole seedling length of every individual seedling and then calculates the average of each roll.

After statistical analysis, the events that show a statistical significance at the p level of less than 0.1 relative to wild-type controls will advance to a secondary cold selection. The secondary cold selection is conducted in the same manner of the primary selection only increasing the number of repetitions to five. Statistical analysis of the data from the secondary selection is conducted to identify the events that show a statistical significance at the p level of less than 0.05 relative to wild-type controls.

4. Cold field efficacy trial

This example sets forth a cold field efficacy trial to identify gene constructs that confer enhanced cold vigor at germination and early seedling growth under early spring planting field conditions in conventional-till and simulated no-till environments. Seeds are planted into the ground around two weeks before local farmers are beginning to plant corn so that a significant cold stress is exerted onto the crop, named as cold treatment. Seeds also are planted under local optimal planting conditions such that the crop has little or no exposure to cold condition, named as normal treatment. The cold field efficacy trials are carried out in five locations, including Glyndon MN, Mason MI, Monmouth IL, Dayton IA, Mystic CT. At each location, seeds are planted under both cold and normal conditions with 3 repetitions per treatment, 20 kernels per row and single row per plot. Seeds are planted 1.5 to 2 inch deep into soil to avoid muddy conditions. Two temperature monitors are set up at each location to monitor both air and soil temperature daily.

Seed emergence is defined as the point when the growing shoot breaks the soil surface. The number of emerged seedling in each plot is counted everyday from the day the earliest plot begins to emerge until no significant changes in emergence occur. In addition, for each planting date, the latest date when emergence is 0 in all plots is also recorded. Seedling vigor is also rated at V3-V4 stage before the average of corn plant height reaches 10 inches, with 1=excellent early growth, 5=Average growth and 9=poor growth. Days to 50% emergence, maximum percent emergence and seedling vigor are calculated using SAS software for the data within each location or across all locations.

E. Screens for transgenic plant seeds with increased protein and/or oil levels

This example sets forth a high-throughput selection for identifying plant seeds with improvement in seed composition using the Infratec 1200 series Grain Analyzer, which is a near-infrared transmittance spectrometer used to determine the composition of a bulk seed sample. Near infrared analysis is a non-destructive, high-throughput method that can analyze multiple traits in a single sample scan.. An NIR calibration for the analytes of interest is used to predict the values of an unknown sample. The NIR spectrum is obtained for the sample and compared to the calibration using a complex chemometric software package that provides a predicted values as well as information on how well the sample fits in the calibration.

Infratec Model 1221, 1225, or 1227 with transport module by Foss North America is used with cuvette, item # 1000-4033, Foss North America or for small samples with small cell cuvette, Foss standard cuvette modified by Leon Girard Co. Corn and soy check samples of varying composition maintained in check cell cuvettes are supplied by Leon Girard Co. NIT collection software is provided by Maximum Consulting Inc.Software. Calculations are performed automatically by the software. Seed samples are received in packets or containers with barcode labels from the customer. The seed is poured into the cuvettes and analyzed as received.

Table 10.

| Typical sample(s): | Whole grain corn and soybean seeds |
|---|---|
| Analytical time to run method: | Less than 0.75 min per sample |
| Total elapsed time per run: | 1.5 minute per sample |
| Typical and minimum sample size: | Corn typical: 50cc; minimum 30cc Soybean typical: 50cc; minimum 5cc |
| Typical analytical range: | Determined in part by the specific calibration. Corn - moisture 5-15%, oil 5-20%, protein 5-30%, starch 50-75%, and density 1.0-1.3%. Soybean - moisture 5-15%, oil 15-25%, and protein 35-50%. |

**Example 6. Consensus sequence**

This example illustrates the identification of consensus amino acid sequence for the proteins and homologs encoded by DNA that is used to prepare the transgenic seed and plants ofthis invention having enhanced agronomic traits.

ClustalW program-was selected for multiple sequence alignments of the amino acid sequence of SEQ ID NO: 1205 and its 12 homologs. Three major factors affecting the sequence alignments dramatically are (1) protein weight matrices; (2) gap open penalty; (3) gap extension penalty. Protein weight matrices available for ClustalW program include Blosum, Pam and Gonnet series. Those parameters with gap open penalty and gap extension penalty were extensively tested. On the basis of the test results, Blosum weight matrix, gap open penalty of 10 and gap extension penalty of 1 were chosen for multiple sequence alignment. Figure 2 shows the sequences of SEQ ID NO: 1205, its homologs and the consensus sequence (SEQ ID NO: 52803) at the end. The symbols for consensus sequence are (1) uppercase letters for 100% identity in all positions of multiple sequence alignment output; (2) lowercase letters for >=70% identity; symbol; (3) "X" indicated <70% identity; (4) dashes "-" meaning that gaps were in >=70% sequences.

The consensus amino acid sequence can be used to identify DNA corresponding to the full scope of this invention that is useful in providing transgenic plants, for example corn and soybean plants with enhanced agronomic traits, for example improved nitrogen use efficiency, improved yield, improved water use efficiency and/or improved growth under cold stress, due to the expression in the plants of DNA encoding a protein with amino acid sequence identical to the consensus amino acid sequence.

Example 7. Identification of amino acid domain by Pfam analysis

The amino acid sequence of the expressed proteins that were shown to be associated with an enhanced trait were analyzed for Pfam protein family against the current Pfam collection of multiple sequence alignments and hidden Markov models using the HMMER software in the appended computer listing. The Pfam protein families for the proteins of SEQ ID NO: 742 through 1482 are shown in Table 11. The Hidden Markov model databases for the identified patent families are also in the appended computer listing allowing identification of other homologous proteins and their cognate encoding DNA to enable the full breadth of the invention for a person of ordinary skill in the art. Certain proteins are identified by a single Pfam domain and others by multiple Pfam domains. For instance, the protein with amino acids of SEQ ID NO: 817 is characterized by two Pfam domains, i.e. GTP_EFTU, GTP_EFTU_D2 and GTP_EFTU_D3.

Table 11.

| PEP SEQ ID NO | NUC SEQ ID NO | GENE ID | Pfam domain name | begin | stop | score | E-value |
|---|---|---|---|---|---|---|---|
| 816 | 75 | PHE0000035_61 | bZIP_1 | 15 | 79 | 41.9 | 2.00E-09 |
| 816 | 75 | PHE0000035_61 | bZIP_2 | 15 | 69 | 42.8 | 1.10E-09 |
| 817 | 76 | PHE0000036_62 | GTP_EFTU | 5 | 227 | 359.5 | 4.70E-1051 |
| 817 | 76 | PHE0000036_62 | GTP_EFTU_D2 | 248 | 315 | 91.9 | 1.70E-24 |
| 817 | 76 | PHE0000036_62 | GTP_EFTU_D3 | 322 | 430 | 207.6 | 2.60E-59 |
| 818 | 77 | PHE0000130_220 | Jacalin | 185 | 318 | 84.5 | 3.00E-22 |
| 1177 | 436 | PHE0000132_222 | PCI | 63 | 159 | 44.2 | 4.00E-10 |
| 819 | 78 | PHE0000134_224 | LEA_5 | 1 | 113 | 151.2 | 2.40E-42 |
| 820 | 79 | PHE0000135_225 | PP2C | 39 | 281 | 226.2 | 6.60E-65 |
| 821 | 80 | PHE0000136_226 | Hist_deacetyl | 31 | 344 | 579.9 | 2.20E-171 |
| 822 | 81 | PHE0000139_229 | peroxidase | 19 | 227 | 239.9 | 4.80E-69 |
| 823 | 82 | PHE0000141_231 | Thioredoxin | 184 | 295 | -0.9 | 2.00E-05 |
| 824 | 83 | PHE0000142_232 | Ras | 11 | 179 | 280.2 | 3.60E-81 |
| 825 | 84 | PHE0000146_236 | Ras | 13 | 174 | 335 | 1.10E-97 |
| 826 | 85 | PHE0000148_238 | Pkinase | 12 | 267 | 323.2 | 3.90E-94 |
| 826 | 85 | PHE0000148_238 | Pkinase_Tyr | 12 | 265 | 70.9 | 3.70E-18 |
| 826 | 85 | PHE0000148_238 | NAF | 310 | 369 | 116.1 | 9.20E-32 |
| 827 | 86 | PHE0000149_239 | AP2 | 29 | 92 | 128.5 | 1.70E-35 |
| 828 | 87 | PHE0000150_240 | Lectin_legB | 23 | 212 | 8 | 2.60E-11 |
| 828 | 87 | PHE0000150_240 | Lectin_legA | 221 | 265 | 37.6 | 3.80E-08 |
| 828 | 87 | PHE0000150_240 | Pkinase | 344 | 496 | -11.1 | 2.00E-07 |
| 829 | 88 | PHE0000189_282 | HSP20 | 49 | 152 | 169.6 | 7.30E-48 |
| 830 | 89 | PHE0000200_293 | PP2C | 22 | 317 | 296.5 | 4.30E-86 |
| 831 | 90 | PHE0000211_304 | Pkinase | 30 | 288 | 384.1 | 1.90E-112 |
| 832 | 91 | PHE0000213_306 | Pkinase | 64 | 322 | 348.7 | 8.50E-102 |
| 1178 | 437 | PHE0000285_375 | bZIP_1 | 27 | 91 | 50.9 | 3.80E-12 |
| 1178 | 437 | PHE0000285_375 | bZIP_2 | 27 | 81 | 40.8 | 4.00E-09 |
| 833 | 92 | PHE0000368_459 | bZIP_1 | 21 | 85 | 51.2 | 3.10E-12 |
| 833 | 92 | PHE0000368_459 | bZIP_2 | 21 | 75 | 38.5 | 2.00E-08 |
| 834 | 93 | PHE0000369_460 | Pkinase | 17 | 269 | 375.3 | 8.50E-110 |
| 834 | 93 | PHE0000369_460 | Pkinase Tyr | 17 | 267 | 78.6 | 1.70E-20 |
| 834 | 93 | PHE0000369_460 | UBA | 291 | 330 | 23.4 | 0.0007 |
| 834 | 93 | PHE0000369_460 | KA1 | 459 | 507 | 89.8 | 7.20E-24 |

(continued)

| PEP SEQ ID NO | NUC SEQ ID NO | GENE ID | Pfam domain name | begin | stop | score | E-value |
|---|---|---|---|---|---|---|---|
| 835 | 94 | PHE0000370_461 | Pkinase | 4 | 260 | 298.7 | 9.60E-87 |
| 1340 | 599 | PHE0000667_770 | Acyltransferase | 174 | 337 | 127.2 | 4.00E-35 |
| 1179 | 438 | PHE0000668_771 | Acyltransferase | 207 | 367 | 167.4 | 3.10E-47 |
| 836 | 95 | PHE0000780_853 | SRF-TF | 9 | 59 | 119.7 | 7.20E-33 |
| 836 | 95 | PHE0000780_853 | K-box | 74 | 173 | 147.6 | 3.00E-41 |
| 837 | 96 | PHE0000782_855 | SRF-TF | 9 | 59 | 113.7 | 4.80E-31 |
| 837 | 96 | PHE0000782_855 | K-box | 73 | 174 | 151 | 2.80E-42 |
| 838 | 97 | PHE0000783_856 | SRF-TF | 9 | 59 | 120.2 | 5.30E-33 |
| 838 | 97 | PHE0000783_856 | K-box | 74 | 174 | 145.6 | 1.20E-40 |
| 1341 | 600 | PHE0000784_857 | K-box | 4 | 103 | 147.6 | 3.00E-41 |
| 839 | 98 | PHE0000785_858 | SRF-TF | 9 | 59 | 119.7 | 7.20E-33 |
| 839 | 98 | PHE0000785_858 | K-box | 74 | 173 | 147.6 | 3.00E-41 |
| 1294 | 553 | PHE0000788_861 | SRF-TF | 9 | 59 | 113.7 | 4.80E-31 |
| 1294 | 553 | PHE0000788_861 | K-box | 73 | 174 | 151 | 2.80E-42 |
| 1295 | 554 | PHE0000789_862 | SRF-TF | 9 | 59 | 113.7 | 4.80E-31 |
| 1180 | 439 | PHE0000790_863 | K-box | 4 | 103 | 150.5 | 4.10E-42 |
| 840 | 99 | PHE0000791_864 | SRF-TF | 9 | 59 | 121 | 2.90E-33 |
| 840 | 99 | PHE0000791_864 | K-box | 74 | 173 | 150.5 | 4.10E-42 |
| 841 | 100 | PHE0000792_865 | SRF-TF | 9 | 59 | 121 | 2.90E-33 |
| 842 | 101 | PHE0000794_867 | SRF-TF | 9 | 59 | 120.2 | 5.30E-33 |
| 842 | 101 | PHE0000794_867 | K-box | 74 | 174 | 145.6 | 1.20E-40 |
| 1296 | 555 | PHE0000795_868 | SRF-TF | 9 | 59 | 120.2 | 5.30E-33 |
| 843 | 102 | PHE0000821_896 | DUF6 | 16 | 153 | 32 | 1.90E-06 |
| 843 | 102 | PHE0000821_896 | DUF6 | 190 | 319 | 45.4 | 1.80E-10 |
| 1342 | 601 | PHE0000880_963 | HAMP | 193 | 262 | 64.2 | 3.90E-16 |
| 1342 | 601 | PHE0000880_963 | PAS | 276 | 388 | 36.7 | 7.30E-08 |
| 1342 | 601 | PHE0000880_963 | HisKA | 397 | 465 | 94.4 | 3.00E-25 |
| 1342 | 601 | PHE0000880_963 | HATPase_c | 510 | 629 | 143.2 | 6.10E-40 |
| 1297 | 556 | PHE0000881_964 | HAMP | 200 | 269 | 65.7 | 1.30E-16 |
| 1297 | 556 | PHE0000881_964 | PAS | 283 | 413 | 29.7 | 9.10E-06 |
| 1297 | 556 | PHE0000881_964 | HisKA | 422 | 490 | 95.9 | 1.00E-25 |
| 1297 | 556 | PHE0000881_964 | HATPase_c | 535 | 655 | 138.8 | 1.40E-38 |
| 1343 | 602 | PHE000088_966 | Response_reg | 276 | 397 | 106.2 | 8.60E-29 |
| 844 | 103 | PHE0001051_1141 | zf-C2H2 | 54 | 76 | 25.1 | 0.00022 |
| 844 | 103 | PHE0001051_1141 | zf-C2H2 | 130 | 152 | 19.2 | 0.014 |
| 845 | 104 | PHE0001053_4292 | NAM | 24 | 155 | 282.4 | 7.60E-82 |
| 846 | 105 | PHE0001054_1144 | KH_1 | 133 | 220 | 13.4 | 0.017 |

(continued)

| PEP SEQ ID NO | NUC SEQ ID NO | GENE ID | Pfam domain name | begin | stop | score | E-value |
|---|---|---|---|---|---|---|---|
| 847 | 106 | PHE0001055_1145 | CBS | 140 | 285 | 37.6 | 3.90E-08 |
| 847 | 106 | PHE0001055_1145 | CBS | 316 | 447 | 48 | 2.80E-11 |
| 849 | 108 | PHE0001057_1147 | Gamma-thionin | 32 | 80 | 77.6 | 3.50E-20 |
| 1344 | 603 | PHE0001063_1153 | CBS | 35 | 175 | 29.2 | 1.30E-05 |
| 1344 | 603 | PHE0001063_1153 | CBS | 193 | 318 | 80.8 | 3.80E-21 |
| 850 | 109 | PHE0001064_1154 | AMPKBI | 304 | 416 | 208.7 | 1.20E-59 |
| 1345 | 604 | PHE0001065_155 | AMPKBI | 298 | 412 | 210.9 | 2.50E-60 |
| 804 | 63 | PHE0001066_1156 | AMPKBI | 734 | 852 | 209.3 | 7.70E-60 |
| 1346 | 605 | PHE0001069_1159 | bZIP_2 | 78 | 135 | 40.6 | 4.90E-09 |
| 1346 | 605 | PHE0001069_1159 | bZIP_1 | 82 | 142 | 47.6 | 3.70E-11 |
| 851 | 110 | PHE0001071_1161 | AP2 | 68 | 131 | 149.2 | 9.50E-42 |
| 852 | 111 | PHE0001073_1163 | Myb_DNA-binding | 14 | 61 | 44.4 | 3.30E-10 |
| 852 | 111 | PHE0001073_1163 | Myb_DNA-binding | 67 | 112 | 55.5 | 1.60E-13 |
| 1181 | 440 | PHE0001074_1164 | NAM | 19 | 146 | 255.1 | 1.30E-73 |
| 853 | 112 | PHE0001075_1165 | Myb_DNA-binding | 22 | 69 | 47.5 | 4.10E-11 |
| 853 | 112 | PHE0001075_1165 | Myb_DNA-binding | 75 | 120 | 51 | 3.50E-12 |
| 1347 | 606 | PHE0001076_1166 | NAM | 9 | 138 | 293.9 | 2.70E-85 |
| 854 | 113 | PHE0001077_1167 | Myb_DNA-binding | 227 | 278 | 44.3 | 3.60E-10 |
| 1182 | 441 | PHE0001078_1168 | NAM | 8 | 137 | 304.2 | 2.10E-88 |
| 1183 | 442 | PHE0001079_1169 | NAM | 14 | 142 | 309 | 7.60E-90 |
| 1348 | 607 | PHE0001080_1170 | AP2 | 135 | 198 | 137.8 | 2.50E-38 |
| 1184 | 443 | PHE0001082_1172 | NAM | 13 | 143 | 299 | 7.80E-87 |
| 855 | 114 | PHE0001083_1173 | AP2 | 23 | 86 | 147.7 | 2.70E-41 |
| 1185 | 444 | PHE0001085_1175 | HLH | 381 | 430 | 50.8 | 4.10E-12 |
| 1349 | 608 | PHE0001087_1177 | Homeobox | 73 | 127 | 74.6 | 2.90E-19 |
| 1349 | 608 | PHE0001087_1177 | HALZ | 128 | 172 | 81.3 | 2.70E-21 |
| 742 | 1 | PHE0001089_1179 | NAM | 14 | 140 | 289.7 | 4.90E-84 |
| 1350 | 609 | PHE0001094_1184 | zf-C2H2 | 106 | 128 | 19.6 | 0.0097 |
| 856 | 115 | PHE0001095_1185 | bZIP_1 | 23 | 87 | 36.4 | 8.90E-08 |
| 856 | 115 | PHE0001095_1185 | bZIP_2 | 23 | 77 | 28.9 | 1.60E-05 |
| 857 | 116 | PHE0001096_1186 | MFMR | 1 | 201 | 374.1 | 1.90E-109 |
| 857 | 116 | PHE0001096_1186 | bZIP_1 | 303 | 367 | 91.7 | 2.00E-24 |
| 857 | 116 | PHE0001096_1186 | bZIP_2 | 303 | 357 | 31 | 3.80E-06 |
| 858 | 117 | PHE0001097_1187 | bZIP_1 | 42 | 95 | 22.7 | 0.00044 |

(continued)

| PEP SEQ ID NO | NUC SEQ ID NO | GENE ID | Pfam domain name | begin | stop | score | E-value |
|---|---|---|---|---|---|---|---|
| 858 | 117 | PHE0001097_1187 | bZIP_2 | 42 | 92 | 19.3 | 0.0073 |
| 859 | 118 | PHE0001098_1188 | GATA | 160 | 195 | 65.5 | 1.50E-16 |
| 860 | 119 | PHE0001099_1189 | MFMR | 1 | 182 | 333.5 | 3.20E-97 |
| 860 | 119 | PHE0001099_1189 | bZIP_1 | 257 | 321 | 95.2 | 1.80E-25 |
| 860 | 119 | PHE0001099_189 | bZIP_2 | 257 | 311 | 38.9 | 1.60E-08 |
| 1351 | 610 | PHE0001100_1190 | bZIP_1 | 187 | 245 | 46 | 1.20E-10 |
| 1351 | 610 | PHE0001100_1190 | bZIP_2 | 188 | 241 | 44.6 | 2.90E-10 |
| 861 | 120 | PHE0001101_1191 | GATA | 181 | 216 | 65.5 | 1.50E-16 |
| 862 | 121 | PHE0001107_1197 | Myb DNA-binding | 24 | 69 | 53.4 | 6.60E-13 |
| 863 | 122 | PHE0001108_1198 | Aminotran_1_2 | 69 | 470 | 61.3 | 2.90E-15 |
| 864 | 123 | PHE0001109_1199 | Aminotran_1_2 | 84 | 469 | 51.8 | 2.00E-12 |
| 865 | 124 | PHE0001110_1200 | Aminotran_1_2 | 61 | 462 | 47.4 | 4.40E-11 |
| 1352 | 611 | PHE0001112_1202 | Aminotran_1_2 | 98 | 491 | 46.2 | 9.80E-11 |
| 1186 | 445 | PHE0001113_1203 | Aminotran_5 | 20 | 363 | 28.9 | 2.40E-091 |
| 866 | 125 | PHE0001115_1205 | Aminotran_1_2 | 92 | 459 | 285.3 | 1.00E-82 |
| 866 | 125 | PHE0001115_1205 | Cys_Met_Meta_PP | 104 | 339 | -275.1 | 0.0034 |
| 867 | 126 | PHE0001120_1210 | Histone | 62 | 137 | 48.9 | 1.50E-11 |
| 867 | 126 | PHE0001120_1210 | CBFD_NFYB_HMF | 68 | 132 | 88.1 | 2.40E-23 |
| 868 | 127 | PHE0001123_1213 | Histone | 92 | 167 | 50.1 | 6.50E-12 |
| 868 | 127 | PHE0001123_1213 | CBFD_NFYB_HMF | 98 | 162 | 89.4 | 1.00E-23 |
| 869 | 128 | PHE0001125_1215 | Histone | 94 | 169 | 51.5 | 2.50E-12 |
| 869 | 128 | PHE0001125_1215 | CBFD_NFYB_HMF | 100 | 164 | 92.7 | 1.00E-24 |
| 870 | 129 | PHE0001126_1216 | Myb_DNA-binding | 123 | 170 | 44.8 | 2.50E-10 |
| 871 | 130 | PHE0001127_1217 | BURP | 299 | 519 | 131.7 | 1.80E-36 |
| 872 | 131 | PHE0001128_1218 | Homeobox | 48 | 103 | 67.3 | 4.40E-17 |
| 872 | 131 | PHE0001128_1218 | HALZ | 104 | 148 | 41.8 | 2.10E-09 |
| 873 | 132 | PHE0001130_1220 | IlvN | 50 | 233 | 112.8 | 9.00E-31 |
| 873 | 132 | PHE0001130_1220 | IlvC | 237 | 522 | 375.3 | 8.50E-110 |
| 1353 | 612 | PHE0001131_1221 | IlvN | 74 | 244 | 189.2 | 8.90E-54 |
| 1353 | 612 | PHE0001131_1221 | IlvC | 248 | 395 | 236.5 | 5.20E-68 |
| 874 | 133 | PHE0001132_1222 | Aminotran_1_2 | 33 | 401 | 489.3 | 4.00E-144 |
| 743 | 2 | PHE0001133_1223 | Aminotran_1_2 | 44 | 419 | 339.8 | 4.20E-99 |
| 744 | 3 | PHE0001134_1224 | Aminotran_1_2 | 44 | 441 | 408.3 | 9.70E-120 |

(continued)

| PEP SEQ ID NO | NUC SEQ ID NO | GENE ID | Pfam domain name | begin | stop | score | E-value |
|---|---|---|---|---|---|---|---|
| 745 | 4 | PHE0001135_1225 | Aminotran_1_2 | 26 | 392 | 492.5 | 4.40E-145 |
| 1187 | 446 | PHE0001136_1226 | Aminotran_1_2 | 31 | 399 | 484.3 | 1.30E-142 |
| 875 | 134 | PHE0001138_1228 | SapB_1 | 59 | 98 | 21.7 | 0.0023 |
| 875 | 134 | PHE0001138_1228 | SapB_2 | 100 | 133 | 23 | 0.00093 |
| 875 | 134 | PHE0001138_1228 | SapB_1 | 145 | 183 | 27.3 | 4.90E-05 |
| 875 | 134 | PHE0001138_1228 | SapB_2 | 186 | 220 | 29.4 | 1.10E-05 |
| 1188 | 447 | PHE0001142_1232 | Methyltransf_3 | 66 | 278 | 415.7 | 5.90E-122 |
| 1354 | 613 | PHE0001143_1233 | HMA | 10 | 70 | 43.3 | 7.20E-10 |
| 876 | 135 | PHE0001145_1235 | F-box | 51 | 104 | 33.3 | 7.30E-07 |
| 877 | 136 | PHE0001146_1236 | Pkinase | 67 | 321 | 269.3 | 6.90E-78 |
| 878 | 137 | PHE0001147_1237 | DUF641 | 22 | 159 | 217.4 | 2.90E-62 |
| 879 | 138 | PHE0001148_1238 | Myb_DNA-binding | 221 | 272 | 48 | 2.80E-11 |
| 1355 | 614 | PHE0001149_1239 | B12D | 2 | 87 | 225.8 | 8.70E-65 |
| 880 | 139 | PHE0001151_1241 | BURP | 355 | 575 | 127.6 | 3.00E-35 |
| 881 | 140 | PHE0001152_1242 | Histone | 58 | 134 | 151.1 | 2.60E-42 |
| 882 | 141 | PHE0001153_1243 | Pkinase | 34 | 292 | 329.8 | 4.30E-96 |
| 883 | 142 | PHE0001154_1244 | Histone | 55 | 127 | 104.9 | 2.10E-28 |
| 885 | 144 | PHE0001157_1247 | DUF760 | 6 | 416 | 592.5 | 3.50E-175 |
| 886 | 145 | PHE0001158_1248 | DUF250 | 243 | 388 | 179.6 | 6.90E-51 |
| 887 | 146 | PHE0001159_1249 | DnaJ | 67 | 134 | 103.9 | 4.20E-28 |
| 888 | 147 | PHE0001167_1257 | YL1 | 11 | 236 | 211.9 | 1.30E-60 |
| 888 | 147 | PHE0001167_1257 | YL1_C | 264 | 293 | 64 | 4.30E-16 |
| 1356 | 615 | PHE0001169_1259 | Chromo | 108 | 158 | 73.2 | 7.20E-19 |
| 889 | 148 | PHE0001171_1261 | Chromo | 62 | 112 | 79.8 | 7.50E-21 |
| 890 | 149 | PHE0001172_1262 | Chromo | 111 | 161 | 73.7 | 5.10E-19 |
| 1357 | 616 | PHE0001176_1266 | Heme_oxygenase | 3 | 205 | 448.6 | 7.40E-132 |
| 1358 | 617 | PHE0001177_1267 | Heme_oxygenase | 3 | 206 | 384.9 | 1.10E-112 |
| 891 | 150 | PHE0001179_1269 | AUX_IAA | 10 | 229 | 299.1 | 7.10E-87 |
| 746 | 5 | PHE0001181_1271 | AUX_IAA | 10 | 228 | 296.6 | 4.10E-86 |
| 1359 | 618 | PHE0001184_1274 | AUX_IAA | 50 | 273 | 324.3 | 1.90E-94 |
| 892 | 151 | PHE0001198_1288 | WRKY | 209 | 267 | 151.2 | 2.40E-42 |
| 892 | 151 | PHE0001198_1288 | WRKY | 381 | 440 | 155.7 | 1.10E-43 |
| 893 | 152 | PHE0001199_1289 | WRKY | 97 | 155 | 144.9 | 1.90E-40 |
| 893 | 152 | PHE0001199_1289 | WRKY | 272 | 331 | 151.5 | 2.00E-42 |
| 894 | 153 | PHE0001208_1298 | AP2 | 119 | 183 | 147.3 | 3.60E-41 |

(continued)

| PEP SEQ ID NO | NUC SEQ ID NO | GENE ID | Pfam domain name | begin | stop | score | E-value |
|---|---|---|---|---|---|---|---|
| 895 | 154 | PHE0001211_1301 | AP2 | 81 | 142 | 117.4 | 3.70E-32 |
| 895 | 154 | PHE0001211_1301 | B3 | 209 | 330 | 110.8 | 3.50E-30 |
| 896 | 155 | PHE0001212_1302 | SAM_2 | 17 | 84 | 65.9 | 1.10E-16 |
| 896 | 155 | PHE0001212_1302 | SAM_1 | 18 | 82 | 36.6 | 7.60E-08 |
| 896 | 155 | PHE0001212_1302 | Pkinase_Tyr | 415 | 710 | 109.7 | 7.70E-30 |
| 896 | 155 | PHE0001212_1302 | Pkinase | 415 | 712 | 292.2 | 8.50E-85 |
| 897 | 156 | PHE0001214_1304 | Pkinase | 400 | 656 | 303.4 | 3.80E-88 |
| 897 | 156 | PHE0001214_1304 | Pkinase_Tyr | 400 | 656 | 147.4 | 3.40E-41 |
| 899 | 158 | PHE0001220_1310 | Pkinase | 40 | 324 | 310.4 | 2.90E-90 |
| 900 | 159 | PHE0001221_1311 | Pkinase | 63 | 347 | 307.5 | 2.10E-89 |
| 901 | 160 | PHE0001225_1315 | Catalase | 18 | 402 | 937.9 | 13.70E-279 |
| 1298 | 557 | PHE0001226_1316 | Catalase | 18 | 401 | 937.7 | 4.30E-279 |
| 747 | 6 | PHE0001227_1317 | Catalase | 27 | 432 | 595.4 | 4.70E-176 |
| 748 | 7 | PHE0001228_1318 | PBD | 336 | 394 | 103.4 | 6.00E-28 |
| 748 | 7 | PHE0001228_1318 | Pkinase | 620 | 871 | 372 | 8.20E-109 |
| 748 | 7 | PHE0001228_1318 | Pkinase_Tyr | 620 | 869 | 109 | 1.30E-29 |
| 902 | 161 | PHE0001238_1328 | Metallothio_2 | 1 | 76 | 138.7 | 1.40E-38 |
| 903 | 162 | PHE0001239_1329 | Pkinase | 67 | 351 | 309.7 | 4.70E-90 |
| 1190 | 449 | PHE0001240_1330 | H_PPase | 9 | 687 | 1072.7 | 0 |
| 904 | 163 | PHE0001241_1331 | YTH | 492 | 582 | 205.8 | 9.10E-59 |
| 905 | 164 | PHE0001242_1332 | Thioredoxin | 31 | 138 | 174.5 | 2.40E-49 |
| 905 | 164 | PHE0001242_1332 | Thioredoxin | 149 | 257 | 187 | 4.20E-53 |
| 905 | 164 | PHE0001242_1332 | ERp29 | 271 | 365 | 172 | 1.30E-48 |
| 1191 | 450 | PHE0001243_1333 | F-box | 53 | 106 | 36.2 | 1.00E-07 |
| 1191 | 450 | PHE0001243_1333 | Tub | 117 | 313 | 182.1 | 1.20E-51 |
| 906 | 165 | PHE0001244_1334 | LIM | 9 | 66 | 54.4 | 3.40E-13 |
| 906 | 165 | PHE0001244_1334 | LIM | 110 | 167 | 66 | 1.10E-16 |
| 907 | 166 | PHE0001245_1335 | W2 | 243 | 319 | 66.1 | 9.70E-17 |
| 908 | 167 | PHE0001246_1337 | zf-C3HC4 | 262 | 302 | 43.2 | 8.10E-10 |
| 749 | 8 | PHE0001247_1338 | Cyclin_N | 67 | 196 | 145.4 | 1.40E-40 |
| 749 | 8 | PHE0001247_1338 | Cyclin_C | 198 | 325 | 52 | 1.80E-12 |
| 1360 | 619 | PHE0001256_1347 | Trypsin | 114 | 309 | 18.9 | 1.80E-05 |
| 1193 | 452 | PHE0001257_1348 | Fer4 | 116 | 139 | 30.6 | 4.90E-06 |
| 1193 | 452 | PHE0001257_1348 | Fer4 | 155 | 178 | 38.5 | 2.10E-08 |
| 910 | 169 | PHE0001258_1349 | Fer4 | 117 | 140 | 28.5 | 1.10E-05 |
| 910 | 169 | PHE0001258_1349 | Fer4 | 156 | 179 | 38.5 | 2.10E-08 |
| 911 | 170 | PHE0001259_1350 | Fer4 | 116 | 139 | 28.6 | 1.00E-05 |

(continued)

| PEP SEQ ID NO | NUC SEQ ID NO | GENE ID | Pfam domain name | begin | stop | score | E-value |
|---|---|---|---|---|---|---|---|
| 911 | 170 | PHE0001259_1350 | Fer4 | 155 | 178 | 38.5 | 2.10E-08 |
| 1362 | 621 | PHE0001265_1356 | efhand | 70 | 98 | 17.9 | 0.034 |
| 1195 | 454 | PHE0001266_1357 | efhand | 77 | 105 | 30.3 | 6.20E-06 |
| 1363 | 622 | PHE0001268_1359 | DUF788 | 1 | 169 | 339.1 | 6.90E-99 |
| 1364 | 623 | PHE0001270_1361 | ERO1 | 64 | 414 | 748.4 | 4.10E-222 |
| 752 | 11 | PHE0001275_1336 | zf-C3HC4 | 257 | 297 | 34.6 | 3.00E-07 |
| 912 | 171 | PHE0001279_1369 | Cystatin | 41 | 129 | 107.9 | 2.60E-29 |
| 913 | 172 | PHE0001280_1370 | Cystatin | 49 | 137 | 80.5 | 4.70E-21 |
| 914 | 173 | PHE0001282_1372 | Chal_sti_synt_C | 342 | 486 | 13.2 | 0.00012 |
| 1196 | 455 | PHE0001283_1373 | ArfGap | 4 | 120 | 173.7 | 4.00E-49 |
| 915 | 174 | PHE0001284_1374 | ArfGap | 4 | 120 | 175.1 | 1.60E-49 |
| 916 | 175 | PHE0001285 1375 | ArfGap | 6 | 122 | 184.5 | 2.30E-52 |
| 1197 | 456 | PHE0001286_1376 | IPK | 17 | 274 | 413.1 | 3.50E-121 |
| 917 | 176 | PHE0001291_1381 | Aa_trans | 27 | 433 | 115.1 | 1.80E-31 |
| 917 | 176 | PHE0001291_1381 | Trp_Tyr_perm | 27 | 368 | -211.7 | 6.50E-05 |
| 918 | 177 | PHE0001292_1382 | Aa_trans | 40 | 446 | 166 | 8.30E-47 |
| 918 | 177 | PHE0001292_1382 | Trp_Tyr_perm | 40 | 443 | -201.1 | 2.20E-05 |
| 919 | 178 | PHE0001297_1387 | DNA photolyase | 15 | 188 | 237.1 | 3.40E-68 |
| 919 | 178 | PHE0001297_1387 | FAD_binding_7 | 219 | 497 | 502.9 | 3.20E-148 |
| 920 | 179 | PHE0001299_1389 | DNA_photolyase | 5 | 176 | 224 | 2.90E-64 |
| 920 | 179 | PHE0001299_1389 | FAD_binding_7 | 212 | 490 | 464.5 | 1.20E-136 |
| 1198 | 457 | PHE0001301_1391 | Ras | 30 | 192 | 290 | 3.90E-84 |
| 921 | 180 | PHE0001303_1393 | PRA1 | 50 | 203 | 227.8 | 2.20E-65 |
| 1199 | 458 | PHE0001304_1394 | PRA1 | 47 | 197 | 221 | 2.30E-63 |
| 922 | 181 | PHE0001306_1396 | PRA1 | 26 | 177 | 171.5 | 1.90E-48 |
| 1365 | 624 | PHE0001311_1401 | NUDIX | 24 | 160 | 78.9 | 1.50E-20 |
| 923 | 182 | PHE0001312_1402 | NUDIX | 22 | 153 | 62.1 | 1.60E-15 |
| 924 | 183 | PHE0001313_1403 | NUDIX | 19 | 165 | 65 | 2.10E-16 |
| 925 | 184 | PHE0001314_1404 | NUDIX | 19 | 159 | 71.7 | 2.00E-18 |
| 1200 | 459 | PHE0001316_1406 | NUDIX | 20 | 150 | 69.9 | 7.40E-18 |
| 1201 | 460 | PHE0001317_1407 | NUDIX | 19 | 165 | 64.4 | 3.30E-16 |
| 926 | 185 | PHE0001332_1423 | Pkinase | 17 | 272 | 336.5 | 4.00E-98 |
| 926 | 185 | PHE0001332_1423 | NAF | 330 | 389 | 64.3 | 3.50E-16 |
| 927 | 186 | PHE0001333_1424 | Pkinase | 19 | 274 | 341.8 | 1.00E-99 |
| 927 | 186 | PHE0001333_1424 | Pkinase_Tyr | 19 | 274 | 70.1 | 6.30E-18 |
| 927 | 186 | PHE0001333_1424 | NAF | 309 | 369 | 108.9 | 1.30E-29 |

(continued)

| PEP SEQ ID NO | NUC SEQ ID NO | GENE ID | Pfam domain name | begin | stop | score | E-value |
|---|---|---|---|---|---|---|---|
| 928 | 187 | PHE0001334_1425 | Pkinase | 14 | 266 | 382.4 | 6.10E-112 |
| 928 | 187 | PHE0001334_1425 | Pkinase_Tyr | 14 | 264 | 86.3 | 8.10E-23 |
| 928 | 187 | PHE0001334_1425 | UBA | 288 | 327 | 22.3 | 0.0016 |
| 928 | 187 | PHE0001334_1425 | KA1 | 456 | 504 | 101.5 | 2.20E-27 |
| 1300 | 559 | PHE0001335_1426 | Pkinase | 46 | 300 | 359.6 | 4.60E-105 |
| 1300 | 559 | PHE0001335_1426 | NAF | 383 | 440 | 103 | 8.00E-28 |
| 929 | 188 | PHE0001337_1428 | AMPKBI | 190 | 277 | 111.5 | 2.20E-30 |
| 930 | 189 | PHE0001339_1430 | AMPKBI | 194 | 281 | 123.1 | 7.10E-34 |
| 931 | 190 | PHE0001343_1434 | Pkinase | 13 | 268 | 341.6 | 1.20E-99 |
| 931 | 190 | PHE0001343_1434 | NAF | 307 | 367 | 124.7 | 2.30E-34 |
| 932 | 191 | PHE0001346_1437 | Pkinase | 12 | 266 | 351.8 | 1.00E-102 |
| 932 | 191 | PHE0001346_1437 | Pkinase_Tyr | 12 | 264 | 72 | 1.70E-18 |
| 932 | 191 | PHE0001346_1437 | NAF | 313 | 374 | 99.9 | 7.00E-27 |
| 933 | 192 | PHE0001347_1438 | Pkinase | 15 | 269 | 345.1 | 1.00E-100 |
| 933 | 192 | PHE0001347_1438 | Pkinase_Tyr | 15 | 267 | 80.1 | 6.10E-21 |
| 933 | 192 | PHE0001347_1438 | NAF | 319 | 378 | 108.3 | 2.10E-29 |
| 934 | 193 | PHE0001349_1440 | Pkinase | 13 | 268 | 341 | 1.80E-99 |
| 934 | 193 | PHE0001349_1440 | Pkinase_Tyr | 13 | 266 | 74 | 4.30E-19 |
| 934 | 193 | PHE0001349_1440 | NAF | 308 | 369 | 112.8 | 8.60E-31 |
| 935 | 194 | PHE0001350_1441 | Pkinase | 13 | 268 | 334.8 | 1.30E-97 |
| 935 | 194 | PHE0001350_1441 | NAF | 307 | 367 | 132.7 | 8.80E-37 |
| 936 | 195 | PHE0001354_1445 | Pkinase | 21 | 276 | 331.7 | 1.10E-96 |
| 936 | 195 | PHE0001354_1445 | Pkinase_Tyr | 21 | 276 | 65.1 | 8.60E-17 |
| 936 | 195 | PHE0001354_1445 | NAF | 310 | 370 | 94.3 | 3.20E-25 |
| 937 | 196 | PHE0001355_1446 | Pkinase | 17 | 272 | 325.1 | 1.10E-94 |
| 937 | 196 | PHE0001355_1446 | NAF | 300 | 368 | 57.7 | 3.40E-14 |
| 938 | 197 | PHE0001356_1447 | Pkinase | 22 | 283 | 325.8 | 6.90E-95 |
| 938 | 197 | PHE0001356_1447 | NAF | 326 | 385 | 100.6 | 4.10E-27 |
| 939 | 198 | PHE0001357_1448 | Pkinase | 16 | 269 | 302.2 | 8.40E-88 |
| 939 | 198 | PHE0001357_1448 | NAF | 309 | 370 | 98.4 | 1.90E-26 |
| 940 | 199 | PHE0001358_1449 | Pkinase | 14 | 266 | 380.4 | 2.40E-111 |
| 940 | 199 | PHE0001358_1449 | Pkinase_Tyr | 14 | 264 | 84.4 | 3.10E-22 |
| 940 | 199 | PHE0001358_1449 | UBA | 288 | 327 | 22.8 | 0.0011 |
| 940 | 199 | PHE0001358_1449 | KA1 | 454 | 502 | 94.9 | 2.20E-25 |
| 941 | 200 | PHE0001359_1450 | Pkinase_Tyr | 17 | 267 | 72.7 | 1.00E-18 |
| 941 | 200 | PHE0001359_1450 | Pkinase | 17 | 269 | 366.1 | 5.00E-107 |
| 941 | 200 | PHE0001359_1450 | UBA | 291 | 330 | 20.9 | 0.0042 |

(continued)

| PEP SEQ ID NO | NUC SEQ ID NO | GENE ID | Pfam domain name | begin | stop | score | E-value |
|---|---|---|---|---|---|---|---|
| 941 | 200 | PHE0001359_1450 | KA1 | 460 | 508 | 84.8 | 2.40E-22 |
| 942 | 201 | PHE0001360_1451 | CAF1 | 15 | 248 | 399.9 | 3.30E-117 |
| 943 | 202 | PHE0001361_1452 | AP2 | 21 | 84 | 135.2 | 1.60E-37 |
| 944 | 203 | PHE0001362_1453 | GATA | 220 | 255 | 68.2 | 2.30E-17 |
| 945 | 204 | PHE0001363_1455 | NAM | 11 | 135 | 260.6 | 2.80E-75 |
| 946 | 205 | PHE0001364_1456 | CBS | 48 | 262 | 43.1 | 8.40E-10 |
| 946 | 205 | PHE0001364_1456 | CBS | 285 | 422 | 99.1 | 1.20E-26 |
| 947 | 206 | PHE0001375_1467 | WD40 | 159 | 196 | 50.2 | 6.30E-12 |
| 947 | 206 | PHE0001375_1467 | WD40 | 201 | 238 | 34.4 | 3.40E-07 |
| 947 | 206 | PHE0001375_1467 | WD40 | 243 | 280 | 41.8 | 2.10E-09 |
| 947 | 206 | PHE0001375_1467 | WD40 | 285 | 322 | 34.1 | 4.30E-07 |
| 947 | 206 | PHE0001375_1467 | WD40 | 327 | 363 | 15.4 | 0.19 |
| 947 | 206 | PHE0001375_1467 | WD40 | 369 | 405 | 10.4 | 1.4 |
| 947 | 206 | PHE0001375_1467 | WD40 | 419 | 455 | 15.4 | 0.18 |
| 948 | 207 | PHE0001377_1469 | Pkinase | 91 | 407 | 238.5 | 1.30E-68 |
| 949 | 208 | PHE0001380_1472 | HEAT | 82 | 117 | 18.3 | 0.025 |
| 949 | 208 | PHE0001380_1472 | HEAT | 158 | 194 | 23.5 | 0.00069 |
| 949 | 208 | PHE0001380_1472 | HEAT | 197 | 233 | 18.6 | 0.02 |
| 949 | 208 | PHE0001380_1472 | HEAT | 236 | 272 | 32.2 | 1.60E-06 |
| 949 | 208 | PHE0001380_1472 | HEAT | 275 | 311 | 29.8 | 8.50E-06 |
| 949 | 208 | PHE0001380_1472 | HEAT | 314 | 350 | 26.6 | 8.00E-05 |
| 949 | 208 | PHE0001380_1472 | HEAT | 353 | 389 | 28.8 | 1.70E-05 |
| 949 | 208 | PHE0001380_1472 | HEAT | 392 | 428 | 26.4 | 9.20E-05 |
| 949 | 208 | PHE0001380_1472 | HEAT | 431 | 467 | 20 | 0.0074 |
| 949 | 208 | PHE0001380_1472 | HEAT | 470 | 506 | 18 | 0.03 |
| 949 | 208 | PHE0001380_1472 | HEAT | 509 | 545 | 21.7 | 0.0023 |
| 949 | 208 | PHE0001380_1472 | HEAT | 548 | 584 | 22.1 | 0.0018 |
| 1301 | 560 | PHE0001381_1473 | SRF-TF | 9 | 59 | 99.2 | 1.10E-26 |
| 1301 | 560 | PHE0001381_1473 | K-box | 75 | 172 | 79.2 | 1.20E-20 |
| 754 | 13 | PHE0001382_1474 | GRAS | 162 | 502 | 266.8 | 3.80E-77 |
| 950 | 209 | PHE0001385_1477 | Cation_efflux | I | 415 | 239.1 | 8.30E-69 |
| 951 | 210 | PHE0001386_1478 | Peptidase_M20 | 107 | 424 | 213.2 | 5.10E-61 |
| 951 | 210 | PHE0001386_1478 | M20 dimer | 214 | 318 | 30.6 | 4.90E-06 |
| 952 | 211 | PHE0001389_1481 | CN_hydrolase | 42 | 219 | 182.1 | 1.20E-51 |
| 953 | 212 | PHE0001392_1484 | Pkinase_Tyr | 89 | 373 | 133.9 | 3.80E-37 |
| 953 | 212 | PHE0001392_1484 | Pkinase | 96 | 373 | 170.8 | 3.10E-48 |
| 954 | 213 | PHE0001394_1486 | SRF-TF | 9 | 59 | 116.9 | 5.10E-32 |

(continued)

| PEP SEQ ID NO | NUC SEQ ID NO | GENE ID | Pfam domain name | begin | stop | score | E-value |
|---|---|---|---|---|---|---|---|
| 954 | 213 | PHE0001394_1486 | K-box | 71 | 174 | 89 | 1.30E-23 |
| 955 | 214 | PHE0001397_1489 | Ran BP1 | 37 | 158 | 159 | 1.10E-44 |
| 956 | 215 | PHE0001398_1490 | Ran BP1 | 39 | 160 | 185.6 | 1.10E-52 |
| 957 | 216 | PHE0001405_1497 | NifU_N | 33 | 160 | 273.5 | 3.80E-79 |
| 958 | 217 | PHE0001411_1503 | PSI_PsaF | 49 | 227 | 452.8 | 3.90E-133 |
| 959 | 218 | PHE0001416_1508 | PA | 70 | 171 | 52.7 | 1.10E-12 |
| 959 | 218 | PHE0001416_1508 | Peptidase_A22B | 238 | 524 | 429.6 | 3.80E-126 |
| 960 | 219 | PHE0001417_1509 | NAC | 35 | 92 | 94.3 | 3.20E-25 |
| 962 | 221 | PHE0001421_1513 | ELFV_dehydrog_N | 31 | 161 | 201.2 | 2.10E-57 |
| 962 | 221 | PHE0001421_1513 | ELFV_dehydrog | 176 | 408 | 391.9 | 8.40E-115 |
| 963 | 222 | PHE0001426_1518 | GATA | 91 | 126 | 65.1 | 2.00E-16 |
| 964 | 223 | PHE0001436_1528 | Meth_synt_1 | 2 | 319 | 594.5 | 8.70E-176 |
| 964 | 223 | PHE0001436_1528 | Meth_synt_2 | 433 | 757 | 753.8 | 9.50E-224 |
| 966 | 225 | PHE0001442_1534 | Ubie_methyltran | 40 | 256 | -109 | 0.00017 |
| 966 | 225 | PHE0001442_1534 | CMAS | 42 | 295 | -155.1 | 0.00012 |
| 966 | 225 | PHE0001442_1534 | Methyltransf_11 | 105 | 203 | 105.4 | 1.50E-28 |
| 966 | 225 | PHE0001442_1534 | Methyltransf_12 | 105 | 201 | 49.4 | 1.10E-11 |
| 968 | 227 | PHE0001452_1544 | ADH_N | 30 | 111 | 82.8 | 9.30E-22 |
| 968 | 227 | PHE0001452_1544 | ADH_zinc_N | 142 | 283 | 134 | 3.70E-37 |
| 969 | 228 | PHE0001463_1555 | Homeobox | 18 | 74 | 86.2 | 9.20E-23 |
| 969 | 228 | PHE0001463_1555 | START | 232 | 456 | 280 | 4.20E-81 |
| 805 | 64 | PHE0001471_1563 | Ammonium_transp | 36 | 460 | 578.1 | 7.80E-171 |
| 970 | 229 | PHE0001477_1569 | Myb_DNA-binding | 15 | 62 | 49.1 | 1.30E-11 |
| 970 | 229 | PHE0001477_1569 | Myb_DNA-binding | 68 | 113 | 50.6 | 4.70E-12 |
| 971 | 230 | PHE0001481_1573 | GAF | 195 | 339 | 35.3 | 1.90E-07 |
| 971 | 230 | PHE0001481_1573 | HisKA | 375 | 440 | 36.4 | 8.90E-08 |
| 971 | 230 | PHE0001481_1573 | Response_reg | 641 | 762 | 14.8 | 2.80E-05 |
| 972 | 231 | PHE0001483_1575 | Glyco_hydro_16 | 29 | 210 | 416.9 | 2.60E-122 |
| 972 | 231 | PHE0001483_1575 | XET_C | 234 | 284 | 99.8 | 7.30E-27 |
| 973 | 232 | PHE0001516_1607 | SelR | 87 | 209 | 274.8 | 1.50E-79 |

(continued)

| PEP SEQ ID NO | NUC SEQ ID NO | GENE ID | Pfam domain name | begin | stop | score | E-value |
|---|---|---|---|---|---|---|---|
| 1367 | 626 | PHE0001519_1610 | WD40 | 9 | 47 | 29.3 | 1.20E-05 |
| 1367 | 626 | PHE0001519_1610 | WD40 | 52 | 89 | 34.3 | 3.70E-07 |
| 1367 | 626 | PHE0001519_1610 | WD40 | 94 | 131 | 49.1 | 1.30E-11 |
| 1367 | 626 | PHE0001519_1610 | WD40 | 136 | 173 | 53 | 9.00E-13 |
| 1367 | 626 | PHE0001519_1610 | WD40 | 178 | 215 | 48.8 | 1.60E-11 |
| 1367 | 626 | PHE0001519_1610 | WD40 | 220 | 256 | 8.1 | 2.7 |
| 1302 | 561 | PHE0001521_1612 | Xan_ur_ permease | 36 | 443 | 195.6 | 1.00E-55 |
| 974 | 233 | PHE0001522_1613 | Xan_ur_ permease | 52 | 463 | 201.3 | 2.10E-57 |
| 1368 | 627 | PHE0001523_1614 | Xan_ur_ permease | 31 | 438 | 183.8 | 3.70E-52 |
| 1369 | 628 | PHE0001527_1618 | ELFV_ dehydrog_N | 44 | 174 | 245.7 | 8.50E-71 |
| 1369 | 628 | PHE0001527_1618 | ELFV_ dehydrog | 190 | 423 | 397.4 | 1.80E-116 |
| 1370 | 629 | PHE00.01528_1619 | ELFV_ dehydrog_N | 47 | 177 | 253.2 | 4.80E-73 |
| 1370 | 629 | PHE0001528_1619 | ELFV_ dehydrog | 193 | 426 | 385.5 | 7.00E-113 |
| 806 | 65 | PHE0001530_1621 | ELFV_ dehydrog_N | 43 | 173 | 299.9 | 4.10E-87 |
| 806 | 65 | PHE0001530_1621 | ELFV_ dehydrog | 188 | 456 | 446 | 4.50E-131 |
| 755 | 14 | PHE0001531_1622 | ELFV_ dehydrog_N | 45 | 178 | 267.3 | 2.70E-77 |
| 755 | 14 | PHE0001531_1622 | ELFV _dehydrog | 194 | 425 | 426.5 | 3.20E-125 |
| 756 | 15 | PHE0001532_1623 | ELFV_ dehydrog_N | 39 | 169 | 250.4 | 3.40E-72 |
| 756 | 15 | PHE0001532_1623 | ELFV_ dehydrog | 184 | 451 | 456.3 | 3.40E-134 |
| 1371 | 630 | PHE0001533_1624 | ELFV_ dehydrog_N | 73 | 203 | 271.9 | 1.20E-78 |
| 1371 | 630 | PHE0001533_1624 | ELFV_ dehydrog | 219 | 462 | 480.3 | 2.00E-141 |
| 757 | 16 | PHE0001535_1626 | ELFV_ dehydrog_N | 55 | 185 | 277.2 | 3.00E-80 |
| 757 | 16 | PHE0001535_1626 | ELFV_ dehydrog | 200 | 443 | 486.1 | 3.80E-143 |
| 758 | 17 | PHE0001536_1627 | ELFV_ dehydrog_N | 67 | 197 | 286.3 | 5.10E-83 |

(continued)

| PEP SEQ ID NO | NUC SEQ ID NO | GENE ID | Pfam domain name | begin | stop | score | E-value |
|---|---|---|---|---|---|---|---|
| 758 | 17 | PHE0001536_1627 | ELFV_ dehydrog | 213 | 456 | 481.2 | 1.10E-141 |
| 759 | 18 | PHE0001538_1629 | ELFV_ dehydrog_N | 33 | 163 | 239.1 | 8.20E-69 |
| 759 | 18 | PHE0001538_1629 | ELFV_ dehydrog | 179 | 420 | 400.7 | 1.90E-117 |
| 975 | 234 | PHE0001539_1630 | ELFV_ dehydrog_N | 229 | 359 | 216.8 | 4.40E-62 |
| 975 | 234 | PHE0001539_1630 | ELFV_ dehydrog | 375 | 622 | 247.2 | 3.10E-71 |
| 1372 | 631 | PHE0001541_1632 | ELFV_ dehydrog_N | 245 | 375 | 242.3 | 8.90E-70 |
| 1372 | 631 | PHE0001541_1632 | ELFV_ dehydrog | 391 | 638 | 255.7 | 8.70E-74 |
| 1206 | 465 | PHE0001564_1663 | GATase_2 | 2 | 165 | 47 | 1.30E-14 |
| 1206 | 465 | PHE0001564_1663 | Asn_synthase | 214 | 458 | 366.1 | 4.80E-107 |
| 1373 | 632 | PHE0001567_1666 | GATase_2 | 2 | 164 | -6.7 | 1.50E-10 |
| 1373 | 632 | PHE000156_1666 | Asn_synthase | 241 | 531 | 383.2 | 3.40E-112 |
| 1374 | 633 | PHE0001568_1667 | GATase_2 | 2 | 166 | -20.1 | 1.60E-09 |
| 1374 | 633 | PHE0001568_1667 | Asn_synthase | 249 | 553 | 422.6 | 4.80E-124 |
| 976 | 235 | PHE0001577_1676 | CBS | 48 | 241 | 36.5 | 8.40E-08 |
| 976 | 235 | PHE0001577_1676 | CBS | 264 | 402 | 88.1 | 2.40E-23 |
| 760 | 19 | PHE0001593_1699 | Transketolase_ N | 7 | 339 | 846.6 | 1.10E-251 |
| 760 | 19 | PHE0001593_1699 | Transket_pyr | 356 | 533 | 240 | 4.60E-69 |
| 760 | 19 | PHE0001593_1699 | Transketolase_ C | 546 | 656 | 73.3 | 6.90E-19 |
| 761 | 20 | PHE0001593_1700 | Transketolase_ N | 7 | 339 | 846.6 | 1.10E-251 |
| 761 | 20 | PHE0001593_1700 | Transket_pyr | 356 | 533 | 240 | 4.60E-69 |
| 761 | 20 | PHE0001593_1700 | Transketolase_ C | 546 | 656 | 73.3 | 6.90E-19 |
| 977 | 236 | PHE0001602_1713 | Ras | 8 | 179 | 262.7 | 6.60E-76 |
| 978 | 237 | PHE0001604_1715 | Ras | 8 | 179 | 266.7 | 4.10E-77 |
| 979 | 238 | PHE0001605_1716 | Ras | 10 | 181 | 268.9 | 8.80E-78 |
| 980 | 239 | PHE0001606_1717 | Ras | 12 | 183 | 262.9 | 5.60E-76 |
| 981 | 240 | PHE0001610_1721 | WD40 | 7 | 47 | 8.3 | 2.6 |
| 981 | 240 | PHE0001610_1721 | WD40 | 52 | 89 | 30.2 | 6.60E-06 |
| 981 | 240 | PHE0001610_1721 | WD40 | 93 | 130 | 25.8 | 0.00014 |
| 981 | 240 | PHE0001610_1721 | WD40 | 136 | 174 | 10.8 | 1.3 |

(continued)

| PEP SEQ ID NO | NUC SEQ ID NO | GENE ID | Pfam domain name | begin | stop | score | E-value |
|---|---|---|---|---|---|---|---|
| 981 | 240 | PHE0001610_1721 | WD40 | 179 | 214 | 14.7 | 0.3 |
| 981 | 240 | PHE0001610_1721 | WD40 | 219 | 256 | 3.5 | 10 |
| 981 | 240 | PHE0001610_1721 | WD40 | 261 | 298 | 42.6 | 1.20E-09 |
| 1375 | 634 | PHE0001611_1722 | WD40 | 1 | 38 | 12.3 | 0.83 |
| 1375 | 634 | PHE0001611_1722 | WD40 | 43 | 80 | 42.8 | 1.10E-09 |
| 1375 | 634 | PHE0001611_1722 | WD40 | 137 | 174 | 27.1 | 5.60E-05 |
| 1375 | 634 | PHE0001611_1722 | WD40 | 182 | 219 | 27.7 | 3.70E-05 |
| 1375 | 634 | PHE0001611_1722 | WD40 | 279 | 316 | 29.5 | 1.10E-05 |
| 982 | 241 | PHE0001617_1728 | Zip | 60 | 378 | 324.9 | 1.20E-94 |
| 983 | 242 | PHE0001624_1735 | CorA | 16 | 382 | 554.7 | 8.40E-164 |
| 1376 | 635 | PHE0001632_1743 | Na_Ca_ex | 53 | 215 | 62.9 | 9.20E-16 |
| 1376 | 635 | PHE0001632_1743 | Na_Ca_ex | 377 | 531 | 83.8 | 4.60E-22 |
| 985 | 244 | PHE0001642_1753 | zf-C2H2 | 35 | 57 | 17.2 | 0.052 |
| 985 | 244 | PHE0001642_1753 | zf-C2H2 | 84 | 106 | 17.3 | 0.051 |
| 986 | 245 | PHE0001649_1760 | zf-B_box | 2 | 47 | 59.1 | 1.30E-14 |
| 986 | 245 | PHE0001649_1760 | zf-B_box | 54 | 99 | 44.6 | 2.90E-10 |
| 987 | 246 | PHE0002017_2128 | zf-C3HC4 | 332 | 372 | 26.9 | 6.30E-05 |
| 988 | 247 | PHE0002023_2134 | zf-C3HC4 | 336 | 376 | 39.6 | 9.90E-09 |
| 989 | 248 | PHE0002024_2135 | zf-C3HC4 | 338 | 378 | 34.5 | 3.20E-07 |
| 990 | 249 | PHE0002027_2138 | Gln-synt_N | 17 | 98 | 132.2 | 1.20E-36 |
| 990 | 249 | PHE0002027_2138 | Gln-synt_C | 103 | 355 | 330.1 | 3.30E-96 |
| 1377 | 636 | PHE0002029_2140 | DUF246 | 113 | 455 | 712 | 3.70E-211 |
| 991 | 250 | PHE0002041_2151 | ubiquitin | 1 | 74 | 151.2 | 2.50E-42 |
| 991 | 250 | PHE0002041_2151 | Ribosomal_S27 | 101 | 148 | 112.4 | 1.20E-30 |
| 992 | 251 | PHE0002042_2152 | HSP20 | 48 | 152 | 180.1 | 4.80E-51 |
| 993 | 252 | PHE0002049_2159 | RuBisCO_small | 58 | 166 | 249.7 | 5.50E-72 |
| 994 | 253 | PHE0002052_2162 | Lig_chan | 1 | 234 | 179.4 | 7.80E-51 |
| 995 | 254 | PHE0002054_2164 | CN_hydrolase | 31 | 220 | 227.7 | 2.20E-65 |
| 996 | 255 | PHE0002055_2165 | Tic22 | 18 | 280 | 219.3 | 7.70E-63 |
| 997 | 256 | PHE0002067_2177 | Myb_DNA-binding | 14 | 61 | 47.5 | 4.00E-11 |
| 997 | 256 | PHE0002067_2177 | Myb_DNA-binding | 67 | 112 | 55.4 | 1.70E-13 |
| 998 | 257 | PHE0002068_2178 | Myb_DNA-binding | 14 | 61 | 47.5 | 4.00E-11 |
| 998 | 257 | PHE0002068_2178 | Myb_DNA-binding | 67 | 112 | 51.1 | 3.40E-12 |
| 762 | 21 | PHE0002070_2180 | Myb_DNA-binding | 14 | 61 | 47.2 | 5.00E-11 |

(continued)

| PEP SEQ ID NO | NUC SEQ ID NO | GENE ID | Pfam domain name | begin | stop | score | E-value |
|---|---|---|---|---|---|---|---|
| 762 | 21 | PHE0002070_2180 | Myb_DNA-binding | 67 | 112 | 51.9 | 1.90E-12 |
| 999 | 258 | PHE0002071_2181 | Myb DNA-binding | 14 | 61 | 44.3 | 3.60E-10 |
| 999 | 258 | PHE0002071_2181 | Myb_DNA-binding | 67 | 112 | 54 | 4.30E-13 |
| 1378 | 637 | PHE0002078_2188 | Sec Y | 108 | 493 | -75.3 | 1.10E-08 |
| 1305 | 564 | PHE0002079_2189 | Sec Y | 77 | 461 | -18.1 | 2.30E-11 |
| 1000 | 259 | PHE0002080_4290 | Sec Y | 77 | 461 | -19.2 | 2.60E-11 |
| 1001 | 260 | PHE0002081_ 2191 | Sec Y | 77 | 462 | -25.3 | 5.00E-11 |
| 1379 | 638 | PHE0002084_2194 | Ion trans | 123 | 389 | 69 | 1.30E-17 |
| 1379 | 638 | PHE0002084_2194 | cNMP_binding | 488 | 590 | 40.9 | 4.00E-09 |
| 1002 | 261 | PHE0002088_2198 | UPF0057 | 7 | 58 | 86.8 | 5.70E-23 |
| 1380 | 639 | PHE0002089_2199 | UPF0057 | 29 | 79 | 81.1 | 3.10E-21 |
| 1381 | 640 | PHE0002090_2200 | UPF0057 | 1 | 51 | 104.1 | 3.70E-28. |
| 1207 | 466 | PHE0002094_2204 | Pkinase | 78 | 352 | 113.2 | 6.60E-31 |
| 1207 | 466 | PHE0002094_2204 | Pkinase_Tyr | 78 | 352 | 96.1 | 9.60E-26 |
| 1003 | 262 | PHE0002095_2205 | efhand | 12 | 40 | 41 | 3.60E-09 |
| 1003 | 262 | PHE0002095_2205 | efhand | 48 | 76 | 38.9 | 1.60E-08 |
| 1003 | 262 | PHE0002095_2205 | efhand | 85 | 113 | 42.1 | 1.70E-09 |
| 1003 | 262 | PHE0002095_2205 | efhand | 121 | 149 | 43.2 | 8.10E-10 |
| 1004 | 263 | PHE0002096_2206 | efhand | 12 | 40 | 41 | 3.60E-09 |
| 1004 | 263 | PHE0002096_2206 | efhand | 48 | 76 | 38.9 | 1.60E-08 |
| 1004 | 263 | PHE0002096_2206 | efhand | 85 | 113 | 42.1 | 1.70E-09. |
| 1004 | 263 | PHE0002096_2206 | efhand | 121 | 149 | 43.2 | 8.10E-10 |
| 1005 | 264 | PHE0002099_2209 | efhand | 274 | 302 | 19.3 | 0.013 |
| 1006 | 265 | PHE0002103_2213 | Pkinase | 81 | 358 | 164.6 | 2.20E-46 |
| 1006 | 265 | PHE0002103_2213 | Pkinase_Tyr | 81 | 358 | 129.8 | 6.50E-36 |
| 1208 | 467 | PHE0002121_2229 | Myb_DNA-binding | 76 | 123 | 45.7 | 1.40E-10 |
| 1208 | 467 | PHE0002121_2229 | Myb_DNA-binding | 129 | 174 | 47.2 | 4.90E-11 |
| 1007 | 266 | PHE0002123_2231 | PEPcase | 11 | 966 | 2512.3 | 0 |
| 1008 | 267 | PHE0002125_2233 | PEPcase | 13 | 967 | 2535.1 | 0 |
| 1009 | 268 | PHE0002127_2235 | PEPcase | 11 | 967 | 2556.7 | 0 |
| 763 | 22 | PHE0002128_2236 | PEPcase | 1 | 883 | 767 | 1.00E-227 |
| 1011 | 270 | PHE0002148_2256 | p450 | 38 | 502 | 285.3 | 1.00E-82 |
| 1012 | 271 | PHE0002163_2270 | Yippee | 1 | 104 | 207.1 | 3.60E-59 |
| 1013 | 272 | PHE0002164_2271 | Yippee | 1 | 108 | 207.4 | 2.90E-59 |

(continued)

| PEP SEQ ID NO | NUC SEQ ID NO | GENE ID | Pfam domain name | begin | stop | score | E-value |
|---|---|---|---|---|---|---|---|
| 1014 | 273 | PHE0002183_2290 | HSP20 | 53 | 156 | 186.9 | 4.20E-53 |
| 1306 | 565 | PHE0002185_2292 | HSP20 | 46 | 149 | 183 | 6.50E-52 |
| 1015 | 274 | PHE0002189_2296 | AA_kinase | 15 | 260 | 199.6 | 6.60E-57 |
| 1383 | 642 | PHE0002194_2301 | Aldedh | 52 | 511 | 791.1 | 5.70E-235 |
| 1016 | 275 | PHE0002202_2309 | Annexin | 14 | 80 | 101.3 | 2.60E-27 |
| 1016 | 275 | PHE0002202_2309 | Annexin | 86 | 152 | 89.2 | 1.20E-23 |
| 1016 | 275 | PHE0002202_2309 | Annexin | 169 | 235 | 65.2 | 1.90E-16 |
| 1016 | 275 | PHE0002202_2309 | Annexin | 244 | 310 | 96.1 | 9.50E-26 |
| 1017 | 276 | PHE0002203_2310 | Annexin | 13 | 79 | 99.8 | 7.10E-27 |
| 1017 | 276 | PHE0002203_2310 | Annexin | 85 | 150 | 72.3 | 1.40E-18 |
| 1017 | 276 | PHE0002203_2310 | Annexin | 167 | 233 | 51.8 | 2.00E-12 |
| 1017 | 276 | PHE0002203_2310 | Annexin | 242 | 308 | 85.3 | 1.70E-22 |
| 1018 | 277 | PHE0002204_2311 | Annexin | 14 | 80 | 85.9 | 1.10E-22 |
| 1018 | 277 | PHE0002204_2311 | Annexin | 86 | 153 | 42.1 | 1.70E-09 |
| 1018 | 277 | PHE0002204_2311 | Annexin | 170 | 237 | 101.9 | 1.70E-27 |
| 1018 | 277 | PHE0002204_2311 | Annexin | 246 | 312 | 99.3 | 9.90E-27 |
| 1019 | 278 | PHE0002210_2317 | Kunitz legume | 33 | 199 | 96.7 | 6.00E-26 |
| 1020 | 279 | PHE0002224_2331 | Di19 | 18 | 236 | 151.1 | 2.60E-42 |
| 1210 | 469 | PHE0002226_2333 | Gamma-thionin | 32 | 80 | 77.6 | 3.50E-20 |
| 1021 | 280 | PHE0002227_2334 | Gamma-thionin | 32 | 78 | 77.9 | 2.90E-20 |
| 1307 | 566 | PHE0002229_2336 | Gamma-thionin | 34 | 80 | 100.7 | 3.90E-27 |
| 1022 | 281 | PHE0002232_2339 | bZIP_2 | 298 | 352 | 48.7 | 1.80E-11 |
| 1022 | 281 | PHE0002232_2339 | bZIP_1 | 298 | 360 | 39.5 | 1.00E-08 |
| 1023 | 282 | PHE0002233_2340 | bZIP_2 | 245 | 299 | 38.7 | 1.80E-08 |
| 1023 | 282 | PHE0002233_2340 | bZIP_1 | 249 | 306 | 39 | 1.40E-08 |
| 1024 | 283 | PHE0002238_2345 | Epimerase | 10 | 253 | 62 | 1.70E-15 |
| 1024 | 283 | PHE0002238_2345 | 3Beta_HSD | 11 | 277 | -51.4 | 2.80E-09 |
| 1024 | 283 | PHE0002238_2345 | NAD_binding_4 | 12 | 237 | 2.9 | 7.20E-09 |
| 1025 | 284 | PHE0002239_2346 | adh short | 6 | 148 | -26.5 | 0.00023 |
| 1025 | 284 | PHE0002239_2346 | Epimerase | 8 | 253 | 56.9 | 6.00E-14 |
| 1025 | 284 | PHE0002239_2346 | 3Beta_HSD | 9 | 279 | -27.1 | 4.40E-11 |
| 1025 | 284 | PHE0002239_2346 | NAD_binding_4 | 10 | 266 | -24.8 | 4.50E-07 |
| 1026 | 285 | PHE0002240_2347 | adh_short | 3 | 157 | -5.1 | 8.50E-06 |
| 1026 | 285 | PHE0002240_2347 | Epimerase | 5 | 250 | 75.3 | 1.70E-19 |
| 1026 | 285 | PHE0002240_2347 | Polysacc_synt_2 | 5 | 251 | -175.5 | 0.0049 |
| 1026 | 285 | PHE0002240_2347 | 3Beta_HSD | 6 | 276 | -39.5 | 3.70E-10 |

(continued)

| PEP SEQ ID NO | NUC SEQ ID NO | GENE ID | Pfam domain name | begin | stop | score | E-value |
|---|---|---|---|---|---|---|---|
| 1026 | 285 | PHE0002240_2347 | NAD_binding_4 | 7 | 263 | -23.5 | 3.70E-07 |
| 1027 | 286 | PHE0002242_2349 | Epimerase | 7 | 251 | 59 | 1.40E-14 |
| 1027 | 286 | PHE0002242_2349 | NmrA | 7 | 225 | -83.8 | 0.0022 |
| 1027 | 286 | PHE0002242_2349 | 3Beta_HSD | 8 | 273 | -22.7 | 2.10E-1 |
| 1027 | 286 | PHE0002242_2349 | NAD_binding_4 | 9 | 235 | -15.3 | 1.10E-07 |
| 1028 | 287 | PHE0002244_2351 | HSF_DNA-bind | 5 | 178 | 300.9 | 2.10E-87 |
| 1029 | 288 | PHE0002246 2353 | PP2C | 104 | 390 | 288.9 | 8.50E-84 |
| 1030 | 289 | PHE0002254_2361 | adh_short | 22 | 191 | 94.5 | 2.80E-25 |
| 764 | 23 | PHE0002258_69 | PAS | 116 | 230 | 22.8 | 0.0011 |
| 764 | 23 | PHE0002258 69 | PAS | 390 | 504 | 10.5 | 0.036 |
| 764 | 23 | PHE0002258_69 | Pkinase | 582 | 870 | 287.7 | 2.00E-83 |
| 765 | 24 | PHE0002259_68 | PAS | 116 | 230 | 22.8 | 0.0011 |
| 765 | 24 | PHE0002259_68 | PAS | 390 | 504 | 10.5 | 0.036 |
| 765 | 24 | PHE0002259_68 | Pkinase | 582 | 870 | 288.7 | 9.90E-84 |
| 766 | 25 | PHE0002260_70 | PAS | 116 | 230 | 22.8 | 0.0011 |
| 766 | 25 | PHE0002260_70 | PAS | 390 | 504 | 10.5 | 0.036 |
| 766 | 25 | PHE0002260_70 | Pkinase | 582 | 870 | 287.7 | 2.00E-83 |
| 1031 | 290 | PHE0002268_2371 | Pkinase | 30 | 288 | 384.1 | 1.90E-112 |
| 1031 | 290 | PHE0002268_2371 | efhand | 335 | 363 | 39.3 | 1.10E-08 |
| 1031 | 290 | PHE0002268 2371 | efhand | 371 | 399 | 29.9 | 7.90E-06 |
| 1031 | 290 | PHE0002268_2371 | efhand | 407 | 435 | 31.6 | 2.40E-06 |
| 1031 | 290 | PHE0002268_2371 | efhand | 441 | 469 | 33.1 | 8.90E-07 |
| 1032 | 291 | PHE0002269 2372 | Pkinase | 79 | 337 | 343.7 | 2.80E-100 |
| 1032 | 291 | PHE0002269_2372 | efhand | 384 | 412 | 28 | 2.90E-05 |
| 1032 | 291 | PHE0002269_2372 | efhand | 456 | 484 | 23.8 | 0.00056 |
| 1032 | 291 | PHE0002269 2372 | efhand | 490 | 518 | 36.7 | 7.20E-08 |
| 1033 | 292 | PHE0002272_2375 | Pkinase | 73 | 331 | 321.7 | 1.10E-93 |
| 1033 | 292 | PHE0002272_2375 | efhand | 448 | 476 | 25.2 | 0.00021 |
| 1033 | 292 | PHE0002272_2375 | efhand | 482 | 510 | 36 | 1.10E-07 |
| 1211 | 470 | PHE0002293_2395 | Inhibitor_129 | 36 | 92 | 87.7 | 3.10E-23 |
| 1211 | 470 | PHE0002293 2395 | Peptidase_C1 | 119 | 343 | 408 | 1.20E-119 |
| 1034 | 293 | PHE0002298_2400 | Dehydrin | 21 | 286 | 140 | 5.80E-39 |
| 1035 | 294 | PHE0002299_2401 | Ribonuclease_T2 | 32 | 218 | 96.1 | 9.70E-26 |
| 1036 | 295 | PHE0002321_2422 | Di19 | 9 | 182 | 62.8 | 9.80E-16 |
| 1037 | 296 | PHE0002331_2432 | MIP | 29 | 264 | 432.6 | 4.90E-127 |
| 1212 | 471 | PHE0002338_2439 | MIP | 16 | 237 | 232.5 | 8.10E-67 |

(continued)

| PEP SEQ ID NO | NUC SEQ ID NO | GENE ID | Pfam domain name | begin | stop | score | E-value |
|---|---|---|---|---|---|---|---|
| 1038 | 297 | PHE0002340_2441 | MIP | 31 | 263 | 445.8 | 5.20E-131 |
| 1039 | 298 | PHE0002354_2455 | GST_N | 4 | 77 | 102.5 | 1.10E-27 |
| 1039 | 298 | PHE0002354_2455 | GST_C | 93 | 201 | 107.2 | 4.20E-29 |
| 1041 | 300 | PHE0002361_2462 | PARP | 252 | 445 | -1.5 | 5.00E-07 |
| 1042 | 301 | PHE0002362 2463 | Glycos_transf_2 | 37 | 234 | 90.2 | 5.70E-24 |
| 1043 | 302 | PHE0002364_2465 | Pyr_redox_2 | 23 | 339 | 224.3 | 2.40E-64 |
| 1043 | 302 | PHE0002364_2465 | Pyr_redox | 201 | 293 | 114.9 | 2.10E-31 |
| 1043 | 302 | PHE0002364_ 2465 | Pyr_redox_dim | 369 | 479 | 170.2 | 4.70E-48 |
| 1044 | 303 | PHE0002365_2466 | HI0933_like | 37 | 358 | -252.7 | 0.0036 |
| 1044 | 303 | PHE0002365_2466 | DAO | 38 | 437 | -32 | 0.0011 |
| 1044 | 303 | PHE0002365_2466 | GIDA | 38 | 362 | -203.5 | 9.40E-05 |
| 1044 | 303 | PHE0002365_2466 | Pyr_redox_2 | 38 | 352 | 244.9 | 1.50E-70 |
| 1044 | 303 | PHE0002365_2466 | Pyr_redox | 209 | 306 | 124.1 | 3.40E-34 |
| 1044 | 303 | PHE0002365_2466 | Pyr_redox_dim | 381 | 490 | 206.4 | 5.80E-59 |
| 1045 | 304 | PHE0002367_2468 | Pyr_redox_2 | 24 | 340 | 235.9 | 8.00E-68 |
| 1045 | 304 | PHE0002367 2468 | Pyr_redox | 202 | 294 | 108.1 | 2.30E-29 |
| 1045 | 304 | PHE0002367_2468 | Pyr_redox_dim | 370 | 480 | 167.2 | 3.60E-47 |
| 1046 | 305 | PHE0002370_2471 | GSHPx | 8 | 117 | 248.2 | 1.50E-71 |
| 1047 | 306 | PHE0002372_2473 | GSHPx | 9 | 118 | 241 | 2.20E-69 |
| 1048 | 307 | PHE0002373_2474 | GSHPx | 11 | 120 | 216.8 | 4.40E-62 |
| 1049 | 308 | PHE0002383_2484 | Cpn60_TCP1 | 40 | 534 | 618.6 | 4.70E-183 |
| 1050 | 309 | PHE0002390_2491 | PMSR | 28 | 181 | 264.3 | 2.30E-76 |
| 1308 | 567 | PHE0002392_2493 | HMA | 6 | 67 | 62.5 | 1.20E-15 |
| 1309 | 568 | PHE0002410_2510 | Pyridoxal_deC | 33 | 383 | 596.4 | 2.30E-176 |
| 1310 | 569 | PHE0002411_2511 | Pyridoxal_deC | 37 | 383 | 354.1 | 2.00E-103 |
| 1051 | 310 | PHE0002414_2514 | Pyridoxal_deC | 33 | 380 | 519.7 | 2.80E-153 |
| 1052 | 311 | PHE0002431_2531 | Rubrerythrin | 133 | 266 | 200.1 | 4.70E-57 |
| 1053 | 312 | PHE0002447_2547 | HSF_DNA-bind | 72 | 234 | 213.5 | 4.20E-61 |
| 1054 | 313 | PHE0002449_2549 | HSF_DNA-bind | 22 | 218 | 162.4 | 1.00E-45 |
| 1055 | 314 | PHE0002459_2559 | F-box | 12 | 57 | 37.3 | 4.60E-08 |
| 1056 | 315 | PHE0002461_2561 | HSF_DNA-bind | 19 | 195 | 351 | 1.70E-102 |
| 1057 | 316 | PHE0002462_2562 | HSF_DNA-bind | 8 | 206 | 179.6 | 6.70E-51 |
| 1058 | 317 | PHE0002464_2564 | HSF_DNA-bind | 9 | 193 | 187.1 | 3.90E-53 |
| 1059 | 318 | PHE0002470_2570 | HSP20 | 50 | 153 | 169.4 | 8.10E-48 |
| 1060 | 319 | PHE0002471_2571 | HSP20 | 49 | 152 | 177.8 | 2.40E-50 |
| 1384 | 643 | PHE0002483_2583 | F-box | 27 | 72 | 22.8 | 0.0011 |

(continued)

| PEP SEQ ID NO | NUC SEQ ID NO | GENE ID | Pfam domain name | begin | stop | score | E-value |
|---|---|---|---|---|---|---|---|
| 1384 | 643 | PHE0002483_2583 | LRR 2 | 182 | 206 | 14.2 | 0.16 |
| 1061 | 320 | PHE0002484_2584 | Sucrose_synth | 6 | 551 | 1368.2 | 0 |
| 1061 | 320 | PHE0002484_2584 | Glycos_transf_1 | 554 | 743 | 100.9 | 3.30E-27 |
| 1311 | 570 | PHE0002485_2585 | Aa_trans | 39 | 424 | 234.8 | 1.70E-67 |
| 1062 | 321 | PHE0002486_2586 | B56 | 64 | 476 | 999.5 | 1.10E-297 |
| 1063 | 322 | PHE0002489_2589 | Cyclin_N | 65 | 197 | 153.6 | 4.80E-43 |
| 1063 | 322 | PHE0002489_2589 | Cyclin_C | 199 | 318 | 33.6 | 6.30E-07 |
| 1064 | 323 | PHE0002490_2590 | Cyclin_N | 61 | 187 | 140.9 | 3.20E-39 |
| 1064 | 323 | PHE0002490_2590 | Cyclin_C | 189 | 312 | 83.5 | 5.70E-22 |
| 1065 | 324 | PHE0002490_3963 | Cyclin_N | 42 | 168 | 140.9 | 3.20E-39 |
| 1065 | 324 | PHE0002490_3963 | Cyclin_C | 170 | 293 | 83.5 | 5.70E-22 |
| 1066 | 325 | PHE0002491_2591 | Cyclin_N | 168 | 293 | 225.4 | 1.10E-64 |
| 1066 | 325 | PHE0002491_2591 | Cyclin_C | 295 | 422 | 168.5 | 1.50E-47 |
| 1385 | 644 | PHE0002494_2594 | Cu-oxidase_3 | 43 | 160 | 228.5 | 1.30E-65 |
| 1385 | 644 | PHE0002494_2594 | Cu-oxidase | 169 | 337 | 251 | 2.10E-72 |
| 1385 | 644 | PHE0002494_2594 | Cu-oxidase_2 | 426 | 564 | 167 | 4.10E-47 |
| 1312 | 571 | PHE0002496_2596 | Aminotran_3 | 83 | 409 | 249.7 | 5.50E-72 |
| 1067 | 326 | PHE0002498_2598 | Pkinase | 139 | 412 | 110.1 | 5.80E-30 |
| 1067 | 326 | PHE0002498_2598 | Pkinase Tyr | 139 | 412 | 135.1 | 1.70E-37 |
| 1068 | 327 | PHE0002302_2602 | Cpn10 | 54 | 145 | 139.7 | 7.10E-39 |
| 1068 | 327 | PHE0002502_2602 | Cpn10 | 152 | 245 | 150.7 | 3.30E-42 |
| 1069 | 328 | PHE0002506_2606 | Reticulon | 81 | 264 | 295.7 | 7.80E-86 |
| 1070 | 329 | PHE0002507_2607 | Myb_DNA-binding | 49 | 100 | 47.5 | 4.10E-11 |
| 1386 | 645 | PHE0002509_2609 | HSP20 | 46 | 156 | 135.2 | 1.60E-37 |
| 1071 | 330 | PHC0002514_2614 | E1_dh | 150 | 450 | 428.7 | 7.30E-126 |
| 1072 | 331 | PHE0002515_2615 | Hydrolase | 15 | 233 | 45.9 | 1.20E-10 |
| 1387 | 646 | PHE0002523_2623 | Thioredoxin | 104 | 206 | 6.4 | 4.00E-06 |
| 1213 | 472 | PHE0002524_2624 | Thioredoxin | 88 | 195 | 24.7 | 7.30E-08 |
| 1388 | 647 | PHE0002527_2627 | cobW | 11 | 187 | 308.1 | 1.50E-89 |
| 1388 | 647 | PHE0002527_2627 | CobW_C | 228 | 322 | 144.8 | 2.10E-40 |
| 1073 | 332 | PHE0002530_2630 | zf-Dof | 40 | 102 | 137.4 | 3.40E-38 |
| 1074 | 333 | PHE0002532_2632 | Ribosomal_L1 | 9 | 211 | 224.6 | 1.90E-64 |
| 1075 | 334 | PHE0002536_2636 | B3 | 146 | 251 | 105.3 | 1.60E-28 |
| 1075 | 334 | PHE0002536_2636 | Auxin_resp | 273 | 354 | 186.6 | 5.50E-53 |
| 1076 | 335 | PHE0002540_2640 | B3 | 123 | 228 | 108.8 | 1.40E-29 |
| 1076 | 335 | PHE0002540_2640 | Auxin_resp | 250 | 331 | 194 | 3.10E-55 |

(continued)

| PEP SEQ ID NO | NUC SEQ ID NO | GENE ID | Pfam domain name | begin | stop | score | E-value |
|---|---|---|---|---|---|---|---|
| 1076 | 335 | PHE0002540_2640 | AUX_IAA | 593 | 779 | -73.6 | 0.0003 |
| 1389 | 648 | PHB0002546_2646 | DSPc | 36 | 131 | 9.7 | 1.70E-05 |
| 1077 | 336 | PHE0002547_2647 | Abhydrolase_1 | 152 | 377 | 42.4 | 1.40E-09 |
| 1079 | 338 | PHE0002552_2652 | ComA | 20 | 289 | 347 | 2.90E-101 |
| 1390 | 649 | PHE0002557_2657 | AP2 | 15 | 79 | 144.1 | 3.40E-40 |
| 1080 | 339 | PHE0002565_2664 | Metallothio_2 | 2 | 82 | 77.3 | 4.40E-20 |
| 1391 | 650 | PHE0002566_2665 | Ribosomal_L39 | 9 | 51 | 100.6 | 4.20E-27 |
| 1081 | 340 | PHE0002581_2680 | Cpn60_TCP1 | 57 | 561 | 638.5 | 4.90E-189 |
| 1082 | 341 | PHE0002582_2681 | Cpn60_TCP1 | 56 | 559 | 617.9 | 8.00E-183 |
| 1083 | 342 | PHE0002583_2682 | Cpn60_TCP1 | 78 | 582 | 643.9 | 1.20E-190 |
| 1084 | 343 | PHE0002586_2685 | Cpn60_TCP1 | 66 | 570 | 637.4 | 1.10E-188 |
| 1085 | 344 | PHE0002588_2687 | Usp | 6 | 161 | 79 | 1.40E-20 |
| 1086 | 345 | PHE0002591_2690 | DUF393 | 65 | 183 | 109.6 | 8.00E-30 |
| 1087 | 346 | PHE0002596_2695 | Myb_DNA-binding | 59 | 104 | 53.9 | 4.70E-13 |
| 1088 | 347 | PHE0002604_2703 | NPH3 | 213 | 497 | 406 | 4.90E-119 |
| 1215 | 474 | PHE0002607_2706 | Sigma70_r2 | 283 | 356 | 51.5 | 2.50E-12 |
| 1215 | 474 | PHE0002607_2706 | Sigma70_r3 | 359 | 440 | 74.8 | 2.50E-19 |
| 1215 | 474 | PHE0002607_2706 | Sigma70_r4 | 452 | 505 | 75.7 | 1.30E-19 |
| 1089 | 348 | PHE0002608_2707 | Sigma70_r1_2 | 257 | 293 | 34.3 | 3.70E-07 |
| 1089 | 348 | PHE0002608_2707 | Sigma70_r2 | 336 | 406 | 84.7 | 2.60E-22 |
| 1089 | 348 | PHE0002608_2707 | Sigma70_r3 | 410 | 492 | 100.9 | 3.50E-27 |
| 1089 | 348 | PHE0002608_2707 | Sigma70_r4 | 504 | 557 | 90.6 | 4.30E-24 |
| 1216 | 475 | PHE0002609_2708 | Sigma70_r2 | 263 | 333 | 75.4 | 1.50E-19 |
| 1216 | 475 | PHE0002609_2708 | Sigma70_r3 | 337 | 418 | 61.5 | 2.40E-15 |
| 1216 | 475 | PHE0002609_2708 | Sigma70_r4 | 436 | 488 | 41.9 | 2.00E-09 |
| 1313 | 572 | PHE0002611_2710 | Sigma70_r1_2 | 253 | 289 | 29 | 1.50E-05 |
| 1313 | 572 | PHE0002611_2710 | Sigma70_r2 | 332 | 402 | 84.3 | 3.40E-22 |
| 1313 | 572 | PHE0002611_2710 | Sigma70_r3 | 406 | 488 | 96.5 | 7.20E-26 |
| 1313 | 572 | PHE0002611_2710 | Sigma70_r4 | 500 | 553 | 97 | 5.00E-26 |
| 1392 | 651 | PHE0002614_2713 | VQ | 45 | 75 | 43.6 | 5.80E-10 |
| 1090 | 349 | PHE0002615_2714 | VQ | 38 | 68 | 49.3 | 1.10E-11 |
| 1314 | 573 | PHE0002616_2723 | Aa trans | 26 | 422 | 391.8 | 9.30E-115 |
| 1091 | 350 | PHE0002622_2739 | Myb_DNA-binding | 155 | 205 | 44.2 | 3.90E-10 |
| 1217 | 476 | PHE0002625_2744 | Glycos_transf_1 | 382 | 561 | 50.5 | 5.10E-12 |

(continued)

| PEP SEQ ID NO | NUC SEQ ID NO | GENE ID | Pfam domain name | begin | stop | score | E-value |
|---|---|---|---|---|---|---|---|
| 1092 | 351 | PHE0002629_2748 | Glyco_transf_20 | 77 | 562 | 782.7 | 1.90E-232 |
| 1092 | 351 | PHE0002629_2748 | Trehalose_PPase | 611 | 846 | 311.9 | 1.00E-90 |
| 1093 | 352 | PHE0002634_2753 | Hydrolase | 15 | 204 | 105.6 | 1.30E-28 |
| 1094 | 353 | PHE0002639_2758 | GH3 | 19 | 576 | 880.4 | 7.40E-262 |
| 1095 | 354 | PHE0002640_2759 | GH3 | 8 | 565 | 952 | 2.10E-283 |
| 1096 | 355 | PHE0002643_2762 | GH3 | 12 | 572 | 1048.2 | 0 |
| 1097 | 356 | PHE0002644_2763 | GH3 | 22 | 574 | 1140.9 | 0 |
| 767 | 26 | PHE0002645_2764 | GH3 | 23 | 585 | 1378.6 | 0 |
| 1393 | 652 | PHE0002648_2767 | SRF-TF | 9 | 59 | 109.1 | 1.10E-29 |
| 1393 | 652 | PHE0002648_2767 | K-box | 69 | 169 | 86.4 | 7.90E-23 |
| 1098 | 357 | PHE0002649_2768 | SRF-TF | 9 | 59 | 105.2 | 1.70E-28 |
| 1098 | 357 | PHE0002649_2768 | K-box | 73 | 172 | 120.3 | 5.00E-33 |
| 1394 | 653 | PHE0002652_2771 | NTP_transferase | 9 | 231 | 52.2 | 1.40E-13 |
| 1394 | 653 | PHE0002652_2771 | Hexapep | 294 | 311 | 5.4 | 28 |
| 1394 | 653 | PHE0002652_2771 | Hexapep | 312 | 329 | 15.4 | 0.18 |
| 1394 | 653 | PHE0002652_2771 | Hexapep | 335 | 352 | 13 | 1 |
| 1099 | 358 | PHE0002661_2781 | NTP_transferase | 5 | 275 | 397.9 | 1.40E-116 |
| 1100 | 359 | PHE0002664_2787 | NTP_transferase | 50 | 325 | 377.3 | 2.10E-110 |
| 1315 | 574 | PHE0002670_2793 | NTP_transferase | 3 | 275 | 317 | 3.10E-92 |
| 1101 | 360 | PHE0002688_2821 | Tubulin | 45 | 244 | 380.4 | 2.50E-111 |
| 1101 | 360 | PHE0002688_2821 | Tubulin_C | 246 | 383 | 267.9 | 1.80E-77 |
| 1102 | 361 | PHE0002689_2822 | Tubulin | 49 | 246 | 341.7 | 1.10E-99 |
| 1102 | 361 | PHE0002689_2822 | Tubulin_C | 248 | 393 | 268.1 | 1.60E-77 |
| 1103 | 362 | PHE0002690_2823 | Pkinase | 185 | 469 | 303 | 4.80E-88 |
| 1105 | 364 | PHE0002710_2843 | Ldh_1_N | 6 | 155 | 152.8 | 8.30E-43 |
| 1105 | 364 | PHE0002710_2843 | Ldh_1_C | 157 | 331 | 160.5 | 3.80E-45 |
| 1106 | 365 | PHE0002715_2848 | Thiolase_N | 48 | 306 | 392.7 | 4.90E-115 |
| 1106 | 365 | PHE0002715_2848 | Thiolase_C | 313 | 437 | 235.9 | 7.60E-68 |
| 1107 | 366 | PHE0002717_2850 | EF1_GNE | 140 | 229 | 183.2 | 5.60E-52 |
| 1108 | 367 | PHE0002721_2854 | Glycolytic | 11 | 355 | 936.1 | 1.30E-278 |
| 1109 | 368 | PHE0002724_2859 | Ribosomal_L19e | 2 | 149 | 320.1 | 3.50E-93 |
| 1316 | 575 | PHE0002727_2860 | Sucrose synth | 11 | 559 | 1379.9 | 0 |

69

(continued)

| PEP SEQ ID NO | NUC SEQ ID NO | GENE ID | Pfam domain name | begin | stop | score | E-value |
|---|---|---|---|---|---|---|---|
| 1316 | 575 | PHE0002727_2860 | Glycos_transf_1 | 562 | 748 | 111.4 | 2.40E-30 |
| 1110 | 369 | PHE0002728_2861 | MFS_1 | 53 | 486 | 33 | 9.40E-07 |
| 1111 | 370 | PHE0002729_2863 | Ribosomal L10e | 1 | 176 | 473.5 | 2.30E-139 |
| 1112 | 371 | PHE0002733_2866 | Ribosomal_S17 | 74 | 143 | 147.1 | 4.20E-41 |
| 1113 | 372 | PHE0002734_2867 | Ribosomal_L7Ae | 25 | 120 | 104.7 | 2.50E-28 |
| 1114 | 373 | PHE0002749_2882 | Suc Fer-like | 55 | 269 | 353.9 | 2.30E-103 |
| 1115 | 374 | PHE0002751_2884 | Ribosomal_S11 | 28 | 146 | 251.9 | 1.20E-72 |
| 1116 | 375 | PHE0002752_2885 | Ribosomal_S2 | 36 | 202 | 314.6 | 1.60E-91 |
| 1117 | 376 | PHE0002771_2904 | Dirigent | 3 | 152 | 37.5 | 3.10E-09 |
| 1117 | 376 | PHE0002771_2904 | Jacalin | 171 | 305 | 116.9 | 5.20E-32 |
| 1118 | 377 | PHE0002790_2925 | p450 | 77 | 520 | 236.7 | 4.40E-68 |
| 1395 | 654 | PHE0002791_2926 | p450 | 73 | 514 | 228.6 | 1.20E-65 |
| 1119 | 378 | PHE0002846_2981 | Trehalose_PPase | 119 | 352 | 316.7 | 3.60E-92 |
| 1396 | 655 | PHE0002849_2984 | UDPGT | 20 | 493 | -63 | 1.70E-07 |
| 1397 | 656 | PHE0002855_2990 | ABCl | 161 | 279 | 196.7 | 4.80E-56 |
| 1398 | 657 | PHE0002856_2991 | ABCl | 165 | 283 | 190 | 4.90E-54 |
| 1120 | 379 | PHE0002864_2999 | Pkinase | 4 | 287 | 402 | 7.70E-118 |
| 1121 | 380 | PHE0002869_3004 | Pkinase | 23 | 304 | 338.1 | 1.40E-98 |
| 1122 | 381 | PHE0002875_3010 | DUF581 | 94 | 148 | 82.7 | 1.00E-21 |
| 1399 | 658 | PHE0002886_3021 | DSPc | 30 | 168 | 155.8 | 1.00E-43 |
| 1400 | 659 | PHE0002888_3023 | DSPc | 46 | 179 | 113.5 | 5.40E-31 |
| 1123 | 382 | PHE0002889_3024 | DSPc | 23 | 161 | 152.4 | 1.00E-42 |
| 1401 | 660 | PHE0002893_3028 | DSPc | 159 | 301 | 90.6 | 4.20E-24 |
| 1124 | 383 | PHE0002896_3031 | DSPc | 46 | 184 | 172.7 | 8.00E-49 |
| 1402 | 661 | PHE0002901_3036 | GATA | 472 | 507 | 71.1 | 3.20E-18 |
| 1403 | 662 | PHE0002906_3041 | GATA | 163 | 198 | 62.4 | 1.30E-15 |
| 1404 | 663 | PHE0002910_3045 | Pkinase | 173 | 526 | 105.9 | 1.00E-28 |
| 1125 | 384 | PHE0002918_3053 | Sugar_tr | 65 | 500 | 316.2 | 5.20E-92 |
| 1125 | 384 | PHE0002918_3053 | MFS_1 | 69 | 460 | 62.3 | 1.40E-15 |
| 1405 | 664 | PHE0002923_3058 | VHS | 14 | 161 | 264.6 | 1.70E-76 |
| 1405 | 664 | PHE0002923_3058 | GAT | 231 | 325 | -6 | 0.0035 |
| 1405 | 664 | PHE0002923_3058 | Alpha_adaptinC2 | 437 | 555 | 154.6 | 2.30E-43 |
| 1406 | 665 | PHE0002928_3065 | JmjN | 11 | 63 | 106.6 | 6.40E-29 |
| 1406 | 665 | PHE0002928_3065 | JmjC | 191 | 307 | 184.6 | 2.20E-52 |

(continued)

| PEP SEQ ID NO | NUC SEQ ID NO | GENE ID | Pfam domain name | begin | stop | score | E-value |
|---|---|---|---|---|---|---|---|
| 1406 | 665 | PHE0002928_3065 | zf-C2H2 | 828 | 851 | 24 | 0.00048 |
| 1406 | 665 | PHE0002928_3065 | zf-C2H2 | 857 | 882 | 20.6 | 0.0051 |
| 1218 | 477 | PHE0002939_3089 | Pro_dh | 125 | 474 | 209.4 | 7.10E-60 |
| 807 | 66 | PHE0002940_3090 | Pro_dh | 113 | 464 | 472.4 | 4.80E-139 |
| 1126 | 385 | PHE0002946_3096 | Sugar_tr | 16 | 478 | 52.2 | 1.60E-12 |
| 1126 | 385 | PHE0002946_3096 | MFS_1 | 25 | 444 | 74.4 | 3.20E-19 |
| 1408 | 667 | PHE0002947_3097 | Sugar_tr | 28 | 361 | -47.7 | 1.70E-05 |
| 1408 | 667 | PHE0002947_3097 | MFS_1 | 36 | 382 | 31.8 | 2.20E-06 |
| 1409 | 668 | PHE0002948_3098 | FGGY_N | 22 | 305 | 21.6 | 1.80E-11 |
| 808 | 67 | PHE0002957_3107 | MIP | 339 | 585 | 194.9 | 1.70E-55 |
| 809 | 68 | PHE0002961_3111 | RRM_1 | 197 | 268 | 62.6 | 1.20E-15 |
| 809 | 68 | PHE0002961_3111 | RRM_1 | 542 | 633 | 45.7 | 1.40E-10 |
| 810 | 69 | PHE0002962_3112 | DSPc | 59 | 193 | 198.9 | 1.10E-56 |
| 1127 | 386 | PHE0002963_3113 | DSPc | 1 | 185 | 116 | . 9.40E-32 |
| 1128 | 387 | PHE0002966_3116 | DSPc | 696 | 838 | 93.6 | 5.40E-25 |
| 768 | 27 | PHE0002976_3126 | Pkinase | 39 | 351 | 197.5 | 2.90E-56 |
| 1129 | 388 | PHE0002984_3134 | Pkinase | 57 | 323 | 288.3 | 1.30E-83 |
| 1410 | 669 | PHE0002987_3137 | GFO_IDH_MocA | 4 | 127 | 147 | 4.30E-41 |
| 1410 | 669 | PHE0002987_3137 | GFO_IDH_MocA_C | 139 | 251 | 62.5 | 1.20E-15 |
| 1318 | 577 | PHE0002997_3147 | WD40 | 166 | 204 | 6.4 | 4.5 |
| 1318 | 577 | PHE0002997_3147 | WD40 | 235 | 272 | 14.8 | 0.29 |
| 1318 | 577 | PHE0002997_3147 | WD40 | 278 | 316 | 30.9 | 3.90E-06 |
| 1318 | 577 | PHE0002997_3147 | WD40 | 385 | 423 | 38.1 | 2.70E-08 |
| 769 | 28 | PHE0002999_3149 | ADH_N | 26 | 160 | 151.9 | 1.50E-42 |
| 769 | 28 | PHE0002999_3149 | ADH_zinc_N | 189 | 332 | 148 | 2.20E-41 |
| 770 | 29 | PHE0003000_3150 | Aminotran_3 | 29 | 367 | 504.4 | 1.20E-148 |
| 771 | 30 | PHE0003001_3151 | Glyco_hydro_38 | 290 | 560 | 451.7 | 8.40E-133 |
| 771 | 30 | PHE0003001_3151 | Glyco_hydro_38C | 670 | 1080 | 441.3 | 1.20E-129 |
| 772 | 31 | PHE0003002_3152 | TPP enzyme_N | 25 | 219 | 111.4 | 2.30E-30 |
| 772 | 31 | PHE0003002_3152 | TPP_enzyme_M | 243 | 396 | -7.1 | 0.00019 |
| 772 | 31 | PHE0003002 3152 | TPP_enzyme_C | 443 | 610 | 20.7 | 4.20E-08 |
| 773 | 32 | PHE0003004_3154 | Citrate_synt | 83 | 460 | 749.8 | 1.60E-222 |
| 774 | 33 | PHE0003006_3156 | Aldo_ket_red | 14 | 293 | 471 | 1.30E-138 |

(continued)

| PEP SEQ ID NO | NUC SEQ ID NO | GENE ID | Pfam domain name | begin | stop | score | E-value |
|---|---|---|---|---|---|---|---|
| 775 | 34 | PHE0003007_3157 | PGAM | 11 | 249 | 143.7 | 4.60E-40 |
| 776 | 35 | PHE0003008_3158 | HSP20 | 96 | 207 | 135.9 | 9.80E-38 |
| 1319 | 578 | PHE0003011_3161 | Pro_CA | 45 | 213 | 79.8 | 7.60E-21 |
| 777 | 36 | PHE0003012_3162 | Trehalase_Ca-bi | 106 | 135 | 57.8 | 3.20E-14 |
| 777 | 36 | PHE0003012_3162 | Trehalase | 163 | 721 | 1035.9 | 0 |
| 1411 | 670 | PHE0003017_3167 | Transketolase_N | 7 | 339 | 804.4 | 5.50E-239 |
| 1411 | 670 | PHE0003017_3167 | Transket_pyr | 356 | 533 | 245 | 1.40E-70 |
| 1411 | 670 | PHE0003017_3167 | Transketolase_C | 546 | 657 | 61 | 3.40E-15 |
| 1412 | 671 | PHE0003018_3168 | ubiquitin | 1 | 74 | 149.1 | 1.00E-41 |
| 1412 | 671 | PHE0003018_3168 | ubiquitin | 77 | 150 | 149.1 | 1.00E-41 |
| 1412 | 671 | PHE0003018_3168 | ubiquitin | 153 | 226 | 149.1 | 1.00E-41 |
| 1412 | 671 | PHE0003018_3168 | ubiquitin | 229 | 302 | 149.1 | 1.00E-41 |
| 1412 | 671 | PHE0003018_3168 | ubiquitin | 305 | 378 | 149.1 | 1.00E-41 |
| 779 | 38 | PHE0003022_3172 | Transaldolase | 25 | 329 | 613.5 | 1.60E-181 |
| 1413 | 672 | PHE0003023_3173 | Carb_kinase | 35 | 305 | 496 | 3.80E-146 |
| 1414 | 673 | PHE0003025_3175 | Mpv17_PMP22 | 114 | 184 | 158.9 | 1.20E-44 |
| 1416 | 675 | PHE0003027_3177 | YjeF_N | 24 | 204 | 272.2 | 9.20E-79 |
| 1417 | 676 | PHE0003028_3178 | 2-Hacid_dh | 25 | 113 | 31.6 | 2.50E-06 |
| 1417 | 676 | PHE0003028_3178 | 2-Hacid_dh_C | 124 | 327 | 266.2 | 5.90E-77 |
| 1418 | 677 | PHE0003030_3180 | Asp | 66 | 428 | 180.4 | 4.10E-51 |
| 780 | 39 | PHE0003031_3181 | AMP-binding | 101 | 586 | 262.8 | 6.00E-76 |
| 781 | 40 | PHE0003035_3185 | Hexokinase_1 | 12 | 230 | 456.7 | 2.60E-134 |
| 781 | 40 | PHE0003035_3185 | Hexokinase_2 | 243 | 500 | 501.6 | 8.20E-148 |
| 782 | 41 | PHE0003037_3187 | Aldo_ket_red | 7 | 308 | 501.5 | 8.80E-148 |
| 783 | 42 | PHE0003044_3194 | Pkinase | 47 | 309 | 309.5 | 5.30E-90 |
| 783 | 42 | PHE0003044_3194 | Pkinase_Tyr | 47 | 307 | 91.2 | 2.90E-24 |
| 784 | 43 | PHE0003056_3206 | Pkinase | 352 | 672 | 250.4 | 3.40E-72 |
| 784 | 43 | PHE0003056_3206 | Pkinase_C | 690 | 748 | 33.9 | 4.80E-07 |
| 1219 | 478 | PHE0003057_3207 | Pkinase | 93 | 392 | 231.2 | 1.90E-66 |
| 1219 | 478 | PHE0003057_3207 | Pkinase C | 410 | 461 | 36.1 | 1.10E-07 |
| 1130 | 389 | PHE0003061_3211 | MatE | 38 | 198 | 128.2 | 2.10E-35 |
| 1130 | 389 | PHE0003061_3211 | MatE | 259 | 422 | 133.6 | 5.00E-37 |
| 1419 | 678 | PHE0003062_3212 | bZIP_1 | 196 | 249 | 28.4 | 2.30E-05 |
| 1419 | 678 | PHE0003062_3212 | bZIP_2 | 196 | 246 | 31.7 | 2.20E-06 |
| 1131 | 390 | PHE0003074_3224 | NTF2 | 10 | 126 | 113.9 | 4.10E-31 |

(continued)

| PEP SEQ ID NO | NUC SEQ ID NO | GENE ID | Pfam domain name | begin | stop | score | E-value |
|---|---|---|---|---|---|---|---|
| 1131 | 390 | PHE0003074_3224 | RRM_1 | 306 | 376 | 39.7 | 8.90E-09 |
| 1420 | 679 | PHE0003088_3240 | Pkinase | 134 | 389 | 335.4 | 8.90E-98 |
| 1420 | 679 | PHE0003088_3240 | Pkinase_C | 409 | 454 | 46.2 | 9.80E-11 |
| 1421 | 680 | PHE0003089_3237 | Pkinase | 140 | 395 | 337.7 | 1.80E-98 |
| 1421 | 680 | PHE0003089_3237 | Pkinase_C | 415 | 460 | 44.2 | 3.90E-10 |
| 1422 | 681 | PHE0003090_3238 | Pkinase | 150 | 406 | 331.2 | 1.60E-96 |
| 1422 | 681 | PHE0003090_3238 | Pkinase_C | 426 | 468 | 33.6 | 5.90E-07 |
| 1423 | 682 | PHE0003091_3239 | Pkinase | 152 | 408 | 324 | 2.40E-94 |
| 1423 | 682 | PHE0003091_3239 | Pkinase_C | 428 | 471 | 48.1 | 2.70E-11 |
| 1133 | 392 | PHE0003101_76 | Globin | 13 | 152 | 113.4 | 5.90E-31 |
| 1132 | 391 | PHE0003101_3969 | Globin | 13 | 152 | 113.4 | 5.90E-31 |
| 1424 | 683 | PHE0003102_3244 | PfkB | 3 | 310 | 292.3 | 8.00E-85 |
| 1425 | 684 | PHE0003102_3579 | PfkB | 3 | 310 | 292.3 | 8.00E-85 |
| 1426 | 685 | PHE0003121_3267 | p450 | 31 | 482 | 286.9 | 3.40E-83 |
| 1134 | 393 | PHE0003124_3270 | APS_kinase | 29 | 184 | 365 | 1.00E-106 |
| 1427 | 686 | PHE0003130_3276 | UDPGT | 9 | 451 | -16.7 | 7.80E-10 |
| 1428 | 687 | PHE0003134_3280 | CH | 15 | 115 | 52.2 | 1.60E-12 |
| 1428 | 687 | PHE0003134_3280 | EB1 | 217 | 264 | 93.1 | 7.50E-25 |
| 1135 | 394 | PHE0003138_3292 | PfkB | 7 | 313 | 371.2 | 1.40E-108 |
| 1136 | 395 | PHE0003139_3295 | PfkB | 18 | 324 | 363.5 | 3.00E-106 |
| 785 | 44 | PHE0003166_3368 | Alpha-amylase | 8 | 429 | -32.5 | 7.70E-08 |
| 1220 | 479 | PHE0003182_3341 | Glycogen_syn | 18 | 661 | 1833.8 | 0 |
| 1137 | 396 | PHE0003190_3389 | HLH | 100 | 151 | 58.5 | 2.00E-14 |
| 786 | 45 | PHE0003191_3390 | HLH | 109 | 160 | 65.5 | 1.50E-16 |
| 1429 | 688 | PHE0003194_3393 | Sina | 130 | 329 | 442 | 6.90E-130 |
| 1430 | 689 | PHE0003195_3394 | Sina | 104 | 303 | 445.2 | 7.60E-131 |
| 1138 | 397 | PHE0003196_3395 | Sina | 105 | 304 | 433.5 | 2.60E-127 |
| 1139 | 398 | PHE0003198_3397 | Sina | 96 | 295 | 414.3 | 1.50E-121 |
| 1431 | 690 | PHE0003199_3398 | Sina | 95 | 294 | 417.1 | 2.10E-122 |
| 1321 | 580 | PHE0003200_3399 | Sina | 102 | 301 | 457.1 | 2.00E-134 |
| 1432 | 691 | PHE0003201_3400 | Sina | 103 | 302 | 434.6 | 1.20E-127 |
| 1433 | 692 | PHE0003208_3410 | DUF250 | 247 | 391 | 172.4 | 1.00E-48 |
| 1140 | 399 | PHE0003211_3417 | H_PPase | 9 | 756 | 1731.2 | 0 |
| 1141 | 400 | PHE0003211_3967 | H_PPase | 9 | 756 | 1731.2 | 0 |
| 1434 | 693 | PHE0003213_3419 | AA_permease | 86 | 546 | 479.9 | 2.80E-141 |
| 1142 | 401 | PHE0003217_3423 | Myb_DNA-binding | 14 | 61 | 49.1 | 1.30E-11 |

(continued)

| PEP SEQ ID NO | NUC SEQ ID NO | GENE ID | Pfam domain name | begin | stop | score | E-value |
|---|---|---|---|---|---|---|---|
| 1142 | 401 | PHE0003217_3423 | Myb_DNA-binding | 67 | 112 | 38.4 | 2.10E-08 |
| 1436 | 695 | PHE0003222_3428 | AA_kinase | 49 | 279 | 234.3 | 2.40E-67 |
| 1436 | 695 | PHE0003222_3428 | PUA | 318 | 392 | 93.1 | 7.60E-25 |
| 1143 | 402 | PHE0003224_3432 | DUF1423 | 102 | 586 | 778.3 | 4.10E-231 |
| 1322 | 581 | PHE0003227_3443 | Histone | 64 | 139 | 44.2 | 4.00E-10 |
| 1322 | 581 | PHE0003227_3443 | CBFD_NFYB_HMF | 70 | 134 | 87.3 | 4.10E-23 |
| 1144 | 403 | PHE0003228_3444 | adh_short | 18 | 186 | 104.4 | 3.00E-28 |
| 1145 | 404 | PHE0003229_3445 | adh_short | 18 | 186 | 103 | 7.70E-28 |
| 1222 | 481 | PHE0003234_3450 | Glycolytic | 11 | 358 | 895.8 | 1.70E-266 |
| 1437 | 696 | PHE0003238_3454 | Glycolytic | 11 | 358 | 927.6 | 4.80E-276 |
| 1223 | 482 | PHE0003239_3455 | FHA | 33 | 108 | 48.2 | 2.40E-11 |
| 1147 | 406 | PHE0003240_3456 | FHA | 32 | 107 | 47.5 | 4.10E-11 |
| 1438 | 697 | PHE0003242_3458 | ABC_tran | 1 | 147 | 111.7 | 2.00E-30 |
| 1148 | 407 | PHE0003243_3459 | ABC_tran | 47 | 233 | 240.5 | 3.20E-69 |
| 1439 | 698 | PHE0003251_3468 | DUF1530 | 110 | 209 | 211.9 | 1.30E-60 |
| 1150 | 409 | PHE0003257_3474 | GRIM-19 | 16 | 141 | 106.8 | 5.50E-29 |
| 1225 | 484 | PHE0003259_3476 | MAP1_LC3 | 14 | 117 | 279.2 | 7.00E-81 |
| 1440 | 699 | PHE0003260_3477 | MAP1_LC3 | 14 | 117 | 274.9 | 1.40E-79 |
| 1323 | 582 | PHE0003261_3478 | MAP1_LC3 | 14 | 117 | 285.4 | 9.50E-83 |
| 787 | 46 | PHE0003266_3485 | ARID | 37 | 146 | 63 | 8.70E-16 |
| 787 | 46 | PHE0003266_3485 | HMG_box | 238 | 305 | 43.6 | 6.20E-10 |
| 1226 | 485 | PHE0003267_3486 | WHEP-TRS | 55 | 107 | 23.7 | 2.60E-05 |
| 1226 | 485 | PHE0003267_3486 | tRNA-synt_2b | 149 | 450 | 262.1 | 9.70E-76 |
| 1226 | 485 | PHE0003267_3486 | HGTP_anticodon | 617 | 705 | 86.2 | 8.80E-23 |
| 1152 | 411 | PHE0003276_3495 | El_dh | 89 | 393 | 468.9 | 5.60E-138 |
| 1325 | 584 | PHE0003277 3496 | El_dh | 87 | 392 | 472.3 | 5.20E-139 |
| 815 | 74 | PHE0003280_3499 | DUF6 | 156 | 286 | 34.5 | 3.30E-07 |
| 1153 | 412 | PHE0003282_3501 | DUF6 | 21 | 154 | 47.8 | 3.30E-11 |
| 1153 | 412 | PHE0003282_3501 | DUF6 | 195 | 324 | 31.2 | 3.20E-06 |
| 1229 | 488 | PHE0003285_3504 | PCI | 265 | 364 | 95.1 | 1.90E-25 |
| 1154 | 413 | PHE0003286 3505 | CTP_synth_N | 1 | 287 | 674.4 | 7.50E-200 |
| 1154 | 413 | PHE0003286_3505 | GATase | 311 | 548 | 271.2 | 1.90E-78 |
| 1155 | 414 | PHE0003287_3506 | CTP_synth_N | 1 | 287 | 671.4 | 6.20E-199 |
| 1155 | 414 | PHE0003287_3506 | GATase | 311 | 548 | 264.9 | 1.40E-76 |
| 1156 | 415 | PHE0003304_3524 | BAH | 36 | 156 | 27.2 | 5.00E-05 |

(continued)

| PEP SEQ ID NO | NUC SEQ ID NO | GENE ID | Pfam domain name | begin | stop | score | E-value |
|---|---|---|---|---|---|---|---|
| 1156 | 415 | PHE0003304_3524 | RRM_1 | 366 | 440 | 15.2 | 0.00061 |
| 1444 | 703 | PHE0003308_3527 | Yippee | 1 | 108 | 209.4 | 7.20E-60 |
| 1157 | 416 | PHE0003309_3528 | Yippee | 1 | 108 | 207.1 | 3.70E-59 |
| 1231 | 490 | PHE0003311_3530 | F-box | 25 | 71 | 23.8 | 0.00053 |
| 1231 | 490 | PHE0003311_3530 | FBA 1 | 215 | 418 | 300.3 | 3.10E-87 |
| 1158 | 417 | PHE0003312_3531 | PTR2 | 96 | 499 | 504.3 | 1.20E-148 |
| 1445 | 704 | PHE0003315_3534 | FHA | 32 | 107 | 45.7 | 1.40E-10 |
| 1446 | 705 | PHE0003320_3539 | Pkinase | 45 | 312 | 296.6 | 4.10E-86 |
| 1159 | 418 | PHE0003321_3540 | Pkinase | 44 | 311 | 297.1 | 2.90E-86 |
| 1160 | 419 | PHE0003327_3546 | Pkinase | 4 | 257 | 288.5 | 1.20E-83 |
| 1161 | 420 | PHE0003328_3547 | Pkinase | 4 | 258 | 236.2 | 6.40E-68 |
| 1162 | 421 | PHE0003330_3549 | IF4E | 1 | 198 | 283.9 | 2.70E-82 |
| 1163 | 422 | PHE0003333_3552 | IF4E | 13 | 237 | 411.1 | 1.40E-120 |
| 1164 | 423 | PHE0003333_4250 | IF4E | 13 | 237 | 411.1 | 1.40E-1201 |
| 1165 | 424 | PHE0003336_3555 | IF4E | 6 | 235 | 485.9 | 4.10E-143 |
| 1447 | 706 | PHE0003344_3562 | Orn_Arg_deC_N | 73 | 368 | 275.8 | 7.70E-80 |
| 1447 | 706 | PHE0003344_3562 | Orn_DAP_Arg_deC | 371 | 551 | 62.3 | 1.40E-15 |
| 1448 | 707 | PHE0003347_3565 | Acetyltransf_1 | 265 | 343 | 56.1 | 1.10E-13 |
| 1448 | 707 | PHE0003347_3565 | Bromodomain | 460 | 548 | 122.8 | 8.90E-34 |
| 1326 | 585 | PHE0003352_3571 | GAF | 158 | 307 | 90.9 | 3.50E-24 |
| 1326 | 585 | PHE0003352_3571 | HisKA | 343 | 408 | 87.5 | 3.70E-23 |
| 1326 | 585 | PHE0003352_3571 | HATPase_c | 455 | 586 | 123.4 | 5.50E-34 |
| 1166 | 425 | PHE0003353_3572 | Sina | 96 | 295 | 414.3 | 1.50E-121 |
| 1449 | 708 | PHE0003354_3573 | bZIP 1 | 181 | 246 | 21.4 | 0.00062 |
| 789 | 48 | PHE0003358_3581 | p450 | 51 | 492 | 111.4 | 2.30E-30 |
| 1232 | 491 | PHE0003360_3583 | ketoacyl-synt | 126 | 371 | 291.6 | 1.30E-84 |
| 1232 | 491 | PHE0003360_3583 | Ketoacyl-synt_C | 379 | 537 | 218.4 | 1.40E-62 |
| 1167 | 426 | PHE0003361_3584 | malic | 119 | 307 | 392.3 | 6.40E-115 |
| 1167 | 426 | PHE0003361_3584 | Malic_M | 309 | 562 | 459.7 | 3.40E-135 |
| 1168 | 427 | PHE0003362_3585 | Hexokinase_1 | 2 | 203 | 248.6 | 1.20E-71 |
| 1168 | 427 | PHE0003362_3585 | Hexokinase_2 | 210 | 454 | 254.1 | 2.60E-73 |
| 1169 | 428 | PHE0003364_3587 | Aminotran_3 | 1 | 160 | -78.8 | 1.00E-07 |
| 1233 | 492 | PHE0003365_3588 | Rieske | 220 | 317 | 106.9 | 5.30E-29 |
| 1327 | 586 | PHE0003366_3589 | Transaldolase | 78 | 417 | 264.8 | 1.60E-76 |
| 1170 | 429 | PHE0003369_3592 | Gp_dh_N | 4 | 154 | 304.8 | 1.40E-88 |

(continued)

| PEP SEQ ID NO | NUC SEQ ID NO | GENE ID | Pfam domain name | begin | stop | score | E-value |
|---|---|---|---|---|---|---|---|
| 1170 | 429 | PHE0003369_3592 | Gp_dh_C | 159 | 316 | 384.5 | 1.50E-112 |
| 1328 | 587 | PHE0003370_3593 | Cyclotide | 54 | 83 | 48.3 | 2.20E-11 |
| 1171 | 430 | PHE0003373_3596 | Metallophos | 168 | 366 | 87.7 | 3.10E-23 |
| 1234 | 493 | PHE0003374_3597 | Glyco_hydro_16 | 39 | 248 | 321.5 | 1.30E-93 |
| 1234 | 493 | PHE0003374_3597 | XET_C | 280 | 335 | 72.3 | 1.30E-18 |
| 1235 | 494 | PHE0003375_3598 | Trp_syntA | 94 | 347 | 486.6 | 2.60E-143 |
| 1236 | 495 | PHE0003378_3601 | MtN3_slv | 13 | 100 | 138.3 | 1.80E-38 |
| 1236 | 495 | PHE0003378_3601 | MtN3_slv | 134 | 220 | 124 | 3.70E-34 |
| 1450 | 709 | PHE0003380_3603 | Cu-oxidase_3 | 35 | 151 | 248.1 | 1.60E-71 |
| 1450 | 709 | PHE0003380_3603 | Cu-oxidase | 161 | 313 | 207.9 | 2.10E-59 |
| 1450 | 709 | PHE0003380_3603 | Cu-oxidase_2 | 420 | 556 | 190.6 | 3.50E-54 |
| 1172 | 431 | PHE0003381_3604 | F-box | 96 | 141 | 13 | 0.33 |
| 1172 | 431 | PHE0003381_3604 | Kelch_1 | 181 | 223 | 6.9 | 0.59 |
| 1172 | 431 | PHE0003381_3604 | Kelch_1 | 225 | 270 | 44.2 | 3.90E-10 |
| 1172 | 431 | PHE0003381_3604 | Kelch_2 | 225 | 270 | 18.4 | 0.023 |
| 1172 | 431 | PHE0003381_3604 | Kelch_1 | 272 | 319 | 36 | 1.20E-07 |
| 1173 | 432 | PHE0003385_3608 | 3_5_exonuc | 75 | 268 | 75.9 | 1.10E-19 |
| 1173 | 432 | PHE0003385_3608 | KH_1 | 318 | 378 | 41.4 | 2.70E-09 |
| 1239 | 498 | PHE0003387_3610 | DUF125 | 45 | 208 | 196 | 8.10E-56 |
| 1451 | 710 | PHE0003388_3611 | Methyltransf_2 | 1 | 87 | -62.5 | 2.00E-06 |
| 1174 | 433 | PHE0003391_3614 | Pkinase_Tyr | 154 | 428 | 123.4 | 5.60E-34 |
| 1174 | 433 | PHE0003391_ 3614 | Pkinase | 154 | 428 | 125 | 1.90E-34 |
| 1175 | 434 | PHE0003392_3615 | Lipase_GDSL | 68 | 390 | 191 | 2.50E-54 |
| 1176 | 435 | PHE0003393_3616 | FAD_binding 3 | 68 | 416 | -75.2 | 2.80E-06 |
| 1176 | 435 | PHE0003393_3616 | DAO | 70 | 327 | -18.3 | 0.00014 |
| 1240 | 499 | PHE0003401_3624 | Steroid_dh | 116 | 262 | 187.2 | 3.60E-53 |
| 1456 | 715 | PHE0003414_3654 | Alpha-amylase | 10 | 396 | -67.3 | 8.70E-06 |
| 1457 | 716 | PHE0003415_3655 | Wzy_C | 367 | 433 | 72.1 | 1.50E-18 |
| 790 | 49 | PHE0003416_3656 | UbiA | 106 | 392 | 59.7 | 8.40E-15 |
| 1241 | 500 | PHE0003417_3657 | Myb_DNA-binding | 24 | 73 | 33.8 | 5.40E-07 |
| 1241 | 500 | PHE0003417_3657 | Myb_DNA-binding | 124 | 171 | 46.9 | 6.20E-11 |
| 1242 | 501 | PHE0003418_3658 | ICL | 21 | 551 | 1223.9 | 0 |
| 1458 | 717 | PHE0003431_3639 | Alpha-amylase | 31 | 433 | 275 | 1.30E-79 |
| 1243 | 502 | PHE0003433_3642 | Hydrolase | 7 | 220 | 56.6 | 7.10E-14 |
| 1459 | 718 | PHE0003434_3643 | Hydrolase | 76 | 274 | 119.6 | 8.00E-33 |

(continued)

| PEP SEQ ID NO | NUC SEQ ID NO | GENE ID | Pfam domain name | begin | stop | score | E-value |
|---|---|---|---|---|---|---|---|
| 1460 | 719 | PHE0003436_3645 | Hydrolase | 4 | 214 | 80.7 | 4.20E-21 |
| 1461 | 720 | PHE0003437_3646 | Hydrolase | 21 | 239 | 54.8 | 2.50E-13 |
| 1462 | 721 | PHE0003447_3678 | AT_hook | 41 | 53 | 7.4 | 1.1 |
| 1462 | 721 | PHE0003447_3678 | DUF296 | 68 | 185 | 198.9 | 1.10E-56 |
| 1244 | 503 | PHE0003450_3681 | WRKY | 106 | 165 | 119.4 | 8.80E-33 |
| 791 | 50 | PHE0003457_3688 | Remorin_C | 393 | 504 | 145.6 | 1.20E-40 |
| 1245 | 504 | PHE0003458_3689 | Remorin_C | 402 | 512 | 145 | 1.80E-40 |
| 1246 | 505 | PHE0003459_3690 | zf-C3HC4 | 166 | 207 | 41.3 | 3.00E-09 |
| 1247 | 506 | PHE0003476_3707 | zf-B_box | 3 | 47 | 47.2 | 5.00E-11 |
| 1463 | 722 | PHE0003483_3729 | Ank | 43 | 76 | 17.6 | 0.041 |
| 1463 | 722 | PHE0003483_3729 | Ank | 77 | 107 | 23.3 | 0.00079 |
| 1463 | 722 | PHE0003483_3729 | Ank | 111 | 144 | 26.2 | 0.0001 |
| 1248 | 507 | PHE0003484_3730 | Ank | 28 | 61 | 24.9 | 0.00026 |
| 1249 | 508 | PHE0003491_3737 | SRF-TF | 25 | 75 | 82.8 | 9.50E-22 |
| 1250 | 509 | PHE0003499_3745 | RWP-RK | 526 | 577 | 110.1 | 5.70E-30 |
| 1250 | 509 | PHE0003499_3745 | PBI | 744 | 827 | 72.3 | 1.30E-18 |
| 792 | 51 | PHE0003502_3748 | zf-C3HC4 | 145 | 186 | 37.6 | 3.70E-08 |
| 793 | 52 | PHE0003506_3752 | Myb_DNA-binding | 65 | 110 | 58.6 | 1.90E-14 |
| 1251 | 510 | PHE0003518_3764 | NAM | 9 | 138 | 304.2 | 2.10E-88 |
| 1464 | 723 | PHE0003519_3765 | NAM | 10 | 139 | 292.4 | 7.70E-85 |
| 794 | 53 | PHE0003522_3768 | NAM | 8 | 143 | 161.4 | 2.10E-45 |
| 1252 | 511 | PHE0003524_3770 | NAM | 18 | 147 | 246.2 | 6.30E-71 |
| 1329 | 588 | PHE0003526_3772 | B3 | 85 | 190 | 110.6 | 4.10E-30 |
| 1329 | 588 | PHE0003526_3772 | Auxin_resp | 255 | 338 | 159.5 | 7.50E-45 |
| 1253 | 512 | PHE0003570_3816 | GRAS | 3 | 352 | 324.6 | 1.50E-94 |
| 1254 | 513 | PHE0003576_3822 | AP2 | 5 | 68 | 129.4 | 9.20E-36 |
| 1330 | 589 | PHE0003581_3827 | F-box | 52 | 105 | 19.8 | 0.0086 |
| 1330 | 589 | PHE0003581_3827 | Tub | 116 | 403 | 582.4 | 3.70E-172 |
| 1465 | 724 | PHE0003582_3828 | ZF-HD_dimer | 179 | 238 | 135.5 | 1.30E-37 |
| 1255 | 514 | PHE0003583_3829 | Myb_DNA-binding | 212 | 263 | 46.2 | 9.90E-11 |
| 1467 | 726 | PHE0003588_3834 | HLH | 146 | 195 | 41.8 | 2.10E-09 |
| 795 | 54 | PHE0003592_3838 | AP2 | 141 | 204 | 126.4 | 7.10E-35 |
| 796 | 55 | PHE0003595_3841 | AP2 | 147 | 211 | 150.9 | 2.90E-42 |
| 1468 | 727 | PHE0003598_3844 | E2F_TDP | 67 | 150 | 145.7 | 1.10E-40 |
| 1257 | 516 | PHE0003599_3845 | E2F_TDP | 5 | 91 | 124.2 | 3.20E-34 |
| 1258 | 517 | PHE0003600_3846 | HLH | 418 | 467 | 51.9 | 1.90E-12 |

(continued)

| PEP SEQ ID NO | NUC SEQ ID NO | GENE ID | Pfam domain name | begin | stop | score | E-value |
|---|---|---|---|---|---|---|---|
| 1469 | 728 | PHE0003606_3852 | HMG_box- | 62 | 131 | 73.6 | 5.70E-19 |
| 1470 | 729 | PHE0003613_3860 | Pyridoxal deC | 33 | 381 | 534.6 | 9.30E-158 |
| 1471 | 730 | PHE0003617_3866 | zf-C2H2 | 80 | 102 | 17.9 | 0.033 |
| 1259 | 518 | PHE0003630_3888 | JmjC | 132 | 240 | 55.4 | 1.70E-13 |
| 1472 | 731 | PHE0003632_3890 | E2F_TDP | 92 | 180 | 149.3 | 9.00E-42 |
| 797 | 56 | PHE0003633_3891 | HLH | 69 | 121 | 58.3 | 2.20E-14 |
| 798 | 57 | PHE0003635_3893 | zf-C2H2 | 209 | 232 | 22 | 0.0019 |
| 1473 | 732 | PHE0003636_3894 | ZF-HD_dimer | 54 | 115 | 134 | 3.60E-37 |
| 1260 | 519 | PHE0003641_3899 | ZF-HD_dimer | 69 | 128 | 136.1 | 8.70E-38 |
| 1261 | 520 | PHE0003644_3902 | HLH | 127 | 178 | 57.7 | 3.40E-14 |
| 1262 | 521 | PHE0003650_3908 | zf-C2H2 | 47 | 69 | 18.7 | 0.018 |
| 1262 | 521 | PHE0003650_3908 | zf-C2H2 | 95 | 117 | 19.7 | 0.0093 |
| 799 | 58 | PHE0003663_3921 | zf-C3HC4 | 329 | 369 | 26 | 0.00012 |
| 800 | 59 | PHE0003670_3928 | AP2 | 20 | 84 | 142.2 | 1.20E-39 |
| 801 | 60 | PHE0003672_3930 | WRKY | 155 | 214 | 135.4 | 1.40E-37 |
| 802 | 61 | PHE0003675_3933 | Response_reg | 16 | 136 | 92.2 | 1.40E-24 |
| 802 | 61 | PHE0003675_3933 | Myb_DNA-binding | 204 | 254 | 48.5 | 2.10E-11 |
| 1474 | 733 | PHE0003676_3934 | bZIP_1 | 192 | 256 | 30.2 | 6.30E-06 |
| 1474 | 733 | PHE0003676_3934 | bZIP_2 | 192 | 246 | 38.6 | 1.90E-08 |
| 1263 | 522 | PHE0003678_3936 | bZIP_ 1 | 154 | 218 | 31.8 | 2.10E-061 |
| 1263 | 522 | PHE0003678_3936 | bZIP_2 | 154 | 208 | 37.1 | 5.50E-08 |
| 1331 | 590 | PHE0003681_3942 | AP2 | 13 | 77 | 140.4 | 4.20E-39 |
| 1475 | 734 | PHE0003682_3943 | Pkinase | 46 | 300 | 341.2 | 1.60E-99 |
| 1475 | 734 | PHE0003682_3943 | Pkinase _Tyr | 46 | 298 | 66.5 | 7.00E-17 |
| 1475 | 734 | PHE0003682_3943 | NAF | 385 | 442 | 100.7 | 3.70E-27 |
| 1332 | 591 | PHE0003683_3944 | Sugar_tr | 37 | 468 | -65.4 | 6.40E-05 |
| 1332 | 591 | PHE0003683_3944 | MFS 1 | 40 | 463 | 26.4 | 8.80E-05 |
| 1333 | 592 | PHE0003683_3945 | Sugar_tr | 37 | 468 | -65.4 | 6.40E-05 |
| 1333 | 592 | PHE0003683_ 3945 | MFS_1 | 40 | 463 | 26.4 | 8.80E-05 |
| 1264 | 523 | PHE0003686_3961 | NDK | 80 | 214 | 341.1 | 1.70E-99 |
| 1265 | 524 | PHE0003740_4042 | CBFD_NFYB_HMF | 33 | 98 | 132.1 | 1.40E-36 |
| 1266 | 525 | PHE0003742_4044 | adh_short | 7 | 144 | -11.7 | 2.40E-05 |
| 1266 | 525 | PHE0003742_4044 | RmID_sub_bind | 8 | 338 | -163.7 | 0.0018 |
| 1266 | 525 | PHE0003742_4044 | Epimerase | 9 | 273 | 266 | 6.90E-77 |
| 1266 | 525 | PHE0003742_4044 | Polysacc_synt 2 | 9 | 330 | -161.6 | 0.0008 |

(continued)

| PEP SEQ ID NO | NUC SEQ ID NO | GENE ID | Pfam domain name | begin | stop | score | E-value |
|---|---|---|---|---|---|---|---|
| 1266 | 525 | PHE0003742_4044 | 3Beta_HSD | 10 | 297 | -69.9 | 6.60E-08 |
| 1266 | 525 | PHE0003742_4044 | NAD_binding_4 | 11 | 251 | -54.1 | 3.60E-05 |
| 1267 | 526 | PHE0003743_4046 | adh_short | 7 | 176 | -21 | 9.70E-05 |
| 1267 | 526 | PHE0003743_4046 | RmID_sub_bind | 8 | 338 | -131 | 2.10E-05 |
| 1267 | 526 | PHE0003743_4046 | Epimerase | 9 | 273 | 257.7 | 2.10E-74 |
| 1267 | 526 | PHE0003743_4046 | Polysacc_synt_2 | 9 | 323 | -159.7 | 0.00063 |
| 1267 | 526 | PHE0003743_4046 | 3Beta_HSD | 10 | 297 | -57.4 | 7.80E-09 |
| 1267 | 526 | PHE0003743_4046 | NAD_ binding 4 | 11 | 248 | -56.6 | 5.20E-05 |
| 1476 | 735 | PHE0003788_4122 | NAM | 2 | 97 | 164.2 | 3.10E-46 |
| 1334 | 593 | PHE0003815_4288 | HAMP | 200 | 269 | 65.7 | 1.30E-16 |
| 1334 | 593 | PHE0003815_4288 | PAS | 283 | 413 | 29.7 | 9.10E-06 |
| 1334 | 593 | PHE0003815_4288 | HisKA | 422 | 490 | 95.9 | 1.00E-25 |
| 1334 | 593 | PHE0003815 4288 | HATPase_c | 535 | 655 | 141.9 | 1.60E-39 |
| 1268 | 527 | PHE0003825 4192 | NAM | 14 | 140 | 297 | 3.10E-86 |
| 1269 | 528 | PHE0003839 4208 | zf-LSD1 | 27 | 51 | 45.6 | 1.50E-10 |
| 1269 | 528 | PHE0003839_ 4208 | zf-LSD1 | 66 | 90 | 54.8 | 2.50E-13 |
| 1269 | 528 | PHE0003839_4208 | zf-LSD1 | 104 | 128 | 58.4 | 2.10E-14 |
| 1270 | 529 | PHE0003885_4266 | Globin | 14 | 154 | 96.2 | 8.90E-26 |
| 1271 | 530 | PHE0003886 4267 | Globin | 13 | 152 | 113.7 | 4.70E-31 |
| 1477 | 736 | PHE0003899_4284 | PHD | 180 | 227 | 60.2 | 6.20E-15 |
| 1272 | 531 | PHE0003927_4321 | PAD_porph | 14 | 368 | 694.5 | 6.70E-206 |
| 803 | 62 | PHE0003945_4522 | zf-C3HC4 | 146 | 187 | 39.2 | 1.20E-08 |
| 1478 | 737 | PHE0003948_4528 | MFS_1 | 84 | 520 | 28.5 | 2.00E-05 |
| 1273 | 532 | PHE0003959_4544 | Response_reg | 79 | 202 | 93.9 | 4.30E-25 |
| 1273 | 532 | PHE0003959 4544 | CCT | 695 | 736 | 57.3 | 4.60E-14 |
| 1274 | 533 | PHE0003961_4662 | ketoacyl-synt | 47 | 295 | 266.4 | 4.90E-77 |
| 1274 | 533 | PHE0003961_4662 | Ketoacyl-synt_C | 303 | 458 | 209.1 | 9.40E-60 |
| 1275 | 534 | PHE0003965 _4553 | bZIP_1 | 277 | 335 | 39.8 | 8.10E-09 |
| 1275 | 534 | PHE0003965_4553 | bZIP_2 | 277 | 334 | 40.9 | 3.80E-09 |
| 1276 | 535 | PHE0003966_4554 | FAE_3-kCoA_syn1 | 20 | 365 | 699 | 3.10E-207 |
| 1277 | 536 | PHE0003977_4565 | HEM4 | 56 | 285 | 85.7 | 1.30E-22 |
| 1479 | 738 | PHE0003981_4568 | PK | 22 | 368 | 809.5 | 1.60E-240 |
| 1479 | 738 | PHE0003981_ 4568 | PK_C | 382 | 513 | 221.1 | 2.20E-63 |
| 1280 | 539 | PHE0003993 4579 | Na_Ca_ex | 147 | 280 | 119.1 | 1.10E-32 |

(continued)

| PEP SEQ ID NO | NUC SEQ ID NO | GENE ID | Pfam domain name | begin | stop | score | E-value |
|---|---|---|---|---|---|---|---|
| 1338 | 597 | PHE0003995_4581 | Succ_DH_flav_C | 6 | 136 | 265.6 | 8.60E-77 |
| 1283 | 542 | PHE0003997_4583 | Epimerase | 12 | 226 | -40 | 0.001 |
| 1283 | 542 | PHE0003997_4583 | NmrA | 12 | 309 | 475.3 | 6.50E-140 |
| 1481 | 740 | PHE0003998_4584 | Auxin_inducible | 1 | 86 | 77.5 | 3.60E-20 |
| 1287 | 546 | PHE0004003_4589 | Transferase | 22 | 447 | 254.6 | 1.80E-73 |
| 1290 | 549 | PHE0004006_4592 | Abhydrolase_1 | 164 | 425 | 36.8 | 6.60E-08 |
| 1482 | 741 | PHE0004008_4594 | Trypsin | 112 | 272 | 16.7 | 2.50E-05 |
| 1292 | 551 | PHE0004010_4596 | Myb_DNA-binding | 347 | 392 | 43.1 | 8.40E-10 |
| 1339 | 598 | PHE0004022_4657 | PHD | 202 | 252 | 55.9 | 1.20E-13 |

Table 12.

| Pfam domain name | accession number | gathering cutoff | domain description |
|---|---|---|---|
| 2-Hacid_dh | PF00389.18 | 13.2 | D-isomer specific 2-hydroxyacid dehydrogenase, catalytic domain |
| 2-Hacid_dh_C | PF02826.6 | -75.7 | D-isomer specific 2-hydroxyacid dehydrogenase, NAD binding domain |
| 3Beta HSD | PF01073.8 | -135.9 | 3-beta hydroxysteroid dehydrogenase/ isomerase family |
| 3_5_exonuc | PF01612.10 | -32 | 3'-5' exonuclease |
| AAA | PF00004.17 | 10 | ATPase family associated with various cellular activities (AAA) |
| AA_kinase | PF00696.16 | -40 | Amino acid kinase family |
| AA_permease | PF00324.10 | -120.8 | Amino acid permease |
| ABC1 | PF03109.6 | -27.6 | ABC1 family |
| ABC_tran | PF00005.14 | 8.6 | ABC transporter |
| ADH_N | PF08240.1 | -14.5 | Alcohol dehydrogenase GroES-like domain |
| ADH zinc_N | PF00107.15 | 23.8 | Zinc-binding dehydrogenase |
| AMP-binding | PF00501.15 | 0 | AMP-binding enzyme |
| AMPKBI | PF04739.4 | 25 | 5'-AMP-activated protein kinase, beta subunit, complex-interacting region |
| AP2 | PF00847.9 | 0 | AP2 domain |
| APS_kinase | PF01583.9 | 25 | Adenylylsulphate kinase |
| ARID | PF01388.10 | -8 | ARID/BRIGHT DNA binding domain |
| AT_hook | PF02178.7 | 14.2 | AT hook motif |
| AUX_IAA | PF02309.6 | -83 | AUX/IAA family |
| Aa_trans | PF01490.7 | -128.4 | Transmembrane amino acid transporter protein |

(continued)

| Pfam domain name | accession number | gathering cutoff | domain description |
|---|---|---|---|
| Abhydrolase_1 | PF00561.9 | 5.5 | alpha/beta hydrolase fold |
| Acetyltransf_1 | PF00583.12 | 18.6 | Acetyltransferase (GNAT) family |
| Acyltransferase | PF01553.10 | 6 | Acyltransferase |
| Aldedh | PF00171.11 | -295 | Aldehyde dehydrogenase family |
| Aldo_ket_red | PF00248.10 | -97 | Aldo/keto reductase family |
| Alpha-amylase | PF00128.11 | -93 | Alpha amylase, catalytic domain |
| Alpha_adaptinC2 | PF02883.9 | -12 | Adaptin C-terminal domain |
| Aminotran_1_2 | PF00155.9 | -57.5 | Aminotransferase class I and II |
| Aminotran_3 | PF00202.10 | -207.6 | Aminotransferase class-III |
| Aminotran_5 | PF00266.8 | -92.9 | Aminotransferase class-V |
| Ammonium_transp | PF00909.10 | -144 | Ammonium Transporter Family |
| Ank | PF00023.17 | 21.6 | Ankyrin repeat |
| Annexin | PF00191.8 | 8 | Annexin |
| ArfGap | PF01412.8 | -17 | Putative GTPase activating protein for Arf |
| Asn_synthase | PF00733.10 | -52.8 | Asparagine synthase |
| Asp | PF00026.13 | -186.1 | Eukaryotic aspartyl protease |
| Auxin_inducible | PF02519.4 | -15 | Auxin responsive protein |
| Auxin_resp | PF06507.3 | 25 | Auxin response factor |
| B12D | PF06522.1 | 25 | B12D protein |
| B3 | PF02362.11 | 26.5 | B3 DNA binding domain |
| B56 | PF01603.8 | -210 | Protein phosphatase 2A regulatory B subunit (B56 family) |
| BAH | PF01426.6 | 7 | BAH domain |
| BRO1 | PF03097.6 | 25 | BRO1-like domain |
| BURP | PF03181.5 | -52 | BURP domain |
| Bromodomain | PF00439.13 | 8.9 | Bromodomain |
| CAF1 | PF04857.8 | -100.5 | CAF I family ribonuclease |
| CBFD_NFYB_HMF | PF00808.12 | 18.4 | Histone-like transcription factor (CBF/NF-Y) and archaeal histone |
| CBS | PF00571.16 | 15.8 | CBS domain pair |
| CCT | PF06203.3 | 25 | CCT motif |
| CH | PF00307.18 | 22.5 | Calponin homology (CH) domain |
| CMAS | PF02353.9 | -177.9 | Cyclopropane-fatty-acyl-phospholipid synthase |
| CN_hydrolase | PF00795.11 | -13.9 | Carbon-nitrogen hydrolase |
| CTP_synth_N | PF06418.2 | 25 | CTP synthase N-terminus |
| CTP_transf_2 | PF01467.15 | -11.8 | Cytidylyltransferase |
| Carb_kinase | PF01256.7 | -66.3 | Carbohydrate kinase |
| Catalase | PF00199.8 | -229 | Catalase |

(continued)

| Pfam domain name | accession number | gathering cutoff | domain description |
|---|---|---|---|
| Cation_efflux | PF01545.10 | -95.7 | Cation efflux family |
| Chal_sti_synt_C | PF02797.5 | -6.1 | Chalcone and stilbene synthases, C-terminal domain |
| Chromo | PF00385.11 | 27.5 | 'chromo' (CHRromatin Organisation MOdifier) domain |
| Citrate_synt | PF00285.10 | -101.5 | Citrate synthase |
| CobW_C | PF07683.3 | 18 | Cobalamin synthesis protein cobW C-terminal domain |
| ComA | PF02679.5 | 25 | (2R)-phospho-3-sulfolactate synthase (ComA) |
| CorA | PF01544.8 | -61.3 | CorA-like Mg2+ transporter protein |
| Cpn10 | PF00166.11 | -7.8 | Chaperonin 10 Kd subunit |
| Cpn60_TCP1 | PF00118.13 | -223.4 | TCP-1/cpn60 chaperonin family |
| Cu-oxidase | PF00394.11 | -18.9 | Multicopper oxidase |
| Cu-oxidase_2 | PF07731.3 | -5.8 | Multicopper oxidase |
| Cu-oxidase_3 | PF07732.4 | 10 | Multicopper oxidase |
| Cyclin_C | PF02984.7 | -13 | Cyclic, C-terminal domain |
| Cyclin_N | PF00134.12 | -14.7 | Cyclin, N-terminal domain |
| Cyclotide | PF03784.3 | 25 | Cyclotide family |
| Cys_Met_Meta_PP | PF01053.9 | -278.4 | Cys/Met metabolism PLP-dependent enzyme |
| Cystatin | PF00031.10 | 17.5 | Cystatin domain |
| DAO | PF01266.11 | -36.5 | FAD dependent oxidoreductase |
| DNA_photolyase | PF00875.7 | -10 | DNA photolyase |
| DSPc | PF00782.9 | -21.8 | Dual specificity phosphatase, catalytic domain |
| DUF125 | PF01988.8 | -10.1 | Integral membrane protein DUF125 |
| DUF1423 | PF07227.1 | 25 | Protein of unknown function (DUF1423) |
| DUF1530 | PF07060.1 | 25 | ProFAR isomerase associated |
| DUF1685 | PF07939.1 | 25 | Protein of unknown function (DUF 1685) |
| DUF246 | PF03138.4 | -15 | Plant protein family |
| DUF250 | PF03151.6 | 125 | Domain of unknown function, DUF250 |
| DUF296 | PF03479.4 | -11 | Domain of unknown function (DUF296) |
| DUF393 | PF04134.2 | 25 | Protein of unknown function, DUF393 |
| DUF581 | PF04570.4 | -3.1 | Protein of unknown function (DUF581) |
| DUF6 | PF00892.9 | 30 | Integral membrane protein DUF6 |
| DUF641 | PF04859.2 | 25 | Plant protein of unknown function (DUF641) |
| DUF760 | PF05542.1 | 25 | Protein of unknown function (DUF760) |
| DUF788 | PF05620.1 | 25 | Protein of unknown function (DUF788) |
| Dehydrin | PF00257.8 | -4.4 | Dehydrin |

(continued)

| Pfam domain name | accession number | gathering cutoff | domain description |
|---|---|---|---|
| Di19 | PF05605.2 | 25 | Drought induced 19 protein (Di19) |
| Dirigent | PF03018.4 | 25 | Dirigent-like protein |
| DnaJ | PF00226.18 | -8 | DnaJ domain |
| E1_dh | PF00676.9 | -90 | Dehydrogenase E1 component |
| E2F_TDP | PF02319.9 | -17 | E2F/DP family winged-helix DNA-binding domain |
| EB1 | PF03271.6 | 25 | EB1-like C-terminal motif |
| EF1_GNE | PF00736.8 | 20 | EF-1 guanine nucleotide exchange domain |
| ELFV_dehydrog | PF00208.10 | -27 | Glutamate/Leucine/Phenylalanine/Valine dehydrogenase |
| ELFV_dehydrog_N | PF02812.7 | 31.8 | Glu/Leu/Phe/Val dehydrogenase, dimerisation domain |
| ERO1 | PF04137.5 | -179.5 | Endoplasmic Reticulum Oxidoreductin 1 (ERO1) |
| ERp29 | PF07749.2 | 10.5 | Endoplasmic reticulum protein ERp29, C-terminal domain |
| Epimerase | PF01370.10 | -46.3 | NAD dependent epimerase/dehydratase family |
| F-box | PF00646.20 | 12.4 | F-box domain |
| FAD_binding_3 | PF01494.8 | -136.6 | FAD binding domain |
| FAD_binding_4 | PF01565.12 | -8.1 | FAD binding domain |
| FAD_binding_7 | PF03441.3 | 25 | FAD binding domain of DNA photolyase |
| FAE_3-kCoA_syn1 | PF07168.1 | 25 | Fatty acid elongase 3-ketoacyl-CoA synthase 1 |
| FA_desaturase | PF00487.13 | -46 | Fatty acid desaturase |
| FBA_1 | PF07734.2 | -39.4 | F-box associated |
| FBPase | PF00316.9 | -170.3 | Fructose-1-6-bisphosphatase |
| FGGY_N | PF00370.10 | -104.7 | FGGY family of carbohydrate kinases, N-terminal domain |
| FHA | PF00498.13 | 25 | FHA domain |
| Fer4 | PF00037.14 | 8 | 4Fe-4S binding domain |
| GAF | PF01590.14 | 23 | GAF domain |
| GAT | PF03127.4 | -7 | GAT domain |
| GATA | PF00320.15 | 28.5 | GATA zinc finger |
| GATase | PF00117.15 | -38.1 | Glutamine amidotransferase class-I |
| GATase_2 | PF00310.10 | -106.2 | Glutamine amidotransferases class-II |
| GFO_IDH_MocA | PF01408.11 | -7.2 | Oxidoreductase family, NAD-binding Rossmann fold |
| GFO_IDH_MocA_C | PF02894.7 | 6 | Oxidoreductase family, C-terminal alpha/beta domain |
| GH3 | PF03321.3 | -336 | GH3 auxin-responsive promoter |

(continued)

| Pfam domain name | accession number | gathering cutoff | domain description |
|---|---|---|---|
| GIDA | PF01134.11 | -226.7 | Glucose inhibited division protein A |
| GRAS | PF03514.4 | -78 | GRAS family transcription factor |
| GRIM-19 | PF06212.1 | 25 | GRIM-19 protein |
| GSHPx | PF00255.9 | -16 | Glutathione peroxidase |
| GST_C | PF00043.13 | 22.3 | Glutathione S-transferase, C-terminal domain |
| GST_N | PF02798.8 | 14.6 | Glutathione S-transferase, N-terminal domain |
| GTP_EFTU | PF00009.14 | 8 | Elongation factor Tu GTP binding domain |
| GTP EFTU_D2 | PF03144.13 | 25 | Elongation factor Tu domain 2 |
| GTP_EFTU_ D3 | PF03143.6 | 14.3 | Elongation factor Tu C-terminal domain |
| Gamma-thionin | PF00304.10 | 9.6 | Gamma-thionin family |
| Gln-synt_C | PF00120.13 | -124 | Glutamine synthetase, catalytic domain |
| Gln-synt_N | PF03951.8 | 9 | Glutamine synthetase, beta-Grasp domain |
| Globin | PF00042.11 | -8.8 | Globin |
| Glyco_hydro_1 | PF00232.8 | -301.8 | Glycosyl hydrolase family 1 |
| Glyco_hydro_14 | PF01373.7 | -231.4 | Glycosyl hydrolase family 14 |
| Glyco_ hydro_16 | PF00722.9 | -65 | Glycosyl hydrolases family 16 |
| Glyco_hydro_38 | PF01074.11 | -125.3 | Glycosyl hydrolases family 38 N-terminal domain |
| Glyco_hydro_38C | PF07748.2 | -93.1 | Glycosyl hydrolases family 38 C-terminal domain |
| Glyco_transf_ 20 | PF00982.9 | -243.6 | Glycosyltransferase family 20 |
| Glycogen_syn | PF05693.2 | -492.3 | Glycogen synthase |
| Glycolytic | PF00274.8 | -158 | Fructose-bisphosphate aldolase class-I |
| Glycos_transf_ 1 | PF00534.9 | -7.3 | Glycosyl transferases group 1 |
| Glycos_transf_2 | PF00535.14 | 17.6 | Glycosyl transferase family 2 |
| Glyoxalase | PF00903.14 | 12.1 | Glyoxalase/Bleomycin resistance protein/ Dioxygenase superfamily |
| Got1 | PF04178.2 | 25 | Got1-like family |
| Gp_dh_C | PF02800.8 | -64.1 | Glyceraldehyde 3-phosphate dehydrogenase, C-terminal domain |
| Gp_dh_N | PF00044.11 | -74.2 | Glyceraldehyde 3-phosphate dehydrogenase, NAD binding domain |
| HALZ | PF02183.7 | 17 | Homeobox associated leucine zipper |
| HAMP | PF00672.13 | 17 | HAMP domain |
| HATPase_ c | PF02518.13 | 22.4 | Histidine kinase-, DNA gyrase B-, and HSP90-like ATPase |
| HEAT | PF02985.9 | 17.6 | HEAT repeat |
| HEM4 | PF02602.5 | -12 | Uroporphyrinogen-III synthase HemD |

(continued)

| Pfam domain name | accession number | gathering cutoff | domain description |
|---|---|---|---|
| HGTP_anticodon | PF03129.9 | -2 | Anticodon binding domain |
| HI0933_like | PF03486.4 | -255.8 | HI0933-like protein |
| HLH | PF00010.15 | 8.2 | Helix-loop-helix DNA-binding domain |
| HMA | PF00403.14 | 17.4 | Heavy-metal-associated domain |
| HMG_box | PF00505.8 | 4.1 | HMG (high mobility group) box |
| HSF_DNA-bind | PF00447.7 | -70 | HSF-type DNA-binding |
| HSP20 | PF00011.9 | 13 | Hsp20/alpha crystallin family |
| H_PPase | PF03030.5 | -377 | Inorganic H+ pyrophosphatase |
| Heme_oxygenase | PF01126.10 | -58 | Heme oxygenase |
| Hexapep | PF00132.11 | 20 | Bacterial transferase hexapeptide (three repeats) |
| Hexokinase_ 1 | PF00349.10 | -110.3 | Hexokinase |
| Hexokinase_2 | PF03727.5 | -131.3 | Hexokinase |
| HisKA | PF00512.13 | 10.2 | His Kinase A (phosphoacceptor) domain |
| Hist_deacetyl | PF00850.9 | -71 | Histone deacetylase domain |
| Histone | PF00125.12 | 17.4 | Core histone H2A/H2B/H3/H4 |
| Homeobox | PF00046.17 | -4.1 | Homeobox domain |
| Hpt | PF01627.11 | 25 | Hpt domain |
| Hydrolase | PF00702.13 | 13.6 | haloacid dehalogenase-like hydrolase |
| ICL | PF00463.9 | -234 | Isocitrate lyase family |
| IF4E | PF01652.8 | -35 | Eukaryotic initiation factor 4E |
| IPK | PF03770.6 | 25 | Inositol polyphosphate kinase |
| IIvC | PF01450.8 | -33.8 | Acetohydroxy acid isomeroreductase, catalytic domain |
| IIvN | PF07991.1 | -75.8 | Acetohydroxy acid isomeroreductase, catalytic domain |
| Inhibitor_I29 | PF08246.1 | 4.9 | Cathepsin propeptide inhibitor domain (I29) |
| Ion_trans | PF00520.18 | -4.5 | Ion transport protein |
| Isoamylase_N | PF02922.7 | -6.5 | Isoamylase N-terminal domain |
| Jacalin | PF01419.6 | 20 | Jacalin-like lectin domain |
| JmjC | PF02373.11 | -8 | JmjC domain |
| JmjN | PF02375.6 | 25 | jmjN domain |
| K-box | PF01486.7 | 0 | K-box region |
| KA1 | PF02149.9 | 25 | Kinase associated domain 1 |
| KH_1 | PF00013.17 | 8.1 | KH domain |
| Kelch_1 | PF01344.13 | 20 | Kelch motif |
| Kelch_2 | PF07646.4 | 20 | Kelch motif |
| Ketoacyl-synt_C | PF02801.10 | -54.9 | Beta-ketoacyl synthase, C-terminal domain |
| Kunitz_legume | PF00197.8 | -32 | Trypsin and protease inhibitor |

(continued)

| Pfam domain name | accession number | gathering cutoff | domain description |
| --- | --- | --- | --- |
| LEA_5 | PF00477.7 | 25 | Small hydrophilic plant seed protein |
| LIM | PF00412.10 | 0 | LIM domain |
| LRR_2 | PF07723.2 | 8.7 | Leucine Rich Repeat |
| Lactamase_B | PF00753.15 | 22.3 | Metallo-beta-lactamase superfamily |
| Ldh_1_C | PF02866.6 | -13 | lactate/malate dehydrogenase, alpha/beta C-terminal domain |
| Ldh_1_N | PF00056.11 | -31.3 | lactate/malate dehydrogenase, NAD binding domain |
| Lectin_legA | PF00138.7 | 19 | Legume lectins alpha domain |
| Lectin_legB | PF00139.9 | -77 | Legume lectins beta domain |
| Lig_chan | PF00060.16 | 8.2 | Ligand-gated ion channel |
| Lipase_GDSL | PF00657.11 | 10.9 | GDSL-like Lipase/Acylhydrolase |
| M20_dimer | PF07687.3 | 12 | Peptidase dimerisation domain |
| MAP1_LC3 | PF02991.5 | -18.8 | associated protein 1A/1B, light chain 3 |
| MFMR | PF07777.1 | -46.7 | G-box binding protein MFMR |
| MFS_1 | PF07690.4 | 23.5 | Major Facilitator Superfamily |
| MIP | PF00230.8 | -62 | Major intrinsic protein |
| Malic_M | PF03949.4 | -143.9 | Malic enzyme, NAD binding domain |
| MatE | PF01554.8 | 59.6 | MatE |
| Metallophos | PF00149.16 | 22 | Calcineurin-like phosphoesterase |
| Metallothio_2 | PF01439.7 | -3 | Metallothionein |
| Meth_synt_1 | PF08267.1 | -167.8 | Cobalamin-independent synthase, N-terminal domain |
| Meth synt_2 | PF01717.7 | -155 | Cobalamin-independent synthase, Catalytic domain |
| Methyltransf_11 | PF08241.1 | 17.1 | Methyltransferase domain |
| Methyltransf_12 | PF08242.1 | 21.4 | Methyltransferase domain |
| Methyltransf_2 | PF00891.7 | -103.8 | O-methyltransferase |
| Methyltransf_3 | PF01596.7 | -120.6 | O-methyltransferase |
| Mpv17_PMP22 | PF04117.2 | -5.4 | Mpv17 / PMP22 family |
| MtN3_slv | PF03083.5 | -0.8 | MtN3/saliva family |
| Myb_DNA-binding | PF00249.18 | 19.1 | Myb-like DNA-binding domain |
| NAC | PF01849.6 | 0 | NAC domain |
| NAD_binding_4 | PF07993.1 | -87.7 | Male sterility protein |
| NAF | PF03822.4 | 25 | NAF domain |
| NAM | PF02365.5 | -19 | No apical meristem (NAM) protein |
| NDK | PF00334.8 | -59.9 | Nucleoside diphosphate kinase |
| NIF | PF03031.7 | -81 | NLI interacting factor-like phosphatase |
| NPH3 | PF03000.4 | 25 | NPH3 family |

(continued)

| Pfam domain name | accession number | gathering cutoff | domain description |
|---|---|---|---|
| NTF2 | PF02136.10 | 6 | Nuclear transport factor 2 (NTF2) domain |
| NTP_transferase | PF00483.12 | -90.5 | Nucleotidyl transferase |
| NUDIX | PF00293.16 | 0 | NUDIX domain |
| Na_Ca_ex | PF01699.12 | 25 | Sodium/calcium exchanger protein |
| NifU_N | PF01592.6 | -13 | NifU-like N terminal domain |
| NmrA | PF05368.2 | -90.6 | NmrA-like family |
| Orn_Arg_deC_N | PF02784.6 | -76 | Pyridoxal-dependent decarboxylase, pyridoxal binding domain |
| Orn DAP_Arg_deC | PF00278.11 | -34.9 | decarboxylase, C-terminal sheet domain |
| Oxidored_FMN | PF00724.8 | -147.7 | NADH:flavin oxidoreductase / NADH oxidase family |
| PA | PF02225.10 | 13 | PA domain |
| PAD_porph | P F043 71.4 | -180.8 | Porphyromonas-type peptidyl-arginine deiminase |
| PARP | PF00644.9 | -55.5 | Poly(ADP-ribose) polymerase catalytic domain |
| PAS | PF00989.12 | 20 | PAS fold |
| PB1 | PF00564.12 | 12.1 | PB1 domain |
| PBD | PF00786.16 | 12.1 | P21-Rho-binding domain |
| PCI | PF01399.14 | 25 | PCI domain |
| PDZ | PF00595.11 | 12.1 | PDZ domain (Also known as DHR or GLGF) |
| PEP-utilizers | PF00391.12 | 10 | PEP-atilising enzyme, mobile domain |
| PEP-utilizers_C | PF02896.7 | -173 | PEP-utilising enzyme, TIM barrel domain |
| PEPcase | PF00311.7 | 25 | Phosphoenolpyruvate carboxylase |
| PGAM | PF00300.11 | -3 | Phosphoglycerate mutase family |
| PHD | PF00628.16 | 25.9 | PHD-finger |
| PK | PF00224.10 | -244 | Pyruvate kinase, barrel domain |
| PK_C | PF02887.5 | -44 | Pyruvate kinase, alpha/beta domain |
| PMSR | PF01625.9 | -62 | Peptide methionine sulfoxide reductase |
| PP2C | PF00481.10 | -44 | Protein phosphatase 2C |
| PPDK_N | PF01326.8 | -87 | Pyruvate phosphate dikinase, PEP/pyruvate binding domain |
| PRA1 | PF03208.8 | 25 | PRA I family protein |
| PSI_PsaF | PF02507.5 | 25 | Photosystem I reaction centre subunit III |
| PTR2 | PF00854.11 | -50 | POT family |
| PUA | PF01472.8 | 2.2 | PUA domain |
| Peptidase_A22B | PF04258.3 | -137.3 | Signal peptide peptidase |
| Peptidase_C1 | PF00112.11 | -115.8 | Papain family cysteine protease |
| Peptidase_C15 | PF01470.7 | -100 | Pyroglutamyl peptidase |

(continued)

| Pfam domain name | accession number | gathering cutoff | domain description |
|---|---|---|---|
| Peptidase_M20 | PF01546.16 | -14.4 | Peptidase family M20/M25/M40 |
| Peptidase_S10 | PF00450.11 | -198 | Serine carboxypeptidase |
| Peptidase_S41 | PF03572.7 | -25.8 | Peptidase family S41 |
| PfkB | PF00294.12 | -67.8 | pfkB family carbohydrate kinase |
| Phytochrome | PF00360.9 | 11 | Phytochrome region |
| Pkinase | PF00069.14 | -70.8 | Protein kinase domain |
| Pkinase_C | PF00433.11 | 14 | Protein kinase C terminal domain |
| Pkinase_Tyr | PF07714.4 | 65 | Protein tyrosine kinase |
| Polysace-Synt_2 | PF02719.5 | -176 | Polysaccharide biosynthesis protein |
| Pro_CA | PF00484.8 | -45 | Carbonic anhydrase |
| Pro_dh | PF01619.7 | -120.5 | Proline dehydrogenase |
| Pyr_redox | PF00070.16 | 5 | Pyridine nucleotide-disulphide oxidoreductase |
| Pyr_redox_2 | PF07992.2 | -20 | Pyridine nucleotide-disulphide oxidoreductase |
| Pyr_redox_dim | PF02852.11 | -13 | Pyridine nucleotide-disulphide oxidoreductase, dimerisation domain |
| Pyridoxal_deC | PF00282.8 | -158.6 | Pyridoxal-dependent decarboxylase conserved domain |
| RHD3 | PF05879.2 | 25 | hair defective 3 GTP-binding protein (RHD3) |
| RIO1 | PF01163.1 | -89.1 | RIO1 family |
| RRM_1 | PF00076.10 | 15.2 | RNA recognition motif. (a.k.a. RRM, RBD, or RNP domain) |
| RTC | PF01137.11 | -36.9 | RNA 3'-terminal phosphate cyclase |
| RTC_insert | PF05189.3 | 25 | RNA 3'-terminal phosphate cyclase (RTC), insert domain |
| RWP-RK | PF02042.5 | 25 | RWP-RK domain |
| Ran_BP 1 | PF00638.8 | -38 | RanBP1 domain |
| Ras | PF00071.11 | 18 | Ras family |
| Remorin_C | PF03763.3 | 25 | Remorin, C-terminal region |
| Response_reg | PF00072.11 | -14.4 | Response regulator receiver domain |
| Reticulon | PF02453.7 | -40 | Reticulon |
| Ribonuclease_T2 | PF00445.8 | -53 | Ribonuclease T2 family |
| Ribosomal_L1 | PF00687.10 | -101 | Ribosomal protein L1p/L10e family |
| Ribosomal_L10e | PF00826.7 | 25 | Ribosomal L 10 |
| Ribosomal_L12 | PF00542.8 | 25 | Ribosomal protein L7/L12 C-terminal domain |
| Ribosomal_L19e | PF01280.9 | -28 | Ribosomal protein L19e |
| Ribosomal_L39 | PF00832.9 | 25 | Ribosomal L39 protein |
| Ribosomal_L7Ae | PF01248.13 | 6 | Ribosomal protein L7Ae/L30eS12e/Gadd45 family |

(continued)

| Pfam domain name | accession number | gathering cutoff | domain description |
|---|---|---|---|
| Ribosomal_S11 | PF00411.7 | -4 | Ribosomal protein S 11 |
| Ribosomal_S17 | PF00366.9 | 1.7 | Ribosomal protein S 17 |
| Ribosomal_S2 | PF00318.9 | -22 | Ribosomal protein S2 |
| Ribosomal_S27 | PF01599.8 | 50 | Ribosomal protein S27a |
| Rieske | PF00355.15 | -7 | Rieske [2Fe-2S] domain |
| RmtD_sub_bind | PF04321.6 | -171.8 | RmlD substrate binding domain |
| RuBisCO_small | PF0010L9 | -20.1 | Ribulose bisphosphate carboxylase, small chain |
| Rubrerythrin | PF02915.7 | -4.8 | Rubrerythrin |
| SAM_1 | PF00536.17 | 11.3 | SAM domain (Sterile alpha motif) |
| SAM_2 | PF07647.5 | 20 | SAM domain (Sterile alpha motif) |
| SPC25 | PF06703.1 | 25 | Microsomal signal peptidase 25 kDa subunit (SPC25) |
| SPX | PF03105.9 | -20 | SPX domain |
| SRF-TF | PF00319.8 | 11 | SRF-type transcription factor (DNA-binding and dimerisation domain) |
| START | PF01852.8 | 25 | START domain |
| SapB_1 | PF05184.4 | 20 | Saposin-like type B, region 1 |
| SapB_2 | PF03489.5 | 20 | Saposin-like type B, region 2 |
| SecY | PF00344.9 | -210 | eubacterial_secY protein |
| SelR | PF01641.8 | -66.5 | SelR domain |
| Sigma70_r1_2 | PF00140.9 | 25 | Sigma-70 factor, region 1.2 |
| Sigma70_r2 | PF04542.3 | 11 | Sigma-70 region 2 |
| Sigma70_r3 | PF04539.4 | 10 | Sigma-70 region 3 |
| Sigma70_r4 | PF04545.5 | 20.7 | Sigma-70, region 4 |
| Sina | PF03145.6 | -48.4 | Seven in absentia protein family |
| Steroid_dh | PF02544.6 | -44.7 | 3-oxo-5-alpha-steroid 4-dehydrogenase |
| Suc_Fer-like | PF06999.2 | -42.4 | Sucrase/ferredoxin-like |
| Succ_DH flav_C | PF02910.9 | -42 | Fumarate reductase/succinate dehydrogenase flavoprotein C-terminal domain |
| Sucrose_synth | PF00862.9 | -134 | Sucrose synthase |
| Sugar_tr | PF00083.12 | -85 | Sugar (and other) transporter |
| Synaptobrevin | PF00957.9 | 25 | Synaptobrevin |
| TPP_enzyme_C | PF02775.9 | 19.7 | Thiamine pyrophosphate enzyme, C-terminal TPP binding domain |
| TPP_enzyme_M | Thiamine PF00205.11 | -23.9 | Thiamine pyrophosphate enzyme, central domain |
| TPP_enzyme_N | PF02776.7 | -70 | Thiamine pyrophosphate enzyme, N-terminal TPP binding domain |

(continued)

| Pfam domain name | accession number | gathering cutoff | domain description |
|---|---|---|---|
| Thiolase_C | PF02803.6 | -30.7 | Thiolase, C-terminal domain |
| Thiolase_N | PF00108.1 | -129.5 | Thiolase, N-terminal domain |
| Thioredoxin | PF00085.8 | -25.7 | Thioredoxin |
| Tic22 | PF04278.2 | 25 | Tic22-like family |
| Transaldolase | PF00923.8 | -49 | Transaldolase |
| Transferase | PF02458.5 | -161.2 | Transferase family |
| Transket_pyr | PF02779.12 | -50 | Transketolase, pyridine binding domain |
| Transketolase_C | PF02780.9 | -15.5 | Transketolase, C-terminal domain |
| Transketolase_N | PF00456.10 | -98 | Transketolase, thiamine diphosphate binding domain |
| Trehalase | PF01204.8 | 25 | Trehalase |
| Trehalase_Ca-bi | PF07492.1 | 20 | Neutral trehalase Ca2+ binding domain |
| Trehalose_PPase | PF02358.6 | -49.4 | Trehalose-phosphatase |
| Trp_Tyr_perm | PF03222.3 | -232.6 | Tryptophan/tyrosine permease family |
| Trp_syntA | PF00290.10 | -149.8 | Tryptophan synthase alpha chain |
| Trypsin | PF00089.13 | -33.2 | Trypsin |
| Tub | PF01167.7 | -98 | Tub family |
| Tubulin | PF00091.14 | -55.7 | Tubulin/FtsZ family, GTPase domain |
| Tubulin_C | PF03953.6 | -10 | Tubulin/FtsZ family, C-terminal domain |
| UBA | PF00627.18 | 20.5 | UBA/TS-N domain |
| UDPGP | PF01704.7 | -265.2 | UTP--glucose-1-phosphate uridylyltransferase |
| UDPGT | PF00201.8 | -151 | UDP-glucoronosyl and UDP-glucosyl transferase |
| UPF0057 | PF01679.7 | 25 | Uncharacterized protein family UPF0057 |
| UbiA | PF01040.8 | -45 | UbiA prenyltransferase family |
| Ubie_methyltran | PF01209.8 | -117 | ubiE/COQ5 methyltransferase family |
| Usp | PF00582.15 | 25.7 | Universal stress protein family |
| VHS | PF00790.8 | -13.2 | VHS domain |
| VQ | PF05678.3 | 25 | VQ motif |
| W2 | PF02020.7 | 25 | eIF4-gamma/eIF5/eIF2-epsilon |
| WD40 | PF00400.19 | 21.4 | WD domain, G-beta repeat |
| WHEP-TRS | PF00458.9 | 10 | WHEP-TRS domain |
| WRKY | PF03106.5 | 25 | WRKY DNA -binding domain |
| Wzy_C | PF04932.4 | 25 | O-Antigen Polymerase |
| XET_C | PF06955.2 | 11.4 | Xyloglucan endo-transgiycosyiase (XET) C-terminus |
| Xan_ur_permease | PF00860.10 | -151.2 | Permease family |
| YL1 | PF05764.3 | 25 | YL 1 nuclear protein |

(continued)

| Pfam domain name | accession number | gathering cutoff | domain description |
|---|---|---|---|
| YL1_C | PF08265.1 | 18.6 | YL1 nuclear protein C-terminal domain |
| YTH | PF04146.5 | 25 | YT521-B-like family |
| Yippee | PF03226.4 | 25 | Yippee putative zinc-binding protein |
| YjeF_N | PF03853.3 | 25 | YjeF-related protein N-terminus |
| ZF-HD_dimer | PF04770.2 | 25 | ZF-HD protein dimerisation region |
| Zip | PF02535.10 | -28 | ZIP Zinc transporter |
| adh_short | PF00106.13 | -46.6 | short chain dehydrogenase |
| bZIP_1 | PF00170.10 | 16.5 | bZIP transcription factor |
| bZIP_2 | PF07716.4 | 15 | Basic region leucine zipper |
| cNMP_binding | PF00027.17 | 20.6 | Cyclic nucleotide-binding domain |
| cobW | PF02492.8 | -10 | CobW/HypB/UreG, nucleotide-binding domain |
| Efhand | PF00036.19 | 17.5 | EF hand |
| ketoacyl-synt | PF00109.14 | -73.6 | Beta-ketoacyl synthase, N-terminal domain |
| Malic | PF00390.8 | 25 | Malic enzyme, N-terminal domain |
| p450 | PF00067.11 | -105 | Cytochrome P450 |
| Peroxidase | PF00141.12 | -10 | Peroxidase |
| tRNA-synt 2b | PF00587.14 | -40.5 | tRNA synthetase class II core domain (G, H, P, S and T) |
| Ubiquitin | PF00240.12 | 19.4 | Ubiquitin family |
| zf-B_box | PF00643.13 | 11.1 | B-box zinc finger |
| zf-C2H2 | PF00096.14 | 19 | Zinc finger, C2H2 type |
| zf-C3HC4 | PF00097.12 | 16.9 | Zinc finger, C3HC4 type (RING finger) |
| zf-Dof | PF02701.5 | 25 | Dof domain, zinc finger |
| zf-LSD1 | PF06943.2 | 25 | LSD1 zinc finger |

**Example 8. Selection of transgenic plants with enhanced agronomic trait(s)**

This example illustrates the preparation and identification by selection of transgenic seeds and plants derived from transgenic plant cells of this invention where the plants and seed are identified by screening a having an enhanced agronomic trait imparted by expression of a protein selected from the group including the homologous proteins identified in Example 4. Transgenic plant cells of corn, soybean, cotton, canola, wheat and rice are transformed with recombinant DNA for expressing each of the homologs identified in Example 4. Plants are regenerated from the transformed plant cells and used to produce progeny plants and seed that are screened for enhanced water use efficiency, enhanced cold tolerance, increased yield, enhanced nitrogen use efficiency, enhanced seed protein and enhanced seed oil. Plants are identified exhibiting enhanced traits imparted by expression of the homologous proteins.

## Table 2

```
PEP SEQ ID NO: homolog SEQ ID NOs
   742:29905  5099 35463 50212 37971  7205  3357  1268 22496 51540 11819 20318
        10222 38718 22493 10753  8421 41364 20242 43974  9385 26860 22396 17613
   743:24338 23824 39432  9401 19972 40978  8769 21722 44262  5056 51264 37200
```

```
21787 25902 10936 39892 20148 49161 28133  6181 20650 27441 18969 38913
32489 49992 13312 16184  4828 46943 41205 49071 10397  7339 19944 19943
42648 36600  9207 44464 38522 14635 50234 50222 19920 19925 16718 35773
41877 10336 48495  4419 48028 48731 28817 28675 43103 26927  6141 19295
18708 50827 22913 19142 51054 46620 32471 19329 38095 52737 44112 45341
52608  5791 30076 25314 25472 11497 40872  4693  8975 52598  8446 52461
38946 28940  9591 10529 26114  9276 13172 14340  4820 21904  1721 43379
36039 11316  3912  1741 18459 39927 19830  8081 37269 11687  5300 23494
13246  2683 38417 23405 15143 52034 45468 29603  6592 30721 38087 51755
 3698 12524 35637 44537 31353 50024 10445 10206 25913 48114 48749 42791
21387 10849 40596  9266 12048 43423  7012  7271 40308  9610 43082 25043
49756 21245 31939  9104 49650 34424 22732 48989 41173 26308 16699 16680
16676 30080  6217  4625 15802 45918  5495 21438 25574  6431 13075 26768
12531 11728 37631 23361 11283 12169 46114 47095  8965 49434 49924  2650
 5963 35736 32005 24156 16109 40928 35182 19922 43609 19104 18925 12752
50767 11054 20123 16169 48202 46030 15257 13444 26259 37916 22644 14526
24883 19187 27669 16254 38855 22614 16772  1985 36998 24378 49177 37515
11608 13654 34414  6468 46418 26697 42857 38841 45929 14052 10098 31078
24706 10155 46907 49409 38743 42417 47872 16354 47873  5756 23229 18229
 3122 30739 49020 29088 16369 28191  4699 41324 37516  5053 35936  1187
 7083  3411 42999  5788 50730  7057 38631 46760  2088 23015 21071 20388
20390  7738 31051 45198 26323 22537 12627  7011 22271 39434 38274 31519
 1640 13446  2795 15980 19291 34210 40973 22734 22253 44564  6715  5213
34281 44764 47403 15832 28987 33530 40372 49787  3110 37760 17283 16838
17667  8771   874  7352 40182 12400  8079 52724  7594 23935 17063  9387
25518 21753 50924 46212 39438 12654 51515  4900 49082 40191 51378 24536
 3217 23056 32561  8404 42177 49830  8445 43114   745  6680 31717  7153
19504  6630 12204 35896 15854 10450 19496 31720 51661 19535 30479 30477
25475 30474 47020 19500 15335 19472 23708 25477 47022 19486 19484 23714
15338 16915 44534 44529 44530 38595 40739 15559 15654 15584 15557 15625
15513 13703 15517 13737 13015 15482 13700 15515 13711 15512 13664 15552
13741 15653 36470 13765 13767 50002 41834 41837 41857 41820 41815 41766
41861 21933  4273  1680  1679 37815 14014 14768 16574 16568 14771 50231
50181 16612 50200 14829 14792 14800 16573 16607 19898 16602 19899 50225
19892 14724 19918 19921 14832 16460 14835 15777 15755 16456 15743 15750
15618 16543 16497 15690 15592 15477 16490 15478 15659 15686 15710 16492
14796 14764 16669 25737 49950 14421 15704 33508 26226  2040 38254  9790
47248 23190 17866  5128 33496 12488 35429
744:23824 39432  9401 40978 19972  8769 21722 44262  5056 51264 37200 25902
21787 10936 39892 20148 49161 28133  6181 20650 27441 18969 38913 49992
13312 16184  4828 46943 41205 49071 10397  7339 19944 19943 42648 36600
 9207 44464 38522 14635 50234 50222 19920 19925 16718 35773 41877 10336
 4419 48495 48028 48731 26927  6141 18708 19295 50827 22913 19142 51054
46620 32471 19329 38095  5791 30076 25314  8446 26441 28053 10529  9591
 9276 26114 13172 14340  4820  1721 21904 43379 36039 11316  3912  1741
39927 18459 19830 37269  8081 11687  5300 23494 13246 38417  2683 23405
15143 52034 45468 29603 30721  6592 51755 38087  3698 12524 35637 44537
31353 50024 10445 10206 25913 48114 48749 21387 42791 10849 40596  9266
 9219  4116 11081 30667 25043 49756 21245  9104 49650 22732 48989 41173
26308 16699 16680 16676  6217 30080  4625 15802 45918  5495 21438 25574
 6431 13075 26768 12531 46114 47095  8965 49434 49924 23361 11728 12169
37631 11283  2650  5963 35736 32005 24156 16109 40928 35182 43609 19922
19104 11054 46030 20123 15257 48202 16169 37916 22644 13444 26259 19187
27669 16254 38855 22614 24378 37515 36998 49177 16772  1985 11608 13654
34414  6468 26697 46418 38841 45929 42857 14052 46907 49409 38743 42417
47872 47873 16354  5756 23229 18229  3122 49020 30739 29088 16369 28191
 4699 20390  7738 21071 20388 31051 45198 26323 12627 22537  7011 22271
39434 38274 13446  1640  2795 31519 15980 19291  5213 34210 44564 44764
34281 22734  6715 40973 35733 22253  6583 28987 49787 47403 15832 19143
40372 37760 17667   874  8771 40182  7352 17283 16838 12400  8079  7594
52724 25518 50924  9387 23935 17063 21753 51515 12654 46212 39438  4900
51378 49082 32561  8404 43114  8445   745  6680 31717  7153 19504  6630
```

```
        38595 12204 19496 35896 15854 10450 16915 19535 51661 31720 30479 30477
        30474 25475 47020 19500 15335 19472 23708 25477 47022 19486 44530 40739
        19484 23714 15338 44534 44529 15557 15559 15654 15584 15625 13737 15513
        13703 15482 15517 13015 15552 15515 15512 13711 13700 13664 13741 36470
        15653 13767 13765 50002 41766 41861 41834 41815 41837 41857 41820 21933
         4273  1680  1679 14014 37815 14768 16568 14829 14771 16574 50231 16612
        50181 14792 19898 16607 50200 16573 14800 16602 19899 14724 19892 19918
        50225 19921 14832 16460 14835 15777 16456 15755 15743 15750 15618 16497
        15592 15477 16490 15690 15478 16543 15659 16492 15686 15710 14764 16669
        14796 25737 49950 14421 33508 15704 26226  2040 38254  9790 47248 23190
         5128 17866 38401
745:23824  9401 39432 40978 19972  8769 21722 44262 51264 37200 21787 25902
        10936 39892 20148 49161 28133  6181 20650 27441 18969 38913 32489 49992
        13312 16184  4828 46943 49071  7339 19944 19943 42648 36600  9207 44464
        38522 14635 50234 50222 16718 35773 41877 10336 48495  4419 23630 48028
        48731 28817 28675 43103 26927  6141 19295 18708 50827 22913 19142 51054
        46620 32471 19329 38095 44112 52737 45341 52608  5791 30076 25314 11497
        25472 40872  8975 52598  4693  8446 26441 28053 52461 24087 28940 38946
        10529  9591  9276 26114 13172 14340  4820 21904  1721 43379 36039 11316
         3912  1741 18459 39927 19830  8081 37269 11687  5300 23494 13246 38417
         2683 23405 15143 45468 52034 29603  6592 30721 38087 51755  3698 35637
        12524 31353 44537 50024 10445 10206 25913 48114 48749 42791 21387 10849
        47459 40596  9266 12048  9219  7012 43423  4116  7271 28370 28290 40308
         9610 11081 43082 30667 25043 49756 21245 27143 31939 49650  9104 34424
        22732 48989 41173 26308 16699 16680 16676 30080  6217  4625 15802 45918
         5495 21438 25574  6431 13075 26768 12531 11283 37631 49924 46114 47095
         8965 49434 23361 12169 35736 32005  2650  5963 24156 11728 16109 35182
        43609 19922 40928 19104 12752 38339 17696  2871 50767 18925 11054 20123
        48202 16169 46030 15257 13444 26259 37916 14526 22644 24883 16999 19187
        27669 38855 22614 36998 49177 24378 37515 16254 16772  1985 11608 26697
         6468 38841 13654 34414 46418 42857 45929 10098 14052 24706 46907 49409
        38743 42417 47872 47873 16354  5756 23229 18229 49020  3122 30739 29088
        16369 28191  4699  1187  7083  3411 42999  5788 50730 41324 37516  5053
        35936 38631 46760  7057 34604 23015 20390  7738 20388 21071 12627 22537
         7011 26323 45198 31051 22271 38274 39434  1640 34210 19291  2795 13446
        31519 22253 44564 40973 15980 34281  6715 44764 22734 47403 28987  5213
        35733 15832  6583 14018  3110 40372 19143 49787  1603 41603  2175  8708
        31517 24882 49774   874  8771 17667 40182 37760  7352 12400 17283 16838
         7594 52724 23935 17063  8079 21753  9387 50924 25518 46212 39438 51515
         4900 12654 49082 40191 51378  3217 24536 23056 32561  8404 49830 42177
        35429 12488   743 35548 33496   744 28273 38401 43114  8445 50002 41861
        41815 41837 41857 41766 41834 41820 16425 21933  4273  1680  1679 14014
        37815 14768 14832 14835 15618 16543 16497 15690 16490 14796 14764 25737
        49950 14421 15704 33508 26226  9790 47248 23190 17866  5128
746: 4204  5785 24478 30884 43649 43650 32382 32388 33001 32383 32380 37018
        46391 46628 46627 46634 21948 34761 22583 42610  4136 32998 10164  7701
         7617 34929  7995 43416 33942  9533 19306 10731 47853 33909 30375 45837
        50786 29093 14871 47965 12935  9418 18791  2639 21243  5834 39646 19964
        39622 19908  7988 36244 39233  4898 45410  9422  9427 42977 46703  7365
        31833 47559 47555 24623 19361 12187   891 17542 33708 50955 19182 19462
        31409 22801 24662 24639 24633 42629
747:26047 47010 49254  7116 35390 32954  6091 30430  4016 36746 36724 46659
        29894 35578  6815 11476 38544 40318 25308 17093 24952 31307  2260  2258
        35713 36532 50655 50668 50553 50623 50558 50523 50629 50661 50659 50632
        50590 50583 50528 50524 50688 50664 50551 50594 50526 50548 50555 50621
        47466 47462 10609 28923 25332 28384 23819 14470  4931 14957 16152 44855
        45562 19414 30879  3603 21073 23053 40226 16648  2806 41351 41389 41372
        41368 41392 42493 42489 42463 42462 31435 50170 32112 32145 32150 47870
        13357  7465  9640 14983 27871 38373 47516 27038 37766 38460 29295 34141
        37948 28245 10600 43401 25958 17994 15358 22791 24328 24308 25848 22071
        16683 32556  3093 16128 46309 31069 23851 16161 31148 15647 20138 26954
         6394 51959 20608 41293  5294 24420 41340 33234 23388  3496 26613 30730
```

```
11809 22205 16709 51673 17005 36227 10917  9117 37725 28622 22167 45842
40405 51335 41356 42333 47216 28148 31568 32947 11080 49967 35202 35090
28267 40576 40736 44507 24235 21533 29577 16432 19881 40430 18177 36576
 9240  9017  2220 33928 10401  7686 13891 50146 43677 14567  1966 35959
10916 18645  2020 38648  1662 31692 13793 50890  4646  9914 52043 43291
49822 49018  5285  1740  5286 47722  7520  8834 14465 14185 16960 48868
21710 27563 48512 49278 10541 27881 51725  6451 34162 35770 42093 42423
 7344  8377 15543 10771 17706 16762 13048  4213 33706 35665  9516 36874
42475 21137 26559 48971 19253 25143  5041 32132  7899 25223 25209 25446
25448  7670 21058 35081 25952  5297  8851 21861 37197 48062 11861 11760
 2281 32400 24585 25476  3289 29304 23459 44885  7514 49840 11738  1729
 7676 28653 48270 27463 34271  2558 42310 41833  4365  4349 35528 33507
33571  9772 13578  3939 38936 43380 26850 19110 34326 42660 47671 26672
 2230 45123 23252 52156 21981 13619  4723  4724 48611 12284 12279  3418
21962 12429 12458 12434 41901 14810 51552 36720 38440 31920 29054 49440
48391 19379 17769 40467  3727  2620 21668 21598 13813 30286 17836 41459
52080 21589 43945 13672 36394  6401 52622 24534 12489 39608  1501 17319
17315 17317 17322 28939  4770 34994 34591 20550  5480 38883 18718 52345
13463 46476 16063 14786 13519 35454 35451 50719 35661 31462 41548 26439
17419 39481 36924 16531 51821 37045 10723 51895 24407 50679 20325 41625
21275 37656 51542 19144 12208 29764 18012 14612 14610 49881 16900 35432
35466 35153 39166 43304 25528 46116 36200 29232 29236  8363 26358 30287
18545 43489  7545  7550  7547  2867  2366 14140 45219 11621 26240 44873
43255 19473 50446 45224 16901 12432  8475 44805  8170 33356 47589  8174
 8178 35052 15384  9072 45222 45218  4798 26061 47929 45247 37287 20969
13635 13824  1847 42947  1805 49850 39087 24988 22606  5465  7458 20338
31207 38098 12431 19479  1298 26549  3863 27494 41814  7372 25911 43357
11073 18230 41674  3763  9279 46736 42432 35890  4745 39378 13195  3087
11669 21454  4085 11711   901 28858  3368  8090 48507 24488  9164 33465
13797 49407 47834 34016 48704 32900 38217  5175 24493  9568 32282 34203
11214 24496  9883 47831 39294 35079  4879  6928 38818 41463 26736 45117
14822 50999  6315  8999 38336 48599 32483 47822 44207 44591 44714 35095
22819 23970 20219 31192 25905  9617 39534 29863  4364 12000 20860 20858
20891 45029 41177 10719 21169 50033 14226 11816  7672 50236 24541 30468
28513 27790 52000 25498 35893 35900 41057 16067 38977 33789 33354  7033
16883 33765 33783  6997 33759  7029  7021 11515 44376 11516 11700 34839
12172 49396 51400  7048  5789 15476  8503 28382  8573 46000 22355 40545
32335 38475 15357 41523 42855  4127 15136 15186 17704 24289 42786 39616
 1952  9005  2016 30084  6698 14398 50946 47905 10467 33248 23900 46820
46377  4366  6194 47148 35345 40891
748:45465 13001 43886 45684 38673 39047 31054 19653 11213 12694 14257 38028
21641 31450 16537  9393  4925 44055 33653  1875 10506 26994 52679 13325
 3833 18680 41316 22057 23831 37933 32949 29136  8009 37217 47449 15761
30467 35643 11026 11028
749:51827 13279  3288  3305 47499 26762 36225 41314 19213  4412 50050 33943
25202 32373 32374 40342 44451 32572 26442  8930 52031 52029 46962 45143
34360 30569 21010 48853 10727
750: 9244 33190 11693 22812 45231 25876  1299 11969 10695 41503  7462 52176
11253 43734 43798  2729 28838 13735 24417
751:35786 47915 37979 39505  5421 22278 28625
752:43019 42034  9298  8796 25930  1931 50943 10263 26632   908 46451 15250
48665 44190 18079 35915 31443 19931 34104
753:36084  7468 35734 36631  4872 22898 42976 14604 42551  7046 41100  2194
 6066 12207 24696
754:31582 46357 38707 38709 15396 15424 49245  8968 31733 42753 40857 36545
39142 21581  7640  2618 40173 25336 37993 10673 47067 15428  3552 38733
46373 46362 41605
755:48144 50641  3725  1369 43563 33490 25538 25964 29283   759 20414 22060
18185 37860 39528 18054 20041 33273 13646 13643  7444 33275 28316 20660
33446 13536 25107  5061 37626 45740 48037 21248 13876 38898 28552 11035
32131 32199 22108 13710 28264 45628 48011 13412 11895 42003  3428   806
33301  9160  6156 36489 52566 20357 21828 33665 24443 29552 36712 52749
```

```
41326 11205  6783  6781 15371 19230  6785  6782 15402 15400 15373 24330
 1992  5714 21945 37378 37874 23356  1371 16140 34183 30035 44000 23205
20707 48485 49043 13309 40210 34250 12998 11872 52037 48613 48101 28084
51431 47876 45634 33448 39474 12956 51955  8737  6426 22754  9349  2948
51640 26554 50371 21166 22142 44516 12086 18521 19398 35921 38071  8374
26230  9070 44400 22699 52659  2121 23923 45803 37203 25411 51521 39894
 8680 24033 18809 44505 44509  6557 16865 26911  7911 37820 19129 15879
37132 48456 31098  4788 13896  6544 29835 38646 28260  2661  6916 46155
15725 11216 18310 12253 38634  5762 13569 33433 26477 36719 25369 47075
12006 31120 10261  9441 40843 27719  5550  6408 12558 30198 30285 29749
39560 36211 29464  1971 46984 15082 24659 47682 52691 27899 16250  8784
28113 37488 34986 11795 21337 26662 43529 43534 37246 33550 43449  8017
27001 47441 34787 17877 20993 14001 45069 24634 41509 52088 52726 35934
45692 14259  2998 48242 45987 26315 45238 43338 41748 34137 32956 11051
45199 45264 51717 15168 26414 43926 20909 18139 49561  5860 47346 20725
 5076 34444 12088 35617 37327 40356  8152  6491 33724  7266  8459 20590
21696 48864 48862 15223 11504 10521 23850 23848  4666 30398  9511 22742
22768 42258 50297 21624 27402 33666 18152 34766  5193 35252  6273 30306
14351 29738 18435 22307 32577 20274 20149 10958 43223 46711 17719 47014
51022 30174 44525  9803 15291 45188 40887   757 23950 28143 21699 22425
14803 42503 41527 26960 30574 47753  3346 20963  1674  2629  2633 43039
47504 22787 40072 50926 10478 23987 16302 41830 41832 45085  6687 34392
13668 13645 13625 13648 32218 41222 28628  9722 23098 23521  3372 33570
24893 23658 25495  2515 52526 10729 46881 34800  8724 50202 36003 23892
38785  4042 29415 32275 50281 19271 19189 22806 25532  6432 33010 10132
27777  5340   975 13421  7775 31543 50847 19705 18747 46840 31965  8333
 8330  8328 12107  4656 17796 43576  7275 10061  9692 47425 11338  9663
29370 43936 13679 37827  1765  1767 12067 24001 36943 21420 17382 50457
36002  1590  1372  4052 38764 33280 22813 38468 39808 33561 30402 27455
23434 48720 33185 20354 13549 28386 47794 17693   962  9719 19889 50608
46726 12128  7608  6980 32596 37250  3086 35496  9551 17185  2226 31679
46753 11359 29254 24810 17820 25761  8915  3695 33167 34354  9347 22744
39348 42277 44162 33865   756 48663 38152 33861 20074 48985 27648 35995
 8900 45343 17815  9690  5891 34756 35618 21929 44455 19084 48369 22852
32198 35381  2393  1370 16640  8776 46466 18735 22323 48090 52752  8579
40499 33797  7163 15679 13598 43535 32742 30235 41794 39718 16313 29279
45847   758 29498 10555 43136 45664 20519
756:48144  1369 43563 26706 25538   759 20414 39528 37860 20041 18054 33273
 7444 33275 28316 20660   755  4912 17673 21248 13876 38898 28552 11035
32131 22108 45628 13412 11895 42003 33301   806  9160  6156 52566 20357
21828 33665 24443 29552 36712 52749 11205 41326 24330  1992  5714 37378
 1371 34183 20707 49043 48485 40210 34250 12998 52037 28084 51431 47876
39474 12956 51955  8737  6426 22754  2948 51640 26554 22142 21166 44516
12086 18521 35921 38071  8374  9070 22699 52659 23923 45803 25411 37203
51521 34275 39894  8680 24033 18809 44505 44509  6557 16865 26911 37820
15879  4788 13896  6544 29835 38646  2661 28260  6916 46155 18310 15725
11216 12253 38634 13569 33433 26477 19178 36719 25369 47075 12006 10261
 9441 40843 27719 12558  6408 30285 39560 36211 29464 46984 24659 47682
52691 27899 16250 28113  8784 37488 34986 11795 43529 43534 37246 33550
43449 27001 47441  8017 14001 24634 41509 35934 52726 45692  2998 48242
45987 26315 45238 43338 41748 34137 37160 11051 42118 32956  5191 45199
45264 15168 26414 20909 43926 18139  5860 20725 47346  5076 34444 12088
35617 37327 40356  8152  6491 33724  7266  8459 21696 20590 11504 10521
23850 23848  4666 30398  9511 22742 46684 42258 50297 21624 27402 33666
18152 34766 35252  6273 30306 14351 29738 18435 32577 20274 10958 43223
 7764 17719 51022 44525  9803 45188 40887   757 28143 23950 21699 22425
14803 42503 26960 30574 47753 20963  1674  2629  2633 43039 47504 22787
40072 10478 50926 23987 16302 41830 41832 23634  6687 34392 23658 25495
23521 23098  9722 41222  8724 28628  3372 36003 24893 46022 33570 23892
38785  4042 29415 25210 50281 19189 19271   975 13421  6432 33010 22806
25532 10132 27777  5340 15672 31543  7775 31965  8330  8333  8328 12107
 4656 17796 43576 10061  7275  9692 47425  9663 11338 43936 13679 29370
```

```
37827  1765  1767 12067 24001  1590 38764  1372  4052 21420 17382 36943
50457 36002 33280 28386 48720 38468 17693 22813 18731 39808 33561 30402
27455 17185   962  9719 50608 46726  6980 19889 12128  7608 32596  9551
35496 37250 30386  3086  2226 31679 11359 34354  9347 22744 42277 39348
50088 44162 20074 48985 35995  8900 45343 17815  9690  5891 34756 35618
21929 44455 19084 48369  2393 35381  1370 16640 22323 46466 18735 50248
48090  8579 52752 40499 30905 33797  7163 15679 13598 43535 32742 30235
41794 39718 16313 29279 45847   758 29498 10555 20519 45664 33861 33865

757:48144  1369 43563 26706 25538   759 20414 39528 37860 20041 18054 33273
  7444 33275 28316 20660  5061 37626 45740  4912 17673   755 21248 13876
 38898 28552 11035 32131 17097 17076 32199 22108 45628 13412 11895 42003
   806 33301  9160  6156 52566 20357 21828 33665 24443 29552 36712 52749
 41326 11205 24330  1992  5714 21945 37378  1371 34183 20707 48485 49043
 13309 40210 34250 12998 52037 48613 28084 51431 47876 45634 39474 12956
 51955  8737  6426 22754  2948 51640 26554 22142 21166 44516 12086 18521
 35921 38071  8374  9070 22699 52659 23923 45803 25411 37203 34275 51521
 39894  8680 24033 18809 44505 44509  6557 16865 26911 37820 15879 37132
  4788  4787 13896  6544 29835 38646  2661 28260  6916 46155 18310 15725
 11216 12253 38634  5762 13569 33433 26477 19178 36719 25369 47075 12006
 31120 10261  9441 40843 27719 12558  6408 30285 29749 39560 36211 29464
 46984 24659 47682 52691 27899 16250 28113  8784 37488 34986 11795 43529
 43534 37246 33550 43449 27001 47441  8017 14001 24634 41509 52088 35934
 52726 45692  2998 48242 45987 26315 45238 43338 41748 34137 37160 11051
 42118 32956  5191 45199 45264 15168 26414 20909 18139 43926  5860 20725
 47346  5076 34444 12088 35617 37327 40356  8152  6491  7266 33724  8459
 20590 21696 11504 10521 23850 23848  4666 30398  9511 22742 46684 42258
 50297 21624 27402 33666 18152 34766 35252  6273 30306 14351 29738 18435
 22307 32577 20274 20149 10958 43223  7764 17719 51022 44525  9803 45188
 40887 28143 23950 21699 22425 14803 42503 26960 30574 47753 20963  1674
  2629  2633 43039 47504 22787 40072 10478 50926 23987 16302 41830 41832
 23634  6687 34392 23658 25495  8724 36003 23521  9722 41222 23098 28628
  3372 46022 24893 33570 23892 38785  4042 29415 25210 50281 19271 19189
   975 13421 25532  6432 33010 22806 27777 10132 15672  5340 31543  7775
 19705 31965  8333  8330  8328 12107  4656 17796 43576 10061  7275  9692
 47425  9663 11338 29370 43936 13679 37827  1765  1767 12067 24001  1590
 38764  1372  4052 21420 17382 36943 50457 36002 33280 48720 28386 38468
 33561 22813 39808 17693 30402 18731 27455 17185  6980  7608 50608 46726
 19889   962  9719 12128 32596  9551 35496 30386 37250  3086  2226 31679
 11359 34354  9347 22744 42277 39348 50088 44162 33865   756 48663 33861
 38152 20074 48985 35995  8900 45343 17815  9690  5891 34756 35618 21929
 44455 19084 48369  2393 35381  1370 16640 22323 46466 18735 50248 48090
 52752  8579 40499 30905 33797  7163 15679 13598 43535 32742 30235 41794
 39718 16313 29279 45847   758 29498 14066 10555 20519 45664
758:48144 50641  3725  1369 43563 26706 25538 25964   759 20414 18185 39528
 37860 20041 18054 33273  7444 33275 28316 20660 33446 37626 45740  4912
 17673   755 21248 13876 38898 28552 11035 32131 17097 22108 45628 48011
 13412 11895 42003   806 33301  9160  6156 52566 20357 21828 33665 24443
 29552 36712 52749 41326 11205 24330  1992  5714 21945 37378 37874 23356
  1371 16140 34183 30035 44000 20707 13309 48485 49043 40210 34250 12998
 11872 52037 48613 48101 28084 51431 47876 39474 12956 51955  8737  6426
 22754  2948 51640 26554 22142 21166 44516 12086 18521 35921 38071  8374
  9070 22699 52659 42313  2121 23923 45803 25411 37203 34275 51521 39894
  8680 24033 18809 44505 44509  6557 16865 26911 37820 15879 48456  4788
 13896  6544 29835 38646  2661 28260  6916 46155 18310 11216 15725 12253
 38634  5762 13569 33433 19178 26477 36719 25369 47075 12006 31120 10261
  9441 40843 27719 12558  6408 30285 29749 39560 36211 29464 46984 24659
 47682 52691 27899 16250 28113  8784 37488 34986 11795 21337 43529 43534
 37246 33550 43449 27001 47441  8017 14001 45069 24634 41509 35934 52726
 45692  2998 48242 45987 26315 45238 43338 41748 34137 37160 42118 11051
 32956  5191 45199 45264 15168 26414 20909 43926 18139  5860 20725 47346
  5076 34444 12088 35617 37327 40356  8152  6491  7266 33724  8459 21696
```

```
       20590 11504 10521 23850 23848  4666 30398  9511 22742 46684 42258 50297
       21624 27402 33666 18152 34766 35252  6273 30306 14351 29738 18435 22307
       32577 20274 20149 10958 43223  7764 17719 51022 44525  9803 45188 40887
         757 28143 23950 21699 22425 14803 42503 26960 30574 47753  3346 20963
        1674  2629  2633 43039 47504 22787 40072 10478 50926 23987 16302 41830
       41832 23634  6687 34392 23658 25495  8724 23098 41222  9722 23521 36003
       28628  3372 24893 46022 33570 23892 38785  4042 29415 25210 50281 19271
       19189   975 13421 22806 25532  6432 33010 10132 27777  5340 15672  7775
       31543 19705 31965  8333  8328  8330 12107  4656 17796 43576 10061  7275
        9692 47425  9663 11338 43936 29370 13679 37827  1767  1765 12067 24001
        1590  1372  4052 38764 17382 21420 36943 50457 36002 33280 48720 28386
       38468 22813 39808 33561 30402 17693 18731 27455 23434 17185  6980 46726
       50608   962  9719 12128 19889  7608 32596  9551 35496 37250 30386  3086
        2226 31679 11359 34354  9347 22744 42277 39348 50088 44162 38152   756
       48663 33865 33861 20074 48985 35995  8900 45343 17815  9690  5891 34756
       35618 21929 44455 19084 48369  2393 35381  1370 16640 22323 46466 18735
       50248 48090 52752  8579 40499 30905 33797  7163 15679 13598 43535 32742
       30235 41794 39718 16313 29279 45847 14066 10555 20519 45664
759:48144  1369 43563 26706 25538 18185 37860 39528 18054 20041 33273 13646
       13643  7444 33275 28316 20660 33446 13536  4912 17673   755 48037 21248
       13876 38898 28552 11035 32131 22108 13710 45628 48011 13412 11895 42003
       33301  9160   806  6156  6155 52566 20357 21828 33665 24443 29552 36712
       52749 41326 11205 24330  1992  5714 21945 37378  1371 34183 30035 44000
       20707 48485 49043 13309 40210 34250 12998 52037 48613 28084 51431 47876
       39474 12956 51955  8737  6426 22754  2948 51640 26554 21166 22142 44516
       12086 18521 35921 38071  8374  9070 22699 52659 42313  2121 23923 45803
       37203 25411 34275 51521 52389 39894  8680 24033 18809 44505 44509  6557
       16865 26911 37820 15879 37132  4788 13896  6544 29835 38646  2661 28260
        6916 46155 18310 15725 11216 12253 38634  5762 13569 33433 19178 26477
       36719 25369 47075 12006 31120 10261  9441 40843 27719  6408 12558 30285
       29749 39560 36211 29464 46984 24659 47682 52691 27899 16250  8784 28113
       37488 34986 11795 26662 43529 43534 37246 33550 43449  8017 27001 47441
       17877 20993 14001 24634 41509 52088 52726 35934 45692  2998 48242 45987
       26315 45238 43338 41748 34137 42118 32956 11051  5191 37160 45199 45264
       15168 26414 43926 18139 20909  5860 47346 20725  5076 34444 12088 35617
       37327 40356  6491  8152  7266 33724  8459 21696 20590 48864 11504 10521
       23850 23848  4666 30398  9511 22742 22768 42258 46684 43271 50297 21624
       27402 33666 18152 34766  5193 35252  6273 30306 14351 29738 18435 22307
       32577 20274 20149 10958 43223 17719 47014 51022 30174 44525  9803 45188
       40887   757 23950 28143 21699 22425 14803 42503 26960 30574 47753  3346
       20963  1674  2629  2633 43039 47504 22787 40072 50926 10478 23987 16302
       41830 41832 23634 45085  6687 34392 13645 13625 41222  9722 23098 23521
       28628  3372 24893 33570 23658 25495  2515 52526 46881 34800 10729  8724
       36003 23892 38785  4042 29415 32275 50281 19271 19189  6432 33010 22806
       25532 10132 27777  5340   975 13421  7775 31543 19705 50847 18747 46840
       31965  8330  8333  8328 12107  4656 17796 43576  7275 10061  9692 47425
       11338  9663 29370 43936 13679 37827  1765  1767 12067 24001 21420 17382
       36943 50457 36002  1590 38764  1372  4052 33280 38468 22813 39808 33561
       30402 27455 48720 23434 33185 20354 13549 47794 28386  3086  6980  7608
       46726   962 50608  9719 19889 12128 32596 37250  9551 35496  2226 17185
       31679 11359 46753 29254 34354  9347 22744 39348 42277 44162 38152   756
       48663 33865 33861 20074 48985 27648 35995  8900 45343 17815  9690  5891
       34756 13673 35618 21929 19084 48369 35381  2393  1370 16640 46466 18735
       22323 48090 50248 22285 52752  8579 40499 33797  7163 15679 13598 43535
       32742 30235 41794 39718 16313 29279 45847   758 29498 10555 45664 20519

760: 9034  7685 24938 15001 41657 44445 49339 17134 24445 28677 22150 13719
       32164 24354 38431 33870 26512 13115 22141 21453  9458  9452  9425 10037
       37817 18025 40719 40720 40724 40692 40716 40744 40740 24483 24456 23727
       24494 23777 24491 24509 23751 23745 23800 26248 26359 26391 47849 48080
       48087 48031 47164 47840 47835 48085 47969 48083 47943 47868 48662 48039
       47160 48035 47842 47162 48007 47863 48013 25564 22073 44961  1819 13497
```

EP 2 484 769 A2

```
22088 23659 25283 25246 26161 25333 23742 25326 30980 36338 36419 36314
31007 30960 30956 30961 36341 36387 30952 36390 36312 36290 31004 36317
47068 36420 37421 30438  9068 18046 45926 24861 45435 43938 11136 52481
25923 26462 20467 31551 32925 27879 40208 40112  6125 28673 16918  6481
46835 46837 30729 30754 40351 37332 22212 27989  8155 50101 25380 13200
46111 52472 52469 46113 14114 20787 48214 17944 20505 46475 27904 35308
31311  6064 10396 10395 50083 49348 33918 18093 23262 45425 33965  6248
52024 31494 32394 35899 19844 28137 28813 26142 47971 32466 38637 10257
 9226  4888 34484 34485 15245 34461 49426  6580 43472  3248 47995 20547
17402 18284 29388  7916  3436 28326 16652 11996 16653 51457 34862 22683
37344 15922 10846 43577 34652 12101 12098 15045 38225 43565 34241 36627
33386 26158 45745  3135 26326 22784 34783  7659 46934 26163 16728 44934
38294 39945 39135 16028  5137 15585 29649 18246 29651 14821  7938 52370
36295 20625 14513  9262 26933 33487 28518 25839 21862 51728 22316 52501
49810 42388 48446  9131 45862 27051 32482 17084 39193 52055 13391  6231
21223 41064 35166 20413 20410 52532 42567 48879 22268 17894  5336 38562
39448 37106 31185  9243 17963 27255 48168 45989 48324 39297 43988 11005
33719 21104 15179 18696 25798 14202 35001  2161 49183 10741 14806  5808
50543  7739  8797 45201 13247 14395 31651 49226 17525 42883 37642 38527
13729 13727 34726 37505 37500 23091 23055 46660  3260 29133 29130  5267
11756 32327 12980 15002 36965  2931 34953  9370 47938 46844 44189 27076
26693 30881 15435 30296 41736 44065  1965 27994 32342 41474 34227 24232
45770 49399 28930 30656 22490 17715 17712 18242 28808  5168 15471 39404
25395 28118 14448 52763 52765 20906 35619 16677 48380 39063 13992 52005
18234 44269 45796  2697 52486 43832 42695 35800 36159 18232  4178 12028
12026 23586 41385 20835 18950 16327  9520  3852 28131  9698 30944 30690
 3076 24148 35535 50848 45937 25614 13197 14097 16984 16986 16630  7434
19061  8821 38890 40265 52149 46131 51018 17114 40925 27137 27139 22049
26942 42104 44366 25690  4113 23875 44806 20172 34209  8693  2555 45930
15909 19389 29572 38075 37212  9940 33540 37871 33836  2437  8512  8538
43808 10491 10490 23006 23004 18986 30872 35991 18408 46375 36597 30279
25014 27632 21547 23505 22840 22930 23566 22891 22864 23590 22841 23561
22925 22883 22856 40036 14430 27299 50428 28652 27710  9044 21895 37205
19363 20117 49554 33833 38942  2541 19783 37331 27467 35816 39569 32250
27519 27518 44836 47954 46310 40232 10928 35863 19980 22824 43858 48699
49376 29195 19902 28491 50485 38353 39959 39957 15355 36554 42513 14440
49457 15202 48884 10378 39828 37714 50063 35029 42678 40378 24119 25983
52061 20665 20052  5226 14307 11880 45109 46190 48756 49504  9853 47726
21393 18949 31153 36356 42646 17009  9500 20307  2195 28585 27275  8587
 4826 51405  5842 25253  2713 51870 34177 41579 49356 27220  8614 14325
 9462 37225 38789  8557 13436 15822 13609 30109 34368  6485  5877 30625
15175 50211 16509 49011 51788 42382 43857 43199 43856 48216 13888 51684
15912 26423 18617 48097 46272 51601 17910 25829 34483 23835 32604 43212
37502 16485 15356 12602 42766 20365 27217 43486  9585 39472  8010 26578
29785 42103 34990 47034 49281 20363 47608 13006  7522 46624 26623  2761
 2763 40176 15381 28485 30074 18391 45908 43588 13596 20820 50514 19822
22981 22980 40486 36794 36412  4445 43995 28737 25430 36552  4611 48839
38288 36281 46640 45540 35541 20013 22998 46108 12936  6203 17650 20529
25970 49971 42449 13628
761: 9034  7685 24938 15001 41657 44445 49339 17134 24445 28677 22150 13719
32164 24354 38431 33870 26512 13115 22141 21453  9458  9452  9425 10037
37817 18025 40719 40720 40724 40692 40716 40744 40740 24483 24456 23727
24494 23777 24491 24509 23751 23745 23800 26248 26359 26391 47849 48080
48087 48031 47164 47840 47835 48085 47969 48083 47943 47868 48662 48039
47160 48035 47842 47162 48007 47863 48013 25564 22073 44961  1819 13497
22088 23659 25283 25246 26161 25333 23742 25326 30980 36338 36419 36314
31007 30960 30956 30961 36341 36387 30952 36390 36312 36290 31004 36317
47068 36420 37421 30438  9068 18046 45926 24861 45435 43938 11136 52481
25923 26462 20467 31551 32925 27879 40208 40112  6125 28673 16918  6481
46835 46837 30729 30754 40351 37332 22212 27989  8155 50101 25380 13200
46111 52472 52469 46113 14114 20787 48214 17944 20505 46475 27904 35308
31311  6064 10396 10395 50083 49348 33918 18093 23262 45425 33965  6248
```

98

```
   52024 31494 32394 35899 19844 28137 28813 26142 47971 32466 38637 10257
    9226  4888 34484 34485 15245 34461 49426  6580 43472  3248 47995 20547
   17402 18284 29388  7916  3436 28326 16652 11996 16653 51457 34862 22683
   37344 15922 10846 43577 34652 12101 12098 15045 38225 43565 34241 36627
   33386 26158 45745  3135 26326 22784 34783  7659 46934 26163 16728 44934
   38294 39945 39135 16028  5137 15585 29649 18246 29651 14821  7938 52370
   36295 20625 14513  9262 26933 33487 28518 25839 21862 51728 22316 52501
   49810 42388 48446  9131 45862 27051 32482 17084 39193 52055 13391  6231
   21223 41064 35166 20413 20410 52532 42567 48879 22268 17894  5336 38562
   39448 37106 31185  9243 17963 27255 48168 45989 48324 39297 43988 11005
   33719 21104 15179 18696 25798 14202 35001  2161 49183 10741 14806  5808
   50543  7739  8797 45201 13247 14395 31651 49226 17525 42883 37642 38527
   13729 13727 34726 37505 37500 23091 23055 46660  3260 29133 29130  5267
   11756 32327 12980 15002 36965  2931 34953  9370 47938 46844 44189 27076
   26693 30881 15435 30296 41736 44065  1965 27994 32342 41474 34227 24232
   45770 49399 28930 30656 22490 17715 17712 18242 28808  5168 15471 39404
   25395 28118 14448 52763 52765 20906 35619 16677 48380 39063 13992 52005
   18234 44269 45796  2697 52486 43832 42695 35800 36159 18232  4178 12028
   12026 23586 41385 20835 18950 16327  9520  3852 28131  9698 30944 30690
    3076 24148 35535 50848 45937 25614 13197 14097 16984 16986 16630  7434
   19061  8821 38890 40265 52149 46131 51018 17114 40925 27137 27139 22049
   26942 42104 44366 25690  4113 23875 44806 20172 34209  8693  2555 45930
   15909 19389 29572 38075 37212  9940 33540 37871 33836  2437  8512  8538
   43808 10491 10490 23006 23004 18986 30872 35991 18408 46375 36597 30279
   25014 27632 21547 23505 22840 22930 23566 22891 22864 23590 22841 23561
   22925 22883 22856 40036 14430 27299 50428 28652 27710  9044 21895 37205
   19363 20117 49554 33833 38942  2541 19783 37331 27467 35816 39569 32250
   27519 27518 44836 47954 46310 40232 10928 35863 19980 22824 43858 48699
   49376 29195 19902 28491 50485 38353 39959 39957 15355 36554 42513 14440
   49457 15202 48884 10378 39828 37714 50063 35029 42678 40378 24119 25983
   52061 20665 20052  5226 14307 11880 45109 46190 48756 49504  9853 47726
   21393 18949 31153 36356 42646 17009  9500 20307  2195 28585 27275  8587
    4826 51405  5842 25253  2713 51870 34177 41579 49356 27220  8614 14325
    9462 37225 38789  8557 13436 15822 13609 30109 34368  6485  5877 30625
   15175 50211 16509 49011 51788 42382 43857 43199 43856 48216 13888 51684
   15912 26423 18617 48097 46272 51601 17910 25829 34483 23835 32604 43212
   37502 16485 15356 12602 42766 20365 27217 43486  9585 39472  8010 26578
   29785 42103 34990 47034 49281 20363 47608 13006  7522 46624 26623  2761
    2763 40176 15381 28485 30074 18391 45908 43588 13596 20820 50514 19822
   22981 22980 40486 36794 36412  4445 43995 28737 25430 36552  4611 48839
   38288 36281 46640 45540 35541 20013 22998 46108 12936  6203 17650 20529
   25970 49971 42449 13628
762:18514 51228 51233 51210 51202 51207 38022 26021  9536 49682 49659 49681
   49657 15798 39833  8005  8052  8054  8056 30738 51308 51338 44263 17897
   14735 15462 28026 41033 41035 36976 24499 14741 15464 33409  8356 14707
   42329 44107 26686 50192 14065  1722 29667 24735 37834 36982 27664 15323
   48161  5811 32709 13537 44230 43929  9056  7114 47320 45972  4199  4880
    4251 32433 41006 41004 32675 41631 50290 34145  4242 49352 51176 24549
   23926 16650 49037 45970 38182 49684 23111  5964 22808 22830 18689  1208
   46299 17160 22953 22944 22950 14744 15465 20384 49865 49837 38183 33236
   33222 33221 33218 21176
763:25916 50750 48752 49039  3339 36252 47836 39572  9829 42936 25319 41018
   40987 40984 41011 41013 41050 40977 41082 43071 43069 43054 43050 41952
   42002 41078 41052 41081 42336 38402 41780 41798 41799 41777 41775 45656
   46710 40158 15731  4998 43026  4953 41752 41749 41751 41745 40136 40129
   40159 23404 38951 38287  4215 41652 41627 41647 25421 25416 25418 39861
   39883 39884 39904 39882  3296  3294  3312 25321 34891 50568 17923 51567
   25315 25309 13837  3317  3313  3319  3233  3235  3240  3261  3269  3272
   36596 12519 13373 33637 12399 34266 34264 12403 12420 12547 12421 12575
   12577 33589 12425 12452 33616 12608 33609 12615 34297 12475 33613 12617
   33519 12481 12636 13339 13341 12497 12498 12499 12515 23551 36636 22780
   22758 19249 32461 13248 30288 36734  4040 45638 30212 17048 25474 39747
```

```
48063  9645 36981 16121 16122 49451 44461 39626  3281 21328 34343  9474
41168 16836  2994 37351 37264 34872 36527 10354  2302 51489 47200 30607
10357 44489 41121 28284 50797  9357 48249 48248 48267 48265 46057 51027
20862 47455 40726 43944 29135 10828 29886  9321 41626 41602 41624 33324
24035 36232 25687 37164  8997  5087 45571  1997 45411 42896 21180 29157
 2375 37856 27094 27998 27997 14909 48615  9090 45733 14387 31630 23467
10405 41787 37946 19808 13057 48843 24646 19185 11659 27778 32203 33274
14992 21153 40257 19194 18429 24131 39848 39849  2527 43493 10203 45601
16333 38167 42011 20393 10793 23109 46518 30662 16555 25718 42586 52722
 3506 37174 37172 23793 36786 16398 41085 49532  3320 40101 40105 31438
 3647 29981 15860  2535 39880  8266 46129  4176  5831 25710  1820 22107
22105 33534 30912 40377 10975 42620 42819 41045 40311 32600  4976  4973
41589 39385 23148 23441 32643 41599 41593 41598  8910  8909 42907 42910
38693 27152 41658 41655 41697 41695 41694 41663 39389  4980 21737 21741
22641 10003 10954 35687 36380 52675 25679 27157 41088 37424  6501 47681
49663  6458 27943 11674 17152 48913  8923 21332 40221  5646  5623  3891
21827 49706 14067 36239 39307 26410 49704 44948 33413 50366 21226 42070
46032  6537 49027 41240 40672 40218 40261  1009 45181 51800 52339 22311
29146 17915 47399 41384 38096 39369 22545 44905 37237 15700 41650 15518
52342 26353 26384 26350 35199  3513  6853 12769 44493 46600  4939  4940
41849 35099  6178 24368  7186  5751  5494 36108 36111  4657  5491 40099
 5749 42496  3497  3471  2438 27222 51349 30836 39684 37095 26618 29085
18720 42069 42820  4944 43025 42046 49088 12215 33747 33750 21102  3569
12237 13370 12517 40069  3264 40194 40186 35336  7957 11619  1007 42570
38250 33385 47688  3969 16259 32654 20661 49531 30582 23694 23701 23698
23695 43624 39651 31327 27244 27973 27966 24640  3247 44926 19979 11899
 2749 22995 33536  5119 26691 44727 16071 13759 16180 47861 26689 39341
35014 31961 40528  4608 13510 41287 32608 48709 19947 34794  1008 31912
11042 13166  5313 26117 21319 32467 28535 27164 28832 39633 40782  7357
35706 30353  2723 50402 41369 40867  4032  4465 12491 12159 16627 11385
30516  1980 15448 27489 47593 40262 40071 40223  8876  8881  8882  8878
34430 32623 25360 25381 25384  8873  8858  8853  8856  4947 29118 41731
29166 29164 46358 46360 40981 21435 21407 25359 25353 25357  5690  9256
 9257 40331 40341 42826 41087 40333 42823 42843 41755 41772 41774 32579
32619 25388 25412 25390 25414 32502 31624 31623 43047 20557 32624  4943
 4951  4969 41900  4977  3298 26534 19270 12642 12482 33546 13376 23407
13796 22447 10947 45518 28715 42492 41805 42490 41803 39408 42488 41801
31578 23548 14457 25134 39857 25705 36702 34122  4534 25683 44117 32629
23401  3258 40972 40337 42044 43044 42048 42033 41924 41922 42037 41999
44133 44161 44158 44141 44138 41966 41956 44136 44130 41962 41960 41989
41995 41993 41926 41927 37507 41307 41040 43022 15493 25391 40156 32173
40131  3237  5849 21419 40190 35924  3665
764: 5239  5236  5232  5235 24744 11215 23045  9311 45344 34170 22936 34174
16651 17350 17135 38575  3635 16222 46652 52022 17346 37366  8159 22302
22296 40288 13047 13049 40289 40287 37562 10023 10772  2578 50637 23223
27675 17705 23036 23037 41827 38725 20526  6078 32530 32551 14399 48449
45366 10459 45529 10368 25566 36619 36622 38364 44143 51549 40370  9493
51879 13224 26369 36894 24410 45626 29492 29420 51590 43566 35977 19347
 765  7013
765: 5239  5236  5232  5235 24744 11215 23045  9311 45344 34170 22936 34174
16651 17350 17135 38575 31057  3635 16222 46652 52022 17346 37366  8159
22302 22296 40288 13047 13049 40289 40287 37562 10023 10772  2578 50637
23223 27675 17705  4189 23036 23037 41827 38725 20526  6078 32530 32551
14399 48449 45366 10459 36619 36622 38364 44143 51549 40370  9493 51879
13224 26369 36894 29138 24410 45626 51590 43566 35977 40598 19347  1720
 766  7013   764
766: 5239  5236  5232  5235 24744 11215 23045  9311 45344 34170 22936 34174
16651 17350 17135 38575  3635 16222 46652 52022 17346 37366  8159 22302
22296 40288 13047 13049 40289 40287 37562 10023 10772  2578 50637 23223
27675 17705 23036 23037 41827 38725 20526  6078 32530 32551 14399 48449
45366 10459 10368 25566 36619 36622 38364 44143 51549 40370  9493 51879
13224 26369 36894 24410 45626 29492 29420 51590 43566 35977 19347   765
```

```
        7013    764
767: 4443 12712 49432 27666 25987  3211 49363 36391 29373  3811 31569  6267
     28231 27108 29510 19930 12428 48789  7089  7331  1097 14496 37469 37476
     45704  9000 27316 35070  7440 40436 46614 12381 50118
768:49065  9683 20128 44528 46346  4545 24463 14489  6048 46818 36932 25582
     33593 28130 37402 35119 48718 31782 50375 49666 50043 19894 20127 30670
     39971 34426 12523 33729 43413 43414 46752 39940 33128 41968  7612 19628

769: 1944 46367  2447 21532 12165 37609 22098 20126  6976 40783 48319 10140
     41859 46776 48406 49091 44199 18441 23598 23611 45658 52114  7719 40841
      2241 40429 31536 24303 20225  8738 51869  8740  4333 13634  5260 12459
     46109 32408 17826 18854  1940 21595 15113 48903 37611 27205  8741 41559
     23493 51050 14327 26880 47261 36906  4072 31416 40452  5871 35389 31103
     16970 30672 51377 26673 47919  5207  8655 47921 33912 34691 25895 51387
     30261  6336 49029 10128 36439 11577 43078 12893 30524  6489  6454  2878
     46729 45975 20741 19990 12507 52497 27913 24687 50988 23984 21178 26132
      5933  5926  2263 50379  3105 29654  2700 23387 47472 11036 36255  9285
     36460 25053  9308 23844  3304 28546 46637 50183 26587 17539 44097 11886
     47679 19159 50363 33705 28559  5070 50830 24670 47350 50843 14600 28524
     13802 14795 25080 34029  4647 46556 14441 30938 37572 29942 11570  9501
     35885 39966 24421  4995 17215 25782 32375 44829 49184 47570 45227 49718
     37706 11818  5945 23699 42384 10185  8688 51820 39244  5075 42437 46580
      5847 13350 19140 30632 49749 25522 47687 14256 12493  9170 16215 50062
      5586 20055 48396 40714 48398 26299 47854 32260 14234 48271 37064  4565
     29500  6157 12317 25851 14400 48894 12243 47081 13477 27269 41387 14825
     41388  3854 20721 40968 25888 21020 40544 42147 37667 49354 28836 42949
     42979 44874 46315 12750 47225 33917 25693  5085 25694  9789 36897 52269
      7896 31738 36630 50399 20405 45487  4207 43629 43630 17509 22725 25095
     34847 16898 16859 32546 16375 43439 43442  7130  7160 13693 46695 26738
     30277  6804 41727 41728 18438  1927 39373 17934 26280 47360 31991 23059
     36556  7384  8025 26427 12007 42562 13071  3380 35351 39912 41706 33659
     12326 25094  7542  9466 49023  8067 42203 24945 51560 38851 37593 11831
      8989 50618 37935 50453 33580 19406 36128 42451 25871 16262  5048  5043
     42454 42353 38714 42374  2652  5049 19432  2649  5672 52464 41061 35414
      7698  4560 37121 25591  7875 42473 35178 28638 25126 51010 17500 42021
     40840 27160 52155 41948 16595 18158 17957 21926  9608  2523 37747  8625
     35769 27024 29069 44178 41117 25120  9681 36513 19464 26948  6905  1570
      3770 48263 33692 13520 28299 13013 32821 31616 34724  7252 16924  9344
     30266 34972 30290  8616  9459 28458  1855 17522 16763 33738 41610 14029
     14031 12111 45108 37518 51481 27744 25996 27506 43129 43012 14318 38157

770:25436 39606 29553 42635 21505  4988 43647 21807 19869 50270 40988 43060
     24441  9522 48737 43141 14359 17985 42224 47203 43341 24040 48921 27672
     21068 39693 46440  5089 47443  9724 51791 50275 25061  5593 42309  4595
     19613 49563 24209 20857 21776 33106 33846 13070 39251 12302 19118 35424
     31352 19176 12315 25012 47862 38142 40920 16608 41091 50326 49402 32159
     43600 18670 19793 40392 29800 11149 30270 15075 22743 22657 47447 44820
     25600 17137 31467 22420 33175 12363 29814 10268 47192 14238 11710 14147
     18424 10129  8040 42120 46996 16902 25082 18136  6914 35275  5221 35876
      9192 11893 28360 23165  4321 25577  8906 18151 43279  1951 33119  6935
     16512 41987 36401 15511 37597 45705 13747 19423 34778  5497  9857  7061
     51106 17668 27902 44883 43885 34176 18338 18188 37672 21647 18089 36102
     28107 41779  7459  5163  4654 31269  3751 34790 20600 29009 48046  8018
     34462 23403 42829 41164 16816 30202 12072 48952 13515 24812 50764 39350
      4703 12299 41609 47703 42563 16646 41422 41383  9821 10664 42404 37957
     50978 39638 39671 37522 27599 39898 34603 12909 19739 14059 39295
771: 9296  5755 35395 36332 36849 14130 26990 30365  2825 50942 26571  8098
     39075 33373 38368 23287 38305 12021 12005 14856  8317  8318  8335 26072
     49605 29697 38007 34754 40727 23801 13508 28114 34875 33910 32458 20144
     12592 18393 43389 43170 39790 24625 23858 20797 20031 32436 28917 48111
     15440 52279  5113  9081 19431  4891 37676 28348 19112 24144 21238 47547
     47077 52263 35514 16671 27443 48027  2676 13615 14686 35065 52206 47605
```

```
       47015 29162
772:34812  6787  7845 39764 19872 50293 27954 27501  8709 32465 39111 48956
      14919 49297 32733 48339 23755 47090 50354 17932 22603  3923 19576 43549
      24649 52564 15127 28230  2172  8438 31118 30240 29925 25189 49197  7261
      22048 15054 12578 16880 35511 14086 28820 23713 19707 31168 41505  2900
      43305  6938 15793 23143 33596 40635  6340 30984 47048 49894 29022  3134
      49576 27363 12146 43671 26605 41592 40886 46395 25765 25884 47902 15622
      42956 31983 38232 13707 23260 15615  5475 51218  8550 33710 38371 43462
      39585 27617  2485 37004 37007 39550 52696 52697  6532 46975 28732 28729
      40157  2027  2029 19168 19337 50030 35870 22390 28427 27418 11514 30541
      18573 45423 41819 18702 12563 48394  3156 37532 47375 47416 33428 13452
      25494  9615 28074 34768 43147 35477  4076 47410 17442 37807  4081 24199
      41189 39712 37133 10299 20048 39717 43225  8689 51012 44335 20147 52261
      26607 22501 50133 13176  7991 41402 30587 19788 50769 17867 16500  6830
       9120  5760  5743 41380 50153 34081 45159
773:14285 43252 38649 11722 25997 35377 25486  4212 23379 52252  7578 15387
      42809 14833 31734 31739 16077 18497  4640 12962  8537 37628 26433 38472
      14060 32919 24442 25487 10242 17270  7416 27273 25639 40862 40861 39636
      23518 41305 38009 43485 39221 31386 12921  6322 30652 45457 13267 24086
      12582 39367 42176 42369 42355 42350 42358 42183 42181 42174 15146 34028
      17022 33147 30578 18688 17980 50638 26765 16360 45852 30260 50078 26932
      32032 25685  8714 42328 32989 27524 27521 27523 27520 18691 43087  4475
      33044 30203 36477 35942 27427 42529 21261 48088  6241 44315 38558  4967
      29477 22384 20463 38090 42438 48504 26642 46042 16590 23339 43914 15640
      13890 44211 42873 42877  8585 36603 42207 30008 29847 42558 10456  9302
      10100 15649 16750 32566 39023 35659 37891 25892 25893 50040  1500 10231
      44540 41937  2078 51275 40517 22580 20022 21039  5555  4710 31860 20814
      31222 46699 47579 41483 17399  5395 52412 19750 44157 32673 26459  6682
      10814 50582 46876 45406 32219 10468  2902 11464 16961 20394 17110 32959
      46330 40904 28008 44129 13034 33798 45650 39217 38382 42632 28322 45433
      39260  5528  4317 50925  3280 36458 18709 29242  6865 11382 45267 12199
       6226 35499  7065 51453 19075 26656 23235  8654 31368  8597 50304 48115
      11681  2015 47424  6488 25786 51014 49683 49692 33197 33203 34870 27319
      31866  9035 22847  2896 22811 40899 19463 43587 13690 18844 13728 38391
      44503 48531 12310 42215  3219 33315 43675 18365 18513 30469 27230  3835
       3840  1664 12289 50878 20899 52429 20936 17680  5364 19718 47743 35979
      36838 24027 48048 50670 27517 20486 14427 41378 42721 42510 24775 49714
      43792  1924 52596 52599 19438 19437 49665 40124 35806 14730 42049 46099
      47869 47005  3057 49261 22395 23355 46232 32783  1535 31104  7649 31033
       4380 21519 23933 44693 12697  3664 36242  7113 11590 19226 40608 47584
      24608 49859 30110 28243 50874 31599 25402 39743 46687 45137  6968  6127
       8452 29653 28540 43429 24123 13744 26995 34572 26417 13133 20462 29089
      50887 43753 31980 19853 17879 27821 29134 41862 36748 30549 42770 51854
       3125 49653 30832 14028 14943 35818 42156 37575 37751 29086 52115 50246
      33382 16072 11389 42363 51028 21461 43146 32104 20757 39512 39513 40121
      39514  7513  3423 39508 39506 46417 10275 47401 46389 51029 10325 15176
       3069 10274 10289  6788 10313 32089 48939 29077 32921 29849 15140 15038
      23329 49534  3672  3039 42659 49541  3767 39827 52343 51354  6389 20555
      30026 48030 34439 23821  5419 17470 36001  6621 32096 36080 14767 18491
      18493 17851 38119  6875 11002 26895  7843 15766 30119 24480  7411 33379
      13101 31251 16036 50585 35034 29124  4908 41352 33491 12368 42901 45738
       3813 50550 46329 25485 10245 10381 35159 18926 51324 49353 42304 49076
      46662 42621 14702 32077  9794 14742 10565 44365 36500 19946 43935 49017
      19798 41043 29526 18490 22209 21023 32216 13141 13069 41054 42507 36974
      37044  3399 20793 20796  3420  7541  7562 49047 42814  7998 45004  6784
      47036 12541 50347  8390 31280  1537 30902 36291 13443 10983 22170 50591
      25052 43068 23438 43726 20018 32288  1593 36997 36091 23666 52374 24213
      47297  4921 34197 49750 42291 15138 15717 11522 10284 10319 32061 27825
      47415 47436 47432 32095 10246 10249 10279 32086 32064 24028 32091 32063
       8153 20790 32228 51618 22134 48855 26923 12561 12516 46287  5359 16779
      47076 16539 42377 44234 24161 15112 15115 12357 39032 22542 38827 15169
      15173  3959 18995 41537 19026 18998 18976 19025 18996 18994 18973 18965
```

```
        18943 18968 19000 37417 15219 43145 40889
    774:22967  4957  6878 15989 42962 24743 50697 35258 35251 25347 34498 35285
        32547 34473 34451 34477 34475 34527 34593 34564 34562 34535 34474 34478
        16000 26793  4818 44716 23278  7741 26125 45538 28697  8831 46077 22200
        11165 28531 18821 51522 13655  1908 39494 23293  2429 30280 51774 41234
         9625 19067 23791 22389 18752  4430 45126 25708 23354 50117  1507 18363
        19572 16563  3866 51564 30236 42072 48927 28517  6001 50567 20784 51436
        23507 49158 38806 29256 29259 29261 50171 12074 38235 18506 10282  7111
        37967 23479  8247 30994 24369  3408 20800  2385 11321 19513 13734 39379
        31621 52335 38179 11066  5828  9203 27608 14572 15436 52152 51191 52736
        12951 29893 28268 17737 42407 16909 42460 51871 18318 42149 42547  5759
        38763 27369  9097  9798 20927 31512 12845 12848 42981 26660  4258 42327
        43656 43659  4966  8416 24023 38301 24316 17622 47572 30241 19975 11703
        25792 16417 48053 52436  6399 49811 43817 30517 47576  2378 17786  7941
        11750 18753 41782 48475 37700 16060 34863 41871 14423 27265 42186  4512
        31029 22423 34279 14370 14273 10995 13140  2542 37308 41195 47082 26884
        43158 45894 46615 23554 26407 19452  7132 32030 28296 12089 11123 19928
         4424 47736 46465 32062  9316 47032 33152 13880 12042 44600 31111 11071
        31202 21434  1770 15124 42031  2253 36590 36592 30257 37097 44901 20573
         5316 50905  7607 19386  4738 21296 30004 39901 14778 25869 22549 39096
        37561 17789 35281 35262  9653 10286 30041 30039 49269 49290 49295 49272
        49266 34503 35255 35260 45574  9967 19262 21157 15877 27173 31149 39796
        22217 17686 50206 50203 38845 18364 38583 10800  6284 34530 22853  5620
        45000  6185 25725 13754  6614 45560 47222 24616 34837  1968 20453 32631
        20451 20468  4687 14608  7324 43067  6407 46516 46510  9791 11391  4930
        40565  4224 32805 37524 51775 43323 17955 28408 18346 38894  9636 45618
        15767 35340 13795  5853 19402 46128 39382 18350 51998 28511 49246 44847
        12190 11318  3031  9885 49654  7567 41629 13532 22917 22354 19971 20310
        40697 23799 31640 35304 45363 30626 19937 19909  9532  2426 27849 36985
         5319 21171 25048 36061 28754 11252 34333  8292 10921 18508 21274 45522
        49751 51151 24073 24066 24067 24095 39678 49394 47341 34454 23767 34500
        36515 10306  8635 35239 34574 26869 42160
    775:26247 20810 15536 22235 38682 19011 42677  8409 49072 36772 36880 37922
        27806 26845 16203  4501 52465 39859 20439 12880  5190 41738 34627 23466
        30149  5192 31520 47245 48959 45925  5766 38708 17821 24796 13749 31264
        47292  5413 29494 13085 27082 48976 42465 45829 36499 32384 49755 49980
        19002  9468 44492 40559 11763 27019 25875 18181 31713  1950 22604  2660
        11097 14129 43277 40130 16919 21515  3925 47456 31239 27055  8204 26265
        10166  4257 22261 38993 12739 42079  9440 11518 17430 33084 19839  4206
         9465 49891 20771 23033 36988 30594 50833 52637 35917 28763 21424  6999
        24229 19146 44988 31898 26086 43461 41453 13594 47240 18085 49539 36664
         2128 46965 44964 35531 21616 50937 23234 42446 43519 46617 21209 32824
        39652 51321 24059 38555 11908 45322  3343 51967  5206  7289 51617 14630
        18296 49386 15638  7140 37247 14830  8457 42906 44495 49892 51225 19673
        22666 49111 51760 23969  4173 21433 28911 46563 49106 39020 38251 48247
        13480 50074 30475  4810 47817  2060 24999 32348 51443 42514 21367 40591
        39870 43692 33089 18415 50190 47493  5185 41462 46562 27914 30115  2333
        31638 49500 23637 38082 35315 46639 15339 19395
    776:43154 42887  8815 43924 36246  9492 30750 42361 12959 32153 37814 14375
        23571 37052 26583  7532 35975 23295 49875 48435  8660  6358 40613  3526
        47729
    777:48327  8465 52709 18961 27380 36263  6110 32092  8444 15425 29742 45610
         4320 23320 33531 14483  2056 30513 17052 25623 45589 21114 13245  8482
        21931  2413 12527 49923 48847 34000 34597 52433 23977 17071 18595 46581
         3194 49900 28315 21746 17534 12131  3226 24193 33322 23949 33674 17775
        35006 38660 30791 52275 26030 31166 14492 12486  4910 35320 22104  4681
        19904  7098 46207  9972 41259  8596 37280 37260 13355 50056  9494  1959
        12265 40465 24987 34114 12931  3715 34748 27376 27372 34752 14392 50499
        45665 22083
    778:51822 27371 21528 37147  6562
    779:39200 13718  6090  7539 39641 31929 32025 31985 31987 31959  8690 29220
        24954 13819 33682 43937 42789 23595  8141 34706 36582 39947  8947  9834
```

```
      3620 26400  4139 40822 26302 40619 28120 26140 13677 33920  6412  6579
     39316 30226 12806 24260 29386 19963 40669 36056 35811 43575  8476 45002
     44135 44424 43580 46936 22333 47609  6230  2781 34127  7937 20536 36319
     25214 22086 28544 43292 31828 46522  9129 42966  2110  5430 21222 21512
     32536 50809 24621 46949  2768 29317 35267 41909 45096 32051 15964 11143
     13204  5538  3158 32118 50476 49957 20533 12228 33453 21968 15483  9105
     35181  8609 23057 43286  8685 36963  5458 27468 20773 47585 29928 21493
     42582 44877  5391  9820 21160 10498 17697 42487 25853 17248 39238 38645
      4026 21985 49135 37196 18317 14922 37978 13874  4638  5610 27679 32957
     32434 12295 16866 41016 21571 47531 40924 30631 26486  4115 15893 40537
      9944 37120 37140  7595 46982 10489 23001 49728 23669 20878 48970  3421
      9826 20017 20200 12973  3669  4464 13220 42693  2680 23296 31246 46929
     31674 36607 15048 19358 49458 18703 19390 10382 40379 19147 23390 35066
      9851 40569 48103 17010 15833  6748  6719  6695  6722  6689  6662  6042
      6659 19391 49716 15705 42783 31172 10006 27064  8728 24836 16393 38457
      3541 38444 10911 47704  6294 21784 30783 35409  6045 44630 17930 28100
     20397 37870 13823 35718  6402 40302 45635 44052 30168  6571 40448 14736
     49040 25809 25791 25785 46142 25787 25784  7586 48064 45805  4306 35331
     29929 48312  3338 10007 37479 28910 47119  4580 44741  6833 50418 22106
     20528 26291 32512
780:30294 49443 14267  4345 22992 29622 21893 17720 30581 27127 22409 39191
     41416 10462 48136 10604 17541 18936 29596 39746 32568 51350 35729 32694
     26733 16815 35442  3010  2531  1970 26238  3994 36656  6819 29780  6882
      9450 12030 37059 13612 18878 33913 22989 12430 21397 29955 15986 13852
      8973 33250 42555 12999 18478 48273 32603  4722 31025 30617 15873  4868
     37998 42099 20780 39344 41981 46178 46179 45556 23292 19790 20105 30964
     37281 44188 27826 13934 33420 28921 32493 36968 25646 27751 46198 17794
     43955 51979 17752  3539 50391 23596 38762 12036  4918  6385 32820 49050
     51711  8316 34581 11854 51171 50130 20508  4074  1615 29444 15023 40046
     14110 39359 33895 29880 27612  7971  4316 28690  9388 22646 15379 47913
     29152 25544 20443 43794 17933  2453 51987 19880 15751  2789 39703 26832
     50642 21483 44819 46572 12611 28844 25956 37342 22833 52075 24618 31787
     21304 51304  6463  5572 23816 23337 18560 48213 45336 46857 38982 34010
      9163 46891 42785 14171 23664 17902 22586  2111  4187  1782 34617  7036
     42742 50052 31741 11047 51701 16809  5412 30737 41277 51934 51030 25027
     23932  9973 36986 37692 40170 16794 37411 47098  5779 41844  9818 44851
     24276 20564 24202 25136 32760 48548 38835 29874 43852 12633 19378 48188
     28710 38465 44069 38934  9515 25946 19050 25666 40215 29114 38830 22273
     25925 34595 30970 52714 20187 25520 40521  9985 42707 14419 50178  2508
     37536 52476 42282 13180 39108 28936  5162  3559 34890 14364 43307 28398
     31217 40688 40001 24130 37900 35941 29515 11127 26915 44943 21695 19995
     50282  1620 48528 52285 11376 35948 26063 21543  5521 11970 37477 15643
     20859 48352 40282 44894  8048
781:32714 13989 30485 21745 48705 45248 19635 52561 46582 50747 24578 26938
     17761 10439  7947 20061 26194 39730 12212 13995 17501 17033 29720 31213
     12987 29190  8140 17995 17242 43810  9470 13996 46922 42196 22368 19761
     32187 19209 31079 12705 31814  4084 18166  9566 23075 36984 27715 39488
     23514 17884 21395 21398 27040 27022 30254 44635 28684 10385  3178 18439
     33821 38237 20825 36958  7824 14932 21074  9670 51738 30214 22741 42892
     30210 18082 18081 17465 18086 32071 32073 32069 46924 32079 32101 18090
     32108 32106 40404 37855 46421 10047 45005 36681 24002  9907 43830 49573
     37554 52307 41414 24000 46577 24293 34185  3974 47992 27398 31449  5895
     12778 30935 11230 50070  8809 30409 46133 17959  2287 44450  1168 21721
     46214 39175 28804 35263 25599 43731 31618 10899 31169  2174 43578 40213
     51653 49599 16322 10189  9001 48888 13969 22581 48623 13990
782:22967  4957 35237  6878 35258 35251  9628 34498  5377 35242 35244 35285
     32547 35289 35294 43741 43766 34473 34451 34477 34475 34527 34593 34564
     34535 34562 34569 34537 34474 34478 46077 22200 11165 28531  2429 51774
     26195 36188  9627  9625 19067 22389  4430 45126 25708 18363 19572 16563
     30236 42072 51436 20784 50567 23507 49158 38806 50171 38235 18506 37967
     30994 24369 45825 10260 11066  5828 27608 15436  3815 42407 18866 35696
     32580 47639 38763 14955 34449 42981 26660  4258 39632 30241 19975 25792
```

```
     16417 52436  6399 49811  7247 43817 18753 18055 22423 34279 14273 10995
      1650 13337 44917 43158 32030 45799 10574  6627 44757 40625 27465 30262
      2888 23231 18526 22750 50905 25456 39376 21296 21041 30004 25869 22549
     25619 17789 35262 35281 10286  9653  9659  9656  9654  9655 10280 34504
     35246 30041 30039  9018 39890 34503 35255 35260 31258 51859  7837 31953
     29769  9643 37794 34530 34532  3185  5271 11527 35347 36012 20280 29975
     17496 35431 44524 50989  4283  2770 45516 46126 51417 29558 38700  3691
     37745 46220 40708 34364 50179 25048 43073 37634  6979 19532 36061 28754
     34333  8292 10921 45522 39678 49394 47341 34500 35239 34574 44772
783:43552 19357 23591 49060 49065 21638 47183 51654 52167 46956 39148  5452
     22696 45703 18471 52567 11269 36989  6534  9161 38985 20798 12102 27017
      4545 52473 20079 30439 24463 25548 25546 24179 14489 14313 40728  5990
     49678 22855 34150 22593 50400 16937 35460 35931 50309 44921 43648  3587
      2581  6993 37951 21322 52574 15706 21992 52761 47467  3404 23251 50102
     20151 11517 10569 41776 48669 17974 36831 12661  6048 20912 25768 46818
     46815  4506 25582 28130 37402 36932  2628  4300 37605 19046 35969 49473
     13516 13511 30545 34139 35119 50375 13161 31782 16186 50116 15278 10255
     49666 48718  3996 19894 20127 52766 19024  6169 46125 39971 34426 25950
     33729 12523 43413 43414  5329 46752 46748 47283 25665 39940 15662 16620
     33128 46298 41968  7612 38760  7121 37415 25519 50525 42340 12927 34004
     33835 19181  9102 19293 45087  7716 14019 27516 46988 50415  2668  7229
     39681 19628 32871 15479 40152
784:37790 14864 47479 37391 23869 36259 19456 44642 14737 19786 18510  7439
     46641 22633  8001 39977 31292 41109 45354  6597 16362 22302 22296 13861
      8593 10751 16365 16386 13720 10017 44113 38477 47177 41784 39749  2083
     16447 34043 48687 17443 20111 28376 27512  4107 26917 23241  7616 40866
      2646 28676 20728 47761 40644 24876 29326 30465 36047 13086 11258  9530
     24853 30547 32190 16569 16889 34620 25097 31914 34909 47103 20695 50993
      8705 31235  6516  5516 47028 40371 51450 25928 34319  7341 18427 47934
     44438 28505 29587 29585 29584 42972 32784 11284 39767 29985 47426 25093
     44761 39224 11364 37182 17664 51510  2280 37924 18091  6433 23815  7709
     51809 37907 42062  8568 28054 43705 41090 45669 23263 19926 37763 19190
     36298 13904 20696 43215 50613 51422 21377 10910 49767 13224 18170 16388
     33903  1219 25119  8399 51590 34964 26479  9036 52183  8591  7689 52590
     43043
785:13219 18713 34284 25428 32396  7846 34418 31437 23119 23139  7307 35664
     23684 44810  8929  9755 49300 12202 46608 16730 39139 36843 44533  8670
     29799 24248 47228 19261 25168 44232  2109 12009 24228 39320 11658 38642
     37102 51732 34673 38327 18905 49130 19829 42292 47326 27243 33137  4605
     47563  3941 19675  5035 44053 17257 12653  4733 34274 11603 51917 38184
     52113 11209 20928 50226  8672 47130  5368 47286 35812 39635
786:14725 20253 37116 31259 46899 13959 43253 11838  1910 47640 30619 13032
     18846 14261  8257 46644 34230 24557 25334 29360 36385 28610
787:15707 12963 33332 50350 31943 40162 11020 24346 41811 50880
788:17497 20380 37509 52053 11757 14181 10985 45685  3448 52645 19375 27151
     10392 11407 49239 20174 29908  9879  2694 42106 39486  6754 16328  2214
     35894 22247
789: 9420 26622 17863 33176 48966 40454 10693 51244 34216  1585  5095 36738
     19555  4335 52089 44531 28200 39596 14952  8981 40959 28692 28693  4363
     23016 26191 31825 48182 29115 18220 38561 46494 28248 35532 16020 14613
     35190 23739 48641 33276 44278 45611 28547 46693 18646 26518 50426 10543
     24611  8935 10547 18386 33122 39906 23582  8304 20683 16179 37340 24158

790:16142 37680 11923  4150 20175  4978 32931 18464 28577 40684 45169 13253
      6835 33118 15947 24556 46808 20516 38003 51211 42882 26667 42854 23488
      8869 22459 11478 45519 39719  4325 50344 31027 49637  8793 23068 22252
     46827  2957  9730
791:39602 48675 11732 41732 43325 10570 14574 43687 17986  1245 52484 46457

792: 9679  9764 14760 33547 30502 35504 34916 10712 27148 21340  2802 42631
     19621  2666 12351 29459 31957 21980 50806 25238 24155 41215 34823 32021
     51553 47302 38853 48170 19416  6388 39261 48812 49984 16371  8319  5967
```

```
     35048 52246 38804 22929 18997  9064  4156 17259
793:31055 10335 24814  3745 15268 48300 14471  8778  9767  2043 50175 22865
     29811 22226 41754 27779 40512 11342 49809 25404 15887 15902 11279 11278
      2947  1087 28478  8197 15792 15773  5110  5115  5112
794:48137 29905 24327 17921 23222 37971 25832 16387  8421 48761 43974 52500
     12978
795: 5379 49842  4058 12248 49848 34447  8559  8558 47832 16134 50162  2071
     49742 28898  3188 21391 19195 30005 17632  9711 14721 42978 11280 24545
     24723 41915 23903 43179 43033 45783 18918 24756 16890 11615 19817 13110
     45448 34084 17724 10326 50407 20268 43126 43063 47202 49737
796: 5379 49842  4058 12248 49848 34447 31588 31585  8559  8558 29536  1499
     47832 16134 44654 30154 47963 11896  5351 35762 22673 52232 10744  7484
     23948 23820 23134 50648 40671 14721 42978 29658 29690 11280 50265 34217
      3467 11833   894 27855 26972 35112 40067 43033 43179 45783 18918 46430
     16890 11615  3181 28998  3155  3179 46525 30613 39910 23021 39011 52331
     50263 24471 49995 23146 27624 49993 43191 44994  6867 41936  8205 10326
     17724 20268 43126 17263 43063 47202 49737
797: 7661  3470 25232  7530  7852 44398 25883 19296 31604 10137 10134 13910
     40791 22166  1698 26333 26961 22024
798: 6247 15734  6650 40375 28082 38908
799:39443  7039 28361 47906 16840 14393 16154 19578  1627   987 29967 49552
     31206
800:29756 12248 49848 34447 44415 29536  1499 47832 49379 36192 14721 42978
     29690 29658 43179 43033 18918 46430 30243 11615  3181 28998  3155 19813
     39908 23020 50263  6867  3025 10326 17724 20268 43126 43063 47202 49737


801:12823 12825 26317 13499 13535 16258 16260 16256 16241 16239 48259 19081
     15774 26901 16066 31294 28972 39956 15829  3929  6386 42190 33635 17890
     10101 11869  4996 10347 39285
802:19548 22488 35624 26178 25450 32940 13900  8117 11420  4324 30623 51576
     33049 35355 31059  7120  3927 28214 31516 37852 13623 25547 33411 27530
     45380 44756 34465  9592 15612 50625 25150 14780 20517 20266  2949 29873
     10334 21145 13264  6132 51088 24424  5420  6770 17478 11654 50792 37403
     27304 28349  3268 20095 21386 29418 24070 14651 15132 40455  3762 50530
     43554  1666 11204 17384 35895 34314 18515 22828 27629 50310 33339 14126
     26448 27156 11340  8148 28720 15240  8382 38151 24672 21079 31075 46427
     19726 51637 14372 41781 23003 36539 35019 48962 11873 19056 51137 16912
      2562 18612 52079 50277 34506 37081  8236 36011 12249 40217  6109 35138
      5560 37547 37542 45573 43077 42357 18273  7336 26493  1809 38587 41875
      3667 11058 36270 47252 11533  8044 33153 34499 23540 22637 17179 24341
     18567 21824  4599  7596 35236 33337  6252 47623 36046 16264 27268 24584
     34914 49057 51636  4193 21508 18972 11953 26236 24464 33017 38132 12113
     49123 13094  4331
803: 9679  9764 13234  5242  1879  1937  6896  8507 17259 41156
804:19789 37468
805: 7413  7414 42316 25039 34806 31714 17804 11250 19488  9637 41591 41286
     11398 10172 41883 13972 22181 44271 20490 48210 25730 41029 30698 23429
      8613 36765 25350 25963 34937 17027 19777  1904  7589 25501  8581  7159
     52296 38695 25713 26892 23308 38953 10996  6645 51993 49450 22336 43548
      3768  3277 22280 17142 21437 44886 13999  3701 44024 24342 33523 14071
      9667 36187 42430  4429 11884  6191 28686 45878 52559  2839 52454 27964
     38918 19420 14265 49521  3024 22435 29710 38781 35820 31106 27348 40626
     49943 32297 45015 15851 16427 10900  4584 11613 42915 22631 48058  4341
     22276 50004 38231 20575 51116 31133 51121 32406 37854 36116 52650 12069
     32289 22849 52416 29342 10151 14585  1746 16688 15040 15042 37654 39122
     23886 44463 37441 29770 36206 15203 21083  4758 31865 32487 31218 26062
     38813 34760 34734 27483 20651 49557  1925 36699 31013 46939 35785 41151
     40794  3917 26235 38061 51517 47494 37517 46730 46731 51610 41829 47930
     33077 20886 26466 48295 11218 10386 33663 33664 33081 15111  4354 26270
     50283 51841 51044 26278 51394 33749 32766 34519 45303 35551 48727 46063
     42781 43218  2439 46526 32240  2010 50473 50479 35847 29448 26264 35849
      4843 27136 31802  5149 36911 10919  9206  5774 11843 27426 19353 40609
```

```
    45965 33191 13633 44707 44085 27403 12304 29794 52470  7201 17389  4274
    41257 17385 38010  8662 47759 17916 27404 28160 25867 26098 16660 22079
    27424 27428 16311 13588 29269 47983 41242 18811 30414  4060 28097 13493
    43100 17634 32361 15076 19586 39188 26956 21144 15149 44282 33267 38511
    33640 33657 23624 44444 40637 23083 18737 28993 16088 25915 43987  6873
    50724 50727 31994  3442 29537  3601 33660 35089 35633 30164  2937  2933
    28810 49647 47571  9582 38582  6917  8050 14336  7379 15198 40879
806:48144  1369 43563 26706 25538 25964   759 20414 39528 37860 20041 18054
    33273  7444 33275 28316 20660  4912 17673   755 21248 13876 38898 28552
    11035 32131 22108 45628 13412 11895 42003  6156 52566 20357 21828 33665
    24443 29552 36712 52749 41326 11205 24330  1992  5714 37378  1371 34183
    20707 49043 48485 40210 34250 12998 52037 48613 28084 51431 47876 39474
    12956 51955  8737  6426 22754  2948 51640 26554 22142 21166 44516 12086
    18521 35921 38071  8374  9070 22699 52659 23923 45803 25411 37203 34275
    51521 39894  8680 24033 18809 44505 44509  6557 16865 26911 37820 15879
     4788 13896  6544 29835 38646  2661 28260  6916 46155 18310 15725 11216
    12253 38634 13569  5762 33433 26477 19178 36719 25369 47075 12006 10261
     9441 40843 27719 12558  6408 30285 39560 36211 29464 46984 24659 47682
    52691 27899 16250 28113  8784 37488 34986 11795 43529 43534 37246 33550
    43449 27001 47441  8017 14001 24634 41509 35934 52726 45692  2998 48242
    45987 26315 45238 43338 41748 34137 37160 11051 32956 42118  5191 45199
    45264 15168 26414 20909 18139 43926  5860 20725 47346  5076 34444 12088
    35617 37327 40356  8152  6491  7266 33724  8459 21696 20590 10521 11504
    23848 23850  4666 30398  9511 22742 46684 42258 50297 21624 27402 33666
    18152 34766 35252  6273 30306 14351 29738 18435 22307 32577 20274 20149
    10958 43223  7764 17719 51022 44525  9803 45188 40887   757 28143 23950
    21699 22425 14803 42503 30574 26960 47753 20963  1674  2629  2633 43039
    47504 22787 40072 10478 50926 23987 16302 41830 41832 23634  6687 34392
    23658 25495  8724 36003 23521  9722 23098 41222  3372 28628 24893 46022
    23892 38785  4042 29415 25210 50281 19189 19271   975 13421  6432 33010
    22806 25532 10132 27777  5340 15672 31543  7775 31965  8330  8333  8328
    12107  4656 17796 43576 10061  7275  9692 47425  9663 11338 43936 13679
    29370 37827  1767  1765 12067 24001  1590 38764  1372  4052 21420 36943
    17382 50457 36002 33280 28386 48720 18731 17693 39808 22813 38468 33561
    30402 27455 17185 19889 50608 46726 12128   962  9719 32596  6980  7608
     9551 35496 30386  3086 37250  2226 31679 11359 34354  9347 22744 42277
    39348 50088 44162   756 48663 33861 33865 38152 20074 48985 35995  8900
    45343 17815  9690  5891 34756 35618 21929 44455 19084 48369  2393 35381
     1370 16640 22323 46466 18735 50248 48090 52752  8579 40499 30905 33797
     7163 15679 13598 43535 32742 30235 41794 39718 16313 29279 45847   758
    29498 10555 20519 45664
807:34384 12235 42953 19232 52634 35965 19121  4693 39407 41359 51086 27860
    40324 25194 25193 49631 33814 12449 17171 20531 24486 50128 33272 32055
     4962 36452 27482 19656 50944 18674 18677 27421 11388 44590 13573 25356
    14848  9007
808:18128 16689 15537 38423  3030  3022 38168 38170  3063  3061  3050  3023
    50416  3200  3861 32918  7592  7590 15484 35307 18253 43197 18678  1655
    38554  9312 15281 45988 28678 19588  8757 29867 26947 14232 48621 49162
    45735 46392 27105 49707 45990 27711  5876 44879 23285 45753  4521 37169
    26031 52557 36301 17107  5383 32464 30760 35404 12144  8239 33210 21273
    34262 14498 31267 48378 34012 15206 29597  6076 31421 50846 13875 52482
    17639  2938 20328 30664 19321 39051 21208 39931 22157 10389 24182  4882
    49367 16514 27690 15290 22702 14503  7385 28227 28171 41271 30265  2039
    44084 50097 13603 29859  4736 43533 42634 27334 22694 10593 36966 27801
    36277 24285 33951 29257 32080  8401  2113 33412  6657 22206 30766 49267
    13464 42549 26111 33124  5875 37073 50894 14408  5148 24458  2574 39630
     3718  8508 17566  6706 19038  2925 23828  1755 42315 29061 23236 37125
    37658 24846  7264 45053 47304 49430 41272 11063 50675 10399 29097 24125
    33697 18033 36365 35130  2386 51094 32207  7797 24709 40832 15270 13347
    22987 21911 23556 45841 46164 42485 45061 40712 29712 35612 39462 32135
    35321 47837 18431 39586 18270 40683 22152 34232 14219  6387 20955 36464
    12657  1795  7569 16391 46410 44863 40016 26213 43412 52492  2098 31915
```

```
     52618 30936 28912 29935 31858 29846  2034 52580 52468 30968 29815 29087
     29895 29014 28992 28228 29841 28203 29111  1999 31834 27317  2030 52522
     30860  2100  2052  2001  1982 52573 31854 31643 31010 31672  1977 29978
     29910 52496 30987 28884 30762 31015  2106 31917 30020 29914 29063 29817
     28146 52524 28181 31708 30971 31890 30985 52427 30014 28095 31745 31861
     52640 52586 52550 30017 28988 28020 52638 30012 28933 28908 28024 28166
     31776 31675 52611 52397 29120 31710 29117 31826 30827 30048 31773 52430
     28075 31797 30894  2077 31742 47828 32842  7615  7025 44910 45846 17855
      8234 37651 32740  6328 16942 49744 15764 33399  6246  1692 40458 36264
     15667 29630  7371 26481 52192 21831 21442  3999  5013  4594 28027 42502
     22539 26037  3213 34895 44780  7001 39773 47713 52738 23975 32541 14474
     24668  5864 34167 49505 49527 49526 49506 49509 49529 49503 49484 49482
     10725 12185 18516  7636 50215 35604 37937 16544 22722 37408  7620 48385
     31804  3565 47506
809:  6956 23642  9476
810:33435 17046 39846 23608 47179  3286  2764 12758 30828 33173 52625 42738
     16711 46706  9412 13620
811:19498 28661 26440 47524  7663  8739  6563 34369 13088 42233 20662 49732
     34797 38267 34140 16165 52641 13877 29338 49629 17579  5161 28778  7340
     29737 10879 12177 30540 44942 30591 45641  6487 26931 24371 10012 35878
     24482 23729 39707 19308 47434 42704 17740 15332  6477  6356  4746 27394
     46191 15230 12001 41743 41724 25554 43377 17738 18621 32102 39079 19317
     32045 33359 34200 14385 50336 14718 12064 34991  3871  4057 46447 16016
     50193 19556 30038  1745 10605 31757 18348  7368 17203 34829 24200 32908
     28477 23415 32460 47131 13024  5612 12861 12755  6198  7880 19336  3846
     31527 51991 40070 32259  9275 17819 35557 20845  5663  4390  5109 33018
     19846 45453 15680 24061 22082 48964 30702 48235 24799 43763 41080 41520
     11666 51401  8294 52135 45621 18179 34966 30030 44674 17491 20491 29578
      9631 11175 27429 27456 50186  8034 43282  3570 44733  3466 22095  9444
      9416 38013 14820 38753 23626
812:11856 49668 43424 13399 12770 20571
813: 9038 50785 11937 31584 42597 17379 27528 15303 23282 34709 22740 23041
     37384 44932 10294  8183 10542 10961 51343 32317 23141 38638 15271 49433
     42642  3275 45444 37589 10068 37348 33141 49236 46013 48911 42682 41296
     13665 30559  9930 24333 34609 22229 19097 17562 29808 32876 16841 17777
     45686  5328 27677  6577 15871 35603 33702 43299 40957 51588  5028 32039
      6793  6376 21205 23118 37804 12130 34517 39268 46847  3679 30322  2299
     45762  1990  4371  9187 19746 42876 15013 52007 32899  4182 30601 17757
     20433 24594 16637 42886  2529 40401  5490  6963  6019 44095  9479 21154
      5489 43861 29198  5732 12316 37768 39078  8179  8296 23294  6075 35510
     27978 50738  5820 32402 48618 41623 21518 12405 19481 50810 31308 38973
     35124  4050 15740 51486 31334 18065  7467 20421 40908 39644 44346 11203
     19368 36260 44792 13905  6360 28556 24434  9677 21161  3951 14315 14298
      6697 11383 16242 37067 12996 15170 21974 26487 18919  2127 30077 30073
      6514 37997 12467 50340 11006 22807  7353  1638 37036  7354  7964  6351
     34469 51867 20990
814:18728 29903  1221 17490  1842 27282  4307 38882 35607 25944  1146 15053
     28182 28516 17417  4517  6122 14841 28105 20139 24614 30064 42481 14087
     14467  9704 17124 52628
815:47338 29678 45856 48975 34300 18774 38310 47751  4505  4503  3868  2992
     30696
816: 2632 41969 19376 20677 52731  3184 25910  7362 33258 46059  7424 33260
     48022 48023 17635 17638 27154 17583  1178 12445 28632 28549 28553 21168
     49191 21773 50073 30138
817: 5902  5904  5908  6734  5981  6023  6025  5953  5910  5983  5986  6014
      6678  6737  6829  6831  6855  6864  6898  6728  6888  5994  6834  5934
      5959  5992  6018  6856  6860  5927 36357 36376 41209 19020 45750 22213
     49729  6836 36378 49307 18036 18038 34410 12439 16149 16318 16084 15308
     37400 37425 16253  2449 37394 37398 16232 16150 37457 16153 16281 16257
     37466 16286 16315 37430 37427  2451 40955 33753 33761 30635 29581 41569
      5721 13783 49002 10769  5719 35880 47334 14924 44803 48175 26751 40030
     38897 39214 37328 10199 52214 35716 16321 35369 31767 35374  6671  5846
```

```
      6063 12152 33614 27487 35370  6703 17648 27584 27589 27621 27591 27558
     27586 27615 27619 27593 37567 34934 49152 40718 38458 48536 34173 49375
     11294 13111 30639 27258  5194 51085  7887 37124 19251 10545  4069  4563
      3981 46570  6052  6021  5840 26790  8884 32689  8295 50427 31595  4629
     27447 33549 21130  4734 42281 31196 18446 50617 49199 32426 28747 28742
     19720  6709  6762  6764  6802  6798  5881 41565 33755 42576 31770 31099
     46833 34963 31097 38815 20652 43722 23968 39186  1834 16294  3733  1863
     16343 30175 38727 29956  9313 30643 16341  6705 48432  6799  6886  5954
      6759  6670  5880  6700  6702  6726  6732  5845  6895  5716  5932  6055
     41571 24957  5718 15824  6769  6792  5930  5928  6825  6795  5839  5841
      6061  6674  5906  5874 34408  6868 18412 34405 34244 20257  6669 22727

818: 5508 29249 17842 49014 28682 50968 17607 18517  1849  2470 41031 14789
     24732 41009  1853  1846 41005 41003 19747 16381 22303 45119 23499 49560
      5507 26602 20532 46180 44104 36514  6585 28083  9497 31134 48323  4172
     40621 29636 32449
819:37926 37928 31676 18532 25226 37829  3011 37629 34899 17822 40445 30482
     47454 40053 45882 16936 52758 19208  8072 27431 21965 36379 39364 18267
     16613 12838  4842
820:26113  5717 29773 28234 30031 24487 21915 11597 10538 10075 24165 24762
      3210 17554 12106 41073 14516 33897 19791 50654 21183 15078 31454  8707
     44021  7301 39793 37849 14120 33976 41478 14302  8215 32582 42452 28626
     46076 28125 28123
821:19567 52382 28269 33929  7862 10141 41860 37198 35630 28683 23158 30155
     48138 17764 52268 38044 17197 27906 35621 50126 25601 40533 27546 10634
     39556 38932 40200 45668 23778  2734  4827 31397 21939 26982 27875 14221
     17833 12240 22745 51762 32376 30561 36769 45603 36215 30058 36437 47594
     47257 11876 52323 50349 48577 43788 39005 41507 45758  4075 13363 15646
      3556 26252 19927 29733 12364 23411 22039  1872  9475 47370 15508 45242
     47815 43099 44035 49897  9542 52539 15624  3106  1978  8084  7935 51154
      9197 19775 47727  6017 23496 14043 25496  9169 25704 41258 48145 20656
     42325 32205 50122 31831  4887  4447  6344  6347 13194 24281 51602 26801
     30748 40503 34237  5549  2576 43094 16758 16112  2291 24060  2148 20180
     44755 33105 27822 20152  3395  7977  8039 30745 48496 40149 49515  5582
     23230 17488 17486 18686 24914 21214 38030 34332 15058 19303 10159  7460
     20415 44852 15574 12906
822: 5557 35796 35791 36183  9096 19480 23191 22031 12091 35074 35313 13892
     41172 20712 22164 17437 14250 18706 13215 22017  8890 17982 19959  9723
     12264 36299  2573  4246 33472 42869 49276 49251 49303 49299 37409 24364
      4881 38072 21520 16907 26075 19315 42491 10278 40855 33740 23268 46686
     35794 30818 11078 45630 26408  6296  1610 40389 17003 17006 11422 16649
     46481 12995 25302 25310  5712 13954 26347 27238 30684 37294 43957 17721
     11310 23279 44840 43877 10608 10612  8203  5311 13332 12554 36736 22178
     22175 22182 22149  1497 12693 35474 17339  7023  2403 52444 40011 50683
     32244 21203 49604 49496 11611 18042  2675 17236 40460 40461 47458  2187
      7668 11641 47293 42694 23901 51311 40524 16580 46026 23181  7763  3322
     52443 33237 34316 34318 34320 41866 49222 41821 36028 26900 39324 32265
     23391 34211 27445 10773 21199 22012 46110 30165 16051 22014  6591 37419
     25991 25985 13027 37422 21201  2805 46210 13870 43334 52235 13060 41015
     37423 25085 25084 32751 25993 46275 46248 22153 12979 32744 51232 12574
     12571 25989 25967  2600 30821 36220 24416 23978 43276 32570 43684  1963
      1961 43678 11193 16235 29949 23954 43685 35953 38208 18658 30116 30820
     30823 34228 36485 24465  6613 36062 34882 12945 38088 44318  9912 30324
     18189
823:26815 48717 30861 24989 15581 44431 42188 44124 44653 51504 47465  2355
     20653 10602 16927 29828 39955
824:41403  6261 52123 21969 37704 28902 44986 31154 17840 16105 30767 45774
      4460 12390 18863 26818 16279 16282 36704 38556 11257 36362 52291 21204
     12717  2592 52039 47970 36760 35573 30132 52549 18807 29843 42614  6649
      4744 27096 36971 40776 46890  3266 50213 52038 49575 18305 22551
825:17040 43628 39381 46611 10780 35701 36601 49856 20610 49715 28437 11245
     16533 37773 20670 12390 33862 26330 25814 16173 16175 28163  2443 25183
```

```
       19434 20836 20115 37982 26603 41714 12716 31372 32611 12628 40738 28043
       39091 20220 42500 16127 44116 50842 50841 42614 38683 20977 22298 22299
       48158  4591 45202 15090 13222 50214 49344 51373 12736 12853 12846 25688
       26488
   826: 7259 26533  2564 46567 22512 45260 21142 48298 39587 23990 24907 35850
       11467  6903 16224 15596 15110 23299 36473  9211  5332 20134 11562 10802
       29016  5619 20605 11048  8564 15367  8866 30671  9446 17196  5381 14667
       29268 20429 50597 49551 23084 15984 28717 23602  5249 25005 12332 18814
       38312 15213  9949 40870  2332 18796  6553 48311 52033 24828 32024 34640
       45699 50542 34344  4233 45079 24543 21559  7559 16944 27391 17058 23705
       46150 45221 28966 47374 26561 27645 39915 18290 43703 35270 38929  6925
       23226 51490
   827:29756  4058 49842 12248 34447  6992  6991  8559  8558 44632  1502 47832
       16134 29658 29690 11280 31163 12604 15523 11223  1529 42248 45783 18918
       17991 46428 27191  3153 35937 46559 39909 23019  3391 50263 24471 49995
       23146  6492 10082  3438  5080  9089 40575  8205 17724 17263
   828:37098 37104 37119 41824  9215 44051 43910 21923 25103 29098 39038  7305
       44648 41397 10266  7224  4423 31879 27638 37931 17825
   829: 8021  9433 43154 38938 38171 29032 51391 14016 33360 42887 37630 24580
       32084  4440 26882 19495 36647  8815  7483  5805 30359 11599 14386 26174
       28085 43220 43394 14289 39468  7690  9258 44811  2042 43259 34774 14546
       26866 19989 29620 18178  2135 43924 31777 36246 22301 20335 39327  2507
        9492 29538 32369 49398 17939 50853 21946  7839 14935 14143 14159 33516
       33554 27800  9527  6071 16058 22228 47598 24641 40642 17844 52723 16962
       42499 49369 46292 26898 52098 18384 42361 33255 26583 51724 44664 18584
       17913  7532 31198 22658 22686 20452 22689 22688 24953  7308 44371 33287
        9535 21756 43176 35975 25849 52072 20430 20450 11274 11275 37150 48045
       33281 10303 17475 17473  9414 32644  4630 21630 51284 50557 21830 49118
       12052 37127 21096 29503 29517  8047 46717 31923 35853  4340  7369 15979
       43188 43184 17051 36946 24568 20838 20871 20869 21090 18505 47662 20875
       39411 10505 12337 11352 11355 52579 11351 45510 34755 19873 19850 39661
       19870  3164 15276  7598 30018 23054  7053  7056  7059  6923  6994  6926
        6990  6289  6961  7017 23295 49875 47731 38114 39052 39050 15041 41042
       45243 11454 38668  1625  1306  1807  4094 44972 48435  2236 29012 29191
       11961 35195 27461 27452 27459 23613 17017  1713  7343 36189 23915 12404
       31086 51484 39296  9865  7585 46065 10676 39858 30213 36286 37069 10786
        2513 40203 18591 34337 22189 12689 13258 43596  9576 49990 38710 13278
        2967 42994 51706 16403 40336  9460 50780  8979 19571 27876  1386 47412
       35018  5503 18231 20404 20402 24794 42996 42965 42937 42871 12396 42932
       42961 42958 42885 42848 12395 42875 42929 42934 42852 48228 21924 32722
       30972 23436 40826 48519 46909 10966 41771 45871 10589 23574  8723 28436
        1060 34525 14420 44347 30334 37040
   830:42689 47668 38122 44238 20994 28234 30031 24487 34951 25854  5005 38861
       28426 24762 14229  3210 10236  1936 34699  7052 32212 14514 47725 33874
       47715 26363 36717 52273 11904 35813 25205 14523 36240 37537 47408  7301
       25290  2637 24474 52616 34394 23859 52729 23318 10988  4679 20479 47486
        8698 48553 28125 28123
   831:43552 29330 23591 49065 47183 52167  4545 20079 30439 24463 42900 14489
       22829 14313 35156 32774 32703 32755 32777  3020  3018 42564 44924 27228
        2889 13296 32468 40953 45824 36093 35969  1716 36507 42592 35119 38285
       52766 19024 46125 40216 43414 22385 22361 22391 16310 39940 40092  2249
       35253 13102 20895 11903 12356 19060 19293 45087  7716 13106 40983 41796
        2668  7229 21618 15479
   832:44950 23591 49060 47183 52167 36085 45703 52567 20079 30439 24463 24179
       14489 22829 14313 35156  3020  3018 42564 35931 43648  3587 26077 47467
        3404 50309 50400 19965 31364 40953  6048 20912 45824 46818 25582 35969
        1716 36507 31782 46125 34426 33729 25950 43413 22361 22385 22391 16285
       39940  1958 28685 28629  9102 19293 45087  7716 13106 40983 50415  2668
        7229 39681 21618 15479 20350 46503
   833:20677 44429 42879 43690 13950 43471  7956 52731 25910  7362 46059 33258
        7424 33260 48023 48022 17635 17638  1178 29364 34630 41938  2484 41853
       12445 40004   816 43851 21105 45994 21773 50073 30138
```

```
834: 3131 16754 16759 26533 46567  2564 31528  2669 45260 52197 21142 48298
     17493 23990 24907 35850 39587  2537 11467  6903 15596 19686 16224 23299
     15110 36473 13763  5332 13750  9211 13775 22487 19753 16219  2567 11562
     10802 20134 36888 29016 20605  8564 22577 11048 12017  8866  9446 30671
     17196 22579  5381 37022  4054 49213 49233  8255 33621 13572 52728  9714
     21001  7071 48650 15213  9952  9949 11542 24137 52033 48311 48313 24828
     50542  4644 13064   941  2738  3193 24543 21559 35830 20903 39114  7559
      3605 46491 39120 21350 16944 23705 17058 27391 36434 35521  1654 35517
     45221 46150 50411 28966 47374 26561 49634 30355 25373 52223 27645 39915
     18290 43703 35270 38929  6925 23226 51490
835: 6059 26533 17656 46567  2564 22512 31528 21142 48298 23990 24907 39587
     11467 42308 35850  6903 16224 15596 23299 36473  5332  9211  7588 13750
     19753 11562 20134 29016 20605  8564 11048  9446 30671 17196  5411  5381
      4054 37022 37043  4056 37025 16805 30439  9250  8255 40509 13572 39529
     44996 31347  2033 24529  6480 22831  9710 43835 28569 31688 33377  5500
     37693 21159 44459 22481 47312 28985 10837 11135  2441  7249  3367 29844
     30499 50790  8288 39575 36007 30050 38873 26846  4809 13977 21133  5000
     41285 50014 14977 30923 38802 28521 48320 29709  3043 32390 47809 32423
     30592 16123 31956 36427 33894 51722 30837 11605 15213 21061 24795 41439
     23814 29824 48852 13920 45088 45090 51283 26224 24828 11345 30550  9761
      4403  5211 16342 23925 27770 25445 24823  9586 15613  3655 23104 16738
     45904  5470 10935 13103 43497 28934  4353 31841 50542 34304 22491 28086
     27948  5134 23444  4614 36732 38901 47758 40778 16995 45388 17136  8700
     38790  4626 36925 10576 44115 45404 12370 38441 26806 18394 18847 28312
     48015 20979 25578 21838 17946  3066 14485  9288 25698 14988 36348 11166
     48909 11947 30376 28087 35489 47122 47204  6693 50804 28711 34239  4231
     19582 51291 40300 43738 42552 18448 40456 42243 31009 10598 16944 27391
     23705 17058 28966 27645 39915 18290 43703 35270 38929  6925
836:10844 35708 35671 26905 45400 36672 22957 22960 36713 10902 48653 18126
     18155 22918  6235 40015 36650 36735 36731  7519 28751 28795 25810 28764
     22511 36737 36739 36762 36743 36710 11365 11368 37774 22990 22954 22985
      9487 52177 36678 36706 36705 36770 36771 19945 48196 22984 22949 22951
     23063  6779 18916 46270 22475  6506  6708 12273 16862 39732 18095  9040
      9041  9043 49624 13038 44016 11668 11050 11046  8908 45780 44046 44049
     44008 36652 42347 37426 36637  5303 12192  1506 35594 49412 12394 12635
      7410 10994 50421 40620 39948 22906 22880 22882 25598 17007 41406 25968
     12189 12140  6803  6806 12162 29637 16964  4158 45536  7760 33799  5091
     18571 23076 23431 50365  2716 12164 24994 24992 16113 12158 18118 12171
     11695 12193 12194  6809 12197  1294   837 24507 42645  1623 32888  2591
     18871 33517  4038 18810 49166 19411 33310 37520 37325 25948 16803 21794
      9409  5462 32885 15192 23042 32625 32609 32587 36648 36642 31215 50352
      8458 37758 10829 22502 43343 28238 23017 45559 35679 35675 21270 43618
     43476 24440 15906 22132 18912 49786  7834 16732  7826 16050 18651 10929
     10927 16299 24832 36766 36767  3487  3532 22879 18930 44159 44153 28767
     28818 22908 28815 28842 28846 12168 44344 17456 15028  9675
837:10844 33514 36713 43986 10902 51756 51757 36650 36735 36731  7519 28751
     22759 25810 36737 36739 36762 36743 36710 10785 52177 36706 44020 36770
     36771 32413 19945  6779 18916 12273 18095  9041  9040  9043 49624 44016
     16270 16272 11668 11046 11050 10441 11015 11008 37426 36637 12192  2673
     48758 34063 35594 49412 12635 29311 41406 25968 12189 12140  6803  6806
     12162 29637 37586 33799 10417  5091 18571 12164 12158 12171 11695 15071
     15086  6050 12193  6809 12194 12197 29441 29417  6805 12867 32146 15192
     23042 32625 32609 31215  8458 10829 10825 22502 39088 43343 17049 45559
     35679 35675 21270 11673 11692 31746 15906 22132 18912 49786  7834  7826
     10817 10820 18651 10929 10927 10926 10924 11016 27384 27407 16299 24832
     18930 44155 44159 44153 16638 12168 24507  5462
838:10844 45606 26905 45400 36672 36674 36676 22957 22960 36713 52162 10902
     51756 51757 18126 18155 22918  6907  6235 40015 36650 36735 36731  7519
     28751 22759 22511 36737 36739 36762 36743 36710 11368 11365 22990 22954
     22985 22911 22914 10785 48900 36669 36709 36706 36678 36705 36771 36770
     32413 22984 22949 22951 27174 23063 37126  6779 18916 12273 16862 39732
     18095  9040  9041  9043 49624 13038 11668 10433 10441 10436 10440 44013
```

```
     11008 36652 42347 36637 37426  5303 12192  1504  1506 48223 48222 31509
      2673 48768 34061 29527 34063 48758 39155 16223 35225 48760 35594 49412
     26701 26703 12635 11414 14541 40502 14280 29877 43617 43562 27742 13698
     29311 29192   836 44344 17456 15028  9174   839  9675  2011 47956  7410
     31330  6699 10994 50421 50306 40620 39948 31116 22880 22906 22882 25598
     49961 43211 49959 17007 41406 52305  2212  9735 11741 19149 47806  3006
     46816   840  9571 22115 31807 52479 37896 12189 12140  6803 12162  6806
     16267 29637 37586 25104 25087 33471  2939 16963 16964 10980  4177  2945
     19661 10123 19210 45536  7760 33799  5091 18571 23076 23431 50365  2716
     12164 24994 24992 16113 52272 12158 12171 11695 15071 16273 12193  6809
     12194 12197 29441 29417 12867  6805 31874 42645 32888  2591  1623 18871
     37325 33517  4038 15192 23042 20558 19620 20588 32609 32625 32587 36648
     36642  8458 10829 22502 39088 43343 28238 23017 38692 17049 45559 35679
     35675 11673 11692 31746 43618 43476 24440 15906 22132 18912 18651 10929
     29434 29437 29439 29442 10927 10926 27407 27384 16299 35982 24832 36766
     36767  3487  3532 22879 11262 37595 37594 44155 44159 44153 22908 12168
      4493 32003
839:10844 45606 45609 26905 45400 36672 36674 22957 22960 36713 52162 10902
     48653 18126 18155 22918 40015 36650 36735 36731  7519 28751 28795 25810
     28764 22511 36737 36739 36743 36762 36710 11365 11368 37774 22954 22990
     22985  9487 52177 36678 36706 36705 36770 36771 19945 48196  6779 18916
     46270 22475  6506  6708 12273 16862 39732 18095  9040  9041  9043 49624
     13038 44016 11668 11008 36652 42347 37426 36637  5303 12192  1506  1504
     48223 35594 49412 12394 12635  7410 50421 10994 40620 39948 22906 22880
     22882 25598 49959 41406 52305 12189 12140  6803  6806 12162 29637 37586
     25104 16964 45536  7760 33799  5091 18571 23076 23431 50365  2716 12164
     24994 24992 16113 52272 12158 18118 12171 11695 12193 12194  6809 12197
     31874 20136 41729 16940  7848 12484 26048 34365 50672 45024 34489 12061
      1294   837 24507  1623 32888 42645  2591 18871 18810 33517  4038 49166
     19411 33310 37520 25948 37325 16803 21794  9409  5462 32885 15192 23042
     32625 32609 32587 36648 36642 31215 50352  8458 37758 10829 22502 43343
     28238 23017 38692 45559 35679 35675 21270 31746 43618 43476 24440 15906
     22132 18912 49786 18651 10929 29434 10927 16299 35982 24832 36766 36767
      3487  3532 22879 18930 28767 28818 22908 28815 28842 28846 12168
840:10844 45609 45606 35708 35671 26905 45400 36672 22957 22960 36713 52162
     10902 26670 22918 40015 36650 36735 36731  7519 28751 28795 25810 22511
     36737 36739 36743 36762 36710 11365 11368 37774 22990 22954 22985 22914
     22911  9487 52177 36678 36706 36705 36770 36771 32413 26128 26144 19945
     48196 22984 22949 22951 23063 18916 46270 22475 12273 16862 39732 18095
      9040  9041  9043 49624 13038 44016 11668 10441 11008 36652 42347  5303
     12192  1504  1506  2673 48758 34063 35594 49412 12394 12635  7410 50421
     10994 45298 22906 22880 22882 48458 25598 17007 41406 52305 25968 25966
     12189 12140  6803  6806 12162 37586 25087  3946 33471 16964  4158  2945
     45536  7760 33799  5091 18571 23076 50365 23431  2716 12164 24994 24992
     16113 12158 18118 12171   842   838 44273 17524  4493 32003 12193 12194
      6809 12197 31874 20136 41729 16940  7848 12484 26048 15192 23042 32625
     32609 32587 36648 36642 26149 31215  8458 37758 10829 22502 43343 28238
     23017 38692 45559 35675 35679 21270 31746 24440 15906 22132 18912 16732
     18651 10929 10927 10926 10924 27407 27384 16299 35982 24832 36766 36767
     22879 18930 13913 28767 28818 22908 28815 28842 28846 12168
841:39128 45606 45609 37602 37638 37624 37606 26905 45400 36672 36674 36676
     22957 22960 43986 52162 10902 26670 48653 51757 51756 22918  6907  6235
     40015 36735 36731  7519 28751 25810 36737 36739 36762 36743 36710 11368
     11365 37774 22990 22954 22985 22914 22911 10785 52177 36669 36709 44020
     36770 36771 32413 48196 22984 22951 22949 23063 18916 46270 22475  6708
      6506 12273 16862 39732 29746 29763 18095  9040  9041  9043 49624 13038
     44016 11050 11668 11046 19297  8908 45780 36652 42347  5303 22124  1504
      1506  2673 48758 34063 35594 12635  7410 10994 50421 45298 22906 22880
     22882 25598 17007 41406 52305 25968 25966  6806 29637 37586 25104 25087
     33471 16964  2939  2945 19661 10123 45536 33799 23076  2716 24994 24992
     16113 18118 15071  3688 32888 42645 18810  2591 18871  1623  1295  1294
       837 24507 16803 49166 33517 15192  4124 23042 20558 32609 32587 36648
```

```
     27660 31215 21286 50355 30007 37758 22502 43343 37757 28238 23017 38692
     45559 35675 35679  9651 43618 43476 18912  7834 18651 29442 29437 10927
     35982 24832 36766  3487 22879 18930 11262 37595 37594 44159 22908 16638

842:10844 45606 26905 45400 36672 36674 36676 22957 22960 36713 52162 10902
     51756 51757 18126 18155 22918  6907  6235 40015 36650 36735 36731  7519
     28751 22759 22511 36737 36739 36762 36743 36710 11365 11368 22990 22954
     22985 22914 22911 10785 48900 36669 36709 36706 36678 36705 36771 36770
     32413 22949 22984 22951 27174 23063  6779 18916 12273 16862 39732 18095
      9040  9041  9043 49624 13038 15490 11668 10441 10433 10436 10440 19297
      8908 45780 44013 11008 36652 42347 36637 37426  5303 12192  1504  1506
     48223 48222  2673 31509 48768 34063 34061 48758 29527 35225 48760 16223
     39155 35594 49412 26703 26701 12635 11414 14541 40502 14280 29877   836
     44344 17456 15028 29311   839 43617 43562 27742 13698 29192  9174  9675
      2011  7410 31330 10994 50421  6699 50306 40620 39948 31116 22906 22880
     22882 25598 49961 43211 49959 17007 41406 52305 25968 25966  2212   840
     19149  3006 47806  9735 11741 46816  9571 22115 31807 52479 37896 12189
     12140  6803 12162  6806 16267 29637 37586 25104 25087 33471  2939 16963
     16964 10980  4177  2945 19661 10123 45536 19210  7760 33799  5091 18571
     23076 50365 23431  2716 12164 24994 24992 16113 52272 12158 12171 11695
     16273 12193  6809 12194 12197 29441 29417  6805 12867 31874  1623 42645
     32888  2591 18871 37325 33517  4038 15192 23042 20558 19620 20588 32609
     32625 32587 36648 36642  8458 10829 22502 43343 28238 23017 38692 17049
     45559 35679 35675 21270 11673 11692 31746 43476 43618 24440 15906 22132
     18912 18651 10929 29434 29437 29439 29442 10927 10926 10924 27407 27384
     16299 35982 24832 36766 36767  3487  3532 22879 11262 37595 37594 44155
     44159 44153 22908 12168
843:33541 41374 25062 15778 51828 51835 45856 44903 43836 19174 45039  9167
     19781 26411 31784 23852 30614 47751  4505  4503 46483 46454 46480 34011
     13889 38247 28383  3868
844:49008 24184  1683 38523 37563 18512 11502 24644  8358 39545  8977 21368
     32661 36707 14417  9727
845: 5099 10568 14674 25832 10753 34807 10388 41217 43974 37088 13213  9385
     44760 24296 43882 27696 14860  8150 24422 24684 52735  9816  8865 14146
     41584  2392 48700 26187 32685 25759 27933
846:13791 48697 19086 49548 13685 12766 15072 32616 40428 19903 20313  1859
     49522 36456 46800 52074 25422 33400 41157 44967 28816 15404 44947 24396
     32662 49456 48683 10755 52195 36730 21785 27083 37163 13269 15545 41978

847: 3827  4903 21900 35319  3037  6816 14586 30176 23420 10036 40373  8857
     39595 38881  2026 13120 20948 29626  4997 11475 32428  1344 11474 37303
     34431
848: 3095 12083 27774 13780  3078  3140 37161 35782 33416 52450 12015 40074
     50875 38055 41906 52651 22371  3129 52508  9599 28806 30996 45393 28049
     27120 32124
849: 6260 30070 28395 33791 32013 11918  2525  6742 21132 40189 40193 52372
     13772 31495 49890 21718 30034 10485 25901 40042 41656 14449 44382 32472
     22982 16852 37627 16392  5376  8598 50361  7197 44300  1210 19073
850:44945 31889 41620  8083 29986 37065 32985 28242 12746 25731 40771 11234
     37003 16899 17997   930 31800 51470 10694 26921 37650 29327 29516 35756
     26215 37309 10524 38719 45877
851:35443 35444  4058 49842 12248 49848  6992  6991  8558  8559  1502 47832
     16134 12288 14721 29658 29690 43651 18918 17991 11615 16890 16979 23809
      3463 35817 50263 24471 49995 23146 22526 29534  2012 34720 37242 32381
     14329  8251  8423 33335  8252 33333 10326 17263 43063 47202 49737 29186
     23171 26553 16974 25696 25682 10020 28797  9589
852:18514 26021  9536 49659 49682 49681 49657 15798 39833  8000  8005  8052
      8054  8056 30738 49140 51338 51310 51308 15462 14735 28026 41033 41035
     36976 24499 14741 15464  8356 30228 44107  5811 32709 13537 10882 45920
     45924 45893 14609 45972 52560 41006 41004 32675 41631 51176 24549 23926
     49037 45932 45927 45946 51862 41094 11971 45970 45325 40080 49684 37716
     11936 40622 22808 22830 22158  8059  2182 20125 49147 23754 41323 18541
```

113

```
        42917   8164 43038 22944 22953 22950 20384 49865 49837 38183 33236 33222
        33218 33221 45567 21176 50210 26983 40990 24656 41300
   853:18514 51233 51228 26021  9536 49682 49659 49657 49681 15798  8000  8005
         8052  8054  8056 30738 49142 51308 51338 51310 17897 44263 15426 28026
        36976 15429 33409  8356 25423 25424 14782 30230 44107 50251 32709 13537
        36033 49727 46216  1913  2457  7114  7109 45972 32385 32433 32675 50290
         4242 34145 49037 45932 45927 44435 45970 41641 24083 15432 20384 49865
        49837 14390 14389 21176 14308
   854:32336 46574 37547 49874 33489  4682 36484 52649 23298 17230 36340 37761
        46425 18048  5522 13331 35976  6486 47349 18858
   855:29756 49842  4058 12248  6992  6991  8559  8558 44632  1502 47832 16134
        12261 37793 29690 29658 11280 31163 11223 15523 12604  1529 42248 18918
        17991 46428 27191  3153 35937 46559 39909 23019  3391 50263 24471 49995
        23146  6492 10082  3438  9364  9089 16031  5080 40575 37474 18200 46141
        10118 47285 36296 45316 11512 48924 34032 26121 17724 17263 47202 44482

   856:28607 28606 28603 28602 50110 28578 41848 45587 19376 33587 20677 20943
        47393  9590 49602 11546  2304 38375 49359 21369 13270 13108 21584 41567
         7956 22764 52734  3184 33256 48023 48022 17638 17635 39386 32349 17583
         1178 43718 34630 29364 12445 41938 46630  2484  6978  2912 28555 15590
        47278 44927 26324 51105 28636 28553 28549  3595
   857:29365 42676 22566 36194 44981  1554 13958 13957 23432 52373 34003 34255
        48079  8841 16024 16006 16029 16025 41229 33704 28044 47777 39035 42758
        40603 28822 20498 20559 25945  3558  5114 41239 33760 33631 36262 19953

   858:41024 41026 15736 35582 11043 28798 22912 44195 38249 35954 16769 17596
        25343 16422 16426 30194 21440 19587 21439  5644 15975 43385 20866  2692
        38425  5204 40084  8713 40085 30795 38685 30794 43080 48506  3253 12662
         5392  4632 50829 44790  5769  4907  4917 28150 24080 50086  4764 51079
        30606 35030 15133 11764 21669 21401 45115 52507 35054 33328 37861 49829
        30252 36749  5199 45811  8643 44042
   859:30868 43645 23121  8952  7241 38819 17012 51126 48997 47615  7857 48579
        33063
   860:29365 18635 28266 10124 33007 45839 25465 10777 30089  2376 12014 47961
        30105 43779  8246 46576  8222 41552 23432 52373 34255 48079 12182 41225
        33704 33703  8982 33700 12038 12041 51652 15869 32665 18908 16361 25382
        20559 25945  3558 18714 25536 22626 29318 28441 20940 21075 34710  5008
        29777  3918 15607
   861:30868 43645 23121  8952  7241 38819 17012 51126 48997  7857 48579 33063
        19516   859 15760
   862:31055 10335 18268 46719 16131 44944 42512 32362 32386 52200  7736 22865
        10067 34419 38885  9634 47724  7691 41770 10362 49809 25401 25404  4294
        15902 15887 11278 11279 19770 48744  3482 48857 24866 42306 19130  6871
        51591 43062 13138 49206 38227  8197 15773 15792  5110  5115  5112 11251
        11276
   863:49868 46963 36179 46509 34974 51516 18940 13109  9145 22974  6527  5081
        21449 27480  3407  5973  2532  3671 29357 24237 48769 42007 21425 20411
        31547 38300 29156 35157 14063 43964 46486 44523 16744 21277 18361 17208
        44062 26225 31144  6575 23047 42401 22899 40393 39487 33796 26145  3303
        19818  9136 34120 13093  2017 30526 52418  5603  6918 41616 44150 15123
        45147 17198 14680 15194  7299 19241 32930  9252 42065 22785  4942 26710
         2983 13652 43791 30278 44075 22660 45629  5231 51676  4576 36182 17756
        24238 37334 27284 34966 41757 40317 40340 42400  6214 34115 46364 23773
        16598 30833  9233 10518 36591 49374 52712 16339 40828 15141 24222 52754
        51425  2740 25806 24971 29237  2818 28014   864 35375 30580 39571 44616
         4695  5460 36399  1352  1628  4051 48454 50169 36941 30850 22324 13025
        23228 14169
   864: 6527  3928 17208 22899 26145 19818  9136 13093 14680 13652 43791 30278
        44075 22660 37334 40340 42400 41757  6214 40317 34115 46364 16339 40828
        52754 51425 25806   863  6466 29237  5460 36399  1352  1628  4051 48454
        14169 39571 30580 28014  2818
   865:36179  6527  3928 17208 22899 26145 19818  9136 29727 14680 50777 13652
```

```
      43791 30278 44075 22660 36182 37334  5794 15781 18249 20116 20088 46981
      46056 23424 25121 49535 21237 23311 30931 23773 49374 52712  7821 31357
      51936 40363 28995 24222 25806  2373 25293 29237 15125 17589 13293  5460
       1352  4051  1628 36399 48454 16579 49034
  866:12166 43909  8629  8608 46793 34214 34066  2536 51367 33016 28338 31255
      39292 51402 22056 38338  8636 20759 46281 48044 47667 17685 12668 13425
      18443 37201 12495 52164 15032 49778 13651 39281 42867  9204 34292 32231
      35789  7314 37273 19956 20373  5924 37084 44152 32410  2624 31958 43895
      51519 15116 30086  6813 27175 52648 41554 36377 46992 48880 47445 39406
      24824  5867 39597 31100 40578 16087 30673 24317  5208 47363  9295 18407
      36490  1493  1612 22453 25323 27020 11652  6900 52495 26133 20281 33518
       1668  9283 42185 25294 26228 11836 43356 11888 43559 26171 27114 25268
      36714 13189 19418 43242 18794 49154 14583 42832 37109 31094 25316 46504
      10908 37387 37210 33330 29160 31052 16678 11265  7708 35203 17592 38387
      11375 26964  5946 19867 19911 39245 28691  5073 18161 23112 10533 30003
       5843  7427 12889 31204 48353 17931 31381 34856 20356 46825 48282 38395
      46604 25521  4236 43561  5210 36400  9172 17188 43749 17943 20273 47750
      35676 32389 30874 14237 35905 48445 32713 37063  6445 45466 18066 25850
       8666  7960 36955  6541 18211 20376 24324 51488 16957 46804 40032 41614
      19643 51747 21930 47223 23215  2483  6324 42014 18867 38032 34533 12560
       5950 12461 33919 36087  7894 21480 23931 44703 35003 13501 20828 45486
      24093 17511 25885 11828 48021  1672 33215 10387 26739  5212 44826 36961
      29287 12863 29801  3285  5027 47975 21735 44645 17837 47763 28373 51093
      32001 45003 51317 23060 31557 30528 16271 38551 23940 38995  3377 31019
       9053 39911 14183 46741 41704 15062  5965  2654 14888 16546 19633  9490
      25203 11485 39199 15265 18028  8068 32588 42198 15852 35886 52452 49250
      16094 37526 15033 49501  7900 41604 31243  9873 24974 35416  7621 10779
      25642 35164 18453 29171 40060  6556 16068  3579 40731 24092 48517 45025
      35286 48100 44506 39247 17211 47851 25640 18224 12325  1636  6906 10238
      41451  6573 51255  3627 28906 10212 14959 32313 18468 13908 42708 32950
      27823 52304 37646 31225 21942 23092 12105 17998 51482 12220  4055
  867:44487 43162 19614 30158 39302 24800 40865 29695 20669 18721  5305  7841
      45816 33647 11943  8389 32188 49472 49470  4694 39351 14122  3453 24537
      48012 21690  6108 38734 39924 19029 23136 49313
  868:44487 43162 30158 39302 24800 40865 29695 20669  5305  7841 33647 11943
       8389 37885 12441 29701 32328 27761 17271 25920  9872 26552 29129 43283
      31587 44332 21978 28712 33418   869 15548 32188 49472 49470  4694 29305
      13451 24537 48012 21690  6108 38734 39924 19029 49313  8223
  869:44487 43162 30158 39302 24800 40865 29695 20669  5305  7841 33647 11943
       8389 17271 27761 26552 29129 43283 25920  9872 20742 31587 32188 49472
      49470  4694 29305 13451 39863 49157 43445 43393  8223   868 34996 13132
      35837 24537 48012 21690  6108 38734 19029 39924 23136 49313 33418
  870: 7693 42923  6083 35519 10139 12529 43745  8995 43873 16515 10442 22531
      32769
  871:23029 23008 23010 27327 27325 11141 30687 26150 18070  4601 30090 38121
      32574 20262 44963  8863 14451 18759 33914 10177 10106 43921 11107 42085
       2617 40185 27962 49460 34501 39937 43844 40231 47411 47407 47414 47406
      29664 50278 17843 25011 51063   880  8092 28218 37450 30763 38083
  872:43482 43484 21844 21818 21842 21847 21815 21870 19202  6996 52446  1349
      19590 34666 16566 45761 19268 23583 11988 40919 16389 16394 16418 43210
      33479 33686 41365 18374 40582 40903 48620 43275  3364 22513 21138 47001
      28415 26397 26366 26360 26392 26389 26362 45523 18783 46256 19580  6355
      31680 45263 25108
  873:37910 10804 33885  2547 17543 22884 31529 29041 17148 52692  8753 20114
      33863  7768  5534  9208 13307 13272 29705 38169  3047 23489 10904 22292
      14100 29108 45737  3231 37674 37458  4270 20572 37917 46290  6384 43595
       7274 34479 33205  3798 46725 38111 18917 51548  9906 42686 45801 28879
      43465  5501 35288 20933 21383 18700 31399  6436 10836  8980 25644  5584
      22046  5450  3337 20585 11989 21030 28594 51201 31314 16725 37753 40535
      47348 18218 38489 24711 19183 28498 37906 36402 49459 50025  5306 49454
      15998 15990 15995 16021 16019 15288 12424  1890 41582
  874:24338 23824  9401 39432 40978 19972  8769 21722 44262  5056 51264 37200
```

```
     21787 25902 10936 39892 20148 49161 28133  6181 20650 27441 18969 38913
     32489 49992 13312 16184  4828 46943 41205 49071 10397  7339 19944 19943
     42648 36600  9207 44464 38522 14635 50234 50222 19920 19925 16718 35773
     41877 10336 48495  4419 23630 48028 48731 26927  6141 19295 18708 50827
     22913 19142 51054 46620 32471 19329 38095  5791 30076 25314  8446 52461
     28053 26441 24087 10529  9591  9276 26114 13172 14340  4820 21904  1721
     43379 36039 11316  3912  1741 18459 39927 19830  8081 37269 11687  5300
     23494 13246 38417  2683 23405 15143 45468 52034 29603  6592 30721 38087
     51755  3698 12524 35637 31353 44537 50024 10445 10206 25913 48114 48749
     42791 21387 10849  9266 12048  9219  4116  7271 28370 28290 40308  9610
     11081 43082 30667 25043 49756  9104 49650 34424 22732 48989 41173 26308
     16699 16680 16676 30080  6217  4625 15802 45918  5495 21438 25574  6431
     13075 26768 12531 23361 37631 11283 12169  2650  5963 35736 32005 24156
     16109 11054 19187 27669 38855 22614 49409 46907 42417 47872 18229 49020
     16369 28191 32627  1187  7083 20390 12627 22537  7011 38274 32561 49830
     42177 35429   743 35548 12488 33496   744 28273 38401   745  6680 31717
      7153 19504 19496  6630 12204 35896 15854 10450 31720 51661 30479 30477
     30474 25475 47020 19500 15335 23708 25477 47022 19535 19484 16915 23714
     15338 19486 19472 44530 38595 44534 44529 40739 15559 15654 15584 15557
     15625 15513 13703 13737 15517 13015 15482 15515 13711 13700 15552 15512
     13664 13741 15653 36470 13765 13767 50002 41834 41815 41857 41861 41820
     41837 41766 16425 21933  4273  1680  1679 14014 37815 14768 50231 16574
     50200 16607 50181 16568 14792 14771 14829 16612 14800 19898 16573 16602
     19899 19892 50225 14724 19918 19921 14832 16460 14835 16456 15777 15755
     15743 15750 16497 16490 15592 15690 15618 16543 15477 15478 15659 16492
     15710 15686 14764 14796 16669 25737 49950 14421 15704 33508 26226  2040
     38254  9790 47248 23190 17866  5128 17667
875:20141 42527 23023 46279 47219  9061 12831  5544  1488  8927 20286 37221
     48423 48586 50235 50932 34772 10138 37662 51475 44621 33585 13411
876:18260 42242 40235 28956 42139 41238 30379 39064 49795 49790 49794 31068
     18168 27471 44656 29339 39069 49817 39501 39346 16850
877:10999  7003 21150 17630 24207 25651 47999  1549 22486 45773 38065 17024
     36247  5651 40777 28916 39310  8846 32141 37980  6310  5366 10912 47719
     47721  6959  9635  6651 43407 22574  7108 41165  9969 38314 23022 15222
     15451  9540 35644  6529 49326 38304  9228 29049 11847 22442  9737 22283
     34629 43500 40374 25483 40209 44853 46146 23011  1900  3677 36693 38262
     39279 24020 33572  3252 25889 33079
878: 5309 16004 44194  6414  3379 34315  4020 31388 30527  8520 30530 38165
      8795 46442 47307 12760 14598 20058 23615 25745 15728 42434 11019  5739
     47728 18892  6958 51953 22040  2455  5321 39080  9484 46143
879:32336 46574 34762 50492 36721 14260 10421 24230 49874 21824 17230 36340
     24566
880:23029 23008 23010 27327 27325 11141 30687 26150  4601 18070 38121 30090
     32574 44963 20262  8863 14451 43921 10106 33914  2617 18759 11107 10177
     42085 40185 27962 49460 39937 43844 40231 47411 47407 47414 47406 29664
     50278 17843 25011 11984 51063   871 38770 37450 28218 30763 38083
881:15657 10734 43594 49726 29080 10974 25156 29181 29180 29163 29182 29185
     50571 32411  2086  9979 18676 18679 27571 20500 43509 43512 29039 29042
     29045 29075 29187 43669 39923 39920 39925 39918 39895 45358 11976 20678
     52428 29019  6193 35385 30206 14144 16090 15336 15277 17072 16455 16372
     45694 45722  1532 47781 13618 19861 12155 35920 39813 39814 39819 39840
     39822 39791 39839 29290 12595 15631 15626 15629 22272 18869  8820  8332
     18673 28015
882:30415 30416 46613 29330 49065 49060 47183 45703 30351 20079 30439 24463
     42900 14489 14313 35156  3020  3018 46028 13988 40986 40953  6048 45824
     52705 19046 35969  1716 36507 50043 34426 40216 43414 43413 22385 22391
     11192 33711  5158 13919 19293 45087 40983 41796  2668 21618
883:10979 32409  5665 26313 25144 25145 25137 25141 43422 42362 42376 30746
     43707 52256 29783 43085 27023 27118 18176  2996 19890  3068  8187 49698
      8966 11826 11830 11823 11820 11827 32498 16379 19453 33992 17036 32554
      5402 11718 15789  8031 21727 13689 11409 18652 49839 21445 39100 45558
     48743 19864 38202 29468 13667 32052 32201 47620 32564 32800
```

```
884:18580  9726 13915 44998  3137 45269 17391 15603 52210 38467
885:24560 40743  9539 48941 37940 46472 33116 43711 10218 50693 32691 17898
    21873  3099 46034 10482 49249 19211 29243 22510 35626 49621 40691
886:44839 44750 36132 10750 29619 30405 21681 19037 36463 34667 32142  9264
     6097 14463 46832 12008 44634 29902 34725  4472 18293 18312 46622 46908
    27101 21038 33405 13179 44165  2488 45066 37565 25239 16236 47110 27700
     6975 29425 26012 12151 18944 15938
887:37668  2779 27006 24252  5324  4689  7119 28012 31718  7481 48457 41491
    51906  8763 37736 16078  3041 36272  8393  8143  8322 41489 38428 18019
    37892 16938 33484 20649 10858 51682 22465 49151 10358
888:32343 32339 13801 15929 23398 15488 37220 49481 14017 37135  8736  9509
    32905 16812  8949 29506 45273 51806 41713
889: 1356 16609 52204 24290 49453 20719 36740 12913 42238 48806 52453  3410
     8549 29221  2235  7633  4102 46434   890 33966
890: 1356 52204 16609 49453 24290 36740 20719 12913 42238 48806 52453  8549
     3410 29221  2235  7633 18332
891: 4204  5785 24478 30884 43649 43650 32382 33001 32383 32388 32380 37018
    46391 21948 34761 31597 22583 32998 10164  4136  7701 34929  7995 33942
     9533  7617 43416 19306 10731 47853 33909  9418 14871 12935 50786 21243
    29093  5834 18791  2639 47965  7055 34690 11867 39646 19964 39622 19908
     7988 36244 17075 50581 17078 17073 50580 39233  4898 45410  3044  9422
     9427 46703  7365 31833 47559   746 10597 24623 19361 22801 24662 24639
    42629 17542 12187 33708 50955 19182
892:12825 50261 26317 31564 16239 10573 21247 30357 36180 20270 10270 50868
     5754 25858 25881 32333  4043 19893  6424 26901 43149 27911  8887 12612
    15806 49703  9689 15811 32453 48978 39956 36359 21466 15829 29078  3929
     6386 12093 35359 32676 45974 36219 25036 42636 51597 11309 48438 24219
    11869  7990  7952  4996 10347 39285 34557 25746 40419 10186
893:12825 37899 31564 16258 10573 45042 41358 17116 44462 31744 50868  5754
    19081 25858 25881 12119 32419 32333 19893  6424 29226 36595 51140 32398
     4043 25151 26901 43149 16066 31294 12612 17338 17327 15816 17553 32453
    48978 39956 36359 15829 29078  3929  6386 42190 20015 15408 10458 40845
    26771 11869  7952  7990  4996 10347 39285 34557 16635 25746 19377 51004
    37453
894:41266 26687 29756 35444 35443 49842  4058 49848 29536 47832 16134 52232
    23134 14721 42978 11280 43179 43033 18918 46430 28239 23535 11615 40421
    23146   796 17082 31596 17792 51316 22526 31649  1690 43191 44994 27624
    49993 41936 24955 17746  5980  3445  8252  8423 33335 33333  8251  8205
    47276 20268  6302 43126 43063 47202 49737
895:29756  4058 49842 23944  8559  2286 16134 11280 41377 37876 39965 22765
    48584 37299 43958 17263
896:48881 26255 26989 49497 49084 11811 11837 33993 37315 11098 42874 29227
    30742 15747 15627 10912 30044 29404 29407 33807  3884 15582  6677   897
    35717 11788 43802 43173 40088 35339 16644 29161 31446 41034  4925 15845
     9718  6902  1878 29904  1875  5514 33969 35108 31780  5150 42688 38255
    19114 29511 24786 25413 21013 29842  2883 32399  5040 48646 41465 34123
    24004 31341 50821 34116 32745 38914 38572 15200 28522 43400 51973 37217
    15632 39466 15761 52604  5029  3705
897:48881 19192 26989 26255 49497 49084 43547 37866 11336 11811 40098 11717
    11837 35913 26404 27335 33993 36649  2114 37315  9375 11098 39394 42874
    29761 29227  5706 16712 18278 41292 30742 13336 15747 15627 10912 30044
    46137 18183 51629 15582  4925 13134 15845  9718  6902  1878 31780 19114
     5150 29842  2883 31341 34116 28522 51973 43400 32786 39174 39459 23140
      896 17831 13310 33094 45557 24389 15632 49588 39466 22747 15761 52604
     5029  3705  6677
898:40715 40748 40753 30668 16469 48826 10234 34565 21317  9504 38204 36807
    48651 26903 26516 12802 41290 50971 39117  7062  6409 13912 40097 52392
    16716 24393 12908 10225  5817 44325
899:49792 22286  6036 21700 23846  9464 32452 30229 23704  8766 17400 10945
    28132  2877  4855 11891 26647 23372 38930  7997 40222 45008 38150 34041
    28960 32195 45677 42042 46038  9746 10297 43454 21789  5393 25470 24277
    48434 29293 22264 19013 50338 48642 29943 29251 19755 19759 13046 19758
```

```
      23025  7086 21048  4743  3142 30715 17001 33130 33135  9529  8230 42119
      42143 44551 30036 35568 38416 20327 51167 23813 11001 49676 19471 44012
      20348 41138 29471   903  1768 39990 13188  9861 19574  8354  3003 10689
      43415 11344   900 33402 26604 24305 16237 36024 33764 52414 40272 38614
      13045 35197 47904  3573  2084  2048 12926 41578 11635 35221 23170 24205
      24243 12954  7351 47569  9688 50828 13790 14846  1758 51508 26371 36744
      29644  6947 15007 11737  6350
  900:49792 22286  6036  9464 32452 30229 23704  8766 17400 10945 28132  2877
      11891 26647 23372 38930  7997 40222 45008 38150 34041 28960 32195 38744
      45677  2343 42042 46038 21881  9746 39736 13858  6081 30895 10297 40871
      21789 31138  5393 14714 25470 13553 29943 29251 35471 15007 11737 12711
        899 13491  6350 23485 19759 13046 19755 19758  7086 39015 42596 17440
      21048 30715 46946 17001 12401  9529  9528 30036 35568 35544 18466 38416
       8497 34308 39710 34310 45164 23813 44012 16283 52222  8907 20348 41138
      20915 16237 36024 33764 40272 52414 38614 13045 35197 47904  3573  2084
       2048 12926 41578 11635 35221 23170 24205 24243 43151  8527 12954 47569
       9688 50828 13790 51508 26371 48830 36744 29644 15738  6947 26604 24305

  901:47010 49254 32954  4016 36724 36746 39426 35578  7107  6815 40318 25308
      31307 17093 24952  2260  2258 36532 50558 50655 50668 50632 50623 50629
      50590 50661 35713 50659 50664 50583 50523 50688 50553 50528 50551 50524
      50594 50526 50548 50555 50621 10609 28923 25332 28384  4931 23819 14470
      44855 19414 30879  3603 21073 23053 40226  2806 41372 41351 41368 41392
      41389 42493 42489 42463 42462 31435 50170 32150 32145 32112 47870 13357
       7465  9640 14983 27871 37766 38460 29295 34141 37948 10600 17994 25848
      22071 32556  3093 31069 23851 16161 31148 20138  6394 51959 41293 20608
      23388 24420 41340 26613 16709 51673 17005 36227  9117 10917 28622 51335
      22167 42333 47216 28148 32947 11080 35090 49967 40576 40736 44507 24235
      16432 40430 18177 19881  9240  9017  2220 33928 13891 50146 14567  1966
      35959 10916  2020 38648  1662 31692 13793  9914 43291 49822 49018 47722
       7520  8834 14465 14185 16960 21710 48868 49278 10541 27881  6451 42093
      42423  7344  8377 15543 17706 52064 16762 13048  4213 33706  9516 36874
      26559 19253 48971 25143 32132 25209 25223 21058 25952  8851 21861 37197
      24585 44885  7676  1729 48270  2558 42310 41833  4365 35528 33507 33571
       9772 13578  3939 38936 26850 19110 34326 42660 47671 26672 52156 21981
      13619 48611  3418 47717  7072 21962  4770 34994 28939 34591 20550  5480
      18718 38883  8376 24524 41060 14786 13519 29244 50679 20325 41625 21275
      37656 19144 12208 29764 18012 49881 35432 35153 39166 22514 30287 18545
       2867  2366 26240 44873 43255 19473 44805 33356 47589  9072 45218 25584
      25557 25576 25587 25559 25553 20969 22521 22519 12431 19479 29863  4364
      12000 20860 20891 20858 45029 10719 41177 21169 50033 24541 50236 34839
      49396 12172 11700  7048 51400 15476  8503 28382  8573 46000 22355 40545
      22516 41523 15357 42855  4127 15136 15186 42786 39616  1952  9005  2016
      30084 22547 22552 14398 50946 33248 23900 46820 46377  4366  6194 47148
      11711  8090  3368 28858 24493  5175 32282
  902:37465 39467 48488 19064 49286 49263 49288 49264 17850 49232 38410 30856
       8268  8214 49748 31458  5919  5900 22826 20387 32901 37549 49333  1525
      34648 18722 39871 13943 34599 30813  3214 36020  2095 28144 27939  4435
      46436 14247 41502  6718 41500 23483 35430 36345 42354 44849 38218 20509
      34925 35200 35193 34539 40806 38715 23414 16857 49617 38632 30809 32573
      44854 35220  7254 42572 41778 41703 23468
  903:22286  6036 23846  9464 32452 30229 23704  8766 17400 10945 28132  2877
      44064 11891 26647 23372 38930  7997 40222 45008 38150 34041 28960 45677
      20442 31141  2343 42042 21881  9746 39736 13858  6081 30895 10297 43454
      21789 25470 13553  5393 14714 40871 31138 29251 15007 35471   899 13491
      12711 11737  6350 23485 19755 19759 13046 19758  7086 39015 42596 17440
      21048  4743 46946 17001 12401 18466 34308 39710 34312 23813 49676 11001
       8907 41138 20348 13789 20915 36024 33764 52414 40272 38614 13045 35197
      47904  3573  2048  2084 12926 41578  8527 12954  9688 14816  1758 51508
      26371 48830 48803 44094 36744 15738  6947  8354  3003  1768  9861 19574

  904:18873 24013 18022 47496 23094 11561 40611  2771 17753 30446  2446 19396
```

```
      29561 36884 51949  8126 25570 51812 36079 13843 15946 15036 38858 49083
      14961 44485 36503  8516 37995 48784 31393 16275
  905:29199 32485 22203 22202  2103 21743 21186 43348 11839  5097  7830  4924
      39061 31253 27587 12958 47927 18736 41171 42520 11181 17219  1956  7883
      17245 15319 46445 33336 33340 25935 35335 48209 51359  6370 49997 23718
       6343 10455 19286 45543 16043 11939 27618 32479 17452 39541 50307 50280
      49700 46566  7043 51292 13949 40668 39620 35438 22438  4282  4952  9202
       8533 35748 42619 43819 43301 14190  7381 17089 47066 47008 14950 20848
      49518 30303 16704  7555  4814  1597 36309
  906: 9623  9622 16332 16357  4261 46642 39591 17025 14493 39914  3398 18730
      16303 17347 44904 24367 46618  1862 17466  5155 16358 43740
  907:13012 31223 41142 38965 27758  9356 19003  9291 13151 16891  3965 14912
      28656 25721 27892  9128 48603 47428 27655  3220 50739 37262 41370  1685
      50723 20493 36928  8468 49813
  908:43019 42034  8796 25930   752 37513 50943 10263 34104 26632 31380
  909:34975 21224 31836 47821 24339  8792 25307 18115
  910:12625 11589 24352  3761 13939  4299 42782 33520 52104  6910 16268 29312
      52251 38627 22308 24128 45715 23818  7200 30493 10968 34025 25827 26840
       2970 28442 23324 52357 48787 38308 16802 28237  9071 22122 37955 26928
      21146  9147  4103 40310 29126 30991 30234 26471 43487  5819 40254 22563
      42669 16767 16893 17988 25250 44023 39583 28262 10318 26355 29827 29682
      47792 34935 42300 52199 38026 46778 46386 23653 24336  8173 48227 37619
      22110 50166 13282  5182  4829 21768 29805 24489 40095 10796 52101 38573
      27043  2009 30393  6441 24720 18531 44007 29716 17883 51381 52289 13404
       9359 48220 28647  4701 41825  1663 15749 29632 44343 12367 27764 22010
      38615 27008 19105 33932  6400 17408 11852 29660 30546 23919 20495  2055
      31429 42760 48874 29011 50824 13388 42390 34182 12839 23079 26351  2588
      16010 46704 35927 29429 34893 20596 23836 44929  2489  8371 45302 33899
      40146 27166 33019  5004 45861 46880 12799 30830 21266 27510 50390 28394
      13453 44443 34976 30360 41470 11314 14812 46850 35945 50432 16765 10511
      38541 46755 47047 21808 47152  4861 47171 44666 31284 52490 37557 21684
      24930  4985 38330 33542 48362 45772 37026 31315 41788 33414 35113 52410
       4822  4585  3618 25033 43065 30377 22350 50981 39518 34195 12206 28665
      40765  5253 36126 46286 19986 36533 47534 43920 19563 36509 51448  8495

  911:12625 11589 18329 30320 11725 24352  3761 13939  4299 26893 42782 33520
      41649 39921 52104  6910 18477  6933 16268 29312 19280 52251 38627 27141
      17557 18480 22308 45715 23818  7200 30493 10968 25998 34025 17480 25827
      17453 17482 17537 18416 19245 17633 26840  2970 17423 17449 17588 17561
      17563 18366 18413 18436 18442 18460 18511 19218 19224 19250 19275 49975
      28442 18507 17395 23324 52357 48787 38308 16802 28237  9071 22122 37955
      26928 21146 28352  9147  4103 40310 41095 29126 30991 30234 44209 43487
       5819 40254 22563 42669 16767 16893 17988 25250 17489 17636 18440 19322
      44023 39583 28262 10318 26355 29827 29682 47792 34935 42300 52199 38026
      46778 46386 23653 24336  8173 48227 37619 22110 50166 13282  5182  4829
      21768 29805 29247 24489 40095 10796 52101 38573 27043  2009 30393  6441
      24720 18531 44007 29716 17883 51381 52289 13404  9359 48220 28647  4701
      41825 15749  1663 29632 44343 12367 27764 22010 38615 27008 27149 19105
      33932 25452  6400 17408 11852 29660 30546 23919 20495 44725  2055 31429
      42760 48874 29011 50824 13388 42390 12839 23079 26351  2588 16010 46704
      35927 29429 34893 20596 23836 44929  2489  8371 45302 33899 40146 27166
      33019  5004 45861 46880 12799 30830 21266 27510 50390 28394 13453 44443
      34976 30360 41470 11314 14812 46850 35945 50432 16765 10511 38541 46755
      47047 21808 47152  4861 17081 43331 47772 23100 47171 44666 31284  6574
      47720  1193 32902  8630 37703 31459 20624 37557 52490 21684 24930 17083
       4985 38330 47303 39991 34298 37808 26162 16189  5883  5565  5887 18419
      18479 19277 35041 17420 48362  4518 17640 45772 37026 38809  7525 45960
      35113 43890 52410  4822 19193 19221  4585  3618 25033 43065 30377 22350
      50981 39518 37154 34195 49747 12206 28665 40765  5253 36126 15362 46286
      19986 36533 47534 43920 19563 36509 23621 41709
  912: 8940 34385 50954 16488  2143 52740  8105 17244 38776 32615 45698 42287
      10309 19814 19381 14139 31231  2363 17535 26283 49486 14218 14571 16384
```

```
     2606 34548 34513 15297 39496 25296
913: 7329 51990 50935 51704 50928 51649 51645 51746 50990 51734 51705 50995
     51772 51763 51680 50964 50962 50900 51765 51737 51742 50892 51642 50869
     50931 50866 51675 28052 17167 34385 24997 50954 37771 22817 16488 52740
     33501 21082 21134  8105 17244 39689 33012 32612 43860 26357 33100 29391
     45698 42287 10309 26321 14139 31231 31039 29635 37349  5797 44456 13073
      3316 17569 17239  2407 26527 42680 12116 16384  2606 45484 49186 46046
     52541
914: 7599 43893 19436  7606  5836 14281 23126  4148  4377 45910 18445 48518
     27003 16053 29409 34880 28459 32878 28297 28295  3610 52095 37034 37047
     45729 10711  9019 34026  7625  8552 34017 31823  7627  7597 34024  2699
      2701 48481 48856 34018 48858  2703 46229  7602 43217 41289 17397 40155
     19309 31589 47917  2724 38933 28051 24261  7623
915:25237 48299 37531 38223  7359 27969  1624 41126 51302 14510  3147 48850
     29613  1196 51301 46587 25995 46363 22382 40827 21694 31550 32896 15723
     13429 52445 46024 38443  2140 38590 34973 38379 49260   916 19201 42279
      4562 36344  6100 17823 28979 51524
916:25237 48299 25408 37531 38223 27969  1624 41126 51302 14510  3147 48850
     29613 25995 46363  1196 51301 22382 46587 40827 21694 32896 31550 15723
     13429 52445 46024 28979 17823   915 29961 51524 20196  3376  9541 38443
      2140 38590 34973 38379 42279  4562 36344  6100 49260
917:31426 52337 26179 38599  9045 22097 34206 32332 12745 52589 50077 40645
     45145 33117 26519 13937 27542 47457  6691  3973 10707 25628 24850 47244
     18855 18803 17127 33931 17793 49392  2156 19285 26955 51101  6524 39076
     23910 39621  4265 49217 19264 27642  4092 25734 19267 48926 34968  8344
     48410 44268 42761 11084 14240 37616 10105 12865  6305 10310 21252 24553
     49655
918:31426 52337 38599 26179 22097 34206 32332 12745 27731 52589 50077 40645
     45145 33117 13937 26519 27542 47457  6691 10707  3973 18855 24850 47244
     33931 18803 17127 43993 11998 49499 24377 51101 23910  6524 39621  4265
     49217  4749 42525 33090 25588 25654  5727 30790 42761 10105 11084 14240
     37616 12865  6305 10310
919: 7486 23255 15525 36321 34712  7544 38748  2036 37730 32550 22638 16478
     16873 16872 16875 44184 44186 17598 16628 39843 41269  9481 44682 42863
     42234 52264 10668 52657 37591 41223 22748 25699 30264 33351 21562  6225
     45464 31109 14570 47208 30622 24355 35083 30447 19692 51767 15863 26570
     18970 39204 23363 46591 48814  5350 17481 32072 52610 26711 26039 50087
     25732 36386 40497  7479 48200 17392 11790 20374 51147 43267 40737 30565
     47431 43927 12983 27165 23645 23668 32299 33756 15437 31683 11789 41376
     48639 42416 45017 51363 13952 49864  3724 40785 49880 51442 51163 35709
     30796 20534 10410 48297 28658 19688 46055 39445 23661 41574 24619 20183
     24863 48181 22834 34042 44732 24135  8787  9854 14255 17253 47040 21076
     20163 49945 42950  5941  5401  4339 11762  6930 28154 25992  3308 25621
     26018 47702 32022  5399 17108 22145 43736  3934 24139  2324 23484 25817
     51884 25722 25719 51034 35842 37732 15324  8110 35330 10730 14636 36841
     43590 32926 48338 13428 34767  6837 13380 10213 42393 35721 43623 46060
      2167 21014 21297 13608 39676 31410 22963  3455  3456 24691 26476  3187
     34793 51823 34770 34769 34771 27809 28256 49302 52477 40348 34795 34798
     48715  7529  2693 35997 31924 22025 10153 25975 44063 15921 26000 34877
      2534 19254 46007 47310 49773  5167  5169 35148  5615 51584 26272 12773
     46822 44738 48451 37272 46252 18425 15174 16487
920: 7486 23255 15525 36321 34712  7544 38748  2036 37730 32550 22638 16478
     17598 16628 39843 41269  9481 44682 42863 42234 52264 10668 52657 37591
     41223 22748 25699 30264 33351 21562  6225 45464 31109 14570 47208 30622
     24355 35083 30447 19692 51767 15863 26570 18970 39204 23363 46591 48814
      5350 17481 32072 52610 26711 26039 50087 25732 36386 40497  7479 48200
     17392 11790 20374 51147 43267 40737 30565 47431 43927 12983 27165 23645
     32299 33756 15437 31683 11789 41376 48639 42416 45017 51363 13952 49864
      3724 40785 49880 51442 35709 30796 20534 10410 48297 28658 19688 46055
     39445 23661 41574 24619 20183 24863 48181 22834 34042 44732 24135  8787
      9854 14255 17253 47040 21076 20163 49945 42950  5941  5401 11762  6930
     28154 25992  3308 25621 26018 47702 32022  5399 17108 22145 43736  3934
```

```
       24139    2324 23484 25817 51884 25722 51034 35842 37732 15324    8110 35330
       10730 14636 36841 48338 34767 13428 13380 45431 45403 13226 45405    4999
       30249 15326 45349 39737 49565    5020    6852    6850 29458 29462 29478 13608
       14442 46678 39676 20277 22963    3481 49126    3486 48785 21658 48782 30602
        3190 51825 34771 34770 34769 32725    1861 31040 49302 28256 52294 15757
       23163 34798 34795    2693 35997 31924 22025 10153 25975 44063 15921 26000
       34877    2534 19254 47310 49773    5169    5167 35148    5615 51584 26272 12773
       46822 44738 48451 37272 46252 18425 15174 47104 33881 49699
921:13266 12777 48505 12380 26575 16708    9384 52678 13041 19695 17748    1967
       29750 23428 24142 33186    8697 52689 37663 34625    1646 15180 17754 32119
        1199 36052 51572    9009
922:45923 42445 26600 21469 13209 42426 43428 15304 33098 19886 45717 11308
       49731 15272 40298    6692 47123    7639 19604 16742    3259 18862 42899 36342
        3150
923:25706 36847 38610 12047    9196    7931 45582    3416 41146    1365 42211 11070
       36381 10799 51962 44914    6105 35625 31427 44417 14291 50700    8249 30995
       14368 20986 43388 17864    1200 10322 36877 40780 28681    9378 39958 43539
        2881 38758
924:25706 36847 38610 12047    9196    1955 46635 45582    3416 28483 16672 47951
       28320 30215 47890    7443 41633    1201 47190 31847    5753 14062 43539 27058

925:24940 25706 36847 38610 12047    9196    1955 45582    3416 28483 16672 47951
       28320 32213 30215    5753 43539 11563
926: 7259 26533    2564 46567 31528 22512 45260 21142 48298 16224 15596    6903
       15110 36473    9211 11562 10802 20134 29016 20605 11048    9446 30671 17196
        5381 49233 49213 33621 13572 29036 22837 15984 36007    5249 28717 23602
       36808 36217 15213    5067    9949 25001 18796    6553 52033 48311 50542 44127
       26833 24543 21559    7559 35830 28966 47374 25373 26561 27645 39915 18290
       43703 35270 38929    6925 23226 51490
927: 7259 46567    2564 22512 31528    2669 45260 52197 21142 48298 17493    6903
       15596 16224 15110 46497 23299 36473 13763    5332    9211 13750 22487    8129
       16805 49213 49233 14667 14258 24448 23941 27693 36268    6828 34087 30271
       51940 36007 46163    5067    9949 37243 18796    6553 48311 52033 24828 50542
       17224 17221 11860    3514    936 26212 24543 21559 20903 39114    7559 35830
       46491 27645 39915 18290 43703    6925 23226 51490
928: 7259 26533 46567    2564    2669 45260 52197 21142 48298 17493 23990 24907
       35850 39587    2537 11467    6903 19686 15596 16224 23299 15110 13763 36473
        5332    9211 13750 13775 22487 19753 16219    2567 11562 10802 20134 36888
       29016 20605    8564 11048 22577    8866 12017 15367    9446 30671 17196 22579
        5381 37022 49213 49233 13828    8255 33621 14667 36350 32584 28281 45331
       17304 39631 13817 32027 21884 36756 52728    9714 21001    7071 48650 15213
        9952    9949 18119    4211 11542 24137 52033 48311 48313 50542 17224 17221
       50216    9473    6974    940    3460 20974    1771 48794 24543 21559 35830 20903
       39114    7559    3605 46491 39120 21350 16944 23705 17058 27391 36434    1654
       35521 35517 46150 45221 50411 28966 47374 26561 30355 52223 25373 49634
       27645 39915 18290 43703 35270 38929    6925 23226 51490    4048
929:31889 29986 28242 40771    8139 28207 26390 28040 28042 28057    9956    9958
       13787    7940 50100 39435 30694 11898 43758    2454 42415 10524 38719 21283
       24172
930:29986 28242 40771 37003 31687 28429    8139 28040    9956 13787 15748 46535
       36292    929    1677 24172 11583 36813 50100 22310 29641 50957 39435 30694
       20568 36228 14008 52595 11898 14863 37309 42415    2454 33877 43758 20137
       22628 10524 38719    850 23416 26016 21283 29113 17997
931: 7259    3131 16759 16754 26533    2564 46567 45260 52197 21142 48298    6903
       15596 16224 15110 46497 23299 13763 36473    5332    9211 13750 11562 20134
       10802 29016 20605 11048    8564    8866 15367    9446 30671 17196    4054 16805
       33621 13572 51940 36007    5067    9949 24795    6553 48311 52033 24828 50542
       12293 47482 24543 21559    7559 20903 35830 39114 16944 27391 17058 23705
       46150 28966 47374 27645 39915 18290 43703 35270 38929    6925    2611
932: 7259 26533    2564 46567 22512    2669 31528 45260 52197 21142 48298 17493
       41990 23990 24907 39587 35850 11467    6903 15596 16224 15110 46497 23299
       13763 36473    5332    9211 22487 11562 20134 10802 29016 20605 11048    8564
```

```
          8866 15367  9446 30671 17196  5381 49233 14667 29036 15984 36007 28717
          9949  4785 18796  6553 48311 52033 14804 50542 44127 24543 21559  7559
         35830 20903 39114 16944 46150 27391 17058 23705 45221 28966 47374 26561
         25373 27645 39915 18290 43703 35270 38929  6925
     933: 7259 26533 46567  2564 22512 45260 52197 21142 48298 23990 24907 39587
         35850 11467  6903 15596 15110  9211  5332 11562 20134 10802 29016 20605
         11048  8564  9446 30671 17196  5381 49233 14667 50597 49551 23084 29268
         20429 15984 28717  5249 23602 12332 25005 18814 38312 36217  9949 18796
          6553 48311 52033 24828 43556 50542 34344 24543 21559 16944 27391 17058
         23705 28966 47374 27645 39915 18290 43703 35270 38929  6925 23226 51490
         43968
     934: 7259 46567  2564 22512  2669 45260 52197 21142 48298 17493  6903 15596
         15110 23299 13763  5332  9211 11562 20134 10802 29016 20605 11048  8564
          9446 30671 17196  5381 49233  8255 14667 30490 47625 51940 35686 15213
          9949  6553 48311 52033 24828 23761 50542 36339 24543 21559  7559 35830
         20903 39114 16944 27391 17058 23705 46150 47374 26561 27645 39915 18290
         43703 35270  6925  2611
     935: 7259 46567  2564 22512  2669 45260 52197 21142 48298 17493  6903 15596
         16224 15110 46497 23299 13763  5332  9211 13750 16805 49233  8255 14667
         52548 33291 25019 52523 33949  7491 11901 51940 36007  9949 50987 16158
         16157  6553 48311 52033  6010  2450 21569 50542 17221 12293 24543 21559
          7559 39915 35270  6925  2611 37485
     936: 7259 26533 46567  2564 22512 31528  2669 45260 52197 21142 48298 17493
         23990 24907 39587 35850 11467  6903 15596 16224 15110 23299 46497 36473
         13763  5332  9211 13750 22487 19753 16219 11562 20134 10802 29016 20605
         11048  8564  8866 15367  9446 30671 17196  5381  8129 16805  9250 33621
         14667 14258 23941 24448 27693  6828 36268 34087 30271 31135 51940 46163
          5067  9949 37243  2329 18796  6553 48311 52033 24828   927 32367 24376
         44137 40912 50542 17221 24543 21559  7559 20903 39114 35830 26823 16944
         17058 27391 23705 46150 45221 28966 47374 26561 27645 39915 18290 43703
         35270 38929  6925 23226 51490 26212  3514
     937: 7259 26533  2564 46567 45260 52197 21142 48298 17493 23990 24907 35850
         39587 11467  6903 16224 15596 15110 23299 36473  9211  5332 19753 11562
         10802 20134 29016 20605 11048  8564 30671  9446 17196  8255 33621 36007
          5067  9949 18796  6553 48311 52033 50542 34344 24112 24543 21559 35830
          7559 20903 39114 16944 23705 27391 17058 28966 47374 26561 27645 39915
         18290 43703 35270 38929  6925 23226 51490 35702 51846 22084
     938: 7259  3131 16759 16754 26533 46567  2564 31528  2669 45260 52197 21142
         23990 24907 35850 39587 11467  6903 16224 19686 15596 23299 15110 36473
         13763  9211  5332 13750 22487 11562 10802 20134 29016 20605 11048  8564
         15367 30671  9446 17196  5381 16805  9250 13865 13893  8255 33621 14667
         31891 38316 21054  4509 50956 26088 22041 15984 36007  5067  9949 30657
         18796  6553 48311 52033 24828 52059 44869 50542 16679 24543 21559 20903
         39114 16944 27391 17058 23705 28966 47374 27645 39915 18290 43703 35270
         38929  6925 23226 23568 30940  3111  4617
     939: 7259 46567  2564 22512 31528  2669 45260 52197 23990 24907  6903 15596
         16224 15110 23299 36473 13763  5332  9211  9446 17196 30671  5381  4054
         37022 16805 13854 50521  8255 13572 14667 48979 45122 51940 14131 15213
          9949 24948 37215  6553 48311 52033 24828 44734 50542 17221 22903 22262
         24543 21559  7559 35830 46491 39120 21350 20903 39114 16944 17058 46150
         23705 27391 45221 47374 39915 18290 43703 35270  6925 33625  2611 16304

     940: 7259 26533 46567  2564  2669 45260 52197 21142 48298 17493 23990 24907
         35850 39587  2537  6903 19686 15596 16224 23299 15110 13763 36473  5332
          9211 13750 13775 22487 19753 16219  2567 11562 10802 20134 36888 20605
          8564 11048 22577  8866 12017  9446 30671 17196 22579  5381 49213 49233
         13828  8255 14667 36350 32584 28281 45331 17304 39631 13817 32027 21884
         36756 52728  9714 21001  7071 48650  9952  9949 18119  4211 11542 24137
         52033 48311 48313   928 23400  4048 50542 50216 24543 21559 35830 20903
         39114  7559 46491  3605 39120 21350 16944 23705 27391 17058  1654 36434
         35517 35521 50411 28966 47374 26561 30355 25373 52223 49634 27645 39915
         18290 43703 35270 38929  6925 23226 51490 20974  1771
```

```
941: 3131 16754 16759 26533 46567  2564 31528  2669 45260 52197 21142 48298
     17493 23990 24907 35850 39587  2537  6903 16224 15596 19686 23299 15110
     36473 13763  5332 13750  9211 13775 22487 19753 16219  2567 11562 10802
     20134 36888 20605  8564 22577 11048  8866 12017  9446 17196 30671 22579
      5381 37022  4054 49213 49233  8255 13572 52728  9714 21001  7071 48650
      9952  9949 13627 24661 11542 24137 52033 48311 48313 24828   834 16863
     35233 46012 50542  4644 13064 24543 21559 35830 20903 39114  7559  3605
     46491 39120 21350 16944 23705 17058 27391  1654 35517 36434 35521 45221
     50411 28966 47374 26561 49634 52223 30355 25373 27645 39915 18290 43703
     35270 38929  6925 23226 51490  3193
942:24411 37005 12345 33629 35015 51313 16280 35571  6369 16761 52290 36815
     20832  4961  5680 29875 18748 16739  5051 48184 34258 30136 41895 24587
     23065 23066 44357 42539  6616  4520 25799
943:35444 35443  1496 16134  1769  9563 37060 37058 11280 50165 45783 17991
     11614 39909 24471 49995 32486 33139  8478 22526  6832  6867 38205 19771
     12344 12398 30122 52124
944:11745 31544 30974  7393  8103 41721 13758 51566 43645 10788 50273  4195
      5234 17012 24041 20700 49139 47615 14726 29917 21921 15039 40996
945:29905  5099  1181 48342 12532 46972 19794 47955  6134 10568 14674 39370
     25832 16385 10753 37319 31248 48761  7866 43974 28461  8590 14013 13213
      9385  9823 26595 31286 37878  8417 14860 16990 38576  6964 28128 48204

946: 6984 47227 15611  5661 39510 49533 26535 18116 39623 48988 49598
947: 3257 49089 27636 22044 21315 47282 46817 47621 18829 52424 26294 50106
     38041 37923  6504 20545 43261 47381  7571 51336  6749 15300  5417  3336
     46241  7772 31395 49360 43222 33329 14464  3734 39313 41513 24409 38110
     47613 22174 11239 16276 47199 13847  4434 14813
948:11215 36320 14864 34170 22936 34174 16651 17350 17135 38575  3635 16222
     46652 52022 17346 37366  6597 25257 39081 22302 22296 13861  9624 10023
     10021  2578 45858  4189 25274 22378 17705 18421 28505 43699 35903 44761
     39224 32551 32530 37924  6814 10459 18091  6433 36622 36619 46616 30736
     18698 33903 26369 36894 24410 45626 37218 11163 33264 47126 43566 15553
      8591  7182  7227  7180 45993 23105 45734 50743 31535
949:12801  8948 16817 27861 11022 14045 44557 52336 44228 38876 36257 12754
     37463 25838 18071  7185 10816 10819  8932 27880 43843 45113 45220 19048
     29882 27883 46254 33268 49155 27882 44619 51293 33251 35146 12581  3478
     40416  7943 18343 24710  5609 20833 29241 24198 22571 25593 42717  6326
     41678 39133 16782  7020 39850 50728 31436  1588 35838 32511 47567 45535
     51989 40141  2524 35964 52605 24864  7509 30958 17280 22037 15758  6533
     41200 51435 37176 12465 27819 37841 20248 18993 20813 41533 32298 29633
     19204 14798 28270 17718 44099 46136 18328 52535 38407 35719 21127 11106
     51325 49620 20278 51075 28374 52084 18723 40041 38779 12257 35851 46938
     45661
950:26510 35008 24745 51314  3594 17365 26508 17590 11511 22956 13753  1974
     10663 26058 15237 23512  8136 38531 50969  7396 31293 36395 20778 38100
     35382 41744 46052 21475 21477 40492 29184 26500 26759  9999 48725 47856
     20186 20263 17199 46953 45288 51236 48695 28609 28590 51492 45399 38163
     47277  1787 28490 50123 13924 14287 37298 14346 31114 14962  8238  7985
      5679 48533 48405 18563 41920  2502 24514 52711 36902  6757 52228 12127
     28501 37131 14632 14195 11962 15836 19430 10074 21713 17996 52319 20708
     38435 22287 47948 28366 44783  9066 46766 35742  5917  5287  8012  4928
     27547  9292 24466 37969 41638 20506 26531 21351 52751 21604 39263 17956
     32767 38033 35839 30570 12374 10746 32491  4496 42077 37568 51449  9415
     39581  8456 20720 33013 42175 42173 41477 16735 16743 40706 16959 22692
     22695 18842 15943
951:50318  4293 18398 35268  2974 52161 33866 43842  1814 37983 38621 43399
     19508  9588 45185 16906 45483 29110 41925 33988 41539 18999 48513 19569
     26566 19847  1888 30655 37355 51951  9707  9709 25303 50260 48951 46515
     22427 21653  4359 26766 28039  2715  9778  4370 26464 36768  3958 13974
      3600  8669  9545 24332 47599 50970 14404 19821 52380 42269 24437 22400
     19220 11178 52011 29127 10831  9085 33109 17940 27432 20444 26429 52040
     46383 14155 10476 41693 27326 28111  4454 45749 22210 30914 45915 45913
```

```
46167 15359 38611 35596 27610 34399 21670  8946  4490 43172 11416 37420
28558 12855  5547 27025 49816 45421  4255 22757 16295 40516 37385 38854
20251 13256 13257 30663  6040 34740 40147 47775 46385 25016 50619 27206
41379 39580 43150 29112 45898 40534 39578 21976 25438 45041 45922  4118
52451 17631 44255 24891 14831 48895 35296  5449 19948 23677 45083 40256
44497 16908 35127 25973 50921  6202 48418 30674 35343 27833 32897 29324
17225 21610 24381 10038 27007 23585 49102 40640 49225 49349 38502 38503
18010  3995  3976 46696 45800 18321 24847 45533 30383 32927 11551 15073
14647 43041 12537 31981  1682 27739  9181 52547 26042 27728 14381 14378
19460 20170 21055 49596 15158 24848 52693 36538 41596 11288 12004 15674
15830 49941  3435 28588  3437 25674 26186 26723 26726 21123 36275  4425
17907 44420 36996 51851 50795 40789 18850 18852 13929 50289 29954 51528
16054 35868 36701 15063 15411 18184 14921 43161 43166 19854  8813 33306
33308  2400 22548 50330 23176 51736 50934 21184 20184  6986 35482 23460
 8472 17351 32945 10214 17887 17064 29509 49899 20027 16697 51847 45934
 5776 47471 47492 40825 48292 24031 18473 37965  9662 33092 32785  7325
15309  7269 16437 23803 49170 15741 41573 24408 14855 14397 28620 26435
49752  6216 33419  6229 46062 42406 22026 49081  9621  6103 13809 37370
12003 25906 29447 35768 20019  1833 27745 47801  4384 24472  4840 52093
50295  9084 14312 30037 10841 35357 40884 14216 12411  5369 12045 16813
43916 39964 48588 50364  3146 29979 34415 35916 46952 25816 13914 31084
11846 52377 18756 31461 24785 44568 10692 31562 20446 43196 31829 38797
16657 43092 50047 33600 43297 45501 26624 40178 22363 51309 48193 20944
20513 25015 48082 43070 21536 28507  1538  1539 16324 45620 24736 13149
15888 37085 36824 25276 38910 47793  5464 37048 46051 23116 36361 13850
31305 10437 33581 23724
952:52106 34417 24542 21463 11027 16968 42837  8570 20840  5427 16178  4991
42050 40450  7164 25703 17360 44074  1811 32290  9837 18843 49940 18173
 1882 27276 33539 29721  4790 11361 18911 31189 16155 20693  3769 40901
 1827 21639 12438 41530 28387 42231 33962 26497 44305 34467 43762 13806
46379 11208 27673 19184 44979 17212  8572  5133 24319  2218 40770 51910
33486 51638 35693 43581 11777 17222 44241 25064  4590 46636 23524  1485
13037  7430  6985 50826  4624 23896  4245  4767 45873  7637 11349 29452
19332 31229  8702 43776 25478 13229  1828  4692  5784 46948 43824 37406
39826 11145 16047 17799 51741 32794 35798 28749 19666 13178 19624 15858
48930 46265 30658 21015 38915 29755  6531 23028 27926  3888 32683 34515
13114 18199 27980 26877 28023 27119 51099 16005 52503 35866 51556 39039
 7087 51276 44471 21018 17899  3400 28153 32848  9824 39257 35591 48359
13933 30179 27687 51290 30425 16434 48281 11190  2674 27671  1543  7495
 3972 29025 47665 49998 31183  2570 48954  7882 11537 24613 52614  9004
50449  5777 41840 43672 33707  2377  4382 37746  8116  9079 16114 26745
 1577  4343 52571 10194 46689 26084 37932 41510 30585  4463  2014  3160
39752  3012 49224 42587 13354 20204 30366 17191 40205 16526  6523 29662
48877 40666 45065 29466 20368  6444 31518 38756 44759 42399 26334  5729
12981  6911  4684 35248 46795 43281 51395 14337 50911  7970 32314 19311
23034  9342 14365 42969
953:16013 43888  3284  3287  3916 35271 30815 49416 18099 18109 18104 14479
18140 18145 14478 18080 18148 18769 49274 15314  1780 44034 10554  1006
22023  3538 35933 51686 35116 16832 15499 49546 32952 14532 18770  7251
32122 12696
954:10844 35671 35708 33514 35705 35703 35727 35724 45400 36672 36674 36713
25284 52162 10902  6884 40015 36735 36731  7519 22759 36737 36739 36743
36762 36710 44111 37774  9487 10785 36771 36770 19945 48196 22949 22984
22951 18916 46270 22475 12273 39735 18123 22027 22034 22038 38991 22030
51923 15470 44016 11668 11008 36652 10906  5303 12192  1506 35594 12394
12635 47874  2273  7410 45298 48458 25598 52305 25966 25968 12189 12140
 6803 12162  6806 29637 33383 25400 25397 34437 37226 40581 27419  3780
 3807  3782  3783 33799 10417 31862 31885  5091 23076 42339  2754 12164
24994 24992 12158 18118 12171 11695 16273 35445 23991 18147 34463 12193
 6809 12194 12197 12348 30293 17888 30848 52385 38701  7573  7752  7751
 6298 47045 23042 20558 32625 32609  8458 37758 10829 10825 22502 37757
28236 17723 38692 17049 21270 15906 18912 49786 16732 16050 18651 10929
```

```
       10926 10927 11041 16296 36766 36767  3525  3495 12168 42335 39439
955: 2099  2410 13091  3174 37296 28313 17516 12132  6664 23470 39470 16473
     6665  6456 32806  2727 39624 42278 47796 28481  3830 38138  9609 43966
    19540 19723  3670
956: 2099 21943 29753  2410 10566  3174 37296 28313 17516 12132 16473 32806
     2727  6664  6456  6665 23470 39470 42278 39624 47796 14057 27532 10944
     1983 28481 29704 23563 20051 52151 33159 51881 24612  3670   955 49228
    35107  3830 38138 44908 16675 19540 28598 19723 38161 28363
957: 7394 37914 26289 37061 17249 19865 22904 36441 32996 43152 11644 24310
    31491  8601 46224 48576 41597 22875 29656 14853 11347 17310 16686 11421
    40120 24076 36615 15056 31136 33867 11261 32534 51723 50589 52338 46174
    34193 34153 45807 43169  5728  6446  5772 20047 38994 26971 37227 45769
    18859 52704 13362 19300  6493 27616 13160 27705  2105 15472 32154 23486
    39355  5626 31257 20221 24169 31423  4522 29832 17936  8102 10743 14754
    31532 33282 35076 28046 13274  3415 28721  9027 40586 29102  7312  7002
    22595 19877 27362 39206  9734 12170 25385 19497 47643 41480 37880 49110
    27936 15849  7782 47421 21091 48955 27399 27343  3848 17431  8894 15399
     5362 44728 28452  5979 26796  4621 12520 18409 21481 25886 10629 42043
    45285 19744 49322 16216 10041 13577 13461 34645 41216 44815  4186 31743
    24544 39546 31282 10154  5225 27009 30100  3071 46121 46196 39690 40772
    24124  7891 34982 33045 45563  4254 43187 24147 26713 45280 34583  5807
    40541 17585 38498  7144 18426 37151
958:47603  6676 40820 22624 36411 49520 18977 33800 47548 18732  7091 47279
    42697 42338 41930 13507 36696 23660 51459 27122
959:21484 18205 11197 15588 13292 11045 37480 33154  6126 30542  3682 10253
    49493 35645 44349  1898 52462 16079 26708 18765 10205 20563 11776 40711
    19372 19371 24337 30078  5787 29144 33403 13529 20617 45402 21198 44419

960:46101 29621 38865  2859 46217 39146 39099 44752 18924  5160 42729  9049
    37527 37641 33679 45335 12020  6824 15208 27473  6112 25021 45992  6375
    27124 48383 12110 39360 26159  5564 42289  9989 35968 22781 11815 40572
    20822 31193 28727
961:24223 34684 36512 13732 36004 35688 13560 24645 43214 18589 42098 35366
    33101 25426 15316 47633 26694  7406 22767 21823 48571 48953 50891 51184
    42737 51206 45210 12375
962: 1369 43563 33490 25538 25964 29283 34738 22060 18185 37860 18054 33273
    13646 13643 33446 25107 13536  5061 37626 45740  4912   755 17673 48037
    21248 32199 13710 28264 48011 13412 11895 36489 24443 11205 41326  6783
     6781 15371  6782 15400 19230  6785 15402 15373 24330 21945 37378 37874
    23356 34183 30035 44000 23205 20707  2767 48485 13309 49043 40210 48101
    45634 33448 51955 21166 44516 19398 35921 26230 52659 42313  2121 45803
    26674 37203 25411 34275 51521 52389 39894  8680 24033  6557 16865 26911
     7911 19129 15879 37132 48456 31098  4788 29835 38646 28260  2661  6916
    15725 18310 11216 38634  5762 13569 19178 26477 28103 24724 47075 12006
    31120 27719  5550  6408 29749 36211 29464  1971 46984 15082 47682 16250
     8784 11795 21337 26662 43529 43534 43449  8017 14001 45069 52088 52726
    45692  7448 14259 45987 26315 45238 34137 11051 32956 42118  5191 51717
    43926 18139 20909 49561 47346 34444 12088 37327  8152  7266  6491 33724
     8459 21696 20590 48862 48864 10521 11504 23848 23850 30398  9511 42258
    21624 18152 22307 20274 20149 43223 46711 47014 51022 30174 44525  9803
    15291 15298 45188 40887 23950 14803 42503 41527 26960 30574 47753  3346
     2629  2633 47504 22787 23987 16302 41830 41832 45085 34392 13645 13625
    32218 23098  9722 23521 50281 19189 19271 33010  6432 25532 31965  8330
     8328 12107  4656 17796 11338  9663 43936 13679 37827  1767 12067 36943
    22813 39808 34354 22744 48985 27648 34756 13673 19084 22852 32198 35381
     1370 16640  8776 46466 18735 22323 48090 52752  8579 40499 10555 43136
    45664 50608 46726 19889 12128
963:18271 30868 47158 26937 49129 38172 39056 43157 33171 37617 14617 51566
    12437 14082  5387 17012 51145 51120  3151  8801 19626 38053 40023  5767
     6366
964:11705 14745 12920 51370 47825 47159 47027 12937 44661 21072 43441 10544
    40432  3310 18697  1653 41810 48755  3480 19603 17958 18261 18913 42532
```

```
      31548 32576 20142 14746 41795 18263 29218 36026  5899 21535  2022 46973
      46895 46898 46866 25980 46958 26011 25978 46954 26015 26009 17953 10721
      51713 51892 36261 17143 52439 24183 40077 51066 45275 16886 30704 51039
      45473 32717 29248  7910 35013 47259 10643 17424  9082 13406 38014 38376
      37079 15232 10384 50883  3983 23752 28783 41218 28892 11983 44240 15012
      52300 26858 32206  9334 44952 11012  9686 25625  4249 44598 24943  2074
      20121 20173 22794 31731 12444 21101  6282 38681 20426 40480 39516 25278
      21825  5483 10293 13193 30976 34700  9389 34657 33967 30399 28280 35229
       6897 12877 13072 50119  2482 38669 11074 50667 16795 45447 34871 22993
      21012  7230 28579 27810 28761 25878 44281 51520 33751 15333  1533 16928
      20070 51808 34301 35848 51816 38394 41816 42811 21829 12308 12503 50837
      38125  1826 48748 47536 12830 18583 47926  4329  8640  6197  3342  1886
      33922 27200 40174 47135 13031 29765 44360  5030  6870 24741  1932  1873
      30863 35781 47740 22370 33788  5815  7457 35867 42692 38392 16116  5146
      30983 16115  4933 45097  6957 46697 48797  8109 41482 28063 15695 37620
      39110 51937 22231  4856 51153 17238 39282 23592 37233  4516 30068  1884
      15142 20734 46156 38771 22818 11436 18893  9658 31324 16486 36049 50491
      33709 46177 41842 26826 27090  3512 44432 26714  2435 48448 39112 23947
      50511  5734 50658 33658 21344 23973 37304 46702 44687 18529 38487 25076
      19976 49229 31662 10991 41046 52406  1609 46960 30529 47629 15505 43349
       5879  9978 24516 23876 23880 24518 23879 44589  1642 27251  1515 16674
      14605  9685 36523 14486 34510 50048 15579 37901 22250  2638 36302 48658
      42110
  965:46456 13168 43464 22532  7877 30312  7745 51500 44193  3536 29604  7565

  966:24983 15118 35524 40442 36414 38325 51826 27738 10350 19272 30562 10084
      18656 27249 34493 28187 27212 31142 25066 17323 39647 29671  2063 36689
      51227 49858  7502 50314 14537 14335  3700 39211 36811 24869 37473  5279
      35247 32894 43680 34356 22832 28604 47739 51916 25767 29296 45594 51997
      34330  4039 39137 44164 49863 46564  2807 52202 38904 51975 33810 31716
      31893 47239 41654 23838 50020 47973 19856 33921 12982 22317 51844 46565
       7228 27341 30993 22331 24024 51166 18163 50394 40485  7152 28532 21294
      38483 12964 14191  4394 31123 26190 38928 25744 19032  4159 34938  6323
      25652 39777 14819 44992 30618
  967: 9424 14023 18659 35024 17302 38569 30552 30554 15491  6192 13242 36076
       5575 45395 15844  4253 26361 17217 19238 33380 17396 12887 25389 39158
       3325 19784 14708 40844 33473 28762
  968:11471 39768 51569 11162 38612 34335  7175  5054 44889  3668 16510 25936
      11211 10893  1810 35269 44951 15273 30858 13271  3622 51041 51040 42001
      48730 48714 42673 31511  1993 31358 18966 40854 40850 25092 30577 37445
      37444 37447 37446 25451 39720 24966 29861 12173 27052 46250 33422  9255
      44216 43515 49424 47989  3246 15320 41208 10361 25110 38153 37139  2432
      33716 33767 33713 33712  2846 33088 41313  3321  3324 11441 43450 34035
      17232  4416 13324 39817 29675 18707 37618 25458 46382 14525  7795 50711
      19108 40536 27311 23959 41735 18547 33461 52555 23035 18083 18546 33721
      51132 27993 35569 17285 46645 29000  2817 10175 41158  3584 33447 10965
      45082  1725  2981 44866 46841 51635 52266 51632 31698 12835 52086 43710
      32358 25482 48765  2019 20779 45776 24917 51025 25771 28779 14817 26978
      37371 43376 27982 24525 38214 23840 37297 44610 18040 28634 33450 13268
      37560 31112 17546 29231 34135  9523  2139  6004  4487  7742 48980 49491
      24852 23256 23290 33085  4160 31570  4587 39916 13562 14047 18379 19905
      28745 32505  6143 22021 51015 32125 31414 31464  5978 38664 10782 48098
      37942 40680  4960 41884  5268 14898 45781 45962 32869  7816 31238 13059
      28570 29826 18127 51051  4132 34970 21846 52609 52748 35904 32542 46538
       6213 16749 21292 21497 20238  3709 52070 33509 34190 40346 10136 50815
      50090 50389 17149  5642  5639 34983  8477 11295 52756 32048 38459 35367
      30906 30907 12077 28928 23657 30028 47474 11623 25337 22214 32214 51173
       2464  3116 49952 31017 28700  3533 51907 52245 47395 44001 43998 47377
      44002 47400 36094  8931 36096 44291 20420  4157  6951 19651  3893 50546
      41588 17410 25439 46838 37410 39007  9377 38811 34815 38233 24094 45048
       5124  7412
  969: 2594 25138 43186 15968 32189 46170 38989  9309 25777 12644 20403 46590
```

```
       6107 13987 34830 13982 27176 41665 41700 36248 16980 10272 48092 31194
      52750 26374 29857 24767 19449  5552 10363 32258 45101 28564
970:51233 51228 38020 38022 38024 49681 49682 49659 49657 15798  8005  8052
       8054  8056 30738 51338 51308 51310 44263 17897 46855 32543 28026 36976
      14774 15464 44702 14741 33409  8356 29991 29973 29989 44107 20620 12198
      52755  4712 52510 13537  2457  7114 45924 45920 45893 45889 45265 45886
       4432 45972 32675 51176 23926 45932 45927 45968 45946  5758  3386 47554
      45970 39750 49684 22808 22830  9405 14042 46245  5554 21711 14428 44059
      38280 24430  5164 30407 35305 22944 22953 22950 20384 49865 49837 38183
      33236 33222 33221 33218 49644 21176  4492 18797
971:19778 19780 43756 31647 37136  7216 35744 35787 29930 41773 49291 23005
      33316 47967 40309 40312 19916 32835 28641 32841 32837 40880 42802 24867
      30420 34826 42728 42727 17700  4035 51497 42237 38245 43302 34386 26525
      15120 12483 23856 39835 24313 52366 25172 48014 17526 16996 24600 24053
      12242 11025 26256 28034 37755 43801 37811 17308 24842 48166  8817 34422
      37167  2741 41152 41155 19727 33492  9639 37373 30603  8852 41867 51440
      20540 28250 43200 20342  2829 29367  6186  8720 50287 43328 49841 49818
      10795 10757 10737 31503 37412 28980 24469  6121 39815 33669 40530 12914
      34233  5281  5282  7438 39779 26489 42881 45940 41741 26425 50952 38397
      18165 37743 17295 40629 46799 10302  7809 52058 42556 30786 42123 44474
      14236 19489  4480 40549 12897 26580 22848 32752 17256 39582 32690 13564
      40142 46786 36526 36524 35912 36529 50992 32315 36658 42987 47610 16316
      33565 37481 37538  8746  8745 27490 27130 27133 27395 48334 33474 33457
      34584 10503 43902  5821 44009  5806  5844  5838  5818  5814  5813 13492
      31439 15752 39392  5591  8996 14150 10423 42373 15744 45867 10536 35134
      21400 20681 20711 20702 48905 15313 25822 29504 31295 28419 11248  6182
      48162 20602 31723  8210  7282  2786 24569 24563 24572 24575 24592
972:41421 41424 15708 41587 23620 11844 19485 21024 16183  7407 46069 48106
      51123 38369  1923 46571 14862 38200 46569 35587 14228 34051 45485 32261
      40745 21860 51127  2811  2809 36288 35522 35205 26027 52563 18692  8157
      43953  5948 19289  7397  7399 15597 15595 23997 12161 36878 33935 39353
      36160 43227 43130 42245 51534 46306 13590  6696  6714  2406 14655  2820
      51464 40354 40350 40358  7946  7881  7914 29213 25195 47981 27579 28215
      19765 40291 21574 20889 45165 43345 17251 25460 23072 26388 50069 52030
      47487 38877 38848 37216 38834 36457 43848  6174 13167 31556 13175 12035
      12040  8053 49212 48176 16363 10317 33858  2688 28854 34907 34904 42395
      44769 20865 17508 15785 38234 28809 43064 35620 29760 40091 52170 51438
      16201 12986 29864  7296 13884 51352 30181 38461 24674 26727 44502  2665
      41688 23288 30419  7667 49116 50735 37636  5597 18387 17800 48907 42421
       5493 34852 49820 31846  5496 50768 50765 33294 38063  4486 44774  8171
      35379 20613 42655 27481 52203 17390 18264 41618 18265 38439 35273 41750
      12473 13926 17533
973:40600  5280  1751 41101 32971 16810  8088 36583 37701 50351 51340  1592
      34044 26967 39430 18711 46112 25164 36267 24129 50537  9339 21450 27788
      38451 32340 40585 18518 32679  2962 33395 43392 48863  2616 33442  2136
      10221 22348 18660 17159 29455 25163 22256  9845 38238 31333  6072  8063
      23333 24099 39393 23370 45213 34186 36799 34773 30291 35097 43202 52615
      38909 30834 46350 51374 47923 52487 35154 19180 23508 32668 52150 37314
      43962 46484  2198 20288 43309 44522 22114 45437 41284 27885 45493 19292
      37292  3361 32858 17510 31143 47380 51886  7217 23048 21990 32074 50494
       3966  4237 22938 49400 38891 22976 45503  7433 43404 43101  7610 12652
      14342 35071 21188 36322 16011 33080  9212  9176  1512 48943 27185 26830
      13842 19049 36940 38829  1792 31387 34788 19764 50644 11207 28741 38289
      12942 26891  4832  5604  8883 40116 27237 26582 30642 24391 36504 11715
      10859 14119 18647 50643 18250 41417  2240 14892  8219 46004  6515  4851
      31552 37189 23506 35938 44681 32903 28565 21637 31403 40319  7422 14454
      45321 14197 32943 31233 44469 43528 20020 44402 46584 21007 45100 19566
      48619 48640 18406 17457 17498 30556 38062 34613 30339 51999 14762 20336
      44561 42667 31909 50586 36679 34030 22761 37986  7030  4476 27381 43871
      39288 15434 33458  7778 46460 28217 47332 51968 30217 28282 10064 14477
      34523 28168 18705 21566 51396 39876 10765 11417 17702 13599 29936 10578
      45156 27804 38532 17361  3920 51933 24204  4760  7106 32597 32136  3124
```

127

```
      20324    6846 29453 34009 33978 17785 40446 26966 11463    8574 12383 43700
      12277 16781 49381    1518    7638 44512 23516 41235 49112 21597    5793 30849
      48104 48923 10642 47140    6791 27430 10586 12774 33232 33188 24343 17529
      35122 30292 47776 45155    7172 18444 15889 14693 14666 14916 19738 10540
      42020
  974:49252 21412 20271    8343 11709 30586    8434 14262 26341 36090 15639 51873
      22613 38924    4729    4748 52121 45853 27522    3626    2728    4393 44667 35771
      47002 21099 49769    7829    5077 15079 11249 50980 20272 50409
  975:48144 26706 25538     759 20414 39528 20041 33273    7444 33275 28316 20660
      21248 13876 38898 28552 11035 32131 22108 45628 42003    9160 33301     806
       6156 52566 20357 21828 33665 24443 29552 36712 24330    1992    5714 21945
      37378    1371 34250 12998 52037 28084 51431 47876 39474 12956 51955    8737
       6426 22754    2948 51640 26554 22142 12086 18521 38071    8374    9070 22699
      23923 39894    8680 24033 18809 44505 44509    6557 16865 26911 37820    4788
      13896    6544 29835 38646 28260    2661    6916 46155 12253 38634    5762 33433
      36719 25369 10261    9441 40843 27719 12558 30285 29749 39560 46984 24659
      52691 27899 28113 37488 34986 11795 37246 33550 43449 27001 47441 24634
      41509 35934    2998 48242 26315 43338 41748 34137 37160 45199 45264 15168
      26414 20909 43926 18139    5860 20725    5076 34444 35617 37327 40356    8152
       6491    8459 10521 11504 23848 23850    4666 22742 46684 50297 21624 27402
      33666 34766 35252    6273 30306 14351 29738 18435 22307 32577 20274 20149
      10958 43223    7764 17719 40887 45188     757 28143 21699 22425 42503 47753
      20963    1674 43039 22787 40072 10478 50926 23987 16302 41830 41832 23634
       6687 34392 23658 25495    8724 50281 12107    4656 17796 43576 10061    9692
      47425    9663 11338 37827    1765    1767 24001    1372    4052    1590 38764 48720
      28386 17185 30386 34354    9347 22744 42277 39348 50088 44162 38152 33865
        756 48663 33861 20074 35995    8900 45343 17815    9690    5891 34756 35618
      21929 44455 19084 48369    2393 22323 18735 46466 50248 52752    8579 40499
      30905 33797    7163 15679 13598 43535 32742 30235 41794 39718 16313 29279
      45847     758 29498 10555 20519 13421
  976:42841 31601    8619 41496    5661 15611 10749 39922 16039 28037 48988 32167
      25505 45038 16083    4893 42838 42864
  977:43625 26019    9993 36476 39437 40769 35173 14099 49032 51111 32552    3186
      14116 29732 40702    6068 20159 36665 21678 20732 45150 37981 34526 33925
      33926    5106 17318 17316 37388 16933 49608 48447    8563 37972 14443 14445
      14446 21225 13405    2544 23281    7478    5731 19362 24111 22169 45337 40628
      35243    6960 45572 45566 40965 49420 49401 45568 45546 31319 27714    7168
      41355 18495 47253 26434 28193 51414 12719 50143 11636 42045    4194 45646
      52047 51161    9253 32817 32818 32816 19806    7038 35327 27803    2491    4269
      33505 37157 30826 30824 30555    3118 37588    4185    9848 38513 24906 16170
      16160 42521 43483 34272 39390 39812 41411 41390 23633 23636 20169 41191
      31560 39321 22225 22854 10736 52351 20210    5596 39073 23885
  978:43625 26019    9993 36476 39437 40769 35173 14099 51111 49032 32552    3186
      14116 29732 40702    6068 20159 36665 21678 20732 45150 39453 34526 37981
      33925 33926    5106 17318 17316 16933 37388 49608 48447    8563 24427 24429
      37972 14446 14443 14445 21225    7478 23281 13405 22169 24111 13159    2544
      19362    5731 40628 45337    6960 35243 45572 45566 40965 49401 31319 45568
      45546 49420 27714    7168 41355 18495 47253 26434 28193 51414 12719 11636
      46147 50143 45646 52047    4194 42045 51161    9253 32816 32817 32818 19806
       7038 35327 27803 18807 26675 33505    4269    2491 37157 30826 30824 30555
       3118 37588    4185 38513 24906    9848 16170 42521 16578 15820 43483 39390
      34272 39812 41411 41390 23633 23636 20169 41191 31560 39321 22225 22854
      10736 52351 20210    5596 39073 23885 22739     977 15184
  979:20176    9993 36476 39437 40769 35173 14099 49032 51111 32552    3186 14116
      29732 40702    6068 36343 20159 36665 21678 20732 45150 34526 37981 33925
      33926    5106 17318 17316 49608 48447 37973 14443 14445 14446 44827 25924
      38653 23860 45572 45566 40965 31319 49401 49420 45546 45568 27714    7168
      43017 41355 18495 47253 26434 28193 51414 12719 12559 33454 51161    9253
      32817 32818 32816 19806 27803 33526 30826 30824    4642 51992 11144 11543
      11395 26786 15937 42521 43483 39390 34272 39812 41411 41390 23633 23636
      20169 41191 31560 22225 22854 10736 52351 20210    5596 39073 23885 23883
      22739
```

```
980:20178 39437 14099 49032 51111 32552  3186 14116 29732 40702  6068 20159
    36665 21678 20732 45150 39453 37981 34526 33925 33926  5106 17316 17318
    49608 48447 37973 14446 14445 14443 11933 30604 49420 45572 45566 40965
    49401 45546 45568 31319 27714  7168 43017 41355 18495 47253 49209 19842
     1825 50038 49793 39458 23609 26434 12719 13295  9299 51161  9253 32816
    32818 32817 19806  7038 35327 28412 27803 33528 30826 30824 21341 11432
     2607 22810 22928 49902 35929 42521 43483 39390 34272 39812 41411 41390
    23633 23636 20169 41191 31560 39321 22225 22854 10736 52351  5596 39073
    23885 23883  9832
981:29573 38485 25984 28354 39748 38505  3336 43811 50656 39255 19736 21143
    30579 43425 46132 10359  2094 38017 28025 12599 36613 20785 11926 25067
    29939 51690 28735 49265 37947  8556 28791 45979 45160
982: 3029 32148 15551 20530 17835 34022 18632 33856 21027 45120 41490 39328
     8994 11341 26480 29475 48596 50961 24118 13244 14972 32962 17353 48948
    48301 49524 17742 18805 28566 12509 30178 35656  2261 17111 44922 20603
    36606 48375  9788 41330 40812 48790 50823 33864 35078 44017 49691 35836
     6210 40139  8524 26741 14522 26981  9554 22681 22680 21471 51832 16366
    34784 21853 51138 43670 24726 24729 34088  2006  8443 47972 42795 12057
    11358 11447 37992 16911
983:29559 17375 32157 14271 10149 22405  7770  6827  2691 10646  7311 24975
    37818 18992 39836 46674  7447 20199 33970 24423 51144 23137 48762  1579
    26555 48801 37767 28494 21212 21499 26872 26725 21231
984:19028 52753 40989  5457
985:36800  4449 11541 51545 35012 25695 21470 46291 39902  1471  7044  9048
    17593 48173 26782 42524 20244 28161 12976 45462
986:38446 19265 45396 45679 33698 20102 33494 22988 40286 40579  1989 37072
    33525 29067 26974 26316 25982 39065  5903 40006  8291 47446 42240  9544
    23284 36871 31209  1513 16885 24015 47111 31329 19652 48331 34263 49758
    10714 20803 40547 11746  8767 31789 42545 22609 23589 22419
987:39443  7039 28361 47906 16840 14393 16154 10337 19578   799 19575 15453
     1627 31206 49552
988:39443  7039 28361 47906 16840 16154 10337 19383 31220 31206 22349
989: 5736  9499 50301 43573 31206 24365
990:28688 10884  7725  7819 50552 48565 48624 40689 40685 28093 32726 37301
    46501 46498 46537 31318 48592 48567 39212 26868 20471 20466 21187 15452
    14799  2714 21287 21267 18520 18522 48647 48629 39645 39642 31110 30954
    15210 14027 52448 50044 34676 34701 34694  4203 17920 27895  8898 15875
    13251 35232 37576 10561 11408 10588 11435 11346 10645 10681 11354 11477
    11413 11343 11539 15892 19670 32559 13811 13838 22070 17862 17889 18813
    18861 18817 17865 17791 17841 17869 17839 17809 17971 17937 17969 17919
    18881 18877 18864 17845 18876 17968 30197 30928 30924 30946 15205 31903
    32036 32730 15231 32831 32829 32756 32776 32778 32780 31975 31999 30875
    30977 32803 13963 23966  5951  4714 39969 39998 40018 39994 39996 40020
    39992 39997 20247 12022 18160  2739 30997 31155 31870 31934 32040 32720
    15229 31147 31125 31873 32697 32700 32754 24054 27689 48143 47429 31354
    15188 30172 30917 30921 30979 31030 31034 31064 31085 31081 31089 31117
    31152 31793 31795 31811 31815 31850 31877 31910 31906 31913 31937 31940
    31945 31963 31969 32037 32046 32068 32070 32075 32692 32706 32723 32727
    32746 32749 32753 32781 32801 32807 32809 32811 30167 30169 36826 29456
    31819 27920 11781 40859 13965 31390 31323 31398 31396 31401 31320 31422
    31420 31345 31342 31338 31371 31348 31351 31367 35608  2951 25069 43785
    50681 17666 38870 38747 38866 38900 27039 39568 38949 38899 17711 38925
    38954 38820 38957 17779 38726 38791 38795 52099 46542 40681 12163 25752
    12659 42288  8474  6251 19427 50624 24622 31376 50626 50587 40654 31921
    31950 21299 21301 25222 13540 38784 50713 41607 45291 41233 41236 41241
     7813 50599 50595 50592 41227 41262  7802  7801 36670 19507 13967 39041
     7240 22766 38298 28399 29081 29952 33618 18559 51541 32008 40682 30743
    10794  5559  7363 40651 40686 40650 40690 24811  2245 37000 18550 40675
    18555  1821 21717 23202 43160 30962 45035 45032 45012 15911 22233 29219
     2667 40649 40652 40653 40648 40677 24718 43875 14322 27366 10835 49377
    48294 25417 46492 15970 42579 45480 12263 12260 19117 19109  3572 14896
    45200 51980 51963 51961 42268  7934 40647 31373 44556 44517  4315  4297
```

```
        8145   1566 31108   6393 31844 22402 36304 48627 44573 37046 37023 44579
       35035 43889 35348 35834 23331 31441 33871 18667 18005 18744 18003 18781
       18742 18739 18642 18717 18712 18695 18778 18662   6747 49247 21778   2816
       30060 29919 29941 19256 24326 32843 17807 32840 17811 17813 17849 48570
       48093 48066 45289 18556   7743 37223 37577 31325 22518 31391   9498 31053
       31058 31062 31056 13807 46461 46467 42951 38354 13964 30299 20971 50573
       50378 50381 50403 50538 50460 50463 50467 50607 50540 50565 50472 50477
       50406 50410 50413 50430 50431 50434 50437 50370 50373 50376 50572 50501
       50503 50439 50506 50575 50509 50512 50569 50535 50536   8578 16696   4708
       36354 42824 20511 12097
   991:15177 50027 40673 34928 13736 48483   8395   2879 40087 47756   3775 50447
       40068 21033 50577 20633 10119 10394 43825 42455 34540   2865 11913   5023
       47385 27965 39291 17787 46852 29238   9507 51282   2397   9569 47308   2163
       20302 43303 10675 22387 43695 19565 28159 40508   6503 11003 37893   6525
       38697 11072 11069   6507 45967
   992: 9433 38656 49103 43154 38938 38171 51391 37630 32084 24580   4440 48682
       19495 36647 34729 28536   7483   5805 30359 28085 43220 14289 39468   7690
       43000   2042 43259 34774 42980 43924 31777 36246 22301 20335 39327   2507
       36833   9492 29538 49398 13427 17939 14143 14159 46104 32296 27800 41845
       32056 47598 24641 43503 17844 49369 46292 52098 26583 44664 18584 17913
        7532 20465 22715 22718   9535 43176 35975 25849 20430 52072   2969   2984
       20447 48045 33281 17473 17475 52661 18606 27377 49508 28829 47631   1578
       22208   7369 15973 42784 12214 45510 34755 15933 16340 15280 43947   7082
        7094 23295 49875 38114 38307 45243 11454   2907 27461 27459   5503 20404
       18231 20402 24794 42996 42871 42958 42885 42965 42875 42937 42932 42929
       42961 42848 32721 30972 40826 48519 46909 10966 41771 33853
   993:49887 51662 50939 28004   5478 50972 13827 15160 45690 28119 14689   2497
       46187 22717 47391 37799   9274 17722 37553 29196 45836 26082 43887 51429
       36428 42285 25234 32306 32300   2306   2309 36856 38816 47850 30695 45672
       25877   8799 20598 21064   7280 16027 16026 27150   4557 25091 27960   9333
       48283   3625 49823 49807 49825 21022 25295 25292 25291 25289 25288 40514
        4410   3521 47627   5218 41444 41443 41447 23985 21513 25259 25263 41440
       41441 25261 10740 47773 47748 47771   3714 11152 13259 11596 51655 40638
       29272 33441 42803   2370 47080 48307 37548 25420 45106 10046 47799 23161
       23162 23051 44275 23024 13255   5284 44257 35695   3639 17692 17714 27360
       36858   4120 40471 40472   4122 23907 17265 44091   5220 22890 22888   7560
       47744 47741 47746 43969 43963 36850 12239 15742 37551 14184   8016 36883
       36885 29176 51633 50979 13036 20475 35341 48935   9113 32999 25140 20772
       34873 22839 29627 44935 49479 38434   4305 33722 38955   5504 42925 18947
        6641 49219   8500 31880 50953 18910 51689 18907 24932   7281   2973   8937
        7268 20877 14495 24809 21619 51687 35415 48029 16468 50950 25116 31617
        6148 45389 22679 51230 17892 21561   3878 31747 46015 18566 29393   7684
       28824 52441 43555 10617 31840 15236 36392   3049 51665 37813 22274   3890
       19266   8957 29426 29976   6450 16207   8449 38553 41705 21555 36747   8065
        2384 23040 51798 38665 52506 19388 19802 12508 16583 37020 25146 30150
       40293 17029   7864 11138 11260 40229 48633 14709 47000 13349 32518 28423
       12857   9751 25691 20628 12200 48992 35780 39125 51048 42205 29714   9782
       33055 49293 16868 21565   7255 14165 28957 22101 28041 38155 42787 52250
       49972 13822 33284   8824 20256 49202 15691   1813 23920 39926 33973 17981
        3244 37919 19682   6500   8407 35290 47419 46351   4920 12815 26661 10832
       46096 29922 43057 50417 37013 38358 32663 11982 27717   6475 14286 12640
       35700   8096 35909 42989 30454 52181 23951 44956 23131 45713 26696 32057
       23039 51657 50982 51627 50879 50881 50872 50876 50985 19729 29116 51692
       25899 50938 51630   1540 38144 47085 52745 47055 48304 47083 48912 48917
       48309 47957 48919 47102 47101 14939 51663 47993   5195 12031 26293
   994:11379 33622 45367   2831 12126 47287 17445 50081   9225 30395 27322   7276

   995:10969 20326 12371 27079 27080 25929 25927   2794 44170 50331   4143 40554
       45167 29969 11938   4101 46249 49791 42530 46080 39699   2170 21268 30298
       20789 41630 17366 10331 44274 39766 20756 27786 46854 17202 48598   7983
       30371 48974 28021 48741 26421 45616 10745 20319 15572   7759 48835 49033
       29923 32585 23189 21914 22497 17231 15821 11885 16792 28637 16306   1957
```

```
      38108 31707  7010 23478 36054 30224 49038 36608  5374 31047  7919 52218
      34853 44742  7628 33285 27184 11465  9121 31964  4778 39738  1802  4598
      20189 36903 27986 27959 33387 50489 33462 46293 19483 13469 41831 31356
       8279 26109 17246  8530 25931 48549 28621 35498 30798 52413 52415 10265
      45277 21763 46839 10002
  996:44989 16049  5445 39551  2391 40909 35763 12747 37437 17701 11374 10094
      33521  3690 11154  9168
  997:18514 38022 26021 49682 49659 49681 49657 15798 39833  8000  8005  8054
       8056 30738 49140 49144 44263 17897 14735 15462 28026 41033 41035 36976
      24499 14741 15464 33409  8356 44107 48161  5811 32709 13537 15068  7114
       2487 44401 41006 41004 32675 41631 50290 34145  4242 49352 51176 24549
      23926 49037 27241 38182 49684 22808 22830 22944 22953 22950 14744 15465
      20384 49865 49837 21473 21744 21633 38183 33236 33222 33221 33218 45567
      21176
  998:18514 51228 51233 51210 51202 51207 26021  9536 49659 49682 49681 49657
      15798 39833  8000  8005  8052  8054  8056 30738 49140 44263 17897 14735
      15462 28026 41033 41035 36976 24499 14741 15464 18130 17514 18132 33409
       8356 44107 50192 14065 48161  5811 32709 13537 45920 45924 45972 52560
       4199 32433 41006 41004 32675 41631 50290 34145  4242 49352 51176 24549
      23926 16650 49037 45932 45927 45946 45968 45970 38182 49684 23111 27910
      37716 22808 22830 22953 22944 22950 14744 15465 20384 49865 49837 21473
      21744 21633 38183 33236 33222 33221 33218 45567 21176 24037 31931
  999:18514 51228 51233 38020 38022 38024 26021  9536 49682 49659 49681 49657
      15798 39833  8000  8005  8052  8054  8056 30738 49140 49144 44263 17897
      14735 32543 46855 41033 41035 36976 14741 33409  8356 29994 44107 37834
      36982 24735 26686 50192 14065 48161  5811 32709 13537  7114 45920 45924
      45893 14582 45972 32385 52560  4199  4251  4188  4880 32433 41006 41004
      32675 41631 34145 50290  4242 49352 45932 45927 45946 45968 19122 52044
      29398 45970 38182 49684 35649 11936 40622  4915 23111 37716 27910  5964
      22808 22830 31256 38069 32480 51982 23067 25762 36100 11582 44858 11946
       1208 18689 46299 22944 22953 22950 14744 20384 49865 49837 21473 21744
      21633 38183 33236 33222 33221 33218 45756  6823
 1000:40111 41318 46280  3788 44409 25638 18336 46181 52049 31005 27387 22192
      17377 32528 45434 40219 38564 38066  7779 21938  4707 19545 39333 16903
      35754 39207 17057 14787 22052 32254 47519 32557  5911  2552 35682 12722
      20092 38297 52352 31982 33876 24280 35840 31593 24937 12989 16325 16382
       9784 33438  1891 21910  5428 30325  7193 17780 38187 32680 49860  8137
      52456  1001  1632 35279  3971 31711 33208 23206 29790 26368  4750  1305
       6973 17570  8967 13691  8943 30608  1869 29228 52390 21314  3906  3765
       9638  1378 16967 47846 50021 11735  5410  8193 49736  2327 42182 27970
      11721 34546 48605
 1001:40111 41318 46280  3788 44409 25638 18336 46181 52049 31005 27387 22192
      17377 32528 45434 40219 38564 38066  7779 21938  4707 19545 39333 16903
      35754 39207 17057 14787 22052 32254 47519 32557  5911  2552 35682 12722
      20092 38297 52352 31982 33876 24280 35840 31593 24937 12989 16325 16382
       9784 33438  1891 21910  5428  7193 30325 17780 38187 32680 33735 34948
       2430  3561 36358 49860  8137 52456  5230 28309 29947 23206 29790 26368
      27245 27225  4750  1305  6973 17570  8967 13691 35616 37974  7318 42424
       4777 11011  8943 30608  1869 29228 52390  4922 51072 15866 37193 31794
      14303 29766 42780 40926 34245 28989 44565 41320 12090 11430 48605  1000
      18525 10652 11400  2032 48934  1522 29606 31726 26096 15312 11083  9126
      11014  3117 21832 23491 28064 12860 10449 44722 25123  5940 32520 36145
       5290 40241 21394 25914 13505 43502 27227 44236 38420 21314  3906  3765
       5038  9638  1378 16967 47846 48089 50021 11735  5410  8193 49736  2327
      42182 27970 11721 34546  3971 31711  1632
 1002:20911 20882 27411 23678  5824 29833  3753 48944 21185 39187 46053 21524
      26774 50694 13770 47086 49234
 1003: 9948 13979  6518 16218 36543 33834 33849 38963 38389 11039 45049 46539
      50612  6801 15732 51423 29047 47108 11740 33644 14163  3439 17699 48556
      26436 43508 31652 45789 45978  2044 36238 21601 21594 21591 33939 15100
      25550 22651 31590 27254 26221 19598 52493 29489 39726  2463 45470 35209
      35224  1973 13044 36018 47864 30593 35600 41687  2116  8486  7156  2759
```

```
        24414 36146 45541  9888  9843 20499 50348 51055 45981 28882 14553 24432
        33624 52639 36593 28951 29043 12025 13871 13840  9895 21399 38390 10767
        10748
  1004:  9429  9948 13979  6518 16057 10504 16218 36543 33834 33849 38963 38389
        17675 17672 11039 27944  9844 45049 15932 46539 50612  6801 35957 47108
         5228 38996 11740 33644 39594 32316 14163  3439 42924 46780 17699 48556
        48493 26436 48472 17386 43508 31652 45789 45978 48515 21601 21594 21591
        33939 26041 15722 17612 48224 49461 15100 25550  4494 21958 22020 21964
        20287 17854 22137 48738 26393  9799 25393 49395 34978 22651 31590  2615
         2631  2635 31335 31321 27254 26221 19598 38015 10636 10603 10607 10630
        10628 10599 10656 29489 50325 39726  2463 45470 35209 35224  1973 13044
        36018 47864 47847 30593 35600 41687 41689  2116 48514 10192  9162  8486
         7156 52184 33097 22345 49436 32598 33367 22036 24951 36146 25461 48468
        19494 21408 51930 43923 10592 31249 45541  9888  9843 20499 50348 48510
        39713 24057 45981 28882 14553 24432 33624 52639 28951 29043 12025 13871
        13840  9895 21399 38390 10767 10748
  1005:12934 20638  7949 32666 14038 36728 51773 10515 23833 24815 19088 19531
        29539 12060
  1006:32941 16013 43888  3916 35271 30815 49416   953  5698 12696 18099 18109
        18104 14479 18140 18145 14478 18080 18148 46144 18769 49274 15314  3657
         7732  5257 17736  1780 44034 35933 16832 15499 49546 32952 18770 45502
        19596 49981 49979  4421 49404  7251 31879 31876 32122 32701 10554
  1007:25916 48752 50750 49039  3339 36252 47836 39572  9829 42936 25319 41018
        40987 40984 41011 41013 41050 40977 41082 43069 43054 43071 43050 41952
        42002 41078 41052 41081 42336 38402 41780 41799 41798 41775 41777 45656
        46710 40158 23155 23156 23153 23151 15731  4998 43026  4953 41749 41745
        41751 41752 40136 40129 40159 23404 38951 38287  4215 41652 41627 41647
        25421 25416 25418 39861 39882 39904 39884 39883  3296  3294  3312 25321
        34891 50568 17923 51567 31725 32459 32455 32477 25309 25315 13837  3317
         3313  3319  3233  3292  3235  3240  3291  3261  3269  3272 36596 12519
        33637 13373 12399 34266 34264 12403 12420 12547 12421 12575 33589 12577
        12425 12452 33616 12608 33609 12615 12475 34297 33613 12617 33519 12481
        12636 13339 13341 12497 12498 12499 12515 23551 36636 22780 22758 19249
        32461 13248 30288 36734  4040 20982 45638 17048 25474 39747 48063  9645
        36981 16121 49451 44461 39626  3281 21328 34343  9474 41168 16836  2994
        37351 37264 34872 36527 10354  2302 51489 47200 30607 10357 44489 41121
        28284 50797  9357 48249 48248 48251 48267 48265 46057 51027 20862 47455
        40726 43944 29135 10828 29886  9321 41624 41626 41602 33324 24035 36232
        25687 37164  8997  5087 45571  1997 45411 42896 21180 29157  2375 37856
        27094 27998 27997 14909 48615  9090 45733 14387 31630 23467 10405 41787
        37946 19808 13057 48843 24646 19185 11659 27778 32203 33274 14992 21153
        40257 19194 18429 24131 39848 39849  2527 43493 10203 45601 38167 16333
        42011 20393 10793 23109 46518 30662 16555 25718 42586 52722  3506 37174
        37172 23793 36786 16398 41085 49532  3320 40105 40101 31438  3647 29981
        15860  2535 39880  8266 46129  4176  5831 25710  1820 22107 33534 30912
        40377 10975 42620 42819 41045 40311 32600  4976  4973 41589 39385 23441
        23148 32643 41593 41598  8910  8909 42910 42907 38693 27152 39389  4980
        21737 22641 10003 10954 36380 35687  5646  5623  3891 21827 36239 14067
        49706 39307 49704 26410 50366 46032 33413 44948 42070  6537 49027 31763
        41240 40672 40218 40261 17915 29146 26353 26350 26384 35199  3513  6853
        44493 46600  4939 24368 40099  5749 42496  3497  3471  2438 27222 30836
        29085 18720 42069 42820  4944 43025 42046 49088 12215 33750 33747 13370
        12517 10702 10704 10701 10674 40069  3264 42605 40194 40186 23694 23701
        23698 23695 43624 31327 39651  3247 44926 19979 26691 28535 40262 40071
        40223  8881  8876  8882  8878 34430 32623 25360 25381 25384  8873  8853
         8858  8856  4947 29118 41731 29166 29164 46358 46360 40981 21407 21435
        25359 25357 25353  5690  9256  9257 40331 40341 42826 41087 40333 42823
        42843 41755 41772 41774 32579 25388 32619 25412 25390 25414 32502 31624
        31623 43047 20557 32624  4943  4951   763  4969 41900  4977  3298 26534
        19270 12642 12482 33546 13376 23407 13796 22447 10947 45518 28715 42492
        41803 42490 41805 42488 41801 39408 31578 23548 14457 25134 39857 25705
        36702 34122  4534 25683 44117 32629 23401  3258 20976 21017 21036 20908
```

```
40972 40337 42044 43044 42048 42033 41924 41922 42037 41999 44161 44141
44158 44136 44133 41966 41962 44138 41956 44130 41960 41995 41989 41993
41926 41927 37507 41307 41040 43022 15493 25391 40156 32173 40131  3237
45245  5849 21419 40190 35924  3665
1008:25916 48752 50750 49039  3339 36252 47836 39572  9829 42936 25319 41018
40987 40984 41011 41013 41050 40977 41082 43069 43054 43071 43050 41952
42002 41078 41052 41081 42336 38402 41780 41799 41798 41775 41777 45656
46710 40158 23156 23155 23153 23151 15731  4998 43026  4953 41751 41749
41745 41752 40136 40129 40159 23404 38951 38287  4215 41652 41627 41647
25421 25416 25418 39861 39882 39883 39904 39884  3296  3294  3312 25321
34891 50568 17923 51567 31725 32459 32455 32477 25309 25315 13837  3317
 3313  3319  3233  3235  3240  3261  3269  3272 36596 12519 33637 13373
12399 34266 34264 12403 12420 12547 12421 12575 33589 12577 12425 12452
33616 12608 33609 12615 12475 34297 33613 12617 33519 12481 12636 13339
13341 12497 12498 12499 12515 23551 36636 22780 22758 19249 32461 13248
30288 36734  4040 20982 45638 30212 17048 25474 39747 48063  9645 36981
16121 16122 49451 44461 39626  3281 21328 34343  9474 41168 16836  2994
37351 37264 34872 36527 10354  2302 51489 47200 30607 10357 44489 41121
28284 50797  9357 48249 48248 48251 48267 48265 46057 51027 20862 47455
40726 43944 29135 10828 29886  9321 41602 41624 41626 33324 24035 36232
25687 37164  8997  5087 45571  1997 45411 42896 21180 29157  2375 37856
27094 27998 27997 14909 48615  9090 45733 14387 31630 23467 10405 41787
37946 19808 13057 48843 24646 19185 11659 27778 32203 33274 14992 21153
40257 19194 18429 24131 39848 39849  2527 43493 10203 45601 38167 16333
42011 20393 10793 23109 46518 30662 16555 25718 42586 52722  3506 37174
37172 23793 36786 16398 41085 49532  3320 40105 40101 31438  3647 29981
15860  2535 39880  8266 46129  4176  5831 25710  1820 22107 22105 33534
30912 40377 10975 42620 42819 41045 40311 32600  4976  4973 41589 39385
23441 23148 32643 41593 41598  8910  8909 42910 42907 38693 27152 41697
41658 39389  4980 21737 22641 10003 10954 36380 35687 52675 25679 27157
41088  5646  5623  3891 21827 36239 14067 49706 39307 49704 26410 50366
46032 33413 44948 42070  6537 49027 19407 21226 31763 41240 40672 40218
40261 26350 26353 26384 35199  3513  6853 12769 44493 46600  4939  4940
41849  6178 35099 40099  5749 42496  3497  3471  2438 27222 29085 18720
42069 42820  4944 43025 42046 49088 12215  6361  6359 33750 33747 21102
12237  3569  6379  6381 13370 12517 10702 10704 10701 10674 40069  3264
40194 40186  7957 11619 23694 23701 23698 23695 43624 31327 27244  3247
44926 40262 40071 40223  8881  8876  8882  8878 34430 32623  8873  8853
 8858  8856  4947 41731 29166 40981 21407 21435 25359 25357 25353  5690
 9256  9257 40331 40341 42826 41087 40333 42823 42843 41755 41772 41774
32579 25388 32619 25412 25390 25414 32502 31624 31623 43047 20557 32624
 4943  4951   763  4969 41900  4977  3298 26534 19270 12642 12482 33546
13376 23407 13796 22447 10947 45518 28715 42492 41803 42490 41805 42488
41801 39408 31578 23548 14457 25134 39857 25705 36702 34122  4534 25683
44117 32629 23401  3258 20976 21017 21036 20908 40972 40337 42044 43044
42048 42033 41924 41922 42037 41999 44161 44141 44158 44136 44133 41966
44138 41962 41956 44130 41960 41995 41989 41993 41926 41927 37507 41307
41040 43022 15493 25391 40156 32173  3237  5849 21419 40190 35924  3665
13166
1009:25916 50750 48752 49039  3339 36252 47836 39572  9829 42936 25319 41018
40987 40984 41011 41013 41050 40977 41082 43069 43054 43071 43050 41952
42002 41078 41052 41081 42336 38402 41780 41799 41798 41775 41777 45656
46710 40158 23156 23155 23153 23151 15731  4998 43026  4953 41751 41749
41745 41752 40136 40129 40159 23404 38951 38287  4215 41652 41627 41647
25421 25416 25418 39861 39882 39904 39883 39884  3296  3294  3312 25321
34891 50568 17923 51567 31725 32459 32455 32477 25309 25315 13837  3317
 3313  3319  3233  3292  3235  3240  3291  3261  3269  3272 36596 12519
33637 13373 12399 34266 34264 12403 12420 12547 12421 12575 33589 12577
12425 12452 33616 12608 33609 12615 12475 34297 33613 12617 33519 12481
12636 13339 13341 12497 12498 12499 12515 23551 36636 22780 22758 19249
32461 13248 30288 36734  4040 20982 45638 30212 17048 25474 39747 48063
 9645 36981 16121 16122 49451 44461 39626  3281 21328 34343  9474 41168
```

```
      16836  2994 37351 37264 34872 36527 10354  2302 51489 47200 30607 10357
      44489 41121 28284 50797  9357 48249 48248 48251 48267 48265 46057 51027
      20862 47455 40726 43944 29135 10828 29886  9321 41624 41602 41626 33324
      24035 36232 25687 37164  8997  5087 45571  1997 45411 42896 21180 29157
       2375 37856 27094 27998 27997 14909 48615  9090 45733 14387 31630 23467
      10405 41787 37946 19808 13057 48843 24646 19185 11659 27778 32203 33274
      14992 21153 40257 19194 18429 24131 39848 39849  2527 43493 10203 45601
      38167 16333 42011 20393 10793 23109 46518 30662 16555 25718 42586 52722
       3506 37174 37172 23793 36786 16398 41085 49532  3320 40105 40101 31438
       3647 29981 15860  2535 39880  8266 46129  4176  5831 25710  1820 22107
      33534 30912 40377 10975 42620 42819 41045 40311 32600  4976  4973 41589
      39385 23441 23148 32643 41593 41598  8910  8909 42907 42910 38693 27152
      41697 39389  4980 10003 22641 36380 35687 10954 52675 25679 27157 41088
      37424  6501 47681  6458 49663 48913  5646  5623  3891 21827 36239 14067
      49706 39307 31763 41240 40672 40218 40261 26384 26353 26350 35199  3513
       6853 44493  4939  4940 41849 35099  6178 24368  7186  5751  5494  4657
      36108  5491 40099  5749 42496  3497  3471  2438 27222 39684 30836 51349
      37095 26618 29085 18720 42069 42820  4944 43025 42046 49088 12215  6361
       6359 33750 33747 21102  3569 12237 13370 12517 10702 10704 10701 10674
      40069  3264 42605 40194 40186  7957  1007 38250 42570 47688 11619  3969
      33385 16259 49531 30582 23694 23701 23698 23695 43624 31327 39651 27244
      27973 27966 24640  3247 44926  1008 31912 13166 28535 32467 39633 11042
      21319 26117 27164  5313  7357 40782 28832 35706 50402 41369  2723 12491
      40262 40071 40223  8881  8876  8882  8878 34430 32623 25384 25381 25360
       8873  8853  8858  8856  4947 29118 41731 29166 29164 46358 46360 40981
      21407 21435 25359 25357 40331 40341 42826 41087 40333 42823 42843 41755
      41772 41774 32579 25388 32619 25412 25390 25414 32502 31624 31623 43047
      20557 32624  4943  4951   763  4969 41900  4977  3298 26534 19270 12642
      12482 33546 13376 23407 13796 22447 10947 45518 28715 42492 41803 41805
      42490 42488 41801 39408 31578 23548 14457 25134 39857 25705 36702 34122
       4534 25683 44117 32629 23401  3258 20976 21017 21036 20908 40972 40337
      42044 43044 42048 42033 41924 41922 42037 41999 44141 44161 44136 44133
      44158 41966 44138 41962 41956 44130 41960 41995 41989 41993 41926 41927
      37507 41307 41040 43022 15493 25391 40156 32173 40131  3237 45245  5849
      21419 40190 35924  3665 51800 22311 52339
1010:33551 28186 20768 27548  7373 38243 36763  8541  9855 40395 34235 25804

1011:12353 17126 17130 17133 10301 46794 46767 46904 30189  9095 17334 10706
      10678 10682 15840 23265  7064 42484 28660 28662 28663  4511 28259  7092
       7096 24683 11536 51803 13917 21886 45475 22216 44407 41420 46536 19602
      30075 28251 29963 21089 50570 36820 49968 26474 43506  7364  1426 39138
      14253 20012 13067 48844 51460  8511 48993 11834 20014 11187 26087 50072
       8113 19260 14918 11169 15589 38253 18981 22277 35180 26979 11139 17138
       8639 10986 14872 34388  9904 51162 24627  2120 52066 15790 13498 15591
      15587 46917 46915 46941 27837 33187  8228 17782 44299 20094 10473 10475
      10495 41511 19044 31417 18223 23654 21053 37897 37918 45515 50801  8861
      45580 21175 38745 48113 48094 22955 52311  6746  6744 42543 22859  5865
      27707 43419 40974 11049 27576 22861 21092 31127 31129  3694 14012 41452
      32939 32942 32937 19139 19134 19137 32270 14500 39049 29476 28115
1012:19612 47356 18890 17004 21113 23498 24197 36488 36276  1947 18793 22645
      25645 49770 14681 36643 20761 52408 19779 17975  4700 31128 38586  9078
      50016 19302 36147 48724 42380 36979 15061 51107 10060 20501 17952 42364

1013:18890 19612 17004 21113 23498 24197 36276  1947 18793 25645 49770 14681
      36643 20761 52408  1444 17671  4338 27390 32886 29589 31190 11526 26237
       8868 44710 10060 20501 17952 49165 36198 43180
1014: 8021  9433 43154 38938 38171 36131 29032 14016 51391 50717 33360 42887
      37630 24580 32084  4440 26882 19495 36647 34729  8815  7483  5805 30359
      11599 28085 43220 39468  7690 43000  9258 44811 13319  2042 43259 34774
      26866 29620 18178 42980 43924 31777 36246 20335 22301  5705  6367 52048
       3370 39327  2507  9492 29538 32369 49398  1943 13427 17939 50853 27288
      11772 42615  7839 14935 14143 14159 46104 33554 33516 27800  6071 47598
```

```
      24641 43503 40642 17844 16962 23719 42499 49369 46292 52098 18384 42361
      14588 26583 44664 18584 17913  7532 31198 22658 22686 20452 22689 22688
      24953 33287 45552  9535 21756 43176 35975 25849 11274 20450 11275 37150
      48045 33281 10303 17475 17473  9414 49118 12052 44413 14107  8027  4630
      21630 51284 50557 37127 24813 21096 29503 29517  8047 46717 31923 35853
       4340 22208  7369 15973 15979 15983 17051 36946 20869 21090 20871 20838
      20875 18505 43706 34755 19850 19873 39661 19870 15276  3164  7598 30018
      23054  6923  6926  6289  6994  6990  6961 23295 49875 38114 45243 11454
       3254  3256 38668  1625 44972 29012  2236 48435  1306  1807  4094 29191
      11961  3441 35195 27461 27452 27459 23613 17017  1713 36189  7343 23915
      31086 39296 12404 51484 39126 28591  9865  7585 46065 10676 39858 36286
      30213 34337 22189 10786  2513 37069  5503 18231 20404 20402 24794 42871
      42996 42965 42937 42932 42961 42958 42885 42848 42875 42929 32722 30972
      23436 50150 13658 13659 13683 13681 12709 40826 48519 46909 10966 12781
      41771 34111
1015:13403 13410 35950 41516 30908 24579 24582  7019  7018 10051 37785 38956
      17637 16826 10659 13936 13938 45553 45554 35227 21381 21379 25329 41684
      13489  7657  7655 13487 42690 42687 10220 10219 51970  5573 40878 45586
       4267  4266  3662 38635  3818  4498 42053 39989  5177 37353 41248 10580
       8806  7218  8146 27187 27870 22068 22065 35784 18203 18201 51924 41553
       1436  7642  9863  7643 34380 34381 29178 23261 31211 29807 22616 22615
      51454 51456  4652  6540 42167 26275 15269 22701 22704 18687 45759 17708
      17707 32606 32591 35405 35364 52720 23000 12205 12209  6969 17113 23766
      23771 36455 36454 30775  4428 45104 38625 33495 42009 23272 20457 12960
      43118 43117 34945 38574 33957 33959  7823 20217 33599 19030 19031  5448
       8188 36035 36038 12122 34373 34827 18984  8823 30363 23638 22690  6373
       8218 48566 27089  2561 26514 19089  2180  2181 39757 39755 39953 39952
      17728 17725 34171 34172 25976 13688 22067 22721 48189 48190  9971  9051
      45114 45111 26312 26311  4863  6751 47146 34201 47149  7374 14616 39789
      39788 22776 27834 22773 27981 28070  9119  9101  4735 38588 38589 49465
      49467 28865 48499 49593 49592  1520 10071 16943 12188 32020  3936 39104
      10805 10861 45726 29612  5566 24766 24764 21757 43759 20737 13361 16380
      51361 28307  6088 30250 41202 16439 16464 13517 13518 36895 36898 51780
      50865 18004 18002  8681  8683 11221 11222 21814 17303 16542 29839  8207
      48818 48821 38355 38337 30472 18342 19885 19879  3171  3172 48477 52767
      17252 37030 30433 31567 12975 14156 14154 33676 33334 40040 40058 48366
      44566 24272 24314 46278 17441 46277 17439  3547  7568 14205 21724 18214
      19172 39504 21293 51185 25335 25361 47747 48469 15872 15874 18254 21021
      45730 31648 43777 43775 37857 37877 42889 41245 41250 44729 11298 35883
      35884 42769 42767 27746 27807 45958 45812 26251 25464 14948 14949 49282
      49262 17621 17625 34845 35399 32041 32044 10032 10035 52109 48492 48491
      20164 20162 26303 26304 24133 24134 42719 42720 51750  5373  7004  7008
      34641 12588 35989 35988 21429 21426 21403 19328 17709 17904  3793 19345
      13011 19346 13009 44394 20615 27609  1649 50722 24733 24731  4636  3535
      35714  8198 43029 43028 20428 20425 40381 31301 31300  5389  8359 36568
      18001  7748 51318 26645 25590  5581  5580 44831 44833 46237 46251 46390
      25571 28851 51062 51061  3930 26484  1918 27630 27631  8408 51507 28304
      41841 41843 11714 11691 11000 17683 42171 38591 45909 45911  3968  3970
      36444 47513 51341 24105 29948 29944 15862 15865 36095 34154 34152 31813
      31186 32819 32838  5335 40935  5198  6047 35287 16035 50136 50134 48402
      43948 33687 32076 21734 21732 44205 50766  1917 48441 45333 22565 50302
      42777 38162 28474 32429 35057 44689 49340  9825 41628 42866 17313 32497
      39489 17155 11053 11052 10907 30910 16244 33343 20748 17176 43203 28091
      51905 29822 40513 36467 12096 41279 29007 29013 24873 26740  7288 20291
      45482 14688 34466 25130 29823 24801  3879 29851 29347 24761 49670 25117
      47762 44219 26192 40933 42894 42893 48804 50388 40607 11077 16524 24206
      44844 29289  6034 29291  6035 32442 20460 32441 46950 28615 34524 49776
      19982 44177 43418 40917 21731 47182 47184 10992 39557 37643  8020  5370
       6232  3702 41561 34453 13970 48069 48067 10967 36173 21928 48580 17564
      39517 50676 11968 50674 11966 43001 29188  8675 18746 48606
1016:18352 18354 51754 16757 47041 32350 15501 47178 44957 41455 13721 32123
       7494 21179 22081  5082 18948 48018 49413  6894  8069 11126 49214 12911
```

```
      11452 37795 26218  8435 30864 29208 26127 34136 27843 49626 49625 41879
      47501 41880 47298  3860 32850 34006 13836 16359 31164  7566  7238 50680
      47911 47537 46010 25345 25344 46993 10377 21716 21714 28768 26097 38089
       4762
1017:18352 18354 51754 16757 47041 34371 32350 15501 47178 44957  9559 14201
      41763 11452 37795 26218  8435 30864  7968 29208 49625 49626 15775 15805
      47298  3860 25345 25344 46993 28768 26097 38089 20524 30364
1018:18352 18354 16757 51754 47041 34371 32350 15501 47178 44957  2590  8833
      48116 14201 41763 37795 46039  8435  5132 47298  3860 18492  9894 29702
      44739 27865  8024 41308 11362 13020  5533 19600  4119 35020 40473 16252
      32823 46234 31611 25345 25344 46993 28768 26097 38089  4762 16301
1019:27100 27763 27791 27789 27787 27794 27098  2186  2228  1559  2389  2225
       2217  2371  2369  2372  2383  2319  2192  2184  1534  2317  2367  2313
       2288  2285  2189  2342  2290  1562  3157  3154  3149  3081  3108  3053
       3183  3067  3128  2390  2408  2409  3084  2402  2401 27077 27769 27103
      11373 19239 31656 11244  4065 24136 45504 24666 42319 10063 38773 11324
      42413  7605  7148 28126 38772 50929
1020:41661 35080 32909 35732 11742 34560 36333 38107  2613 12049
1021:19021 11632 21132 40189 33901 36089 24294 31495  4659 47780  4837  1307
      30697 10877 47514  6423  7349 49720 50361  7197 41911 46607
1022:21372 42946 17781 44607  2352  9942  8657 50353  4481 45071 10165 16783
      15264  6152 31977 52442  7316 21913 25378 25375 29366  1946 37867
1023:16935 49964 34147 44607 13981  2352 23854 28293 45071 21044 45073 25378
      25375 44657 37867 29885 40073 18072
1024:14963 23090 47477  7963 48119 30947 43804 37968 33158  3800 33074 14755
      14753 34831 27389 27495  9519 24116 41077 42842 10868 51279 15077  5144
      29699 27529  8496  7913 12331 16200 40305 40304 43013 17962 25769 24964
      43809 44923  6301  4191 17610 47546 51817  2272 42734  6812 17695 28679
       6543 15099 52437 23085 33228 33226 42027 35808 24114 43336 39603 21885
      35810  8336 29073 48960  9362 50545 26157  6124 10174 18590 42063 19787
      18281 34987 41317 35058  3444 50232 33371 26052 44311  7948 48303 45429
      45426 12019 39303 45605 45608 19672 15151 48201 43997 47405 31938 39298
      24855 24003 30628  5655 36852 47549 47586 12645 34117 47789 52281 46545
      33878 44787 16009 19801 44601 22332  8403  8398  7650 31095 31113 31093
      49648 15227 25887  9222 49090 42026 13616 40749  8926  8924 26899  4885
      34411 29106 26568 11600 22339 45727  9577 31309 18939 39780 10703 10705
      36795  2257  6473  6476 29481 31995 32513  9410 18282 20781 22723 27373
      36223 23549 23547 44916 44919 29937 29119 20265  7395 21256 46337 46342
      47573 39728 20947 11730 39727 39729 43426  2007 51289 44649 14425 34692
      21922 52315 48990 48991 37431 37435 46896 20705 21564 21531 21527  5888
       5433  7548 47497 48604 35365  4583 10918 21365 28623 29278 29284 48285
       1027 41851 42840 15834 28600 28596 28572 28575 28580 43805 26246 11132
      14487 38800 43226  3498 47991 47996 48002 26558 27462  9607  6467 23385
      12271 48655 22275 17448 40431  9941 24571  8366 38469 16617 13417 26014
       5870  5851 36581  3035 19658 47528 47525 23267 33125  4291 14985 51245
       3908 44684 12566 12568 42865 46130 27499 14010 46926 14852  5976  5972
       5975 21065 35217 48851 14124 25610
1025:47477 44239 21971  9234  7963 12285 30947 37968 33158 33074 14755 14753
       9519 24116 41077 42842 10868 51279 32204 15077  5144 29699 46932  8496
       7913 39347 12331 16200 39024 39017 40305 40304 43013 17962 25769 24964
      43809 44923  6301  4191 17610 47546 51817 42734  2272  6812 17695  6543
      52437 28679 21885 39603 35808 43336 24114 35810  8336 48960  9362 26157
      50545 47114 27565  6124 10174 22180 18590 22589 33071 43732  2262 21316
       6116 14347 42063 18281 14311  3914 34987 41317 35058 15117 46554  3444
      20290 50232 26052 44311  7948 48303 45429 45426 45608 48201 45605 31938
      39298 39303 43997 19672 47405 15151 24855  5655 30628 24003 36852 47586
      47549 12645 34117 47789 25008 44624 52281 44562 46545 16009 19801 22332
      31095 31093 31113 49648 20193  6495 23536  9577 18939 10705 36795  2257
       6473 29481 32513 31995  1026 38921 29058 24331 14740 32986 18282 27373
      29059 23547 23549 44916 44919 29937 29119 20265  2091 21256  7395 46342
      46337 47573 20947 11730 39728 39727 39729 43426  2007 51289 44649 34692
      14425 21922 29598 51355 48991 48990 37435 37431 46213 46192 46896 46238
```

```
     20705 21527 21564 21531 47484 30106 21365 29278 29284 28623 30659 32234
     16855 29871 42840 15834 28572 28600 28596 28580 28575 43805 26246 11132
     14487 38800 43226 26558 27462  9607  6467 23385 22275 48655 12271 17448
     40431  9941 24571  8366 23647 38469 16617 13417 26014  5870  5851  3035
     43390 19658 47528 23267 33125  4291 51245 44684 12568 12566 42865 27499
     14010 46902 46928 46926 14852  5976  5972  5975 21065
1026:23090  7963 12285 30947 43804 37968 33158  3800 33074 14755 14753 34831
      9519 41077 42842 10868 51279 15077  5144 29699 46932 27529  8496  7913
     12331 16200 39024 39017 40305 40304 43013 17962 25769 24964 43809 44923
      6301  4191 17610 47546 51817  2272 42734  6812  3017 17695  6543 15099
     28679 52437 33228 33226  7564 21885 43336 39603 35808 24114 35810  8336
     48960  9362 26157 50545  6124 10174 22180 29829 18590 43732  2262 21316
     12928 40061 42063 18281 42589 14311 34987 41317 35058 15117 46554  3444
     20290 50232 26052 44311  7948 48303 45426 45429 45608 48201 31938 45605
     39303 39298 47405 43997 19672 15151 24855 30628 24003  5655 36852 47549
     47586 12645 34117 47789 52281 46545 16009 19801 44601 22332  7650 31095
     31093 31113 49648 15227 20193  9577  1025 25256 18939 39780 10703 10705
     36795  2257  6473 32513 31995 14740 32986 18282 27373 29059 23547 23549
     29937 29119 20265  2091  7395 21256 46342 46337 47573 20947 11730 39728
     39727 39729 43426  2007 51289 44649 34692 14425 21922 31035 14174 46787
     29598 51355  6612  3924 48991 48990 37431 37435 46213 46192 46896 46238
     20705 21531 21527 21564 47484 30106 21365 29284 28623 29278 30659 32234
     16855 29871 42840 15834 28572 28600 28596 28580 28575 43805 26246 11132
     14487 38800 43226  3498 47996 47991 48002 26558 27462  9607  6467 22275
     48655 12271 23385 17448 40431  9941 24571  8366 23647 38469 16617 13417
     26014  5870  5851  3035 47528 23267  4291 51245 44684 12566 12568 42865
     27499 14010 46902 46928 46926 14852  5972  5976  5975 21065 35217
1027:47477  7963 37968 24116 42842 15077  5144 40305 40304 43013 17962  6301
     51817  2272 42734 17695 28679 15099  6543 52437 42027  3056 35808 24114
     39603 21885 35810 43336  8336 29073 48960  9362 50545 26157  6124 10174
     27782 43732 18221 14347 42063  3914 30620 48303 45429 45426 12019 45608
     31938 48201 19672 39303 43997 15151 47405 45605 39298 24855 24003 30628
     47549 47586 12645 47789 52281 46545 19542 19801 22332 49648 25887  9222
     49090 42026 13616  8924 26899  4885 40749 34411  8926 11600 29106 26568
      9577 31309 10705  2257  6473 29481 25610 14124 48851  1024  3750 14740
     32986 18282 20781 22723 27373 36223 44916 44919 51289 44649 52315 21365
     28623 29278 29284 28572 28600 28575 28596 28580 26246 38800 27462  9607
      6467 12271 22275 48655 23385 17448 40431  9941 24571 23647 16617 13417
     26014  5851  3035 43390 19658 33125 51245 44684 46130 27499 46902 46926
     46928 21065 48285
1028:35767 21327 11148 29547 17909 24426 12567 32225 11787 46951 36812 21850
     45547 13216 21352 20391  2736 40451 38750 19988 24665 48328 18955 31165
     23233 28244 21666 49689 23178 30245 18498 23995 24405  3653 39383 32182
     42254  3564 19840 27070 39874 19836 25306 25301 15810 14403 22627 50771
     41971 41882  6118 35690 46394 31482 47997 47325 43715 21067  6555  3722
      3132 35353 10614  4990 24227 44047 43434 22569 49806 23144  5014 22197
     34427 28718 34204
1029:38122 26113 44238  5717 20994 29773 21915 28234 30031 34951 11597 25854
     10538 10075  5005 38861 24762  3210 14229 17554 10236 12106  7052 32212
     41106 42657 38868 38997 17266 34079 47966 37142 45239 43095 35738  4135
      4396 29856  4574  9374 43016 20978  5859  7301 51047 18144 52233 24474
     45627  4438 40081 48828 40551 35760 40284 19809 37317 37006 23088 14991
     27753 32582 42452 20479  8698 37659  4968 43972 23674 28125 28123 25773
     11829 17548 46253
1030:21428  4984 18190 34407 11922 23194 31545 41838 31546  6681  6648  5822
     10686 11062  7136  1969  5435 33219  5437 49384 51135 51112 51108 51139
     15015  4402  4311  9546  9550 40035 46969 46437 48776 21625 15831  7512
     14369 12673 25513 22282 15503 43719 32220  7833 48367  8518 33757 24669
      3729 29616 43634 34242 23573 24597 47039 34215 47054 42516 33406  4272
     20011 32987 20966 18316 29427 45817 13545 19826 14622 35641 51783 51781
     40921 35139  6483  6617 50885 10150 10171 21934 50933 51017 21779 50323
     39018 13883 11588  3551 47220 14887 26449 52421 44348 14499 50508 50474
```

```
      50438 50408 50442 50435 50510 50504 50433 50465 50483 50480 50387 50405
      50471 52265 25122 21841 21834 21812 21809 21730 21804 21762 21839 21786
      21783 21811 21788 21806 21780 21837 45304 28543 37148 21894 22353 28948
       8920 19554 21819 50333 50736 50103 48091 31312 31310 40410 48239 26606
      44892  9780 10500 23943 50787 16636 36006 11151 44670 15262 47878 15259
      38674 11210 48199 48845 17169 42165 21049 20984 42665 13369 44457 38102
      20021 50080 50204 21117 13481  4510 34859 36118 37675 42833 49255  6668
       7521  7517 46276 51777  1775  2405  3002 18959 49649 39950  3954  7237
      42534  5568 22562 36777 39102 18802 33850 18734 40048 40848 14358 17118
       1812  5229 51384 22089 17517 23452 35426  1791  1817  1815  1794  1556
      15823 24348 52211 39159 39179 51421 11137  5363 39335 38905 29123 50647
      48836 38158 45531 13398 49098 10208  9284  9289 47666 19234 43284 47673
       4163 39475 22013 22016 11233 44544 51177 51172 51208 51181 51178 43786
       8394 31199 31203 33604 15826 33784 31803 51213 51234 51238 51241 51270
      51229 51271 33331 30886 52165
1031:43552 29330 49065 47183 20079 30439 24463  5046 42900 14489 22829 14313
      35156 32774  3020  3018 42564 27228  2889 13296 44924 28631 40986 32468
       3997 40953  2316  2318 45824 36093 46815 19046 35969  1716 36507 42592
       2341  2346 35119 52766 19024 38285 46125  8338 40216 43414 22361 22385
      22391 16310 39940  2347  2348  2350 40092  2249 35253 13102 20895 12356
      19060 11903 13106 40983  2668  7229 21618
1032:43552 30415 30416 29330 20079 30439 24463 24179  5046 42900 14489 22829
      14313 20912  2316  2318 45824 36093 46815 19046 35969 36507  2341  2346
      50274 42204 31383 46125  8338 40216 25950 33729 22391 39940  2348  2347
       2350  4844  6297 40137  7716 13106 41796  2668 21618
1033:29330 49065  4545 20079 30439 24463 24179  5046 42900 14489 22829 14313
      35156 32777 32774 20912  2316  2318 45824 36093 46815 35969 36507  2341
       2346 42204 50274 31383 46125  8338 22385 22361 22391 39940  2348  2347
       2350  4844  6297 50888  7716 13106 41796  2668 21618 46095 38740  1032
       5107
1034:33353  2213 14288 13715 29189 22171  2784 30888 24926 34187 33723 22619
      22620 25180 44261 51957 47254 25025 41739 39758 20914 30094  9478 33452
      16985 32256 33469  6780 11598 49292 44569 37908 42661  4556 10616 27828
      16212 12466 12234 41711 13586 38742 45808 36098 36447 27712 12795 35835
       8245 20239 51356 22505 32866 13286 17297 50094  8307 12134 20644 11415
      12751 27908  7267 25455 22177 11241 11242 37442 23475  8479 26399  3032
      44617 36157 45105  4532 42974 50161 35707  3902 15945 23770  7889 40275
      35525 34060 50772 40414 28967 19506 13241 48643 35101  5989 49314 24187
      25733 18114 44647 41715  7222 40457 40601 41328  2518 38386 20704 20837
      36506  7962 28098  2509 18777 31998 33445 35465 48666 47274  1660 36363
      42470
1035: 2054 22190 39314 50440  7994 37809 10158 10157  7066 17790 39109 24370
       9216 45709  2130  4554 46344  7984 52431 39141  5861 51502 52583 51501
      50259 41051  5949 30804 47987 12275  1824 36518 20372 20154 35491 25821
      19701
1036: 8163 41637 19900  1659 34560  7236 10535 29445 23842 43194 33537 21887
      38051 27041
1037:47473 46598 17151 44685 38846 43455 52190 35256  2193 13154  9011  8998
      22325 14093 18434 39040 18849 32978 13389 10427 10428 47344  7455 38826
       6089  6086 28252 32677 13155  8209  2305 30564 48795 34544 28725 31837
      48054 45416 15689 24925 50197 48710 43213 42763 49651 36114  6810 26646
      22573  4087 18903 18902 15982 46067  3329  3333 27709 27706 27703 33886
      43688  5270 10366 23515 21413 50704 40334 32522 30857 43406 27643 27649
      48638 22536 35272  4284 10839 12176 28983 31322 18542 32892 19703 17611
      48120 48783 28240 32176 21944 18988 49852 50189  3228 35996 18228 45579
       4073 20065 50690  4053 33217 34585 33470 18857  3586 37705 41797 28746
      32263  9765 22461 13014 42344 42343 42341  8653 51462  5558 28047 28060
       3757 47396 34573 34450  7987 33529 43915 20619 40269  2202 42624 36632
      47264 15694 12392 51125 41467
1038:47473 46598 17151 44685 30139 38846 43455 52190 35256  9011  8998 22325
      14093 18434 39040 18849 32978 13389 10428 10427 47344  7455 38826  6089
       6086 28252 32677 13155  2305 34390 48795 48710 34544 28725 24925 31837
```

```
       48054 45416 15689  6448 50197 43213 36114 42763 49651  6810 26646 22573
        4087  4089 15982 18903 18902 46067  3329  3333 27709 27706 27703 33886
       43688  5270 10366 23515 21413 50704 40334 32522 30857 43406 27643 27649
       48638 22536 35272  4284 10839 12176 28983 31322 18542 32892 19703 17611
       48783 48120 32176 28240 21944 18988 49852 50189  3228 35996 20065 50690
       33217 45579  4053  4073 34585 33470 32263 18857  3586  9765 28746 37705
       41797 22154 13014 42343 42344  8653 51462  5558 28060 28047  3757 47396
       34573 34450 48574  7987 33529 43915 30195 30191 20619 40269  9758 42624
       36632 47264 14965
1039:47024 26844 21908 16117 21537 46782 22931  1830 28344  8899 24256 48838
       23209 26681 23225  5678 45369 41319 34138 37362 48230 30497 15698  4892
       44490 18197 40894 18828 38018 41635  7076 41130 44098 20161 20108 50715
       32006 11911 24734  4483 35901 10466 48645 23138 18784 20667 36844  6046
        2936 23606 34814 34586 13762 26106 37413 29848 13100 37386 26288 18172
        1707  6465 26402 42641 15992  3145 34159  6099 34039 52438 35636 12631
       45549 23366  2035 18060 21196 19592 40853 31402 49145  9281 25081  3427
       49753 30079 35735  6325 30563 27313 48525 17460 33960  6254 11676 45884
       21380 51795  3141 50559 44823 16681 49779 31827  6998 32979 31444 32323
       20592 16454 10406 25406  4623 10376 42672 10792 46029 50334 40851 27999
       40756 51688 51667  4219  4217  6510 31499 28664 12680 10333 10328 10304
       18117 18143 18047 20285 25249 44326 47998  6658 26690 28849 23214 25802
       30597
1040: 2551 50367  4025 49463 51554 45075 43443 46771 23728 19455 27121 18068
       18088 34048 26539 15029 36195 45233 15522 51102 18030 30966 23353 42559
       39357  7454 42348
1041:17182 51843 39714 34606  3457 23937  7095 11766 38281  7101 42431 18359
       13882 47448 31946 26381 26383 26382 26379 30361 50475 15883  4256 32620
        9745 24973 37214 30553 41919
1042:18225  6883 22706 29005  9432 51285 28877 26916  6455 46231 45520 34322
       38540 20827 49148 39105 34362 49672 22267  8862  7982 35669 50920 52537
       46777 22306 12850 14248 19151 21129 50975 22473 21177 27759 45623 22599
       14297 17165 38696 22009 33662 32007 45342 30557 52546 50816 28941 16971
       12526 41783  5882 22428 36970 10034 16370 44925 35585 31247 30666 31008
       49824 10887 47280 27028 19217 39123
1043:19095 51981 34663 23570  9916 25678 25680 11013  7981 36683 25251 51658
       34980 34998 36155 38412 21086 43088 51192 43780  2778 45883  9439 30458
        2835 30724 36725  6233 33408  7147  3989 34290 46774 16647 44078 40360
       16667 14645 29200 31735  2659 16168 35704  5707 50498 43570 18401 30520
       45747 25658 32353 48453  8760 13021  8842 14886 27923 18610  8461  4235
       43823 35663 42616 26104 41683  7658 38414 38508  8775 16317 47557 52299
       29707 12726 47343  2062 51082 44639 11323 18611 26223 28420 39269 49004
        8433  3406 28224  2294 23893 14189 47899 12413 23810 26024  4902 12157
       33426 32191  4202 27765  7257 24909 13923 38442 41436 25481 12301 23332
       50756  6673  1871 51988 20113 14380 31287 32424 12974 19240 35036 50328
       52364  2664 33143 37382 43940 43899 24203 17226 44201 19036 12984 29076
        8289  4519 16670 40543  2788  8055 18006 48950 17122 38290 45544 22910
       38356 11120 13448 49310 19997 18454 46333  8567 45314  6274 37208 37209
       21025  4671 11076 15681 25859 28333 25748 33595 10370 46661 45157 47226
       34730 29288 31115 10713 25715 44595 17429 26269 26718 48163 29361 47427
       21417 46987 22827 35737 26874 25717 20099 13845 48016  2459 38438 45550
       14225 35456  1604 15803 43849 34647 37288  3060 24860 47284 30192 14235
       16658 35552 19579 24270 25758 43605 31500 24950 31916 13240 26803 32100
        9547 31668 17145 51190  6970 27552 20743  6576  1045 22436 26773 51896
       30708  3034 33642 31177 45741 40888 25000  9757  5122  9866 24462  3882
       33318 27783 46528 21234 34816 35589 44208 43431 13585 14581 48747 40553
       44848  6647 29608 29669 47656 33717 42880 37607  5469 26078 34067 48205
       16659 13089 42835  3644 11965 33432 50466
1044:42608 35846 34663 50600 31046 39761 14597  7090  1669 27583 35212 44144
        9069 22959 12988 36990 44692 16557 47409  2757  2756  2758 25170 46102
       25330 13239 25281 37733 32761 43403 21086 21679  2961 34075 33964 32200
       21726 41865 39230 45505 17848 51842  7000 20476 12918  2778 45883 30218
       30624 52363 13304 38495  2808  9142  6368  4030  9246 36725 13281 32652
```

139

```
 7147 25435 28769  6087 49920 25617 40248 25581 47507 16667 25988 50053
49579 20747 19697  6582 27492 47651 29345 13131 31735  6953 24845 36186
26993 26292 49912 33020 10200 16446 17104 33050 35662 28875 34992 20690
 8086 11426 36491  6106 31867 23763 10435 14750 22612 15394 19429 52346
 2745 20763 32353  7886 34409 39440 13602 15302 35575 15306 28499 17727
22398  9587 24727 42997 12875 30348 10400 41139  9496 13849 38146 34900
49025 11870 40646 36237  8525  1702 12814 38452  9337 29812 15671 20754
 6457 49283 47557 26789 51439  7208  4799 31709 47343 12726 45235 30590
46153 44639 37359 16600 20996 45445 11513 10499 29734 52619 19671 23544
 8261 27072 39237 20050 26551 49735 29167  2119  2399  3829 26649 24559
47318 45078 49079 24363 52386 49289 26085 26641  9180 25663 47812 29107
 4524 11496 42385 18638 41542 24929 40930 20489  6177  7909  6559 21278
18032 11390 41391 31297 30471 18455 14827 33673 32018 31849  9355 24671
14106 43108 10072  4202 12789 38981 34455 37842  6667 14088 10472 46718
28101  2979 20120 37335  6858 35129 25552 37660 11322 21771 37156 39970
38442  9173 10147 38548 30318 23874 26851  5059 25481  9512  3465 31375
 5595 48070 32311 45687 51158  2598 37685 50756 14383  2049 52316 43123
39070 30740 48314  6673  1871 21050 28918 49658 15266  2861  1960 30611
32424 10654 13440 12974 13053 14640 14214 27195 31899 10913 11664 28695
29508 26152 21333 26655  1600 27797 27957 39183  3563 14224 33143 16143
19461 42428 36278 30042 42482  7242  9088 11793 24203  8978 19150 14142
47073 29711 19036 33040 14040 16819 27588  3841 27302 27300 16070 42480
 8290 35167 36778 16619 45279 31316  7041  8329 35586  6547  7167 13164
 2345 17628  9549 39511  3674 40475 26617  8628 42283 12735 45914 33388
52671  6595 46283 18751 36151 34981 27396 23841  5901  1818  2898  9760
31779 20279 13076 40869 47205 25575 14095 18233 38356 21156 11120  5697
35725 10191 11185 31412 34746  4491  6599 40181 38518 21228 13448 49310
30705 33345 26930 42170 19997 44140 23277 43040 41564 44496  5315 18588
32168 45086 19523  1796  9141 33940 34491 11110 33859 47290 20622 21025
23857 48315 44895 33056 29201 45254 42868 37236  7204 49329 49739 17504
14951 43268 39145 20673 31016 29281 28330 47413 25748 22223  4355 35739
19167 23782 38213 20896 45710 21855 44380 28495  3634  7926 27485 41685
45474 23323  3059 18928 15185 29741 39234 45588 20375 48163 17768 22304
34626 48065 47427 21417 24074 28519 30151 21172 30207 31265  3571 34505
39003 35740 35737 15688 15586 26874 30387 34351 17015 34223  3950 30024
21736 25781 47475 47808 15258 13845 40593 40721 30937 51134 14873 35928
42817 13298 45386 14225 35456 13311  6840 22564 22412 50342 19320 15803
14194 21087 16736  4021 33223 22359 51193 23511 26775 37288 18946 21645
31887  2935 16106 29416 15397 24127  6513 13262 32222 33262  9115 14371
14411  7429 30330 37787 10039  6096 40474  8631 42000 30301 11670  5127
49782 51190 44520  7905 27216  9721 47655 19175 19136 17950 35047  6576
36466 36406 50194 21888 34926 32494 46701 25681 12944 39030 43716 37295
42808 35295 37943 42847 26750  5668 49220 43769 40950 17074 17095 19442
36468 36474 40888 36448 45741 36471 25000 46171 42650 42911  9984 31277
18018 45276 52320 22535 24695 40464 40589 16673 37261  3749  9694 19053
20647 50321  7419 29724 22471 34912 37695 12281 36975  1568 31966 22381
21485 22284 29141 47656 35538 10989 40518 13254  4504 42290 31526 33168
37911 25258 27075 48918 14317 28355 13089 14349 43750 44066 31736  9612
23266 21360 14069 14723 33432 13252 37837 41206 46790  9884
1045:19095 51981 23570  9916 25678 25680 11013 25251 51658 34980 34998 36155
38412 21086 43088 51192 43780  9439 30458  2835 30724 36725  6233 33408
 7147  3989 34290 46774 16647 44078 14645 40360 29200  2659 24845 16168
35704  5707 50498 43570 18401 30520 45747 25658  8760 13021  8842 14886
27923 18610  8461  4235 35663 42616 26104 41683  7658 38414 38508  8775
16317 52299 29707  2062 11323 18611 26223 28420 39269 49004  8433  3406
28224  2294 23893 14189 47899 12413 23810 26024  4902 12157  9355 33426
32191 27765  7257 24909 13923 41436 25481 12301 23332 20113 14380 31287
32424 14640 19240 35036 50328 52364  2664 37382 43940 43899 17226 44201
19036 12984 29076  8289  4519 16670 40543  2788  8055 48950 17122 38290
45544 22910 46333  8567  6274 19523 33940  9141 37208 37209  4671 15681
11076 37872 25859 28333  4355 33595 10370 46661 45157 47226 34730 29288
31115 10713 25715 44595 17429 26269 26718 29361 46987 22827 25717 20099
```

```
        48016  2459 38438 45550  1604 43849 34647  3060 24860 47284 30192 14235
        16658 35552 19579 24270 25758 43605 31500 13240 26803 32100  9547 31668
        17145 24264  6970  3034 30708 33642 31177  1043 16707  9757  5122  9866
        24462  3882 33318 27783 46528 21234 34816 35589 13585 48747 40553 44848
         6647 29608 29669 33717 42880 37607  5469 26078 34067 16659 42835  3644
        11965 50466 26773
1046:22655 19547 31331  9368 52293 33778  6725  6730  6727 44121 42762 38466
        38729 28121 34338 47692  2476 41791 39721 45517 36855 40602 51455  8673
         3494 21452 42968 19649 16185 14121  6161 11634  7944 25219 22755 44231
         2505 14644 27766 27433 32456 27271 34631  4919 52393 45637 51269 44320
         6253 27831 31032 13170 31905 14505 48816 15431 19428 46273  9649  8211
         8911 31337 25517 32695 52126 26908 50456 36508 41501 21820 43961 32246
        44128 46482  9242 15788 26949 29172 38094 18870 10228 35360 22260 44050
        41606 25649 39270 30429 12955 31139 44334 32852 15433 21977 50155 19120
        23049 17532 22851  9905 50562 32808 20203 51392 52587 27955 45742  3115
        42101 22878  6443  6440 10897  6459 32391 38986 19100  8659 51472  4290
        10938 12788 31468  1793 25437 32166  4149 23953 47243  4457  2264 20809
        45527 19451 17521 35605 12216 41809 44226 24159 43708 39409 13830 18362
        29926  6098 48508 18021  9712 34433 45642 17808  8936 19768 43228 28790
        30321 23776 11941 46961 43597 43601 12837 19538 40760 45928 19524  7933
        47590 21042 25962 46994 21390 27708 51836 14954 20961  1987 16554 35507
        19710 29549 39012  5622 15684 29384 29378 40303 29911 16553  5539 36659
        52576 31011 50482  7737 48123 20064 44546 46043 50424 19019 35434  4415
        46396 40833 11883 30588 14573 50531 15450 25861  2234 16655 42568  6300
         6304 13584 13016 51064 20932 19797 12891 11353 26653 47026  9890 45869
        22338 50687 10252 12378 51815 35208 44175 11456 29932 26343  3817 16562
         4422 35485 18856 26865 30953 31179 10198 34702  4603  1047 49746 49188
        11157 32826  2073 33832 27324 11484 29689 36453 46543 25872 11419  6511
         2577 26415 37432 18193 52225 40255 51696 25909 35391 30845 25896 31993
        42796 10349 25198 40539  7415 28000 35254 29443 45956 24069 24890 37665
        36991
1047:22655 31331  9368 52293 33778 44121 42762 38466 38729 34338  2476 41791
        39721 45517 36855 40602 51455 21452 42968 19649 16185 14121  6161 11634
         7944 25219  2505 14644 27766 27433 32456 27271 34631  4919 52393 45637
        51269 44320 27831 14505 48816 15431  9649  8211  8911 31337 25517 32695
        52126 26908 50456 36508 41501 21820 32246 43961 44128  9242 46482 15788
        29172 26949 38094 10228 18870 35360 22260 44050 41606 25649 39270 30429
        12955 44334 31139 32852 15433 21977 50155 17532 23049 22851  9905 50562
        51392 27955 45742  3115 42101  4290  1793 25437 32166  4149  2264 45527
        43708 24159 39409 13830 48508 18021  9712 45642 43228 30321 23776 11941
        46961 43597 43601 43526 40760 45928 19524  7933 47590 21042 51836 20961
         1987 16554 29911 16553  5539 31011 50482 48123  7737 20064 44546 35434
        11883 25861  2234 16655 42568  6300  6304 13016 51064 20932 12891 19797
        11353 26653 47026  9890 45869 30484  1046  1841 36991 50687 16231 10252
        35208 44175 11456 29932 26343  3817 16562  4422 18856 26865 18193 52225
        40255 51696 25909 35391 31993 10349 25198  7415 28000 35254 29443 45956
        37665 32826  2073
1048:22655 19547 31331  9368 52293 33778  6725 44121 42762 38466 38729 34338
         2476 41791 39721 45517 36855 40602 51455 21452 42968 19649 16185 14121
         6161 11634  7944 25219 22755  2505 14644 27766 27433 32456 27271 34631
         4919 52393 45637 51269 44320 27831 14505 48816 15431  9649  8211  8911
        31337 25517 32695 52126 26908 50456 36508 41501 21820 43961 32246 44128
        46482  9242 15788 26949 29172 38094 18870 10228 35360 22260 44050 41606
        25649 39270 30429 12955 31139 44334 32852 15433 21977 50155 23049 17532
        22851  9905 50562 32808 20203 51392 27955 45742  3115 42101 38986  8659
         4290 12788 10938 31468  1793 25437 32166  4149  2264 45527 19451 24159
        43708 39409 13830 48508 18021  9712 45642 17808  8936 19768 43228 30321
        23776 11941 46961 43597 43601 43526 40760 45928 19524  7933 47590 21042
        21390 27708 51836 20961  1987 16554 29911 16553  5539 31011 20064 44546
        19019 35434  4415 46396 40833 11883 30588 14573 50531 15450 25861  2234
        16655 42568  6300  6304 34889 43615 12501 41679 47626  4062 47026  9890
        45869 33730 13029 41143 16104 16710 50687 16231 10252 51815 12378 44175
```

```
          11456 29932 26343  3817 16562 37320 26769 52122 31475 12076 44267 31179
          11419 18193 52225 40255 51696 25909 35391 31993 42796 10349 25198 40539
           7415 28000 35254 29443 45956 24069 24890 37665 29072
    1049:52690 12013 47959  3070  2076 29628 26963 22163 30199 37202 29728 40995
          44235 33605 29131  7713  8699  2776 29343 17805 36156 43606 45204 46935
          26811 29771  2394 28138  1696 27204 40211  8730 11256 33822 13007 17964
          45006 50383  7922 22246 30478 16776 17616  3403  4579 16926 48278 47733
          35105 14633 38766 38239 38768 46100 34221 34224 35872 22376  8499  2387
          12184 44762 41988 52317 37645 48500 23744 22248 29789 38761  2709 52687
          52686 32169 34403 50836 24798 41327
    1050:18292 50976  8181 50633 32011 11075 40065 37380  7028  8692 36583 37701
          37076 50351 51340 38717 34456 35555 39430 24382 37436  6739 20464 18693
          46511 19629 51104 39342 24704  5105  9341 38661 27788 16923 42810  4332
           7355  8038 39844 45315 10797 33358 43392  2962 33395 47912 46585 15703
          48865 27947 27595  6449 47129 33667  3651  8819  2136 25468 33915 22348
          18660 23501 19807  8791 15959  2787  6072 31333 26298 40616 42792 43619
          39393 43761 19541 20873 10564 34186 34350 52174 12233 43202 38602 28124
          12276 30835 52615  4456 43668 41932 33762 10585 17989 48891 42643 49182
          23454 36604 27274 37314 16030 22693  7009 36530 15171 42092  2198 25287
          43288 38313 49584  7075 45437 41282 27885 38836 10553 20222 48000 19292
           6038 19660 45707 32860 37292 47960 17908 10452 27236 47378 48897 43897
          51886 23103 48393  7217 42439 36544 32074 32920 50496 12695  3964 28286
          14081 41594 49400 29528 38891 16288 45503 20684  9191 37053  7433 37470
          27203 29602 45548  1586 10227 12941 28794 50838 47233 39887 47012  6800
           2522 36322 27857 16011 40349 40383 37699 13583 36141 23082  3430 26490
          26830 13842 36940 10520  1789 42982 43514 49021 22797 19762  6776 23326
          38276 28738 38879 12125 33481 26910 37428  8883 40117  4337 27237 26722
          26582 35814 30642 24391 43520 11372 30716 14301 11715 10859 15311 16786
          38759 19057 33069 41418  3758 39765 23785 34295  8216 23904 35631 41723
          36137 14243 31554 33950 41224 43626 10044 33225 27370 37603 40076 35411
          50412 26036 18782 40294 34251 14452 52398 14306 14197 42367 48316 32943
          31234 38103 44469 40184 20020 44402 50817 19365 26124 29887 24606 48616
          41949 18423 39071 35583 22297 36064 33741 17498 40519 31369  9178 10391
          34677  7575 34472 47560 26222 14763 50335 20337 11822 43510 50362 50586
          23837 11301 36679 34030 21937 22762  2082 37987 16685 32133  7030  3589
           8321 32834  7908 36950 15817 43171 27381 15539 43871 42718 15439 24265
           7778 48750 36214 31268 30219 31126  5238 51647 10090 14477 16166 18899
          26712 51053 19258 29686 21566 17575 51397 10765 49406 48459 34325  9111
          19837 11785 32656 29936 16139 10578 45156 49861 34649 35973 37756 26592
           3920 30636 40568  4760  7106 32639 13119 14466 17816 35487 41461 36805
          10990 30227  6136 31074 20246 25355 37797 14355  8448 43698 51494 14176
          14684 14711 42942 10356 13696 12765 26764 10108 43153 43155 40444 35467
          26243 51945 23439 14153 17581 27329 44824 45488 16781  1518 38315 20198
          13739 44385 45939 11176 52014 13755 45942 34267 52105  3616 11225 17370
          21597 37035  5793 28512 27685 20024 20026 46405 48922 48937 10642 50436
          47141 27430 10586 12774  8677 14020  5535 31374 43096 43097 34487 40627
          40273 43037 50947 19991  7172 18444 17643 22383 20657 14696 14665 36021
           6205
    1051:26619 48186  1310 10567 11848  4068  4071 17560 17558 17552 51280 11147
          28158 45436 45432  6349  6151 51070  8656 25656 20512 21206 39033 16428
          19970  4319 25159 24218 38341 27659 23690 41280 41283 49571 22201 10188
          21879 44083 17859 23087 39967 39634 38862 23564  7190 15878 32279 46762
          22118 31457 46573 45809 14037 38447 28855 34939 22654 47231 21890  9996
           4698 39022  9929 19305 19327 49128 23676 48798 23187 23012 12121 44068
          39147  2732 10700  4633 40604 33879  1599 46423  3490 20235 29501 31530
           9556 38291 42662 15730 21654 46037 22881 40758 42585 22838 28134 22255
          43177 28169 33696 20691  2278  6932 35459 29518 14757 29335 11591 17595
          46976 44372 42508 12402 16202 13390 38828 44286  4164 18202  7400 51129
          14905 42097 33948 52485 10581  1470 12591 25260 36620 28802 38059 21777
          23464 36486  1309 23762 40678 23577 24321 26331 45998 20343 22520  5487
          34101 32525
    1052:14293 12728 10791 24350 12714  6293 41790 12496 12384 38241 24268 35710
```

```
      9716 16950 19549 16611 26049  6713 36426 27743 14870 46823 50513 46789
     36413  9303 50793 52358 30272  3692 19897 47978 40296 26044 41136 51681
     38139 38516 49510 46758 32590 25729 44169 35384 26071 41425 26076 52119
     29784 29205 10296 27397 39085 11959 22318 43048 23655 28045 13921  6786
      2499 47430 27971 18375 25215 51243 11155 49725 18308  9574 51527 26046
     52629
1053:35767 21327 11148 29547 17909 24426 32225 12567 36812 11787 46951 21850
     45547 13216 20391 21352 40451 38750  2736 19988 48328 18955 31165 23233
     21666 28244 49689 30245 23178 18498 23995 24405  3653 39383 32182 42254
      3564 19840 27070 39874 19836 25306 25301 15810 22627 14403 46548 41882
     41971  6118 35690 46394 31482  3132  6555  3722 35353 10614  4990 24227
     44047 43434 31317 22569 49806 23144
1054:35767 21327 11148 29547 24426 17909 12567 32225 36812 46951 11787 13216
     21850 45547 20391 21352  2736 40451 38750 19988 48328 31165 18955 23233
     21666 28244 49689 30245 23178 18498 23995 24405  3653 32182 39383  3564
     39874 19840 27070 19836 25306 25301 15810  3505 52209 33515 41882 23758
     52654  9897 23757  6118 35690 18644 52533 47440 11975 32879  6632  8283
     52463 47900 51574 11255  6555  3722  3132 35353 10614  4990 24227 43127
     44047 43434 31317 22569 49806 23144 47189 16182 10073  2640 49814
1055:37825  6498 38257 44494 10142 31219  4248 13431  7808 24090 14984 36900
      3355 13839 24996 15967
1056:35767 21327 11148 29547 17909 24426 12567 32225 11787 36812 46951 21850
     45547 13216 21352 20391  2736 38750 40451 19988 48328 18955 31165 23233
     28244 21666 49689 23178 30245 18498 23995 24405  3653 39383 32182 42254
      3564 19840 39874 27070 19836 25306 25301 15810  7734 52602 13207  7902
     22515 34427 28718 14403 22627 18684 41971 41882  6118 35690 39658 41525
     46394 30134 47997 47325 14085 19373 39203 43715 21067 11682  6555  3722
      3132 35353 10614  4990 24227 43127 44047 43434 31317 22569 49806 23144

1057:35767 21327 11148 29547 17909 24426 12567 32225 46951 36812 11787 21850
     45547 13216 20391 21352  5977 29183  2736 38750 40451 19988 24665 48328
     18955 31165 23233 28244 21666 49689 23178 30245 18498 43107 23995 24405
      3653 39383 32182 42254  3564 19840 27070 39874 19836 25306 25301 15810
     47855  1635 52209 33133 33515 18684 41882 41971  6118 35690 41508 46393
     47355 45754 44614 52278 25055  6555  3722  3132 35353 10614  4990 24227
     44047 19045 43434 31317 22569 49806 37129 23144 20383 34033  1058  5341

1058:35767 21327 11148 29547 24426 17909 12567 32225 46951 11787 36812 21850
     45547 13216  5977 29183  2736 38750 40451 19988 48328 18955 31165 23233
     28244 21666 49689 23178 30245 23995 24405 39383 32182 42254  3564 19840
     27070 39874 19836 25306 25301 15810 47855  1635 52209 33133 33515 41882
     41971  6118 35690 41508 46393 31482 47355 45754 44614 52278  6555  3722
      3132 35353 10614  4990 24227 43127 44047 19045 43434 22569 49806 23144
     34033  1057 16338 20383
1059: 8021 49176  9433 49103 38656 43154 38938 38171  9728 29032 51391 33360
     42887 37630 24580 32084  4440 19495 36647  8815  7483  5805 47084 30359
     11599 14386 26174 28085 43220 47209 43394 14289 39468  7690 43000  9258
     44811 13319  2042 43259 34774 26866 19989 29620 18178 42980  2135 43924
     31777 36246 22301 20335 52048  3370 51095 39327  2507  9492 29538 32369
     49398 13427 17939 27288 11772 21946  7839 14935 15067 14143 14159 33516
     33554 27800  9527  6071 42294  5171 16058 22228 47598 24641 40642 17844
     52723 16962 42499 49369 26898 46292 43089 33466 52098 18384 42361 33255
     26583 51724 44664 18584 17913  7532 31198 22658 22686 20452 22688 22689
     24953  7308 44371 33287  9535 21756 43176 35975 25849 20450 11274 11275
     37150 48045 33281 10303 17475 17473  9414 32644 12052 49118  4630 21630
     51284 50557 21830 37127 21096 29503 29517  8047 46717 31923 35853  4340
      7369 15979 43188 43184 17051 36946 24568 20838 47662 20871 20869 21090
     18505 39411 20875 10505 12337 11352 11355 52579 11351 45510 43706 34755
     19873 19870 19850 39661  3164 15276  7598 30018 23054  7053  7059  7056
      6923  6289  6926  6994  6990  6961  7017 23295 49875 38114 39052 39050
     15041 41042 45243 11454  3256  3254 38668  1306  1807  4094  1625 29191
     44972 29012 48435  2236 11961 35195  3441 27461 27452 27459 23613  1713
```

```
       7343 36189 17017 23915 39296 12404 31086  7585 10676 30213 39858 36286
      46065 34337 22189 10786  2513 37069 40203 18591 12689  9576 13258 43596
      49990 42994 38710 13278 51706 40336  2967 16403  9460 50780  8979 19571
       1386 47412 27876 35018  5503 20404 20402 18231 24794 42996 42885 42965
      42937 42871 42932 12396 42961 42958 42848 12395 42875 42929 42934 42852
      48228 21924 32722 30972 23436  7858 50150 40826 48519 46909 10966 12781
      41771 24531 14420 44347  1060 34525  8723 23574 30334 45871 28436 10589
      47333   829 33463 37040
1060:  8021 49176  9433 43154 38938 38171  9728 36131 29032 33360 42887 37630
      24580 32084  4440 26882 19495 36647 34729  8815  7483  5805 47084 30359
      11599 28085 43220 39468  7690 43000  9258 44811 13319  2042 43259 34774
      19989 29620 18178 32592 42980  2135 43924 31777 36246 22301 20335 52048
       3370 39327  2507  9492 29538 32369 49398  1943 17939 50853 27288 21946
       7839 14935 14143 14159 33516 33554 27800  6071 42294 47598 24641 43503
      40642 17844 52723 16962 23719 42499 49369 46292 26898 43089 33466 52098
      18384 42361  4006 33255 26583 51724 44664 18584 17913  7532 31198 22658
      22686 22688 20452 22689 24953  7308 44371 33287  9535 21756 43176 35975
      25849 20450 11274 11275 37150 48045 33281 10303  9414 32644  4630 21630
      51284 50557 49118 12052 21830 37127 21096 29503 29517  8047 46717 31923
      35853  4340  7369 15979 43188 43184 17051 36946 24568 20838 20871 20869
      47662 18505 21090 20875 39411 10505 12337 11352 11355 52579 11351 43706
      34755 19873 19850 39661 19870  3164 15276  7598 23054 30018  7053  7059
       7056  6994  6923  6926  6289  6990  6961  7017 23295 49875 38114 39052
      39050 15041 41042 45243 11454  3256  3254 38668  1625  1306  1807  4094
      44972 48435 29191  2236 29012 11961 35195 27461 27452 27459 23613 17017
       1713  7343 36189 23915 31086 12404 39296 51484  9865  7585 46065 10676
      39858 30213 36286 37069 40203  2513 34337 22189 12689 10786 13258 18591
      43596  9576  2967 38710 13278 49990 51706 42994 16403 40336  9460 50780
      19571  8979  1386 47412 27876 35018  5503 20404 18231 20402 24794 42996
      42885 42965 42937 42871 42932 12396 42961 42958 42848 12395 42875 42929
      42934 42852 48228 21924 32722 30972 23436  7858 50150 40826 48519 46909
      10966 41771 30334  8723 23574 14420 10589 45871  1059 44347  7705 24531
      28436   829 33463 37040 47333
1061:  9336 19171 27611  8177 30062 13466 38670 10955 15846  7026  1998 31818
      31820 49639  6561 28843 51496 44754  2986 13023  4637  3121 14509 25633
      35417  6707 27702  1778 17603 49028  3425 51175 23628 23497 44526 28541
      19999 28194 10183 10180 10184 16089 16086 49030  8233 24366 45452 20876
      30774 51872  3810  7293 16916 20593 18929  7865 48017 15815 27016 28784
      30411 13962 49550  9633  6943  6946 23227 23826 25352 44676 22763  5833
       8442 29865 39781 13945  2952 30862 15065 28792  1595  6266 40399 36212
      22443 10471 28757 33068  8529  8526 38672 20043 18565  9140 48349 21106
      47650 24528 27138 43353 22173 28506 45708 27699 21431 49801 51023 36057
      19752 19754  7380  7377 52613 11031  9052 22440 24807 48149  2630 51911
      30045
1062:23345 23946 10875 50682 50650 36328 27812 36449 18771  8566 49237  8420
      31830 22524  9847 41170 14626  2930  2440  9348 33297 28800 43652 38577
      27733 27748 43735 51346 24748 13911 15407 34954 13774 17765 38831
1063:51827 37791 30932 41314 47499 19213  8641 17924 18785 51463 15337 26442
      10327 52029 52031 52026 46962 45142 25101 25529  4780 36181  9604 52205
      52219 50634 47509 51008  6851  7022 51829 14664 49418 41131 45329 21970
      48853 13334  8914 19785 14300
1064:51827 13279  3288  3305 13301 12354 47499 34034 41314 36225 26762 19213
      43807 14276  6377 32373 25202 32374 40342  2041 15196 11018 52031 52029
      46962 45143 32653  8013 21010 48853 34129 34400  1065
1065:51827 13279  3288  3305 13301 47499 34034 12354 41314 36225 19213 43807
      14276  6377 32373 25202 32374 40342  2041 15196 11018 46962 45143 32653
       8013 21010 34129 34400
1066:21321  2178 48775 42078 28796 31296 46638 33996 25561 18180 41958 27525
      34175 31696 24141  4153 15656 11945 43437 42607 46493 51369 45711 48807
      48810 13928 37333  9201 42134 43020 33852 32175 42270 42261 22484 22480
      36370 27917 27918  8379  5327 20216 51342 10494 43613  8956  5131 44897
      30900 48656 50813 31878 50818 14967 51344 22598 48060 42476 40365 51347
```

```
         51345 18792  5650 28805 12757 12753 12063 25572  3666  3865 43557 33532
          8838 52062 52063 45142 45257 51339 45274 40560 40656 34040 46830 14511
         22714 42577 49108  9483  3332 37094 40558 26220 29933 40555 51071 12143
         21343 16785 29650 32595 46921 45306 46918 46858 36417 33220 21970 49437
         47327  4077 23343 23360 30882 45286 33838  7683 19725 28881 42041 19722
         42047 22334 48837 13333 22343 22347 36951 30483
    1067:32941  6987 13567 13503  3287  3284 41982  5066 22942 47657 16052 42402
         46589  5325 34148 32439 45826 38432 45102 52023 24821 27488 37177 12270
         19198  2864 42816 24140 39363 33407 52229  1780 45984 28071 26839  2315
         45502 19596 21474  7251 26340 20487
    1068:43293 11995  4541 45723 12903 24385  8386 40246 23641 26183 35043 29064
         27337 42086 39151 37678 28198 10776 26444 51557 45569 22946  7790 49819
         12821  7788 12794 12824 51580 10747 13105 13125 13196 49315  7784 12790
         15197 25488  7212  5958 25609 25580  6024  6020 12379  7378 15569 33691
         36315 22432 47004 20777  3856 38639 21122 50798 41734 39879 39800 39875
         39845 14577 43408 37099 24435 50051 16292  2993 18552  4548  2719 16400
          5684  7925 17847 35692  3790 39799 39330 35651 35654  3567 43490 47236
         51237  1857 51544 28654 24113 17642 51698  8602 23955  8308 28369 19655
         19570 33289  4409 19617 52394 41386 15156  6735 51117  2442 33990 19551
         44301 45391 32495  2893 17140 36789 29207 11231 16351  6283 12994  8293
         39837 50789 31430 25255  6234 41473 44294 34045  6596  4817 19111 22871
          3802 48288 32881 41991  3597  2866  2474  8254 50228 21047 43496 48232
         14997 51782 17732 25184 32149 14009 24686 14323 11160 39323 22450 39258
         35613 33319 19340 51100  5761 36393 35832  6043 34638 14720 34567 37802
          1509 52257 31384 31816 28667 16808 38537 14639 34911 19530 13546 52391
         46791 31761 21576 24068  2153 12314 20951 43337  4600 31483 21265 27838
         36690 31798 12634 23306 39982 24508 30536 44279 35565 50195 47541  4807
         15942  5520  2854  6093 18954 10517 25032  5120 13192 19522 26987  4142
         14105 12365 21264 11423 33041  2743 43452 25556 23147 26346 46763 47196
         20207 26398 16561  1641 29450  7127 49180 22188 47237 15252 25524 24309
         16973 15069 37583 44448 38704  6060 50596  2197  5436 48261 19093 19158
         34023 27572  9417 21326  8231 50751  9768 14357 24870 32729 52041 47783
         31090  5693 49452 50308 30374 14055 34054 15135 27886 36834 16662 31990
         38964 19659 19664 44580 29542 30650 20925  8716 10031 18839 23425 25671
         40631 32763 37912 32098  5982 51883  4216  3113 45597 11489 31370  3104
         45651  3931 17068 47035 32655 39331 51908 49187 49190 31504  5991 25612
         43326 43463 32276 48474 34404 34412 20073 16012 16737 19760 39659 10807
         40281 40476 17977 29693  7863 22006 29909 20968 15107 25500 43723 43206
         23026  6117 14637 28887 35561 42152 33082  6013 25493 30627 40462 20831
          6312 15207 50775  4448 18135  6766 36251 13323 25057 45952 37455 19008
         14676 18755 36334  6053  4741 14151 14883 32230 35678  4593 19334 49321
         21415  2964 19940 26780 23640 18488 42464 14968  7285 47734 37108 12362
         34610 13087 19866 46370 17619 50773 25504 25129 25154 25533 25530 25558
         17468 27306 36579 27346 27310 27303 27342
    1069:47520  3036  5936 19387 45197 29070 48809 10558  3948 31750  4351 13769

    1070:32336 46574 45872  9514 50209 28486 40125 17954 29670 34353 51650 17773
         44608 10448 24186 42135 17230 10457 28072
    1071:30418 52129 48365 26064 11300 38346  1671  9093  5943 27354 49917  8484
         18186 25175 32758 33591 33615 39225 47489 32202 41025 52361  4031 42018
         13249 44363 25432 28771 47505 25965 49577 32789 36165 33014 28222 10425
          2059 37155 19421 52353  2742 42890 42912  5757  3588 48999  1689 20746
         31678 50358 44662 46149 20972 48003 40426 45593  7735 12724 22588 27182
         49738 29140 42312  2118 26648 36161 24358 52384 38408 29104 19333 18634
         41541 40931  6196  7907 19212 19128 50799 50781 25845 27498 45166 43115
         10998  9626 26732  3913 46716 24719 28099 48520 16424 32248 51707 21220
         22075 29193 11021 23495 50721 25509 51223 14502 46848 32670 19034  8925
         27210  4808 27905 25010 26659 26153 21331 27961 17175 33140 16146  6954
         38321 15907 33039 16820 21906 27219  8341 37414 40477 12784  9743 37503
         22335 36152 15145 31775 15031  1867 23732 10798 35750 31407 10197 11183
         40187 21244 47618 26952 47256 33347 44142 23413 52172 41562 44498 41645
         45081 19519  1790  9143 29322 13878 44015 19283 20791 37238 49331  9310
```

```
        17502 14947 43265 30275 25840  3720  5632 19163 23781 34884 38212 45706
        44358 48740  8045 25842  8302 45581 19392 44029 43712 20377 17830 34623
         3365 30173  3578 34486 39002 36040 32451  2895 17019 19314 17129 15449
        45225 30095  8125 15004 45183 18154 27824 40979  5752 21764 28574 16734
        25860 51164 28560 16108 37112 32226  9060 47532 13450 13816  7421  8043
        51743 18960 37647 22900 30222 40453 42698 24096 52138 46071 39968 17600
        20314  5183  5343 38363 38360 19132  4839 18620 19294 28639 50820  5002
         4620 47984  7516  9402 38036 16323 31440  8701 37702 31636 11337 34522
        11825  7360 25254 41297 52405  3502  2733 16701  2587 37039  1542 38736
        45659 22375 51467 21489 22281 29924 13228 12350  4528  9717 41110 52525
        10685 43725 14840  7223 22379 39008 30114 34065 19478 48585
  1072:50422  4414  8427 48972 15165 27168 34188 40792 41010 36318 15546 23445
        38984 18504 41975  4867 11817 14706 48424  3493 25165  3628 41560 28390
        15842 43164 35021 48652 16914 50940 31224 27408 33617 27412 49257 19106
        10789 25831 30354 13662 28630 21675 40376 35211 29661 33181 10982 28496
        46743 40244 39960 44403 10161 32329 16061 46906 23587 42057 18577 43453

  1073:29233 11978 32392  4870 19133 49149 49124 29767 16208  3166 33113 33155
        33149 33146 17314 37459 47347 18372 37959 41159 23644 42501  8315
  1074:38786 48024 39763 52491 52688 39692 36469 23783 15735 23258 42351  7016
        22187 20370 13779 18768 30782 11034  8041 48932 31863 15843  5143 35827
        12460 15104 36305 45506 38344 13435  2517 32087  8124 20920 28327 26447
        37103 38525 11243  8337 32105  3661 46489 35860 24247 29479 25830 22421
        39256 46140 16249  9672 40487 16969 15325  3953 29918 40251  6578 40922
        37960 36612 13343 35983 33654  6538 46470 13442 52480 48671 24593 51368
        21359 45683 52725 39201 42674 16877 39167 38383 23988 41581 43771
  1075:18502  7591  6620 22356 46040 39579 43864 26051 16266 46930 31105 45577
        18886 45943 14000 46933 49713 40818 23450 21526
  1076:18502  7591 46653 18500 35542 41030 47147  1728 41725  6135 40435 19087
        35408 11960 45577  4152 32138 43901 49664 43230
  1077:32704 10784 11122 36621 33206 34664  9642 40183 43435 48275 49215 44686
        15395  5587  7584 50322  9241 51571 49203 50079 40723 43066  7695 48236
        28913 44691  7825 31210 27515  4906  7239 47881 39115 14906 44906 46151
        14015 33678 10864  9903  5174 17100 38807 28279 45736 47288  2420 17646
        41345
  1078: 2478 41984 21866 19459 48564  3834 19043 20427
  1079:39739 32583 14607 19585 52600 49564 23992 28811  5738 43091 46654  5708
        41471 41469 21693 24551 26284 51511 52489  4852 15561 28644
  1080:37465 37614 48860 19064  4198 49288 49286 49263 49264 17850 13593 38410
        30853 30856  6413  5688  6390  8268  8214  3723 49748 29931 31458 22826
        40320 40323 40313 45844 34648 39871 13943 30813 34599 26327  5898  2095
        29836  5893  5872 49005 28144 27937 31083 29834 48726 35430 36345 44849
        38218 42751 42752 29505 34925 34539 35200 35193 45270 39683 46648 21523
        46507 33544 27977 25387 24939 51407  8976 21378  3212  3301 21817 52449
        27776 30809 23962 44854 35220 44479 42572 41778 41703 19915  5866
  1081:40779 40245  6626 39933 21613 21578 21609 23128 28196 26446 38550 36067
        21615  5962  7214 36316 22128 32120 22433 38640 23921 47329 37101 39700
         5683 36375 39315 19611 51239  1856  2827 13097 18299 17948 44491 51001
        23934 16376 16344 16378 39830  4407 15159 15153 46801 40829 16913 16374
        11232 35826 20675  2266 16396 33434  8419 47636 43102 23775  4145 19341
        19047 18325 14717 34570 31762 26454 35888 31759 21577  2155 28655 27839
        44397  8624 17755  1930  1929  1926 33043  2744 52239 31453 15253 25525
        40481 15096 51608  3568 51894  8285 19079  4125 33742 34055 36835 49826
        29460 15098 15103 23427 40632 49169 40781 14591 51885 44547 44542 14102
        35060 48470 16406 43631 52741 35549 40809 40808 40804 40805 40807  7233
        42754 33110 40145 32728 26798 49195  3169 31758  2696  1084  4649  1829
        12583 25837 31525 34286 43949  7912 28888 37710 14215 24615 15383 20255
        29297 18487  6016 27242 15890 26563 49167 47238 49423 45602 31270 28730
        14882 36634 30531 31477 19351 11648 11649 21411 50917 28734 23746 49164
        49173 19868 23717 19458 34920 31339 15341 43231 51130 20419 13558 15342
        37904 25527 24065 21573 28587 36797 31932 24498
  1082:27946 19179 28176 28167 43294 11997 32716 26139 49101  4542 45724 12904
```

```
    8384 24383 40779 39933 29066 14777 27640 18894 18302 18275 18276 50863
   50774 50778 50776 18298 18294 18304 18295 18269 21613 21578 21609 36517
   42084 39152 51595 12470 28196 45570 22947 36045  8346 49821 36072  7789
   12797 12822 36036 12827 12793 51604 49364 36099 38550  7781 12829 36067
   49317 36041 48422 21615  9991  7785 36065 12791 15215 25490  6028  6022
   29837 12382  5638  5613 22128 14594 43990 43411 40594 24433  5916 24589
   46160 16293  2975 18554  4564 38149  2722  6891  5783 16401 36708  5683
   48345 38972 34370 39315 35652 35666 35668 35677  3566 43470 19611 51239
   21692 22587 20641 51543 44491 23934 41103  8309 46302 28371 19657 46945
   33286 33290 39830 40796  9526 18922 15981  2323 37984 19616 46853 47568
   47564 24245 46801 12412  6736 51122 40997 33989 26339 19553 44331 44330
   16346 45390 32478 34289 26506  5434 17906 17914 36790 29209 26148 16353
   39856 28202 50191 16395  6380 50788 29037 25252  4336 23487  6236 27267
    2886 11093 41472 44292 13484 34637 33434 34670  4813 22872 25800 28140
    3799 48286 32883  3621  8256 50230  2475 24700 42366 21070 18319 43495
   48234 17731 46705 25186 32151 14007 24689 11158 22452  2336 50560 14938
   14911 33321 17687 36396 35831  6044 34639  6015 37803 52255  1511 31359
   31817 30609  8492 38539 14614 38880 19534 50794 13548 27493 27496 27500
   28139 28142 28147 28149 28164 28172 28179 28197 28201 31759 21577 18274
    4975 24072 41321 10360 12312 43335 20946  4602 14908 21263  8108 36692
   31799 16588 27887 12639 23304 39983 24816 25396 30551 44277 26472  3838
   33576 47543 23558 28880  4806 15944 11367  5515  2853 25030  6094 18952
   10343  5118 22392 18962 19521  4141 23152 28078 21338 14103 17755 12366
   11556 21262  3366 34401  1930  1929  1926 43451 50207 25537 23145 26348
   44350 46764 47198 20191 26396 15991 29451 36207  7128 41098 49179 22185
   47241 25525 15096 51608 37584 44447 52090  6062 45624 50665 27798  4232
    5440 48260 11170 19092 19155 49141 49223 34019 15066 42295 16920 40687
   50752 14113 24868 14339  9770 32732 51196 47785  5691 45178 49455 50285
   30372 34055 15092 27884  3726 23942  9110 31992 38980 24881  3354 39881
   44576 47764 33688 29543 51198 41099 30679 34428 20956 49826 29460 15098
   49066 15101  8717 10030 18841 23427 25673 32765 37913 32099 15648 15644
   16445 49169 49171 40781  5985 51885 48321  8258  3114 23375 44547 44542
    3748 32344  9166 18008  5016 22671 15702 49192 49189 49068  5993 25613
    4982 32853 32274 43322 43329  4180 43320 40809 40808 40805 40807 40804
   48476 48482  7169 12114 11007 20440 46523 24889 40280 40495 10344 15095
   49195 15109 25502 43724 43209 14628 10601 23741 10351 38702  4649 19237
   44010 43143  7912 18301 18303 18307 18979 37710 14215 24615 15383 20255
   42153 25174 47359 18487 25497 40443 11350 15403  3199  3198 35102 20830
   15344  4444 15369  6313 15410 15380 46810 27242 27534 50904 50871 24064
   50897 15212 50906 50870 35560 41184 50867 25904 24793 48701  9294  9251
    9124 10778 35162 49167 15406  4408  4739 15409 22597  6768 15413 15372
   15370 15375 15376 15412 15414 21950 36250 13375 12294 18974 14166 41431
   42116 16075 37460 35672 35647 19009 14677 46074 41944 15378 18757 36335
    6054  4742 42533 35196 31270 27062 28730 50458  4589 49323 11648 11649
   21411  2965 51715 16023 13595 13597 50917 28734 20795 34374 34425 26101
   42447 23746 30225 49164 49173  5079 17059 18489 14971  7286 47735 19475
   37110 18484 19482 23539 51918 43032 38320 34608 40784  5318 16741 19458
   46371 17617  5215  2921  2924  2929 45196  3474  3447 20103 20101  3476
   20106  3446 48343  8938  8562 34920 13558 14902 15342 37904 25527 42799
   17469 18891 27670 16793 36109  9561 18895 18897 21573 40774 24515  9358
   43907 24513 13543 24533
1083:32716 40779 40245  6626 39933 35044 26184 21613 21578 21609 11394 23128
   28196 26446 38550 36067 21615  5962  7214  5638  5613 36316 22128 32120
   22433 38640 23921 47329 37101 39700  5683 36375 39315 19611 51239 21692
   22587 20641  1856  2827 13097 18299 17948 44491 51001 23934 16376 16344
   16378 39830 15981  4407 15159 15153 46801 40829 16913 16374 21660 11232
   35826  2266 16396 29037  8419 47636 43102 23775  4145 19341 14717 34570
   31762 26454 35888 31759 21577  4975 24072  2155 28655 27839 44397  8624
   48237 14103 34401  1930  1929  1926 33043  2744 52239 31453 15253 25525
   40481 15096 51608  3568 51894 19079  4125 33742 51196 34055 36835 49826
   29460 15098 15103 23427 40632 49169 40781 14591 51885 44547 44542 14102
   35060 48470 16406 43631 35549 40809 40808 40804 40805 40807 42754  7233
```

```
       33110 40145 32728 49195  3169 31758 41322  4649 12583  7912 37710 14215
       15383 20255 29297 18487  6016 27242 49167 49423 45602 31270 28730 30531
       31477 19351 21411 50917 28734 34425 23746 49164 49173 19868 23717 19458
       34920 15341 43231 31339 51130 20419 13558 15342 37904 25527 24065 21573
       37252
  1084:19179 27946 26139 40779 40245  6626 39933 14777 21613 21578 21609 11394
       23128 28196 10775 10770 18196 26446 38550 36067 21615  5962 29837  5638
        5613 22128 43411 37101  6891 39700  5683 36375 39315  3566 19611 51239
       21692 22587 20641  1856  2827 13097 18299 17948 44491 51001 23934 16376
       16344 16378 41103 39830  9526 15981  4407 15159 15153 46801 40829 44331
       16346 34289 26506 16913 16374 21660 11232 35826 20675  2266 16396 29037
       33434  8419  2284 11010 43102  4145 17687 19047 18325 14717 34570 31762
       26454 50794 28201 21577  4975 24072  2155 28655 27839 44397  8624 18962
       19521 17755  1930  1929  1926 33043 26348 20191 52239 31453 15253 25525
       40481 51608 50665  3568 51894  8285 37312 35172  8097  8594 42295 19079
        4125 33742 51196 34055 15092 36835 47764 49826 29460 15098 15103 23427
       40632 49169 40781 14591 51885 44547 44542 35060 14102 48470 16406 43631
       52741 35549 15702 49068 25613 40809 40808 40805 40804 40807 42754  7233
       33110 40145 32728 15095 49195  3169 31758 43209  4649  1829 12583 25837
       31525 34286 43949 28587 31932  1081 39084 36797 24498  7912 28888 37710
       14215 24615 15383 20255 29297 18487  6016 15890 26563 27242 49167 47238
       49423 45602 18133 31270 28730 30531 31477 19351 11648 11649 21411 28734
       23746 49164 49173 34608 19868 23717 19458 34920 31339 15341 43231 51130
       20419 13558 15342 37904 25527 24065 21573
  1085:28199  1866  7482 46081 48284 51965 35795 36425 29899 26716  5523 26099
        4166 17262  7473 36101  8622
  1086: 3787 25803 35040 49785 28038 31160 24637  3947  9992 20068 49403 46078
       27531 52565  5276 44361 27556 44677 52071 24250 33766 20674 22483 50784
       46006 37138 51641 49986 48057 49772 31250  7426 50718 21236 45885 50527
       21728
  1087:10335 18268 24814  3745 15268 48300 14471  8778  9767  2043 50175 22865
       41754 29811 22226 27779 40512 11342 49809 25404 11279 20197   793  2947
        8197  5110
  1088: 2521 48537 49464 31592 23221 29853 32083 36374 51019  8822 21738
  1089:14077 43221 37015 31404 21460 14073 14070 45896 27652  5770  3501  3504
        3473 20072 20699 51656 20694  3100 32747  3080 40701 18990  8169 24439
       52387 12991 36273 29336  8192  7480 13212 33779  9092 27594 36914  3654
        9695 42535  4706 47691  4702  4677  4709 14722 47335 30692 30801 36382
       12900 14625 30811 51995 42458 14128 37569 17694 37644 26828  2751 23179
       27469 27505  8176 27650 27564 38011 18213  2946  4310 27628 24242 27074
       24730 44308  8546  6794  7577 52698 15896 36912 29672  4645 40969 38871
       33211 24171 50159 41468 52159  4433 25147 18549 21661  3604 10087 38501
       39338 18880 42861 34340 20341  1915 30255 40409 14314 29729 20521 51503
       51523 36497 20965  6291 35974 32014 28984 22786 22373 43850 49680  2425
       49581 49603 38328 45746 45755 26380  3469 29240 33564 27474 27625 27644
       27600 27681 18011 18009 31954 30233 16074 32111 34995  3101 21590 41931
       41933 21821 21816  6037 35509 21840 11339  8828 24168  1617 35505  2396
       43894  1906 31537 19502  3678 41913 22434  8829 41979 29159 38372 41869
       48108 50292 50257 13289 22500 27046 40633  3741 18103 40927 17325 42858
       40031  4831 52746 49489 29308 31140  5773 10173 10093 41342 30442  5375
        7273 33683 18824 40505 32707 16729 42273 13932  8112 11866 39809 32708
       37389 21682 25244 11201  9152 41939 20988 20587 20643 21323 21329 34840
       51364 27147 24555 20724 44813 43784 37279 11272 45900 45931 45933 19403
       45892 45895 18754 26475 10415 32523 44978 52498 34809 48340  8274 27922
       46200 48854  2339  5863 27661 43460 21062 37448 45714  1994 24550 30651
       30598  2799  3560 21140 39932 18623  8808 47417 48211 44180 51419 44965
       32197 34796 33623  4983 23560 26424  7762 42217 34753 12743 12785 25243
       22138 46242 12227 27012 49075 47539 48825 22184 47069 12024 46365 22219
       46942 47091  7079 36550 12905 27561  4474  2297 42992 40892 35970 32822
        3433 23898 30332 44987 25433  9013 32430 12181 12688 11857 30388 47007
       15122 48526 48575 49673 29363 49338 34854  1995 19354 18279 28946 15162
       31271 31266 46688 46468 31413 28714 34910  2845  2847  3468  3507  3509
```

```
44375 10683 29484 29507 22291 13521 36684 33815 39701 23246 11581 47392
42557 49916 12620 24288 40966 40907 28533 26779 25158 34905 50223 23044
51592 33357  2569 37276 37321 20098 37175 45234  3143  6842 19769  5733
44606 44605 33693 49898 49833 51362 52178 42926 50791 27560 32238 33994
15084 27557 17730 18108 46332  6517 42652 15950 45211 17528 37543 42517
24561 18719 24379 36069  9682 18738 38228 49966 40799 44596 20381 28834
19318 48415 50346 21310 45352 18418 25362 11468  4405  4383 15444 42573
 1897 37941 37211 37323 21600 23965 44026 45439 48081 48293 32715 20389
44125 23101 23099 48407 18819  8306 21128  1508  1531 16571  2566 44258
15841 32867  4362  4151 26538  8167 52419 26395 50099  5404 24485 48426
48667 33792 31230 34751 17359  7461 30152 51976 52094 43662 27538 46490
 4023 11437 51005  4352  8271 50740  5202 36050 34164 30605  9151 46601
24617  1484 12454 44329 17824 20918 46158 15891 15928 15925 15949 15923
15897 15920 13470 18397  6330 42418 13026  9835 17218 41835 19732 24102
13187 30988 25013 29302 25351 36920 38219 17144 46805 52127 23341  8679
50164 42301 12480 15088  9995 42560 17106 32734 30967 33439 14249 25047
16684 34307 32165 49093  6128 51305 41941 12180  5001  7504 15952  7310
26305 30840  3857 16126 36964 23315 36574 45360 35413 11889  6686 32686
 1313 41485 33590  6566 45018 12869 46846 50515 46517 48788 43003 22751
27507 48893  8934 26207 45936 37686 16348 39318 16350 17691 36917 23216
29493 10113  2229 10953 21889 10824 10112 12518  5645 35188 23097 22263
10834 26233 27656 49627  4732 37383 17195 47433 21141 17332 21110 37486
46796 17210 47574 47606 17193 47582 47604 34184 34212 34179 21648 21583
21621 34229 21542 21580 34897 21587 21650 17299 17296 17292 21575 21548
34901 21330 36586 21545 36563 47435 36587 36566 34903 24481 36585 40531
48019  3503 26680 45731 40252 16703 51547  2814 19526 29421  9315 18991
21422 21457 42494 21410 20583 21320 20612 20580 20640 20637 20635 20614
21418 21325 20616 21455 21459 39286 35322 21492 21362 21626 21496 21623
46772 37068 41375 52076 32237  8577 36388 31389 44118 17286 23750 18014
46380 15467 10510 31002 30982 30950 48735 35935 41976 36306 36782 28965
51575 25200 27162  4527 41540 34334 14663  6031 41935 27867 27566  8226
 8229 41823  9703  7158 20321  1991 19422 42594 16227 44834 47263 45980
43743 48095  6140 17495 15900 14540 14538 19731 49163 16807 18300 26980
14083 14080 14108 14078 14098 14075 14072 50176 40057 49134
1090: 9657 21960 19609  2216 45612 42038 20005 37274
1091:37547 37542 10190 50303 26432  6797  9807  8582  4825 31531 21824 35803
 5858 10419 16822  1935 27979 42747 10487 30120 36867 15621 25177  3126
 6550  5347 22816 42903 19313 26663 45362 34888 21865 32127 48968 49319

1092:27925 25541 26853 32951 13617 42788 34804 47154 51915 35611 37159 47818
29428 39941 31859  6011 35037 49775 40960 25603 33468 48829 21755 20195
33348 23509 23510 14650  8272 20296 23843 35427 44364  7126 46714 20991
18551 33455 28919 40118 24835 37091 24492 39189 26644 10717  5671 23689
51833 43755 51766  8311 34143 10758  4854 32378 20100 22327  7892 28837
23680 52626 46048  8327 18447 30183 16814  3000  5510 11402 15368 43774
32845 28788 16095 32532 42839 30901 11535 47843 47469 26120 31894 11067
35593 41813 48357 30566 40047 27927 22408 28328 38860 29165 30099 49686
28391  9094 19342 14231 38195 34696 48832  2679 32054 43789 32211 15617
46127 52139 49623 27573 28324 47191  3227 24251 10058 21147 46759 40775
47757  7436 37632 10484  7740 50782 32889 16436 45907  8532 24049 41668
 3353 32955 26616 35562 34128 30764 16408 23342  7631 38606 33689 26074
47221 39984 26812 51903 23759 44968  3638 33651 25471 40366  9392  6635
50244 40123 18633 19078 17644 35461 36936 47042 47888 39519 15021 39520
49812 34950 15430 30248  7309 48846 38277 19397 49382 24892 21164 42598
43134  7653 34435 35093 44328 31272  1551  1838 43536 50695 15349
1093:39278 25947  4847 16949  2670 35828 18056 21751 44822 40007 12231 36937
52182 39433 51651  1717  9805 10196 30514 30340 45696  6224 38240  4009
 2850 45216 16191 48766 52553 16988  3083 38406 41822 25648 20227 36059
34097 36625  2545 52092 14187  8057 21570  2058 13186 48896 41964  7840
29579 37241 30127  7388 51852 26781 28177 11099 29751 16073 39048 20301
13237  8033 16597 44037 12864 35418 42067  4824 12053 41651 51562 35529
 4044  7787 34309 20697 35470 45887 38579 34166 34149 36364 43417 27868
```

```
          3659 15685 26707  8604 51605 37600  6357  1553 45476  7980 35999 40113
         42010 42209 41133 46397 22979 30869  7587  8248 35609 20418 15443 45323
         23686 20306 41244 39246 26957 45671 33535  8244 35539  5590 40819  4737
         50716 13781 44828 43397 39305  8916 22636  2710 23456 16629 39456 46047
         23270  2602 11307 11412 13162  1547  4459 10269 45757 48370 33238 14161
         24170 31845 46712 18569  2238 29096 26310 36243 26634  8818  4100 33626
         27036 16694 10217  2501 49536  9461 50814 49131 21852
    1094: 7199  4443 12712 49432 49439  4766 41361 27668 43812  1777  1832 30097
         12307  8267 48372 25987  3211 36391 29373 27108 27909 39537 51512  2824
         34689  9400 14290 44396 17965 32414  2891 37902 42474
    1095: 7199  4443 12712 49432 49439  4766 41361 27668 43812  1777  1832 30097
         48372 12307  8267 25987 36391 29373  3211 27108 27909 39537 51512  2824
          4022 44396 32414 17965  1575 49549
    1096: 7199  4443 12712 49432 49669 46221 25987  3211 36391 49363 29373  6267
         37476 41923 31466 14482
    1097: 4443 12712 49432 27666 12381 50118   767 47213 25987  3211 36391 49363
         29373 19930 29510 12428 18102  7089 48789  7331 37476  7440 46614
    1098:10844 45400 36713 43986 25284 10902 26670 39169 39164 48653 51756 51757
          3812 36735 36731  7519 22759 36737 36739 36743 36762 36710 10785  4987
          4989 36770 36771 32413 48196  6779 18916 46270 22475 12273 51923 11668
         11050 11046 19297  8908 45780 44046 44049 44013 44008 11008 36652 10906
         37426 36637 10923 12192 49412 33114 12635 51593 19382 33303  9012 21246
         31355  3402 50533  4779 15956 15209 12504 39907 39930 39928 39903 39905
         21193 39900  2932 39309 42284 43840  1393 32868 12189 12140  6803 12162
          6806 29637 37586 44312 28165  6972 38616 35468 13746 34420 13743 29776
         24455  4175  4177 31970 33799 10417  5091 18571 23076 45945 12164 12158
         12171 11695  8649  4972 27132 13857 46529  1301 20581 12193  6809 12194
         12197 16046 14945 30941 28878  4106 32625 32609  8458 10829 22502 39088
         17049 35670 11673 11692 11677 31746 15906 22132 18912 49786 10817 10820
         18651 10929 10926 11016 27384 27407 36766 36767 44155 12168 12059
    1099: 9335  4667 46990 35324  6160  5546 11932 39423 39427 23504 23503 41644
         47882 33774 37336 30825 51668 24742 14679  6404 29017 28819 38135 17970
         38603 37835 37832 49935  4308 49341 41281  8182 49074 36814 20004 20006
         37864 27215 42252 41315  6319 28055  5781  1315  6841  6820 29341 32636
         32637 17942 45203 17903  3182  3599 14089 15958 19279  3475 11129 11130
         33553 13826  2990 32443  8543  4747  2832 41409 41407 17415 17412 45376
         45378 40787 26006  5205 22664 51830 45138 51251 51256 51938  2708 42630
         37249 27967 14041 30103 14090 34098  6750  9871 40220  9951 31808 22259
         34542 23183 13074 22617 23731 11882 13514 37017  9553 26203  8523  8522
          6983 37731 32370 26202 33961 27577 16032  3203  3229  3205 26926 23939
         23938 29816 29804 24225 23528 46120 15931 21251  4134 40906 34607 50702
         25583 41802 11111  7793  7791 49125 18499 18501 20066 15549 49311 49334
         48563  5094 10176 33026  6265  9139  7195  6102  4004 48558 26870 27365
         34940 26496 26498 26277  4955 28489 10872 10870 43918 23374 50539 10823
         10889 33746 23334 23336 12929 16844 21558 17331 17336 17335 29927 45583
         43318 10896 45797  6299 18113 43494 24258  5261 21864 27291 34363 47386
         49537 47358 20053 47361  2298 49553 35026 47094 36875 14687 16356 32594
         44740 44807 10984 24933 24935 24936 28155 39610 36779 51611 36774 36776
         28592 26924  8606 30445 45590  1589  4725 24019 29052 31241 33573  9946
         31988 21636 24201  5689 26731 39016 24705 24701 40693 40694 40698 47844
         29074 29071 46083 42076 21664  4240 20316 35278 24151  2780 36861 48473
         36948  8504 22196 22199  9725  8502 19691 48400 20554 48562 41854  5562
         26068 48397 31925 46352 48559 41230 41228 12871 45454 22558 17763 23426
         15864 42531 40096 37821 22736 22733 20727 39366 37338 37337 39365  1100
         23894 50468 27056 36729 24643 24521 44911 48759 23541 16948 36995 35925
         29868  1883 31101 48987 46194 46018 50172 24345 42483 40019 26988 42750
         13825 38978  5765 21504 26996 15683 28059 50144 18129 28830 27553 44796
         35919 32906 32251 29564  5288 34713 16230 16226 46209 16228 16229 41190
         51403  2852  2849 13931 39449 39429 39082 36307 16195 38112 23670  2321
         38109  7699  7697  7717  7662  7688 43767  6922 10292 50041 22544 29354
         10648 31221 23805  8494 27464 44880  8051 29149 22062  7999 42368 38675
         17776  5326 11524  4555 47705 16251  6280 46215 45613 23198 40552 43896
```

EP 2 484 769 A2

```
        42583 35245 20202 12416 19435  3746 32521 27924 30871 51447 26920 27045
         9598 18481 40813  8161 31091 19076  8122 27903 39416 39399 39421 36133
        36134 36135 36555 36577 28529 28869 28889 12500 12912 14436 21973 50339
        11004 48371 32121 49994 28645 20437 17665 17606 17602 11875 37652  7959
         6353 43295 39388 17032 17035 17056 17021 17026  9159 51866 10049 10050
        10057 10053 39454 49709 43527
  1100:  9335  4667 15026 14802 46990 37661 35324  6160 26897  3684  5546 11932
        39423 39427 23503 23504 30825 51668  1099 43527 24742 14679  6404 29017
        28819 38135 17970 38603 37835 37832 49935  4308 49341 41281 52334  8182
        10583 49074 20004 37864 27215 41315 46103 17885  6319 28055  5781 39942
        21875  2268  2171  1315  6841  6820 29341  3617 13381 32636 32637 17942
        45203 20584 17903  3182  3599 14089 27502 20469 15958 19279  3475 11129
        11130 12890 44846 10345 33553 13826 38302 47654  2990 32443  4747  2832
        41409 41407 17415 17412 45376 40787 26006  5205 22664 51830 45138 45139
        51251 51938  2708 27603 42630 37249 27967 26059 30103 14090  8734 20347
        34098 32117 25342 36605 11227  6750  9871 40220  9951 31808 38411 34542
        22259 23183 17861 13074 22617 23731 37728 11882 13514 35386 26203  8523
         8522 34717  6983 37731 32370 26202 25354 33961 19674 27577 24915 16032
        33368  6418  3203 26926 23939 23938 29816 27991 29804 24225 17741 23528
        51888 46120  4669 49449  2911 15931 21251  4134 40906 34607 34605 50702
        23211 25583 41802 28308  5996 11111  6212  7793  7791 31156 49125 18499
        18501 45953 20066 20067 15549 49311 49334 48563 41337  5094 10176 33601
         6335 11665 33026  6265  6102  9139  7195 47865 42212 37356 49279  4004
        48558 26870 27365 14996 34940 26496 26498 26277 45899 40463  4955 28489
        10872 10870 11803 43918 23374 49414 12397 50539 21760 10823 10889 33746
        23334 12929 16844 21558 17331 29927 45583 14687 16356 32594 24933 28155
        39610 36779 51611 28592 24705 40694 40693 29071 29074 46083 42076 36948
        19691  5562 20554 48562 41854 48400 48397 26068 41228 41230 12871 15864
        42531 37821 22733 22736 48987 50172 46194 46018 24345 39449 39429 39082
        23670  2321 38112 38109 39416 39399 39421 36133 36134 36555 28869 12500
        12912 14436 21973 50339 11004 48371 32121 28645 25868 22191 28421  4012
        17606 41362 15838 10438 11875 37652  7959  6353 43295 39388 17032 17035
        17056 17021 17026  9159 51866 10049 39454 49709
  1101:50138 46865  7771  7773  7776 29433 29432 29410 29412 29408 29431 42199
        22625  7805 22426 16176 22647 31349 32059 32185 21770 29436 29438 28288
        27663 27633 28335 28462 28337 28430  6311 18884 27667 28287  6220 11091
        11092  6333 45184 12700 47021 22653 16148 16151 16082 16055 30309 41970
        42127 28357  6239  6331 29402 39807 26862 15997 16002  2351 32012 32038
        32160 32163 32109 32181 32107 32082 52236  4834 34650 19041 16523 41176
        41246 41249 41251 41182 41974 42122 42200 41338 42060 41336 42206 41180
        41204 41210 41201 41214 42121 42166 42168 42208 42051 42054 41252 41980
        49904 28847 28870 28874 28943 28926 28947 28950 42090  5262 17627 43021
        51719 41102 42157 14927 50762 30056  7783 27813 26748 49847 49869 20604
        28545  5771 33459 13866  7015 49870  5915  5892 28839 28841 28845  3740
         6218 40468 34813 34808  3711 16177 30108 12836 25549 12772 41198 22665
        49844  8277 31574 31659 31541 30851 30915 31628  7235 32245 48938 48958
        48940 41105 52530 46671 46675 46670 41123 16972 41977 25747  3393  7335
         9135  2609  2636 49849 41334 10179 37128 37165 37146  7323 38454 38482
        38481 38436 18259 51103 44259 22240 44260 43457 46415 11521 40396  2145
        52727  5563 30792 30814  3952  7731 41097 42124 24677 24679  2731 46679
         2860 23627 46867 46869  3738 45001 22659 25340 11544 34156 24274 12764
        39834 21703 21707 21705 49871 46683  7724 46698  7501 36640 40466 46677
         7729 31261 15908 40014 52292 39215  4536 41675 41312 51252 52286 24374
        14321 42161 13714 23394 51277 22476 22479 22498 22474 22449 15924 22454
        16525 18801  7123 17287  6679 12768 35002 44321 44316  8227 21388  8300
         8281  8303 31184  3708 41125  6421 34416  7727  7726 46680 16496 16491
        16520 16521  7774 21772  6281  6278 51259 33182 12023 41118 32210 32193
        49906  3744  3736  1865  7730 49882 26831 12667 42059 42088 29405 41310
        27634 28457 28922 28920 28904 28389
  1102:49955 29348 29349 29465 29328  7278 14406 14413 31343 31346 29352 44252
        44272 44224 44270 44223  6307  6223  6238 11087 11088 11915 12540 44251
        29306 30534 15994 45866 36077 13148 39267 40501  8814 11892 11114 11117
```

151

```
         24312 30054 26730 49908 49930 45835 32067 23108 12833 14379 49932 10393
         14416 39831  6215 24185 12543 22663 10355  8275 49051 29270 29273 29274
         29277 22211 37190 27755 49910 18780 18865 37449 27835 32710 16825 28944
         28108 21790 21791 51183  4064 29332 14435 50588 29307 31048 18519 33787
         20408 38332 23696  7646 32002 25647 49936  4088  4086 29355 29371 29374
         14433 29303 39995 35490 25543 40012 11387 35961 48135 25670 37792 43541
         32457  8353  7093 49928 49929 51699 51702  6250  6279  4097  4096 18860
         51221 48381 18105 29485  8280  8301 14409 29376 29309 44227 44220 44242
         44222  6277 18112 30725 49965 44244 21733 44247 14438 29379 29385 49962
         14415  6243 29298 21729 49907 49960
1103:40052 21700 10316 47891 16042 12917 41622 11868 19252 26257 31077 27221
     13991 14697 46893  7557 45022  7835 41517 30327 11800 15701  4437 40339
     26758 43074 36346 52459 38140 31141 20442 23458  3119 16345 47708  8988
     27816 25569  5439 45164 26426 52222  6569 20915 22623 24101  3583 18417
     28206 44427 10375 21742 49903  7582 26002 23362  8515 16414 21295 48830
     48803 38647
1104:17893 27332 50831 21233 21307 17178 26668 43498 50098 49710 11773 51796
     11713 39195 39526 18685 11282 17223 37031 25586  6365 22887 34961 39043
      3529 16041 50748
1105: 6120 51659 51430 49094 49092 40002 12225 20223 12938 41826 12810 38935
     28261 51787 31043  3590 26537 41535 33212 23825 30846 34438 18923 45619
     25371 49431 21803 31036 31897  8897 14976 31948 51594 14907 31344 47710
      3197 33202 35752  7756 26857 13524 11404 13722  4926 15085 51920  1684
     20935 19450 20515 51535 17599  9850 10162  5129 34739 11733 28220 10728
     18077 37181 49671 45564 51876 46399 50276 28110 25841 41486 30480 30476
     46768 17282 11443  7712 34296 34802 35902  5003  9146 39938 37451  7601
     36957  6590 34634 35984 21436 26057 52017 23531 35222 35223  5454 12232
     44872  9426 24788  3414 30488  2322 10523 48110 33058 23712 28109 50448
     12818 48883 25917  7432 12813 50180 50151 50157 50154 10932 36482 42326
     26079 46419  8762 52399 26499 11980
1106:14929 37621 10040 21488 13592 26301 34020 46202 28428 33890 12418 33857
     12443 38236  3348 19978 33120 14575 35338 22642 25739  7085 43131  1972
      7027 18013  4705 52593 19166 36327 26406 14893  8342 20371 23845  8748
     30913 33443 40898 30755 34246 46666 47389 50441 44785 28861 39205 48172
      9408 32026  6471 22941  3650 14096 29736 28850  7366 10874  4400 35572
     14692 37057 32283 42302 43178  5702 32898  3033 30126 48767 50141  9190
      2154 49156 24740 51671 21165  3189 32771 48006 47672 41547  9932 39222
      2456 39618 10133 33239 18593  2024 45271 27110 22772 23107 45418 29881
      4169  4067 22635 27476 17276 39543 39410 30770 30492 30660  3065  2995
      2596 27780 21882 21037 25870 26314 17091 17882 15190 20438  7986 37171
     11328  3005 24620 52694 14264 46019 27081 45268 31400 23584  7418 25776
     42764 35765 19301 43477 50360 38462 43395 18457 22156 50131 26004 33437
     21557  5263 11814  3454  7989 49035 24607 50135 49448 10110 52057 26445
     43396  7099 24829  7492 12479  2292 36923 26574 44031  4616 16499 37625
      1629 18668 52248 45059  5188 14942 15420 15966 41521 29015 13206 23716
      7250 36158 35543 27858 28696 26020 39287 27852 47478 23996 19841 15787
      2999 30727 39792 17905 26612 17565 34124 49143 24530 34248 21174 39802
     51113 41639  8415 29002 14551 14548 25879  8493 27674 30307 27503  4971
      6854  6796  6383 52282 29010 26756 10552 15664 15637  8120 12514 16829
     14869 14061 52145 13986 27604 48206  8023
1107:26717 40493 31664 36796 29346 16196  6584 24297 40422 40657 30166 23550
     47165 47624 51306 30305 30302 28035  6672 14361  8180 49614 42495 12339
      4205 50454 42391 21822  9457 44636 45317  6030 11200  9641 23219 21672
     27211 38812 22937 35009 25919 29413 20892 26631 39004 37089  6142 35967
     24763  3417 27032
1108:28890 50651 18496 32462 38398 14784  8777 35311 10962 35824 19748 32736
     38565 18027 39190 41454  2690 24599 52543 43839 36164 14345 45507 19360
     20112 40264 19546 29253 23455 32029 40109  9771  1605 38246 19981 21659
      2897 47480 21306 10009  8381 31646 31278 20623 27995 31434  3919 27601
     50315 46097 38323 31195 12685 23317 12191 39896 15445 38814 13360 39264
     25442 14995 14994  8447 10658 17513 33671  5252 47928  4223  3980 46540
      6565 16582  4018 17572 20188 45794 39254 50673  4080 48802 48780 11038
```

```
          13003 22905  5308 37056 13225 16130  6211
     1109:11985 46284  3764 44406 25655 18331 52036 18779 14975 17401 45340 49270
          38064  9486 42726 48892 36511  4462 35753 13143 17031 14781 22015  7260
          32563  5914  2557 35685 20085 38292 50951 36233 20096 40679 14654 45477
          49566 27509  5145 35453 44651 36816 25441 21158 12463 40279  5778 25835
          23847 50620 18395  2830 23792  6502 27247 52207  5096 27330 51334 10493
          24210  2721 30382 20483 38159 35249 39820 23271 49733  2331 42191 42466
          38738 33506 34372 33645 49194 15052 48268 38499 52460 42705
     1110:11174 12266 33905 10254 47910  8424 21754  4544 36842  8963 21421 44766
          24967 50593 50563 52325 10766 50709 18306 17213 22429 29356 47144 49178
          50459 40526 44362  9700 50645 30595  7671 40614 42593 40768 14716 25078
          25068 48414 41428 49866 19678 18921 15799 22466 46416  7714 51385  8490
           4540 41585 47174 13439 51204  9876 20409 36802 11363 26219  2519 29403
           6329  6661 37783 46304 24039  8835 22601 17041 29700  8987 22059 26509
           3577  5897 45526 46255 37498 28554 46145  4954 24806 24056 35726 19939
          15119 48117
     1111:21259 26455 42272 21572 34908 15157 27475 43523 49854 18750 44156  2252
          10224 26199 35422 36462  4795 17770 13810 52042  8028  2101 10508  2423
           8510 38272 21994 15108 20685 48933  3102  1756 27030 49547 22266 21539
          15274 13145 15442 35852 35958 36700 23822 40143 49064 37796  5139  1517
          44660 35421 24022 21966 22932  8004  8007 26165 47711  8571 21544 35086
           4716  9091 49468 16827 19924 48594 35228 24772 26546 18605  2395 12953
          28740 36839 42963 39687 14246  3302 11312
     1112: 9390 46312  3730 44386 22162  4816 18311 52009 22218 18023 18355  9322
           9862 35723 16997 14749 21988  7277 32581  5938  2584 35711 20060 38275
          38741 31232 16904 10715 12993 29825 50450  2516 28528 44773 12947 19874
          44775 41617 14164 14432 14431 34423 14705 22975 20504 39862 38594 29796
           6129 49927  8922 20459 14402 10005 32893 35206 20745 51582 49717 32090
           2358 42226 46862 12112
     1113:39653 28122 10277 23032 43814  8431 22897 30244 37639 36565 32284  8451
          24912 20893 37346  7450 22541 29004 39806 43181 32649  8111 36944 32262
          40197 24181  3858 34946 37894 28635 20818 27061 42740 48056 11220 36234
          20057 37083  8455 25818 41612 51409 51797
     1114:27985 31018  9015 11451  9346 24329 20456  9763 21674 15519 12892 24257
          23929 52743  7304 17112  2480 52435 12044 19773 19399 28893  5447 50120
          47628 17999 13863 45228 52520 50627 26387 32793  2211 14878 41534 42162
           9083 12462 45530 48739 51260  2906 24176 21173 51513 39477 37166 16773
          22313 17207 34867 35169 33325 29878 41550 48668 15047
     1115:14556  6879  4665  8939 39542 45319  3814 44446  7361 18373  4776 19469
           3397 17929 43135 48229 26788 20842 51073 15693  6885 44778 35050  1984
          14881 36686 33261 39239 22365 13916 51672 35855 46444 34341 51921  6758
          34057 22072 14533 38101 22509 20312 41141 36371 10367 52130 19835 40307
          29717 17519 23043 47124 44622  6512 20591 37350 28595  9506 38757 12656
           3461 26637 15087 21925 45192  9924 20782 37115 34231 37143 10059 16103
          46588 34842 45700 18411 25692 29544 15183 18153 23784 41027 35201 31864
          15916 21324 46328  2277 37113 35276 34336 19298 38605 19669 38359 29775
           3915
     1116:34612 45295  3820 39136 28726 46122 47166 27466 50196 33841 33839 34299
          42305 15715 31751 31788 33680 38542 44532  8665 12283 16044  8400 16878
          36398 46477 34367 51281  6755 35004 20839 13502  2360 11186  3392 52739
          16174 38046  2819 51943  3596  4740 16508  5200 14299 30161 28413 14039
          17507 50423 17678 45089 16188 38529 19074 23114 33241 48509 42125 25903
          51893 11578 28885 45189 44472 50986 45631  4244  5654 48450 18630 39377
          19349  6721 21688 43975  4613 17771 38194 35175  9687 50022 23972 32917
          25774 46348  2270 41194 50329 17540 43361 41896 26626 28278 27297 46685
          41022 41992 21458 18932 14544
     1117: 5508  5506 21006  4221 45307 29249 17842 44560 30396 30391 30390 46809
          50968 44571 19367 44577 14880 14859 26914 18517 41003 41005  1846 41009
          14789  2470  1849 24732 41031  1853 33868 22492 43367 19747 34857 43646
           7466 30314 35779 41107 15390 12457 37929 16381 22303 45119 49642 37456
          11503 32449 28782 50849 51971
     1118:14160 48696 48122 52652 27438 40982 41019 21521 27409  5441 27406 21522
```

```
      28473   3139 43879 10029 19709 22035 48674 44383 49347 16787 15971 17421
      34636 48612 41543 41698 16163 47390 40655 36201 44574 20492 44711 25788
       8087 16062 13706 41556 41432 17520 26747 17018  2031 34600 26744 33499
      26093  4011 24527 42004 29625 39825 26091 19640 23275 46595  3630 29624
      22568 51406 50800 26367 50965 42440 23908 12435  8370 12389  5173  4675
      45430 48543 44513  7973  6589 50763 14561 45834 18787  9869  9892  6362
      46054  1550 46512 36383 26070  3088 11131 14076 31941  9505 38345 32379
      50822 26749 10651 44478 33032 33034 33004 33006 33029
1119:33754 29424 39943 20194 39209  6624 24831  8310  9801  4858  3576  7890
      28859 23681 52624 30184 16831 28786 30897 11534 51789 47848  1587  1681
      48358 12684 12692 27928 28325 24287 10483 47990  4945  4226 43653 10764
      17871 40916 30761 12303 14101 18915 21676 21602 35943  7654 45095 46619
      20039 35300
1120:25662 40052 21700 23846 35558 38886 12917 21941 51776  3867 10951  9613
      22927 13834 14697 13991 25900  7835 45022  8396 34671 26017 18503 35158
      26758 36346 46740 23458 49049 46448 28611 38746 39736 44860 30895 18639
      36005 36430 20658 12046 31447 51939 40338 48729 31855 39015 42596 17440
      11499 24512 46946 25569 13433 13432 24728 43525 20276 20292  5439 12292
      34308 39710 34310  8667 34860 45164 21405 31657 23418 16882 52222 20731
      38500 38116  1608 14788 26973  5414 26976  4651  2115 20915 36803 36806
      22623 24145 50898  4889 10375 49645 23362 16414 21295 48803 48830 44094
      15738 43950
1121:25662 40052 21700 35558 23846 31513 31515 12917 17030 21941 17170 22129
      51776 10951 51115 13834 25900 19263  4350 45022 52699 21525 34671 26017
      43185 29280 33046 36346 46740 38140 23458 31141 20442  2343 49049 46870
      46448 28611 39736 30895 10297 18639 28406  4773 28258  9076 35329 26757
      42128  2417 51939 42444 40338 31855 39015 42596 17440 24512 46946 25569
      13432 13433 24728 43525 20276 20292  5439 34308 39710 34860 45164 19413
      26249 45021 52222 36030 37734 20915 36803 36806 22623 17128  2793 10375
       5380 49645 30525 23362 16414 21295 48830 48803 44100 15738 15853 10069


1122:30890 25020 48589 26688 18396 33944
1123:43960 23364 38570 22221  9981 26992  3988 22667  3537  4981 34993 31290
       5476 11990 14025 43256 19222 48527  1399 50495 38037 14208  9629 41220
      43402 39798  1124 50901  7632 41037 11902 51337  1389  9754  2549
1124:23364 33435  9981 26992 18138 10447  2764  3988 34864 43433 22667 13019
       4981 31290  5476 34993 11990 14025 43256 19222 48527  1399 50495 38037
      14208 20752  1123 34924 41220  9629 43402 39798 50901 19348  7632 41037
      11902  1389 51337 48607 30630  9754 45471
1125: 7828 14960 16320  1939 42985  2958 12474  6924  2916 39590 32872 40875
      23081 28548 33047  8860 24344 41396 31884 41918 28530 13485  4165 39121
      13330 26261 27226 12852  6941 46369 43868 13082 20156 13644 21181 40900
      40905 25410 24262 11189  7292 16722  5090 49872 50973 42230 23208 18845
      10920  3696  8184 39107 24826 23352  9199 50240 15993 22842 22845 44102
      25213  8627  8648  8651 43440  1567 17300 27817  3609  6863 47345 32015
      29422 11879 46261 46257 44825 46259 24747 44758  3378 13984 15486 35488
      50313  3405 30998 32009  7402 15121 24413 33714 23711 24693 42569 25199
       6227  3760
1126:45660 52313 17028 21798  3238 46649 19490  5709 13118 30021 52529 36279
      42352 39414 28253 44480 25723 50864 15573 15602  1708 50268 14125 10211
      38270 16410 12142  9050 19384 40757  7279 33042 50850 45438 15283 48178
       4260 12027 44878 12259  4167 47062 21414  7157 14889  7234 49238 45063
      18287  1545 48778 19326 42311 38839 49026 38912 27873 24673 22249 12873
      43119 48262 52509 33257  5763  9195 11061  5627 52545 29469  9114  7508
      51466 46925 51037 50899 43709 23232  9990 30504 11431  7507  3027 47935
      38242  9936 15167 15500 29214 34921 21318  4003 25153 50873 35117 25664
      39694 31665 22504 23629  6609 38987 39240 29560 43720 19645 51585 23829
      13637 20474 18927 27321 32877  9123 32975 44519 50332 20449 14966 19468
      14094 27065 39150 12440 30153 40411 36687  9158  7376 12393 49719 13458
       7134  4334  7780 49963  3290  1576 25986 25624 12456 33200 22366  8715
      19730 15729 11530 29583 31150  2152 13401 11744 36722 32864 24905 24690
      19757  4911 27958 51909 50660 35576 47614 49610 44510 22731 12701 26635
```

```
       7755   6857  47224   6767   8551  14847  13078  34578  29888  30267  43093  28743
       9572
1127:13695  38570  49410   3335  44027  11585   3988  34864  43433  22667   3537   4981
      49498  36614  23237  16190  50139  39798  22970  34243  43572   9754   2549  12058

1128: 4981  21792  25124   6666  50337  26241  47941  20901   7888  30010  39607  21282
      51089  38754  13730   3873  38755  43402  43537  49991  15856
1129: 5239  23045  36320  14864   8299  50382  11986  28028  11433  41363  42378  15782
       7203  27620  23917   8812  18404   3706   9859  25257  39081  25100  13047   9624
      10023   1446  25595   9047  34007  33003   1159  45281  14998   6615  13566  34551
      44761  20526  32530  37924   6814  10461  23815  21987  10610  17751  13224   3222
       4027  12472  20234  16966  11163  47126  33264  15553  45993  48417  23105  45734
      50743  31535
1130:23522  27866  36817  17747  11964  43728  12136  12915  44801  17624  35096  17436
      21983   8696  43551  15022   8011   6111  20077   2428   2353  12672   7593  51351
       8896  24226  19851   8121  44297  40705   4875  17984  26772   1548  36668   3585
      29519  28716  50737  40762  38207  52208  36092  23102  17649  47889  28835  17464
      29574   6195  13860  35843  21953   7794  39851   2359  44284  47587  39756  49061
      15724  44465  38720  48840  27158  12655  42154  50241  28780  26001   5600  50082
      41028  50556  25247  40154   4727  15027   4697   3136   7622  20945  33415  48118
      22239  26753  20160  34109  26888  17487  38874  38260  11313  30046  25934  49616
      34590  28666  25943  14531  15475   4312  22499  27181  20143  47595  14405   3731
      41479   9915  50844  31605  11510  43310  26611  11712  46944  49336  37347  21191
      45177   9858  12535  38557   1637  26946  15139  30022   7253  23490  48142   8592
       5299  48834  47979  19278  11196   9273  44449   9567  50839  10854  51315   9596
      36053   7942  33768  41047  27654   5641  23218  17676  26666  33557  35766   2427
       6955  45739  14837  11206  24304  42744  27740  24586  30047  45857  24603  37107
      14123  17261  39961  29934  22630  25386   4079  25118   9841  52191  18120  33851
      19996  29395  45916  17228  12809  38229  47841  45443  27713  29706  51625  22793
      25197  20960  44473  38317   8985  19072  48489  12658  25115  44798   7700  48925
       8388   3520  28613  26504  39891  24904  52241   6398  45460  30289  52160  48430
      26804  47551  35751  38070  19625   3982   4138  37903  27727  36097  28058  15059
      25218  40704  14414  40415  47884  10209  42195  44673  14851  21622
1131:32602  17087  11707   4389  12698   2695  25167   9193  10258  39457  39531  43317
      33891  18131  11287  32836  43676  35280   5355  32626
1132:51848  40164  12600  45348  10909  51110  26155  26156  18604  24879  42471  24782
       1528   8580  14691  46527  28422  28443  28445  37812  22802  46003   1271   5406
      18238  12715  38990  16133  40918  34516   5747  49656   9397  12557  38335   5403
       9802  22234   9419  45350  17080  48541  48542  45347  48254  22326  35553  35581
      35579
1133:51848  40164  12600  45348  10909  51110  26155  26156  18604  24879  42471  24782
       1528   8580  14691  46527  28422  28443  28445  37812  22802  46003   1271   5406
      18238  12715  38990  16133  40918  34516   5747  49656   9397  12557  38335   5403
       9802  22234   9419  45350  17080  48541  48542  45347  48254  22326  35553  35581
      35579
1134:44340  49273  23735  36949  22676  42818   9644  52554  31754  15530  41682  35546
      35951  49638  30494  47839  13165  31283  29788  34049   9766  14602  26795   9229
      35433  40003  32895   4227  50628  21554  35799  19715  48042  19935  43913  10827
      25912  34512  11643  30216  33619  27035  33567  45249  16120   1487  32243  26556
      13953   8165  33568  40674  31328  17131  37230  34102   9220  28706   4484  36162
      24570  49160  40258  24349  48377  10016  10509  44304  44603  51697   7786  45055
      34252   3922  36520  20751  25190  27987  14770  38536  39973  41872  26107  52327
      40885  14643  14634  40119  43167  41346  25657  27768  32034   3103  35131  21005
      32589  13068  38177  37838  37372  12330   2868  33160  27422  49258  31658  41055
      49137  44959   8706  38189  19561  16828  45230  42342  19470  27010  26094  38057
      10514  37887  43658  27539   4059  52658  52632  34732   4033   8743   6338  20542
      48159  30159  27853   9875  18885   9827  13318  24163  33201  31978  37681  40895
      20954  46161  40233  36442  27000   8128  44466  52175  20367   1753  15915  45377
      14973  22058  11134  27894  31971  10639  15720  30778  50420  25508  45450  40427
      27005  47019  46995  47016   1572  29210  47150  49487  51303  15182  47342   6603
      15580  43752  32812  50419  39129  38604  34107  29713  16129  13671  43473  13496
      26581  21285  21686  49876  49926  13544  50036  45472  25852  18663  45787  11393
```

```
      6309 21567 20299  1760 13459 36083 24032 51758 13447  9184 25443  6482
     50779 27233 15043 33320 19417 11065 35106 33860 11538 19191 24300  5152
      6422 10684 20320 20481  3889 25999 31911 15226  7750 48677 25058 43076
     52096  5142 44166 26787 16505 34218 49766 35606  7192 38449 46313 46398
     17163 14026  8726 37062 44504
1135:15813 13063 46366 46979 46408 34327 23328  8422 33809  7503 35946 41494
      5451  9221 15606  1425  1424 20245  1803 23513 52253  3429 51307 51461
     45300 25726 23605 14137 41175 44452 37395 20206 50907 24910  1887  9065
     49555  9376 10638 34643  4916 23373 46135 44548  7284 38283 20224 52666
     31933 15447 46235 14491 49007 47453 34287 32682 35447 12546 44563 29521
     36759 22999 16951 32911 38546 20046  7226 33456 11566 10812 30461 14603
     12949 41044 13799 35862 44969 16238 47508 17873 33307  9155 25425 21673
     31748 32634 12759 13661 30735 27893 38426 38324 37748 29642 11095 41415
     11743 52067 27318 48779  5037 20264  4804 40510  1521 17515 50923 27290
     47660  4413 50636 14933 43447 40821 17567 41690 51694 19639 28030 33230
     51478  5254 31066 14662  3534 35887  2112 10833 44096 47089 39339 48866
     23927 23774 51020 11685 27140 26116  4066 35439 40033  3549 22672  8687
     52584 37640 32880 32179 38352 42317 38902 16879 17827 32377 39655 23864
     22952 25317 35650 17891 29722 38847 43696 49306 52107 25179  2310 17601
     36580 39740 23878 47678 32463 45266 29319 33183  4037 41807  5669 11226
     18570 20543 12727 18853 11655  1527 19985 28864 18964 35344 23542 15005
      7088 46681 38969 25940  6430 34321  5660  2980 37670  2081 52466 39867
     10830 38215 29139 34848 40230 38788  7570 20131 42644 44458 20107 11747
     11684 23095 19145 47503 26944 24236  4218  4452 11855 35908 33467  6942
     39939 10960 23240 22697 26189 24554 43530  7553 50377  2944 48291 11195
     28550 16421 10256 26274 49831 46626 47321 48128 35342 30083 20254 46737
     42453 32272  2682 36660 29657 16987 33923 48796 43542 35730 31801 15988
     44131 44106  7546 15261 28723 35163 11360 51383 15474 34273 10891  4634
     38560 38903 16217  4485 34220 25265 15661 51678 36196 29390 27795 26029
     36992  3779  7141 19776 48005 20924 11791 29463  4281  4381  4905 37028
     32496 18252 45173 15282  3832 24459 12651 12453 23736 38803  1673 43436
     26875 30319 38774 19446 24862 26678 40247 45760 34571 38286 20934 26835
     50233 20668 27680 50649 18733 34575 15260 50147 15565 39553 37490 36810
     25221 28935 31727 13966 38073 16748 46008 44808 39640  3519 23723 23721
     27720
1136:15813 13063 46979 46366 46408 34327 33809 35946  5451  9221  1425  1424
     11401 46578  1803 52253  3429 45300 51461 25726 23605 14137 44452 37395
     20206 50907  1887 49555  9065 34643  4916 46135 23373 40793 20224 31933
     15447 14491 49007 47453 34287 32682 35447 12546 36759 22999 16951 32913
     32911 38546 20046 33456  7226 27113 10812 30461 14826 12949 44969 16238
     47508  8958 25425 32634 31748 13661 12759 30735 27893 30866 29642 11095
     52067 48779  4804 40510  1521 50923 50825 47660  4413 50636 36193 43447
     40821 41690 51694 19639 20461  5254 33230 28030 20626 51478 31066 47089
     39339 23927 23774 51020  4066 35439 40033  3549  8687 37640 34219 32880
     32179 42317 17827 39655 23864 25317 35650 17891 29722 38847 49306 52107
     25179 17601 36580 39740 23878 32463 45266  4037 11226 18570 20543 18853
     11655 19985 28864 18964 35344 23542 15005 38969  7088  6430 34321  2980
     37670  5660 52466 39867 46505 10830 38215  4674 34848 40230 38788 42644
     44458 20107 11747 11684 47503 19145 26944  4218 11855 35908  6942 39939
     10960 22697 26189 24554 50377  2944 48291 11195 10256 26274 49831 46626
     48128 35342 30083 20254 32272 36660 35981 16987 43542 35730 46955  7546
     15261 28723 35163 11360 51383 15474 11108 27439 25265 15661 51678 25797
     45365 36196 41302 29390 36992 40102 20924 29463 36163 32605 21167 38992
     13522 12651 25407 36043 12453 23736 38803  1673 26875 43436 30319 38774
     19446 24862 26678 40247 34571 38286 20934 26835 50233 27680 50649 18733
     34575 15260 50147 15565 39553 37490 25221 31727 38073 46008 39640  3519
     23723 23721 27720 31432 22131 35132
1137:14725 52201 10011 16466 47640  1910 30768 30619  7921 25334 30733 12564
     14818 28409 24167 27172 40599 14005
1138: 3360 42349  1430 45596 47575  7162 19228 10014  5726 45051 37529  1429
     12100 43713 25764
1139: 3363  3360 14158 14180 42349 27049 15739 45838  3822 47575 52243 22584
```

```
        2678 43229 12100  1166
1140:33253 20760 25298 36125 36129 36130 15697 21361 37677 37707 33410 33485
     34288 33555 36048 36051 29683 22133 13055 43874  3373  3375  9472 28924
     23730 27907 36127 16834 10229 36105 10216 45091 28669 45595 26752 21767
     51856  8489 26654 41014  7792  8206 17055 40643 11202 17383 42346 29666
     24048 31580 17749 20758 10640 25419 33263 36019 36081 36082 40315  9381
      9380 36176 36074 36190 36078 48723 18043 18044 42590 16111 23196 35380
     25099 37248 48130 46552 12522 36253 11769 50983  8202 36103 46555 26110
     46553 26108 51693 40330 30926 45633 14396 17979 38796 23291 51118 36148
     27562  6625  6629 34112 22730 32023 26103 26105 37001 36042 23177 50152
     39643 15801 30460 29629  2726 34883 34885 15960 10871 43468 15954 27941
      3636 43999 36168 36169 31137 52733 31907 26209 17086 20718 13415 38543
     41903 25286 31521 14245 41994 18351 51287  5289 52478 34305  2494 32359
      1934 32863  2887 27385 28509 13973  7074 17320  5246 31073 41717  2821
     20470  1190 14521 46631 19257 29381  6773  7604 18381 39584  3458  7758
      3781  8269 19699 35077 36107 36104 49585 23199 36110 36171 36178 36055
     36933 38970 23200 36294 32687 32640  4904 30817 41344
1141:33253 20760 25298 36125 36129 36130 15697 21361 37677 37707 33410 33485
     34288 33555 36048 36051 29683 22133 13055 43874  3373  3375  9472 28924
     23730 27907 36127 16834 10229 36105 10216 45091 28669 45595 26752 21767
     51856  8489 26654 41014  7792  8206 17055 40643 11202 17383 42346 29666
     24048 31580 17749 20758 10640 25419 33263 36019 36081 36082 40315  9381
      9380 36176 36074 36190 36078 48723 18043 18044 42590 16111 23196 35380
     25099 37248 48130 46552 12522 36253 11769 50983  8202 36103 46555 26110
     46553 26108 51693 40330 30926 45633 14396 17979 38796 23291 51118 36148
     27562  6625  6629 34112 22730 32023 26103 26105 37001 36042 23177 50152
     39643 15801 30460 29629  2726 34883 34885 15960 10871 43468 15954 27941
      3636 43999 36168 36169 31137 52733 31907 26209 17086 20718 13415 38543
     41903 25286 31521 14245 41994 18351 51287  5289 52478 34305  2494 32359
      1934 32863  2887 27385 28509 13973  7074 17320  5246 31073 41717  2821
     20470  1190 14521 46631 19257 29381  6773  7604 18381 39584  3458  7758
      3781  8269 19699 35077 36107 36104 49585 23199 36110 36171 36178 36055
     36933 38970 23200 36294 32687 32640  4904 30817 41344
1142:18514  9536 49681 49682 49659 49657 15798 39833  8000  8005  8052 30738
     49144 49140 51338 51308 44263 17897 15462 14735 46855 32543 28026 36976
     24499 14741 14774 15464 44702 33409  8356 29977 44107 50696 11931 23046
     46411 41669 32709 13537  7114 45920 45924 49438 41730 34594 45287 14580
     14578 13631  4477 45972 32385 31575 20317 32675 41631 52558 51176 24549
     23926 16650 49037 45932 45927 45968 45946 45881 25185 11495  2159 26852
     26692 45614 26438 45970 20829 28581 22290 13478 20331  3776 38282 29623
     44765 43654 41600 30043 19127 26658  2841  3624 52368 14529 15020 36907
     15465 14744 14776 14779 20384 49865 49837 21473 21633 21744 38183 33236
     33222 33221 33218 14458 26683
1143:23780 34037  4859 23623  2801  2730 28619 16008 11767  3177 18641 24457
     18640 19637 47797
1144:45345 23527 13315 21725 31406 39140 36977 10077 34208  8870 24224 35371
     11925  5909  5931 46879 22120 21019  3218 12939 10262 43358 52422 21956
     16958 31602 28539 28945 16719 22916 47176 48212 17880  8658 16593 35512
      4568 47107  2510 14343 26214 25890  9831 40830 13130 50731  9975 34596
      4877 23868 51136  3168 45879 39949 33346 20676 42561 15263  9445 25113
     51972 22503 34378  9238 15919 11172 29299 21430 44743 19290 20983 46002
     35628 52270 51159 39441 18345 13660 34059 16014 26419 37392 28573  1145
     43165  4938 21498 26033
1145:45345 13348 25267 23527 13315 39140 30548 37884 24708 10077  8870 24224
     11925 46879 51631 22120 29040 21019  3218 12939 10262 43358 52422 21956
     28945 16719  2053 47176 17880 48212  8658 35512 47107  2510 10680 26214
     14343 50731  9975  4877 23868 51136  3168 45879 39949 20676 42561 15263
      9445 25113 51972 22503  9238 29299 15919 11172 21430 44743 51751 32972
     22796 14213  6929 31809 32791 27511  9840 25653 20983 46002 35628 52270
     51159 39441 18345 13660 34059 35098 49796 26419 28573  1144 36450 43165
     21498  4938 26033
1146:38769 45068 52628   814 14468 16843 18728  1831 49200 40013 49242 15913
```

```
      12369  1221 17490 29903  4307 28182 17417  6122  4517 42481
1147:29858 28235 36352 37920 39241 35873 24899 46236  8586  9750 20168 28336
      30177 14904  1445 22444 35264 17121 35949 29250  6772 46218 14424 22022
      48436 37729 16018  9396 32650  1223 28799 45124 47749 47866 26985  6989
      13236 21663 22372  5803
1148:37240  8314 36403 28670 36016  2672 38192 35121  8046 16633 27597  8348
      35104 25728 27497 46667  4181  4222 14748 44694 44061  4104 40856 34259
      20701 22991 29959 43891 40236 46294 49041 17961 32307 27224  7978 30163
      20714 48516 47265 48471 27921 40133 18309 24715 46871 18053 15910 45666
      28872 50005 49933 31558 26698 42804 50963 49235 36969 43934 24397  3719
      42957 10103 22634 13157 40103  8460 28758 13128 38445  7563 28068 20826
      44109  2520  8733 31960 44395 44393 26170 43621  3074  6505 50049 43553
      49606 20788 52355 39853 16952 23999 27034 49318 29586 26083 42748 40115
      40542 30629 12135 50279 49389  7744 17926 15800  2008 24208 28785 47088
      22066 13794  6144 41928  8260 13456 15818 48822 43693 45864  8360 29798
      51375 46106 51710 16818 32567 47249 19343 42094 19560 20109  6977  2892
       3530 21491 48059 43681 43679 36166  9033 17690 32548 30111 40086 33538
      21385 22441 15374 10973 38927 12179 21517 28276 11768  8790  1779 15504
       1438 32177 16621 29665 11428 21997  5474 48498 45368 13288 42025 45252
       8437 34494 34520 13275 17795 39170 33384 33883 23123 24340 14444 27543
      32804 42970  4129 36785 32847 42345  2005 50006 14901 17717 13927
1149:30787 29743 29744  4184 13784 36404
1150:46301  9839 24511 31205  4559 50684 26258 21060  3785 20806 39774 23192
      37016 44425 14790 43822
1151: 1441 36930 51621 13030 15427 11457  7188 14986
1152:52129 38346  9093 13366 27354 47489 32202 41025 40433 42018 13249 43637
      49901 20075 16666 20749 38343  9200 33014 10425  2059 37155 20767  9407
      42890  5896 10346  5757 45652 27450  3588 48999 40504  8618 20746 46692
      20740 16599 37357 20972 45424 47387  7735 23617 23830 24562 36161 49287
      30538 40931  7270 48050 40114 27498 14109 43115  7624 26732 44019 48520
       5878 32248 38035 21220 21057  9149 50721 25509 52230 19994 28949 30633
      46586 48376 16146 32277 38321 46756 27232 30065 47852 37414  8341 34207
       7537 22335 50385 15145  7256 25555 45868  5699 34743  4489 40187 21244
      26513 44142 19519  1790  9143 44015 20791 34534 32833 43806 16261  9310
      17502 50755 39143 25840  3720  3876  8302 16946 19392 44029 43712 19923
      29739 39228 48160 17830  3365  3578 19314 15449 24875 18154 21579 18682
       6843  7536 51164 37112 33233  9060 15393 10774  8584 14133  1325 33112
      15330  4551  5343 38363 38360 37952 18620 45171 16713 37999 23457 15106
      41153 46968 50296  2185 31636 11337 34522  7360 11825 20952 52405  3502
      16701  1542 38736 24982 13228 12350  4528 41110 52525 10685 43725 14840
       9691 42509
1153:50396 45856 13379 35448  3901 26800 48975 36416  6637 22788 18774 49743
      47751  4505  4503 48659 48680 17301 48660  8071  3868
1154:38842 12222  7749 36926  1693 13214 23703 37962 45338 41996 28170 15296
      38571 18236 14367  5021  5022 16411 17802 23305 41410  3075 39772 26953
      18851 42028  7358 21546 38117 10546  7330 32352  2224  2222 51195 14490
      51226  2711 29223 23652 47483 29548 29158 42178 15000 51222 31191 51899
      45364 46977 35574 21511  7347 23552 27328  2200 16589 19404 38584 31060
      45598 11445 34894 52131 40008 43643 47988 43363  2296 42012  1565 32236
       9485  5154  7295 46742 45969  8339 40663 51266  8553 12966 35502 40344
      11171 49105 51263  4753 49956 20910 51388 46455 25181 23597 37270 38403
      26495  9702 38039 52474 17834  6656 24436 46173 33777 17294 43316 24081
       6288 21009 27190 45744 33511 29725 14074 33378 26365 10852 44160 23651
      16932 43061 38131 49104 48246 27585 28434 17582 17987  2147 46462 40364
      32810 35516 12925  8913 34942  5540  5801 32527 24961  8732 20190 35858
      41278 20885 15481 11009 35610 10369 46814 46597 34558 33095 10426 41335
      33295 23276 43768 19977  6586 21529 33259 51612  9787 39855 27264 34294
      12154 48164 21162 42565  6655  9732 33169 16192 13353 33630  9194 42091
      14212  2465 43046  4024 19529  6720 47511  7382  6327 17655  5296 17147
      10178  4756 47309 34633 50359 35592  9086 40797 22756 43584 40624 11907
      35639 41021 17788 26364 24834 29797 31377 42575 47661 20382 24804 25231
      26122 24307  9032  6844 43456 19070 15859  8312 12521 32641 37256 45670
```

```
      20177 19154  1544 52118 52166 37452 48590 47556 43207 39418 33773 23688
      30157 51312 48322 49058 50177 23062 36584  9731 49077 45681 44355 31288
      24947  8751 44245 20713 18074  1694 32618 46506 20401 16085 50298 46361
      34601 24963 22870  3464 30990 32345  4547  4546 45954 21003  6639 16905
      46980 28077 41856 16144 51009  1754 43981 15714 22456 37254 41135 46971
      19833 42571 30986 20597 17177  9014 51261 23618 51257 15385 15386 51219
      15080 45688 26643 44552 33178 37961 31689 25811 46770 42159 21291 43754
      35361 10688 23462  6070 33060 23639  8749 17098 14766 34441 30269 43607
       1155 26521 48560 51220 38952 42136 27280 29038 42862 25444 14981 47907
      22978 51265  5541 35407 26945  2209 27449 41083 51897  1697  9269 29996
      36208 50578 47674 51194 27170 27129  3341 50916 42275  9878 51224 24737
      26345 37633 51579  5047 19339 38568 24360 50429 12756 51900 23700 27349
      33793 47266  8078 19380 14783 17358 43996 22130 46913 46544
1155:38842 12222  7749 36926  1693 13214 23703 37962 45338 41996 28170 15296
      38571 18236 14367  5021  5022 16411 17802 23305 41410  3075 39772 26953
      18851 42028  7358 21546 38117 10546  7330 32352  2224  2222 51195 14490
      51226  2711 29223 23652 47483 29548 29158 42178 15000 51222 31191 51899
      45364 46977 35574 21511  7347 23552 27328  2200 16589 19404 38584 31060
      45598 11445 34894 52131 40008 43643 47988 43363  2296 42012  1565 32236
       9485  5154  7295 46742 45969  8339 40663 51266  8553 12966 35502 40344
      11171 49105 51263  4753 49956 20910 51388 46455 25181 23597 37270 38403
      26495  9702 38039 52474 17834  6656 24436 46173 33777 17294 43316 24081
       6288 21009 27190 45744 33511 29725 14074 33378 26365 10852 44160 23651
      16932 43061 38131 49104 48246 27585 28434 17582 17987  2147 46462 40364
      32810 35516 12925  8913 34942  5540  5801 32527 24961  8732 20190 35858
      41278 20885 15481 11009 35610 10369 46814 46597 34558 33095 10426 41335
      33295 23276 43768 19977  6586 21529 33259 51612  9787 39855 27264 34294
      12154 48164 21162 42565  6655  9732 33169 16192 13353 33630  9194 42091
      14212  2465 43046  4024 19529  6720 47511  7382  6327 17655  5296 17147
      10178  4756 47309 34633 50359 35592  9086 40797 22756 43584 40624 11907
      35639 41021 17788 26364 24834 29797 31377 42575 47661 20382 24804 25231
      26122 24307  6844 43456 19070 15859  8312 12521 32641 37256 45670 20177
      19154  1544 52118 52166 37452 48590 47556 43207 39418 33773 23688 30157
      51312 48322 49058 50177 23062 36584  9731 49077 45681 44355 31288 24947
       8751 44245 20713 18074  1694 32618 46506 20401 16085 50298 46361 34601
      24963 22870  3464 30990 32345  4547  4546 45954 21003  6639 16905 46980
      28077 41856 16144 51009  1754 43981 15714 22456 37254 41135 46971 19833
      42571 30986 20597 17177  9014 51261 23618 51257 15385 15386 51219 15080
      45688 26643 44552 33178 31689 37961 25811 46770 21291 43754 10688 23462
       6070 33060 23639 14766  1154  5617 46913 34441 30269 43607 51220 38952
      42136 27280 29038 42862 25444 14981 47907 22978 51265  5541 35407  2209
      27449 41083 51897  1697  9269 29996 36208 50578 47674 51194 27170 27129
       3341 50916 42275  9878 51224 24737 26345 37633 51579  5047 19339 38568
      24360 50429 12756 51900 23700 27349 33793 47266  8078 19380 14783 17358
      43996 22130 48560
1156:45876 32058 15216 17153 52488 10201 47328 15978 11180 43739 48524 47695
      12763  3451  8372
1157:18890 19612 47356 17004 21113 23498 24197 36488 36276  1947 18793 22645
      25645 49770 14681 36643 20761 52408  1444 17671 43608 37613 31190 19302
      11526 26237  8868 44710 37464 46707 14743 39877 47099 36198  1013 13948
      43180  6874 34658  6845  3004 26849 10060 20501 17952 49165  8764
1158:16572 40700 26763  5101 37029 32242 37499 39162 17872 20953 45397 11640
      49361 22705 15334 50519 22560 51889 18428 17053 14547 37111 10027 47515
      13925 31927 18581 16034 19800 38175 30612 49000 31451 18192 18194 17662
      14024  9259 13886 26608 23795  5295 24605 28210 23890 39680 17506 32128
      43479 50300  7746 50520 37713 23307 31839 17733
1159: 5235 24744 36320  8299 26232 14864 50382 11986 30510 47037  1724 11433
      41363 42378 19196 21935 15782  2473 27620 37326  7203 42264 23917  8812
      18404 16702  3706 44884 44149  9859 25257 39081 25100  9624 13566 34551
      44761 20526 49800 37924  6814 10461 23815 24878  1129 38019  7118 34387
      21987 46230 17751 10610 13224 32473 15796 11370  3222 12472 20234 11163
      47126 33264 51590 34686 35977 15553 10697 45993 48417 48439 23105 45734
```

```
      50743 31535 33003 14998  1446 25595  9047 34007
1160:47143 24150 22003 39097 39660 43980 15669 11125  2223 14801 34100 34095
      34091 34099 27287 34085 30350 43300 23449 28927  3554  2720 44776 24822
      52635  4849 22151 12054  9538 22611 28451
1161:47143 24150 22003 39097 39660 43980 15669 11125  2223  7832 34095 34100
      34091 27287 34099 34085 48360 31781 12033 43263 30838 41192 23449 44132
      11044 33896 45901 24008  2720 13639  5121  9538 22611 28451 47242
1162:11586 11631 39778 45850 29423 20494 25834 47819 20561 18664 10025 27841
       9795 45292 45310 45293 50640 50609 50610 37041 46088 46089 11594 52502
      13342 11650 26824  8785 47908 30973 34382  2380 10733 13663 29109 35160
      38961 22358 46092 46093 52318 27057 10145
1163:11586 11631 40490 39778 45850  6936 21382 48969  2813  4292 29423 20494
       5852 25834 14356 27351 44954 33985  1165 14678 13022 45292 45310 45293
      50609 50610 47132 48425 52324 52349 52321 46088 11594 46089 34328 13340
      11650 49705 37289 50967 31132 30432 42574 45290 46884 30999 39515 38961
      23787 22358 46092 46093 52318 27057 38960 19051
1164:11586 11631 40490 39778 45850  6936 21382 48969  2813  4292 29423 20494
       5852 25834 14356 27351 44954 33985  1165 14678 13022 45292 45310 45293
      50609 50610 47132 48425 52324 52349 52321 46088 11594 46089 34328 13340
      11650 49705 37289 50967 31132 30432 42574 45290 46884 30999 39515 38961
      23787 22358 46092 46093 52318 27057 38960 19051
1165:11586 11631 11630 11629 39778 45850 29423 20494 14356 25834 13022  1164
       1163 26517 19051 45292 45310 45293 50609 50610 46088 11594 46089 13340
      11650 37289 16774 34142 50967 31132 43967 35435  9752 27541 38961 22358
      46092 46093 52318 27057 38960
1166: 3363  3360 14158 14180 42349 27049 15739 45838  3822 47575 52243 22584
       2678 43229 12100  1139 18637
1167:24981 10342 10997 25007 24977 25003 24976  9573 40750 40747  9600  4543
      13604 43405 22236  9616 20805  5147  9562  9578  9581  9564 35804 33497
      21313  2575 30803 26550 43511 43660 17735 41572 16429 25132 51691 10584
      23537 37162 50469 45441 24722 45906 37393 49985 30462 47011 47294 51444
      42221 22582 47648  3977 50896 13835 33477 27033 30170 28831  3271  6526
      41066 36191 46579 28192 25473  9860 22315 14987 27069 31942 36369 43714
      40104 51983 20009 42386 11656 28311 13475 44476 20023  1510 42262 19153
      17966 42941 48217 43641 43666 18383 51045 24770 21300 18562 36626 50532
      18451 33743 23663 47755 10606 17293 43182 30143  4105 31872 39885 15658
      20802 43049 35053 51091 24372 11612 28212 48614 12359 32481 49390 20710
      12333 43247 26385 40839 20472  9580 49059 21495 51199 22241 45019 41899
      41881 19440  5176 48337 23533 51358 51426 44500 47596 10822 33196 17868
      51614 17928 17538 39031 13441 24191 38388 37219 19059 30331 23289 47669
      36070  2875  6171 27448 35185 20087 20091 43030 26141 22877 19083  6913
      50502 39976 39978 14990 12230 26154 22726 16413  9555 13513 45822 11821
      27491  2512 35776  9560 24079 37100 26428 40952 40948 40951 48183 30532
      22232 43638 22238  9583 28018 28017 27793 28019 28036 35577 25847 50414
       9558 45190 27580 14453 43189 46784
1168:32714 30485 21745 48705 45248 13705 24392 35301 47470 19635 52561 46582
      50747 24578 35822 10439  7947 17761 34205 20061 20034 22836 26194 24023
      39730 27248 26970 26890 10477 12212 17501 17033 29720 31213 11951 45499
      12987 11177 29190 32857 40735  8140 13137 26785 10374 17995 43970 17242
      20567 43810  9470 46922 32187 19209 12705 31079 42196 22368 17884  4535
      52128 21398 21395 27022 27040 18439  3178 28684 30210 42892 22741 30214
      18082 17465 18081 18086 32106 32079 32101 32073 46924 18090 32069 32071
      37855 46421 45005 10047 36681 24002 24000 11230 31049 43578 40213 34329
      51653 21857 42108 42107 35400 35396 37511 35398   781 43306 17085 41852
      45374 49599 16322 10189  9001  9003 48888 22581 48623 44450 21721  2287

1169:26848 15489  2681  6962  4368 11182 42850 46260 23314 47649 43647 17682
      12670 13424  7173 23625 19767 37199 39180 49324 49530 24192  7924  1699
      28873 47909 41148  6180 11928 40988 37534 42461 10637  8032 21182 17858
      38399 19954 12511 46066 31677 35911  3477 31749 32617  2627 34268 27294
      27292 27596 48737 33231 30088 50054 36575  7920  7510  7543 33312 43827
      43866 48388  2472  3097  2653 21799 47305 10572 34002 14545 40577 31627
```

EP 2 484 769 A2

```
24760 39160 41166 31242  9297 31326 25661 28188 43757  8755  7014 27805
23534 38824 26855 26401  9741  7151  8950 40801 11593 33981 47275  4214
52277 34959  6027 22712 47450 15754 25949 46319 42192 35056  1737  4144
 4280 12286 33524 52054 25897 46650 20682 32405 14557 29774 43558 39667
27116 48902 50095  2233 20146 40259 18369 14193 40617 13218 19605  7810
27888  2232 27929  6521 42834 52676 49466 12347  2183 49938 27123 12315
25012 33300 40920 15974 51834 41997 32993 11397 21229 46441  7958 34276
21599 51212 10330 21503 32948 49380 17729 44352 48632 29351 46206 43663
32417 11952 11981 43438 34366 27286 29524 22743 25600 31488 12252 37300
18016  7853  4923 43190 50218 29792 44309 31760 52597 48329 43359 10644
35844 19557  2978 32934  5970  6101 31493  5093 48914 41115 40847 33072
47364 35462 29582 48165  1874 19007 40852 10088  6536 24318 28416  4387
17096 41096 52309 13328 34703  3752  9828 38273 17269 14412 44618 47057
42731 12071 48256 46203 11680 29333 21687  7049  4495 38136 40135 16902
46996 49545 18764 40958 23212 29950 12619 37844 37282 40239 42916 11057
35063 29321  5221 24091 38967 20334  3544 30211 30238 51398 40751 16860
33214 51646 14672 45795 41987  5214  6743 12043 36863 49045 15511  4784
30081 45705 47652 16307 28955 14456 16278 12570 46819 25907 17838 23930
46182 22288 28974 29262 34653 27453 27460  7445  9857 19274 14756  6348
47985 33179 33991 31021 14179 15064 13356 40044 10338 44056 39198 10726
18188 45498 45524 49741 31871 36102  8638 34225 22096  6775 11297 37810
44940 39865 31044 32264 36174 12573 19138 29009 12095 17531 41231 42828
42831 42829 23403 42425 24108  3314  9618  9606 16551 23009 23002   770
 5278 39295 12299 25643 29359  1634 11914 10215 41383 14865 51253  2468
13653 37522 27599 27872 33831 34105 24755 23086 32301 19766  9966 13951
18636  4208 25898 11647
1170:39424 39202 31006 16775 15498 10941 10940 10943 39497 16248 15485 44004
39465 40166 23130 23110 23106 23133 23113 15389 23245 20888 36628 15315
24692 28991 20753 40574 24470 24990 24941 24924 24944 34786 34805 34782
34869 34868 25075 25072 35483 25018 35480 24995 24998 46197 43906 43900
31666 40406 40407 15487 34598 37888 16720 16771 17404 12859 17079 47134
31699 31703  9806  2361  2357  2338  9817  9830 31566 40207  3772 35216
40910 44416  2123 15239  3843 10532  3040 43951 43956 43880 43930 45640
10235 15521 15388 15514 15460 15457 44856 44040 44794 44802 44041 44861
44044 44898 39036 15516 15317 15392  2812  2199  2880  2840  2901  2863
 3009  2132  2972  2168  2991 45643  9108 22258  2365 35854 19774 46334
46338 31810 31812 31805 31806 31791 35971 44899 44678 44675 44006 40202
38906 34741 25038 24972 24991 24968 24970 15463 15571 31245 31785 31786
23210 47924 10127 45991 42696 36445 39502 40161 40163 22112 46602  3362
17512 51778  5796 21991 43248  2335  2328  2330  2645  6712 38512 27252
34146  7611 15456 15529 16243 16245 15353 15421 15423 15461 15480 16263
44585 37566 20538 49827 16747 23779 48967 33436 26805  6607  4622 10239
10237 40206 31764 31755 31756 24697 18241 26792 14587 20907 18235 44288
 9099 22578 48465 47860 18208 47807 18210 47802 47827 13738 49174  7196
34615 51986 24946 25063 25035 24922 24942  1719 27272  8485 48910 34543
 2856 31730 31752 27915 27169 27890 27912 27067 27086 26755 31740  9026
 9028 39419 39415 30688 30683 30680 14734 32227 37229 37024 37454 51533
 6318 12866 30711 30677 30691  9804  9814  9777 48823  1806  1804  1801
20566 50486 31244 39787 39784 48886 17703 41255 41254 22270 23472 44369
 5223 25277 36516 34846 31670 31673 31701  9779  9781 51931 35761 21336
31174 35764 19141 28410 22557 33302 48600 42394 41718 45140 40882 44005
48573 37311 25660 25684 39118 42771 12502  9793 47136 45417 39395 39391
45941 28301 18206 50148 28001 27440 27091 27088  4130 11569 19284 30511
50699 34707 20269  3710 40204 12328 12329 23594 12087 29617 33198 40890
20283 12361 22915 40868 11411 48963 19135 20618 32186 47301 16854 24699
11273 50678  5018  5015 17120 19843 41634 39422 30142 20736 16452 20739
39463 34733 31851 52344 28470 41140 22738 44392 41144 31951 24109 38079
41873 32510 41074 47867 41878 41876 41676 47281 11096 31976 31979  1656
 6264 41128 40238 21802 16298  6164 43689 22923 21556 36715 13329  5598
49056 28002 39398 39412 44342 13479 11637 23906 29143 40079 40063 13243
 7392 13409  6808 40913 39783  6588 48276 41366  3138 31419 40934 39785
 6619 11739 23249 48569 48568 26123 25499 22269 23471 35743 35745  7851
```

161

```
        23031   7847  17492  21869  21125  21126  21121  20104   6644  40936  40937  45121
        40940  21907  40814  18212   6704   6272  40943   6269   6738   6731   6733   6711
        27567  24403  48272  48305  48368  48308  48266  41670  48551  41672  27068  45576
        41256  41733  35064  16214  16780   2061  16777  31691  18096  31722  31728  11371
        16800  16797  28272  39451  46374  31853  31852  43427  15351  15285  15348  15318
         9808  25042  34747  34780  25041  34777  34779  24949  25778  39570  39447  40198
        39460  41719  27996  15418  40266  45645  39387  15286  16291  16287  39446  34481
        52194  14233  36522  19042  49948  24497  48034
1171:25507  43941  23866  50725  27818  15391   7408  11832  27159  44881  33397  36505
       42023  10122   5826  47097  45592   9630  42239   2612   1706  12079  21040  31260
       20939  20213   9265  24419   4296  31724  37390  37789  18266  48936  22093  49146
       26530  15172  14989  12553  42039  14222  16069  50392  51179   3062  39601  25894
       45116  51708   4508  50397  11286  11320  11311  11285  44076  10276  15011  41079
       15009  37071  49422  28029  51559  39319  15224  36293   5409  42006  17938  21782
       48185  47940  47838  15600  27405  29375  29369  30117  30055  30061  29372  30096
       30092  30124  30156  30118  30123   3887  33361
1172: 4230   6988  30584  48721  29576  27444  45663  35801   8783   4131  42266  18585
       3096  22448  41275  37696  48597  10045  16213  10888  18762  27878
1173:49138  11118  37322   3845   3979  14843  32688  13117   6139  44842  26375  31973
       27198  15770  43466  26721  38199  18062   7707
1174:16013   3287   3284  47544  19054  29411  21892  40529  50855  44034  14915  38716
       51686  35116  22265  18770  45502  19596  39452
1175: 1644  46599  23604   8990  14657  33247  44249  51146  51946  11940  29883  41137
       7283  47468  36578  25629  40639  16405  48736   7675  38863  38580  15762  51143
       6176  17870  24428   8941  24263  26511   6971  34391  50160  20672  48146  42469
       42472  48395  19005  24353  43201   5869   8888   4678  28986   8646  42022  25325
       33813  46773  18528
1176:48546  12084  47463  21606  12832  39250  14506  39106  49099  11645  23243   7961
       52643  45014  43599  34822  10202  29255  20182  43195  23525  34355  18097  49562
       45278   9665  11085  44182  12010  10801  39149  44704  38405  46875  31240  16888
       7417   9902  51615   7648  27178   5545  50998  34157  27551  36433  10860  16206
       23423  47172  22482  50395  35147  10373  14360  42735  15366  18486  49763  34036
       29752  35210  49419  11651  12358   7618  48351   7918   1876  20523  35570   9933
       52140  52141  11736  11734  44298  31014  23492  20345   8334  37859  24415  44597
       25457  27545  38340   2490  41747  38012  32910   6007  35293  12536  35038  48480
       23600  26836  29638  12415   3385
1177:24425  31188  41558  31201  48401  44088  52154  20435  39759  51434   5725  34181
       45600  46735  43142  16516  28823  34661  47357  39480   6535  30533  27115  32438
       42064  51278  50803
1178: 2632  41969  42260  22047  14382    856  10679   3595  23967   5060  29953  41848
       19376  45587  18458  43560  33587  20677  42879  44429  13950  43471  47393   9590
       38375  43690  11546  49359  13270  21369  20943  13108  49602  21584   2304  33902
       41567   7956  22764  33370  42921  22245  52731  52734   3184  25910   7362  33258
       46059   7424  33260  33256  48023  48022  17635  17638    833  38348  15578  35333
       46797  11366  46414  24267  17583  12445  29364  34630  41938   2484  41853   6978
       46630  24189  35966    816  43851  21105  45994  21773  50073  30138  40004  51819
       37977  33053   6337  36893   3306  31302  17523  26324  44927  15590  47278  16994
       37478  36269   7955   7647  12965  21168  49191
1179:21219  26137  30310  18313  13680  32642  51838  33157  52483  35183  45891   8711
       45890  34222  11894   7244  35363   4588   2920  33350  33323  32638  32647  32621
       43855  37173  21657  41545  40881  43853  14044  35425  36139  35515  35492  36793
       14704  35184  25755  13124  23812  27605   3676  30449  48547  28981  17371  21899
       19082  42581   9488  31695  39691  48679  44949  10631
1180:10844  27737   5277   5302   5259  26905  45400  36672  22960  22957  26670  22918
       6235  36650  28751  28795  25810  28764  22511  11365  11368  37774  22990  22954
       22985  22914  22911  52177  36709  36678  36770  22984  22949  22951  23063  46270
       22475  16862  39732  18095   9040   9043   9041  49624  13038  10441   1506   1504
       2673  48758  34063  35594   7410  10994  50421  39010  39054  39013   5187  22906
       22882  22880  48458  25598  17007  41406  16964   4177   2945  19231   7760  45536
       23076  50365  23431   2716  24994  24992  16113  18118  31874   1294    837   5462
       24507   1623  15192  23042  31215  37758  22502  43343  23017  45559  35679  35675
       21270  21284  28152  24832   3532  22879  18930  28767  28818  28365  22908  28815
```

```
          28842 28846
     1181:29905  5099 35463 48204   945 19066 25832 16385 10753 37319 31248 48761
          43974 28461  8590 14013  9385 26860  9823 26595 31286 37878 14860 38576
          16990  6964 28128 12532 19794 47955 46972
     1182: 5099 10568 37971 41769 47527 10753 30535 43974 32172 16837 41538 34541
          13213  9385 26377 14860 27695 49036 49073 24177 38115 34110 24886 24781

     1183: 5099 39280  7679  4137 17626 50835 16799 10753  4298  8421  7866 12597
          43974 51413 12313 13213  9385 32372 14860 25637 51098 19594 46228  4775
          37376 32387 48326 37535 39223 46561 38739 50012
     1184:29905  5099 10568 14674 37971 15563 10753 30535 37134 20242 43974 14167
          45256 13213  9385 26377 14860 46824 21241 18315  7873
     1185:19123 19119 11626 11609 11607 19165 52240 30316 19199 11584 11624  4857
           1742 52242 22220 33656  5676 41381 40980 19125 19157  2452  1712  1710
           1711  4670 40939 47337 22850 10625 28975 46139 19663 38154 43748 22109
          15255 31962 41008 11116 11554 43410 50356
     1186: 7463 46533  2604 33038 11805  7893 38799 42853 31869 44876 36008 32239
          11890 39337 22445 35680  4442 32403  9815 17641 46205 51372 10079 41343
          40470 23397 38944 20153 50996 51824 35632 48608 12340 34681 32610  8095
          40835 44379 52644 17066 34919 38256  2134 27223 52636 18826  3021 31379
           7901 25182 51550  6490 48238 39698 44123 21920 46708 32184 29640 31487
          13582 26470 26453  8378 12469 21120 42526 25229 43504  6200 24714 38404
          45409 49325 30539 45682 39544  7635  9668  3216 42223  6427  9132 37019
           8595 24539 39562 33240 14811 36909 37608 10422 26569 27134 15294 28868
          26814 15882 19559 11657  9919 14292 28705 44405 14328  8008 34949 19528
           6528 51810 30521 10464 14145 14177 52238 32470 14092 33209 29631 30491
          46432  1658 37284 23349 28640 19685 13482 24956 27357 11858 48617 49198
          23321  8605  4431 42825 45398 15204 39361  9852 26456  3869 50759 27784
          47659 33612 23286 26164 22319 43491  5388 36284 13382 28247  4243 30281
          52171 34031 22735 21312 34865 50059 20400 25467 39818 41183 37170 30135
           6391 13829  2548 51901 51902 47121  4883  5885 22458 29286 22902  9157
          26778 30899 16753 13217 48008  2977 37724 22591 33363 51420 47897 46897
          43729 14182 25133 21582 23863 26628 14899 22344 23916 48404 48413 48411
           1611 48409 37715 49851 48637 10062 38448 10117 12534 11949 39325 36258
          43610 24769 23790 21761  2179  8135 35226  1498 36481 11240 19085 43036
          48215 40369 39919 34050  5240 36561 33882 49277 49150 20297 23378 43015
          33138 23569 51445 41295 41568 19805 28500 38691  6049 24038 36682  1752
           2398 38624 33292  8774 24400  6411 28081 38322 32060 45820 51077 32266
          22973 15634 48355 38004 10126 15295 43942 46271 19896
     1187:24338 23824  9401 39432 40978 19972  8769 21722 44262  5056 51264 37200
          21787 25902 10936 39892 20148 49161 28133  6181 20650 27441 18969 38913
          32489 49992 13312 16184  4828 46943 41205 49071 10397  7339 19944 19943
          42648 36600  9207 44464 38522 14635 50234 50222 19920 19925 16718 35773
          41877 10336 48495  4419 23630 48028 48731 28817 28675 43103 26927  6141
          19295 18708 50827 22913 19142 51054 46620 32471 19329 38095 44112 52737
          45341 52608  5791 30076 25314 11497 25472 40872  8975  4693 52598  8446
          52461 28053 26441 24087 10529  9591 26114  9276 13172 14340  4820 21904
           1721 43379 36039 11316  3912  1741 18459 39927 19830  8081 37269 11687
           5300 23494 13246 38417  2683 23405 15143 45468 52034 29603  6592 30721
          38087 51755  3698 12524 35637 31353 44537 50024 10445 10206 25913 48114
          48749 42791 21387 10849 40596  9266 12048  9219  7012 43423  4116  7271
          28370 28290 40308  9610 11081 43082 30667 25043 49756 21245 27143  9104
          49650 34424 22732 48989 41173 26308 16699 16680 16676 30080  6217  4625
          15802 45918  5495 21438 25574  6431 13075 26768 12531 23361 37631 11283
          12169  2650  5963 35736 32005 24156 16109 11054 20123 48202 16169 46030
          15257 13444 14526 37916 26259 19187 27669 38855 22614 49409 46907 42417
          47872 18229 16369 28191 32627 20390 12627 22537  7011 38274  1640 17667
            874  8771  7594 52724 17063 23935 32561  8404 49830 42177 35429 12488
            743 35548 33496   744 28273 38401  8445 43114   745  6680 31717  7153
          19504 19496  6630 12204 35896 15854 10450 31720 51661 30479 30477 30474
          25475 47020 19500 15335 23708 25477 47022 19535 19484 16915 23714 15338
          19486 19472 44530 38595 44534 44529 40739 15559 15654 15584 15557 15625
```

```
     15513 13703 13737 15517 13015 15482 15515 13711 13700 15552 15512 13664
     13741 15653 36470 13765 13767 50002 41834 41815 41857 41861 41820 41837
     41766 16425 21933  4273  1680  1679 14014 37815 14768 50231 16574 50200
     16607 50181 16568 14792 14771 14829 16612 14800 19898 16573 16602 19899
     19892 50225 14724 19918 19921 14832 16460 14835 16456 15777 15755 15743
     15750 16497 15690 16490 15592 15618 16543 15477 15478 15659 16492 15710
     15686 14764 14796 16669 25737 49950 14421 15704 33508 26226  2040 38254
      9790 47248 23190 17866  5128
1188:34071  5125  5576 18666 10863 27836 37180 17376 33726  8800 23581 11749
     29759 11957 33391  8750 52383 52388 12196 16721  4453 12464 35882 25079
     36589  5492 31158 37822 38974 46728 52612  8158 24399 32904 50249  3048
      2823 18073 28542 30710 21479  9611 41942 52407  7386 13277  1705 12255
     49248  5203 13199 17591 51598 28953  5136 28932 39074 41914 18327 22901
      2843 41360 25669 25866 19539 42656 38311 13821 26038 34750  8825 21029
     44378 43492 24688 49572 46064 21366 42017  2870 34278 32915 49574 38959
      5499 41528 14964 38347 17556 33476 27437  5486 15535  5467 15528 15502
      5459 13773 37514 21111 34480 34502 24106 48444 21109  5484 49765 24017
     39210  5636 50401 36750 40846 42856 18107 42830 37895 19310 46474 12123
      4949  1541 29225  5443 37775 19962 32488 17393 43912  5078  3279 22507
      1687 45578 43795 39306  9684 38427 10248 38419 35207  3251  7580 52536

1189:40748 40715 22186  2766 37875 10234 48826 34565 21317 25453 24783  3940
     36840  3382 31082 42813 39293 46176 21952
1190:33253 20760 25298 36125 36129 36130 21361 37677 33410 33485 34288 33555
     36048 36051 29683  3373  3375  9472 28924 23730 27907 36127 16834 10229
     36105 10216 45091 28669 45595 26752 21767  8489 26654 41014  7792  8206
     17055 40643 11202 17383 42346 29666 24048 31580 17749 20758 10640  8362
     25419 33263 36019 36082 36081 40315  9380 36176 36190 36074 36078 48723
     18043 18044 42590 16111 34858 35380 25099 48130 44484 46552 12522 36253
     11769 50983  8202 36103 46555 26108 46553 51693 40330  1141  1140 30817
     15243  4904 41344 14396 30926 17979 45633 36148 27562  6625  6629 26103
     37001 36042 23177 34883 34885 27941  3636 43999 36168 36169 31137 52733
     31907 26209 51287 52478 35077 36107 36104 49585 23199 36110 36171 36178
     36055 36933 38970 23200 36294 32687 32640  5246
1191:24654 41238 31362 17614 18168 35383 27471 14316  5275 18037 49817 39501
     17342  1868
1192: 7531 23881 38137 27984 52475 34622
1193:12625 11589 18329 30320 24352  3761 13939  4299 42782 33520 41649 52104
      6910 18477  6933 16268 29312 19280 52251 38627 27141 17557 18480 22308
     45715 23818  7200 30493 10968 34025 17480 25827 17453 17482 17537 18416
     19245 17633 26840  2970 17423 17449 17588 17561 17563 18366 18413 18436
     18442 18460 18511 19218 19224 19250 19275 49975 28442 18507 17395 23324
     52357 48787 38308 16802 28237  9071 22122 37955 26928 21146  9147  4103
     40310 41095 29126 30991 30234 43487  5819 40254 22563 42669 16767 16893
     17988 25250 17489 17636 18440 19322 44023 28262 10318 26355 29827 29682
     47792 34935 42300 52199 38026 46778 46386 23653 24336  8173 48227 37619
     22110 50166 13282  5182 21768 29805 24489 10796 52101 38573 27043  2009
     30393  6441 24720 18531 44007 29716 51381 52289 13404  9359 48220 28647
      4701 41825  1663 15749 29632 44343 12367 27764 22010 38615 27008 19105
     33932  6400 17408 11852 29660 30546 23919 20495 44725  2055 31429 42760
     48874 29011 50824 13388 42390 34182 12839 23079 26351  2588 16010 46704
     35927 29429 34893 20596 23836 44929  2489  8371 45302 33899 40146 27166
     33019  5004 45861 46880 12799 30830 21266 27510 50390 28394 13453 44443
     34976 30360 41470 11314 14812 46850 35945 50432 16765 10511 38541 46755
     47047 21808 47152  4861 17081 47772 23100 47171 44666 31284  6574 37557
     17083  4985 38330 23621   911 43579 41709 33542 40573 28455 47303 39991
     34298 37808 26162 16189  5565  5883  5887 18419 18479 19277 35041 17420
     48362  4518 17640 45772 37026 38809 31315 41788 33414 12238 46471 35328
     29557 22716 45960  8495   910 51448 25225 12932  8875 20853 48126 37087
     26864 35113 43890 52410  4822 19193 19221  4585  3618 25033 43065 30377
     22350 50981 39518 37154 34195 49747 12206 28665 40765  5253 36126 46286
     19986 36533 47534 43920 19563 36509 47720
```

```
1194:34192 39197 10623 29095
1195:50677  1362 47684 48464 48870 27063 20093 28760 11263 14134 35376  6000
     38220 49722 29569  9062 22374 51043 32757
1196:25237 48299 25408 37531 38223 27969  1624 41126 51302 14510  3147 48850
     29613 13429 52445 46024   915 29961 28979 17823 51524 20196  3376  9541
     38443  2140 38590 34973 38379 49260   916 19201 42279  4562 36344  6100
     46587 25995 46363 22382
1197:31571 14773 36502 28310 12494 24808 42798 28185 47105 14462 14476 37313
     19743 31686 46446  7303  8919 48384 40160 43238  4986  7831 46239 38216
     33427 30519 26502  7112  4812 10548
1198:17040 43628 39381 46611 10780 35701 36601 49856 29762 52581 52575 19364
     47964 49715 28437 16533 11245 37773 20670 48387 33862 26330 16175  2443
     25183 17328  6058 20220 39091 33786 42498 16420  3016 16127 44116 50842
     50841 11381 17829 22298 22299 41333  4591 48158 17726 26262 38608 33641
     27623 23706 15090 13222 50214 50213 21749 43682  7181 51373 12736 12853
     12846
1199:17748  1967 52689 37663 34625  1646   921 23817 17754  9009 32119 51572

1200:25706 36847 12047  9196 11299 46635 45582  3416 10799 51962 41146  6105
     36381  1365 42211 11070 44914 35625 31427 44417 14291  8249 30995 20986
     14368 50700 43388 17864 40780 28681  9378  2881   923  9495 38758 43539
     12792
1201:25706 36847 38610 12047  9196 46635 45582  3416 28483 16672 47951 28320
     30215 47890  7443 41633  5753 14062   924 43159 27058   925  5045 11563
     37598 43539 47190
1202:14203 45387 33365 36973 26936 23834 48550 51861 42297 21353 29216  8953
     43674 17973 47127 17201 32235 33808 24292 20181 23865  5956 11627 36618
     26468
1203:35165 29601 36351
1204:48792 24595
1205:28787 17886 28339  7210 41905 12702 24286  9016  6881 50247  8107 45508

1206:49259  3598 36397  6688  2539 27734  5111 13303 37723  7142 31721 40695
     40527 34144 47061 27799  2203  2274  2585  9198 10372 35437  9171 42016
     19858 11748  7673 33078  9792 10720 31492  1374 12335  1601 18716 18603
      6249  2460 27420  2671 19683  5273 50602 45299  7320 21255 16849 37782
     48636 26463 30978 31088 13879 14550 13099 16665 47187 42888 44060 12082
     24567 50997  1619 37521 29020 17258 23446 40268 46458 15677 44918  7423
     18838 44977 47418 28894 52457  4488 29890 50461 11480 11037 34106 50834
     20980 23981 21603 20950 39786 23410 12343 32028 20887 25743 39650 42332
     19966 47518 21951 41450 27848 42702  4482 13626 29008 26405 42945  2108
     28709 24211 38474 46159 41075 41104  8225 21940  2862 45194  8347  9224
     12622  5750 47886 45752 43983 16507 17241  3276 51790  9183 14823 16284
     29325 36280 10193  9451  1881 51768 17698 50652  5237 14480 28687 30883
     22508 40954  2237 51068 51119 14794 14828 40500 14854  7799 50754  5505
      5502  6415 10742 43289 21534 48195 33134 30413 24856  4190 31719 19415
     39290 35960 35419  6877 46321 19756 46345 14565 40385 10250 48876 37559
     33246 17077 24591 20205  4210 31001 47485 32050 17881 18599 18524  2934
     25516 42392 46663 52258 47898 13185 34764  7258 22935 52379 34161 50174
      7209 51620  7155  3607  8037  8101 17190 14589 48494 41828 33733 24254
      3167 33288 35802  7032  9237 51670 50991  6173 44058 48849 22123 43683
     39493 42229 35829 25051 46400 31252 47922 23481 22352  8610 49633 30283
     52103 32846 32768 35334 23832 52395  7535 44174 38609 44768 41619 35788
     12562 51006 42700 14504 14611 34619 26354 23802 11865  7133  7135 17043
     18627 41887  3263 18625  1670 17658 29145 41456 52220 41476 28929 35759
     51026 35394 13052 41708 13666 28719 43622 10768 27180 11704 32134 10809
     39547  4313 32446 25480  5069  7800 12506 25128  1552 41169 43260  9352
      4015 26794 30193 33070 14209 33502 42733 17629 45490 17306 33817 16764
      7081 31955 44835  9557 41457 21217 21218 43799 43800 14354 16093 30016
     18790 18786 40630  3516 39873 18579 28931 45491  1661  9320 43011 32325
     45966 37836 24774 13509 44204 36331 37833 46629 22294  8361 23693 37086
     24359 44613 13994 17972 19094 15510 16751  2004  1736 45721  4247  6545
```

```
        30408    2079 26827 18661 23649 19023 21305    3851 45919 36557 18087 48732
        25364    9428 27345    5868 37365 50384 28033 47306    2131 38400 34654 34669
         4731 38552 37152 45309    2303 21611 48403 28828 13568 12050 51357 11128
        24803 17767 39600    2976 44604 51003 47215 47185 23269    7302 28393 16706
        13050 40730 14759 40710 40763 35234 51506 32712 38473 38476 32711 47351
         1373    8589 52623 47461 46401    1704 21456 34616 36882    8074    9739 14461


   1207: 9678 27436 33298    6987 34402    2942    9431    8961 36010 36616 51944    9100
        15211 41311 32719 39317 52328    4672 12626 12630 42816 51958 33407 44896
        44890 44859 44891 35062    7526 38878 21474 33699    7556
   1208:18514 38022 26021 49682 49659 49681 49657 15798 39833    8005    8054    8056
        30738 49140 51308 51338 44263 17897 14735 15462 32543 46855 40944 40971
        40942 28026 41033 41035 36976 14741 15464 33409    8356 44107 26686 14065
        50192 48161    5811 32709 13537 47320 32385 46970 41006 41004 32675 41631
        34145 50290    4242 49352 51176 24549 23926 16650 49037    3905     762 40034
        38182 49684 23111    5964 22808 22830 22953 22944 22950 14744 15465 49865
        49837 21473 21744 21633 38183 33236 33222 33221 33218 21176 18689
   1209:24752 34196 41746 45935    2910 29892
   1210: 6260 30070 28395 33791 32013 11918    2525    6742 21132 40189 40193 52372
        13772 31495 49890 21718 30034 10485 25901 40042 41656 14449 44382 32472
        22982 16852 37627 16392    5376    8598 50361    7197 44300
   1211:23368    4628 30933    7487 42815    9030 42712 42713 16693 16691 27340 30239
        43640 30703 41448    3875 32770 51764 48867    8754 46751 10756 32802 31989
        39981 32669 39362    3886 43387 33148 24675 17766 26342 35085 35125 46502
        21373 27078 44615 36044 21276    4913    4899 44718    4411 10898    5385 24885
        32929 21335 38889 19457    9676 45103 45092 43815 43821 24387 46488 37950
        47635    8607 40420 34624    2368 23804 31442 21384 51288 48831 29802    1914
        44744 19352 28527    4648 23616 49977 45459 48670    7248 43793 43770 43773
        43772    9403 32790
   1212:47473 34781 44685 35307 17451 30139 43197 31549 47800 45735 49707 45990
         4521 35404 33210 14498 48378 13875 38911 25106 49367 27729    7385 28171
         6631 22061 13603    4736 17034 31542    8401 27201 13154 47686 30766 49267
         1536 26784 46885 52620 24458 36224    8508 20359 23828    2838    9134 42478
         9647 37114    4752 47304 41272 11063 51094 32135 24979    8454 12888 14093
        18434 39040 26213 25597 30819 37651 42710 10428 33399    6242 23679    1692
         1691 13155    8242 24325 42602 10811 27044 33886 47601 16037 26950    5270
        50783 25568 50242 45107 50243 27527 21446 50704 48420 30507    8991 17559
        43406 43604 30925 42502    4974 25515 12176 38801 29983 14429 47765    9073
        31476 38433    4726    8653 51462 10233 34568 34452    5864 34167 48574    9367
        33529 43915 18516 38141 30195 30191 40269 26182 26151 26177 26185 26169
        26173    2202 22722 42624 36632 24089 48127 47264 40488    4109 47383 18244


   1213:31615 36848 35513 46223
   1214: 5643 19969 27575 12933    8201 50919 19445 29592 42613 12901 29897 43585
         5025 20817 52404    8470 38659 24817 16639    8364 24007 16368 20992 49003
        41067 41070 38067 41063 41093 38050 51536 10803 38068 41065    1514 50374
        21689 44081 31425    2325 37278 28342    5444 45034 17341 42029 49799 20989
        46268 42845    8463 41442 20999    7105 52322 28961 47313 23253 47340    6464
        46027 30496 51249 26854 11056 51186 52647 23614 47612 22404 35346 18804
        19929 32569 25280 19957 41127 50272 39500 50320 41304    5670    8467 15105
        20998 45229 45232 45251    8583 11595 26167
   1215:14077 43221 37015 14073 31404 21460 14070 45896 27652    5770    3501    3504
         3473 18990 40701    8169 24439 52387 36273    8192 27594 51995 26828    2751
        23179 27469 27505    8176 27650 27564 38011 18213    2946    4310 27628 24242
        27074 24730 44308    8546    6794    7577 52698 15896 42755    6067    8221 41785
        29672 36912    4645 41804 41808 25228    6069 40969 24171 52159 41468    4433
        18549 21661    3604 10087 38501 39338 42861 34340 20341 35974 28984 22786
        43850 49680    2425 49581 49603 38328 45746 45755 26380    3469 29240 33564
        27474 27625 27644 27600 27681 18011 18009 31954 30233 16074 41933 10024
        21816 21821    6037 35509 21840 11339    8828    1617 31537 43894    1906 19502
         3678 41913 22434    8829 41979 29159 38372 41869 48108 50257 50292 40633
         3741 27046 18103 10173 41342 30442 33683 18824 11866 32708 21682    9152
```

```
20988 20587 34840 20724 44813 43784 37279 11272 45900 45931 45933 19403
45892 45895 10415 32523 12229  8274 27922 46200 48854  2339 27661 43460
 1994 30651 30598  2799  3560 39932 18623  8808 44180 51419 44965 32197
34796 26424 42217 12743 12785 25243 22138 12227 27012 49075 38381  7079
36550 12905  4474 27561  2297 42992 40892 25433  9013 32430 12181 12688
30388  8015 47007 48526 48575 18279 28946 15162 31266 31271 46688 46468
31413 28714 34910  2845  2847  3468  3507  3509 44375 29484 29507 22291
13521 33815 47392 11581 39701 23246 12620 24288 40966 40907 28533 25158
26095 34905 51592 50223 33357  2569 20098 37175 37321  6660  6842 19769
 5733 44606 44605 33693 32238 17730 18108 46332 42652 15950 17528 42517
37543 14310 24561 18719 24379 36069  9682 32162 18738 38228 49966 40799
44596 19359 20381 28834 27814 19318 48415 50346 21310 45352 18418 25362
11468  4405  4383 15444 42573  1897 37941 37323 21600 23965 44026 45439
21593 48081 44511 48293 23101 23099  8306 18819 16571  2566 44258 32867
 8167 52419 26395 50099 24485 11850 48426 48667 34751 17359  7461 30152
51976 52094 43662 27538  4023 51005  8271 50740  5202 36050 34164 30605
 9151  8355 46601 46158 15891 15920 15923 15925 15949 15928 15897 13470
 6330 42418 13026 24102 19732 13187 30988 25013 29302 25351 36920 52127
42301 50164 12480 15088  9995 42560 17106 14249 26266  6128 51305 41941
12180  5001 15952  7310 26305  3857  4680 43744  4342 36574 45360 35413
43241 39734 29674 14348 33602 37364  6566 45018 12869 46846 50515 46517
48788 43003 22751 27507 27854 22700 43081 14148 37222 36917 23216 29493
 2229 10824 12518 35188  5645 10834 26233 27656 40531  9315 18991 21422
21410 21457 20583 42494 21455 21459 39286 35322 21362 21626 21492 21496
46772 37068 41375 18675 23750 46380 47270 15467 10510 48735 35935 17505
41540 14663  6031 41935 27867 27566 41823 20321  1991 42594 19422 16227
 6140 17495 15900 14538 14540 24927 49163 16807 18300 46258  3347
1216:37015 31404 14073 21460 14077 43221 14070 45896 27652  5770  3501  3504
 3473 18990 40701  8169 12991 36273  8192 27594 51995 26828  2751 23179
27469 27505  8176 27650 27564 38011 18213  2946  4310 27628 24242 27074
24730 44308  8546  6794  7577 52698 15896  6067  8221 41785 29672 36912
41808 25228 40969 38871 33211 24171 41468 52159  4433 21661  3604 10087
38501 39338 18880 42861 34340 20341 20965  6291 35974 28984 22786 43850
49680  2425 49581 49603 38328 45755 45746 26380  3469 29240 33564 27474
27625 27644 27600 27681 18011 18009 31954 30233  3101 21590  6037 35509
21840 21816 21821 11339  8828 24168  1617 19502 43894  1906 31537  3678
 1933 22434 29159 38372 41869 48108 50257 50292 13289 22500 40633  3741
27046 18103  4831 52746 49489 29308 31140 10173 30442  5375  7273 33683
18824 11866 39809 32708 34432 37389 21682 25244 11201  9152 20988 20587
20643 21323 21329 34840 27147 24555 20724 37279 43784 44813 11272 45900
45931 45933 45892 45895 18754 26475 10415 32523 44978 52498 34809 48340
 8274 46200 48854  2339 52732  5863 27661 43460 21062 37448 45714  1994
52213 30651  2799  3560 21140 39932 18623  8808 47417 48211 51419 44180
44965 34796  4983  6663 23560 26424  7762 42217 34753 12785 12743 25243
22138 12227 27012 49075 27266  7079 36550 12905  4474 27561 28803  2297
42992 40892 30332 44987 25433  9013 32430 12688 11857 30388 47007 48526
48575 18279 28946 15162 31271 31266 46688 46468 31413 28714 34910  2847
 3468  3507  3509 44375 10683 29484 29507 22291 13521 36684 33815 39701
23246 11581 47392 42557 49916 12620 24288 40966 40907 28533 26779 25158
34905 50223 51592 23044 22077 51721 29726 33357  2569 37276 37321 20098
37175 45234  3143  6842 19769  5733 44606 44605 33693 32238 33994 27557
15084 17730 18108 46332  6517 42652 15950 17528 42517 37543 24561 18719
24379 36069  9682 18738 38228 49966 40799 19318 48415 50346 21310 45352
18418 25362 11468  4405  4383 15444 42573  1897 37941 37211 37323 21600
23965 44026 45439 48081 48293 32715 20389 44125 16571 44258  2566 42398
32867  4151 26538  8167 52419 26395 50099  5404 24485 48426 48667 33792
31230 34751  7461 30152 51976 52094 43662 27538 46490  4023 11437 51005
 8271 50740  5202 52224 32407 36050 34164 30605  9151  1484 24617 12454
44329 17824 20918 46158 15891 15928 15925 15949 15923 15897 15920 13470
18397  6330 42418 13026  9835 41835 17218 30988 25013 29302 25351 36920
17144 38219 46805 52127 23341  8679 50164 42301 12480 15088  9995 42560
17106 32734 30967 33439 14249 25047 16684 34307 11780  6128 51305 12180
```

```
        5001   7504  15952   7310  30840   3857  36780  16126  43513  28319  24993  45360
       35413  32686  13676   4451   4455  49857  49885  46846  50515  46517  48788  43003
       22751  27507  36501  38266  30776  44588  43081   5372  14974  48240   7603   9620
        5227  34675   9455  36917  23216  29493   2229  21889  10953  10824  12518  35188
        5645  10834  26233  27656   4732  49627  17195  47433  21141  17332  21110  37486
       46796  17210  47574  47606  37383  17193  47582  47604  34184  34212  34179  21583
       21648  21621  34897  21542  21587  21580  34229  17299  17296  17292  21650  34901
       21575  21548  47435  21330  21545  36586  36587  36566  36563  24481  34903  36585
       40531  51547   2814  19526  29421   9315  18991  21422  21457  42494  21320  21410
       20640  20583  20612  20637  20635  21418  20614  20580  21325  20616  21455  21459
       39286  35322  21492  21362  21626  21623  21496  46772  37068  41375  52076  23750
       18014  46380  15467  10510  48735  35935  41976  36306  34334  41540   6031  27867
       27566   8226   8229  41823   9703   7158  20321  19422  42594  16227   6140  17495
       15900  14538  14540  19731  49163  16807  18300  26980  14083  14080  14108  14078
       14075  14098  14072  28593   9909  42117  17368  46073
1217:12703  20230  19550  43375  27283  15886  35187   3207  29999   3202   3204  30922
        3239   3242  30843  30816  22561   9926   9954   9928  20081  20078  39619  30470
       30464  31180  30444  31182  30463  31176  30435  30441  40571  47376   8650   8678
        8686   9360   9961   5579  45383  45971  12605  13313  45976  45973  13365  45997
       13372  45999  44646  44644  44723  44749  44751  44578  35192   3208   3234   3209
        3243   3267   3262   3270   3273   3274  31577   3352  20232  20218  20233  11660
       44715  45357  44717  45382  45353  45351  30885  29960  29993  30847  30844  31631
       31632  44587  44583  44584  44659  10895  44641  44719  44746  44701  44663  45419
       45442  21507  21514  21553  21632  21516  21550  21551  21667  21596  21627  21629
       21631  21588  21656  21662  21563  21697  21698  21702  21704  51748  43540  43571
       43544   3680  43574  43592  43546  43564  43568  43593  43620  43569  15350  15352
       14695  31729  43532   9025  39157  45420  49931  46288  12704  29655   9891   9957
        9931   9898   9950   5578  39536  52306  52302  52310   4803  52376  52312  51752
       52378  49070  20025  37862  45806  16309  29680  21592  21510  43366  27261  39277
       32967  33030  33028  33002  32995  33087  32969  33033  33096  32938  32936  33086
       33061  32932  32963  33025  33000  33123  33065  33035  33129  33093  32973  32997
       33127  33161  33132  33136  33163  33164  33804  31663   6818  52685  36475  29988
       29990  30649  30645  30644  30648  30709  30714  30720  30689  30757  30749  30753
       30779  30781  30806  30808  30802  30812  31572  31573  30959  31581  31603  31660
       31655  31661  31633  31667  31693  31694  31700  30723  30726  31702  31704  40169
       32632  17901  11992   8243  32964   6382  20032  20037  20056  20036  20054   8674
        9331   9351  38535  44110  23619  10403  11863   2833  41405   9361  26008  22662
       50271  23912  27963   9319   9324  47588  40325  43244  26544  26545  43245  26548
       43251  26560  26562  43270  26564  43274  26588  44101  27413  43285  26593  44070
       43308  26594  27344  44072  27375  43311  26625  26627  26630  43315  43340  26633
       43342  27253  27259  43344  43347  27260  44103  27386  44108  43368  43370  43371
       27279  27347  27379  44073  44028  27285  27289  27308  44030  27383  27350  44080
       27309  27312  44033   9282   9354   9327   9332  36424  39273  38694  38706  38699
       38652  38655  38677  38680  13733   6184   2208  18262  34529  26200  35378   2760
       47696  45977  23895  47186  16033  23936  29840  12544  12580  45949  45948  13344
       13338  45995  19299  15935  51880  21254   8644   8645   8676   9280   9287   9350
        9960   9868   9893   9927  10650  10657  10653  43908  51805  15550  49335  48561
       33023  26871  46888  26279  42083  20579  13731  15955   2424  13531  13525  43919
       50517  34943  12478  10886  37042  33752   3323  30641  30000  30784  30788  15347
       30880  30878  30877   9900  16756  16760  47583   2589   2582   2586   2583  14699
        3293  31610  31607  31608   3299   3318   3315   3300  29974  29965  29972  30678
       30682  30686  30676  30747  30744  30854  30852  30873  30916  30920  30930  30948
       30927  30949  30955  30951  31579  31629  31626  31613   5024  36752  34497  24858
       12808  45414  12817  12819  12820  12844   2210  44791  12847  28157  39611   6009
        8080  25415   5912  36487  13527  13587   7511  49371  44539   9748  46085  39629
        2421  12812  52381  52402  52403  52371  52350  25230   9278  48557  35642   3485
        8647  42412  41400  17123  47581   3683  39161  43240  26541  12675  39163  39165
       24044  24047  12737  12732  12738  12734  12780  12782  12713   9896  38592  41664
       41660  44555  44550  15972  45440  12856  52653   1786   1785   9436  11446  12879
       12881  11460  12741   1639   1607  48793  11450  46359  46372  46356  12708   2362
        2314   2364   2283   8623   9290   9379  12851  12876  21509  44788  12783  12787
       12874  44786  44782  44753  44784  12854  44724   3328  13561  38038  47693  13550
```

```
     13552  4874 16481  2219  2337  2215  2256  2334  2340  2311  2251  2246
      2282  2928  2960  2923  2956  2955  2954  2919 44554 12878 44626 44697
     51749  4805  8682 51744  8406 22729 15346 43598 45413 45415 39168 33801
     27352 27315 44039 43339 44105 27414 24026 24045 23769 24051  9923 43382
     17663  3295  3297 43258 46341 46343 12710 31131 32759 32779 32678 32672
     43374 43378 12569 12576 12579 12584 12585 12618 12606 12610 12613 12616
     12643 12646 12647 12650 13555 13528 12671 12677 13557 13523 12678 12681
     33195 32933 33194 33193  4095  1783  1781 44623 44620 44690 44745  5621
     20179 44582 44726 46920 46829 46783 46807 46860 46836 46785 46806 46859
     46779 46882 46831 46916 46887 46788 46750 46749 46834 46856 46747 46727
     46803 46914 46861 46864 46754 46724 38888 12638 15957 15961  9023 44553
     49708
1218:34384 24196 42846 20609 12235 16234 32454 39181 40615 14805 35640  9489
     49280 42953 33701 13768 19232 35965 19121  4693 39407 41359 29368 51086
     27860 40324 10647 17759 25193 25194 33814 20531 31896 46384 32326 32088
     44985 37765 33272 27482 32055  4962 24486 50128 36452  2556 24523 43982
     34719 33493 17158  4017 19993 22472  5074  2750 43083 16472 45653 27762
     19656 40038 34932 34468 50944 18677 18674 33652  5701  4914 32696 34672
     27421 42544 44337 13221 45023 19004 26065 42164 11919  6435 11388 44590
     13573 51600 29502 25356 35986 49879 24100 14848
1219: 9311 37790 47479 23869 19456 44642 14737 19786 18510  7439 31057 31061
     30209 46641 22633  8001 39977 31292 41109  6597 25257 22302 22296 13861
      8593 10751 50637 41784 39749 10017 47177  2083 16447 34043 44113 38477
     13720 17443 23241  7616 48687 40866 20111 28376 27512  4107 26917  2646
     28676 20728 47761 40644 29326 24876 30547 36047 13086 30465 11258 24853
     29587 29585 29584 32784 11284 42972 39767 44761 38725 41827 20526 39224
     15903 11364 37182 17664  2280 51510 21124 49784  6433 23815  7709 51809
     37907 42062  8568 28054 41090 45669 43705 23263 19926 37763 19190 36298
     20696 13904 43215 51422 50613 21377 10910 49767 13224 18170 29138   784
     41908 37218  8399 51590 34964 26479  9036 52183  8591  7689 52590  7231
     43043
1220:48873 18592 12447 47675  4751 36113 46872 39196 18694 36972 15094 29344
     12109 20794 27015 44781 31022 28226  1688 44696 37368 34521 37666 21404

1221:  814 14468 52628 38769 45068 18728  1831 16843 49200 40013 49242 15913
     12369  1842 27282  4307 38882 35607 25944  1146 15053  2858 20510  4685
     28182 28516 17417  4517  6122 14841 28105 24614 20139 30064 42481  9704
     14467 14087 17124 32849 47379 48497 17490 29903
1222:28890 50651 18496 32462 38398 14784  8777 35311 10962 35824 19748 32736
     38565 18027 39190 41454 13851 18063  2690 24599 52543 43839 36164 14345
     45507 19360 20112 40264 19546 29253 23455 22874 32029 40109  9771  1605
     38246 19981 21659 47480 21306 10009  8381 31646 31642  4111 39896 38814
     13360 12749  8014 37343 25442 39264 14995  8447 26119  5252  6565 16582
      4018 38948 24986 20192 13345 48802 48780 11038 22905
1223:29858 28235 36352 33855 37920 39241 35873 24899 46236  8586  9750 20168
     17121 35949  1147 51914  5803 35602 29250 46218 14424  9396 16018 21663
     22372 28799 45124
1224:14606 15093
1225:27976 47883 11470 45833 49970  5746 29652 31565 10341 37078 52527 51969
     47939 21652 47994  1323  9887 44163 39116 50312 32320 45716  9137 13122
     41445 17289 48882 37228 46267 51416 43911 22103 29215 46339 38798 11944
     35137 35306  6646  7110 45394  8481 43665 26491 29723 40761
1226:37329 34958 13438 13235 49702 13008 24390 40816 14295 29659 26573 14615
     42626 10808 11317 40483 22943 29901 34005 45851 30006 34470  6190 43139
      1739 40425 31490 28534 47145 50631 10144 44387 42220  9917  5624 20293
     24965 37204 16824 34492 40976 35947 33955 25434 28112 51644 13956 39093
     17062  2422 39098  5536 23393 13547 33811 21349 16941 34902  4797 11663
     35655 31768 43737 33695 43603 37519 38892 35694  9236 47742 25127 48946
      3524 46107 17817  1895 37939 34406  3422  1784 18795  7202 49740 18084
     42523 23182 41497 20090 33245 24490 20621 38893 28359  8836 25282 32217
     48086 22161 48713 46036 13468 25034 25833  9796 40010 28088  7645 12244
     15114  3855 49954 23873 51286 29350 41760 24820 36703 42703 24901 47053
```

169

```
       5583 32114 40661 25594  4755  3743 14376  3331 15102 24849 28853 13682
      48010 39340 22111 46691 12872 28567 16778  9317 44779 23014 22746  3479
      44215 34988 50299  7452 10512 43841 25489 42263 22055 23303  4673 48221
      45332 23325 29103 44082 40525  7177  2614  5201
 1227:52002 50744 37587 34962  1324 16141  4631 40171  6551 27270 13761 17803
      16766 39829  1530  3345 17527 21004 44612 19832 28392  9706 23610 34445
      43352  3895 32208  8392 36698 35446 35350 51589 11702 26799 34716 41546
      11150
 1228: 1442  6479 39284 33843 10925 35457 33732 17206 15711 49243 52585 13922
      13998  7939 27537 21851 51097 15542 36663  2698 44198  5519
 1229:20477 15733 12442 33562 52198 19001 26013 41017 11675  6208  8326  6303
      46075 17326  3092  4500 20353  9762  9231  9330 32635 44266 38271 29419
      25162 20305 22397 20687  6790 38976 16715 10109  8413  4650 28463 49042
      49309 28793 18798
 1230: 9424 14023 18659 50958 15953 29046 14391 18414 28464 20241 49275 45135
      51169 42724 45395 15844  2242 14708 47232 36210 16435
 1231:27555 51683 49723 44637 10885 30589 19895 43254 51948 18039 50311 43351
      12408 29068 51170 38248 25279 19493 44428 22602 44536 38497 14891 26597
      29570 48460 18341  3675 13465 23572 51000 28175  9179 51174 49789 50096
      13308 36905 25233 48109 17268 41768 18564 35648  2773 12629 22430 10470
      31489 22470 45545 17205 30380 40786 39461 20243 33728 43459 48332  6675
      44412 45007 32500 40764 17476 30741 41889 25955 46912  4668 23220 50208
      41985 21665 40929 45020 14641 21059  5925 19700 11587 51875 15214  3107
      18035 40386 45170 50345 15556 40243 44816 49762 51197 38043 10340 23738

 1232:41894 24271 13098 39564 39431 31385 24859 14999 44287 49241 51785 27125
      34821 49759 28777  4379 28776 13717 25981  9267 27863 33872 40938 21947
      21949 13116 22080 37377 37379  6817 39521 15343 20008 18347 36869 33522
      15786 15784 42891 26507 34027 17518 10076 10081 51616 51092 15398 36697
      22415 24362 49601 52369 47197 34930 37235 24311  9502 44938 44993 21620
      36896 14210  5033  5034 12707 22590 37330 11575 42612  4497 31474 19933
      45129 24505 18987 36755 24787 24921 16993  6085 35993 32651  7456  7428
       7669 48733 32814 32815 22567 22708 49595 19681  5408 10431  5384  5382
      40441  3013 29275 23631 29276  5921 16845 26483 18883 19650  5922 30434
       8085  8945 30576 31433 27374 23861 11392 42515 22523 26760 10853 10856
      18613 29056 45047 31360 43264 39380 31539 31540 18041 48949 19644 12078
      12081  9382 44514 37123 44518 32560 31612 11679 21878 41076 48501 40991
      48502 40992 50221 50224 25790  9776 34680  5063 39832 19477 38198 38201
      37183 37184 20549 48078 29267 29383 18848 11425 11427 18945 48075 34361
      33574 16755 48419 37345 37510  9899 43516  2471  7431  5937 33972 37434
       9343  6287  7104 47577 24596 27014 27802 40094 46723 16645 39708 40754
      43531 21008 23175  3123  3120 41710  5971  1823 20003 20000 12895 10070
      26253 20237 46412 27339  7859  4196 42405 30087 47353 30509 20957 22091
      41580 41583 20864 26206 42759 31737 34488 34490 34547 34549 19401 11194
      34191 21221 40946 20185 25972 24273 35174 18124 13815 52231 43112 17278
      43973 16444 44865 43971 44864 44857 35530 31124  7524 22652 12990  7322
       3307 25589 34592  9054  9055 24854 29035 18031 13495 11378 28748 44937
      49724  5810 47163 20030 33927 33180 13280 51046 29320 23579 41150 28901
      17571 39272 14434 33057  1727 50936 44966 28897 51471 23340 52019 46353
       4301 34555 51703 40548 14460 35152  5481 51295 48685 33818 40110 31363
      47597 52260 49022 33266  7540 17967 18358 18380 33980 13327 12884 13844
      49087 16811 19325 37496 36639 21748  3612 21750 29845 12510 31456 12297
      44324 25358 28814 51433 13476 17949 44374 41186 43635 17125 26925 19961
      19552 44980 29023  2070 40498 22926 43444  9849 22193 22194 30025 35349
       9819  6320 23204 48148 44229 37401 31669 43834 50851 28076 30769 41740
      49712 46828 47700 15881 40659 40660 31208 12699 34789  8232 41557 51712
      51096 28999 47212 47774 50023 22342 44611 41947 48824 21190 14353 39509
      41764  7895  7898  7856 29265 37090 32126 20458 23168 23310 38633 42622
      49570  2540  5244 48887 28233 33244 18237 33795 45060 42967 45782 44280
      20364 45964  2642 25772 25770 39308 44319 52663  8652 34918  5667 45565
      18672  1492 42898 26621 31697 46297 32152  5723 17857 47550 45027  1675
      27582 27415 32140 34944 11119 28649  7474 36751 38463 38464 29600 38413
```

```
      46547 50319 34665 49569 15636 29121 52021  4664 13983 36791 34324 36788
      18452 41114 17016 12804 34688 34742  6623  1555 23546 16700 21608 35749
      35747 47324 12410 13601 18808 46134 28096 28022 51133 51131 13445 32645
       4979 43121 23443 27614  3991 35259 10285 10931 19622 10288 10933 11023
      44293 15655 14469 36551 36573 51468 15218 52216 48290 27718 48628 28292
       3484 26867 11296 14559 43002 19690  2869 47880  4730 12609 14535 32047
      51469 26129 48084  3356 26332 28812 15024 15025 18368  5527 30187  5530
      30188 34508  9521 14978 40424 29230 10264 49305 22237 39026 45845 10182
      16459 22254 19687 22257 28883 46303 51487 44436  9338 30731 44439 16463
      44437 52035 52006 19680 26620 40175 51380 48133 19098 21376 11133  9448
      28400 28402  4168 29331 51333 29329 18389 19749 16643 23201 19647 25341
      26842 27231  6740 47790 47791 19636 19684 19642 37381  1657 13610 36792
      36410 15771 44119 16550 14517 14536 14520 13909 13895 13868 14524 14627
      14624 14619 14592 14629 14671 15520 14595 14669 14590 13941 14621 14701
      14566 14656 14568 13935 14569 14729 14542 13906 14593 14738 14703 14534
      14698 14539 14732 15531 14694 14659 16530 14772 16471 16532 16467 16461
      16577 16576 16586 16470 14515 16610 15524 16503 16498 16552 15564 16547
      28563 26409 27574 37762 37764  3685 51465 37105 46643 18938 30427 21112
       7154 42142 49613 25040 49632 45076 45074 46324 36451 35952 37679 19448
      43481 19648
1233:18654  8286 33790 33772 48156 38074 38078 37712 41793 13393 11579 12446
      51565 16522 51563 29546  1772  8872 50666 52588 51858 36308 35653 26536
      12391  4937 42743 19646 30870 19467 15623 37484 45947 48197 45512  4161
      36667  5100 22116 21916 15948 34331 35282 47823 48051 29831 38666 39371
      45174 45125 17620 23682 25372 29803 24121 12186 41492 11115  2255 48231
      25102 34698 23480 52348 30011 34687 18760 20770 37352 25951 23798 47138
      10665 14157 10661 38962 33396 33392  6893  6822  1912 33366 33369 33854
      49583 49582 11616 40292 23448 17000 12682 16290 12679 14543  9998 20424
      15050 20201 41555 43090 46812 28405 47976 25096 48708 48707  7500  7518
      49016 49010 21356 23248 17220 49096 32094 33578  1889 18076  5055 30957
      30104 27478 32862 34855 25716 13371 39103 15850  2657  7328 34163 18360
      48033 40134 44736 35155 51561 34253 47526 40838 34376 32586 19544 15783
      14937 50913 25742  9548 35126  5567 52077 51568 10365  4277  4279  2162
      22002 30141 47373 28557 37737 13947  6079 40089 16451 33091 39669 26281
       5442 26282 36172 27859 51745 52283  6890 28377 29513 45843 50908 21752
      24117 46199 50444 11124 25751 52132  9223 28385  2963  8615  8138 25864
      37909
1234:40546 23620 11844 19485 43122 48106 16183  7407 46069 38369 51123 21024
      46569 34051 14228 32261 45485 40745 21860 51127 40043  2811  2809 35522
      35205  8157 31832 23997 37976 13590  6714  6696  2406 14655  2842 51464
      40354  7881  7946  7914 29213 25195 47981 28314 19765 50069 47487 37776
       5032 34347 34349 20729 15905 47646 47644 38877 12767  6174 12882 49212
       8053 48176 16363 10317 33858  2688 28854 34907 34904 13163 11442 10283
      40584 31846  5496 33294  4486 44774 52203 17390 18264 38439 50614 25709

1235:51491 33451 48630  5088 14814 29681  9945 52221  8845 39436 21392 26188
      10348 42075  5611  3909 33558 34199 33556 34194 33552 33579 34198 33583
      33575 33586 40100  7117 41702 49001 34933 23545 17674 49205 40127 19499
       6934 16536 42948 43219 28061 19717  6237  6221 30759 11406 45912 46378
       7515 27983 26056 36303 51664 36143  8756  5571 32017 45456 24502 52168
      39086 26420 51090 29125  9118 45654 27235 18840 46413 24610  6763 25560
      10163 29034 39679  5365 39249 49600 31200 23538  9399  4567 13786 14104
      42675 37975 13777  3472 33937  3880  2791 11459 34062 45963  9329 23052
      26886  2571 17394 35151 26431 37635 46381 52117 52013 43237 39864 20083
      51452 30785 14670 40108 11842 11377 43216 42859 24451 25608 43898  4367
      28341 17447 25318 30810 26902 31281 11473 34737 47079  3232 35069 26386
      37363  8380 23914 33162 52234 15459 37122  8373  9398 47530 48179  7692
      35450 31497 18699 42711 49757  7687 51878 50541 11247 13265  3728 36594
      47269 21298 39055  3660 13223 14270 44422 13563 42109 29703 21918 19323
      22820  1616 20259  7449 13576 19533 11173 30131 21402 25322 48131 28141
      23080 39929  6685 11564  9443 33104 17917 25712 47641 22053 49808 25264
      21370 46978  5222 20548  6077 10295 33560 10013 28195 30337 44377 52083
```

```
      1762 13460  2069  6848 16998  9449 51700 21269 49640 28249 50726  2783
     26615 49120 34078 45327 21967 37285  6082  8770 23297 29310 19665 34459
     39662 14473 41294 17594 27263 43639 28707 47788  4047 27578 27281 35494
     11698 19537 41449 17818 51666 24721 49635 28914 13081 16853 17689 31886
     49619 29958 29708 11604 19512  1495 47194  4801 12623 10718 22775 38844
     15614 15129 30523 30326 16746 50812 21213  4573 23347 36222 37697 39401
     11304 20448 36934 30799 48692 47760 26906 18568 33498 38515 35815  9713
     18767 41049  5429  5394  5426  5396  5456  5431  5432  5453 15538 39499
     39243 39194 27163 39266 39213 39173 50114 50140 27167 30369 30342 29575
     22752 30373 50142 30503 30453 30451 30400 29610 22779 22778 30450 30481
     30370 30367 30346 29607 29541 29648 22783 30317 30505 22724 50137 22749
     30486 30397 30394 22782 30457 30341 29530 29605 30423 30508 30401 30424
     30421 30404 29535 50132 29647 30315 30456 30378 29571 22805 27262 48751
     40556 38329 36653 28671  4615 40811 40773 48073 33930 29314 49918 29315
     50832 49893 49915 50035 50029 50058 50013 50011 50003 50015 49983 49919
     49947 49976 49987 49978 50107 50066 50108 50104 50064 50068 29486 33924
     25573 49760 44426 39471 48169 11493 22289 12676 37610  5461 12056 41973
     42013 40128 51187 16634 16564 37671 16642 32960 15329 42779 36564 47562
     47051 14895 46009 39754 10026 22661 40837 22165 38749 25463 40009 42797
      3985
1236: 3652  4643 26458 33803 41145 25697 52417 49253 49513 23665 31050 27066
      3161 47362 42935 43269 14035  8058 15150  6876 16247 41643 27684 26810
     36432 30707 13862 37396 17363 50188 19630 26557 48691 17069  9112 20361
     38118 52015  8531 37655  8019
1237:12377 16336 45500 24215 36136 34507 33825 35204 25668 48487 16575 13408
     31411 35526 49804 33820 12687 31045 43751 41484
1238:38593 38093 31765 44225 51928 10527 26461
1239:22329 39490  5151 37988 25017 19737 11212 26565 22948  4538 23174 29921
     49764 46746  1735 18000 10125 44628 27514  6316  4391 31875 11678 35985
     51366 16246  5386 26413 38299 22600 36994 21961  8195 15035 45492 51165
     42944 37265 40353 26160 13416 14230 13158 25305 34697 24750 42337 49393
      1651 20298 51390 13713  3144 16991 19724 29212 47953 39134 52100  7211
     16125 36857 48153 19096 10315 46733  4286 28589 35775 39696 36297 36389
     12039 27314 19126  7245 32545 24030 46300 37541 49312  2511 33430 25526
     37358  6638 32540
1240:45392 52621 33099 23882 40800 46098 36256 46318 16581 12147 10195 40985
     48428  8006 32660 28826 46521 23748  9277 43524  5310 28866 22553 25074
     47030 34811
1241:30500 24025 33611 49951 21614 37487 31273 47029 30259 24638  9103 35638
     50741 39682  5795  6364  6653 18125 16099  7693 42920 42923 50669 51850
     19491 33052 48875 17115 48198  4558 17260 10430 39219 47267 34082 42468
      5062 41261 11104 34303 28404 47617 15034  9846 41237 16565 45077  6587

1242:39577 39576 40494 29567  7613  9075 37738 37405 51935  9910 17192  3739
     19520 42658 17099 23482 16137 25606 46162 21116 38384  6420 22924 34744
     40742 16098 11592 29673 34261 49803 30223 19716 39276 51954 44658 45223
     35150  8661 42082 21605 49586 47133 39733 39731 12280 37819 36758 19248
     13051 33062  9477 31919 28753 47619 37585 47442 34121 21448 35128 46532
     46603 37582 42860  8798 21290 15189 25368 46668 18162 47289 23986 18146
      9974 20931 25139  9386 46117 33958 37545 52189 22556 30204 42776 30758
     21303 42410  8521 10671 36120 43694 46802 14584  8391 29860  8190 37564
     49218  1709 35774  9394  9395 14484
1243: 1460 42457 47382 24447 39810 43632 47195 25846  6654 26403 39674 11967
     42872 17677 38787 22585  5715 33588 38644  3369 36324 34528  3809 34775
     37805 16194 21649  1461 12160 40634 51084 48348
1244:12823 50261 31564 16239 16256 16258 16241 16260 21371 14519 45011 13054
      5754 19081 32514 52467 32337 30130 26901 43149 16066 28972 17338 17327
     27142 27145  6386 40250 22643  5889 18057 11869 10347 39285  4596 26081
     20692 26586
1245:39602 41732 43325 10570   791 18983 17986
1246:14760 17162  5455 45793 11462 14263 37740  5242 22431 38804 34496 38001
     46633 23142
```

```
1247:42408 41632 50757  5179 38279 25365  3735
1248:46119 19968 31641  1463 22517 33165 44283  2950 38735 52341
1249:39128 12589 33514 37602 45400 30232 36676 22960 22957 26271 52162 10902
     48673 22918 40015 22759 25826 31448 11368 11365 37774 22990 22985 22954
     36669 32413  4276 15181 19945 39760 29746 29763 18094  8626 44016 11668
     19297  8908 45780 11008  5303  1504  1506 27658 35594 12394 12635 10353
     49230 47770 43024 16015 10056 17061 50284  5367 22906 22880 22882 25598
     41435 52305 45258 52308 25104 25087 44312 29170  2939 40587  4177  4175
     33799 23076  2752 24994 16113 18118 19018 51753 20340 38524 40699 21163
     34013 39841 31563 34038 45040  4124 20558 36648 32587 26149 38692 17049
     11859 11673  1633 49786 10929 17050 24832  3459  3550 22879 34283 37595
     37594 42931 27732 27735 22908 16638 25540
1250:35972  1573 23301 20045 33782 23077 39185 27364 23909 34715  6057  8194
     39552 15963 47858  6724 45186  4936 27781 35879 14064 14885 27724 46632
     46464 13306 15795  1964 37944 22422 22424 22416 22418 20868 23120 18649
     22846  9177  5823 28425 28689 52142 26337
1251: 5099 35463 32578 49421 45982 10568 25832 16387 10753  1988 30535 37134
     41364  7866 48522 20242 43974 38480 18175 25793  2349 13213  9385  8241
     14860 38923  1464
1252:29905  5099 41124 16480 39283 16387 10753 43655  8421 52423 41364 20242
     43974 22530 24246 13213  9385 23964 11237 40403 40612 47784 18602 16397
     15003 48689 28772 22525 24484
1253:38679 46357 38707 38709 38703 15424 15396 22001 45449 44022  1503  2023
      2000  7917 16297 51033 16300  7640  2618 38626 38623 38732 38688 38686
     51427 17545 18385 38486 40173 36809 46154 40767 26201 42126 47067 15428
     28526  3552 38658 38731 38730 38678 38662 38712 38711 38628 38629 38733
     38752 38751 38651 46373 46362 16632 42647 41605  7420 52069
1254: 5379 41266 26687 29756 35443 35444 12248 49848 34447  6992 47832 28009
      3853 48192 49662 39592 29658 29690 36987 42248 30751 11164 10497 13110
     50263 24471 40421 11920 46354 22526  6056 14217 35556  8555  6618 43034
      8252 33333 33335  8251  8423 10326 17724 47276 43063 47202 49737 35393
      9373
1255:34762 27647 15567 20630 36846 20455 45830 26370 25374 12268 40795  8385
     17230 10457 28905 22800 45237 51761 22958 28774
1256:21345 51624  7464 52551  5012 17976
1257:38907 45217 13574 14914 29876 42372 19016  1468  1494 35681 34550  5019
      8637 10883 11628 47490  5248  1472 17180 10412 42334 13358 41132
1258:14980  6945  6949 11580 11576 11552 11549 11573 11602 11555 11553 10848
     11529 11498 11523 11491 11488 10845 11525 10843 19116 11519 11626 11609
     11607 19119 19123 19169 19165 19162 52240 30316 19199 10783 10818 10821
     10851 11490 11494 11545 11584 11601 11606 11624  4857 50019  1742  1744
     52242 29962 29957 36415  5952 28699 10563 33656 29021 12423 29852  6859
     11810 12725 41381 21932 40932 10790 10752 10810 19113 10813 10781 10840
     10815 19125 19157 50032 50034 50042 50045 50065 36560 46023 40665 49777
      5251 52591 15610 24323 50017 50039  1714  1711  1712  1710 26841 26838
      1748  1749 48716 20386 12723 13400 19160 19608 26328 26318 20422 33842
     12406 41951  6314
1259:29533 31130 17088 34357 40842 30242 30455 14758  5463 47044 20799 12733
     42197 17896 38052 46087 46086 39663 45294  1766
1260:20379 35023 23870 52572 16929 45823 23377 35312 23186  5104 52314 33608
     46739  4792 39936 28080 21288 52188 44296 17860 19693  5011  5017 47369
      1798
1261:14725 20253 31259 37116 46899 43253 13959 10011 47640  1910 30619  8257
     14261 18846 13032  1137 43360 14005 45917  5884 13607
1262: 4449 23902 37429 11541 25933 34985  1471  7044 39902  9046  9048 17593
     22386 41945 20029  9214 41916 13538 22388 48173 26782 26700 42524 47295
     20244 39866   985 48977 35889  8106  5352 30789 18280 37717 36718 25511
     51081 45462  8944  4458  7600 10855
1263:23656 52582  9125 19425 49502 32795  7146 43205 12721 25836 19447 45779
      5653  3990 22328 46596 52288  7150 34728 30125
1264:19316 15166 49492 24624 21720 12318 24717 36241 12352 34094  6041 25077
     12311  5036  6150 14501 43175 35231 30422 12674 32667 33527  4361  4304
```

```
      47464 48945 24841 46084 29545  7879  5907 28959  9107 18665 24501 46243
      16874 28765 45528 19355 20786 21085 38293 16476 23796 30807 17330 31102
       3574 41274  1938 41498 35033 36691 51695 46863 46219 45489 47830 45764
       8324 51799  5720 15473 27716 52607 27613 45854 20049 13171 51474 38817
      32437 13177 35755 51272 26984 16197 44800 28925  6717 28465 25596  5832
      10867 35310  4197 28617 26416 39605 23692 52375  4061  4234 14488 40321
      23756 44688  7069 11277 44960 18942 48049 32469 22054 44187 33607 44575
       3191 32183  7350 49048  5532 41806 24320 29793 28680  7609 43372 42537
      25954 25957 48277 22809 42299 37253  6275 19409 23319 46355 42741 25873
      15081  6497 16717  3026 17653 51740 47787 49153  2927 46583 23386  4241
      19233  7034 35469  9699 33054 41921 22858 17008 27361 18382 16724 49525
      29313 14900 24703  8154 22866 38104 45648 44313 16692 32130 28978 13152
      19634 39329 39985 44467 31685 33668 52730 49485 44748 31162 52137  3390
      14330 36694 28958 20717 39944 30771 14979 12545 31157  5855 43113 37847
      23344 40896 39354 20485 19860 41475  9002  5905 28976 13150 52411 16978
       3546 48124 41673 13261 31178 41671 41667 52420 28952  7497 19410 26567
      13305 21858 13147 33073 13174 28643 38840 46368 13181 48141 12731 12940
      12943 39495  5317 24110 42679 16475 46485  7078 18376 12775 21056 25031
      11179 40860
1265:48688 40177  7641 47707 12686 23369 47074 51250 25953 29252 14198 14204
       9570 40447 40301  9749 20755 47065  2481  7198  5371  5353 41039 31645
      39397 48920 20844 20847 20852 20856 11548 50060 51254 10291 20496 35584
      34151  4099  9666 18297 51076 46903 45863 26817 20445 42746 52538 24884
      33634 38284 29446 49298 38361 18258
1266:25941 40766 10513 34646 39178 25677  5798 43246 16489 16458  9632 34566
      46469  9882 10596  9886 43582 34906 21854 44939 45478 22369 33449 39034
      44640 22464 18207  5126 32797  6029  9652  9438 23437 52212 23348  3225
      40201 22407 12450  6378  7102 43421 11439 50158 44248 50156 44731 46404
      46549 22377 33545 38596 50452 49514 31715 20282 33224 38393  2448 31173
       5140 18064  5339 30145  9664 51877 28766 33265  8719 23469 12669 26515
      39444 37853 11270 20970 39248 22698 23421 44955 42827 33946 50903  4719
      34457 48072 50294 45182 21309 31684 27048 24098 13726 47300 51216 48155
       2066  5323 20679 12245 45134 36733 15616 34997 29454 44974 15279 31654
      32416 22760 36144 49946 43298 52262 36541 42422 12029 23167 42546 32519
      26073 28651 50734 23350 33037 40583  9836 51182 19068 27767 29194  5425
       7263  5423 22090 37956 44134 35149 27951 29053  8340 11672 16119 19338
      43778 38916 37404 40670 29787 43978 37788 26055 42331  6552 47452 30102
       2641 18544  4653 35597 28367  5473 25631 37471 23517  7972 34302  3795
      36918  7505 33313 48350  5913  3419 29984  4348 38424  7243 48602 51603
      51837 14791 32427  3945 31473 26066 31900  6416 31394 26651 14309 32558
       3543 25630 32953  9947 11801 34358 11751 13630 22351  5740 19225 46657
      46035 39612  8912  8287 12104  6189 23247 18067 41946 11974 15492 38507
      15466 32792 22364 23074 28174 45370 21993 21257 33005  7844 17772 45905
      41839 19426 21979 29830 23993 36525 34240  8972 19287 19259 19907  2765
       7682 35540  8369 13687 47006 24063 10152 40402 26290 26968 45297 30196
      48973  7827 22977 36711 12247  3797 29358 25720 36407  5745  3180 35923
      13613  7804 16895 13575 23244  6530 23124 33975 33131 43642 43664 35809
      30693 51759 34851 13210 17264  6777 28204 30082 25160 10480  7915 40180
      43501 25650  7291 13302 17801 24923 33389  2097 21560 36783 23312 20716
      16147  2137 27901 28294  2434 21063 29590 13422 33848 20894 24298 36199
      49798 15839 16603 10099 38567 37759  8837  1267 43742 19662 12141 44345
      31822  5894 19960 50067 11455 39804 19505 37749  2997 43704 50481 28702
      37141  3531  9022 12334 16545 16535 32346 32368 32347  5258 42356 32366
      26820 26822 32445 16474  6745 31888  7334  6753 42664 11246  2677 11146
      37691 48253 46560  8453 25083 29048 13632 17409 44197  2563 22064 35368
      21389 13040 35758 46320 51415 12120 12118 12137 12117 38926 47003 36213
      46765 18989 12139  8100 45903 43232 10443 42189 15365 39428
1267:25941 40766 10513 34646 39178 25677  5798 43246 16489 16458  9632 34566
      46469  9882 10596  9886 43582 34906 21854 44939 45478 33449 22369 39034
      44640 22464 18207  5126 32797  6029  9652  9438 23437 52212 23348  3225
      40201 22407 12450  6378  7102 11439 43421 50158 44248 50156 44731 46404
      46549 22377 33545 38596 50452 33224 31173 38393  2448  5140 18064  5339
```

EP 2 484 769 A2

```
30145  9664 51877 28766 33265  8719 23469 12669 26515 39444 37853 11270
20970 39248 22698 23421 42827 33946 50903  4719 34457 48072 50294 45182
21309 31684 27048 30274 25701 49109 17002  1486 24098 13726 47300 51216
48155  2066  5323 20679 12245 45134 36733 15616 34997 29454 44974 15279
31654 32416 22760 36144 49946 43298 52262 36541 42422 12029 23167 42546
32519 50734 23350 33037 40583  9836 51182 19068 27767 29194  5425  7263
 5423 22090 37956 44134 35149 27951 29053 25563  8340 11672 16119 19338
43778 38916 37404 40670 43978 37788 42331 26055  6552 47452 30102  2641
18544 35597 28367  5473 25631 37471 23517  7972 34302  3795 36918  7505
33313 48350  5913  3419  4348 38424  7243 48602 51603 51837 14791 32427
 3945 31473 26066 31900  6416 31394 26651 14309 32558  3543 25630 32953
 9947 11801 34358 11751 13630 22351  5740  5722 19225 46657 46035 39612
 8912  8287 12104  6189 23247 18067 41946 11974 15492 38507 15466 32792
22364 23074 28174 21993 45370 21257 33005  7844 17772 45905 41839 19426
21979 29830 23993 36525 34240  8972 19287 19259 19907  2765  7682 35540
 8369 13687 47006 24063 10152 40402 26290 26968 45297 30196 48973  7827
22977 36711 12247  3797 29358 25720 36407  5745  3180 35923 13613  7804
16895 13575 23244  6530 45647 23124 33975 33131 43642 43664 35809 30693
51759 34851 13210 17264  6777 28204 30082 25160 10480  7915 40180 43501
25650  7291 13302 17801 24923 33389  2097 21560 36783 23312  2137 20716
16147 27901 28294  2434 21063 29590 13422 33848 20894 24298 36199 49798
15839 10099 38567 37759 49873  8837 19662  1266  2894 39428 12141 44345
31822  5894 50067 19960 11455 39804 19505 37749  2997 43704 50481 28702
 3531  9022 12334 32445 16545 16535 32346 32368 32347  5258 32366 26820
26822 42356 16474  6745 31888  7334  6753 42664 11246  2677 11146 37691
48253 46560  8453 25083 29048 13632 44197  2563 22064 35368 21389 13040
35758 46320 51415 12120 12118 12137 12117 38926 47003 36213 18989 46765
12139  8100 45903 43232 10443 42189 15365
1268:48137 29905  5099   742 27992 22396 17613 35987 18330 17298 44893 16172
37544 50212 34971 25832 16387 10753  8421 41364  7866 20242 43974 13213
 9385 26860 24422  8150  8151  3357 22496 11819  7205 20318 51540 10222
22493 38718
1269:26245 28520 11871 22399 50604  3443 25155 48467 20366 49373  3051 29938
29971 47023 24629 15804 17713 23154 51596  5418 22860 40295 14875
1270:40164 51848 12600 35088 45348 10909 51110 26155 26156 18604 24879 42471
24782  1528  8580 14691 28422 46527 28443 28445 37812 22802 46003  1271
 5406 18238 12715 38990 16133 40918  5747 34516  9397 12557 25178  1133
 1132 25503 45372  5529  5999 12226 47738  5509 12224 41382 46910 38335
43332 43330 24739  6032  9671  8874  8903  3159  8905  8871  5995 23980
29224 47752 47732 29222 23765 29031 29029 29027  5998 29024  4993  4994
 5403 22234  9802  9419 45350 17080 48541 48542  9175 17578 34957 30459
23132 39993 13675 28271 14091 38492 18822 43327 45347 48254 22326 35553
35579 35581
1271:51848 40164 12600 35088 45348 10909 51110 26155 26156 18604 24879 42471
24782  1528  8580 37812 22802 40918  9397 12557  1133  1132 25503 25178
45372  5529  5999 12226 47738  5509 12224 41382 46910 38335 43332 43330
24739  6032  9671  8874  8903  8905  8871  3159  5995 23980 29224 47752
29222 23765 47732 29027 29031 29029  5998 29024  4993  4994  5403  9802
22234  9419 17080 48541  9175 17578 39993 30459 34957 23132 10390  8282
11963  1270 19216 38258 37800 21635  1557 28271 14091 13675 38492 18822
17240 18598 43327 46656 40606  6899 45347 48254 22326 35553 35581 35579

1272:52347  5553 22575 40507 11699 40380 22495 37683 38491 41549 47685 47689
35757 22019 18171 29719  7572 21877 52660  6442 42019 14333 21640 36014
28388 16355 42671 44558 35559 40437 42320 42973 33172 52673 37438  5026
38244 15199 24188 39963 50742 33242 18334 13285 14344 33483 15361 33543
 2382  7143 33235 38047 35778 38418  6209 51874 39559 26478 21766 13579
36547 36571 13462 38618 38620  2904 26770 25971  3805  3806  3824 18596
19936 19958 16220 27135 41069 17305 40270 40271  5058  3223  8742 24217
48957 42133 28504 12648 46211 37744  9080 16110 28825 38174 38178 43838
 5628  4357  9480 43257 24473 49256 44414  9491 19581 29030 31508 31671
32773 37302 19998  6522 43321  5850 40408 40388 13526  5438 17381 20586
```

175

```
        37882   4386 23849 15794 45179 30426
   1273:38365 32940 25450 26178 33582 29696 18052 25150  2526 14842  4606 24738
        18410 29676 51155 18017  7838 40641 12323 45359 16364 32593 42666 14115
        37012 37014 48981 52163 41640 34064 48147 51860 48132 48134  7652 51996
        48631 14275 51718  1701 41268 42747 28433  1907 28990 23389 25002 19845
         6684  2158 30328  7807 12972 41464 16409  3344 20902 24526  3340  5141
        26236 13094 13066 33372 18935 18430  5966
   1274:24271 41894 13098 39431 39564 31385 24859 14999 44287 49241 27125 51785
        49759 34821  4379 28777 13717 25981  9267 27863 33872 40938 13116 40153
        22080 37377 37379  6817 39521 15343 20008 18347 36869 33522 15786 15784
        26507 34027 17518 10076 10081 51616 51092 15398 36697 22415 24362 49601
        52369 47197 34930 37235 24311  9502 44938 44993 21620 36896  5033  5034
        12707 22590 37330 11575 42612  4497 31474 19933 45129 24505 18987 36755
        24787 24921 16993  6085 35993 32651  7428  7456  7669 48733 32814 22567
        22708 49595 19681  5408 10431  5384 40441  3013 29275 23631 29276  5921
        16845 18883 26483 19650  5922 16848 30434  8085  8945 30576 31433 27374
        23861 42515 22523 10853 10856 18613 29056 45047 31360 43264 39380 31539
        31540 18041 48949 19644 12078  9382 44514 37123 44518 32560 31612 11679
        21878 41076 40991 48501 40992 48502 50221 25790  9776 34680  5063 39832
        19477 38198 37183 37184 20549 48078 29267 29383 18848 11425 18945 48075
        34361 33574 16755 48419 37345 37510  9899 43516  2471  5937  7431 33972
        37434  9343  6287 47577 24596 27014 27802 40094 46723 16645 39708 40754
        21008 23175  3123  5971  1823 20003 12895 10070 26253 20237 46412 27339
         7859  7860  4196 42405 30087 47353 30509 20957 22091 41580 41583 20864
        26206 31737 34488 34490 34547 34549 19401 34191 11194 21221 40946 20185
        25972 24273 35174 18124 13815 52231 17278 43112 43973 16444 44865 43971
        31124  7524 22652 12990  7322  3307 25589 34592  9054  9055 42913 24854
        29035 13495 18031 11378 28748 44937 49724  5810 47163 20030 33927 33180
        13280 51046 29320 23579 28901 17571 39272 14434 33057  1727 50936 44966
        28897 51471 23340 52019  4301 46353 34555 51703 40548 14460 35152  5481
        51295 48685 33818 40110 31363 47597 52260  7540 17967 18358 18380 33980
        13327 12884 13844 49087 16811 19325 37496 36639 21748 21750  3612 29845
        12510 31456 12297 44324 25358 28814 51433 13476 17949 44374 41186 43635
        17125 26925 19961 19552 44980 29023  2070 40498 22926 43444  9849 22193
        22194 30025 35349  9819  6320 23204 48148 44229 37401 31669 43834 50851
        28076 30769 41740 49712 46828 47700 15881 40659 40660 31208 12699 34789
         8232 41557 51712 28999 51096 47212 47774 50023 22342 44611 41947 21190
        14353 39509 41764  7895  7898  7856 29265 32126 37090 20458 23168 23310
        38633 42622 49570  2540  5244 48887 28233 33244 18237 33795 45060 42967
        45782 44280 20364 45964  2642 25772 39308 44319 52663  8652 34918  5667
        45565 18672  1492 42898 26621 31697 46297 32152 17857 47550  1675 27582
        27415 32140 34944  7474 36751 38463 29600 38413 46547 50319 34665 49569
        29121 52021 13983 36791 34324 36788 18452 41114 12804 17016 34688 34742
         6623  1555 23546 16700 21608 35747 35749 47324 12410 13601 18808 46134
        28096 28022 51133 13445 32645 43121 23443  3991 35259 10285 10931 19622
        10933 10288 11023 44293 44295 15655 14469 15218 19200 36935 41514 39259
        26543 32798  3484 14559 26867 11296 43002 19690 43007 47880  2869  4730
        35032  8166 36153 19393 14535 32047 26131 29688 25675  3356 26332 15024
        15025 18368  5527 30187  5530 12309 21093  4691 24898  6154 32097 26007
        40424 29206 10264 49305 22237 39026 45845 16459 22254 19687 22257 40045
        46303 51487 44436  9338 44439 16463 44437 52035 52006 30731 19680 26620
        40175 51380 48133 11133  9448 28400 51333 29331 29329 18389 19749 16643
        23201 19647 25341 26842 27231  6740 47790 47791 19636 19684 19642 37381
         1657 13610 36792 36410 15771 44119 16550 14542 14536 14520 13909 13895
        13868 14524 14517 14627 14624 14595 14592 14671 14619 14539 14534 14629
        15520 14669 14590 14701 14568 14566 13941 14656 14569 14621 14738 14729
        14593 14703 13935 14698 14694 15564 14732 16532 16467 16577 16576 16586
        16461 15534 15531 14775 16470 14772 16471 16530 16610 15594 16503 16498
        16552 16549 15753 15727 13867 15524 14659 16615 16528 15635 15601 14515
        13899 15713 16506 15763 15668 16465 16448 16547 15759 15630 15562 13864
        16495 15692 15660 13906 16548 15721 15719 15666 14747 15696 16449 15605
        15642 16443 14769 15628 16441 15633 15718 13903 28563 26409 27574 37762
```

176

```
         37764 46894  3685 37105 46643 18938 30427 21112  7154 42142 49613 25040
         49632 45076 36451 35952 37679 19448 19648  7409 42584 29179 20473
    1275:29362  2486 16935 37192 20038 19714 13981  2352 50353  4481  1665 23854
         28293 29754 45071 52442 21913 29366 13454 15996 33655
    1276:21375 35067 37779 16102 36421 10135 24928 22596 47488 31263 13776  8276
         30009 31037 10114 12492 38453 52669 37546  8491 13848 10170 25073 33213
         49925 38085 49922 10324 49921 15577 19514 11813 40479 50372 12034 36922
          8172 25641 40064  6915 50075  8772 45282 27359 34850 28223 25090 51016
         17373 22728  4927 17587 12786
    1277:33904 29907  5272 29389 10502 36123 42668 21740 17355 26887 46189 31455
         44192  1844 10732 27479  7930  5525 20553 34799 45146 16976 12342 22008
         23396 50184 51437 15540 40618 41765
    1278:36610
    1279: 8811 18934 26761 48813  1614 45112 29334  7861 51932 39935  8466 20130
         13392 30867 34849 45848 42706 42801 34090 34093 34130  6539 23280 44218
          3992 21108 26147 30049 13084
    1280:35215 40915 51942  2075 13500 43138 33279 20329  4655 30537 52684 52683
          1916 12262 15776 45320 49231 18683 49181 48463 35265 49159 31431 41648
         20541 44314 29913 21035 49095 37985 19012 34810 26118 32705 20295 22790
         52534  5407 24347 40962 52153 39083 34092 34704 36993 33725 33987 17090
         42307  3637 15797 31881  4239 38843 51024 50237  6346 26754 45511 17550
         21215  2915 29094 22155 33423 26935 45534 12487 51080 44404 22018 49538
          9988 27106 50266  2093 35387 35998 25672  2882 43273 43266 12907 27974
         28968 35698 18456 38780 39349 32961 52710 44370 51215
    1281:48462 46927 44975 39368 12409 35554 29235 23224 32293 38534 19734  8984
         22543 35261  9680 23346 38265 15576 49114  9471
    1282:    0
    1283:10055 43828 37843 51530 47046 17367 33758 10096  4036 10052 10086 10089
         10092 10121 40810 26591 26596 26590 49132 49136 49133 47895 37277 11105
         11103 10411  8810  7855  3580 47064 23520 23871  2207  2874 25942 29246
         50500 30344 24918 39058 52519 24018 41590 19583 27900 38309  3987 43204
         29889 10115 10083 12969 16726 15977 16007 37846  9729 18463 33934 18420
         40567 48929 19063 30831 23376 17119 24006 34476 10019 10241 10240 49196
          4575  3623
    1284:    0
    1285:41575 17357 11267  5266 12530 34554 17154  4479 35177  8612 34342  9881
         32517  6849 30221 48734  4819 38378
    1286:    0
    1287:48150 38494 40017 35955 36029 22815 44570 50291 50258 50262 50256 38794
         24663 39803  8974  9021 37525 41434  8123 34553 34695 47829 10010 43058
         17739 31505 35567  6452 20819 22092 39702 32607 19804 36828 23543  2068
         42739  3334 22176 42293  4328 51811 11380 37495 17184 32515 26092 26090
         18799 52627 29003 29142  5537
    1288:    0
    1289:49417 11486 23726  4123 14913 18971 46082 41800 11055 27830 25757  5092
         34632 30719 40580 28551 49914
    1290:34556 33781 39144 37293 37397 12300  6710 10619 47767 14437  5390 49559
         17444 32891 40352 41207 45099 30646 27257  6352 15837 48774 40347 48296
          6363 19207 29795  7435  1652 51069  6219 26579 26679 40148 40249 36761
         37492 35861 25201 46669 45983 20898 16925
    1291:40332  9271  4436
    1292:31918 19170 46208 49316 49937 48657 41419 51035 46690 44679 48800 35059
         23463 33884  4358  4789 43499 10550  4566 11302 22546 22803 11405
    1293:    0
    1294:10844 36713 43986 10902 51757 51756 36650 36735 36731  7519 28751 22759
         25810 36737 36739 36762 36743 36710 10785 52177 36706 44020 36770 36771
         32413 19945 48196  6779 18916 12273 18095  9040  9041  9043 49624 13038
         44016 16272 16270 11668 11046 11050 10441 19297  8908 45780 11008 37426
         36637 12192  2673 48758 34063 35594 49412 12635 29311 41406 12189 12140
          6803  6806 12162 29637 37586 44312 33799 10417  5091 18571 12164 12158
         12171 11695 15086 15071  6050 12193  6809 12194 12197 29441  6805 29417
         12867 32146  3688 15192 23042 32625 32609 31215  8458 10829 10825 22502
```

```
        39088 43343 17049 45559 35679 35675 21270 21284 11673 11692 31746 15906
        22132 18912  7834  7826 18651 10929 10927 10926 10924 11016 27407 27384
        16299 24832 18930 44159 44155 44153 16638 12168 24507
  1295:39128 37602 37606 37638 37624 46285 36672 36674 36676 22957 22960 43986
        52162 10902 26670 51757 51756 22918  3839  3819  3836 36735 36731  7519
        28751 25810 52025 31478 42654 22511 36737 36739 36762 36710 11368 11365
        37774 22990 22954 22985 22914 22911 10785 52177 44020 36771 36770 32413
        48196 22984 22951 22949  6779 18916 12273 16862 39732 18095  9040  9041
         9043 49624 13038 18920 44016 16272 16270 11668 11050 11046 10441 19297
         8908 45780 36652 42347 37426  5303 22124 12192  1504  2673 16223 48758
        34063 35594 39130 49412 12635 29311   839   836 44344  7410 10994 45298
        39124 22906 22880 22882 40062 25598 41406 52305 25968 12140  6806 12162
        29637 37586 39695  2945 19661 10123 33799 10417  5091 18571 23076 23431
        50365  2716 12164 24994 24992 16113 46289 18118 15086 12194 29441  3688
        32146 15192 32609 27683 31215 21286 50352 37758 22502 39088 43343 37757
        28238 23017 38692 46314 46331 17049 45559 35679 35675 11673 31746 18912
        18651 43350 10927 27407 27384 17050 16299 24832 36766  3487 22879 22908
        16638 12168
  1296:39128 45606 37602 37638 37624 37606 26905 45400 36672 36674 36676 22957
        22960 36713 52162 10902 48653 51757 51756 18126 18155 22918  6907  6235
        40015 36735 36731 28751 22511 36737 36739 36762 36743 36710 11368 11365
        37774 22990 22954 22985 22914 22911 10785 36669 36709 36706 36705 36678
        36771 36770 32413 23675 48196 22984 22951 22949 27174 23063  6779 12273
        16862 39732 18095  9040  9041  9043 49624 13038 18920 11668 11050 11046
        10441 10433 10436 10440 19297  8908 45780 36652 42347 36637 37426  5303
         1504  1506 48223 48222 31509  2673 48768 34061 48758 34063 16223 29527
        35225 48760 39155 35594 39130 49412 26703 26701 12635 29311 11414 14541
        40502 14280 29877   839   836 44344  9675 17456 15028 29192 43617 43562
        27742  9174 13698  2011  7410 31330 10994 50306 50421  6699 45298 39124
        22906 22880 22882 25598 49961 49959 43211 17007 41406 52305  2212 52479
          841   840  9735 19149  3006 47806 37896 11741 22115  9571 52179 31807
        46816 15566  6806 29637 37586 25104 25087 33471  2939 16964 16963  2945
        19661 10123 19210 45536  7760 33799 23076 23431 50365  2716 24994 24992
        16113 52272 18118 15071 31874 32888 42645  2591 18871  1623 18810 15192
        20558 23042 32609 32587 36648 27660 31215 37758 22502 39088 43343 37757
        28238 23017 38692 17049 45559 35675 35679 11673 31746 43618 43476 24440
        18912 18651 29434 29442 29437 29439 10927 27407 27384 35982 24832 36766
         3487 22879 37595 37594 22908 16638
  1297: 1342 16910 28698 40090  7681 27189 42506 30518 37798 50749 22362 16687
        17146 13426 14252  7165 33569 49969  6244 20834 17951 49884 50635 49594
         9109 31279  7534  6345  7769 20680 39483  7733 36559 39227  9423 18834
         6172 28659 39725 51525 42146  5245 31298  5378 35000 14384 52674 47529
         4683  7345 45161 51546  4627 35881 42554 17037 41653 48684 19745 38966
        26879 40824 27188 26859  2374 28773 10842  1996 18314 33076 25711 26962
        33327 22670 50001 47690 39156  9922 35302 39184 46722 42359 38920 41701
        34580  5301 17349 44850  8149 48191 45880 12322 33875  6628 32285 43023
        44602 45240 23687 27851 24653 19288  3426 33121 44481  9340 17418 51731
        42303 27037 26485 51952 12138 49843 41531 25399  5344 39742 43474  2625
        46325 41325 32813 41347  7854 26856 34514 18601 26837 17647 37080 16065
        13351 36013 37291 11448 28173 52078 50316 15233  3643 17388 16433 48036
         5050 24190 14048 31392 31591  2301 19974  8030 18494  1334  7220
  1298:47010 49254 26047  7116 35390  6091 32954 30430  4016 36724 46659 29894
        35578  7107  2133  6815 11476 38544 40318 25308 17093 24952 31307  2260
         2258 36532 35713 50655 50558 50632 50623 50629 50590 50668 50659 50661
        50523 50688 50664 50528 50583 50553 50524 50551 50594 50526 50548 50555
        50621 47466 47462 10609 28923 25332 28384 23819 14470  4931 14957 16152
        44855 45562 19414 30879  3603 21073 23053 40226 16648  2806 43193 37462
        49244 41372 41368 41392 41351 41389 31435 42493 42463 42462 42489 50170
        32150 32112 32145 47870 13357  7465  9640 14983 27871 38373 47516 27038
        37766 38460 29295 34141 37948 10600 28245 43401 25958 25961 17994 15358
        24328 22791 25848 16683 22071 32556  3093 46309 16128 31069 23851 16161
        31148 15647 20138 26954  6394 51959 41293 20608  5294 23388 33234 24420
```

```
      41340 22710 26613 30730  3496 11809 22205 16709 51673 17005 26010 26005
      25921 24220 36227  9117 37725 10917 28622 51335 22167 40405 45842 41356
      42333 47216 28148 31568 32947 11080 35090 49967 35202 40576 40736 44507
      24235 21533 29577 44359 50163 25531 42591 16432 40430 19881 18177 36576
       9240  9017 50000 40725 18548 30571 43507  2220 51065 33928 10401  7686
      13891 50146 14567 43677  1966 35959 10916  2020 18645 38648  1662 31692
      13793 50890  4646  9914 52043 43291 49822 49018  1740  5285  5286 47722
       7520  8834 14465 16960 14185 21710 48868 27563 48512 49278 10541 27881
      51725  6451 34162 35770 42093 42423  7344  8377 15543 10771  7232 17706
      52064 16762 13048  4213 33706  9516 35665 36874 42475 21137 26559 19253
      48971 25143  5041 32132  7899 25209 25223 25446  7670 21058 25952 35081
       5297  8851 21861 37197 40359  7042 40438 46166 48062 44384 11760 11861
      39113  2281 12800 18875  7370 24585 32400 25476  3289 44885 29304 23459
       7514 49840 11738  7676  1729 48270 28653 27463 34271  2558 42310 41833
       4349  4365 35528 33507 33571  9772 13578  3939 38936 26850 43380 19110
      34326 42660 47671 26672  2230 45123 23252 52156 21981 13619  4723  4724
      48611 12279 12284  3418 47717  7072 21962 12429 12458 12434 17769 19379
      48391 40467  3727 49440 36720 38440 29054 51552 31920 13813 30286 41459
      17836 21668 21598  2620 52080 21589 17317 17319 17322 17315 28939  4770
      34994 34591 20550  5480 18718 52345 13463 46476 16063 14786 13519 41548
      31462 35661 29244 17419 36924 39481 16531 26439 37045 10723 50679 20325
      21275 37656 51542 19144 12208 29764 18012 14610 14612 49881 35432 25528
      43304 22514 36200 46116 30287 18545 43489 26682  7550  7545  7547  2867
       2366 45219 14140 11621 26240 44873 43255 19473 45224 16901 50446 12432
       8475 44805  8170 33356 47589  8178  8174  8432 15384 35052  9072 25584
      25587 25557 25576 25559 25553  4798 22521 22519 48507 13797 24488  9164
      33465 49407 34203 29863  4364 15128 26372 12834 29099 51660  4083   747
      40891 36313 12000 47323 14719 20860 20891 20858  2987  2989 29204 17141
      45029 41177 10719 21169 50033 14226 11816  7672 24541 50236 28513 27790
      52000 25498 30468 35893 35900 41057 41544 19069 10120 20578  6821 11862
       8436 16097 16067 38977 33789 33354  7033 16883 33765 33783  6997 33759
       7029  7021 11515 44376 11516 34839 49396 12172  7048 51400 11700  5789
      15476  8503 28382  8573 46000 22355 40545 22516 32335 38475 41523 15357
       4127 42855 15136 15186 42786 39616  1952  9005  2016 30084 22547 22552
       6698 14398 50946 47905 10467 33248 23900 46820 46377  4366  6194 47148
      35345 41814
1299:  6611  5674 51904 32303 35615 43734  2729 10695 25876
1300:  7259  3131 16754 16759  2564 46567 31528 22512 45260 52197  6903 16224
      15596 15110 23299 36473 49233 49213 13832 33621 13572 14667 44139 43591
      50802  3689 13113 11315 15984 25131  5067  9949 18796  6553 48311 52033
      50542 24543 21559  7559 35830 46491  6925 23226 51490
1301:10844  5084 45400 30232 36672 36674 36713 52162 10902 26670 39164 51756
      51757 40015 36735 36731  7519 22759 22511 36737 36739 36762 36743 36710
      37774 10785 36771 36770 32413 26146 48196  6779 18916 12273 44016 11668
      11050 11046 11008 36652 10906 37426 10923  5303 12192 35594 49412 12635
      51593 19382  9012 33303 50533  3402 21246  4779 31355 15956 12504  7410
       7138 25598 42284 43840  1393 32868 12189 12140  6803 12162  6806 29637
      37586  2941 31970 33799 10417  5091 18571 23076 45945 12164 12158 18118
      12171 11695 12193 12194  6809 12197 29417 30941 16046 14945  4124 32609
      32625 50352 31215 21286 31216 50355 30007 50368 50369  8458 37758 10829
      22502 11673 11692 43476 43618 24440 15906 22132 18912  7826  7834 10817
      18651 10929 27407 27384 17050 16299 36766 36767 16638 12168 13857
1302:45305 45308 16171 10582 46264 47033 46967 47038 47113 46269 47117 47115
      47072 47070 47137 46296 46266 47139 46986 46985 46317 16412 10577 45859
      25815 46014 41874 16939 36009 45676 31576  3488 42259 31606 24357 22029
      43614 33421 47697 49836 46463 21046 45591 28503 26205 52065  8540  3850
      17878 16211 21412 15178 31275 31289  6939 21671 38723  5330 37590  8721
       3849 10857 13684 23772 50343 30777 23928 27092  2411 17405 20007 47161
      24335 28211  3500  5935 18710 52440 19816 52143 41291 35674 46647 32103
       8752 32448 29568 16560 20666 45786 37970 52603 44171  4794  2064  4836
      20271 49711 38887 27747 33283 51840  8804 35031 52401 40326 11954 15962
      16870 37508  8351 39628 32741 33375 22907  1582  7453 23188 51514 26338
```

```
      26742 46011  7084 30258 14555 28964 28962 50630 34311 27410  4894 47018
      47820 41722 17568 43976 27750 10591 38777 36175 40397 31237  6606 25224
      11792 47602 39999 42790 37708  5947 40489  8704 46340  9456 19492  4815
      51865 19932 32331 51634  9838  9738 27192 49365 47153 20122 22045 14599
       5809  2747 20089 15650 11709 36927 42822 30586  4811 49480 39741  5635
       4696 44841 35298 35111  5123 47173  8434 42246 38124 33059 34960 14507
      38296 48052 13725 43475 21487 39549  9301 14172 44440 16314 17544 14262
      26341 44812 42601 22649 26268 18469 14844 27026 36090 50425 44818 17344
      10095 16519 21103 24373 15639 25492 36629 31507 45133 20059  8664 35667
       6594 28469 40055 38409 17194 40515 15904 43458  1490 27229 32762 10028
      41615 34683 36336 40172 47156 51873 12150 14917  8099 29912  2496 51299
      35944 37501 15019 38924  4729  4748 13504 13570 24565 37558 25071 34735
      50487 43111 46487 14839 11878  2028 15779 44667  2728  3626  4393  5542
      41836 41309 35771 51639 48061  9965  9968 22451 21701 21099 49769 12387
      46873 13640 11444 52148  6756  5400 42298 24648 12552 24667 29033  9693
      18820 13080 25392 48676 20097 27704 34803 22414  9982 30428 10948 24913
      25780 21250 50980  8127  5274 47059 29211 11167 36938 15676 40297 25152
      37581 10042
1303:19028 18216   984
1304:    0
1305:40111 41318 46280  3788 44409 25638 18336 46181 52049 31005 27387 22192
      17377 32528 45434 40219 38564 38066  7779 21938  4707 19545 39333 16903
      35754 39207 17057 14787 22052 32254 47519 32557  5911  2552 35682 12722
      20092 38297 52352 31982 33876 24280 35840 31593 24937 12989 16325 16382
       9784 33438  1891 21910  5428 30325  7193 17780 38187 49860  8137 52456
       1001  1632 35279  3971 31711 33208 23206 29790  4750  8943 30608  1869
      29228 52390  4922 51072 48605  1000 18525 10652 11400  2032 48934  1522
      29606 31726 26096 15312 11083 11014 21314  3906  9638  1378 16967 47846
      50021 11735  5410 49736  2327 42182 27970 11721 34546 17570
1306: 8021  9433 43154 38938 38171  9728 29032 14016 51391 37213 47422 50717
      32078  2151 29301 31510 33360 38456 42887 37630 24580 32084  4440  1822
      19495 36647 28536  8815  5805 47084 30359 11599 14386 26174 28085 43220
      43394 14289 43000  9258 44811 37286  2231  2042 28050 43259 34774 14546
      19989 29620 24702 32592 50253 16917 42980  2135 31777 36246 20335 22301
      25627 12175 39327  2507 11089  8488 47658 30616  9492 29538 32369 49398
       1943 13427 17939 27288 11772 21946 43644 42615  7839 14935 15067 19107
      26412 14143 14159 46104 30069 29731 33516 33554 13656 27800  9527  6071
      42294  5171 30750 32056 16058 47598 24641 43503 40642 17844 52723 16962
      42499 49369 51994 46292 26898 33466 52098 18384 42361  4006 33255 14588
      26583 41892 51724 44664 18584 17913  7532 31198 22658 22686 20452 22688
      22689 24953  7308 44371 33287  2769 45552  9535 21756 43176 35975 25849
      20450 11275 11274 37150 48045 33281 10303  9414 12052 49118  4630 51284
      21630 50557 32644 44413 14107 37127 33847  2227 24813 11661 34708 21096
      29503 29517  8047 46717 35853  4340 15979 10143  8723 23574 28436 22945
      14420   829 33463 44347 45871 10589  1060 34525  1014  5741 30334 37040
      47333  1059  7705 24531 36325 34111 23217 52326 28162 48233 43188 43184
      17051 22876 24568 36946 11352 11355 52579 11351 43706 34755 19873 19850
      39661 19870  3164  7598 15276 23054 30018  6990  6961  6926  7053  7059
       6994  6923  6289  7056  7017 23295 49875 39052 41048 39050 15041 41042
      15008 45243 11454  3256  3254 27452 27459 48443 36537  8660 48529 52617
      36221 13231 10957 43818 31071 16416 29480 50910 35907 22528 40107 18591
      37069 40203 12689 13258 43596  9576 50780 49990 38710  9460 51706 40336
      42994 13278 16403  6358 37055 24540 24794 42996 12395 42965 42937 42871
      42932 42961 42958 42885 42848 32722 30972 23436 50150 42396 13658 13659
      40826 48519 46909 10966 41771
1307:19021  4582  6258  6260 30070 28395 33791 32013 11918 11632 40390 40362
       4019 26287 18122 48530 20002 47180 11610 15527 27646 38077 40314 50026
       3803 21119 48983 24178 49835 42550 21132 40189 40193  4318  6729 36089
      51532  5446 10244 43994 40368 33901  6542 24294 31495 39524  3631 12419
       4659 10418 47780  4837 34718 36635 29593 49720 10015 16367  8365 46607
      41212  1021  5939 41911 25370 22969 50361  7197 44300 10877
1308:52091 46546  7897 30892 21149 31561 40037 10148 39019 25045 15144 48071
```

```
        2959 23239 27060  4513 19949 47423 19229 11853 40994 12203 32516 37027
       17345 40240 12720 14200  3932
  1309:26619 48186  1310 10567 45617 11848  4068  4071 17560 17558 51280 11147
       45436 28158 45432  6151  6349 51070  8656 25656 20512 34879 21206 13397
        7425 42427 39033 19970 16428  4319 25159 24218 21441 38341 27659 23690
       20884 44122 18256 41280 41283 49571 22201 10188 44087 21879 44083 17859
       23087 34260 39967 38862 39634 45246 46031 23564 37936  7190 15878 44430
       46762 22118 36836 31457 43550 45809 46573 14037 28855 38447 47231 22654
       21890 45054  9996  4698 39022  9929 19327 19305 49128 23676 23012 23187
        8789 12121 44068 41089 39147  6652 36025  2732  1870 47980 49507 21118
       47893 10700 33879  1599 46423 16092 25447 46090 32984  1051 21416 32525
        3801 25939 48174 52606 16081 31460 30385 51327 14112  7225 16656 45785
       41551  3515  3517  3490  3518 20235 31530 29501  3658 42662 49185 37483
        7137  1911  8418 33503  1885 24291 19632 48040 20082 33566 32085 41193
       42928 38821 15730 21654 46037 31187 13291 40758 27775 16430 29820 35622
       15847 27773  6003 42585 22838 22863 41002 28134 22255 43177 29850 50529
       35420  1953 40234  7869 16682 45375 29518  6932 20691 35459 28169  2278
       33696 14757 29335 38828 11591 17595 44372 42508 46976 16202 12402 13390
       44286 14905 51129  7400  4164 51801 52485 18202 28971 33948 16731 36347
       39301 10581  1470 12591 42365 32209 16269 34352 25260  3163 36620 18157
       32628 20062 28802 28909 38059 21777 23464 36486 42640 26331 45998 20343
        4846 14931 22520  5487 34101
  1310:26619 48186 45617 11848  4068  4071 17560 17558 51280 11147 28158 45436
       45432  6151  6349 51070  8656 25656 20512 34879 21206 13397  7425 42427
       39033 16428 19970 17398 25159  4319 24218 21441 38341 27659 23690 18256
       44122 20884 41280 41283 49571 22201 10188 44087 21879 44083 17859 23087
       34260 39967 38862 39634 45246 46031 23564 37936  7190 15878 44430 33971
       32279 46762 22118 36836 31457 43550 38793 45809 46573 14037  2205 28855
       38447 34939 47231 22654 21890 45054  9996  4698  9929 19327 19305 49128
       23676 48798 23012 23187  8789 12121 44068 41089 39147  6652 36025  2732
        1870 47980 49507 21118 47893 10700  4633 40604 33879  1599 46423 32984
       46090 16092 25447  3801 25939  1051 21416 32525 48174 52606 16081 31460
       30385 51327 14112  7225 41551 16656 45785 38504 47936  3515  3517  3490
        3518 20235 29501 31530 42662  3658 49185 37483  7137  1911  8418 33503
       19632  1885 24291 48040 41193 33566 46387 20082 30237  5346 42928 34589
       32085  6162 38821 21654 15730 46037 31187 13291 40758 29820 27775 16430
       35622 15847 27773  6003 42585 22838 22863 41002 22255 29850 43177 28134
       50529 35420  1953 40234  7869 16682 45375  6932  2278 35459 29518 28169
       20691 33696 14757 29335 17595 38828 11591 44372 46976 42508 12402 16202
       13390 44286 14905 51129  7400  4164 52485 18202 51801 33948 38549 28971
        6520 39301 34352 36347 42365 25260  3163 32209 16269  1470 10581 18157
       12591 36620 32628 16731 20062  6159 13104 42456 32944 17020 22413 10065
       49320 32065  3686 37719 35865 23164 31227 28802 28909 38059 21777 23464
       36486 42640  1309 40678 23762 23577 24321 26331 45998 20343  4846 14931
       22520  5487 34101
  1311:16606 22684 39413 12513 37711 42429 31843 38822 52247 34886  8765 33117
       40696  3434 23530 51929 43116  5166 36119 39094 17255 49612 41197 13961
       37050  4607  4537 27896 17615 21919 47670  2279 33064 12267 36027  8634
       38488 28562 49217 45084 32887  8671 51205  2725 13396  3371 42918 20664
        5007 41577 24749 26465 28255 32492 39072 40741 47718
  1312:47078  8960 43833 14223 40027 10432  3349 37780  7405 36617 11841 27592
       47258 30732  9740 42253 15134 29062 14418 39897 40511 40051 52274 39208
       14034 34359 33908  4721 19033 23662 26297  1761 41720 12706 44303 37482
        4704 16894 15057 52601 22300 24058 42902  1626  4401 19698 40988 11927
        9391 36465 46094 11528 41692 29906 31553  6406 32421 50554 52329 44176
       15131 12637 45328  2608 29946 32855  6554 29685 43141 21464  5513 39092
       13756 46041 28183  3432 36602 20552  3090  5269 44067 20654 46079 41007
       27568 33440 19792 13002 11771 17925 22293 16017 39154 24887 40439 39974
       18800 33126 41897 39000  7070 10787 14658 52125 27401 38377 18901 23562
       51537 45046 19015 45330 17233 10210 18789 31470 45821 22462 46568 11942
       24517 42255  9880  3862 24716 50061 29387 15914  7298 44962 15126  4523
       42685 26231 43233 46152 13971 10486 49781  9235 18758 23526 28525 26685
```

181

```
41113    4845  19444  27194    4965    6610  45384  26669  31350  49989  42030  12315
25012    6598  31092    5768    6354  31304  30201  27590  34317  22146  20962  33823
52085  36624  22179  45996  29496  11807    2025  35590  32364  46058  32273  22550
49802  37612  39053  18371  23089  26295  24934  28006    9430  51609  32684    7477
32509  29491  48466  23998  17168  44421  16511  38350  25320  20703  28561  25766
 3046  17734  18476  20076    5548    3956  22940  37742    6439  13748  25795  35684
21715  47398  37580  50443  17340  27897  37996  32565  49913  22909  28997  17549
50840  43156  26885  50488  39067  38278  13805    6147  12894  43469  20294  26437
40224  51853    3943  23359  46186  47950  32788    9353  52146    1491  51483    9736
22893  19708  46625  39886  42599  19619  49701  20124  31452  34158  25327  47246
39670  10080  20589  33916  37597  45705  45318  48815  27183  44200    3523  45644
20634    9293  26894  44470  39172  32422  36329  47932  25727  39805    2885  30829
14946  10761  35362    6026  28571  49107    6862  48889  37673  36017  43521  31771
17609  21586  29337  11720  43803  12407  45401  47986  23683    7761  36661  34843
 5594    1695  30765  15599  50707  22314    4514  35475  39299  45154  45152  10107
20851  34076  52254  24143  27637    4690  42105  26640  26715    9810  12299    4866
15228  47974  45693  26729    9372  20395  20399    2597  17288  19891    2104  47384
35476  19914  23257  23395  42991    5068  34021  36726  49641  24558    7300  47193
16221  45481    7969  19307  48479  38049  10308  48107  14294  19350
1313:37015  31404  14077  43221  14073  21460  14070  45896  27652    5770    3501    3504
 3473  51656  20072  20699  20694    3080    3100  32747  18990  40701    8169  24439
52387  12991  36273  29336    8192    7480  33779  13212    9092  13384  27594  36914
 3654    9695  42535    4706    4702  47691    4709    4677  14722  47335  30692  12900
30801  36382  14625  30811  51995  42458  37569  14128  17694  37644  26828    2751
23179  27469  27505    8176  27650  27564  38011  18213    2946    4310  27628  24242
27074  24730  44308    8546    6794    7577  52698  15896    6067  29672  36912    4645
40969  38871  33211  24171  50159  52159  41468    4433  25147  18549  21661    3604
10087  38501  39338  18880  42861  34340  20341    1915  30255  40409  14314  29729
20521  51503  36497  37416  13250  38143  20965    6291  35974  32014  28984  22786
22373  43850  49680    2425  49581  49603  38328  45755  45746  26380    3469  29240
33564  27474  27625  27644  27600  27681  18011  18009  31954  30233  16074  32111
34995    3101  21590  41931  41933    6037  35509  21840  21821  21816  11339    8828
24168    1617    2396  35505    1906  43894  31537  19502    3678    1933  41913  22434
 8829  41979  29159  38372  41869  48108  50292  50257  13289  22500  40633    3741
27046  18103  40927  17325  42858  40031    4831  52746  49489  29308  31140    5773
10173  41342  10093  30442    5375    7273  33683  18824  40505  32707  42273  16729
13932    8112  11866  39809  32708  37389  21682  25244  11201    9152  41939  20988
20587  20643  21323  21329  34840  51364  27147  24555  20724  37279  43784  44813
11272  45900  45931  45933  19403  45892  45895  18754  26475  10415  32523  44978
52498  34809  48340    8274  27922  46200  48854    2339    5863  27661  43460  21062
37448  45714    1994  30651  30598    2799    3560  21140  39932  18623    8808  36823
47417  48211  51419  44180  44965  32197  34796  33623    4983  23560  26424    7762
42217  34753  12743  12785  25243  22138  46242  12227  27012  49075  47539  48825
46365  22184  47069  12024  22219  46942  47091    7079  36550  27561  12905    4474
28803    2297  42992  40892  35970  32822  23898  30332  44987  25433    9013  32430
28668  12181  16806  12688  11857  30388  47007  15122  48526  48575  49673  29363
49338  34854    1995  19354  18279  28946  15162  31266  31271  46688  46468  31413
28714  34910    2845    2847    3468    3507    3509  44375  10683  45521  29484  29507
22291  13521  36684  33815  39701  23246  11581  47392  42557  49916  12620  24288
40966  40907  28533  26779  25158  34905  50223  23044  51592  22077  51721  29726
33357    2569  37276  37321  20098  37175  45234    3143  13233  15060    6842  19769
 5733  44606  44605  33693  49898  49833  51362  52178  42926  27560  50791  32238
33994  15084  27557  18108  17730  46332    6517  42652  15950  45211  17528  37543
42517  37938  24561  18719  24379  36069    9682  18738  38228  49966  40799  44596
28834  20381  19318  48415  50346  21310  45352  18418  25362  11468    4405    4383
15444  42573    1897  37941  37211  37323  21600  23965  44026  45439  48081  48293
32715  20389  23101  23099  48407  18819    8306    1508    1531  21128  16571    2566
44258  42398  15841  32867    4362    4151  26538    8167  52419  26395  50099  29609
 5404  24485  48426  48667  33792  31230  34751  17359    7461  30152  51976  52094
43662  27538  46490    4023  11437  51005    8271  50740    5202  36050  34164  30605
 9151  46601  24617    1484  44329  12454  17824  20918  46158  15891  15928  15925
15949  15923  15897  15920  13470  18397    6330  42418  13026    9835  17218  41835
```

```
19732 24102 13187 30988 25013 29302 25351 36920 38219 17144 46805 52127
23341  8679 50164 42301 12480 15088  9995 42560 17106 32734 30967 33439
14249 25047 16684 34307 32165 49093  6128 51305 41941 12180  5001  7504
15952  7310 26305 30840  3857 16126 50176  1089  3450 40057 49134 24664
20110 36574 45360 35413  6566 45018 12869 46846 50515 46517 48788 43003
22751 27507 48893  8934 26207 45936 37686 16348 39318 16350 17691 36917
23216 29493 10113  2229 10953 21889 10824 10112 12518 35188  5645 23097
22263 10834 26233 27656 49627  4732 37383 17195 47433 21141 17332 21110
37486 46796 17210 47574 47606 17193 47582 47604 34184 34212 34179 21648
21583 21621 34229 21542 34897 21580 17299 17296 17292 21587 21650 21575
21548 34901 21545 21330 36586 47435 36563 36587 36566 34903 24481 36585
40531 48019  3503 45731 40252 16703 41129 51547  2814 19526 29421  9315
18991 21422 21457 42494 21410 20583 21320 20612 20580 20640 20637 20614
20635 21418 21325 20616 21455 21459 39286 35322 21492 21362 21626 21496
21623 46772 37068 41375 52076 32237 31389  8577 36388 23750 18014 46380
15467 10510 31002 30982 30950 48735 35935 41976 36306 36782 37494 13230
37649 28965 51575 25200 27162  4527 51669 41540 34334 40662 14663  6031
41935 27867 27566  8226  8229 41823  9703  7158 20321  1991 42594 19422
16227 44834 45980 47263 43743 48095  6140 17495 15900 14538 14540 19731
49163 16807 18300 26980 14083 14080 14108 14078 14098 14075 14072 41485


1314:24872 46449 30793 49846  7630 13387 44210  8487 49591 43869 41762 44900
44870 44868 31614 25626 24510 31472 51626  8548  8517  8545  3371 17661
17660  3842 10085  7549  6690 17454 49208 31622 11333 35728 19276 10571
26724 15895 18059 22814 44093 27864 38471 45961 45985 45959  2085  2777
26834 33898 12012
1315: 9335  4667 15026 14802 46990 37661 35324  6160  3684 26897  5546 11932
39423 39427 41644 23504 23503 47882 33774 37336 30825 51668  1099 43527
42519 24742 14679  6404 29017 29018 28819 38135 17970 38603 37835 49935
 4308 49341 41281 52334  8182 49074 36814 20004 20006 37864 27215 42252
41315 46103 28055  6319  5781 39942  2268  6841 29341 32636 32637 17942
45203 20584 17903  3182  3599 14089 27502 20469 15958 39226 19279  3475
11129 11130 44846 33553 13826 38302  2990 32443  8543  4747  2832 41409
17415 17412 45376 45378 40787 26006  5205 22664 51830 45138 45139 51251
51938  2708  2706 42630 37249 27967 26059 30103 14090 34098 32117 25342
36605  6750  9871 40220  9951 31808 38411 34542 22259 23183 17861 22617
13074 23731 11882 13514 37017 26203  8523  8522  6983 37731 26202 25354
33961  5250 27577 24915 16032 33368  3203  3229  3205  5466 26926  2705
23939 29816 29804 24225 23528 51888 46120 49449  4669  2911 15931 21251
 4134 40906 34607 50702 20503 50327 25583 49201 41802 28308 11111  7793
 7791 49125 18499 45953 20066 20067  2072 23685 15549 49311 49334 48563
41337  5094  3401 10176 33601 33026  6265  2469  9139 42212 49279  4004
48558 26870 27365 32562 34940 26496 26498 26277 40463  4955 28489 10872
10870 43918 23374 49414 12397 50539 10823 10889 33746 23334 23336 12929
16844 21558 17331 17336 17335 29927 45583 45797  6299 43494 18113 43318
10896 21864 27291 24258  5261 34363 47361  2298 47386 49537 14687 16356
32594 24933 24935 24936 28155 39610 36779 51611 36776 36774 28592 26924
45590  1589 30445  8606  4725 24019 29052 31241 21636 24705 24701 40693
40694 40698 47844 29074 29071 46083 21664 20316 42076  4240  2780 24151
35278 48473 36861 36948  8504  9725 22196 22199  8502 19691  5562 26068
48400 20554 48562 41854 48397 48559 31925 46352 41228 41230 45454 22558
12871 17763 23426 40096 15864 42531 37821 22736 22733 20727 39366 39365
37337  1100 27056 36729 23894 24643 50468 48759 24521 44911 23541 36995
16948 35925 48987 42483 46194 46018 50172 24345 42750 40019 26988 13825
38978 15683 21504  5765 44796  5288 35919 28059 50144 28830 18129 27553
39449 39429 39082 36307 38112 16195 23670  2321 38109  7699  7697  7717
 7662  7688  6922 50041 10292 43767 17387 22544  8494 27464 44880 29354
23805  8051 10648 31221 29149 42368 38675  7999 22062 47705 46215 43896
42583 17776  5326 39399 39421 39416 36133 36135 36134 36555 36577 28529
28869 28889 14436 12912 12500 21973 50339 11004 48371 32121 49994 28645
25868 17784 17665 17606 17602 41362 10438 11875 37652  7959  6353 43295
39388 17026 17021 17035 17032 17056  9159 51866 10049 39454 49709
```

```
1316: 9336 19171 27611  8177 30062 38667  1483 10955 15846  7026  7024  1998
     31820 49639  6561 28843 51496 44754  2988  2986 13023  4637  3121 14509
     25633 35417  6707 32799 27702 49028  3425 44048 51175 23628 23497 44526
     28541 19999 28194 16089 16086 49030 44971  8233 24366 45452 20876 30774
      7293 51872  3810 16916 20593 18929  7865 48017 15815 27016 23913 28784
     30411 13962 49550  9633  6943  6946 23227 23826 25352 44676 22763  5833
      8442 29865 13945  2952 30862 15065 28792  1595  6266 40399 10471 36212
     22443 28757 33068  8529  8526 38672 48349 21106 47650 10632 22173 42663
     28756 18704 39614 49308 34069 28506 45708 27699 21431 49801 51021 45094
     19752 19754  7380 52613 11031  9052 22440 24807
1317:18191 31107 23733 28508 28752  7715  2943 30133 40970 40836 13740 32355
     12968  6643 51114 18156 41341  3409 28576  7077 33945 17993 33979 48325
     14117 34923 43697 52354 51268  5117
1318:45695 26136 27953 18400 17378 49009 15809  8807 19849 46997 45215 37324
      1747 38643  3058  3712  3716 29966  3721  3717 29998 47268  9447 22127
      6207 30263 23149 14549 30137 48433 30543 32174 41499 13657 24880 34898
     24897 41659 24412 14363 39888 47155  4869  4658 48455 39716 15301 44410
      5354 28614 12691 15152 35914  3389  6564 51792  8132 51978 20042 31928
     44339  9365
1319:44867 19973 30665  2707 13894 40949 11646 41789 34073  4641 13368 15880
      7850 30718 31465 37990 16584 38526  8505 18370  4992 31382 30975 21230
     21232 10168 51479 20723 52505 32000 15609 15533 27990 47229 24071 22394
     18727 41174 16570 24698 35484 31151  5298 52700 33888 45788 19913 26735
     10267 10946 34866 45255 18326 25029 40491 22367 46605  8250 37689 48712
     51157 16518 31534 45062  9889 16821  5348 24874 20226 42251 21723 37727
     16107 27112 28701  8325 38437  2191 32851 52715 24115  3540 11509 33464
     31653 29562  7680 23760 24438 45921 39530  6944  1734  3737 37830 10160
     14926 36373  7051 10759 11731 46745 25707 44148 30842 36623 10534 37168
     17458 25269 51849 14284 38613 37622 43820  2762 16953 48771 39372 31479
     23259 37556 13629 19884 52471 16727 52144 45831 10290 19631  9185 24395
     49615 18953 33293 20985 12624 42024  8826 33115 17710 26526 25339 33880
      8684  3245 45494 44276 51410 27256 47859 11547 34421 42757 45765 33355
     10627 41965 46033 31882 16312 15544  7145 32875 33252 15665 24085 24084
     36999  7574 43314 43312  6484 28612 35520 32171 20900 48772  8345  9098
     23371 33296 42267 49483  6084 34651 11806  3374 28401 29155 29154 12666
     22160  8189 31020 43782  9786 40864 26540 22554 27240 52542  4929 21272
     41112 31303 51619 51623 18106 47810 42414 46874 20258 38342 28703 28704
     33229 33249 33254 28439 33954 30362 51267 26451 44814  7194 38230 36480
     13692  6008 51857 19910 29782 14178  4526 21396 36754 27930 20686 30053
     52578  5713 34178 39265 15825 18509 24548 19006 13414 15221 51214 32444
      5485 34965 21781 14530  3206 25161 36031 14579 48827 16527 16529 35257
     16091 11935 25812 48601 23417 35772 45957 38722 39723 12596 37573 33977
     48878  2774 34577  5293 16740 19901 13395 42604 41276 24269
1320: 3877 24839 46761 21249 12115 23559 10575  5424 38040 16804 47481 20735
     37863 40299 25431 35388 48942 47116 18339 19859 33748 52136 51180 25585
     41518 34628 10102 24495 41367 50089 34642
1321: 3363 14158 14180 42349  1432 18587  8469 47575 50245 25070 12100  6290

1322:44487 43162 30158 39302 24800 40865 29695 20669  5305  7841 33647 11943
      8389 51807 35926  4192 14554 49590 52173 32188 49470 49472 39351 14122
      3453 12218   867  8217 47779 24537 48012 21690  6108 38734 39924 19029
     23136 49313 44203
1323:27976 47883 11470 45833 49970  5746 29652 31565 10341 37078 52527 51969
     47939  1225 12376  3892 16193 34538 25196 40761 45009 51839 24394 42318
     44250 35214 27544 39090  6115 12607 47201 43163 20249 11219  7923 22534
     36899  9325 35250  9182 35922 46775  8816 16038 28277 23601 42606  2735
      1440  9938  9510 29282 38134 21652 47994  9137 13122 41445 48882 46267
     51416 37228 29215 22103 43911 11944 35137 35306  6646  7110 45394  8481
     43665 26491 29723
1324:41546 34716 11150  1227 40106 34446  7068 14296  8387 50922  5742 40329
     16225 28827 50744 52002 37587 34962 40171  6551 27270 13761 17803 16766
     39829  1530  3345 17527 21004 44612 19832  9706 28392 23610 34445  8392
```

```
        36698   3895 32208 43352 35446 35350 51589 11702 26799   4631
   1325:38346   9093 13366 27354 32758 33591 33615 39225 47489 32202 41025 40433
        42018 13249 43637 49901 20075 16666 20749 38343   9200 33014 10425   2059
        37155 20767   9407 42890   5896 10346   5757 45652 27450   3588 48999 40504
         8618 20746 46692 20740 16599 20972 45424 48003 47387   7735 12724 23617
        23830 24562 36161 49287 30538 40931   7270 48050 40114 27498 14109 43115
         7624 26732 44019 48520   5878 32248 38035 21220 21057   9149 50721 25509
        52230 19994 28949 30633 46586 48376 33140 16146 32277 38321 15907 46756
        30065 27232 47852 37414   8341 34207   7537 22335 50385 15145   7256 25555
        45868   5699 34743   4489 40187 21244 26513 44142 19519   1790   9143 34534
        32833 43806 16261   9310 17502 50755 39143 25840   3720   3876 48740   8045
         8302 16946 19392 44029 43712 19923 29739 39228 48160 17830   3365   3578
        19314 15449 24875 18154 21579 18682   6843   7536 51164 37112 32226 33233
         9060   1152   1975 42509 15393 10774 14133   5343 38363 38360 18620 16713
        37999 23457 15106 41153 46968 50296   2185 31636 11337 34522   7360 11825
        20952 52405   3502 16701   2587 37039   1542 38736 45659 24982 13228 12350
         4528 41110 10685 43725 14840   7223 22379 15330
   1326:10613 19780 19778 41907 36845 18740   9962   4848 13156 22078 42459 48389
        12601   7391 41488 11887 16118 18745 35238   9042 21836   2656 29488 32010
        31647 37136 28672   7216 43035 10539 38470 39300 39275 41792 41084   8118
        35744 35715 35793 29930 33316 49291 23005 47967 40309 40312 39242 14838
        13872 41350 19916 32835 28641 24896 50714 25098   7818   2554 27956   4238
        46558 47315   2117   6228 49100   9646   6255 44862   7131 40387 30654 18958
        24867 44077 34826 30420 44389   8844 13335 18763 42728 42727 42725 30160
        25592 31690 51497 21502 22125 47535 47538   2712 45050   2196 37283 42595
        52721 49351   3984 47712 47366 47368 48241 24771 22922 43383   2259 15936
        43006 16198   1569 17281 22533   1645 42237 38245 17414 16167 18357 14824
        13993 44817 37879 48333 48306 45013 28973 36367 15120 23856 39835 30506
         4259 32935 51235 22139 47354 49953 39609 18289 25635 11029 24313 52366
        43059 13581 50393 24754 48014 17526 16996 16992 24652 39724 25756 34434
        20339 39229 26728 27161 24053 11025 12242 19735 26783 45467   8410 49688
        51964 35699 36431 28510 49685   5696 51386 18982 46183 47931   7757   4147
        51189 30599 25188 12776 34442   8264   3611 46594 31930 43801 17308 37811
         4288 29529 43846 43946 27214 44213 17645 49097 46738 25192 29292 29745
        15148 47805 46826 34422 36651 35046 40083 41152 41155 30489 34874   2308
        30336 45763 18137 41753 12386 26349 48167 24694 12149 25545 46937 19727
        50404   2953 26469 42403 33492 27004 51726 41867 36216 36124 20540 51440
         5812 10594 24005 45880 13146 47730 25634 22892 47634 47892 24647 11696
        28250 43200 18657 13383 50105   1928   9595 35980 46247 10519 14601 20342
        29367 50287 43328 52340 26650 43733 18026 47050 20304 49841 49818 10757
        10737 10795 45513 24908 45702 33736 30148 21115   5854 37051   8235 31996
        24469   6121 28980 40530 39815 33669 44973 12885 41437 18933 29435   5281
         5282   7332 34058 51913 13065 37232   7438 29987 39779 26489 45940 41741
        26425 42881 50317 50952 38397 18165 37743 17295 40629 46799 10302 25169
        42553 47206 10481   4346   8430   6138 20574 20576 20539 20570 20565 12897
        26580   8483   8501 32731 17760 39824 17254 35720 48264 16604 48125 47949
        47946 40142 46786 21826 21651 35918 21655 36519 36521 33374 25750   1516
        39821 13473 13472 13474 36633 42987 47610 37481 37538   8746   8745 27490
        27128 27126 40093 41910 29470 41446 24446 48334 33474 33457 34584 10503
        35892 44009   5806   5844   5838   5818   5814   5813   5821 43925 43903 34745
        13492 15752 50167 50182 39392 33142 35501 49597 10423 42373 15744 45867
        35392   2917   2914 19055 23974 23945 41062 20681 21400 20711 20702 48905
        15313 31295 28419   6568 13095 13367 18399 18523 48162 20602 31723   7282
         2797 38698 33739 37195 45072 47616 21996 12133 12985 24569 24563 24572
        24575 24592 12349 38269 18142   9248   2873 36866
   1327: 9037   7678 41563 40025   3491 29091 25825   7777 21833 24969 37354   3910
         4531   3872   3942   3896 20480 20482 16947 17555 30308 24778 27678 15246
        35373 49428   3249 15819 10847 12103   3133   3894   9260 32865   5334 38563
        31952 33718   4876 32524 51297 12148 26028 26809 46845   5342 30297   2293
        45767 52004   4179 18963 16631   2553   3935 27296   5518 39127 29197   3613
        23327   3949 11399 39252 13394 30728 36492 14350 36121 26494 40967 19269
         6334 21849 15555 22640 21796   7161 21451 47218   4715 24315 12291 48219
```

```
      25796 21644 34922 18540 50859 31524 27862 36142 11348 11987 36549 37718
       7579 49175 22753  1757 52020 26023
1328:11574 14731 14728
1329: 7591 49611  5961 30313 20582 10528 43826 10762 49888 26878 48102 52713
       4663  2037  7390 11453 31415 45577 39775 43120  4823 43031 30412 30929
      49677 41181 40590 50227 38252 26529 34531 36572 45497 17479  9911 45058
      15965  3864 33780
1330:24654 30822 37540 31362 17614 13960 20315 31068  3640 29151 17204 13600
      16064 22305 18168 33305 38349 31365  2021 13647 45855 49817 39501 13792
      51058
1331:29756 12248 49848 34447 31585 31588  1499 47832 20063 48622 15055 36418
      42286 23134 50648 14721 42978 29658 29690  8066 30560 43179 43033 45783
      18918 46430 36000 44837 16890 11615  3181 28998  3155  3179 39910 39908
      23021 38775 23020 39011 20627  6499 50263 24471 49995 40421 29497  6867
      12251  8205 10326 17724 20268 43126 43063 47202 49737
1332:11174 12266 33905 10251 10254 47910  8424 21754 33504 11264 52016  4544
      17369 19803 36842  8963 21421 44766 24967 52325 50593 50563 10766 50709
      18306 22429 17213 28515 47144 29356 49178 40526 44362 50645  9700 30595
       7671 40614 29639 33936 42593 40768 31753 14716 25068 25078 41428 48414
      49866 19678 18921 11729  7965 24547 40469 15799 22466 46416 51385  8490
      47592  4540 36887 30032 40595 11930  6206 33174 24468 14279 46246  2176
      34913  4763  2254 16931 34077 34056 41585 12816 50574 50606  2436 13439
      51204  9876 20409 20959 11363 29399  4761  2519 29403  6329 28896 29400
      25112 22393 37604 11113 43346 33727 49540  1840 17321  7262  7996 37653
      40400 48336  6661 37783 49478  8835 22601 29700 17041 22610  9254  8987
      22059 26509  3577  5897 45526 46255 37498 28554 46145 24806 24056 35726
      52672 19939  4155
1333:11174 12266 33905 10251 10254 47910  8424 21754 33504 11264 52016  4544
      17369 19803 36842  8963 21421 44766 24967 52325 50593 50563 10766 50709
      18306 22429 17213 28515 47144 29356 49178 40526 44362 50645  9700 30595
       7671 40614 29639 33936 42593 40768 31753 14716 25068 25078 41428 48414
      49866 19678 18921 11729  7965 24547 40469 15799 22466 46416 51385  8490
      47592  4540 36887 30032 40595 11930  6206 33174 24468 14279 46246  2176
      34913  4763  2254 16931 34077 34056 41585 12816 50574 50606  2436 13439
      51204  9876 20409 20959 11363 29399  4761  2519 29403  6329 28896 29400
      25112 22393 37604 11113 43346 33727 49540  1840 17321  7262  7996 37653
      40400 48336  6661 37783 49478  8835 22601 29700 17041 22610  9254  8987
      22059 26509  3577  5897 45526 46255 37498 28554 46145 24806 24056 35726
      52672 19939  4155
1334: 1342 16910 28698 40090  7681 27189 42506 30518 37798 50749 22362 16687
      17146 13426 14252  7165 33569 49969  6244 20834 17951 49884 50635 49594
       9109  7534  6345  7769 39483 20680  7733 36559 12902 39227  9423 18834
       6172 28659 39725 51525 42146  5245 31298  5378 25979 35000 14384 52674
      47529  4683  7345 45161 51546  4627 35881 42554 17037 41653 48684 19745
      38966 26879 40824 27188 26859  2374 28773 22119 10842  1996 18314 33076
      25711 26962 33327 22670 50001 47690 39156  9922 35302 39184 46722 42359
      38920 41701 34580 17349 13841 12051 44850  8149 48191 45880 33875  6628
      32285 43023 44602 45240 23687 27851 24653 19288  3426 33121 44481  9340
      17418 51731 42303 27037 26485 51952 12138 49843 41531 25399  5344 39742
      43474  2625 46325 41325 32813 41347  7854 26856 34514 18601 26837 17647
      37080 16065 13351 36013 37291 11448 28173 52078 50316 15233  3643 17388
      16433 48036  5050 24190 14048 31392 31591  2301 19974  8030 18494  1297

1335:21427 27598 17070 17067 17065 24043 18898 38367 46720 49544 29461 30300
       7221 23440 32295  1839 48898  7993  8022 10956 41529 35614 33947 25828
      13107 30019 24843 19782 44931 33763 12930  4418 51786 10976 25616 37033
      23127 51898 31080 19099  3387 51038 32772 10587  4034  7872 51042 10323
       9602  4404 33156 33941 33151 27305 45212 13846 44777 15419 51845  5775
      52226 52227 28129  6204 44336 51152 46185 49745 44185 50720  9603 40306
      25454 27570 13232 39539 47521 47523  9605 35689 35691 51926  5748 18553
      10243  9584 10271 32268 41726 13512 13506  3388 36236  2626 48180 25331
      45678 35011 48252 25328 27435
```

```
1336:35932 21242 18980 41086  7446 45944 29148 20407 20648  3311 16770 16752
     46496 17348 32004 43870
1337:    0
1338:14993 11532  2913  7124 35857 12957 44665 39029 40563 41306  5942 48728
     48906  8847 12923 21084 30647 40168  7297 33083 36436 44843 26102  6608
     39771 26934 33745 17013 29323 22821 30200 19694  5664 19942 27179 48947
     48310 40274 25783 25714 38091 45361 16847 13752 36832 46623 32946 13887
     34068 41758 36209  9513  9305 15651 45832  4783 37898 50031  6276 44541
     42129 24418 49862 40520 21444 20028  4958 46700 41487 48257 25217 38076
      9925  4578 48461 19577 19618 29563 45720 21709 52360  7472 13539  2150
     36088 20155 30040 38528 34582  9742 28646 25805 20322  8003 44594  6869
      3629 22148 37144 23599 19090 31332 31366 40459 31299 36493 15010 14331
      1942 32508 18806 49687 33199  8042 14283 31895  3492 41864  7704  4461
     20369 13694  4878  1667 20926 14242 39417 15405 20488 48187 14809 15017
     31463 47871 23720 45636 17157 47737 17876  2718  6789 11140 42247 18291
     21989 10850  2804 33974 23808 26026 28221 17426 22183  8073 19934 20133
     20916  6966 19831 47339  8955  8805  7054  4609 44599 45532 23242 24120
     29132 42768 34936 52569 49667 38374 35821 44549  1715 41265 50269 12338
     13745 19197 18931 51032 33594 20930 35978 15249 20577 29401 24631 10111
     23749 25856  3844 47009 15247 40752 14410 51451  4010 24180 34379 41917
     32233 14439 18187 26352 45158 41185  2265 28257 38636 41998 39062 45250
     29779 32309 34967 36980 18061 47251 17812 14653 38792 30681 42323 10806
     22477 51531 51622 22136 38999 16308 14675 38650 52134 42381 15438 49366
     15244 42132 21995 34277 45324
1339:27988 27581 25208 46336 26913 15652 32158 24576 52494 41707 33889  8142
     45236 16823 32601 11434  2504 33648 39345 30093 21765 16347 20715 27107
     48719 33744  4395 43236
1340:21219 26137 30310 18313 13680 32642 51838 33157 52483 35428 35183  8711
     45891 34222 11894 45890 39691  1179 10631 48679 44949  5157 39119  7244
      4588 35363 33350 33323 32638 32621 32647 43855 37173 15089 21657 40803
     50646 41545 40881 23162 18837 43853 14044 35425 36139 35518 35515 35495
     35492 36793 14704 35184 25755 13124 23812 27605  3676 30449 28981 17371
     21899 19082 48593 48545 19255 10207 20351 42581  9488 31695 36112 36115

1341:27737 26905 45400 36672 39169 48653 18155 18126 22918  6235 40015 36650
      7519 28751 28795 25810 28764 22511 36743 36709 36678 36770 48196 22984
     22949 22951  6506 46270 22475  6708 39732 16862 18095  9040  9043  9041
     49624 13038  8908 45780 44008 42347 37426  5303 50421 10994 39013 39900
     39907 39905 39903  2932 25598 17007 25968  3946 10537 21000 39917 16964
      4175  4158 45536  7760 33799 23076 23431 50365  2716 16113 24992 24994
     18118   838 44273  4493 17524 32003   842  6050 49330 39476 42371 12274
     49350 46201  1294   837  5462 24507 37325 42645  1623 32888  2591 33517
      4038 18871 18810 32885 19411  9409 33310 49166 37520 21794 25948 39253
      7951 16803 37669 14196 15192 32625 36642 31215  8458 37758 22502 43343
     23017 45559 35679 35675 43618 43476 24440 35982 24832  3532  3487 28767
     28818 28365 28815 28842 28846   836 44344 17456 15028  9675
1342:20808 28698 40090  7681 27189  1334  7220  1297 32178 35741 42506 38598
     13766 30518 37798 39242 50749 22362 20423 38945 33938 48799 44984 16687
     17146 13426  8844 14252  7165 33569  6244 20834 17951 49884 50635 13968
      7534  6345 16329  3593 29079  2196 12811 37283 20680  7733 36559 17846
     24797 39077 39227  1569  9423 18834 10305  6172 51525 42146 52116 31298
      5378 26286 25979 25227 43125 43765 23250 35000 39835 14384 45161 51546
      4627 39027 17037 41653 26879 13581 29474  3933 26422  4278 40824 27188
     26859 50893  2374 51189 30599 30338 15130 28034 22119  1996 17109 48711
     18314 49895 22670 39156 44559 33480 46722 42359 25506 37443 17349 19888
     13841 12051 44850 48191 24789  6628 43023 32285 44602 45240 23687 27851
     24653 19288  3816 33121 44481 22143  9340 51731 42303 27037 47578 49843
     41531 25399 23725 38947  5344 39742 38160 29367 41325 26856 34514 18601
     45702 26837 17647  4507 16065 28980 24469  6121  7367 11448 50316  3649
     25969 15233  4014 27144 44265 45940 41741 26425 42881  5264 29449 40142
     46786 33565 52514 10503 35892 43903 34745 11719  3643 17388 25822 11248
     16433 48036  5050 14048 31392 31591 11531  8210 33739 19974  8030  7139
```

```
      18494 25050
1343: 8196 48984 42895 25820  6188 23122 37826 30573 30311 36498 30575 37439
      50112 44789  5666 46959  3582 33424 14930 15918  4604 32371 51382 19763
       8320  6774 27213 24713 19400  2648 16479 27842 13326 27156  3957  8855
      36349 45622 21856 48374 27627 42627 44508 34265 51637 19597 26443 25207
       9286 32401 20484 22215 15193 25724 49113 49797 46931 27268 24404 36249
      22594  7333
1344: 3827  4903 21900 35319 33775 33776 10739  3037  6816 14586 30176 23420
      10036 40373  8857 39595 50201 28209 13120 20948 37650 29626  4997 29327
      11475 27484 43863   847 45802 34431 26215 47694 29516 16801 35756  3900
      23853 17187 32428  2295 37303
1345:44945 31889 41620  8083  2412 29986 37065 32985 28242 12746 25731 18470
      40771 11234 37003 16899 31800 10694 13120 52595 14008 14863 37309 10524
      38719 45877 44086   850 23416
1346:50110  6593 36558 35120 47701 39464 20150 40566 22245 52734  3184 17638
      17635 27382 46274 46414 24267 45819 24189  9913 16975 15917  4897  5156
      36773 38478 21168 49191 40199 47502 42580  5675 28618
1347:29905  5099 35463  4572 10753 26134 43974 45256 13213  9385 26377 51573
      14860 21241 24062 46824 14455 24422  8150  8151
1348:46551 15857 35444 35443  4058 12248  6992  8558 43027 29540  1502 47832
      16134 14721 33806 42978 11280 40355 37687 18918 17991 46430 46428 23809
      48386 46557 10497 50263 24471 35722 45428 22526 11973 19511 17743 33510
      21363  6867  1976 40658 27042  5652  8252  8251  8423 33335 33333 10326
      47276 50407  6302 43126 17263 49737 34825 25176
1349:21844 21818 21842 43482 43484 21847 21815 21870 19202  6996 19268 23583
      50046 16540 49405 51570  9518 16389 21486 51919 48986 51922 24588 17472
      33479 18374 40582 34383  6771 32718 35874 15808  2550 26720  8061 10420
      14970 16663 26360 26366 26397 26392 26389 26362  6355 24768 31680 34666
      19590
1350: 2479 25367  9046  9048 28069 43943 31796 37255 17593 33500 34721 29748
      48173 26782 26700 42524 20244 19706 47637 39236 24636 44333 38782 10404
      15765 49054 49119 31336 33559 45542 45462 36723
1351:42946 23740 44607 22986 36140 31824 47169  2595 51794  2352 42436 33548
      20432 25089  1665 29754 21044 45073  8357  2599 46919 48805 24241  3792
       8840  7215 49523 26473 44721  3619
1352:49868 46963 13614 36179 26060 46509 42905 13534 42938 42909 37839 18050
      48427 34974 51516 13109 18940 37991 33401 37989  8918  9145 22974  6527
      21449 27480  3407 39797 44909 13079 47517  5973  3928  2532  5086  3671
      29357 24237 23465  5512 48769 42007 13090 36865 21425 31547 27208 38300
      28274  3697 29156  1922 35157  8780 13812 14063 43964  7050 34927 46486
       3130 44523 16744 39893 21277 18361 17208 22455 44062 44920 26225 31144
       6575 23047 42401 17912 28178 22899 40393 20631  7554 39487 33796 26145
      28380 28379 24658 24628 36300 28375 24630  3303 19818  7765  9136 29727
      13093  2017 30526 52418  5848  5603  6918 34395 41616 44150 15049 15123
      45147  6074 17198 14680 31902 15194  7299 50777 34759  1892 19241 45045
      32930  5103  5358 11472  4942 26710  2983 41466 13652 43791 30278 44075
      22660 30002 45629 26986 24959 19668 21541  5231 51676  4576 36182 26089
      17756 27792 32659 24238 37334 27284 22469  1602  1731  1733 50949 50945
      41056 41059 41053 11266 14320 14518  5072 47723 44933 35534  2836  2834
       2837  5794 15781 16851  4303 12256 43567 46424 16181 46422  9300 24601
      28708 28713 29055 29051 41032 18249 20116 20088  9189 38226 40340 42400
       6214 40317 41757 34115 46364 25121 23424 49535 46056 21237 46981 23311
      30931 43790  6263 32739 37092 41393 51771 20921 39523 30389 49476 20775
      42383 36032 24920 24916 27697  5856 11808 22555  2080  2381 17435 14338
      18285 36553 35068 30992 34464 37739 36384 52193 41611 31712 34818 18558
      37721 38919 15663 19610 23773 49374 16598 30833 52712 50909  9233 24958
      22100 10518 36591 36271 44411 12269 26040 49271 21643 21646  1648 24052
       2414 35500 10649 11694 49268  7821 31357 13198 13203 32129 17329 20289
      11662  5974 11688 16339 40828 51936 40363 32224 43055 17551 38971 29866
      47495  7356 32914 16277 40398  4841 45792  5804 45193 45209 45191 45206
      41196 17547 15141 28995 43764 50584 43760 12002  8599 22857 22843 24222
      48099 52754 51425 10865  1489 43005 42163  9921 38622 22069 38601 38619
```

```
          38597  2740 25806 20595 25741 38805 38810 38808 31378  2560  8536 20346
          22570 20358 40482 40605 40484 38832  7932 16541 48672 44499  7954  7953
           5988  5984 18572 12961 20569 11874 34377 18868 20167 27940 39232 39059
           6306 12298  6397  6308 11559 52662 24971 14319 14305 39816 14682   863
           6466  2373  4093 40941  9318  4901  4950 25293 14700 47542 40075 31947
          44120 26482  5780 49517 29237 15125  8747 39095 27131 38578 17589 13293
          45067  5647 24153 11905 36218 39972 12807 35956 39801 20897 11429 30098
          38767 35452 35356 28014  2818   864 35375 39571 44616 30580  4695 16579
          49034   865 14207  1854 36360 20722 51128 49328  6701 22309 40478 32043
          27218 35481  8498 15322 38295 19101 40237 23335  7272 32538  3073 23567
          42296 48770  2177 34722  1594 27018 27013  5460 39637  7490  7499  7496
           7493 16981 43686 48454 50169 36941 30850 25736 25735 49974 38676 34660
          38224 13025 23193 23195 23228 14169  4051 36399
 1353:31974 32305 37571  7815 37910 19052 25236  8412 14620 35326 42433 46886
          38600 18900 38690 10804 20250  8983  4162 33885 26143  2547 20551 38370
          31361 14751  3591  2702  1613  6187 13322 17543  2753 22884 31529  1941
          32890  4581 28650 39934 29041 40418  3614 25863 18669  2848 17148 37475
          28056 40798 44179 52692 36741 20645 23899 19876 43787 18896 16132  7626
          52147  4935 40283 46849  8753 52001 24216 20114 33863  7768 39028  5534
           8075 13018  9208 13307 31792 36472 13272 22478  2090  2089 22675  6332
          29705 38169  3047 44090 23489 10904 18622 39677 22292 44976 48399 16484
          14100  4571 28903 18209 30889  8933 45737 45412 26035 29566 22572 15422
           3231 37674  4619 39101 35994 14366 37458 33326 50037 34001 32393  6461
          11100 17166  8782  2538 10287 41038 10876 35635 13942 50076  4270 31968
          26876 29884 23409 21843 50493 17164 28488 51505 31682 19983  3960 34282
          51331 39954 20572 12321 42821 29663 47645 46016 39686  5730 17463  3823
          31306 26821  8349 20709 33982  9614 47250 42884  2704 31485 22135 43522
          35657 48318 23519 13856 46105 40188 20140 48302 23502 19366 29001  8535
          37917  9701  3489  4970 32671  7287 32923 42061 46290 46676 32922 28396
          24104 28321 11479 51581  6384 48347 11236 48280 48535  4470 20562 39014
          12952  2658  8411 23030 30865 49294 27701 24240 47875  3162 18024 21490
          48654  4796 43595 43583  3687  7274 25065 34479 50505 35963  9213 16136
           3798 22360 34714 26054 17900 19027 46725 38111 34202 18917 51548  9906
          42686 32981 43355 21354  5501 20933 35288  5584 22046  5450 14447 33349
           3337  1986 22799 20585 23747 20396 11989 21030 51201 28594 37753 31314
          16725  6570 20240 45070 42964   873 41582 14867 41395 39374 39068 14868
          42633 11161  6396 41526 38105 38489 30675  9579 10890 39479 32839 45537
          51156 46347 38566 24711  3575 17307 19183 28498  7317 19103 24014 37906
          43446 13317  4439 44221 36402 15494 43813  8026 19356 43278 49454 50025
          49459  5306 15998 15990 15995 16001 16019 16021  9601 15288 12424  1890
          11567 11565
 1354: 7876  7874 52032 49838  7664 44290 12977 52356 22965  3986 28938 10641
          18535 15827 49878 19857 21216  5472
 1355: 5618 42681 27378  2275 46983 13112 38968 28487 12477 10402 23264 37037
          33417 27155 21691 44483
 1356:  890 33966  4102   889 46434 18219 51498 49453 24290 36740 20719 12913
          42238 48806 52453  8549  3410 29221  2235  7633 16609
 1357:44650 12771  1358 13184 34587 32476  7166 36638  4399 35406 35403 35397
          15468 33670 29778 38521 36440 34801  1962 27651  6760 28358 16876 21170
           2354 46781 52087 51330 19335 50761 40078  5589 26045 17530 19919 48047
          48258 26577 40126 23635 10446 36757 34053 46715
 1358:44650 34587 32476 36638  4399 35406 35403 35397 15468 33670 29778 34801
          43192  1962 27651 28358 16876 21170  2354 46781 52087 19335 50761 40078
           5589 26045 19919 48047 48258 26577 40126 10446 36757 34053 46715
 1359: 4204  5785 24478 30884 43650 43649 32382 32383 33001 32388 37018 46391
          46628 46627 31597 31087  6723 41226 29862 24780 39646 19964 39622 19908
          36244  7988 30638 30452  3044 31833 47555 47559 23607 19177 26959 28963
          30893 26940  7338 42629
 1360: 5006 20398 42578 43673 31997 26053  7928 34160 49889 22439  9345 34655
          14890 46514 31644 21139 43280 40945 15987 25960 20923 20766 15248 20919
          20883 20824 20922 20823 21683 20890 20850 20846 20841 20801 21680 20880
          20855 20804 20843  9877 39420 10321 32991 32992 28597 28599 16954 19599
```

```
44838 45632  2663  2717 46439 23383 47316 17455 46621 37258 24334 36287
24152 37831 17174 47814 19525 12849  9937 43132 36326 35841 38940  8889
10754 23027 49973 20816 35566 18788 44054 51404 25187 32958 14278 32016
13284  2798 46911 29128 37075 51007  5837  3499 49679 47813 22795  6119
21501 15251 15254 40722 13092 29964 10460 12919 25216 19677 46184 50125
28010 24266 35337 51802 40056 11384 19906 29499 22494 13374 13377 21585
49168 31146 52169 15321  6494 18830  6496 31076 32827 41904 32828 34080
 6175 34989 18474 14849 38852 51526 22540 12862 39782 31821 29997 41481
36646 17139  9454 13043  9218  2165  2046 20854  5360 28362 49285  9437
23667 28104 45356 28106 42649 45175 50886 50884  6149 32475 44888 44887
10232 46541 10230 11101 26080 48634 44302 14388 47522 22007 50977  2477
51677  7319  2188  3944 38058 38056 22709 42954  4597 30596 28840 18629
36495  8902 43086 21211 18624 13434 34979 38941 40276 49227 18956 47638
30220  6694 15287 18020  5649 33694 36058 31908  8298 29203 31842  1561
41211 39235 49370 35325 29869  4932  4112 36337 38884 14941 26777 20158
31533 36546  5307 39558 22618 13530  8397 30253 40384 52367 30276 50018
28895 26197 18344 39216 39218 16440 16442 12665 40893 40873 29595 52082
52718 26229 25346 48478 30437 30440 32042  6165  9434 32425 14845 15675
12946 15985  8600 39627 38190 35045 28977 46118 23338 16159 26210 28092
49982 25297 25348 33488 18775 18773 19883 19882 26576 27657 50464 27919
33952 49896  8036 37550 38145 14710 50895 52577 49519 49988 31445 23078
10716 32646  2909 26907 43854  4250 50008 27031 40831 29599  3511  7489
14152  8854 20171 32308 44317 25324  2610 17267 13611 40165 19955 42138
24931 25125  4001  3921 14186 24479 15885 38939  7346  2206  3001  3885
15604 38895 22962 34941 36781 29153  6605 17684 27832  6741 31405 12805
 2905 21494 45427  4110 11716 18227 35962 31901 24126 46534 50733 20537
22051 42213 28079  4833 41943 24532 34984 30797 18914 41001 15898 51709
41532 50862 12448 14199  6168 33869 45778 42998 18741 47896 44434 51863
20311 12471 29677 25632 48225 41524 51458 17778 21468  3431  7007 15568
 7031 35284 21227 49388 12490 31501  5098 33771 32775 32825 37824  1948
37207 42222 18286 22011 13420  7006 22117 52081 19711 17438 50168 43701
27011 44535 22489  2546 19852 27972 12156 35673  8901 25469 18335 39823
34679 48287 47591 44902 48540 43467 37245 47811 28332 28331  5102  4873
17814  5920 35464  3907 12641 24871 48702 41893 24166  4713 21189 20783
 7836 26629 50357 51950  5786 52010 46326 41301 20546 22822  6006 34736
 5531  5640  3747 34509 32738 32613  6012 52544 13359  4769 51882 24759
18367 49589 40707  5039 28937 13136 43612 43611 17023  6872 41902  6927
35440 51477 20642 42330  6982 35458 21634 29615  8761 37750 16080 48781
32657 32658 38206 45016 26737  4029 24642  6761 47826  7906 12590 13976
18539 33734  5890 34083 42169  8402  8405 14690 40496 28736 50745 14282
34518  8917  8951 36978 48649 16982 16983 22380 16349 25105  9696  5543
27338  8710 38303 43287  5816  5790 32418  4128 34618 14876  4586 39769
19509 26639  5044 11060 39668 24535 24538 33317 34838  5349 15791 41243
16956 35266 34293 51529 51262 33048 34559 24107 44233 32192 47211 32194
13997  7849 26344 34836 20208 49304 44183  5233 49697 49696 35145 36916
28608 20594  8864
1361:  6145 12066 27458  5799 10952
1362:27063 28760 11263 20093 14134 35376 38220 38222 49722  9062 29569 22374
      51043 33737  1195 33620 32757 35856 46843 21472 19022 50677
1363:41329 17918 31783 18111 24919  5599 14175 12556 48317 18527  3112 22468
      38188 47968
1364:19733 15547 36685 23632 39548 26025 44633 45784 23160 26609 32302 50267
      28448 14481 48753 18533 18422 37623 52434 17372  4271 15867 40733 11254
       7206 19917  8514 49046  7533 10463 18392 38054 21708 20938 40664 48690
      34440 34436
1365: 2201 25706 36847 38610 12047 45582  3416  8249 30995 14368 20986 17864
      33685  1200 10322 36877 44322 40780  9378 28681 10167 26542  2881   923
       9495 39958 43539 42211 41146 47500
1366:31067
1367: 3257 49089  9517 13227 14227 40140  7753 22719  2782 36409 21315 47282
      46817 39507 40000 19441  4499 44958 32699  6201 34269 35042 38331 41303
      11335  7348 34070 35240 33636 14036 34413 33639 16457 40597 50106 37923
```

```
      35697 41850 43398  9773 52196 44720 36231 41134  4862 23050  3336 42709
      41299  2655 28833 51493 13486 27932  4171 47404 22485 28031  9503 41219
      24520 47786 36408  8094 33031 39946 27968 17459 38110 34086 27202 10479
      11229 34731  9122 10562 16791 48746 33584
1368:45305 45308 16171 12916 47072 47115 47113 46967 46266 47070 47033 46269
      47139 47117 47038 46986 46296 47137 46317 46985 46264 16412 49252 10577
      21628 25815 46014 41874 16939 45676 31576  3488 42259 31606 24357 22029
      33421 47697 49836 46463 21046 45591 28503 26205 52065  8540  3850 17878
      16211 21412 31275 31289  6939 21671 38723 51399  5330 37590  8721  3849
      13684 23772 50343 10525 30777 23928 27092  2411 47161 20007 28211 18710
       5935 52440 19816 52143 35674 41291  8735 32103 32448 29568 16560 20666
      45786 37970 52603 44171  4836  2064 20271 49711 38887 27747 51840 33283
       8804 35031 46263 40326 11954 15962 16870 21986 47235 39628  8351 32741
      22907 33375  7453  1582 12282 23188 51514 26338 26742 46011  7084  3608
      30258 17413 15014 28964 28962 50630 34311 27410  4894 47018 47820 43976
      27750 36764 38777 10591 36175 40397 31237  8343  6606 11792  5704 39999
      16796 42790 37708 11121  9456 19492  4815 51865 19932 32331 51634  9838
       9738 26819 27192 51052 47153 20122 22045 14599  5809 20089  2747 15650
      11709 36927 42822  4811 30586 49480  5635 39741  4696 44841 35298 35111
       5123 47173  8434 42246 38124 33059 34960 14507 38296 48052 43475 21487
      39549 14172 44440 16314 17544 14262 26341 44812 42601 22649 26268 18469
      27026 36090 50425 17344 10095 16519 21103 24373 15639 25492 36629 29580
      31507 45133 20059  8664 35667 40055 38409 17194 15904 40515 43458 46429
       1490 27229 32762 10028 10971 41615 34683 36336 49397 33394 40172 47156
      51873 12150 14917 40815  8099  2496 29912 22613 51299 24830 37501 15019
      38924  4729  4748 13504 13570 24565 29260 33482 33021  2728  4393  3626
      44667  5542  9965  9968  3351 21099 49769 39403 22789 22414  9982 22792
      37008 30428 24913 10948 25780 21250 50980  8127 47059 29211 11167 36938
      15676 48041 20599 50605
1369:48144 26706 25538 29283   759 20414 22060 18185 37860 39528 18054 20041
      33273 13646 13643  7444 33275 28316 20660 13536 33446 25107  5061 37626
      45740   755  4912 17673 48037 21248 13876 38898 28552 11035 32131 32199
      22108 13710 45628 48011 13412 11895 42003 33301   806  9160  6156 36489
      52566 20357 21828 33665 24443 29552 36712 52749 41326 11205 24330  1992
       5714 21945 37378  1371 34183 30035 44000 23205 20707 48485 13309 49043
      40210 34250 12998 52037 48613 48101 28084 51431 47876 45634 33448 39474
      12956 51955  8737  6426 22754  2948 51640 26554 21166 22142 44516 12086
      18521 19398 35921 38071  8374 26230  9070 22699 52659 23923 45803 37203
      25411 51521 34275 39894  8680 24033 18809 44505 44509  6557 16865 26911
      37820 15879 37132 31098  4788 13896  6544 29835 38646  2661 28260  6916
      46155 11216 18310 15725 12253 38634  5762 13569 33433 26477 19178 36719
      25369 47075 12006 31120 10261  9441 40843 27719  6408 12558 30285 29749
      39560 36211 29464  1971 46984 24659 47682 52691 27899 16250  8784 28113
      37488 34986 11795 21337 26662 43529 43534 37246 33550 43449  8017 27001
      47441 14001 24634 41509 52726 35934 45692  2998 48242 45987 26315 45238
      43338 41748 34137 32956 11051 42118  5191 37160 45199 45264 51717 15168
      26414 18139 43926 20909 49561  5860 47346 20725  5076 34444 12088 35617
      37327 40356  6491  8152  7266 33724  8459 21696 20590 11504 10521 23850
      23848  4666 30398  9511 22742 22768 42258 46684 50297 21624 27402 33666
      18152 34766 35252  6273 30306 14351 29738 18435 22307 32577 20274 20149
      10958 43223 17719 47014 51022 44525  9803 45188 40887   757 23950 28143
      21699 22425 14803 42503 26960 30574 47753 20963  1674  2629  2633 43039
      47504 22787 40072 50926 10478 23987 16302 41830 41832 23634 45085  6687
      34392 13668 13645 13650 13625 13674 13648 32218 23098  9722 23521 41222
      28628  3372 24893 23658 25495 33570 52526  2515 34800 46881 10729  8724
      50202 36003 23892 38785  4042 29415 32275 50281 19271 19189  6432 33010
      25532 22806 10132 27777  5340   975 13421  7775 31543 19705 50847 18747
      46840 31965  8330  8328  8333 12107  4656 17796 43576  7275 10061  9692
      47425 11338  9663 29370 43936 13679 37827  1765  1767 12067 24001 36943
      21420 17382 50457 36002  1372  4052  1590 38764 33280 22813 39808 38468
      33561 30402 27455 48720 23434 33185 28386 20354 47794 13549 37117 50608
      46726 19889   962  9719  7608  6980 12128 32596 37250  9551  3086 35496
```

EP 2 484 769 A2

```
         17185   2226 31679 46753 11359 29254 34354   9347 22744 39348 42277 44162
         33865    756 48663 38152 33861 20074 48985 27648 35995   8900 45343 17815
          9690   5891 34756 13673 35618 21929 44455 19084 48369 22852 32198 35381
          2393   1370 16640 46466 18735 22323 48090 50248 52752   8579 40499 33797
          7163  15679 13598 43535 32742 30235 41794 39718 16313 29279 45847    758
         29498  14066 10555 43136 45664 20519
 1370:48144  43563   1369 26706 25538    759 20414 22060 18185 37860 39528 18054
         20041  33273 13646 13678 13643   7444 33275 28316 20660 13536 33446 25107
          5061  37626 45740    755   4912 17673 48037 21248 13876 38898 28552 11035
         32131  32199 22108 45628 48011 13412 11895 42003 33301    806   9160   6156
         36489  52566 20357 21828 33665 24443 29552 36712 52749 11205 41326 24330
          1992   5714 21945 37378   1371 34183 30035 44000 23205 20707 48485 13309
         49043  40210 34250 12998 52037 48613 48101 28084 51431 47876 45634 33448
         39474  12956 51955   8737   6426 22754   2948 51640 26554 21166 22142 44516
         12086  18521 19398 35921 38071   8374 26230   9070 22699 52659 23923 45803
         37203  25411 51521 34275 39894   8680 24033 18809 44505 44509   6557 16865
         26911  37820 19129 15879 37132 31098   4788 13896   6544 29835 38646 28260
          2661   6916 46155 11216 18310 15725 12253 38634   5762 13569 33433 26477
         19178  36719 25369 47075 12006 31120 10261   9441 40843 27719   5550   6408
         12558  30285 29749 39560 36211 29464   1971 46984 24659 47682 52691 27899
         16250   8784 28113 37488 34986 11795 21337 26662 43529 43534 37246 33550
         43449   8017 27001 47441 14001 24634 41509 52726 35934 45692   2998 48242
         45987  26315 45238 43338 41748 34137 11051 32956 42118 37160   5191 45199
         45264  51717 15168 26414 18139 43926 20909 49561   5860 47346 20725   5076
         34444  12088 35617 37327 40356   6491   8152   7266 33724   8459 21696 20590
         10521  11504 23848 23850   4666 30398   9511 22742 22768 42258 46684 50297
         21624  27402 33666 18152 34766 35252   6273 30306 14351 29738 18435 22307
         32577  20274 20149 10958 43223 46711 17719 47014 51022 44525   9803 45188
         40887    757 23950 28143 21699 22425 14803 42503 26960 30574 47753 20963
          1674   2629   2633 43039 47504 22787 40072 10478 50926 23987 16302 41830
         41832  23634 45085   6687 34392 13668 13645 13625 13674 13648 32218 23521
         23098   9722 41222 28628   3372 24893 23658 25495 33570   2515 52526 46881
         34800   8724 10729 36003 23892 38785   4042 29415 32275 50281 19189 19271
          6432  33010 25532 22806 10132 27777   5340    975 13421   7775 31543 19705
         50847  18747 46840 31965   8330   8328   8333 12107   4656 17796 43576   7275
         10061   9692 47425 11338   9663 29370 43936 13679 37827   1765   1767 12067
         24001  36943 21420 17382 50457 36002   1372   4052   1590 38764 33280 22813
         39808  38468 33561 30402 27455 48720 23434 28386 33185 20354 13549 47794
         37117  50608 19889 46726    962   9719 12128   7608 32596   6980 37250   9551
         35496   3086 17185   2226 31679 46753 11359 34354   9347 22744 39348 42277
         44162  33865 38152    756 48663 33861 20074 48985 27648 35995   8900 45343
         17815   9690   5891 34756 13673 35618 21929 44455 19084 48369 22852 32198
         35381   2393 46466 18735 22323 48090 50248 52752   8579 40499 33797   7163
         15679  13598 43535 32742 30235 41794 39718 16313 29279 45847    758 29498
         14066  10555 43136 45664 20519
 1371:48144   1369 43563 26706 25538    759 20414 39528 37860 20041 18054 33273
          7444  33275 28316 20660   4912    755 17673 21248 13876 38898 28552 11035
         32131  17097 22108 45628 13412 11895 42003    806 33301   9160   6156 52566
         20357  21828 33665 24443 29552 36712 41326 11205 24330   1992   5714 37378
         34183  20707 49043 48485 40210 34250 12998 52037 48613 28084 51431 47876
         39474  12956 51955   8737   6426 22754   2948 51640 26554 22142 21166 44516
         12086  18521 35921 38071   8374   9070 22699 52659 23923 45803 25411 37203
         34275  51521 39894   8680 24033 18809 44505 44509   6557 16865 26911 37820
         15879   4788 13896   6544 29835 38646   2661 28260   6916 46155 15725 18310
         11216  12253 38634   5762 13569 33433 19178 26477 36719 25369 47075 12006
         10261   9441 40843 27719 12558   6408 30285 39560 36211 29464 46984 24659
         47682  52691 27899 16250 28113   8784 37488 34986 11795 43529 43534 37246
         33550  43449 27001 47441   8017 14001 24634 41509 35934 52726 45692   2998
         48242  45987 26315 45238 43338 41748 34137 37160 42118 32956 11051   5191
         45199  45264 15168 26414 20909 18139 43926   5860 20725 47346   5076 34444
         12088  35617 37327 40356   6491   8152   7266 33724   8459 20590 21696 10521
         11504  23850 23848   4666 30398   9511 22742 22768 46684 42258 50297 21624
```

192

```
      27402 33666 18152 34766 35252  6273 30306 14351 29738 18435 32577 10958
      43223  7764 17719 51022 44525  9803 45188 40887   757 28143 23950 21699
      22425 14803 42503 26960 30574 47753 20963  1674  2629  2633 43039 47504
      22787 40072 10478 50926 23987 16302 41830 41832 23634  6687 34392 23658
      25495  8724  9722 23098 23521 36003 41222 28628  3372 24893 46022 33570
      23892 38785  4042 29415 25210 50281 19189   975 13421  6432 33010 22806
      25532 10132 27777 15672  5340 31543  7775 31965  8330  8328  8333 12107
       4656 17796 43576 10061  7275  9692 47425  9663 11338 13679 43936 29370
      37827  1767  1765 12067 24001  1590 38764  1372  4052 36943 17382 21420
      50457 36002 48720 28386 22813 38468 39808 33561 17693 18731 30402 27455
      23434 17185   962  9719 50608 46726 19889  7608  6980 12128 32596  9551
      35496 30386 37250  3086  2226 31679 11359 34354  9347 22744 42277 39348
      50088 44162 38152 33865   756 48663 33861 20074 48985 35995  8900 45343
      17815  9690  5891 34756 35618 21929 44455 19084 48369  2393 35381  1370
      16640 22323 46466 18735 50248 48090 52752  8579 40499 30905 33797  7163
      15679 13598 43535 32742 30235 41794 39718 16313 29279 45847   758 29498
      10555 20519 45664
1372:26706 25538 13876  9160 33301   806  6156 21945 37378 51431 51955  6426
      51640 18521 38071 39894  8680 26911 37820 15879  4788 29835 38646  6916
      36719 25369  9441 46984 48242 41748 34137 35617 37327 40356 30306 18435
      22307 20274 20149 43223  7764 51022 20963 43039 22787 23987 16302 23634
       6687 34392 23658 25495  8724 50281 13421   975 15672 12107 43576 10061
      47425 24001 48720 17185 30386 34354 22744 42277 39348 50088 44162 38152
      33865   756 48663 33861 35995  5891 35618 19084 30905
1373:49259  3598  6688  2539 27734  5111 36397 31228 31122 13303 37723  7142
      31721 40695 40527 47061 34144 27799  2203  2274  2585  9198 10372 27722
       9247 35437  9171 42016 19858 11748  7673 42008  7754 33078  9792 31492
       1374 12335 18603  1601 18716  6249  2460 27420  2671 19683  5273 50602
       5256 45299 21255 16849 31088 30978 14550 13879 13099 13121 16665 17990
      42888 47187 44060 12082 30713 24567 50997  1619 37521 29020 17258 17277
      23446 40268 46458 15677 44918  7423 18838 44977 47418 52457 28894  4488
      29890 11480 50461 37407 11037 34106 34588 50834 23981 20980 21603 20950
      39786 12343 23410 32028 25743 20887 39650 42332 47518 19966 21951 41450
      42702 27848  4482 29008 26405 42945 28709 24211 24212  2108 38474 46159
      41104 41075  8225  2862 21940  8347  9224  5750 12622 47886 43983 45752
      17241 16507  3276  9183 51790 14823 16284 29325 36280  9451 10193 51768
       1881 17698 50652  5237 14480 28687 30883 40954 20417  2237 51068 51119
      14794 14828 14854 40500  7799 50754  8576  5502  5505  6415 43289 48195
      21534 33134 30413 24856  4190 31719 19415 39290 35960 35419  6877 46321
      19756 46345 14565  9525 40385 10250 48876 37559 33246 17077 24591 20205
      19223  4210 47485 31001 32050 17881 18599 18524 18557  2934 42392 52258
      46663 47898  7258 52379 34764 50174  7155  7209 34161 22935 51620 13185
      24279  3607  8101  8037 30182 17190 14589 41828 48494 33733 24254  3167
      33288 35802  7032  9237 51670 50991  6173 44058 48849 43683 22123 39493
      35829 42229 25051 46400 31252 23481 22352 47922  8610 52103 49633 30283
      52110 32768 32846 35334 52395  7535 38609 44768 41619 35788 12562 51006
      42700 14611 14504 46426 20659 34619 26354 23802  1206 11865  7133  7135
      17043 18625 18627  3263 41887 41456  1670 17658 29145 41476 13666 43622
      10768 27180 11704 32134  5069 10809 39547  7800  4313 32446 12506 25480
      25128 31955 44835  9557 41457 21218 21217 14354 18790 18786 40630 39873
      18579 28931 45491  1661  9320 45966 32325 37836 24774 13509  8361 44613
      22294 30408 45721  4247  2079 26827  6545 18661 23649 21305  3851 19023
      36557 47306  2131 38400 34669 34654  4731 38552 45309 37152  2303 21611
      48403 28828 12050 51357 11128 24803 17767 39600  2976 51003 47215 47185
      23269  7302 28393 16706 13050 40710 40730 14759 40763 51506 32712 38476
      38473 32711 47351  8589 52623 47461 46401  1704 21456 34616 36882  8074
       9739 14461
1374:49259  3598  6688 27734 36397  2539  5111 31228  5825 31122 13303 37723
       7142 31721 40695 40527 47061 34144  2203  2585  2274 27799  9198 10372
      27722  9247 35437  9171 42016 19858 11748  7673 42008  7754 33078  9792
      31492 18603  1601 18716  6249  2460 27420  2671 19683  5273 50602  5256
      45299  7320 21255 16849 31088 30978 14550 13099 13879 16665 47187 42888
```

```
      17990 44060 12082 24567 50997  1619 37521 29020 17258 17277 17279 23446
      40268 46458 15677 44918  7423 44977 18838 47418 28894 52457  4488 29890
      11480 50461 11037 34106 50834 23981 20980 21603 20950 39786 12343 23410
      32028 20887 25743 39650 19966 47518 42332 21951 41450 27848 42702  4482
      29008 26405 42945  2108 28709 24211 24212 46159 38474 41104 41075  8225
       2918  2862 21940  8347  9224 12622  5750 47886 45752 43983 17241 16507
       3276  9183 51790 14823 16284 29325 36280  9451 10193  1881 17698 51768
      50652 40316 14480  5237 28687 30883 40954 20417  2237 51119 51068 40500
      14828 14794 14854  7799 50754  8576  5505  5502  6415 43289 21534 48195
      33134 30413 24856  4190 31719 19415 35960 39290 35419  6877 46345 46321
      19756 14565  9525 40385 10250 48876 37559 33246 17077 24591 20205 19223
      31001 47485  4210 32050 17881 18599 18524 18557  2934 42392 52258 46663
      47898 22935 52379 34161 34764  7209  7258 51620 50174  7155 13185  3607
       8101  8037 30182 17190 14589 48494 41828 33733 24254  3167 35802 33288
       7032  9237 51670 50991  6173 44058 48849 43683 22123 35829 39493 42229
      25051 46400 31252 23481 22352 47922  8610 52103 49633 30283 52110 32768
      32846 35334 52395  7535 44173 41408 38609 44768 41619 35788 12562 51006
      42700 14611 15307 14504 46426 34619 26354 23802  1206 11865  7135  7133
      17043 41456 41476  1670 18625 18627  3263 41887 17658 29145 52220 13666
      43622 10768 27180 11704 32134 10809  5069 39547  4313 32446  7800 25480
      12506 25128 44835 31955  9557 41457 21217 21218 14354 18790 18786 40630
      39873 18579 28931 45491  1661  9320 24774 32325 45966 37836 13509  8361
      44613 45721  4247 30408  2079 26827 18661 21305 23649  6545 19023  3851
      36557 47306  2131 38400 34669 34654  4731 38552 45309 37152  2303 21611
      48403 28828 13568 12050 40005 51357 11128 24803 17767 39600  2976  2982
      51003 47215 23269 47185  7302 28393 16706 13050 40730 40710 14759 40763
      35234 51506 32712 32711 38473 38476 47351  1373  8589 52623 47461 46401
       1704 21456 34616 36882  8074  9739 14461
1375:  3257 49089 19992 27636 22044 14227  9517 42540 40140 37865 17503 26922
      22719  2782  7753 21315 47282 46817 47621 39507 40000 28891 50564 50106
      38041 37954 37923 37921 36117 34952 34844 34820 24388 51628 52362 48390
      28467 26939 45902 44217 50490 35697  9363 29060 41850 43398  9773  7115
      35646 45777 32681 40263 38181 24827 44720 36185 36231 41134 45743 36023
      23050  3336 43811  6123 48408 13898 17797 37185 12373 30443 50173 12290
      50084 51296 50185 35115 10905 11520 19871 46001 37231 22417 10950 24520
      18650 14051 46453 48250 35087 26863 48996 50902 17462 25512 41595 35061
      21972  1584 44475 24088 31023  6153 22685 40610 17670 15083 24609 10398
      33027 23071 29382 11972 43518 16745 49934 47925 48746  5686  5687 22033
       5682  5711 33584 28791  9813
1376:18433 41036 10408 16538 13855 27477 10901  3602 10903 15155  3327 36931
      23500 18812 45748  3007 23316 51248 18390 43391  4786  6952
1377:21302 31481 19385 32988 20997 23971 22844 36945  5498  9406 13622 37191
      18906 15641 24401 16695 34132  7803  6434 11558 50653 20157  4146 31790
      35176 12910
1378:40111 41318 46280  3788 44409 25638 18336 46181 52049 31005 27387 22192
      17377 32528 45434 38564 38066  7779 21938  4707 39333 16903 35754 39207
      17057 14787 22052 32254 47519 32557  5911  2552 35682 12722 20092 38297
      52352 33876 24280 35840 24937 16325  9784 33438 17780  1891 21910  5428
       7193 30325 38187 32680 33735 34948  2430 49860  8137 52456  5230 28309
       1001 35279 31711  3971  1632 33208 19227 49127  8830 23206 29790 26368
      27225  4750 17570  1305  6973  8967 13691 35616 26193  7318 42424 37974
       4777 11011 27916 23889 41519 22121  2621 25667  8175  8943 30608 52390
       1869 29228 14303  4922 51072 42780 31794 37193 34245 40926 15866 29766
      44565 28989 41320 12090 10652  1000 18525 48605 48934 11400  2032  1522
      29606 31726 26096 15312  9126 11083 11014 23491  3117 21832 28064 12860
      44722 10449  5940 25123 32520 36145 40241  1383 32983 38420 21394 13505
      25914 21314  3906  3765  5038  9638 50021 11735  5410  8193 49736  2327
      42182 27970 11721 34546 16967
1379: 9964 38120 36960 40734 40729 27277 27298 47060 49031 11418 30356 26975
      10409 30417 21409 40029 41221  9314 38520 38517 52664  3079 49494 49475
       8331 33829 28789 33166 42244 36534
1380: 1381 19487 33066 35783 20349  3215 39311 39503  9106 49578 31868 46193
```

```
        19215 25275 49387 12417 23646 38123 11796 43386 20958 22243 49628 17209
        25409 43746  3055 20911 20882  1519 50009 28275 32912 11386 41858 31635
         2431 21339 42683 30943 26242 10611  7710  7904 33145 43098  1622 19282
        21152  8893  8509  9864 40858 25398 45615 34931 46050 41504 44625 32031
        28586 41940 10298  7442  8788 29970 41566 48421 50380  6847 45385 46045
        26677 10169 26505 23412 48243 46998 46999 52552  6339
1381:33066 35783 20349  3215 39311 39503 49578  9106 31868 19215 25275 49387
        12417 38123 11796 43386 20958 22243 49628 36204 17209 25409 43746  3055
        20911 20882  1380 32912 50009 28275  1519  2431 21339 30943 26242 43098
         1622 19282 21152  8893  8509  9864 45615 25398 40858 46050 41504 10298
        29970  8788 28586 41940 41566 48421 50380  6847 26677 10169 45385 46045
        23412  7767 48243 52409 46998 46999 52552  6339
1382:52426 11191 27676 46892 32782 46316 35807 28722 39006 45458  8062 32281
        28205 18749 49115 52633  8284  4871 13455 39177 39498 35277 22172 13975
        36695 44809 44285 51947 10964 12899 30358 40788 30335  2684  9552  9531
         2689 44442 20646 26705 10380 30800 31650
1383:41012 28306 19419 14188  8471 37883 41398 18078 39176 45226 41349 47402
        47397  8091 23622  2985 23612 23381 21758 41179 31161 17428  1718 43633
        39838  4007 49172 44206 15164 39334 32614 46049 12512 27307 33513 14685
         3754 21706 29879 15382 28432 50055 50729  3955 34096 37601 38950  3094
        42227 10595 17758 37964 10281 11999 11303 17173 48635 29175 42603 22063
        25611 37648 23911 23872 43239 25686 24980 14874  8885 48995 27295 52045
        34375  3645  4323 12896 34763 15242  9674  4946 23643 18724 46323 14558
        34682 15458 10181  9148 38496  5517 42265 52056 24604 41522  4117 14006
        41495 40713 50852 18333 46500 45865 12598  6429 40532 14050 48373 34791
        11079 21207 42522 16664 33844 25166 24573 44341  9130 22997  8273 47962
        13571 51643 20863 24635 41394 19812 15328 15267 49660 30969  7806 49695
        51960 41020  7067 24386 46757 16517 13814 32161 29234 21506 19465 28190
        26532 45463 19324 25449 22687 45026 37049 13985 24138 30268 31594 11357
        11950 16884 22144  6438 49834 22629 47170 25753 17911 14251 46530 51424
        33661 18015 43004 23691 33170 52158 20881 26138 19855 21406 12992 29694
        52458 30653 25539 42411 29810 10579 10710 22720 48330 33968  9812 35845
         6410 38351 40961 11794  5606 31486  9650  6104  3042  5969 29473 48171
        18951 48916 18101 39402 16698  2815 15496 14239 52303  4388 16156 16205
        41068 15364 23283 11708 43721 50966 20086 45052 10131 28136  6921 16504
        15051 20284 19984 50703 32474  8588 51056 25285 40357 11238 13589 46524
        11560 26320 33681 25022 36536 28254  9186  3545 48754 42056 45818 31313
         6073  8426  3255 20560 30052 51057 17483 12153 32093  1909 47632 42800
        13556  4441 29217 19424 46068 29264 37032 48555  5422 30033 41506  3998
        14341  5625 43478  1750 47958 47885 45771 30101 28724 43884 35598 17745
        23408 52681  3663  9908 27186 52499 44997 35283 32854  4539 30352 49542
        33816 41260 31523 47920  2467 38422 26702 42241 48908 42651 13126 38176
        15772  9467 49587 39442 33342 29396 35352 34471  9797 34668 44592 14401
        18195 34892 25862  3771 52682  2126  6317 26791 39648 19741 38319 23451
        39988 39262
1384: 8621 51031 40328 27117 51011  5416 40361 14879  5830 51855 14079 51733
         2269 32702 32748 33794 32750 27400  8156  8904 42148  3632 26244 46713
        24431 23430 13208 17312 20355 11410 21982 44353 16768  3993 42939  2872
        47372 14241 34611  4285 44488 18536  6167 37467  4864  8200 13123 12549

1385:21801 15756 21797 40588 21800 11994 16869 16867 16871 13724  8542  8539
        47092 23117 23115 10379 17798 24255 30251 11775 17681 19696  9953  4344
        19203 24012 24011 21863 21868 27442 10424  3562 15769 23855 22527 42691
        44527 47845  2164 12236 45875 45241 45874 45955 45938 43829 37153 46227
         4569 12145 37187 36366 44913 10273 10978 17102 10066 48742 27785 19875
        48140 50111 14056 17186 13320 50305 51412 51411  6765 50861 14162 29735
        12660 42716 22222 21195 25220 25111 32870 13818 26523 32724 38306 22126
        49385 19091 19206 28213 45728 13061 30466 30487 30347 30345 29684 19244
        30349 19834 18879 51013 31254 42232 45732 28605 17291 45951 16830  3548
        18743 45712 13621 13636 33008 23406 32976  3794  3015 51240 26572  9304
        34155 21078 44593 14785 14969 22028  9708 45163 25562 52425 30063 27698
         6549 23150 30295 37118 16265 41288 18729 40755  3542 29174  1591 19152
```

```
     51648 20774 17853 17852  4376 39332 20655 13771  4417 44668 43917 10622
     16723 30113 22576 13901 37271  1901 38099 45205 30855 40050 48808 50974
     47545  6002 30185  5108 32966 33677 32968 33649 33646 33650 32990 33675
     46435 46403 46609 46459 47207 46612 47210 46407 42639 42505 22506 35133
     19562 10530 52111 52112 49580  6901 36921 36919 36913 36915 28657 29813
     36876 36910 36881 24046 50516 11724 36801 29870 32310 28942 39132 39599
     19987 40382  9138  9116 16922 11550  6470 17574 17580 17577  8185 17576
     17573 22769 22771  3784 17425 17422  4635 35230 26296 46473 23476 30333
     14956 33845 33828 47754 10590  2772  2792 36150 42058  5692 21810 36203
     36202 11812 50462 12341  6548  5659  5631  5657  5629  6462  7179  6460
      7176 35075 21197 44418  1558 49044 18815 37259  5180 32743  3170  3165
     37257 36873 11557  8429  3938 44795  4108 33204 17054 19824 19825 19811
     19815 19820 24792 24776 24777 24791 25676 30107 30085  9534 10146 31026
     31003 31000 31024  9537 29340 38585 48901 45379 47451 36429  8880 28915
      5243 27938 34226 24895 36862 36859 21874 21897 21876 21898 21905 24581
     21901 21872 21902 21835 21903 38029 38042 37440 33189 38048 38025 38002
     38023 38027 38000 38045 38006 38005 38021 13808 19564 22406 21148 10501
     20807 31514 40667 19543 25191 31197 42897 46851 50254 41430 50113 50255
     30712 50252 41427 16138 32404 27874 14623  6950 14836 38547 33628 25462
     50250 10429 48077 18725 17427 29532 40082 32537 32533 32501  1560 22198
     19751 32529 32535 32506 25760 32504 32531 32503 23532 47580  8877 17654
     52518 52517 13804  1852 47049 13800  1845 17652  1848 17657  1843 29611
     40242 25241 40214 11977 20323  2190 30247 25622 11991 11697 30256 44680
      7246  9133 42736 42715 42730 42732 52068 32319 32223 10690 28674 11235
     14011 44214 25615 42158 11281 30112  6137
1386:49103 43154 38938 38171 33360 38456 37630 24580 32084 19495 36647 28536
      7483  5805 30359 11599 26174 28085 43220 39468  7690  9258 37286  2231
      2042 28050 24702 43924 36246 25627 12175 39327  9492 29538 32369 17939
     14935 19107 15067 14143 33516 13656  6071 47598 24641 40642 17844 42499
     49369 46292 26898 52098 26583 44664 18584 17913  7532 22658 22686 20452
     22689 22688 33287  9535 43176 35975 25849 20450 11275 11274 48045 33281
     10303  9414 51284 21630 50557  4630 44413 14107  8027 12052 49118 32644
      7885 29377 21830 15509 37127  2227 24813 11661 33847 21096 29503 29517
     46717 35853  4340 15979 43188 43184 17051 11352 11355 52579 11351 34755
     19873  3164 23054 30018  6926  7053  7059  6923  6289  7056  6990  6961
      6994  7017 23295 49875 45243 11454  3256  3254 35195 27452  8660 23613
      1713 36537 36221 36189 48443 29480 10957 16416 23915 17017 48529 43818
     39296 52617 13231 31071 22528 37055 24540 32722 30972 48519 46909 10966
     47412 35018
1387:31615  3528 49630  5153 29758 52562 29090 18098 19510 11765 46868 46335
      4372 16835 36848 39042 15901 16798 31070 35513 46223 22529 37375 11469

1388:42370  2051  3646 23576  7451 35323 11955 10987 44327 36149 16334 40746
     40717 44991  7696 50549 20522 38385 24758 47352 42701 44655 50205  3483
     16003 14272 17494 11622 39322 16594 28472 22713 20556  9935  2785 49528
     35508 17337 37840 37889 37915 37869 37886 37873 37845  4934 33011 30304
     28471  2746  2748  2775 41767  4397 38193  5968 51739 35580 15292 45469
     39842 45187 31922  5561 27358 42052 28229 34674 52180 19667 12886 12217
     37307 26460 18075 20663 23384 48539 26601 25248  7967 17584 22295 40790
     33341 42919 18272 49607 30072 19273  6889 38857 41263 33690  9087 46842
     18239  5601 35486 45718  2169 10001 14884 23061 51770 24790 27741 34887
     44126 35833 47565 37709 11706  6642 18761 35073 43432 35537 15220 15241
     38519 36075  4228 37966 25551 17101 50915 22102 42387 51974 51246  2493
     47558 41636 39588 14925  7660  3756 20362 32599 10970 37530 28225 29263
     19952  6163 50706 18561 45455 20119 36438 34015 33177 36435 17273 14002
     14003 14004  2173 40522 30919 25171 39962 35110 13751 12742  9435 47510
     14508 35990 45128 42805 45153 28538 34656 32507 32081 39021 49516  3975
     13346 44995  3870 41120 32294 42943 25004 26951 17536 41696 49661 49567
     12221 45214 47699 52592  8768 32019 16450 49490 21081 47063 48842 18340
     15780 39400 28492  6826 44875 29740  2445  2444 22437 27093 18937 31769
      2146 28334  6567 48440 23254 23958  9227  8729 51322 26524 18985  1800
     43932 43931 19242  8131  8262 33672 14858 50127 33632 35084  5592 19595
```

```
     29294 43369 45986 34776 11701 39666 11461  5526 16373 50807  3196 36562
     38148 29556 45151 39554 25479 50819 15201 12427  5695 43384 20229 37823
     44181 28102 45555 35523 51353 25383 48872 43249 39089 23891  8462 39869
     40343 11327 11332 11159 52287 18320 43876 32115  9800  6948  5987  1574
     18277 20867 10414 10620 50451  2686  8224 48074  7992 19312 45719 35595
     37268 12858 35746 39482 27653
1389: 4981 31290  5476 11990 34993 14025 43256 48527 19222  1399 50495 38037
     20815 14208  1123 20752 34924 14127 39798 41037 51337 11902 48607
1390:34447  1499 47832 16134 48622 14721 42978 43179 43033 18918 46430 36000
     44837 50263 49995 23146  1331  9774  6867  8205 20268 43126 17263 43063
     47202 49737 11492 50994
1391:18243 52330 38097 20309 10371 15712 45149 31119 19941 33022 18437 15354
     12324 17216  8147 40412  4466
1392:48452 10677 47887 42276
1393:10844 35671 35708 33514 26905 35727 35724 35703 35705 45400 30232 36672
     36676 22957 22960 36713 10902 51756 51757 18126 22918 40015  3812 36735
     36731  7519 28751 36737 36739 36762 36743 36710 11368 11365 37774 22954
     22990 22985 22914 22911 10785 36669 36709 36771 36770 32413 26128 26144
     19945 48196 22984 22949 22951 18916 46270 22475 12273 29763  9040  9041
      9043 49624 51923 13038 11050 44046 44049 11008 36652 10906  5303 22124
     12192  1504  2673 31509 35594 49412 26703 26701 12635 50533  3402  7410
     10994 50421 31330 39928 39907 39930 22880 22906 22882 30756 39309  2932
     25598 41406 52305 12189 12140  6803 12162  6806 37586 25087 44312  2941
     28165 35468 38616 33471 24455  2939 16964  4175  2945 31970 33799 10417
      5091 18571 23076 45945 12164 12158 11695 12193  6809 12194 12197  1098
     12059 30941 16046 14945 28878 23042 20558 32609 36648 32587 26149 31215
     50352 30007 50368  8458 37758 10829 22502 39088 45496 37757 43343 28238
     23017 17049 45559 31746 43476 43618 24440 24444 18912 49786 16733  7826
      7834 16732 16050 18651 10929 29442 29437 10926 35982 24832 36766 36767
      3487  3459  3550 22879 11262 18930 37595 37594 22908 12168 42284
1394:15026 14802 37661 15161  3684 26897 42774 42519 42878 52334 37801 33998
     42252 34644 17885 46103 43797 39942 21875 20744 20750  2268 40021 29245
      3617 13381 13142 13144 20584 27502 20469 40923 39226 44846 10345 12890
     47654 38302 18833 27603 26059  8734 21279 22823 32117 25342 49905 38334
     38411 20231  3028 17861  4045 32232 37728 35386 37017 36310  9553 34717
     32370 25354 26175 42236  5250 28584 22682 26746  2796 14475 19674 24915
     13062 13058 33368  2705 27991 14170 17741 51888  2911  4669 49449 11929
     50327 20503 51200 23211 51868 49201 41802  5996  6212 31156 12664 45953
     29483  2072 44713 29487  8002  3401  6335 33601 12505  2469  5524 28073
      9139  5873  6102  7195 47865 42212 37356 49279 14996 47533 29105 11617
     35819 16331 45899  9024 11803 28857 12397 49414 21760 44381 24894 27736
     13757 29668 41986 32630 28090 26135 16934  9006  5997 16162 19131 29122
     15416 10097  8895 45701 28860 36037 29940 30841 42477 44147 25026 43042
     38837 25868 17784 45584 26847 28421 15838 41362 43243 34189 49730 35213
     45662 19071 12476
1395:14160 48696 48122 28473 52747  3139 22669 43879 48899  3732 48523 10029
     19709 31424 22035 25311  4830 48674 16787 52717 23957 25028 15971  2142
     17421 18628 34636 48612  4550 41698 16163 50657 47390 40655 44574 15469
     17374 33475 20492 18597 44711 23862 25788  8087 42756 16062 13706 41556
     41432  2031 26747 17520 17018 26093 26744 34600 33499 42004 29625 26091
     24527 19640 23275  4011 39825 46595  3630 29624 22568 51406 50800 26367
     42440 50965 12435 23908  8370 12389  5173 30981 45430  6589 44513  7973
     48543 50763 45834 14561 18787  9892  9869  6362 46054  1550 36383 26070
      3088 11131 14076 31941  9505 38345 32379 50822 26749  7718
1396:17597  6114 42807 12070 12073 12065 12068 34398 40956 22886 11501 34396
     29614 18643  4800 34306 10156 34397 19433  6838 39384 42984 34280 49488
     24838 18169 33103 38856 13413  2244  2276  4717  1606 39751 38380 10972
     15340 12016  5071 12037 21088 21107  6417 37786 28862 11086 21364  4091
      4835 21374 21355 21357  3681 50877 10008 20639 19912
1397:39589 20514  1398 18449  3358  9980 22411 13467 36443  9453 41041 35372
     36106 11292 37958 49024 27526 43174 39697 20520 18575 31496 42201 25142
     16022 10866 34074 38196 33150 48841 17716  2415  5247 29239 35506  7189
```

```
      17978 39066 41357 20275 14940  8725  2271 16661 29026 25271 20033 11481
      38728  9720 43862 33404  8554 39656 12278 35309 39045 13385 16788 34052
       6199 36330 52531 38931 49694 28886 52764 46646 51274 39478 47394  9383
      40863 27730 28246 32351 19115 30610 14266  9307 11877 14084 52556 42699
      37868 31892 20378 18836 28364 24454  8992 20849 30640 21963 34819  5923
      37722 45384 11458 22147 39375 27757 28648 22346 14834  8839 37360 24888
      35683 10454 12254 37690 51864 15835  5802  7005 51831 50145  2458 30501
      37137 29809 44609 49942 39053 48681 24504  2890  7651 25060 26991 12436
      13300  5588  9920  8076  8921  6560 11290 30431  1893 27209 17832  7950
      43730 52760  4002 44338 41512 51150  4821 52668 27054 29838 36282 35472
      11293 15401  3192 32544 52102 24175 13238 35790 41818 50639 23070 18726
      28750 11017 39425 25440  2565 19035 48379 23989 51109 34448 30057  2644
      10091 14552 38493 49832 22971 46495 13386 34758 20525 46225 12272 24460
      47052 47188 42087 19536 47093 34881 36947 32982  6892 10130 38016 12426
      10709 25567 13033 23392 42193 28755 44669 24825 26276 24819 43602 30273
      24911 23064 20303 27367 51329 36688 28642 15018 48076 10760 48442  7342
       6065  6880 16790 47444 35092 24050 17227 32832 38958 12528 25204 14168
      20792 44930  3381 12129 48595  4082 23565 18240 22798 19344 27827 41493
       9067 12055 23435 50286 25466 17467  4688 21500 50746 15074 25484 46550
      11851 50930 37070 49643 40523  1774 17618 37694 14459 42180 10892 29730
      32156 11505 26263 14136  2344 47804  7728 38538 15709 32312 44477 29591
      33715 27536 41188 24295 28616 27626 32916 27725  4289 26807 49815 47336
      37688 52276 37523 11466 26196 32365  8928 35412 48152 10022 51389 14173

1398:39589 20514  3358  9980 22411 13467 36443  1397 15899  9453 41041 35372
      36106 49024 27526 43174 39697 20520 18575 31496 42201 25142 16022 10866
      34074 38196 33150 48841 17716  2415  5247 29239 35506  7189 17978 39066
      41357 20275 14940  8725 29026 25271 20033 38728 19887  9720 43862 33404
       8554 39656 12278 35309 39045 13385 34052  6199 36330 52531 38931 49694
      28886 52764 46646 51274 39478 47394  9383 40863 27730 28246 32351 19115
      30610 14266  9307 11877 14084 52556 42699 37868 31892 20378 18836 28364
      24454  8992 20849 30640 21963 34819  5923 37722 45384 11458 22147 39375
      28648 27757 27336 32664 14834  8839 37360 35683 24888 10454 12254 37690
      51864  5802 15835  2458 29809 44609 39053 24504 49942 48681  2890  7651
      25060 26991 12436 13300  5588  9920  8076  8921  6560 11290 30431  1893
      27209 17832  7950 43730 52760 52668 27054 29838 36282 35472 11293 15401
       3192 32544 52102 13238 24175 35790 41818 50639 23070 28750 11017 25440
       2565 19035 48379 23989 51109 34448 30057  2644 10091 14552 38493 49832
      22971 46495 13386 34758 20525 46225 12272 24460 47052 47188 42087 19536
      47093 34881 36947 32982 30144 10130  6892 38016 12426 10709 25567 23392
      13033 32221 28755 42193 44669 24825 26276 24819 43602 30273 23064 20303
      27367 36688 28642 15018 10760 48442  7342  6065  6880 16790 47444 35092
      24050 32832 38958 12528 25204 14168 20792 44930  3381 12129 48595  4082
      23565 18240 22798 19344 27827 41493  9067 23435 12055 50286 25466 17467
       4688 21500 50746 25484 15074 29397 43545 24132  4865 31480 21719 23529
       5357 46550 11851 50930 49643 37070 40523  1774 37694 42180 29730 10892
      32156 11505 14136 26263 47804  7728 38538  2344 32312 44477 29591 33715
      27536 41188 24295 28616 27626 32916 27725  4289 26807 16558 49815 47336
      37688 52276 37523 26196 32365  8928 35412 48152 10022 51389 14173
1399:23364 38570 22221  9981  3335 26992 18138 10447  3988 34864 43433 22667
       3537  4981 34993 31290  5476 11990 20752  1123 34924 41220  9629 43402
      18978 39798 39795  1124 50901  7632 41037 11902  9754  2549 45471  4170
      50495
1400:33435 26992  2764  4981  7060 41955 26032 10043 28347 15930  9629 41220
      18978 39798 33900  3828 38875 14861 22825  2559 27931  1788 23983 39794
      44307 32844 48764 52646 22867
1401: 4981 21792 25124  6666 50337 26241 47941 20901  7888 30010 21282 38754
       3873 12718 43402 18978 44671 44581 26802 43537 39770 42638
1402: 1945 29430  8220 18100 11921  6546  9261 13955  8199 15861 30205 40195
      41348 38130 28744 34443
1403:32549 50273 36535  1596 45313 17012 51124  3221  6395 36022 14944 21795
```

```
1404:14512 14450 16483 18245 17364 20252 39522 25908 52333 31214 41642 22320
      9705 10312 15583 15687 14564 15225 23715 12826
1405:14797 46821 45691 11403 49052 36283 36060 23358 15016  6572 27756  7966
     20332 40540 14857 26904 12729 18051  9697  8859 21131 43208 45208  1723
     49069 35550 40138 35805 46402 22204 33956  4078 18255 20861  6425  8722
     37945 22921 50484 11671 42131 43234 41167 46072  6257 28007 10667 51376
     22538 18034 48644 20698 16337 49622  8575 23979 27554 23867  1643  6371
     15363 51818 17247 21538 32787 45284 22608 49216 48544
1406:26958 26254 24244 29757 17828 17161 44092 44172 43992 47768  4478 11064
     12320 11639 15620 15716 32737 46349 47766  9715 27196 44057 17039 10618
     32170 45371 19214
1407: 2057 36775 40345  4963 15235 23650 10307 45689 31065
1408:28583 42151 45660 51300 17028 52313 29551 21798  3238 19490  5709 46649
     13118 30021 16556 39414  7665 28253 27353 25723 44480 15573  1708 50268
     14125 10211  8744 38270 12142 16410  9050 19384 50850 40757 33042  7279
     45438 12027 15283  4260 48178 44878 12259 47062  4167  4183 21414 23309
     26522  7157 14889  7234 48274 49238 45063 18287 48778  1545 30001 19326
     42311  4165 38839 49026 38912 27873 24673 22249 12873 21281 43119 33257
      5763  9195 11061  5627 52545  7508 52742 51466 46925 51037 43709 23232
      9990 30504 11068 11431  7507  3027 38242  9936 15167  6602 10444 15500
     29214 21318 25153  4003 50873 28627 35117 39694 31665 25664 22504 23629
      9063 39240 29560 43720 23829 19645 51585 13637  4662 20474 27321 34285
     32877  9123 32975 44519 50332 14966 14094 19468 19454 27065  7294 33720
     39150 12440 30153 40411 36687 23208  9230  7376 16856 12393 13458 49719
      7134  4334 49963  3290 28368  1576 25986 12456 25624  8827 33200 22366
     19730 16559 11530 31150  3609 36478 13260 11744 36722 46257 24905 32435
     36479 36483 12485  5857 24564 27958 50660 35576 47614 44510 22731 12701
     26635  6857 47224  7755  6767 52133  9963 13078 14847 47937 34578 48931
     29888 30267 40538
1409:39304 27691 16056 14897 37858 38421 38211 30027  9874 48678  7523 28431
     12287 30029 45790 22242 29900 12950 44990 24253 44264 19161 50941 35940
      3508 11633 46923 16592 11396 32648  4529 39271 26734  9543 41756 41199
     23697  7629 44146  3008 51685 23922 36901 12841 16335 34291 25700 12924
     22227 25427 38689  4838 25024 40267 28454 40914  4896 51509 46322 13423
     11900  7383 32257  9934 51332 16453 10959  9970 17416 18619 40367 20733
     24461  3642 19728 46282 33271 50622 37472 22873 24626 21240 25607 51716
      7634  7401 35891 29786  2289 38084 39044
1410:43543 37770 16352 40823 36548 17250 30406 19951 19950 42095 37927 37828
     47952 38490  8779  9669  4374 10416 44983 37009 36819 24837 37011 46433
     47157 44545 16319 11625 27446 37657 28005 47255 11440 19281  1816  3510
     26034 14866 42435 15619 51379 19443 31063 49411 13010 34046 18092  2810
     23180 23093 41817 33270 29643 48038 25918 41959 26115 23447 41413 27434
     22607 45840 40506 39555 39535  2500  3176 51674 51606  9020 43250 40963
     34346 11864 48226 11326 39566 19304 40897 41253  3796 25523 23797 20228
     22457 24903 47071  9661  3707 41247  1864  1808 51735 22674 47314  9328
     43009 32693 11906 50811 43965 21192 16076 28135  5700  2844 19014  1703
     37251  8759 37339 40228 24818 39979 50854 48154 42443 26614 11198 13294
     38259 38362 11508 34999 20821 49122 23238 18353 39060 20035 49211  5570
     27945 43365 43364  7040  7037 40440 42210 16305 46593 24029 44652 47118
     26003 27417 20811  2419 48511 19591 13183 26503 15507 39220 52762 30329
     35402 33933 27796  3522 25880 49754  5614 37596 37574 37552 37615 24602
     10621 21712 48861 37433 24034 17272 39561 19638 22230 23823  8352 30246
     40623 35114 38833 13202 11040 38783 18167 16415 16494 48998 14497 24160
     25749 43140  6270 21642 33906  9232 47857 48610 34134 37491 50324 21759
     19040
1411: 9034  7685 24938 15001 41657 44445 49339 17134 24445 28677 22150 13719
     32164 24354 38431 33870 26512 13115 22141 21453  9452  9425  9458 10037
     37817 18025 40719 40724 40720 40692 40716 40744 40740 24491 23745 24509
     23751 23800 24483 24456 23727 24494 23777 26248 26359 26391 47849 47162
     48080 47840 47835 48087 48083 47164 48085 48039 48031 47969 47943 47868
     47160 48662 48035 47842 48007 47863 48013 25564 22073 44961 22088  1819
     13497 25246 23742 26161 25333 25326 23659 25283 36338 30980 36419 36317
```

```
36314 31007 30956 30960 36312 30961 36341 36387 30952 36420 36390 31004
36290 47068 37421 30438  9068 18046 24861 45435 43938 11136 52481 25923
26462 20467 31551 32925 27879 40112 40208  6125 28673 16918  6481 46835
46837 30754 30729 40351 37332 22212 27989  8155 50101 25380 13200 46111
52472 52469 46113 14114 20787 48214 17944 20505 46475 27904 35308 31311
 6064 10396 10395 50083 49348 33918 18093 23262 45425 33965  6248 52024
31494 32394 32395 35899 19844 28137 28813 26142 47971 32466 38637 10257
 4888 34484 34485 15245 34461 49426  6580 43472  3248 47995 20547 17402
18284 29388  7916  3436 28326 16652 11996 16653 51457 34862 22683 37344
10846 43577 34652 12101 12098 15045 38225 43565 34241 36627 33386 26158
45745  3135 26326 22784 34783  7659 46934 26163 16728 44934 44936 38294
39945 39135 16028  5137 15585 29649 18246 29651 14821  7938 52370 20625
36295 14513  9262 26933 33487 28518 21862 25839 51728 22316 52501 49810
42388 48446  9131 45862 27051 32482 17084 39193 52055 13391  6231 21223
41064 35166 20410 20413 52532 42567 48879 22268 17894  5336 38562 39448
31185  9243 17963 27255 48168 45989 48324 39297 43988 11005 33719 21104
15179 18696 14202 35001  2161 49183 10741 14806  5808 50543  7739  8797
45201 13247 14395 31651 49226 17525 42883 37642 38527 13729 13727 37505
37500 23091 23055 46660  3260 29130 29133 11756  5267 32327 12980 36965
15002  2931 34953  9370 47938 46844 44189 27076 30881 15435 30296 41736
44065  1965 27994 32342 41474 34227 24232 45770 49399 28930 30656 22490
17715 17712 18242 28808  5168 15471 39404 25395 28118 14448 52763 52765
20906 35619 16677 48380 39063 13992 52005 18234 44269 45796  2697 52486
43832 42695 35800 36159 18232  4178 12028 12026 23586 41385 20835 18950
16327  9520 28131  3852  9698 30944 30690  3076 24148 35535 50848 45937
25614 13197 14097 16984 16986 16630  7434 19061 38890 40265 52149 46131
51018 40925 17114 27137 27139 22049 26942 42104 44366 25690  4113 44806
23875 20172 34209  8693  2555 45930 15909 19389 29572 38075  9940 37212
33540 37871 33836  2437  8512  8538 43808 10491 10490 23006 23004 18986
30872 35991 18408 46375 36597 30279 27632 25014 21547 22925 23561 22891
23590 22856 22864 22883 22841 23505 22840 22930 23566 40036 14430 27299
50428 28652 27710  9044 21895 37205 19363 20117 49554 33833 38942  2541
37331 27467 35816 39569 32250 27519 44836 46310 47954 40232 10928 35863
19980 22824 43858 48699 49376 29195 19902 28491 50485 38353 39959 36554
42513 14440 49457 15202 48884 10378 39828 37714 50063 35029 40378 42678
24119 25983 52061 20665 20052  5226 14307 11880 45109 46190 48756 49504
 9853 47726 21393 31153 36356 42646 17009  9500 20307  2195 28585 27275
 8587  4826 51405  5842 25253  2713 34177 51870 41579 49356 27220 14325
 8614  9462 37225 38789  8557 13436 15822 13609 30109 34368  6485  5877
30625 15175 50211 16509 49011 51788 42382 43857 43199 43856 48216 13888
15912 51684 26423 18617 48097 46272 51601 17910 25829 34483 23835 32604
43212 37502 16485 15356 12602 42766 20365 27217 43486  9585 39472  8010
26578 29785 42103 34990 47034 49281 20363 47608 13006  7522 46624 26623
 2761  2763 40176 15381 28485 30074 18391 43588 13596 50514 20820 19822
22981 40486 36794 36412  4445 43995 28737 25430 36552  4611 48839 38288
36281 46640 35541 20013 22998 46108 12936  6203 17650 17651 20529 25970
49971   761   760 31555 13628
1412:28094 50028 36459  6509 18356 40673 34928  2643 44145 43727 45804 40087
17094 47756  3775 52249 31967 36570 42993  3648 25056 25037 42455 41601
44915 29898 34540  4570 25882 19370 50544 26492 45283 17275 49368 52513
17324  4948 52516  1903 13782 14049  3201  3837 20905  6558 37851 45262
44586 33309 46792 23994 21136 49445 49444 49442 49447 13287 18472 38705
15299 45446 15284 52512 44460 40022 33563 30015  5255 48982 34635 49469
49471 49474 49477 39639 13283  5314 30171 50914 21210 40179  7614 24590
31274 49958 16209 14334 40413 17284 10004  5031 18432 42684 17156 18586
22668  8773 48693  9188 10474 22000 31042 41339 12748 11306 38721 37489
35859  2356 50698 38106 46744  9775 23555 24650  4263  9010  8064 33311
 7867 23159 30965 52046 17477 29495 39722 19828 39291 42035 42040 38147
11112 11109 30323 43319 49012  8534 42389  3309 20260 46852 52237 45118
42214  2530  4378 31236  4373  9597  9575  2125 52708  2397  5681 15737
52018 22460 30190 30392 32139  9210 46575  2634 39986  4553 34429 51730
15969  7149 29747 15558 27504 47611 30583 47977 14244 37178 46721 27486
```

200

```
       36908 36066 51538 20040 12587 48591 13449 38657 34348 24055 29698 48009
       36787 30876 15187 24097 30495 41786 20212 31972  5116 48994 21280 43224
       38357 18653 46244 37781 27935 16833 26307 44168 15455 12744  6372  9977
        5397 16330  2800 11102 19689 33393  1618 50149 47168 32859 18159 27171
       13638 20964 20385 13182 49693 35142 45461 37963 34552  7976 13083 43448
       37953 12948 38197 41570 29992 20765 31705 49383  8642 51242 29457 44941
       48786 23365 10937 30904 47647  3778  7047 39538 49721  7929 30621 52570
       24712  7656 30701 20416 39613 52677 20764 47262 40278 20236 14638 11082
       39289 46305  7528 36235 29300 13297  8049 38264 15976 21747  4375 46226
       19848 49086 52631 46606 29916 46966 14058 43296 26969 22896 27569  7337
       38318 27721  3557  9263 18975  1732 34495 49761 46070  2495 44025  2300
       42420  5569 22113 19219 38864 27771 51977 32322  7552 38641 22322 51142
       43303 15289 10675 36939  8547 30515 30903 30898 28159 43747  6158 40508
        6503 32415 20001  1631  6525 51769 38697 11069
1413:44882 50912 26873 52396 45339 30752 17290 14793 43084 35777 13885 52656
        7387 19148  7470 16626 52511  6428  9411 42611 34896 25135 30129 29806
       39615 42541  4140 51887 44423  7184 19740 34834 14068 35436  6931 12796
       38849 12621 36496 27846 19527 19319 14923 36285 44953 20308  8528 12542
       22835 23013 45860 46974 13321  9867  7527 48791 37266 14618 19838 47331
        8879 17243  4612 52157  8450 44515 27723 18825 45168  2141 50889 52521
       27754 39356  4114 43816 30773 45422 26585 32420 40322 50129 19742 26050
        8886 22996 31181 13669  5634 43333 34270 17433 34180
1414:28863 25212  6937 26394 50686 50710 25813  1981  4347 41122 41163 36942
       17875 36423 36422 13035 42194  2320 29945  2572 25059  5827 41983  2416
       44763 34536  8428 34579 45539 27250 17688 44845 18626 49193 24078  1524
        7927  2107  5960  8703 20406  4661 43290 25338 51925 48484 39485  2157
       16040  8162 27085  8134 12178 50115 41812 23963 51209 40059  3014 28876
        2498 40277 16605 21552 13273 32830 44735  7812  9369 40327 16567 11094
        3148 47260  3615 29531 45381 40335
1415: 8942 41646 28466 26100  5744 44323 17333 27662 31625 34824 10626 20987
       16892  3241 23578 15195 38479 38081 42904 46240 39057 51985 19039 35599
       14739 11667 33592 40151 10496 36609 22463 51583 25974 16438 27193 44629
       18485 52301 52298  2603 34685 45667 28184 26250 21080  7327 47439 49210
        2755  4895 29028 24751 34323 29896 13494 12550 50566 26816  5172 28340
       25510  5338 21813 28456 34131 22774 37290 43977  7315 13096 16145 12779
        8691 42914 45148  2102 51231 52217 36953 32907 13881 32113  8305 17038
       10018  7073 28994 28996 46307 29915 22312 28089 35545 29818  6478 44246
       18616 47944 38080 13944 11329 19058 44709 29440 18823 42566 41371 16623
        6866 16233  8263 18827 20688 18337 35547  8089 51294  1837 33338 11059
       49441 43627  3963 26501 18715 33603 30512  3359  7313 13649 26909 23073
       41536 13000 29555 20502 25206 43883  5181 22522 34977 32432  9769 12971
       24477 27053 31038
1416:52396 14793 43084 35777 13885 52656 16626 14646 29806 39615 38484 24676
       22099 20071 37506 43198 44643 11506 14068 26919 23442  8528 12542 22835
       24476  9753 13321  9867 48791 48904 14661 11934  3152 48833 29618 52157
        1621 51408 25211 27808 38113  9983 48817 43420 44036  8035 49019 51927
       39192 51036 22656 51217 10339 10559 13669  5634 43333 20636
1417:47438 52187  9997 24506 20812 45272 31502 28351 24082 50598 43905 48664
       18141 47420  8270 51371 21805 46450 47698 23603 18957 38129 48392 29594
       43010 24356  9939 26176 36154 44071 44821  7619 51499 28435 48820  3350
        7814  9153 45680 29082 16965 19967 23952  4427 35792  7437 32318 10320
       16240 45897  9404 48335 44167 31774 18150  1954 24877 44999 22330 28694
       39625 11033 39604 34257 26813  8464 14652 44454 48777 19863  6405 42235
       29092 18248 51002 31522  5792 11881 11188 14903 13712 22251 15310 23888
        2326 46138 37772  7404 37093  4533  9059 24651 23007  2050  9811 36205
       48534 16654  3072  6222 11618 28480 11487 16839  4552 32555 24519 12798
       17047 45408 19601 47096 39001 44627 11153 16164 16897 46311 37555  6437
       16493 44408 44367  9619 46531 43008 39343 22961 43616 44356 37306 24753
       22862 37528 36655  8850 33184  9217 36086 11770  2204 33207  4561 17237
       14377 16255 38724  3673 30572 23380 47512 28048 12433 19654  5658 30059
       26217 27355 40911 13559 36542 16501 44521 13169 49067 43783  1581 23672
       12843 10624  7129 38607 35588 47056 11507 41000 48207  4884  1700  4287
```

```
        50010 21775 33075 25977 49080 17485 33024 25824  3527 24902 13902 26043
        11032  5800  7674 33144 17132 10259 42908 29995 28449 27607  4268 49512
        16846 10977 33429 13907  3766 35731 43480 17403 42538 43538 36611 37461
        46169  9442 21530 36666 16101 22050 47217 10451 47903 47901 18889 33243
        42940 16921  6640  7666 26684 39665 37002 42419 48139 38450 51188  4956
        17659 19394 23575 37578 46479 30282 21880 39565 44254  6904 28127 26211
        18576 33633 30963 13918 34089  8849 11689  8383 18174 19704 42055 15070
        38778 50611 28356  4090  3440 43262 26450 12241  2514 34817 26709 48698
        20967  2129 34458 51323 44089
1418:37949 52540 30121 16591 14149 42450 32539  8970 17783 27050 14958 45130
        36798  2851 32874 32873 23203 24978 24010 49805 17992 39889 37234 29514
        42870 39009 16048 12167 24016  5052 34563 40253  5955 10054  5320 15154
        45599 33009 19589 42983  2533 38559 28219 49117  9955 46610 10949 38617
        15454 36323  6633 47234 38506 51551 29821 26457 23135 32553 17092 45030
        45033 44946 45028 45037 45057 45036 45010 45056 19878 41759 32440 48218
        50093 39525 24283 24278 43979 43922 28867 30186 35479 23884 42975 36015
        15941  5196 49355 22341 22337 17362 38156 44373 27898 28289  9371  4450
        31772 35910 32144 28283 49301 22085 25823 45870 33304 42066 45798 29101
         4426 42071 23786 42036 52359 47106 21462 42113 27692 42112  7306 17214
        17947 11845 17945 27829 37186 38630  2579 46091 49855
1419:41024 41026 19466 28151 47540 45697 18832 15736 35582 16422 30194  5616
        15975  2884 43385 20866 32155 40084  5204  8713 40085 16462 51258 42081
        36071  1449 21401 21669 46732
1420:36320 37399 45253 26232 14864  8299 45344 50382 30510 28028 39811 30558
        47037 51793 45296 13946 22076 26598 36872 17744 17762 46443 40570 49078
        11754 48869 23737 29050  6581 28970 37341  7174  7766 10247 51558 52400
        38031 41187 37326  7203 29392 10314 35503 38008 25789 18904  2543 15415
        49690 40066 43110 38509 20135 32575 51941 13028 10556 20132 10104 38186
        24402 51441 20478 20706 30706 32180 39358 11156  6285 25257 19584 39081
        25100  9624 48096  1422 14135 49362 18467 30128 27811 16784  9524 37244
        12094 24299 37924 45366  6814 29173 21124 49784 10368 25566 23815 36068
        13699  2968 18482 12327  8959 13316 49240  1423 39405 36122 13551  4295
        49734 52707 17750 27688 13224 32473 15796 11370 19236 33907 23557 10488
        17927 11638 44368 12472  7561 15526 28145 44706 15554 16930 20145 44468
        29523 28514 28343 37741 35875 40290 35871 18690  2307  7538 29492 29420
        34955 11163 47126 33264 51590 51539 22678 22703 46877  8781 24844 36510
        35977 34956 10697  7231  7227  7180  7182 45993 48439 48417 19347  1720
        23105 45734 50743 31535 33352 46148  1421 18872 44038
1421:36320 37399 45253 26232 14864  8299 45344 50382 30510 28028 39811 30558
        51793 45296 13946 22076 26598 36872 17744 17762 46443 40570 49078 11754
        23737 48869 29050  6581 28970 37341  7174  7766 10247 51558 52400 38031
        41187 37326  7203 29392 10314 35503 38008 18904 25789  2543 15415 49690
        40066 43110 38509 20135 32575 51941 13028 10556 38186 10104 24402 51441
        20478 20706 30706 32180 39358 11156  6285 25257 19584 39081 25100  9624
        48096  1422 14135 49362 18467 30128 27811 16784  9524 37244 12094 24299
        37924 45366  6814 29173 21124 49784 10368 25566 23815 36068 13699  2968
        18482 12327  8959 13316 49240  1423 36122  4295 13551 39405 52707 49734
        17750 27688 13224 32473 15796 11370 19236 10488 33907 23557 17927 11638
        44368 12472  7561 15526 28145 44706 15554 16930 20145 44468 29523 28514
        28343 35875 37741 40290 35871 18690 18631  2307 29492 29420 34955 11163
        47126 33264 51590 51539 22678 22703 46877  8781 24844 36510 35977 34956
        10697  7231  7227  7182  7180 45993 48439 48417 19347 23105  1720 45734
        50743 31535 18872 44038  1420 33352 46148
1422:36320 37399 45253 26232 14864  8299 45344 50382 30510 28028 47037 39811
        51793 45296 13946 22076 26598 36872 17744 17762 46443 40570 49078 11754
        48869 29050 23737  6581 28970 37341  7174  7766 10247 52400 51558 38031
        41187 37326  7203 29392 44043 10314 35503 38008 25789 18904  2543 15415
        49690 40066 43110 38509 20135 32575 13028 51941 10556 20132 10104 38186
        24402 51441 20478 20706 30706 32180 39358  6285 11156  8159 25257 19584
        39081 25100  9624 48096  1421  1420 18872 44038 33352 46148 24299 37924
        45366  6814 29173 10368 25566 23815 36068 13699  2968 18482 12327  8959
         1423 36122  4295 17750 52707 13551 39405 49734 27688 13224 32473 15796
```

```
          11370 38983 19236 17927 10488 33907 23557 33893 11638 44368 12258 32924
          12472  7561 15526 44706 28145 15554 16930 20145 44468 29523 28343  7538
          35875  2307 28514 37741 40290 35871 18690 18631 29492 29420 11163 34955
          47126 33264 51590 51539 22678 22703 46877  8781 24844 35977 34956 10697
           7231  7227  7180  7182 45993 48439 48417 23105 19347 45734  1720 50743
          31535 14135
    1423:36320 37399 45253 26232 14864 45344  8299 50382 30510 28028 30558 39811
          51793 45296 13946 26598 22076 36872 17744 17762 46443 40570 11754 49078
          23737 48869 29050  6581 28970  7174 37341  7766 10247 51558 52400 38031
          41187 37326  7203 29392 10314 35503 38008 18904 25789  2543 15415 49690
          40066 43110 38509 20135 32575 51941 13028 10556 20132 10104 38186 24402
          20478 51441 20706 30706 32180 11156 39358  6285  8159 25257 19584 39081
          25100  9624 48096  1421 18872  1420 44038 33352 46148  1422 14135 49362
          18467 30128 27811  9524 16784 37244 12094 24299 37924 45366  6814 29173
          21124 49784 10368 25566 23815 36068 13699 12327 13224 15796 11370 32473
          38983 19236 33907 23557 17927 10488 11638 44368 33893 12258 32924 12472
          44706  7561 15526 28145 15554 16930 20145 44468 29523 28514 37741 28343
          35875 40290 35871 18690  2307 18631  7538 11897 29492 29420 11163 34955
          47126 33264 51590 51539 22678 22703 46877  8781 24844 35977 34956 10697
           7231  7227  7180  7182 45993 48439 48417 19347 23105  1720 45734 50743
          31535 36122  4295 17750 52707
    1424:14304 46979 13063 15813 41666 46408 46366 27622 34327 11849 12997 33809
           7503 35946 41494  5451  9221 15606 13940 11401 46578  1803 52253 45657
           3429 51307 45300 51461  1858 25726 40883 10116 23605 14137 41175 44452
           8367 28731 37395 36528 20206 50907 24910 14528 15446  1887 26547 49555
           9065  9376 10638 13430  8506 26181 37130  8313 34643  4916 46135 23373
          47087 24900 44548 45655  5656  7284 38283 20224 15768 52666 31933 15447
          46235 42637 29781 14491 49007 13670 47453 20165 34287 32682 35161 44563
          47795  2221 13201 13263 29768 31620 36759 22999 30669 16951 15598 41023
          20084 19938 32913 32911 47918  7125  4326 42960 38546 32269  6005 11566
          20046 33456  7080  7226 27113 23588 48004 10812 19247 39025 30461 48626
           5957 14603 14826 35939 36860 12949 41044 20507 35862 44969 16238 47508
          33307  9155  8958 21673 31748 32634 12759 13661 30735 27893  2038 30866
          52280 38426 14373 38324 37748 33269 13129 29642  4229 31904 32970 11095
          41415 25009 52067 27318 48779 29467 17352 39573 40510  4804  1521 24149
          26652 26719 23924 17515 50923 27290 39567 50825 13709 47660  4413 50636
          14933 43447 29168 19568 11424 34238 12594 40821 12970 17567 34169 41690
          21028 46188 10672 21348 21346 21342 42625 25844 51694 50805 20461 19639
          44683 51478 33230 20626 28030  5254  5186 31066 18322 32110 14662 39492
          14877 42005 27602  3534 44096 47089 45326 18671 48866  4420 23927 23774
          38943 51020 18045 11685  5065 27140 26116  4066  4369 35439 10881 40033
           3549 22672 38825  8687 35527 17922  6861 37640 12593 34219 32880 32179
          42317 42314 45043 24865  3821 38902 17827 36671 39655 23864 22952 25317
          28852 12565 35650 17891  1880  3759 29722 38847 43696 49306 52107 25179
           2310 36580  6716 39740 23878 47678  5608 46900 46811 32463 45266 29319
          33183 35864 31681 41807  4037  5669 34705 11226 18570 12727 20543 47709
          11356 29472 18853 11655  1527 37374 19985 28864 31766 18964 35344 23542
          15005 38969 25940 46681 45849  7088 25245  6430  2081  5660 37670  2980
          25491 34321 52466 39867 29791 46505 38215 10830 29139  4674 34848 40230
          38788 40260 20131 42644  7570 20107 44458 11747 11684 23095 22043 46005
          26944 26999 24236  4218  4452 11855 35908 33467  6942  7868 13591 42096
          39939 51087 11723  9270 10960  3633 43845 44804 52120 48625 23240 44747
           4718 39563 18247 26189 22697 24554  7553 50377  2944 35039  5345 48291
          44154 11195  7219 37267 18450 16421 47877 10256 26274 43235 17935 46626
          49831  2528 47321 48128 30083 35342 20254 31121 43589 37735 30918 42453
          32272  2682 36660 35981 33344 29657 16987 48796 33923 43542 35730 46955
          25801 31801 15988 44131 22639 44106 41273 25235 24322  7546 15261 24164
          28723 35163 11360 23419 51383 12211 42145 25459 33364 11259 15474  4634
          10891  4485 38903 16217 34220 38560 11108  2404 27439 20870 52008 52568
          19062  9809 32334 15575 16513  3774 21998 28065  7045  9943 25265 15661
          51678 36196 45365  9673 27795 29390 28582 36992 48005 19776  7141  3779
           7677  9783 12388 40102 26022 45451 37316 13490 13701 15037  3777 24546
```

```
      9856 35634 33598 22632 31778 33227 29006 18614 24773 20924 22401 30071
      7706 43878 34561 11791 16616 14668 19369 29463  4381  4281  4905  1135
     25922  9074 36163 48055  9144  2433 42749  5471 23707 49999 45136 42321
     35241 37028 32605 21167 15044 38992 32496 18252 13522 47945 50288 17274
     12306 17150 30634 31340 23313 47112  3832 15282 45173 28733  1136 31432
     22131 35132 36569 47296 31706 25775 18198 17189 48289 19065 42511 23789
     24459 44501 12651 36043 25407 12453 33376 29692 26875 43436  1673 38803
     23736 30319 13205 38774 42114 25865 19446 24862 26678 48043 40247 45760
     34571 38286 20934 26835 50233  5886 20668 32861 27680 19186  5159 34575
     15260 50147 15565 51984 39553 42397 36245 37490 36810 25221 31727 47914
     37275 36372 34119 38073 16748 46008 44808 38937 14648 39640 37905  3519
     23723 23721 27720  6262 50534 19243
1425:14304 46979 13063 15813 41666 46408 46366 27622 34327 11849 12997 33809
      7503 35946 41494  5451  9221 15606 13940 11401 46578  1803 52253 45657
      3429 51307 45300 51461  1858 25726 40883 10116 23605 14137 41175 44452
      8367 28731 37395 36528 20206 50907 24910 14528 15446  1887 26547 49555
      9065  9376 10638 13430  8506 26181 37130  8313 34643  4916 46135 23373
     47087 24900 44548 45655  5656  7284 38283 20224 15768 52666 31933 15447
     46235 42637 29781 14491 49007 13670 47453 20165 34287 32682 35161 44563
     47795  2221 13201 13263 29768 31620 36759 22999 30669 16951 15598 41023
     20084 19938 32913 32911 47918  7125  4326 42960 38546 32269  6005 11566
     20046 33456  7080  7226 27113 23588 48004 10812 19247 39025 30461 48626
      5957 14603 14826 35939 36860 12949 41044 20507 35862 44969 16238 47508
     33307  9155  8958 21673 31748 32634 12759 13661 30735 27893  2038 30866
     52280 38426 14373 38324 37748 33269 13129 29642  4229 31904 32970 11095
     41415 25009 52067 27318 48779 29467 17352 39573 40510  4804  1521 24149
     26652 26719 23924 17515 50923 27290 39567 50825 13709 47660  4413 50636
     14933 43447 29168 19568 11424 34238 12594 40821 12970 17567 34169 41690
     21028 46188 10672 21348 21346 21342 42625 25844 51694 50805 20461 19639
     44683 51478 33230 20626 28030  5254  5186 31066 18322 32110 14662 39492
     14877 42005 27602  3534 44096 47089 45326 18671 48866  4420 23927 23774
     38943 51020 18045 11685  5065 27140 26116  4066  4369 35439 10881 40033
      3549 22672 38825  8687 35527 17922  6861 37640 12593 34219 32880 32179
     42317 42314 45043 24865  3821 38902 17827 36671 39655 23864 22952 25317
     28852 12565 35650 17891  1880  3759 29722 38847 43696 49306 52107 25179
      2310 36580  6716 39740 23878 47678  5608 46900 46811 32463 45266 29319
     33183 35864 31681 41807  4037  5669 34705 11226 18570 12727 20543 47709
     11356 29472 18853 11655  1527 37374 19985 28864 31766 18964 35344 23542
     15005 38969 25940 46681 45849  7088 25245  6430  2081  5660 37670  2980
     25491 34321 52466 39867 29791 46505 38215 10830 29139  4674 34848 40230
     38788 40260 20131 42644  7570 20107 44458 11747 11684 23095 22043 46005
     26944 26999 24236  4218  4452 11855 35908 33467  6942  7868 13591 42096
     39939 51087 11723  9270 10960  3633 43845 44804 52120 48625 23240 44747
      4718 39563 18247 26189 22697 24554  7553 50377  2944 35039  5345 48291
     44154 11195  7219 37267 18450 16421 47877 10256 26274 43235 17935 46626
     49831  2528 47321 48128 30083 35342 20254 31121 43589 37735 30918 42453
     32272  2682 36660 35981 33344 29657 16987 48796 33923 43542 35730 46955
     25801 31801 15988 44131 22639 44106 41273 25235 24322  7546 15261 24164
     28723 35163 11360 23419 51383 12211 42145 25459 33364 11259 15474  4634
     10891  4485 38903 16217 34220 38560 11108  2404 27439 20870 52008 52568
     19062  9809 32334 15575 16513  3774 21998 28065  7045  9943 25265 15661
     51678 36196 45365  9673 27795 29390 28582 36992 48005 19776  7141  3779
      7677  9783 12388 40102 26022 45451 37316 13490 13701 15037  3777 24546
      9856 35634 33598 22632 31778 33227 29006 18614 24773 20924 22401 30071
      7706 43878 34561 11791 16616 14668 19369 29463  4381  4281  4905  1135
     25922  9074 36163 48055  9144  2433 42749  5471 23707 49999 45136 42321
     35241 37028 32605 21167 15044 38992 32496 18252 13522 47945 50288 17274
     12306 17150 30634 31340 23313 47112  3832 15282 45173 28733  1136 31432
     22131 35132 36569 47296 31706 25775 18198 17189 48289 19065 42511 23789
     24459 44501 12651 36043 25407 12453 33376 29692 26875 43436  1673 38803
     23736 30319 13205 38774 42114 25865 19446 24862 26678 48043 40247 45760
     34571 38286 20934 26835 50233  5886 20668 32861 27680 19186  5159 34575
```

```
      15260 50147 15565 51984 39553 42397 36245 37490 36810 25221 31727 47914
      37275 36372 34119 38073 16748 46008 44808 38937 14648 39640 37905  3519
      23723 23721 27720  6262 50534 19243
 1426:49788 26665 17133 13764 35144 35143 35168 35194 35191  3283  3282 30189
       9095 17334  9077 23265  7064 42484 15256 24683 13917 21912 21886 45475
      44407 46536  9744  8603  3967 51141 24500  8639 10986 14872 34388 34389
       9904 24627 51162  2120 52066 15790 13498 35141 35140 35186 35118 35109
      46941 46917 46915 11330 11331 11334 11305 27837 14613 35189 35190 35136
      29044 24259 44737  8228 24275 24282 17782 20094 24231 42479 10473 10475
      15934 10495 10492 24233 22920 22919 42379 31417 32286 23654 43954 21053
      37918 37897 45515 50801  8861 21175 45580 41712 41681 49886 25959 38745
      18069 38671 31944 20261 36063 10413  1647  8115 21334  1835 28115 49828
      28450 41967  1011 29476 10914 43586 32674  8993 30162 31857 17045 42467
       4771 49783 36405 48113 48094 22955 52311 42543  5356 22859  5865 34103
      27707 27576 22861 35170 35171 16135 31129 31127  3694 21447 14012 41452
      32937 32939 32942 19139 19137 19134 32270 14500 39049 50570 30075 28251
      26474 36820 49968 29963  7364 48993 43506 22277 21089  8511 20012 11169
      11834 11187 39312 20436 20874
 1427:22894 22895  5178 17806 42807 29150  5673  5677 51320 31637 31634 22968
      22933 22972  9959 15191 51298 13873 22004 21999 43168 40732 47676 47677
       6912 50576  8414 25779 18169 21200 13413  2247  1580  2250  2248 39751
      39753 43505 44672 44699 23207 51148 20872 22005 21107 21975 13378 39153
      21374 21357 21355 21358 27197  5918 41608 50877 51446  9987 41353 41354
      41373 41426 41460 41433 41423 41458 41438 41429 41399 41332 34792 21239
      46308 14713 24960 31883 17172 14022 35219 30989 45080 15848 41404 19077
      24042  1676  1546 32450  1678 37570 26589 36959 39455 36662 30939 13488
      23433 30887
 1428:31428 28633 39847 35897 18029 39664 33111  9421  3413 35299 28453 27047
      44003  4765  4941 12883 30381  1526 45950 50341 13173  2899 48819 25023
      28003 34917 27639 13606 42548
 1429: 3360  3363 14158 25764 43713  1138 24154 34841 51074 23673 24122 26584
       1321 47680  6290 42257 39533 33887 23811 31012 42349 27049 45838 15739
      21271 46957  2124  1430 45596 47575  7162 34723 30661 37994  5726 10014
      12100 37529  8144
 1430: 3360 14158 43713  1138 24154 25764 23673 34841 51074 24122 26584 42349
      27049 15739 45838 21271 47575 41092  7162 34723 30661  5737  2826 37994
      50245 19228 10014  5726 45051 32796 19439 48538 37529  1429 24234  8144
      12100
 1431: 3363  3360 14158 14180 34841 25764 51074 43713  1138 24154 23673 42349
      27049 15739 45838 21271  2124  3822 47575 43229  3897 12100 23734
 1432: 3363 14158 14180  1321 47680  6290 42349 27049 15739 45838  2124 21271
      48364 47575 34723 30661 41092  7162 50245  2826 19228 10014 32796  5726
      45051 48538 19439 24234  1429 37529 12100  8469
 1433:44839 44750 36132 10750 29619 30405 21681 19037 38127 34667  9264  6092
      46832 30343 48341 35091 37637 40557 39231 48346  9150 44634  4472 18293
      23157 21917 46908 27101 49372 33015 13190 13179 27850 18594 31145 17432
       7722 46682 10549 52097 28493 21793  9733 45132 52665 38922 34072 47110
      18149 27700  6975 29425 12151 19405 36904
 1434:52703  7058 52027  5135 14727 50478 50518  4008 39673 23399 11369 19412
      47916 50561 48915 36184  9413 29512 41331 50386 44032 38545  1905 45334
       4330 30436  6908 52365  5170 47151 28624 10531  3089  3045 46388 17983
      12922 50601 27869 50662  4322  5209 26664 35423  2790  7903  2737 33444
      28372 20671 25702  7471 22650 24805 24009 17229 30839 32484 38415 13760
      48177  8441 32066 23523 13605 22737 43959 14374 49652 26998 10708 45176
      45407 47798  3592 51067 25579 27559 18681 13716 51966 12967 32622 36727
      46175  2503 41111 45675 25689 44698  7811 33731 25054 29554 24522 48773
      13723 40561 44486 43859 43667  5488 35094 35629 14631 50948 42089  3926
       3881 30067 51555 25270 25266 25300 25299 51779  8029 27889 29057 11571
      29258 10311  8758 48961 40817  9508 34947 10332 49771 11030 17605 51078
      44572 16705 52706 28969  6392 24746 12183 16668 18462 19518 22159 22207
      41232 42080 19679 18324 38173 23422 42990 48745 12336 27393 40039 49556
       5585 40834 12355 37579 26322 21685 43796 47311 26843 50547 14527 47371
```

```
15807 17608 47167 17446  1899 17586 50616 43928 32499 31598  4592 34247
35292 47683 44907 27845 44191 30700  3704 47498 12195 16199 29718 50918
28871 50758 35797 50882 33381 46651 18217 28821 26896 28848 37816 13930
41108 27425 12372  8323 48354 48437 48361 32412 40947 35623  4964  3330
 8186 35536 19173  5710 21959  4041 11168 33425  1764 52716 27356 51518
50689 44928 23357 23702  4220  2065 34835 18121 31559  2822 36854 27540
27535 46017 46376 34125 34126 50007 32447  7498  6636 31619 16887 44567
45495 29490 27239 50984 50085  2379 48582 50057 36531  2662  6095 33102
18215 27073 22244 37066 27533 39484 17235 12018 11993 30717 14268 30722
35235 36645 23069 40709 11835 48885 47181 50705 10453 37599 40167 21467
44708 23382 18110 18574 24077 47125 24075 24021 52432 45093 12525 21135
25049 34727 28502 29147 19474 12548 48157 41162 44538 43847 31041 43661
34108 24725 47128 47175 16289 33819 52186 35478 28460 33036 35455  7551
19246 49462 22224 45301 19702 17450 23956 21043 49296 47663 15137 42618
43488 22804 45766 44453 17343 45768 51599 46694 46665 50121 46664 28728
18578  7721  7702  6131  6133 19607 27682  4768 21476 48722 41298 38663
39540 35877 14277 20879 13533 37497  3195  4774  5241 26285 26227 35017
35082 27815 25366 21859 50712 25403 51679 39715 37194 13778 39744 46655
43939 48279 41613 45162 48532 31276 40394 30859  9008 34785 44797  3606
 2593 25565 21909 51049 47120 20738 21617  3250 24195 27071 43109 43106
47803 50264  5648  3054  5835  3236 51956 27847 27844 42653 18835 14422
49055 38654 16622 43831 11224 17810 22691 21612 35712 14192 30091 24380
42202 25242 33986 39776 43430 24103 31072 16864 43373 33983 49121 46889
 8712 13419 40564 45346 51060 20730 14752 19719 32292 41576 40449  5829
40225  8668 15560 28856 21097 28011 20941 28013 27278 28016 21100 41963
41950 20937 41957 42068 21095 41961 28032 17309 26452 20973 43045 43018
42179 21016 24655 24678 21034 42986 24681 24682 24657 43051 43014 24632
42995 25273 25262 42141 24685 25304 25349 25313 21002 43052 21066 42250
42216 42219 42249 42225 42228 42184 42187 42032 20975 42271 42930 42955
42111 42952 42922 42140 42933 42115 20942 42274 42137 42144 21011 43075
43072 43079 20981 30284  2462 37158  4126  2492  9822 25740 35358 35906
23185 31936 45131 38917  2047 47100 46905 50579  6321 49013 50229  8473
47824 36355 39899 34833 20209  8513 49085 13565 23474 50691 43865  6183
43881 28807 21867
1435:23300 20441 42851
1436:13410 30908 12451 24582  7018 37785 38872 10659 13938 45554 21379 25329
13487  7655 42687 10219 51970 45585 40877  4266 38635 42053 39987 37353
 8806  8146 27870 22065 35784 18201 51924  7643 34381 29177 23261 29807
22615 51456  5292  3396 32974 13708 15269 22704 45759 17707 32591 35364
23000 12209 17113 23771 36454 45104  4428 33495 23272 12960 43117 38574
33959 33599 19031  8188 36038 34827  8823 22690  8218 27089 26514  2181
39755 39952 17725 34172 21432 22721 48190  9051 45111 26311  6751 47149
14616 39788 22773 27834 28070  9101 38589 49467 28865 48583 49592 10071
12188  3936 44391 10861  5566 24764 21443  1505 13361 28307 29634 41202
16464 13518 36898 50865 18002  8683 11222 21814 16542  8207 48821 38337
18342 19879  3172 52767 37030 31567 14154 33334 40058 44566 24314 46277
17439  7568 21724 19172 39504 51185 25361 48469 15874 21021 31648 43775
37877 42889 41250 35884 42767 28537 45812 25464 14949 49262 17625 35399
32044 10035 48491 20162 26304 24134 42720  5373  7008 12588 35988 21426
19328 17904  3793 13009 19346 20615  1649 24731  3535  8198 43028 20425
12085 31300 25157 40434  3641 26695  1894 42074 24306 51318 26645 25590
 5580 44833 46251 25571 28851 51061 26484  1920 23273 27631 28304 41843
11691 11000 45911  3970 36444 24105 29944 15865 36095 34152 31186 32838
40935  6047 16035 50134 43952 32076 21734 21732 44205 50766 42777 38162
22565  1917 48441 45333 50302 32497 39489 11052 10907 30910 16244 20748
17176 43203 33343 51905 12096 29007 24873 26740  7288 20291 43128 25130
45482 24801 29565 23453 47043  3879 29851 29347 24761 10204 16842 44310
47833 18349 31639 18746  1015  4201 29188  8675 48606 33362 13554 44871
42617  8265  8114 48431  4959 39868 42893 50388 11077 29291  6035 32441
20460 34524 43418 47184 30911 39557  8020  6232  3702 34453 13970 48067
36173 48580 39517 50674 11966
1437:28890 50651 18496 32462 38398 14784  8777 35311 10962 35824 19748 32736
```

```
     38565 18027 39190 41454 13851 18063  2690 24599 52543 43839 36164 14345
     45507 19360 20112 40264 19546 29253 23455 32029 40109  9771  1605 38246
     19981 47480 21306 10009  8381 31646 27995 39896 15445 38814 13360  8014
     37343 25442 39264 14995 31586  8447 26119  5252  6565 16582  4018  1222
     12803 38948 20192 13345 48802 48780 24301 11038 22905  3899 19795  4111

1438:37240  8314 36403 28670 12360 38192 49435 40874 42971 18058  8046 49327
     16633  6039 23461 14576 19331  4610 19558 40999 52332 22592 12422 35316
     10894 27021 35104 27497 25728 46667 38869  5637 31848  8727 14748 15238
     44061  4104 40856 20701 22991 18648 32571  3085 29959 40236 46294 49041
     17961 42324 35218 27224  7978  7842  7720 39274 37206 45639 20714  8060
     51814 49006 33684 48516 47265 21077  8731 27921 25874  5138 40133 10507
     21955 24715 29137 50109 32526 15910 28872 50005 45666 37145 42528 49933
     23710  6271 11683 26698 41161 36969 18475 24397 32698 30891  3719 42957
      2966 49845 10103  5042 30699 16100 21098 45479  6995 13157  8460 24857
     38445 13128  7563 20826  2520  8733 28568 28781 26528 26170 43621 43553
     50049 48503 20788 49606 17234 52355 33067 39853 16596 15884 20497 26067
     10862 16952 23999 49318 27034 29586 26083 14111 38166 15699 42748 40115
     40542 27686 39352 49391 51059 30629 44202 46940  4028 30180 14982 42745
     12099  7744 27331 27451 28785 18582 22066 52284  6144  7878 48822 15818
     43693 48706 48703 13299 23827  2003 49780 45864 46123 51203 51714 29798
     33802  6940 46106 29169 48965 21151 16818 32567 47249 46883 10516 14673
     19343 42094 20109  6977  9463 40285  3530 23794  9154 43681 36166 17690
      9033 32548 30111 33538 17623 15940 21540  8070 15374 10973 38927 21517
     28303  3462 32177 38823 25994 16621 40144 11428 21997  5474 52680 48498
      1148  2803 23877 17200 45252 39170 33883 23123 24340 15828  1797 34047
     18655 42970 44730  4129 36785 32847 10615 27877 43056  2005  3452 46769
     14901 21677
1439:45607 45649 30023 12305 11642  8213 18403 27234 49675  4909 51083 10364
     35028 19721 20300 44771
1440:27976 47883 11470 45833 49970  5746 29652 31565 10341 37078 52527 51969
     34538  1225 25196 12376  3892 16193 40761 11219 21652 47994  9137 35137
     35306  6646  7110 45394  8481 43665 26491 29723 42606
1441:13030 14986  1151 19573 26912 27392 27665  1850 15855 47142 33995 36753
     36599 52642  7188  9482 19017 14407 10048 49543 33638 39675 27772 12246
     10226 28180 43354 51621
1442: 1228 34345 10925 33843 35457 39284 33732 23173 15711 49646 49636 13998
     13922 52585  7939 27537 14953  2698 44198  5577 15542 36663 21851  5519
     51097
1443:38221 48245 16096 28982 26356 38896  5331  8368 12740 35493 21845
1444:18890 19612 17004 21113 23498 24197 36276  1947 18793 22645 25645 49770
     14681 36643 20761 52408 43608  1157 15417  8764 27207 18887 34482 31190
     19302 11526 26237  8868 44710 37464 14743 46707 39877  7122 39171 47099
     36198  1013 13948  6874 43180 10060 20501 17952
1445:29858 28235  6403 36352 37920 39241 35873 24899 46236  8586 17121 35949
      1147 51914  5803 29250  6772 46218 14424 22022 48436 37729 16018  9396
      1223 45124 28799 47749 47866  6989 13236 21663 22372 14904 30177 28336
      9750 20168
1446: 5239 36320  8299 14864 50382 11986 47037 28028 30510  1724 11433 41363
     42378 15782  2473 27620  7203 37326  3635 44043 42264  8812 18404  3706
     44884  9859 25257 39081 25100  9624 13566 34551 44761 23038 20526 49800
     37924  6814 10461 23815 24878  1129 38019  7118 34387 21987 46230 17751
     10610 13224 32473 15796 11370  3222 12472 16966 11163 47126 33264 51590
     34686 35977 15553 10697 45993 48417 48439 23105 45734 50743 31535  9047
     34007  1159 45281 33003 14998
1447:15163 12319 11917 42486  6295 26883  8617 20762 28417 28447 28446 26300
     28378 28418 28381 10434 28189 25926 26636 47109 14054 14053  2045 41058
     11799 33051 29715 27472  7187 52028  8191  6166 33873 18909 20431 27301
     15147 20392  3127 48001 28329 34254 47653 19615 38164 19330  7213 26166
     44399 27856 20629 11568  7398 31949 26704 19476 33398  4046 11268 28770
     31096  9986 49996 27635 38533 36266 16977 32253 43691 41890 13542 47273
     40592  8278 41071 38713  4327 13352 43053 20611 28739 28523 12649 16945
```

```
      3394 36830 37664 18182 32341 25990 49284 28482 28407 28440 28414 49949
     28438 20352 28346  5283  9565 18134 26172 26180 11449  8695 12870 15275
      9127  4850 26336 26234 26373 26376 26239 50856 38126 26378 26260 44441
     50701 50071 41147 41178 41119 41116 41154 41213 41863 28265 28263 28285
     18465 28444 28353 28350 28476 28475 28479  2857 34545 27513 26208 26335
     28411  5574 24162 41953  7703 12092 28241 41888 41886 41891 41868 38838
     26309 27029 14761 27333 27293 16641 26997 26168 26329 26204 26198 26267
     27002 27027 26306 33314 50760  8794 36265 46508 29406 45725 14765 48068


1448: 3109 31167 48648 51613 38684 13483 47553 21927 32033  6256 29679 44114
     23648 15682 31986 38210 31171 36588 47367 30051 22711 46947  2418 37010
     13541 46262  9918 50692  8867 30780 15678 27550 45373 41898 51326 25377
     26797 24503 47566  3786 11824 52060
1449:41024 41026 19466 28151 47540 45697 15736 35582  1419 46732 33606 30194
      5616 15975  2884 43385 20866 32155  5204 40084  8713 40085 16462 51258
     42081 36071
1450:21801 21800 21797 40588 15756 16871 16869 16867 42609  8539  8542 47092
     23117 23115 10379 17798 24255 30251 11775 17681 19696  9953 35049 35007
     35055 24012 24011 21868 21863 27442 15769 23855 22527 42691 47845  2164
     12236 45875 45241 45874 45955 45938 23330  8803 12145 36494  6474 42375
     37187 36366 44913 10273 10978 10066 48742 17102 27785 19875 13320 17186
     50111 14056 50305 51412 50861 22222 21195 42716 25220 12660 25111 32870
     13803 13818 32724 26523 19091 52504 38306 49385 22126 18701 31484 46513
     15876 42409 45259 19205 19206 23213 36226 36229 36254 30466 30345 29684
     30487 30347 13061 30349 19834 18879 51013 42232 31254 16830 51432 18743
     45712 13621 26572  9304 14785 45163 25562  9708 34749 20166 18729  3542
     29174 20774 17853 17852 39332 20655 13771 44668 43917  4417 10622 16723
     30113 13901 22576  1901 37271 38099 45205 30855 40050 50974 47545 30185
      5108 33650 32990 33677 33649 33646 32968 33675 32966 47207 46612 46403
     46609 46459 46435 46407 47210 42505 22506 35133 19562 52111 52112 49580
      6901 13798 36921 36913 28657 36915 36919 36910 36881 29813 36876 24046
     50516 11724 36801 29870 32310 28942 28232 27606 39132 17573 17580 17574
     17577  8185 17576 22769 22771 22770  3784  3808 15006  4070 35930 40802
     29482 51578  6374 41885 52003 12062 47214 32255 32280 32271 32252 32304
     46473 23476 18222 41412  3384 24249 37318 10590 36150 42058  5692 32215
     21810 36203 36202 11812 27840 21568 50462 21289 12538 12555  5631  5659
      6462  5629  5657  6460  7179 35075 21197 33610 29872 37259  3170  3165
     32743 37257  5180 36873 11557  8429  3938 33204 44795  4108 19824 19811
     19825 19815 19820 24792 24777 24776 24791 25676 30107 30085  9534 31000
     31003 31024  9537 31026 10146 29340 29588  5243 47451 28915 39685 45379
     27938 34226  7796 34008 15746  4133 48901 36429  8880 51160 24895 36859
     36862 21897 21905 21902 21898 21876 21872 21903 24581 21874 21901 21835
     38042 38029 33189 37440 38002 38048 38025 38000 38027 38023 38006 38005
     38045 38021 13808 19564 22406 31514 10501 20807 19543 40667 25191 34256
     45127 31197 42897 38547 46851 30712 50254 41427 50252 16138 27874 14836
     14623  6950 33628 32404 50113 41430 50255 25462 50250 10429 48077 18725
     17427 40082 29532 32531 32537 32535 32501  1560 22198 19751 32533 32506
     32529 32504 25760 32503 23532 47580  1848  8877 17652 17654 52518 52517
      1845  1852 13804 17657 13800 47049  1843 29611  8130 40242 25241 40214
     11977 20323  2190 30247 25622 11991 11697 30256 44680  7246 42736  9133
     42715 42730 42732 52068 32319 32223 32196 11235 14011 44214 25615 20726
     25819
1451: 5511 36745 27549 36870 36868 11782 11779 11778 11784 48194 11761 11759
     11774 10329 36967  6965 39762 44390 40975 44388 32363 32360 32357 32356
     32330  2388 27104 24833 30498 21769 46499 48552 47745 30473 49446 40227
      7476 15497 15495 25534 10930 16477 41972  3383 30013 22869 29854 18618
      4853 49853 49425 23976  8208 40417 49427 49429 47058 22403 52515  2092
     37082 24598 52215 15532 44793  6807 46021 15377 10352 23764 45751 25542
     49332 23887 49053  5064 12730 32994 18007 50092 27323 50845 28066 41401
     44433 49909 21026 10735 19010 13788 35627 12080 49883 20995 30615 39046
     36368 44832 21194 29819 46409 46025  2922  2926 31926  4262 28317 44196
     39593 47947 13077 22889 22868  1743  4886 14808 45355 27109 33431 25605
```

208

```
      25602 25604 48609 35318  3904 25535  9249  9870 26929 43991  8611 19080
      14274 28318 48412 33512 14910  4676 35027 46520 12210 38268  3265 35992
      52528 13869 48572 35303 24453 24449 24452 24450 27111  5405 13978  5361
       9029  5333 17183 17181  7576 38581  3038 32137 11758 11755 36864 11797
      11804 11802 11798 28302 28305 28298 28300 28323 28117 42600  4406  4525
      37077 41934  6342 10223 36311 44767 45110  4385 15506
1452:39574 12213
1453:    0
1454:13314 41907  9165  4356 36353 36879 14269 34576 18882  7822  3449 48859
      43183 17117  3091 26965 32116  7769  8253 44045 15936  2461 15593 33840
      33824  9272 24384 44354 22042 22087 19235 39688 34213 27146 18483  7100
       7103 35123  4772 21051 28497 45467  8410 21739 45775 20044 25363  1730
      50753 14712  1763 22707  4660 34969 26418 38765 12032  3903 27084 19862
      18941  4728 44695 23367 52244 48429 21848 33299 46478 42772  7723 16714
      42536 41686  6268 33769 21549  4154  4314  5057 26676 35533 27508  7711
      11572 33108 50238 35179 46327 40288 13042 13049 13039 13047 40289 10023
      38092 26430 25937 41160 41827 38725 11289 11291  9901 26273 50808 35051
      47778  4209  3804 14138 51273 32965 51495 30384 51473 22622 38366 34621
      39980 47782 11979 48581 23768 48203 27059 26825
1455:15673 50685  9785 50445 16789 46044 23788 48763 28216
1456:13219 34284 18713 32396 25428  7846 34418 23139 23119  7307 35664 23684
      44810  8929  9755  9756 49300 12202 46608 16730 39139 36843 29799 24248
      47228 19261 25168 44232  2109 12009 24228 39320 11658 38642 51732 34673
      38327 18905 49130 19829 47326 42292 27243 33137  4605 47563  3941  5035
      44053 17257 12653  4733   785  8297 50858  1598 11603 51917 38184 52113
      11209 20928 50226  8672 47130  5368 47286 39635
1457:20010 35449 13407  2002 39975  7191 14689  2497 34168 37799 33892 38430
      24194 37553 16625  4468 15670  5703 25738 39705 29316 45098 49674 21607
      42130 32306 32300 14472 28116 36856 38816 17960  8986 36886 45810 45813
      17042 17011 45828 45814 17044 45815 45827 38975 42723 11152 25857 42442
      29272  2370 37548 45106 47799  5284 35695 50198 50199 50219 50217 41677
      27360 36858 40472 40471  4122 23907  4120 44091  5219  6519 15812  2466
      19503 19501 49346 49343 49345 25088 49357 49342  7560 36890 36853 36850
      36851 36822 36889 36825 15742 14184  8016 37551 36883 36885  5216  5217
       5197 15745 36891 25899 42322 36829 36818 36675 36673 36821 36827 36657
      36680 36654 36677 36892 35022 47642 48112
1458:12223 34113 21896 18251 18818 15894 18816 46991  4686  2138  4275 13139
      13135 20360  3656 23322  6113  1686  5694 13219  5724  2940 13004 34678
       9593 38333  4754 47706 30909 33999 38086 30368 25429  8077  5184  5189
       1564  9245  5477  6419 37806 27694 19606 38263 39878 27950 27949 20267
      10551 32354 10560  7581 14563 38261 10557 14560 24583 14141 39396 11958
      11956  8133 22357 43781  3831 15926 29855  2855  2160 46406 26943 15030
      37367 34165 46222 14807  4467  4471 23709  4793 51428 22340  9842  3755
      22621 19641 42073 46115 14206 47933 40028 25891 13785 37369 17354 20776
      15046  6778  7846 34418 38687 52012 31437 23139 20454 42775 26130 36929
      36954  6622 36952 23473 29687 30637 32291 29266  7307 17856 35664 11653
       5010 31856 36804  8843 49204  3883 27177  8929  3742  9755 42504 47630
      51577 44306 46592 19156 14032 44243 47491 49558 51319 10934  3326 46124
      46964 47879 12663 15345 22677 18377 30522 50770  8969 36843 14046 44799
      21423 25618 44533 25620  1776 42360 13580  1773 35135 25808 31600 12250
      12468 40391  8519  3898  5468  7375 32633  6981 30448  8670 12075 23018
       3911 36567 16881 29799 30934 44351  4398 38133 41929 20601 29920 33826
      24248 44700  5083 38514 52757 51587 52759 38530 38510 36742  2647 32053
      23722 23580 22777 11726 13833 13831 11727 16187  6286 48151  6292 32249
      10763 11438 19261 50708 46673 25168 13153 18537 18538 44253 13742  6259
      32324 44079 30685  2087 23807 21891 30734 11840  7171 52702  1919 12011
      41680 23184 39131 47031 37149 29772 43133 12572  5929 21957 26881 35564
      13642 43989 50239  5633 15331 29380 15360 29414 15293 26610 15541 23274
      45141 15305 39527 39532  5944  1523 29065 14928 24985 16858 24984 51729
      39872 21045 12898 31471 45044 49207 28062 31498 32884 34236 34234  3098
      18609 18608 40759 18607 49408 49415 49063  6920 50732  6919 29229 42623
       7820 16326 35601  2685 37934  5304 13897  2687 14220  2267 48416  8168
```

```
22966  1563 10939 11500 43837 52271 52295 43124 22140 44011 43985  3826
 6051 22321  3224 10407 23166 48871 33984 20211 33216 25149 38642 11658
35898  9209 37102 35410 10033  7488  8350 28801 24174 12551  9469 12533
10838 52447 39657 39654 37682 20769 21052 23960 23961  1836 23897  4515
 3019 51607 50959  3077 50960 51732  2876 33277 15927  4252 24398 19408
25754 18530 21094 23172 18405 23169 13364 43717 42628 51476 46798 50124
 5662 20080 17604 36540 38128 38326 12219 15868 18905 49130 44151 47769
45312 20215 20214 38429 41847 41846 35869 35016 13641 21260 48686 42722
40902 48208 16502 19827 15870 44705 47326 42292 45031 35660  2067 33137
 4605 30942 31583 44830 17895 10469 34602 40026  8962 17356  1949 51804
14362 37418  9156 50860 28397 50857 23302 29891 12762 41267  5337 12842
42542 45195 23096 20069 47437 50671  2096 43324 45674 18226 45673 27099
25379 27095 27097 27102 49337  3173  8425 42714 47563 20330 31732 48025
17434 34765  8240  8237 36289 29202 11024 14642 46195 46204  7035 19124
11753 21478 30568 15999 12683 12690 30772 37848 17411 38203 49221 39037
47388 38180 25044 25046 18257 27368  6170 35010  3555 16407 16404 23753
45261 38191  8848 20689 37263 41203 32321  6240 26699 41742  3937 27416
34693 16124 16955  5482 47714  4309  2622 47716  2013 14033 51168 45604
46431 42988 20607 19675 19515  5035 28484 26657 10869 10873  7817 22094
48363  7936  3052 33460 18888 37493 43933 41699 18776 40049 32049 21347
34511 24284 24302  3424  2605 34915 10670 30896 32764 42588 17014 13191
46420 22467 29084 34274  4733 45180  3825 47230 23593 39473  4759 40150
30544 35314 26977 23982 39469  8632 31291 42927 36177 16431 13005 10669
 8633 47622 22885  9594 48244 52455 41954 40196 42849  9759 40192 34133
 8480  8544 37539 25148 39672  7485 25173 39649 40964 41737 40998  5602
25938 48757 42015  3278 39852  6839 21202 11912 16204 14850 52667 10922
42773 42806 42778 14894 51917 38184 18049  2239 35198 36716 21936 44237
 3693 10880 52113 11209 28601  8569  8565 35563 26126 26216 31469  4200
42793  4264 29100  4757 28775 19819 19823 14332  4174 23477  8672 25394
41270 44018 33478 14021 12603 48486  9031 29968 33278  7326  3962 14596
 8561 47130 49015 34876  3553 17406  5368 24552 47286  7403 11752 36641
36644 35812  7183 35658 21253 21258 27423 43867 51360
1459:26671 40876 52744  2971 12201 45561  4549  5605 36167 37754 37850 46901
49877 42670  7475 37310 14815 36837 52267 31408 15217 43657 52630 34118
47365 26467 35072 17471
1460:47382 12868 41072 24447 52744 45625 14132  2018  2971 10465 25855 45561
 4549 24784 24406  6453  5605 37754 48121 39810 43632 43105 49877 14494
 4782 39182 42670 47195 37698 25846 41870 13127 12828  1902 13211 11620
 6447  8119  9039 11948  7644 26403  4392 29550 11967 42872 20434 17677
38787 43137 48269 44982 22585  5165 26743 33588  5715 14030  7475 37310
49511 48811 20333 35823 23822  1630 38644 23806 26069 14815 36324 14324
36837 34528 52267 34775 31408 15217 37805  7583 16194  6683 43657 21649
52630 27454 34118 48348  1243 46878 17407 14936 17471  1459 35072 51365
 1738 10522 23402 20949 13853  4049 47365  1461 12160 40634 47552 49378
24707 51084 21155 43272 11325 41264 34460
1461: 1460 42457 47382 24447 52744 45561  4549 27942 17774  9057 48121 39810
43632  5312 49877 14494 25846 39674 11967 20434 42872 17677 38787 43137
23822 48811 20333 35823 14030  5715 49511 33588 14324 36324 34528 16194
34775 37805 21649 46878 48348  1243 17407 47365 48105 26112 40634
1462:48026  1896 31262 14920 49939  3230 13471 24221  4063 43148  7321 33643
37930  6887 19796 14426  8160
1463:46119 19968 31641 28208 10878  2950 38735 52341  8620  1248 22517 33165

1464:48137  5099 32578 49421 45982 37971  1251  2144 25832 10753 48761 43974
13213  8241 38923
1465:20379 24475 23870 35312 34711 51247 35274 39936  7558 36956 24660 28080
 1851 21288 16585 52188 17860  9323  5322 15726  7265 19693  5011  5017
26889 23839
1466:48344 51784 11271 35100 36138 17461 30231 24574 51890 15951 12840  2149
20632 47664  9994  8259 13624 32431 11319 43984 36230 11916 10915 38998
45509 47247  5764 20527 50796 19517 22168  1583 10078
1467: 4857 22220 17103 11090 28291 51586 27820 50398 25334 41381  6604  6601
```

```
        34393 34339 45172 43892 44631 18874 18967 48587  9648 29982 41149 50603
        10187 24962 24577  8694  4098 16624 44970
   1468:38907 45217 13574 14914 29876 19016 42372  5248 26861  1759  1472 17180
        41855  9747  4791 34828 19676 10412  8971  7441 10993 51727 43702 41132
         1257 50091 13358 35681  5019
   1469:42256 47025 18461 42812 45244  8892  7694 24840  4530 26599 39913  3978
        35103  8560 37074  2506
   1470:26619 48186  1310 10567 45617 11848  4068 11147 45432 28158 45436  6349
         6151 51070 21206  7425 39033 24218 27659 23690 18256 41280 41283 49571
        22201 10188 21879 44083 17859 23087 39967 38862 39634 23564  7190 15878
        46762 22118 31457 46573 45809 14037 22654 21890  9996  9929 49128 23676
        23187  8789 44068 39147  2732 47980 49507 21118 47893 33879 46423 32984
        46090  3801 25939  1051 21416 32525 16092 25447 48174 52606  3515  3517
         3490  3518 20235 29501 31530  9556 38291 42662 15730 40758 27775 16430
        35622 29820 27773 15847  6003 42585 22838 28134 33696 20691 28169 28802
        38059 21777 23464 36486 26331 45998 20343 22520 10581 12591 36620
   1471:36800  4449 46291 35473 21470 25695  3175 51545 35012 47982 11541 37429
        23902 25933 34985 21465  9048  9046 28067 49568 37726  4458 10855  7600
         7871 27752 38979  8944  1262 20518  3791 46452 30403  9214 41916 20029
        22386 41945 13538 22388 37778 37777 48173 26782 26700 42524 47291 47295
        47299 33627 47319 47322 47317 35889  8106 39866   985 48977 18280  5352
        30789 37717 51149 28161 18204 32882  8375 29522  3703 36718 25511 52297
        27470 51081 12976 11924 25932 45462
   1472:45217 38907 13574 19016 42372  1468  1494 34550 35681  5019  8637 12539
        11628 10883 10412 41132 42334 13358
   1473:20379 19799  5130 31935  6967 16690  1979 34711 11686 51247 45311  2122
         1851 16614 16587 16618 16585 52188 28759 41662 41515 19713 37592 19593
         6634  8964 40212 19693  5011  5017 46165 21871 26520
   1474:23656 52582  9125 49502 19425 32795  7146 43205 10826 12721 45779  5653
         3990 16534 24239 22328 46596 17669  7150 46813 38850 41586 29285 16896
         1263 30125 30567 12296 16482 25240  7506  2243 18378 27641 34728 37239
        19447 25836
   1475: 7259 26533  2564 46567 31528  2669 45260 52197 21142 48298 17493 23990
        24907 39587 35850  6903 16224 15596 15110 23299 36473 13763  5332  9211
        13750 19753 11562 20134 10802 20605 11048  8564  9446 30671 17196 49233
        33621 13572 14667 44139 43591 50802  3689 13113 11315 25131  5067 24795
        25006  5735 18796  6553 48311 52033  7469 36598  1300 48890 19810 50542
        34344 51393 15645 15327 34659 24543 21559 20903 39114  7559 16944 27391
        17058 23705 28966 47374 26561 27645 39915 18290 43703 35270  6925 23226
        32856 23058
   1476: 5099 35463 10568   945 19066 48204 25832 16385 10753 48761 41364  6752
        52423 20242 31159 43974  8590 26595 24422  8150  6964 16990
   1477:18766  1921 18164 21235 24680 40703 48694 13418  5479  6341 12614 26638
         8082 32143  2166 32928 47561 43144  7945 11217 28424  2651  3581 38988
        13457 49609  2601 42959 11184 33107 37504  1571 27726 49768  7747  9239
        33827 22195 34757 13702 30410 24173 49358 27087 48521 25109 41716 18323
        42794 38737 18534
   1478:35497 14394 20129 32278 27975 40550 47476 29646 29645 27760 42218 14683
        11910 47017 47013 10300 32241 21069 13859 40132 25636 46734 42518 41621
        18772  2580 33830  9326  4302  6179 24802 20016 50497 14715 37533 46157
        25376 52655 14934 18600 51891 47894  4000  4005 11690 18543 46061 47460
        44212 23918 26325 40024  3961 52719 15097  3874 48554 20344 21883 45575
        40423 40562 40636 15091 24036 36461 12385 48848 44256 40676 24146  4360
        30140  2908 18402 33997 32977 38034 47330 32287  3847  4502 28403 25114
        48928 29394 16399 31212 12761 52670 52051 52052 30146 30147  6146 49867
        50615  9205 15234 16989 13820 52185 22605 42448 36962  2568 22446 43904
        42844 24351 22559  4121  2312  7170 23197 27388 29951  7207  7178 11540
        46672 52594  5224 20929 39709 42280 22994  4802 48356 48382 31838 13290
        23671 41761 30066 48578 18288 31835  5291 14660 24765 32490 37361  8440
        20904 50220 39617 13056
   1479:45791 33533  6130 15570 13697  5862 16419 36034 39711 34861 29691  7870
        21032 37512 34878  6600 48032 44770  8718 14562 46438 50455 46168  7063
```

```
49911 37890 38060  6508 11142  9268 31609 16402 38396 32267 25272  8954
41691 10662 10691 10655 10666 10633 10635 10698 10722 10724 30600 48490
17484 42102 20412 50663 35354 33597  6033  7884 34614 10963 20606 33963
51348 23129 28954 12108 25843 35825 19772 23125  6245 48255 16861  7097
25659  3773 39854 52050 27457 12455 39860  3789 42441 28468  3859 30208
45144 40054 16423 31285 26808  3699 42497 18831 10000 43409 35291 27749
38455 16274 31418 15608 49944  5607 43381 38859  4225 37096 22964 45888
11228 44014 14649 35317 45514 18388 34832 19623 39704 39706 10696 10660
45551  1799 13704 19102  9976 19374  4720 48129  4711 52701 31226  3064
37305 20917 37224  5415 30805  4781  5556 43313 16045 21482 16210 11482
 8891 17679 17874 16390  4469  4890 12414 17380 43104 32980 35332 29525
13402 50927 27153 51912 20535 51328 35401  8786 12174  3082 22032 40849
37784 32397 46709 49618 45525 24757 28899 28900  8439 32035 37752  3713
 5551 52108 26767  4618 24467 52695 22410  9366 26829 27891 21984 10738
51720 47272  4013 46989  4577 23553  9660 14254 22074  8832 17105 39336
45207 28345  5630 20913 34014 19188 22939 28156  2903 44289 44912 14211
36274 42155 37021 21774 32247 41912 33770 24157 42172 35005 23743 13276
39951 18283  5782 14118  8104 14733 21031 47600 12632 17941 27934 17474
24361 42100 46233  6080 45064 15939  2623 36197 11199 10526 31028 31031
 2619  2828 29083 34662 46172 12346 26319 46658 16601 47607  5265 20544
26918 10687 27246 15814 32338 25405 39745 20118  4473 37038 39326 34313
17060 23351  7798 52259  7389 40993 33390 22983 48661 33911 29271 46020
50507 37881 40122 47816 27320 14352 50522 37684 23905 49495 35025  9833
16059 25794 28907 50470 13980 42150 19164 13017 33953 32735  8212 21308
31175 10699 12586 37769 37720  5398 39491 12124  8093  6472  6469 39450
13437  8663 31538  6909 33577 37925 35441 27199 26941 33837 27952 29980
44638  7974  7975  7979 38209 14326 47942 37188 21311 47271 30945 24375
16377  3412 43636 44543  5685 25312 19627 51813 25086 11786 11783 43517
35297 51418 11909  9058 50187 37054 25514 19712 31506 24214 24779 43872
13686  8802  7290  4446 25763 48020 38185 29353 32229 22648 22934 31170
 1726 24851 36073 36446 46519  1877 21954
1480:42836 44712 10942 51480 10383 18615  4639  1860 49062 39598 17499
1481:22279 33192 11483 33785 36983 10981  4860  6811 29520 42765 36170  5009
 9306 34249
1482:15441 52073 32147 25807 51485 33812 26776 37179 16383 31984 43362 36784
38867 17311 46295 33805
```

A plant cell with stably integrated, recombinant DNA comprising a promoter that is functional in plant cells and that is operably linked to DNA from a plant, bacteria or yeast that encodes a protein having at least one domain of amino acids in a sequence that exceeds the Pfam gathering cutoff for amino acid sequence alignment with a protein domain family identified by a Pfam name in the group of Pfam names consisting of bZIP_1, bZIP_2, Meth_synt_1, Homeobox, Succ_DH_flav_C, RWP-RK, Meth_synt_2, CTP_synth_N, WD40, Sigma70_r2, Sigma70_r3, Fer4, Sigma70_r4, Sigma70_r1_2, CMAS, Sugar_tr, Rubrerythrin, Pro_dh, Ldh_1_C, START, HATPase_c, Cpn10, Glycos_transf_1, Glycos_transf_2, Pkinase, KH_1, cobW, Ldh_1_N, DUF393, SecY, PCI, SRF-TF, IF4E, Lectin_lega, MatE, Dehydrin, Lectin_legB, Ank, 2-Hacid_ dh_C, Tic22, Chal_sti_synt_C, AA_kinase, ELFV_dehydrog_N, HLH, Ribonuclease_T2, HEM4, AT_hook, Peptidase_A22B, tRNA-synt_2b, Suc_Fer-like, Glyco_transf_20, MFS_1, HMA, Ketoacyl-synt_C, Steroid_dh, Hydrolase, Peptidase_CI, Ion_trans, Aa_trans, peroxidase, GAF, Cu-oxidase, ABC1, PMSR, B12D, Chromo, Lipase_GDSL, Ran_BP1, DUF125, Lig_ chan, GAT, Tub, NPH3, BAH, GFO_IDH_MocA, DUF6, Orn_DAP_Arg_deC, F-box, 3_5_ exonuc, NUDIX, Cyclin_C, Trehalase_Ca-bi, Acyltransferase, MtN3_slv, zf-B_box, PUA, AMPKBI, Peptidase_M20, Transaldolase, ketoacyl-synt, Cyclin_N, HisKA, Ribosomal_L7Ae, Methyltransf_11, Methyltransf_12, Hexapep, Ribosomal_S2, Jacalin, ERp29, MFMR, Usp, DUF641, Pyr_redox_dim, Auxin_resp, Inhibitor_I29, Transferase, cNMP_binding, BURP, Epimerase, Ribosomal_L39, Metallothio_2, Pyr_redox_2, WRKY, GSHPx, Kelch_1, Kelch_2, Aminotran_1_2, ABC_tran, UDPGT, Cystatin, YL1, AMP-binding, NTP_transferase, HALZ, Kunitz_legume, HSP20, DUF581, FGGY_N, Aminotran_3, PHD, B56, Aminotran_5, PSI_PsaF, malic, zf-C2H2, HEAT, UPF0057, Asn_ synthase, K-box, HAMP, PTR2, SapB_1, Ammonium_ transp, SapB_2, GATase, Pyr_redox, Cu-oxidase_2, Cu-oxidase_ 3, Cyclotide, Asp, M20_dimer, PA, Thiolase_C, FHA, YjeF_N, Citrate_synt, GTP_EFTU_D2, GTP_EFTU_D3, PK, GATA, Thiolase_N, Glycogen_syn, WHEP-TRS, B3, EF1_GNE, FAD_binding_3, ComA, Remorin_C, FAD_binding_7, RmlD_ sub_bind, CBS, ELFV_dehydrog, YL1_C, zf-Dof, Ribosomal_S11, ArfGap, GRAS, Metallophos, Annexin, Ras, NAC, Acetyltransf_1, Ribosomal_S17, NAF, DUF246, GST_C, CN_hydrolase, Na_Ca_ex, DUF1423, Ubie_methyltran, p450, PP2C, NAM, Histone, GST_N, Tubulin, 2-Hacid_dh, Ribosomal_L19e, CCT, Malic_M, PK_C, VHS, IPK, HSF_DNA-bind, Tubulin_C, Sina, JmjC, CH, Catalase, DUF250, HMG_box, PfkB, Yippee, DSPc, Pkinase_C, UbiA, Ribosomal_ S27, ADH_zinc_N, Zip, Globin, JmjN, Cys_Met_Meta_ PP, HI0933_like, GH3, Bromodomain, ERO1, DAO, DUF760, Methyltransf_2, Gp_dh_C, HGTP_anticodon, Methyltransf_3, Aldo_ket_red, Thioredoxin, NmrA, SelR, LEA_5, Orn_ Arg_ deC_N, Polysacc_synt_2, Gp_dh_N, NifU_N, GFO_IDH_MocA_C, Gamma-thionin, FBA_1, H_PPase, ADH_N, Heme_oxygenase, AUX_IAA, NAD_binding_4, Auxin_inducible, LIM, Response_reg, Dirigent, E2F_TDP, Di19, Alpha_ adaptinC2, efhand, ICL, Rieske, GTP_EFTU, ARID, adh_short, Transket_pyr, AA_permease, TPP_enzyme_C, NDK, RRM_1, Trypsin, Pro_ CA, Hexokinase_1, CBFD_NFYB_HMF, Glyco_hydro_38C, TPP_enzyme_M, TPP_enzyme N, Hexokinase_2, 3Beta_HSD, DUF788, Wzy_C, E1_dh, Glycolytic, RuBisCO_small, ZF-HD_dimer, DUF1530, PARP, Pyridoxal_deC, IlvC, Ribosomal_L1, Alpha-amylase, EB1, CorA, Sucrose_synth, PGAM, IlvN, MAP1_LC3, DNA_photolyase, PAD_porph, Abhydrolase_1, Glyco_hydro_16, NTF2, CobW_C, GATase_2, Cation_efflux, Gln-synt_C, VQ, DUF296, W2, Sam_1, SAM_2, Gln-synt_N, Transketolase_C, PEPcase, GRIM-19, Pkinase_Tyr, DnaJ, MIP, PRA1, Trehalose_PPase, Transketolase_N, LRR_2, KA1, Mpv17_PMP22, Reticulon, Trp_ syntA, YTH, Aldedh, zf-C3HC4, GIDA, Trp_Tyr_perm, UBA, PB1, PAS, Carb_kinase, zf-LSD1, CAF1, Xan_ur_permease, Hist_deacetyl, Cpn60_TCP1, XET_C, Ribosomal_L10e, Trehalase, ubiquitin, Glyco_hydro_38, AP2, Myb_DNA-binding, APS_kinase, PBD, FAE_3-kCoA_syn1 wherein the Pfam gathering cuttoff for said protein domain families is stated in Table 12 ; wherein said plant cell is selected from a population of plant cells with said recombinant DNA by screening plants that are regenerated from plant cells in said population and that express said protein for an enhanced trait as compared to control plants that do not have said recombinant DNA; and wherein said enhanced trait is selected from group of enhanced traits consisting of enhanced water use efficiency, enhanced cold tolerance, increased yield, enhanced nitrogen use efficiency, enhanced seed protein and enhanced seed oil.

A plant cell as defined above, wherein said protein has an amino acid sequence with at least 90% identity to a consensus amino acid sequence in the group of consensus amino acid sequences consisting of the consensus amino acid sequence constructed for SEQ ID NO: 742 and homologs thereof listed in Table 2 through the consensus amino acid sequence constructed for SEQ ID NO:1482 and homologs thereof listed in Table 2.

A plant cell as defined above, wherein said protein is selected from the group of proteins identified in Table 1.

A plant cell as defined above further comprising DNA expressing a protein that provides tolerance from exposure to an herbicide applied at levels that are lethal to a wild type of said plant cell.

A plant cell as defined above, wherein the agent of said herbicide is a glyphosate, dicamba, or glufosinate compound.

A transgenic plant comprising a plurality of the plant cell as defined above.

A transgenic plant as defined above, which is homozygous for said recombinant DNA.

A transgenic seed comprising a pluarility of the plant cell as defined above.

A transgenic seed as def. above from a corn, soybean, cotton, canola, alfalfa, wheat or rice plant. Non-natural, transgenic corn seed as def. above, wherein said seed can produce corn plants that are resistant to disease from the Mal de Rio Cuarto virus or the *Puccina sorghi* fungus or both. A transgenic pollen grain comprising a haploid derivative of a plant cell as def. above.

A method for manufacturing non-natural, transgenic seed that can be used to produce a crop of transgenic plants with an enhanced trait resulting from expression of stably-integrated, recombinant DNA comprising a promoter that is (a) functional in plant cells and (b) is operably linked to DNA from a plant, bacteria or yeast that encodes a protein having at least one domain of amino acids in a sequence that exceeds the Pfam gathering cutoff for amino acid sequence alignment with a protein domain family identified by a Pfam name in the group of Pfam names consisting of bZIP_1, bZIP_2, Meth_synt_1, Homeobox, Succ_DH_flav_C, RWP-RK, Meth_synt_2, CTP_synth_N, WD40, Sigma70_r2, Sigma70_r3, Fer4, Sigma70_r4, Sigma70_r1_2, CMAS, Sugar_tr, Rubrerythrin, Pro_dh, Ldh_1_C, START, HATPase_ c, Cpn10, Glycos_transf_1, Glycos_transf_2, Pkinase, KH_1, cobW, Ldh_1_N, DUF393, SecY, PCI, SRF-TF, IF4E, Lectin_legA, MatE, Dehydrin, Lectin_legB, Ank, 2-Hacid_dh_C, Tic22, Chal_sti_synt_C, AA_kinase, ELFV_dehydrog_ N, HLH, Ribonuclease_T2, HEM4, AT_hook, Peptidase_A22B, tRNA-synt_2b, Suc_Fer-like, Glyco_transf_20, MFS_1, HMA, Ketoacyl-synt_C, Steroid_dh, Hydrolase, Peptidase_CI, Ion_trans, Aa_trans, peroxidase, GAF, Cu-oxidase, ABC1, PMSR, B12D, Chromo, Lipase_GDSL, Ran_BP1, DUF125, Lig_chan, GAT, Tub, NPH3, BAH, GFO_IDH_MocA, DUF6, Orn_DAP_Arg_deC, F-box, 3_5_exonuc, NUDIX, Cyclin_C, Trehalase_Ca-bi, Acyltransferase, MtN3_slv, zf-B_box, PUA, AMPKBI, Peptidase_M20, Transaldolase, ketoacyl-synt, Cyclin_N, HisKA, Ribosomal_L7Ae, Methyltransf_11, Methyltransf_12, Hexapep, Ribosomal_S2, Jacalin, ERp29, MFMR, Usp, DUF641, Pyr_redox_dim, Auxin_resp, Inhibitor_I29, Transferase, cNMP_binding, BURP, Epimerase, Ribosomal_L39, Metallothio_2, Pyr_redox_2, WRKY, GSHPx, Kelch_1, Kelch_2, Aminotran_1_2, ABC_tran, UDPGT, Cystatin, YL1, AMP-binding, NTP_transferase, HALZ, Kunitz_legume, HSP20, DUF581, FGGY_N, Aminotran_3, PHD, B56, Aminotran_5, PSI_PsaF, malic, zf-C2H2, HEAT, UPF0057, Asn_synthase, K-box, HAMP, PTR2, SapB_1, Ammonium_transp, SapB_2, GATase, Pyr_redox, Cu-oxidase_2, Cu-oxidase_3, Cyclotide, Asp, M20_dimer, PA, Thiolase_C, FHA, YjeF_N, Citrate_synt, GTP_EFTU_D2, GTP_EFTU_D3, PK, GATA, Thiolase_N, Glycogen_syn, WHEP-TRS, B3, EF1_GNE, FAD_bindmg_3, ComA, Remorin_C, FAD_binding_7, Rm1D_sub_bind, CBS, ELFV_dehydrog, YL1_C, zf-Dof, Ribosomal_S11, ArfGap, GRAS, Metallophos, Annexin, Ras, NAC, Acetyltransf_1, Ribosomal_S17, NAF, DUF246, GST_C, CN_hydrolase, Na_Ca_ex, DUF1423, Ubie_methyltran, p450, PP2C, NAM, Histone, GST_N, Tubulin, 2-Hacid_dh, Ribosomal_L19e, CCT, Malic_M, PK_C, VHS, IPK, HSF_DNA-bind, Tubulin_C, Sina, JmjC, CH, Catalase, DUF250, HMG_box, PfkB, Yippee, DSPc, Pkinase_C, UbiA, Ribosomal_S27, ADH_zinc_N, Zip, Globin, JmjN, Cys_Met_Meta_PP, HI0933_like, GH3, Bromodomain, ERO1, DAO, DUF760, Methyltransf_2, Gp_dh_C, HGTP_anticodon, Methyltransf_3, Aldo_ket_red, Thioredoxin, NmrA, SelR, LEA_5, Orn_Arg_deC_N, Polysacc_synt_2, Gp_dh_N, NifU_N, GFO_IDH_MocA_C, Gamma-thionin, FBA_1, H_PPase, ADH_N, Heme_oxygenase, AUX_IAA, NAD_binding_4, Auxin_inducible, LIM, Response_reg, Dirigent, E2F_TDP, Di19, Alpha_adaptinC2, efhand, ICL, Rieske, GTP_EFTU, ARID, adh_short, Transket_pyr, AA_permease, TPP_enzyme_C, NDK, RRM_1, Trypsin, Pro_CA, Hexokinase_1, CBFD_NFYB_HMF, Glyco_hydro_38C, TPP_enzyme_M, TPP_enzyme_N, Hexokinase_2, 3Beta_HSD, DUF788, Wzy_C, E1_dh, Glycolytic, RuBisCO_small, ZF-HD_dimer, DUF1530, PARP, Pyridoxal_deC, I1vC, Ribosomal_L1, Alpha-amylase, EB1, CorA, Sucrose_synth, PGAM, I1vN MAP1_LC3, DNA_photolyase, PAD_porph, Abhydrolase_1, Glyco_hydro_16, NTF2, CobW_C, GATase_2, Cation_efflux, Gln-synt_C, VQ, DUF296, W2, SAM_1, SAM_2, Gln-synt_N, Transketolase_C, PEPcase, GRIM-19, Pkinase_Tyr, DnaJ, MIP, PRA1, Trehalose_PPase, Transketolase_N, LRR_2, KA1, Mpv17_PMP22, Reticulon, Trp_syntA, YTH, Aldedh, zf_C3HC4, GIDA, Trp_Tyr_perm, UBA, PB1, PAS, Carb_kinase, zf-LSD1, CAF1, Xan_ur_permease, Hist_deacetyl, Cpn60_TCP1, XET_C, Ribosomal_L10e, Trehalase, ubiquitin, Glyco_hydro_38, AP2, Myb_DNA-binding, APS_kinase, PBD, FAE_3-kCoA_syn1; wherein the gathering cutoff for said protein domain families is stated in Table 12; and wherein said enhanced trait is selected from the group of enhanced traits consisting of enhanced water use efficiency, enhanced cold tolerance, increased yield, enhanced nitrogen use efficiency, enhanced seed protein and enhanced seed oil, said method for manufacturing said seed comprising:

(a) screening a population of plants for said enhanced trait and said recombinant DNA, wherein individual plants in said population can exhibit said trait at a level less than, essentially the same as or greater than the level that said trait is exhibited in control plants which do not express the recombinant DNA,
(b) selecting from said population one or more plants that exhibit the trait at a level greater than the level that said trait is exhibited in control plants,
(c) verifying that said recombinant DNA is stably integrated in said selected plants,
(d) analyzing tissue of a selected plant to determine the production of a protein having the function of a protein encoded by nucleotides in a sequence of one of SEQ ID NO:1-741; and
(e) collecting seed from a selected plant.

A method as defined above, wherein plants in said population further comprise DNA expressing a protein that provides tolerance to exposure to an herbicide applied at levels that are lethal to wild type plant cells, and wherein said selecting is effected by treating said population with said herbicide.

A method as defined above, wherein said herbicide comprises a glyphosate, dicamba, or glufosinate compound.

A method as defined above, wherein said selecting is effected by identifying plants with said enhanced trait.

A method as above, wherein said seed is corn, soybean, cotton, alfalfa, wheat or rice seed.

A method of producing hybrid corn seed comprising:

(a) acquiring hybrid corn seed from a herbicide tolerant corn plant which also has stably-integrated, recombinant DNA comprising a promoter that is (a) functional in plant cells and (b) is operably linked to DNA that encodes a protein having at least one domain of amino acids in a sequence that exceeds the Pfam gathering cutoff for amino acid sequence alignment with a protein domain family identified by a Pfam name in the group of Pfam names consisting of bZIP_1, bZIP_2, Meth_synt_1, Homeobox, Succ_DH_flav_C, RWP-RK, Meth_synt_2, CTP_synth_N, WD40, Sigma70_r2, Sigma70_r3, Fer4, Sigma70_r4, Sigma70_r1_2, CMAS, Sugar_tr, Rubrerythrin, Pro_dh, Ldh_1_C, START, HATPase_c, Cpn10, Glycos_trans_1, Glycos_transf_2, Pkinase, KH_1, cobW, Ldh_1_N, DUF393, SecY, PCI, SRF-TF, IF4E, Lectin_legA, MatE, Dehydrin, Lectin_legB, Ank, 2-Hacid_dh_C, Tic22, Chal_sti_synt_C, AA_kinase, ELFV_dehydrog_N, HLH, Ribonuclease_T2, HEM4, AT_hook, Peptidase_A22B, tRNA-synt_2b, Suc_Fer-like, Glyco_transf_20, MFS_1, HMA, Ketoacyl-synt_C, Steroid_dh, Hydrolase, Peptidase_CI, Ion_trans, Aa_trans, peroxidase, GAF, Cu-oxidase, ABC1, PMSR, B12D, Chromo, Lipase_GDSL, Ran_BP1, DUF125, Lig_chan, GAT, Tub, NPH3, BAH, GFO_IDH_MocA, DUF6, Orn_DAP_Arg_deC, F-box, 3_5_exonuc, NUDIX, Cyclin_C, Trehalase_Ca-bi, Acyltransferase, MtN3_slv, zf-B_box, PUA, AMPKBI, Peptidase_M20, Transaldolase, ketoacyl-synt, Cyclin_N, HisKA, Ribosomal_L7Ae, Methyltransf_11, Methyltransf_12, Hexapep, Ribosomal_S2, Jacalin, ERp29, MFMR, Usp, DUF641, Pyr_redox_dim, Auxin_resp, Inhibitor_I29, Transferase, cNMP_binding, BURP, Epimerase, Ribosomal_L39, Metallothio_2, Pyr_redox_2, WRKY, GSHPx, Kelch_1, Kelch_2, Aminotran_1_2, ABC_tran, UDPGT, Cystatin, YL1, AMP-binding, NTP_transferase, HALZ, Kunitz_legume, HSP20, DUF581, FGGY_N, Aminotran_3, PHD, B56, Aminotran_5, PSI_PsaF, malic, zf-C2H2, HEAT, UPF0057, Asn_synthase, K-box, HAMP, PTR2, SapB_1, Ammonium_transp, SapB_2, GATase, Pyr_redox, Cu-oxidase_2, Cu-oxidase_3, Cyclotide, Asp, M20_dimer, PA, Thiolase_C, FHA, YjeF_N, Citrate_synt, GTP_EFTU_D2, GTP_EFTU_D3, PK, GATA, Thiolase_N, Glycogen_syn, WHEP-TRS, B3, EF1_GNE, FAD_binding_3, ComA, Remorin_C, FAD_binding_7, RmlD_sub_bind, CBS, ELFV_dehydrog, YL1_C, zf-Dof, Ribosomal_S11, ArfGap, GRAS, Metallophos, Annexin, Ras, NAC, Acetyltransf_1, Ribosomal_S17, NAF, DUF246, GST_C, CN_hydrolase, Na_Ca_ex, DUF1423, Ubie_methyltran, p450, PP2C, NAM, Histone, GST_N, Tubulin, 2-Hacid_dh, Ribosomal_L19e, CCT, Malic_M, PK_C, VHS, IPK, HSF_DNA-bind, Tubulin_C, Sina, JmjC, CH, Catalase, DUF250, HMG_box, PfkB, Yippee, DSPc, Pkinase_C, UbiA, Ribosomal_S27, ADH_zinc_N, Zip, Globin, JmjN, Cys_Met_Meta_PP, HI0933_like, GH3, Bromodomain, ERO1, DAO, DUF760, Methyltransf_2, Gp_dh_C, HGTP_anticodon, Methyltransf_3, Aldo_ket_red, Thioredoxin, NmrA, SelR, LEA_5, Orn_Arg_deC_N, Polysacc_synt_2, Crp_dh_N, NifU_N, GFO_IDH_MocA_C, Gamma-thionin, FBA_1, H_PPase, ADH_N, Heme_oxygenase, AUX_IAA, NAD_binding_4, Auxin_inducible, LIM, Response_reg, Dirigent, E2F_TDP, Di19,

Alpha_adaptinC2, efhand, ICL, Rieske, GTP_EFTU, ARID, adh_short, Transket_pyr, AA_permease, TPP_enzyme_C, NDK, RRM_1, Trypsin, Pro_CA, Hexokinase_1, CBFD_NFYB_HMF, Glyco_hydro_38C, TPP_enzyme_M, TPP_enzyme_N, Hexokinase_2, 3Beta_HSD, DUF788, Wzy_C, E1_dh, Glycolytic, RuBisCO_small, ZF-HD_dimer, DUF1530, PARP, Pyridoxal_deC, I1vC, Ribosomal_L1, Alpha-amylase, EB1, CorA, Sucrose_synth, PGAM, I1vN, MAP1_LC3, DNA_photolyase, PAD_porph, Abhydrolase_1, Glyco_hydro_16, NTF2, CobW_C, GATase_2, Cation_efflux, Gln-synt_C, VQ, DUF296, W2, SAM_1, SAM_2, Gln-synt_N, Transketolase_C, PEPcase, GRIM-19, Pkinase_Tyr, DnaJ, MIP, PRA1, Trehalose_PPase, Transketolase_N, LRR_2, KA1, Mpv17_PMP22, Reticulon, Trp_syntA, YTH, Aldedh, zf-C3HC4, GIDA, Trp_Tyr_perm, UBA, PB1, PAS, Carb_kinase, zf-LSD1, CAF1, Xan_ur_permease, Hist_deacetyl, Cpn60_TCP1, XET_C, Ribosomal_L10e, Trehalase, ubiquitin, Glyco_hydro_38, AP2, Myb_DNA-binding, APS_kinase, PBD, FAE_3-kCoA_syn1; wherein the gathering cuttoff for said protein domain families is stated in Table 12;

(b) producing corn plants from said hybrid corn seed, wherein a fraction of the plants produced from said hybrid corn seed is homozygous for said recombinant DNA, a fraction of the plants produced from said hybrid corn seed is hemizygous for said recombinant DNA, and a fraction of the plants produced from said hybrid corn seed has none of said recombinant DNA;

(c) selecting corn plants which are homozygous and hemizygous for said recombinant DNA by treating with an herbicide;

(d) collecting seed from herbicide-treated-surviving corn plants and planting said seed to produce further progeny corn plants;

(e) repeating steps (c) and (d) at least once to produce an inbred corn line;

(f) crossing said inbred corn line with a second corn line to produce hybrid seed.

The method of selecting a plant comprising cells as defined above, wherein an immunoreactive antibody is used to detect the presence of said protein in seed or plant tissue.

Anti-counterfeit milled seed having, as an indication of origin, a plant cell as defined above.

A method of growing a corn, cotton or soybean crop without irrigation water comprising planting seed having plant cells of claim 1 which are selected for enhanced water use efficiency. A method as defined above comprising providing up to 300 millimeters of ground water during the production of said crop.

A method of growing a corn, cotton or soybean crop without added nitrogen fertilizer comprising planting seed having plant cells as above, which are selected for enhanced nitrogen use efficiency.

## Claims

1. A plant cell with stably integrated, recombinant DNA comprising a promoter that is functional in plant cells and that is operably linked to DNA from a plant, bacteria or yeast that encodes a protein having at least 90% identity over the full length of SEQ ID NO: 1480.

2. The plant cell of claim 1 further comprising DNA expressing a protein that provides tolerance from exposure to an herbicide applied at levels that are lethal to a wild type of said plant cell.

3. The plant cell of claim 2 wherein the agent of said herbicide is a glyphosate, dicamba, or glufosinate compound.

4. A transgenic plant comprising a plurality of the plant cell of claim 1

5. The transgenic plant of claim 4 which is homozygous for said recombinant DNA.

**6.** A transgenic seed comprising a plurality of the plant cell of claim 1.

**7.** The transgenic seed of claim 6 from a corn, soybean, cotton, canola, alfalfa, wheat or rice plant.

**8.** Non-natural, transgenic corn seed of claim 7 wherein said seed can produce corn plants that are resistant to disease from the Mal de Rio Cuarto virus or the *Puccina sorghi* fungus or both.

**9.** A transgenic pollen grain comprising a haploid derivative of a plant cell of claim 1.

**10.** A method for manufacturing non-natural, transgenic seed that can be used to produce a crop of transgenic plants with an enhanced trait resulting from expression of stably-integrated, recombinant DNA comprising a promoter that is (a) functional in plant cells and (b) is operably linked to DNA from a plant, bacteria or yeast that encodes a protein having at least 90% identity over the full length of SEQ ID NO: 1480; and wherein said enhanced trait is selected from the group of enhanced traits consisting of enhanced water use efficiency, enhanced cold tolerance, increased yield, enhanced nitrogen use efficiency, enhanced seed protein and enhanced seed oil, said method for manufacturing said seed comprising:

> (a) screening a population of plants for said enhanced trait and said recombinant DNA, wherein individual plants in said population can exhibit said trait at a level less than, essentially the same as or greater than the level that said trait is exhibited in control plants which do not express the recombinant DNA,
> (b) selecting from said population one or more plants that exhibit the trait at a level greater than the level that said trait is exhibited in control plants,
> (c) verifying that said recombinant DNA is stably integrated in said selected plants,
> (d) analyzing tissue of a selected plant to determine the production of a protein having the function of a protein encoded by nucleotides in a sequence of one of SEQ ID NO:739; and
> (e) collecting seed from a selected plant.

**11.** The method of claim 10 wherein plants in said population further comprise DNA expressing a protein that provides tolerance to exposure to an herbicide applied at levels that are lethal to wild type plant cells, and wherein said selecting is effected by treating said population with said herbicide, preferably said herbicide comprises a glyphosate, dicamba, or glufosinate compound.

**12.** The method of claim 10 wherein

> (i) said selecting is effected by identifying plants with said enhanced trait; or
> (ii) said seed is corn, soybean, cotton, alfalfa, wheat or rice seed.

**13.** The method of claim 10, wherein an immunoreactive antibody is used to detect the presence of said protein in seed or plant tissue.

**14.** A method of producing hybrid corn seed comprising:

> (a) acquiring hybrid corn seed from a herbicide tolerant corn plant which also has stably-integrated, recombinant DNA comprising a promoter that is (a) functional in plant cells and (b) is operably linked to DNA that encodes a protein having at least 90% identity over the full length of SEQ ID NO: 1480;
> (b) producing corn plants from said hybrid corn seed, wherein a fraction of the plants produced from said hybrid corn seed is homozygous for said recombinant DNA, a fraction of the plants produced from said hybrid corn seed is hemizygous for said recombinant DNA, and a fraction of the plants produced from said hybrid corn seed has none of said recombinant DNA;
> (c) selecting corn plants which are homozygous and hemizygous for said recombinant DNA by treating with an herbicide;
> (d) collecting seed from herbicide-treated-surviving corn plants and planting said seed to produce further progeny corn plants;
> (e) repeating steps (c) and (d) at least once to produce an inbred corn line;
> (f) crossing said inbred corn line with a second corn line to produce hybrid seed.

**15.** Anti-counterfeit milled seed having, as an indication of origin, a plant cell of claim 1.

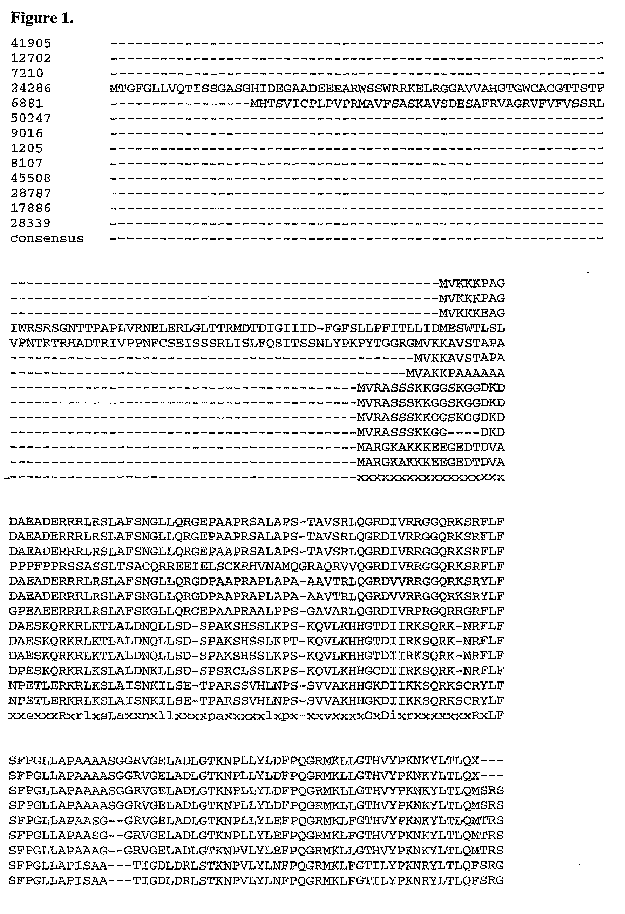

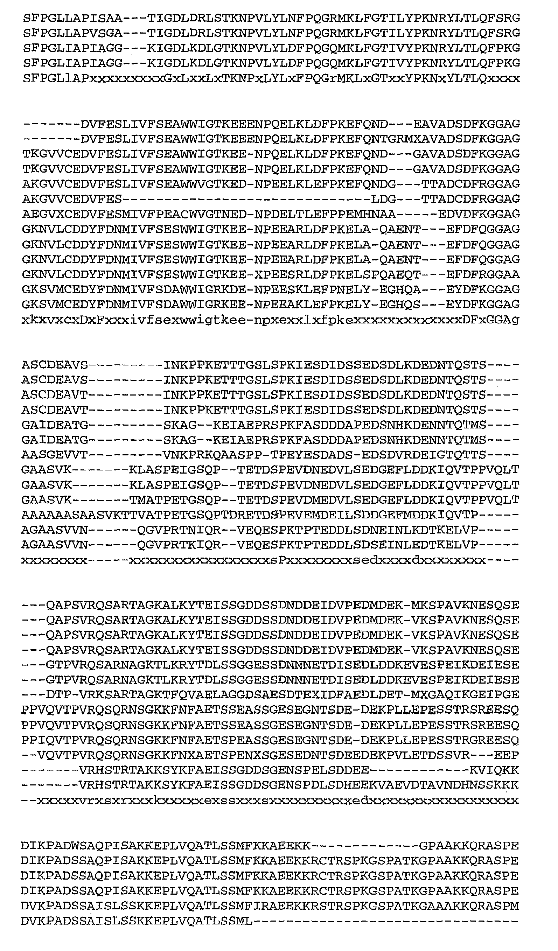

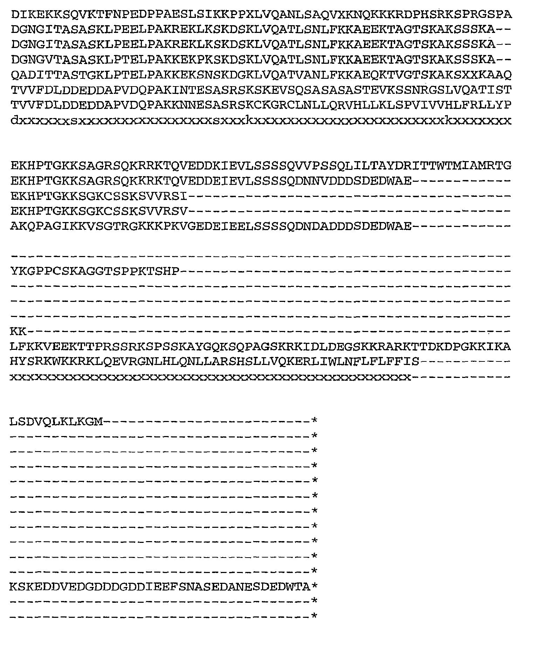

# Figure 1.

```
41905     ------------------------------------------------------------
12702     ------------------------------------------------------------
7210      ------------------------------------------------------------
24286     MTGFGLLVQTISSGASGHIDEGAADEEEARWSSWRRKELRGGAVVAHGTGWCACGTTSTP
6881      ---------------MHTSVICPLPVPRMAVFSASKAVSDESAFRVAGRVFVFVSSRL
50247     ------------------------------------------------------------
9016      ------------------------------------------------------------
1205      ------------------------------------------------------------
8107      ------------------------------------------------------------
45508     ------------------------------------------------------------
28787     ------------------------------------------------------------
17886     ------------------------------------------------------------
28339     ------------------------------------------------------------
consensus ------------------------------------------------------------
```

```
------------------------------------------------------MVKKKPAG
------------------------------------------------------MVKKKPAG
------------------------------------------------------MVKKKEAG
IWRSRSGNTTPAPLVRNELERLGLTTRMDTDIGIIID-FGFSLLPFITLLIDMESWTLSL
VPNTRTRHADTRIVPPNFCSEISSSRLISLFQSITSSNLYPKPYTGGRGMVKKAVSTAPA
----------------------------------------------MVKKAVSTAPA
--------------------------------------------MVAKKPAAAAAA
-----------------------------------------MVRASSSKKGGSKGGDKD
-----------------------------------------MVRASSSKKGGSKGGDKD
-----------------------------------------MVRASSSKKGGSKGGDKD
-----------------------------------------MVRASSSKKGG----DKD
--------------------------------------MARGKAKKKEEGEDTDVA
--------------------------------------MARGKAKKKEEGEDTDVA
--------------------------------------XXXXXXXXXXXXXXXXXXXX
```

```
DAEADERRRLRSLAFSNGLLQRGEPAAPRSALAPS-TAVSRLQGRDIVRRGGQRKSRFLF
DAEADERRRLRSLAFSNGLLQRGEPAAPRSALAPS-TAVSRLQGRDIVRRGGQRKSRFLF
DAEADERRRLRSLAFSNGLLQRGEPAAPRSALAPS-TAVSRLQGRDIVRRGGQRKSRFLF
PPPFPPRSSASSLTSACQRREEIELSCKRHVNAMQGRAQRVVQGRDIVRRGGQRKSRFLF
DAEADERRRLRSLAFSNGLLQRGDPAAPRAPLAPA-AAVTRLQGRDVVRRGGQRKSRYLF
DAEADERRRLRSLAFSNGLLQRGDPAAPRAPLAPA-AAVTRLQGRDVVRRGGQRKSRYLF
GPEAEERRRLRSLAFSKGLLQRGEPAAPRAALPPS-GAVARLQGRDIVRPRGQRRGRFLF
DAESKQRKRLKTLALDNQLLSD-SPAKSHSSLKPS-KQVLKHHGTDIIRKSQRK-NRFLF
DAESKQRKRLKTLALDNQLLSD-SPAKSHSSLKPT-KQVLKHHGTDIIRKSQRK-NRFLF
DAESKQRKRLKTLALDNQLLSD-SPAKSHSSLKPS-KQVLKHHGTDIIRKSQRK-NRFLF
DPESKQRKRLKSLALDNKLLSD-SPSRCLSSLKPS-KQVLKHHGCDIIRKSQRK-NRFLF
NPETLERKRLKSLAISNKILSE-TPARSSVHLNPS-SVVAKHHGKDIIKKSQRKSCRYLF
NPETLERKRLKSLAISNKILSE-TPARSSVHLNPS-SVVAKHHGKDIIKKSQRKSCRYLF
xxexxxRxrlxsLaxxnxllxxxxpaxxxxxlxpx-xxvxxxxGxDixrxxxxxxxRxLF
```

```
SFPGLLAPAAAASGGRVGELADLGTKNPLLYLDFPQGRMKLLGTHVYPKNKYLTLQX---
SFPGLLAPAAAASGGRVGELADLGTKNPLLYLDFPQGRMKLLGTHVYPKNKYLTLQX---
SFPGLLAPAAAASGGRVGELADLGTKNPLLYLDFPQGRMKLLGTHVYPKNKYLTLQMSRS
SFPGLLAPAAAASGGRVGELADLGTKNPLLYLDFPQGRMKLLGTHVYPKNKYLTLQMSRS
SFPGLLAPAASG--GRVGELADLGTKNPLLYLEFPQGRMKLFGTHVYPKNKYLTLQMTRS
SFPGLLAPAASG--GRVGELADLGTKNPLLYLEFPQGRMKLFGTHVYPKNKYLTLQMTRS
SFPGLLAPAAAG--GRVGELADLGTKNPVLYLEFPQGRMKLLGTHVYPKNKYLTLQMTRS
SFPGLLAPISAA---TIGDLDRLSTKNPVLYLNFPQGRMKLFGTILYPKNRYLTLQFSRG
SFPGLLAPISAA---TIGDLDRLSTKNPVLYLNFPQGRMKLFGTILYPKNRYLTLQFSRG
```

```
SFPGLLAPISAA---TIGDLDRLSTKNPVLYLNFPQGRMKLFGTILYPKNRYLTLQFSRG
SFPGLLAPVSGA---TIGDLDRLSTKNPVLYLNFPQGRMKLFGTILYPKNRYLTLQFSRG
SFPGLIAPIAGG---KIGDLKDLGTKNPVLYLDFPQGQMKLFGTIVYPKNRYLTLQFPKG
SFPGLIAPIAGG---KIGDLKDLGTKNPVLYLDFPQGQMKLFGTIVYPKNRYLTLQFPKG
SFPGLlAPxxxxxxxxxGxLxxLxTKNPxLYLxFPQGrMKLxGTxxYPKNxYLTLQxxxx


-------DVFESLIVFSEAWWIGTKEEENPQELKLDFPKEFQND---EAVADSDFKGGAG
-------DVFESLIVFSEAWWIGTKEEENPQELKLDFPKEFQNTGRMXAVADSDFKGGAG
TKGVVCEDVFESLIVFSEAWWIGTKEE-NPQELKLDFPKEFQND---GAVADSDFKGGAG
TKGVVCEDVFESLIVFSEAWWIGTKEE-NPQELKLDFPKEFQND---GAVADSDFKGGAG
AKGVVCEDVFESLIVFSEAWWVGTKED-NPEELKLEFPKEFQNDG---TTADCDFRGGAG
AKGVVCEDVFES-------------------------LDG---TTADCDFRGGAG
AEGVXCEDVFESMIVFPEACWVGTNED-NPDELTLEFPPEMHNAA-----EDVDFKGGAG
GKNVLCDDYFDNMIVFSESWWIGTKEE-NPEEARLDFPKELA-QAENT---EFDFQGGAG
GKNVLCDDYFDNMIVFSESWWIGTKEE-NPEEARLDFPKELA-QAENT---EFDFQGGAG
GKNVLCDDYFDNMIVFSESWWIGTKEE-NPEEARLDFPKELA-QAENT---EFDFQGGAG
GKNVLCDDYFDNMIVFSESWWIGTKEE-XPEESRLDFPKELSPQAEQT---EFDFRGGAA
GKSVMCEDYFDNMIVFSDAWWIGRKDE-NPEESKLEFPNELY-EGHQA---EYDFKGGAG
GKSVMCEDYFDNMIVFSDAWWIGRKEE-NPEEAKLEFPKELY-EGHQS---EYDFKGGAG
xkxvxcxDxFxxxivfsexwwigtkee-npxexxlxfpkexxxxxxxxxxxxxxDFxGGAg


ASCDEAVS---------INKPPKETTTGSLSPKIESDIDSSEDSDLKDEDNTQSTS----
ASCDEAVS---------INKPPKETTTGSLSPKIESDIDSSEDSDLKDEDNTQSTS----
ASCDEAVT---------INKPPKETTTGSLSPKIESDIDSSEDSDLKDEDNTQSTS----
ASCDEAVT---------INKPPKETTTGSLSPKIESDIDSSEDSDLKDEDNTQSTS----
GAIDEATG---------SKAG--KEIAEPRSPKFASDDDAPEDSNHKDENNTQTMS----
GAIDEATG---------SKAG--KEIAEPRSPKFASDDDAPEDSNHKDENNTQTMS----
AASGEVVT---------VNKPRKQAASPP-TPEYESDADS-EDSDVRDEIGTQTTS----
GAASVK-------KLASPEIGSQP--TETDSPEVDNEDVLSEDGEFLDDKIQVTPPVQLT
GAASVK-------KLASPEIGSQP--TETDSPEVDNEDVLSEDGEFLDDKIQVTPPVQLT
GAASVK-------TMATPETGSQP--TETDSPEVDMEDVLSEDGEFLDDKIQVTPPVQLT
AAAAAASAASVKTTVATPETGSQPTDRETDSPEVEMDEILSDDGEFMDDKIQVTP-----
AGAASVVN------QGVPRTNIQR--VEQESPKTPTEDDLSDNEINLKDTKELVP-----
AGAASVVN------QGVPRTKIQR--VEQESPKTPTEDDLSDSEINLEDTKELVP-----
xxxxxxxx----xxxxxxxxxxxxxxxxsPxxxxxxxxsedxxxxdxxxxxxxx----


---QAPSVRQSARTAGKALKYTEISSGDDSSDNDDEIDVPEDMDEK-MKSPAVKNESQSE
---QAPSVRQSARTAGKALKYTEISSGDDSSDNDDEIDVPEDMDEK-VKSPAVKNESQSE
---QAPSVRQSARTAGKALKYTEISSGDDSSDNDDEIDVPEDMDEK-VKSPAVKNESQSE
---QAPSVRQSARTAGKALKYTEISSGDDSSDNDDEIDVPEDMDEK-VKSPAVKNESQSE
---GTPVRQSARNAGKTLKRYTDLSSGGESSDNNNETDISEDLDDKEVESPEIKDEIESE
---GTPVRQSARNAGKTLKRYTDLSSGGESSDNNNETDISEDLDDKEVESPEIKDEIESE
---DTP-VRKSARTAGKTFQVAELAGGDSAESDTEXIDFAEDLDET-MXGAQIKGEIPGE
PPVQVTPVRQSQRNSGKKFNFAETSSEASSGESEGNTSDE-DEKPLLEPESSTRSREESQ
PPVQVTPVRQSQRNSGKKFNFAETSSEASSGESEGNTSDE-DEKPLLEPESSTRSREESQ
PPIQVTPVRQSQRNSGKKFNFAETSPEASSGESEGNTSDE-DEKPLLEPESSTRGREESQ
--VQVTPVRQSQRNSGKKFNXAETSPENXSGESEDNTSDEEDEKPVLETDSSVR---EEP
-------VRHSTRTAKKSYKFAEISSGDDSGENSPELSDDEE----------KVIQKK
-------VRHSTRTAKKSYKFAEISSGDDSGENSPDLSDHEEKVAEVDTAVNDHNSSKKK
--xxxxxvrxsxrxxxkxxxxxexssxxxsxxxxxxxxxxxedxxxxxxxxxxxxxxxxxx


DIKPADWSAQPISAKKEPLVQATLSSMFKKAEEKK------------GPAAKKQRASPE
DIKPADSSAQPISAKKEPLVQATLSSMFKKAEEKKRCTRSPKGSPATKGPAAKKQRASPE
DIKPADSSAQPISAKKEPLVQATLSSMFKKAEEKKRCTRSPKGSPATKGPAAKKQRASPE
DIKPADSSAQPISAKKEPLVQATLSSMFKKAEEKKRCTRSPKGSPATKGPAAKKQRASPE
DVKPADSSAISLSSKKEPLVQATLSSMFIRAEEKKRSTRSPKGSPATKGAAAKKQRASPM
DVKPADSSAISLSSKKEPLVQATLSSML---------------------------------
```

```
DIKEKKSQVKTFNPEDPPAESLSIKKPPXLVQANLSAQVXKNQKKKRDPHSRKSPRGSPA
DGNGITASASKLPEELPAKREKLKSKDSKLVQATLSNLFKKAEEKTAGTSKAKSSSKA--
DGNGITASASKLPEELPAKREKLKSKDSKLVQATLSNLFKKAEEKTAGTSKAKSSSKA--
DGNGVTASASKLPTELPAKKEKPKSKDSKLVQATLSNLFKKAEEKTAGTSKAKSSSKA--
QADITTASTGKLPTELPAKKEKSNSKDGKLVQATVANLFKKAEQKTVGTSKAKSXXKAAQ
TVVFDLDDEDDAPVDQPAKINTESASRSKSKEVSQSASASASTEVKSSNRGSLVQATIST
TVVFDLDDEDDAPVDQPAKKNNESASRSKCKGRCLNLLQRVHLLKLSPVIVVHLFRLLYP
dxxxxxxsxxxxxxxxxxxxxxxxxxsxxxkxxxxxxxxxxxxxxxxxxxxxkxxxxxxx


EKHPTGKKSAGRSQKRRKTQVEDDKIEVLSSSSQVVPSSQLILTAYDRITTWTMIAMRTG
EKHPTGKKSAGRSQKKRKTQVEDDEIEVLSSSSQDNNVDDDSDEDWAE------------
EKHPTGKKSGKCSSKSVVRSI---------------------------------------
EKHPTGKKSGKCSSKSVVRSV---------------------------------------
AKQPAGIKKVSGTRGKKKPKVGEDEIEELSSSSQDNDADDDSDEDWAE------------


------------------------------------------------------------
YKGPPCSKAGGTSPPKTSHP----------------------------------------
------------------------------------------------------------
------------------------------------------------------------
------------------------------------------------------------
KK----------------------------------------------------------
LFKKVEEKTTPRSSRKSPSSKAYGQKSQPAGSKRKIDLDEGSKKRARKTTDKDPGKKIKA
HYSRKWKKRKLQEVRGNLHLQNLLARSHSLLVQKERLIWLNFLFLFFIS-----------
XXXXXXXXXXXXXXXXXXXXXXXXXXXXXXXXXXXXXXXXXXXXXXXXXX-----------


LSDVQLKLKGM-----------------------*
----------------------------------*
----------------------------------*
----------------------------------*
----------------------------------*
----------------------------------*
----------------------------------*
----------------------------------*
----------------------------------*
----------------------------------*
KSKEDDVEDGDDDGDDIEEFSNASEDANESDEDWTA*
----------------------------------*
----------------------------------*
```

## REFERENCES CITED IN THE DESCRIPTION

### Patent documents cited in the description

- US 60638099 B
- US 5858742 A
- US 5322938 A
- US 5641876 A
- US 20020192813 A1
- US 757089 A
- US 706946 A
- US 310370 P
- US 5593874 A
- US 5420034 A
- US 6433252 B
- US 6090627 A
- US 5188642 A
- US 5728925 A
- US 5094945 A
- US 5627061 A
- US 5633435 A
- US 6040497 A
- US 5463175 A
- US 20030083480 A1
- US 20030135879 A1
- US 4810648 A
- US 2003010609 A1
- US 6107549 A
- US 6376754 B
- US 20020112260 A
- US 5250515 A
- US 5880275 A
- US 6506599 B
- US 5986175 A
- US 20030150017 A1
- US 4196265 A
- US 5015580 A
- US 5550318 A
- US 5538880 A
- US 5914451 A
- US 6160208 A
- US 6399861 B
- US 6153812 A
- US 5159135 A
- US 5824877 A
- US 5591616 A
- US 6384301 B
- US 4959317 A
- US 5527695 A
- US 6194636 B
- US 6232526 B
- US 5780708 A
- US 6118047 A
- US 6399861 A

### Non-patent literature cited in the description

- **S.R. EDDY.** Profile Hidden Markov Models. *Bioinformatics,* 1998, vol. 14, 755-763
- **FISCHHOFF et al.** *Plant Mol Biol.,* 1992, vol. 20, 81-93
- **TANIGUCHI et al.** *Plant Cell Physiol.,* 2000, vol. 41 (1), 42-48
- **RUSSELL et al.** *Transgenic Res.,* 1997, vol. 6 (2), 157-166
- **BELANGER et al.** *Genetics,* 1991, vol. 129, 863-872
- **STACY et al.** *Plant Mol Biol.,* 1996, vol. 31 (6), 1205-1216
- **KLEE, H.J. et al.** *MGG,* 1987, vol. 210, 437-442
- **MISAWA et al.** *Plant J.,* 1993, vol. 4, 833-840
- **MISAWA et al.** *Plant J.,* 1994, vol. 6, 481-489
- **SATHASIIVAN et al.** *Nucl. Acids Res.,* 1990, vol. 18, 2188-2193
- **DEBLOCK et al.** *EMBO J.,* 1987, vol. 6, 2513-2519
- Antibodies: A Laboratory Manual. Cold Spring Harbor Laboratory, 1988
- *Goding,* 1986, 60-61
- **AN et al.** *Plant Cell,* 1989, vol. 1, 115-122
- **DEPICKER, A. et al.** *Mol Appl Genet,* 1982, vol. 1, 561-573
- **BARKER, R.F. et al.** *Plant Mol Biol,* 1983, vol. 2, 335-350
- **MCDOWELL et al.** *Plant Physiol.,* 1996, vol. 111, 699-711